# EUROPEAN PATENT SPECIFICATION

(11) **EP 2 661 487 B1**
(45) Date of publication and mention of the grant of the patent: **28.06.2017**
(21) Application number: 12732303.8
(22) Date of filing: 04.01.2012
(51) Int. Cl.: C12N 1/19, C12N 15/53, C12N 9/02, C12P 7/44, C12N 9/16, C12N 9/00, C12N 15/81

(54) **BIOLOGICAL METHODS FOR PREPARING ADIPIC ACID**
BIOLOGISCHES VERFAHREN ZUR HERSTELLUNG VON ADIPINSÄURE
PROCÉDÉS BIOLOGIQUES POUR PRÉPARER DE L'ACIDE ADIPIQUE

(30) Priority: 03.05.2011 US 201161482160 P; 05.01.2011 US 201161430097 P
(43) Date of publication of application: 13.11.2013
(73) Proprietor: Verdezyne, Inc., Carlsbad, CA 92010 (US)
(72) Inventor: PICATAGGIO, Stephen, Carlsbad, CA 92010 (US); BEARDSLEE, Tom, Carlsbad, CA 92010 (US)
(74) Representative: Ali, Suleman
(86) International application number: PCT/US2012/020230
(87) International publication number: WO 2012/094425

(56) References cited:
- WO-A2-2011/003034
- WO-A2-2011/003034
- US-A- 5 962 285
- US-A1- 2010 291 653
- US-B1- 6 331 420
- DATABASE GENBANK [Online] 27 April 1993 'Acyl-coenzyme A oxidase II precursor [Candida tropicalis]', XP003030261 Database accession no. AAA34362
- JAE-YEON CHOI ET AL.: 'The saccharomyces cerevisiae FAT1 gene encodes an acyl-CoA synthetase that is required for maintenance of very long chain fatty acid levels.' THE JOURNAL OF BIOLOGICAL CHEMISTRY vol. 274, no. 8, 19 February 1999, pages 4671 - 4683, XP002904758

## Description

### Field

The technology relates in part to biological methods for producing adipic acid and engineered microorganisms capable of such production.

### Background

Microorganisms employ various enzyme-driven biological pathways to support their own metabolism and growth. A cell synthesizes native proteins, including enzymes, in vivo from deoxyribonucleic acid (DNA). DNA first is transcribed into a complementary ribonucleic acid (RNA) that comprises a ribonucleotide sequence encoding the protein. RNA then directs translation of the encoded protein by interaction with various cellular components, such as ribosomes. The resulting enzymes participate as biological catalysts in pathways involved in production of molecules by the organism.

These pathways can be exploited for the harvesting of the naturally produced products. The pathways also can be altered to increase production or to produce different products that may be commercially valuable. Advances in recombinant molecular biology methodology allow researchers to isolate DNA from one organism and insert it into another organism, thus altering the cellular synthesis of enzymes or other proteins. Such genetic engineering can change the biological pathways within the host organism, causing it to produce a desired product. Microorganic industrial production can minimize the use of caustic chemicals and the production of toxic byproducts, thus providing a "clean" source for certain compounds.

### Summary

Provided herein are engineered microorganisms that produce six-carbon organic molecules such as adipic acid, methods for manufacturing such microorganisms and methods for using them to produce adipic acid and other six-carbon organic molecules. Also provided herein are engineered microorganisms including genetic alterations that direct carbon flux towards adipic acid through increased fatty acid production in conjunction with increased omega and beta oxidation activities, methods for manufacturing such organisms and methods for using them to produce adipic acid and other six-carbon organic molecules. The engineered microorganism of the invention is genetically modified Candida yeast.

The invention provides a composition comprising genetically modified Candida yeast comprising: (a) an altered acyl-CoA oxidase activity, which altered acyl-CoA oxidase activity results from a disruption, deletion or knockout of (i) a polynucleotide that encodes a POX4 polypeptide whereby the acyl-CoA oxidase activity is reduced or removed, wherein said POX4 polypeptide comprises the amino acid sequence of SEQ ID NO: 39, an amino acid sequence 90% or more identical to SEQ ID NO: 39, or an amino acid sequence that includes 1 to 10 amino acid substitutions, insertions or deletions with respect to SEQ ID NO: 39; and (b) an altered long chain acyl-CoA synthetase activity, which altered long chain acyl- CoA synthetase activity results from a disruption, deletion or knockout of (i) a polynucleotide that encodes a FAT1 polypeptide whereby the long chain acyl-CoA synthetase activity is reduced or removed, wherein said FAT1 polypeptide comprises the amino acid sequence of SEQ ID NO: 51, an amino acid sequence 90% or more identical to SEQ ID NO: 51, or an amino acid sequence that includes 1 to 10 amino acid substitutions, insertions or deletions with respect to SEQ ID NO: 51.

Thus, provided herein in some embodiments are engineered microorganisms capable of producing adipic acid, which microorganisms comprise one or more altered activities selected from the group consisting of aldehyde dehydrogenase activity (e.g., 6-oxohexanoic acid dehydrogenase activity, omega oxo fatty acid dehydrogenase activity), fatty alcohol oxidase activity (e.g., 6-hydroxyhexanoic acid dehydrogenase activity, omega hydroxyl fatty acid dehydrogenase activity), glucose-6-phosphate dehydrogenase activity, hexanoate synthase activity, lipase activity, fatty acid synthase activity, acetyl CoA carboxylase activity, acyl-CoA hydrolase (e.g., ACH; thioesterase) activity, acyl-CoA thioesterase (e.g., TESA) activity, acyl-CoA synthetase (e.g., ACS1) activity, long chain acyl-CoA synthetase (e.g., FAT1) activity, acyl-CoA sterol acyl transferase (e.g., ARE1, ARE2, or ARE1 and ARE2) activity, acyltransferase activity (e.g., diacyl-glycerol acyl transferase, DGA1, LRO1, or DGA1 and LRO1) activity and monooxygenase activity.

In certain embodiments, the microorganism comprises a genetic modification that adds or increases the aldehyde dehydrogenase activity (e.g., 6-oxohexanoic acid dehydrogenase activity, omega oxo fatty acid dehydrogenase activity), fatty alcohol oxidase activity (e.g., 6-hydroxyhexanoic acid dehydrogenase activity, omega hydroxyl fatty acid dehydrogenase activity), glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, enoyl-CoA hydratase activity, 3-hydroxyacyl-CoA dehydrogenase activity, acyl-CoA hydrolase activity, acyl-CoA thioesterase activity and/or acetyl-CoA C-acyltransferase activity. Also provided in some embodiments, are engineered microorganisms that produce adipic acid, which microorganisms comprise an altered monooxygenase activity. Provided also herein in some embodiments are engineered microorganisms that include a genetic modification that reduces the acyl-CoA oxidase activity, acyl-CoA synthetase activity, long chain acyl-CoA synthetase activity, acyl-CoA sterol acyl transferase activity, and/or acyltransferase activity (e.g., diacyl-glycerol acyltransferase).

In some embodiments, an engineered microorganism includes a genetic modification that includes multiple copies of a polynucleotide that encodes a polypeptide having aldehyde dehydrogenase activity (e.g., 6-oxohexanoic acid dehydrogenase activity, omega oxo fatty acid dehydrogenase activity), fatty alcohol oxidase activity (e.g., 6-hydroxyhexanoic acid dehydrogenase activity, omega hydroxyl fatty acid dehydrogenase activity), glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, enoyl-CoA hydratase activity, 3-hydroxyacyl-CoA dehydrogenase activity, acyl-CoA hydrolase activity, acyl-CoA thioesterase activity and/or acetyl-CoA C-acyltransferase activity. In some embodiments, 2, 3, 4, 5, 6, 7, 8, 9, 10, 15, 20, 25, 30, 35, 40, 45, 50 or more copies of the particular polynucleotide are present in the microbe. In certain embodiments, an engineered microorganism includes a heterologous promoter (and/or 5'UTR) in functional connection with a polynucleotide that encodes a polypeptide having aldehyde dehydrogenase activity (e.g., 6-oxohexanoic acid dehydrogenase activity, omega oxo fatty acid dehydrogenase activity), fatty alcohol oxidase activity (e.g., 6-hydroxyhexanoic acid dehydrogenase activity, omega hydroxyl fatty acid dehydrogenase activity), glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, enoyl-CoA hydratase activity, 3-hydroxyacyl-CoA dehydrogenase activity, acyl-CoA hydrolase activity, acyl-CoA thioesterase activity and/or acetyl-CoA C-acyltransferase activity. In some embodiments, the promoter is a POX4 or POX5 promoter or monooxygenase promoter from a yeast (e.g., Candida yeast strain (e.g., C. tropicalis, C. viswanithii)), or other promoter. Examples of promoters that can be utilized are described herein. The promoter sometimes is exogenous or endogenous with respect to the microbe.

Also provided herein is an engineered microorganism that produces adipic acid, where the microorganism includes an altered monooxygenase activity. In certain embodiments, an engineered microorganism comprises a genetic modification that alters a monooxygenase activity. In some embodiments, an engineered microorganism includes a genetic modification that alters a monooxygenase activity selected from the group consisting of CYP52A12 activity, CYP52A13 activity, CYP52A14 activity, CYP52A15 activity, CYP52A16 activity, CYP52A17 activity, CYP52A18 activity, CYP52A19 activity, CYP52A20 activity, CYP52D2 activity, and/or BM3 activity (e.g., from B. megaterium). In certain embodiments, an engineered microorganism includes one or more genetically modified monooxygenase activities selected from the group consisting of CYP52A12 activity, CYP52A13 activity, CYP52A14 activity, CYP52A15 activity, CYP52A16 activity, CYP52A17 activity, CYP52A18 activity, CYP52A19 activity, CYP52A20 activity, CYP52D2 activity, and/or BM3 activity. In some embodiments, the monooxygenase activity is encoded by a CYP52A12 polynucleotide, a CYP52A13 polynucleotide, a CYP52A14 polynucleotide, a CYP52A15 polynucleotide, a CYP52A16 polynucleotide, a CYP52A17 polynucleotide, a CYP52A18 polynucleotide, a CYP52A19 polynucleotide, a CYP52A20 polynucleotide, a CYP52D2 polynucleotide, and/or a BM3 polynucleotide. In certain embodiments, an engineered microorganism includes one or more monooxygenase activities encoded by polynucleotides selected from the group consisting of a CYP52A12 polynucleotide, a CYP52A13 polynucleotide, a CYP52A14 polynucleotide, a CYP52A15 polynucleotide, a CYP52A16 polynucleotide, a CYP52A17 polynucleotide, a CYP52A18 polynucleotide, a CYP52A19 polynucleotide, a CYP52A20 polynucleotide, a CYP52D2 polynucleotide, and/or a BM3 polynucleotide. In some embodiments, the genetic modification increases monooxygenase activity. In certain embodiments, the genetic modification increases the copy number of an endogenous polynucleotide that encodes a polypeptide having the monooxygenase activity (e.g., 2, 3, 4, 5, 6, 7, 8, 9, 10, 15, 20, 25, 30, 35, 40, 45, 50 or more copies of the polynucleotide). In certain embodiments, an engineered microorganism comprises one or more polynucleotides that includes a promoter (e.g., promoter and/or 5'UTR) and encodes a polypeptide having a monooxygenase activity. The promoter may be exogenous or endogenous with respect to the microbe. An engineered microorganism in certain embodiments comprises one or more heterologous polynucleotides encoding a polypeptide having monooxygenase activity. In related embodiments, the heterologous polynucleotide is from a yeast, such as a Candida yeast in certain embodiments (e.g., C. tropicalis, C. viswanithii), or from a bacteria, such as Bacillus bacteria in some embodiments (e.g., B. megaterium).

In certain embodiments, an engineered microorganism comprises a genetic modification that alters monooxygenase reductase activity. In some embodiments, an engineered microorganism includes a genetic modification that alters a monooxygenase reductase activity selected from the group consisting of NADPH cytochrome P450 reductase (e.g., CPR, from C. tropicalis strain ATCC750), NADPH cytochrome P450 reductase A (e.g., CPRA, from C. tropicalis strain ATCC20336), NADPH cytochrome P450 reductase B (e.g., CPRB, from C. tropicalis strain ATCC20336) and/or cytochrome P450:NADPH P450 reductase (e.g., B. megaterium). In certain embodiments, an engineered microorganism includes one or more genetically modified monooxygenase reductase activities selected from the group consisting of NADPH cytochrome P450 reductase (e.g., CPR), NADPH cytochrome P450 reductase A (e.g., CPRA), NADPH cytochrome P450 reductase B (e.g., CPRB) and/or cytochrome P450:NADPH P450 reductase. In some embodiments, the genetic modification increases the copy number of an endogenous polynucleotide that encodes a polypeptide having monooxygenase reductase activity (e.g., 2, 3, 4, 5, 6, 7, 8, 9, 10, 15, 20, 25, 30, 35, 40, 45, 50 or more copies of the polynucleotide). In certain embodiments, an engineered microorganism comprises one or more polynucleotides that includes a promoter (e.g., promoter and/or 5'UTR) and encodes a polypeptide having a monooxygenase reductase activity. The promoter may be exogenous or endogenous with respect to the microbe. In some embodiments, the polynucleotide is from a yeast, and in certain embodiments the yeast is a Candida yeast (e.g., C. tropicalis, C. viswanithii). In some embodiments, the polynucleotide is from a bacteria, and in certain embodiments the bacteria is a Bacillus bacteria (e.g., B. megaterium).

An engineered microorganism in some embodiments comprises an altered thioesterase activity. In some embodiments, an engineered microorganism comprises a genetic modification that alters the thioesterase activity, and in certain embodiments, the engineered microorganism comprises a genetic alteration that adds or increases a thioesterase activity. In some embodiments, the engineered microorganism comprises a heterologous polynucleotide encoding a polypeptide having thioesterase activity. In certain embodiments, an engineered microorganism includes a genetic modification that alters a thioesterase activity selected from the group consisting of acyl-CoA hydrolase activity (e.g., ACHA, ACHB, ACHA and ACHB, from C. tropicalis), acyl-CoA thioesterase activity (e.g., TESA, from E. coli), and/or acyl-CoA hydrolase and acyl-CoA thioesterase activity. In some embodiments, the genetic modification increases the copy number of an endogenous polynucleotide that encodes a polypeptide having thioesterase activity (e.g., 2, 3, 4, 5, 6, 7, 8, 9, 10, 15, 20, 25, 30, 35, 40, 45, 50 or more copies of the polynucleotide). In certain embodiments, an engineered microorganism comprises one or more polynucleotides that includes a promoter (e.g., promoter and/or 5'UTR) and encodes a polypeptide having a thioesterase activity. The promoter may be exogenous or endogenous with respect to the microbe. In some embodiments, the polynucleotide is from a yeast, and in certain embodiments the yeast is a Candida yeast (e.g., C. tropicalis, C. viswanithii). In some embodiments, the polynucleotide is from a bacteria, and in certain embodiments the bacteria is an Enteric bacteria (e.g., Eschericia coli). Examples of polynucleotide sequences that encode peptides with thioesterase activity, and polypeptide sequences with thioesterase activity are provided herein (e.g., SEQ ID NOs: 42-47)

An engineered microorganism in some embodiments comprises an altered fatty alcohol oxidase activity. In some embodiments, an engineered microorganism comprises a genetic modification that alters the fatty alcohol oxidase activity, and in certain embodiments, the engineered microorganism comprises a genetic alteration that adds or increases a fatty alcohol oxidase activity. In some embodiments, the genetic modification increases the copy number of an endogenous polynucleotide that encodes a polypeptide having fatty alcohol oxidase activity (e.g., 2, 3, 4, 5, 6, 7, 8, 9, 10, 15, 20, 25, 30, 35, 40, 45, 50 or more copies of the polynucleotide). An engineered microorganism in certain embodiments comprises a heterologous promoter (e.g., endogenous or exogenous promoter with respect to the microbe) in functional connection with a polynucleotide that encodes a polypeptide having fatty alcohol oxidase activity. In some embodiments, the engineered microorganism comprises a heterologous polynucleotide encoding a polypeptide having fatty alcohol oxidase activity. In some embodiments, the polynucleotide is from a yeast, and in certain embodiments the yeast is Candida (e.g., a C. tropicalis strain).

An engineered microorganism in some embodiments comprises an altered 6-oxohexanoic acid dehydrogenase activity or an altered omega oxo fatty acid dehydrogenase activity. In some embodiments, an engineered microorganism comprises a genetic modification that adds or increases 6-oxohexanoic acid dehydrogenase activity or omega oxo fatty acid dehydrogenase activity, and in certain embodiments, an engineered microorganism comprises a heterologous polynucleotide encoding a polypeptide having 6-oxohexanoic acid dehydrogenase activity or omega oxo fatty acid dehydrogenase activity. In related embodiments, the heterologous polynucleotide sometimes is from a bacterium, such as an Acinetobacter, Nocardia, Pseudomonas or Xanthobacter bacterium in some embodiments.

An engineered microorganism in some embodiments comprises an altered 6-hydroxyhexanoic acid dehydrogenase activity or an altered omega hydroxyl fatty acid dehydrogenase activity. In some embodiments, an engineered microorganism comprises a genetic modification that adds or increases the 6-hydroxyhexanoic acid dehydrogenase activity or omega hydroxyl fatty acid dehydrogenase activity, and in certain embodiments, an engineered microorganism comprises a heterologous polynucleotide encoding a polypeptide having 6-hydroxyhexanoic acid dehydrogenase activity or omega hydroxyl fatty acid dehydrogenase activity. In related embodiments, the heterologous polynucleotide is from a bacterium, such as an Acinetobacter, Nocardia, Pseudomonas or Xanthobacter bacterium in some embodiments.

An engineered microorganism in some embodiments comprises an altered fatty acid synthase activity. In some embodiments, an engineered microorganism comprises a genetic modification that alters fatty acid synthase activity. In certain embodiments, an engineered microorganism includes a genetic alteration that adds or increases fatty acid synthase activity. In some embodiments, the genetic modification increases the copy number of endogenous polynucleotides that encode polypeptides having fatty acid synthase activity (e.g., 2, 3, 4, 5, 6, 7, 8, 9, 10, 15, 20, 25, 30, 35, 40, 45, 50 or more copies of a polynucleotide). An engineered microorganism in certain embodiments comprises a heterologous promoter (e.g., endogenous or exogenous promoter with respect to the microbe) in functional connection with a polynucleotide that encodes a polypeptide having fatty acid synthase activity. In some embodiments, the engineered microorganism comprises a heterologous polynucleotide encoding a polypeptide having fatty acid synthase activity. In certain embodiments, the fatty acid synthase activity is provided by one or more polypeptides having fatty acid synthase activity (e.g., a single subunit protein or multi subunit protein). In certain embodiments, the fatty acid synthase activity is provided by a polypeptide having fatty acid synthase subunit alpha (e.g., FAS2) activity, fatty acid synthase subunit beta (e.g., FAS1) activity, or fatty acid synthase subunit alpha activity and fatty acid synthase subunit beta activity. In some embodiments a fatty acid synthase activity comprises a hexanoate synthase activity. In certain embodiments, the polynucleotide is from a yeast, and in certain embodiments the yeast is a Candida yeast (e.g., C. tropicalis, C. viswanithii). Examples of polynucleotides that encode fatty acid synthase molecules (e.g., FAS1, FAS2) are provided herein (e.g., SEQ ID NOs: 31 and 32).

An engineered microorganism in some embodiments comprises an altered hexanoate synthase activity. In some embodiments, an engineered microorganism comprises a genetic modification that alters hexanoate synthase activity. In certain embodiments, an engineered microorganism includes a genetic alteration that adds or increases hexanoate synthase activity. In some embodiments, an engineered microorganism comprises a heterologous polynucleotide encoding a polypeptide having hexanoate synthase activity. In certain embodiments, the hexanoate synthase activity is provided by a polypeptide having hexanoate synthase activity. In certain embodiments, the hexanoate synthase activity is provided by a polypeptide having hexanoate synthase subunit A activity, hexanoate synthase subunit B activity, or hexanoate synthase subunit A activity and hexanoate synthase subunit B activity. In some embodiments, the heterologous polynucleotide is from a fungus, such as an Aspergillus fungus in certain embodiments (e.g., A. parasiticus, A. nidulans).

In certain embodiments, an engineered microorganism comprises a genetic modification that results in substantial (e.g., primary) hexanoate usage by monooxygenase activity. In related embodiments, the genetic modification reduces a polyketide synthase activity.

An engineered microorganism in some embodiments comprises an altered lipase activity. In certain embodiments, an engineered microorganism includes a genetic alteration that adds or increases a lipase activity. In some embodiments, the genetic modification increases the copy number of endogenous polynucleotides that encode polypeptides having lipase activity (e.g., 2, 3, 4, 5, 6, 7, 8, 9, 10, 15, 20, 25, 30, 35, 40, 45, 50 or more copies of a polynucleotide). An engineered microorganism in certain embodiments comprises a heterologous promoter (e.g., endogenous or exogenous promoter with respect to the microbe) in functional connection with a polynucleotide that encodes a polypeptide having lipase activity. In some embodiments, the engineered microorganism comprises a heterologous polynucleotide encoding a polypeptide having lipase activity. In certain embodiments, the lipase activity is provided by one or more polypeptides having lipase activity (e.g., a single subunit protein or multi subunit protein). In certain embodiments, the lipase activity is provided by a polypeptide comprising all or part of the amino acid sequence of SEQ ID NO: 28 or 29, and sometimes the polypeptide is encoded by a polynucleotide of SEQ ID NO: 27. In certain embodiments, the polynucleotide is from a yeast, and in certain embodiments the yeast is a Candida yeast (e.g., C. tropicalis, C. viswanithii).

An engineered microorganism in some embodiments comprises an altered acetyl-CoA carboxylase activity. In certain embodiments, an engineered microorganism includes a genetic alteration that adds or increases a acetyl-CoA carboxylase activity. In some embodiments, the genetic modification increases the copy number of endogenous polynucleotides that encode polypeptides having acetyl-CoA carboxylase activity (e.g., 2, 3, 4, 5, 6, 7, 8, 9, 10, 15, 20, 25, 30, 35, 40, 45, 50 or more copies of a polynucleotide). An engineered microorganism in certain embodiments comprises a heterologous promoter (e.g., endogenous or exogenous promoter with respect to the microbe) in functional connection with a polynucleotide that encodes a polypeptide having acetyl-CoA carboxylase activity. In some embodiments, the engineered microorganism comprises a heterologous polynucleotide encoding a polypeptide having acetyl-CoA carboxylase activity. In some embodiments, the engineered microorganism comprises a heterologous polynucleotide encoding a polypeptide having acetyl-CoA carboxylase activity. In some embodiments, the polynucleotide is from a yeast, and in certain embodiments the yeast is Candida (e.g., a C. tropicalis strain). In some embodiments an acetyl-CoA carboxylase polypeptide is encoded by a polynucleotide comprising the sequence of SEQ ID NO 30.

An engineered microorganism in some embodiments is a non-prokaryotic organism, and sometimes is a eukaryote. A eukaryote can be a yeast in some embodiments, such as a Candida yeast (e.g., C. tropicalis, C. viswanithii), or a Yarrowia yeast (e.g., Y. lipolytica), for example. In certain embodiments a eukaryote is a fungus such as an Aspergillus fungus (e.g., A. parasiticus or A. nidulans), for example.

In some embodiments, an engineered microorganism comprises a genetic modification that reduces 6-hydroxyhexanoic acid conversion. In related embodiments, the genetic modification reduces 6-hydroxyhexanoic acid dehydrogenase activity or omega hydroxyl fatty acid dehydrogenase activity.

In certain embodiments, an engineered microorganism comprises a genetic modification that reduces beta-oxidation activity, and in some embodiments, the genetic modification renders beta-oxidation activity undetectable (e.g., completely blocked beta-oxidation activity). In certain embodiments, the genetic modification partially reduces beta-oxidation activity.

A fatty acid-CoA derivative, or dicarboxylic acid-CoA derivative, can be converted to a trans-2,3-dehydroacyl-CoA derivative by the activity of acyl-CoA oxidase (e.g., also known as or referred to as acyl-CoA oxidoreductase and fatty acyl-coenzyme A oxidase), in many organisms. In some embodiments, an engineered microorganism comprises a genetic modification that alters the specificity of and/or reduces the activity of an acyl-CoA oxidase activity. In certain embodiments, the genetic modification disrupts an acyl-CoA oxidase activity. In some embodiments, the genetic modification includes disrupting a polynucleotide that encodes a polypeptide having an acyl-CoA oxidase activity. In certain embodiments, the genetic modification includes disrupting a promoter and/or 5'UTR in functional connection with a polynucleotide that encodes a polypeptide having the acyl-CoA oxidase activity. In some embodiments, the polypeptide having acyl-CoA activity is a POX polypeptide. In certain embodiments, the POX polypeptide is a POX4 polypeptide, a POX5 polypeptide, or a POX4 polypeptide and POX5 polypeptide. In certain embodiments, the genetic modification disrupts an acyl-CoA activity by disrupting a POX4 nucleotide sequence, a POX5 nucleotide sequence, or a POX4 and POX5 nucleotide sequence.

In some embodiments, an engineered microorganism comprises a genetic modification that increases beta-oxidation activity. In certain embodiments, the beta-oxidation increase in beta-oxidation activity is the result of an increase in activity in one or more activities involved in beta-oxidation. In some embodiments, the genetic modification increases the copy number of an endogenous polynucleotide that encodes a polypeptide having an activity involved in beta-oxidation (e.g., 2, 3, 4, 5, 6, 7, 8, 9, 10, 15, 20, 25, 30, 35, 40, 45, 50 or more copies of the polynucleotide). An engineered microorganism in certain embodiments comprises a heterologous promoter (e.g., endogenous or exogenous promoter with respect to the microbe) in functional connection with a polynucleotide that encodes a polynucleotide that encodes a polypeptide having an activity involved in beta-oxidation. In some embodiments, the engineered microorganism comprises a heterologous polynucleotide encoding a polynucleotide that encodes a polypeptide having an activity involved in beta-oxidation. In certain embodiments, beta oxidation activity that is increased is an acyl-CoA oxidase activity, and in some embodiments the acyl-CoA oxidase activity is an activity encoded by the POX5 gene. In some embodiments, the altered activity (e.g., increased activity) is provided by a polypeptide encoded by a polynucleotide native to the host organism (e.g., multiple copies of the native polynucleotide, promoter inserted in functional connection with the with the native polynucleotide). In certain embodiments, the polynucleotide is from a yeast, and in certain embodiments the yeast is Candida (e.g., a C. tropicalis strain, C. viswanithii strain).

In some embodiments, an engineered microorganism comprises a genetic modification that increases omega-oxidation activity. In some embodiments, an engineered microorganism comprises one or more genetic modifications that alter a reverse activity in a beta oxidation pathway, an omega oxidation pathway, or a beta oxidation and omega oxidation pathway, thereby increasing carbon flux through the respective pathways, due to the reduction in one or more reverse enzymatic activities.

An engineered microorganism can include a heterologous polynucleotide that encodes a polypeptide providing an activity described above, and the heterologous polynucleotide can be from any suitable microorganism. Examples of microorganisms are described herein (e.g., Candida yeast, Saccharomyces yeast, Yarrowia yeast, Pseudomonas bacteria, Bacillus bacteria, Clostridium bacteria, Eubacterium bacteria and others include Megasphaera bacteria.

Also provided herein are engineered microorganisms including genetic alterations that direct carbon flux (e.g., carbon metabolism) towards the production of adipic acid by increasing production and/or accumulation of fatty acids and increasing omega oxidation and beta oxidation activities. In certain embodiments, the genetic alterations are selected to maximize production of adipic acid from certain feedstocks (e.g., sugars, cellulose, triacylglycerides, fatty acids, the like and combinations thereof). In some embodiments, an engineered microorganism comprises a genetic modification that reduces activities associated with generation of biomass and/or carbon storage molecules (e.g., various storage triglycerides), or with utilization of fatty acids for energy via beta oxidation and in certain embodiments, the genetic modification renders the activities associated with generation of biomass and/or carbon storage molecules or utilization of fatty acids for energy undetectable. In some embodiments, the activity associated with generation of biomass and/or carbon storage molecules is an activity that generates phospholipids, triacylglycerides, and/or steryl esters. In certain embodiments, the activity associated with generation of biomass and/or carbon storage or utilization of fatty acids for energy is selected from acyl-CoA synthetase (e.g., ACS1) activity, long chain acyl-CoA synthetase (e.g., FAT1) activity, acyl-CoA sterol acyl transferase (e.g., ARE1, ARE2, or ARE1 and ARE2) activity, and/or diacyl-glycerol acyl transferase (e.g., DGA1, LRO1, or DGA1 and LRO1) activity.

In some embodiments, an engineered microorganism comprises a genetic modification that alters the specificity of and/or reduces the activity of an acyl-CoA synthetase activity. In certain embodiments, the genetic modification disrupts an acyl-CoA synthetase activity. In some embodiments, the genetic modification includes disrupting a polynucleotide that encodes a polypeptide having an acyl-CoA synthetase activity. In certain embodiments, the genetic modification includes disrupting a promoter and/or 5'UTR in functional connection with a polynucleotide that encodes a polypeptide having the acyl-CoA synthetase activity, and in some embodiments, the genetic modification includes disrupting a portion or all of the nucleotide sequence which encodes the polypeptide having acyl-CoA synthetase activity. In some embodiments, the polypeptide having acyl-CoA synthetase activity is an ACS polypeptide. In certain embodiments, the ACS polypeptide is an ACS1 polypeptide, an ACS2 polypeptide, or an ACS1 polypeptide and ACS2 polypeptide. In certain embodiments, the genetic modification disrupts an acyl-CoA synthetase activity by disrupting an ACS1 nucleotide sequence, an ACS2 nucleotide sequence, or an ACS1 and ACS2 nucleotide sequence. In some embodiments, an acyl-CoA synthetase activity is disrupted by disrupting an ACS1 nucleotide sequence substantially similar to the nucleotide sequence of SEQ ID No: 48. In certain embodiments involving disruption of an acyl-CoA synthetase activity, a polypeptide corresponding to SEQ ID NO: 49 is below the limits of detection using currently available detection methods (e.g., immunodetection, enzymatic assay, the like and combinations thereof), in a host organism.

In some embodiments, an engineered microorganism comprises a genetic modification that alters the specificity of and/or reduces the activity of a long chain acyl-CoA synthetase activity. In certain embodiments, the genetic modification disrupts a long chain acyl-CoA synthetase activity. In some embodiments, the genetic modification includes disrupting a polynucleotide that encodes a polypeptide having a long chain acyl-CoA synthetase activity. In certain embodiments, the genetic modification includes disrupting a promoter and/or 5'UTR in functional connection with a polynucleotide that encodes a polypeptide having the long chain acyl-CoA synthetase activity, and in some embodiments, the genetic modification includes disrupting a portion or all of the nucleotide sequence which encodes the polypeptide having long chain acyl-CoA synthetase activity. In some embodiments, the polypeptide having long chain acyl-CoA synthetase activity is a FAT1 polypeptide. In certain embodiments, the genetic modification disrupts a long chain acyl-CoA synthetase activity by disrupting a FAT1 nucleotide sequence. In some embodiments, a long chain acyl-CoA synthetase activity is disrupted by disrupting a FAT1 nucleotide sequence substantially similar to the nucleotide sequence of SEQ ID No: 50. In certain embodiments involving disruption of a long chain acyl-CoA synthetase activity, a polypeptide corresponding to SEQ ID NO: 51 is below the limits of detection using currently available detection methods (e.g., immunodetection, enzymatic assay, the like and combinations thereof), in a host organism.

In some embodiments, an engineered microorganism comprises a genetic modification that alters the specificity of and/or reduces the activity of an acyl-CoA sterol acyltransferase activity. In certain embodiments, the genetic modification disrupts an acyl-CoA sterol acyltransferase activity. In some embodiments, the genetic modification includes disrupting a polynucleotide that encodes a polypeptide having an acyl-CoA sterol acyltransferase activity. In certain embodiments, the genetic modification includes disrupting a promoter and/or 5'UTR in functional connection with a polynucleotide that encodes a polypeptide having the acyl-CoA sterol acyltransferase activity, and in some embodiments, the genetic modification includes disrupting a portion or all of the nucleotide sequence which encodes the polypeptide having acyl-CoA sterol acyltransferase activity. In some embodiments, the polypeptide having acyl-CoA sterol acyltransferase activity is an ARE polypeptide. In certain embodiments, the ARE polypeptide is an ARE1 polypeptide, an ARE2 polypeptide, or an ARE1 polypeptide and ARE2 polypeptide. In certain embodiments, the genetic modification disrupts an acyl-CoA sterol acyltransferase activity by disrupting an ARE1 nucleotide sequence, an ARE2 nucleotide sequence, or an ARE1 and ARE2 nucleotide sequence. In some embodiments, an acyl-CoA sterol acyltransferase activity is disrupted by disrupting an ARE nucleotide sequence substantially similar to a nucleotide sequence corresponding to SEQ ID NOs: 52 and/or 54. In certain embodiments involving disruption of an acyl-CoA sterol acyltransferase activity, a polypeptide substantially similar to an amino acid sequence corresponding to SEQ ID NOs: 53 and/or 55 is below the limits of detection using currently available detection methods (e.g., immunodetection, enzymatic assay, the like and combinations thereof), in a host organism.

In some embodiments, an engineered microorganism comprises a genetic modification that alters the specificity of and/or reduces the activity of a diacylglycerol acyltransferase activity. In certain embodiments, the genetic modification disrupts a diacylglycerol acyltransferase activity. In some embodiments, the genetic modification includes disrupting a polynucleotide that encodes a polypeptide having a diacylglycerol acyltransferase activity. In certain embodiments, the genetic modification includes disrupting a promoter and/or 5'UTR in functional connection with a polynucleotide that encodes a polypeptide having the diacylglycerol acyltransferase activity, and in some embodiments, the genetic modification includes disrupting a portion or all of the nucleotide sequence which encodes the polypeptide having a diacylglycerol acyltransferase activity. In some embodiments, the polypeptide having diacylglycerol acyltransferase activity is a DGA1 polypeptide. In certain embodiments, the genetic modification disrupts a diacylglycerol acyltransferase activity by disrupting a DGA1 nucleotide sequence. In some embodiments, a diacylglycerol acyltransferase activity is disrupted by disrupting a DGA1 nucleotide sequence substantially similar to the nucleotide sequence of SEQ ID No: 56. In certain embodiments involving disruption of a diacylglycerol acyltransferase activity, a polypeptide corresponding to SEQ ID NO: 57 is below the limits of detection using currently available detection methods (e.g., immunodetection, enzymatic assay, the like and combinations thereof), in a host organism.

In some embodiments, an engineered microorganism comprises a genetic modification that alters the specificity of and/or reduces the activity of an acyltransferase activity (e.g., LRO1). In certain embodiments, the genetic modification disrupts an acyltransferase activity. In some embodiments, the genetic modification includes disrupting a polynucleotide that encodes a polypeptide having an acyltransferase activity. In certain embodiments, the genetic modification includes disrupting a promoter and/or 5'UTR in functional connection with a polynucleotide that encodes a polypeptide having an acyltransferase activity, and in some embodiments, the genetic modification includes disrupting a portion or all of the nucleotide sequence which encodes the polypeptide having an acyltransferase activity. In some embodiments, the polypeptide having acyltransferase activity is a LRO1 polypeptide. In certain embodiments, the genetic modification disrupts an acyltransferase activity by disrupting a LRO1 nucleotide sequence. In some embodiments, an acyltransferase activity is disrupted by disrupting a LRO1 nucleotide sequence substantially similar to the nucleotide sequence of SEQ ID No: 58. In certain embodiments involving disruption of an acyltransferase activity, a polypeptide corresponding to SEQ ID NO: 59 is below the limits of detection using currently available detection methods (e.g., immunodetection, enzymatic assay, the like and combinations thereof), in a host organism.

Also provided in some embodiments are methods for manufacturing adipic acid, which comprise culturing an engineered microorganism described herein under culture conditions in which the cultured microorganism produces adipic acid. In some embodiments, the host microorganism from which the engineered microorganism is generated does not produce a detectable amount of adipic acid. In certain embodiments, the culture conditions comprise fermentation conditions, introduction of biomass, introduction of glucose, introduction of a paraffin (e.g., plant or petroleum based, such as hexane or coconut oil, for example) and/or combinations thereof. In some embodiments, the adipic acid is produced with a yield of greater than about 0.3 grams per gram of glucose added. In related embodiments, a method comprises purifying the adipic acid from the cultured microorganisms and or modifying the adipic acid, thereby producing modified adipic acid. In certain embodiments, a method comprises placing the cultured microorganisms, the adipic acid or the modified adipic acid in a container, and optionally, shipping the container.

Provided also in certain embodiments are methods for manufacturing 6-hydroxyhexanoic acid, which comprise culturing an engineered microorganism described herein under culture conditions in which the cultured microorganism produces 6-hydroxyhexanoic acid. In some embodiments, the host microorganism from which the engineered microorganism is generated does not produce a detectable amount of 6-hydroxyhexanoic acid. In certain embodiments, the culture conditions comprise fermentation conditions, introduction of biomass, introduction of glucose, and/or introduction of hexane. In some embodiments, the 6-hydroxyhexanoic acid is produced with a yield of greater than about 0.3 grams per gram of glucose added. In related embodiments, a method comprises purifying the 6-hydroxyhexanoic acid from the cultured microorganisms and or modifying the 6-hydroxyhexanoic acid, thereby producing modified 6-hydroxyhexanoic acid. In certain embodiments, a method comprises placing the cultured microorganisms, the 6-hydroxyhexanoic acid or the modified 6-hydroxyhexanoic acid in a container, and optionally, shipping the container.

Also provided in some embodiments are methods for preparing an engineered microorganism that produces adipic acid, which comprise: (a) introducing a genetic modification to a host organism that adds or increases monooxygenase activity, thereby producing engineered microorganisms having detectable and/or increased monooxygenase activity; and (b) selecting for engineered microorganisms that produce adipic acid. Provided also herein in some embodiments are methods for preparing an engineered microorganism that produces adipic acid, which comprise: (a) culturing a host organism with hexane as a nutrient source, thereby producing engineered microorganisms having detectable monooxygenase activity; and (b) selecting for engineered microorganisms that produce adipic acid. In some embodiments the monooxygenase activity is incorporation of a hydroxyl moiety into a six-carbon molecule, and in certain embodiments, the six-carbon molecule is hexanoate. In related embodiments, a method comprises selecting the engineered microorganisms that have a detectable amount of the monooxygenase activity. In some embodiments, a method comprises introducing a genetic modification that adds or increases a hexanoate synthase activity, thereby producing engineered microorganisms, and selecting for engineered microorganisms having detectable and/or increased hexanoate synthase activity. In related embodiments, the genetic modification encodes a polypeptide having a hexanoate synthase subunit A activity, a hexanoate synthase subunit B activity, or a hexanoate synthase subunit A activity and a hexanoate synthase subunit B activity.

In some embodiments, a method comprises introducing a genetic modification that adds or increases an aldehyde dehydrogenase activity (e.g., 6-oxohexanoic acid dehydrogenase activity, omega oxo fatty acid dehydrogenase), thereby producing engineered microorganisms, and selecting for engineered microorganisms having detectable and/or increased 6-oxohexanoic acid dehydrogenase activity or omega oxo fatty acid dehydrogenase relative to the host microorgansim. In certain embodiments, a method for preparing microorganisms that produce adipic acid includes selecting for engineered microorganisms having one or more detectable and/or increased activities selected from the group consisting of an aldehyde dehydrogenase activity (e.g., 6-oxohexanoic acid dehydrogenase activity, omega oxo fatty acid dehydrogenase), fatty alcohol oxidase activity (e.g., 6-hydroxyhexanoic acid dehydrogenase activity, omega hydroxyl-fatty acid dehydrogenase), glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-hydroxyacyl-CoA dehydrogenase activity, and/or acetyl-CoA C-acyltransferase activity.

In certain embodiments, a method comprises introducing a genetic modification that adds or increases a fatty alcohol oxidase activity (e.g., 6-hydroxyhexanoic acid dehydrogenase activity, omega hydroxyl fatty acid dehydrogenase activity) thereby producing engineered microorganisms, and selecting for engineered microorganisms having a detectable and/or increased 6-hydroxyhexanoic acid dehydrogenase activity or omega hydroxyl fatty acid dehydrogenase activity relative to the host microorganism. In some embodiments, a method comprises introducing a genetic modification that adds or increases a thioesterase activity, thereby producing engineered microorganisms, and selecting for engineered microorganisms having a detectable and/or increased thioesterase activity relative to the host microorganism.

In certain embodiments, a method comprises introducing a genetic modification that reduces 6-hydroxyhexanoic acid conversion, thereby producing engineered microorganisms, and selecting for engineered microorganisms having reduced 6-hydroxyhexanoic acid conversion relative to the host microorganism. In some embodiments, a method comprises introducing a genetic modification that reduces beta-oxidation activity, thereby producing engineered microorganisms, and selecting for engineered microorganisms having reduced beta-oxidation activity relative to the host microorganism. In certain embodiments, a method comprises introducing a genetic modification that reduces activities associated with generation of biomass and/or carbon storage molecules and/or utilization of fatty acids for energy, thereby producing engineered microorganisms, and selecting for engineered microorganisms having reduced activities associated with generation of biomass and/or carbon storage molecules and/or utilization of fatty acids for energy relative to the host microorganism. In certain embodiments, a method comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism. In some embodiments, a method comprises introducing a genetic modification that increases omega- oxidation activity, thereby producing engineered microorganisms, and selecting for engineered microorganisms having increased omega-oxidation activity relative to the host microorganism.

Provided also herein in certain embodiments are methods for preparing a microorganism that produces adipic acid, which comprise: (a) introducing one or more genetic modifications to a host organism that add or increase one or more activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase activity, acyl-CoA thioesterase activity, enoyl-CoA hydratase activity, 3-hydroxyacyl-CoA dehydrogenase activity, and/or acetyl-CoA C-acyltransferase activity, thereby producing engineered microorganisms, and (b) selecting for engineered microorganisms that produce adipic acid. In some embodiments, a method comprises selecting for engineered microorganisms having one or more detectable and/or increased activities selected from the group consisting of 6-oxohexanoic acid dehydrogenase activity, 6-hydroxyhexanoic acid dehydrogenase activity, glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase activity, acyl-CoA thioesterase activity, enoyl-CoA hydratase activity, 3-hydroxyacyl-CoA dehydrogenase activity, and/or acetyl-CoA C-acyltransferase activity, relative to the host microorganism.

In certain embodiments, a method comprises introducing a genetic modification that reduces 6-hydroxyhexanoic acid conversion, thereby producing engineered microorganisms, and selecting for engineered microorganisms having reduced 6-hydroxyhexanoic acid conversion relative to the host microorganism. In some embodiments, a method comprises introducing a genetic modification that reduces beta-oxidation activity, thereby producing engineered microorganisms, and selecting for engineered microorganisms having reduced beta-oxidation activity relative to the host microorganism. In certain embodiments, a method comprises introducing a genetic modification that reduces activities associated with generation of biomass and/or carbon storage molecules and/or utilization of fatty acids for energy, thereby producing engineered microorganisms, and selecting for engineered microorganisms having reduced activities associated with generation of biomass and/or carbon storage molecules and/or utilization of fatty acids for energy relative to the host microorganism. In certain embodiments, a method comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism.

Also provided in some embodiments are methods for preparing a microorganism that produces 6-hydroxyhexanoic acid, which comprise: (a) introducing one or more genetic modifications to a host organism that add or increase one or more activities selected from the group consisting of 6-oxohexanoic acid dehydrogenase activity, glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-hydroxyacyl-CoA dehydrogenase activity, and/or acetyl-CoA C-acyltransferase activity, thereby producing engineered microorganisms, (b) introducing a genetic modification to the host organism that reduces 6-hydroxyhexanoic acid conversion, and (c) selecting for engineered microorganisms that produce 6-hydroxyhexanoic acid. In certain embodiments, a method comprises selecting for engineered microorganisms having reduced 6-hydroxyhexanoic acid conversion relative to the host microorganism. In some embodiments, a method comprises selecting for engineered microorganisms having one or more detectable and/or increased activities selected from the group consisting of aldehyde dehydrogenase activity (e.g., 6-oxohexanoic acid dehydrogenase activity, omega oxo fatty acid dehydrogenase activity), fatty alcohol oxidase activity (e.g., 6-hydroxyhexanoic acid dehydrogenase activity, omega hydroxyl fatty acid dehydrogenase activity), glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-hydroxyacyl-CoA dehydrogenase activity, and/or acetyl-CoA C-acyltransferase activity, relative to the host microorganism. In certain embodiments, a method comprises introducing a genetic modification that reduces beta-oxidation activity, thereby producing engineered microorganisms, and selecting for engineered microorganisms having reduced beta-oxidation activity relative to the host microorganism. In some embodiments, a method comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism.

Also provided are methods that include contacting an engineered microorganism with a feedstock including one or more polysaccharides, wherein the engineered microorganism includes: (a) a genetic alteration that blocks beta oxidation activity, and (b) a genetic alteration that adds or increases a monooxygenase activity, a genetic alteration that adds or increases a fatty acid synthase activity, and/or a genetic alteration that adds or increases a hexanoate synthetase activity, and culturing the engineered microorganism under conditions in which adipic acid is produced. In some embodiments, the engineered microorganism comprises a genetic alteration that adds or increases fatty acid synthase activity and/or hexanoate synthetase activity. In certain embodiments, the engineered microorganism comprises a heterologous polynucleotide encoding a polypeptide having fatty acid synthase subunit alpha activity, and in some embodiments the engineered microorganism comprises a heterologous polynucleotide encoding a polypeptide having fatty acid synthase subunit beta activity. In certain embodiments, the engineered microorganism comprises a heterologous polynucleotide encoding a polypeptide having hexanoate synthase subunit A activity, and in some embodiments the engineered microorganism comprises a heterologous polynucleotide encoding a polypeptide having hexanoate synthase subunit B activity. In certain embodiments, the heterologous polynucleotide independently is selected from a fungus. In some embodiments, the fungus is an Aspergillus fungus, and in certain embodiments the Aspergillus fungus is A. parasiticus. In certain embodiments, the microorganism is a C. tropicalis strain, and in some embodiments, the microorganism is a C. viswanithii strain. In certain embodiments, the microorganism is a Yarrowia yeast, and in some embodiments, the microorganism is a Yarrowia lipolytica strain.

Provided also are methods that include contacting an engineered microorganism with a feedstock comprising one or more paraffins, wherein the engineered microorganism comprises a genetic alteration that partially blocks beta oxidation activity and culturing the engineered microorganism under conditions in which adipic acid is produced. In certain embodiments, the microorganism comprises a genetic alteration that increases a monooxygenase activity..
In some embodiments, the genetic alteration that increases monooxygenase activity comprises a genetic alteration that increases monooxygenase (e.g., Cytochrome P450) reductase activity. In certain embodiments, the genetic alteration increases the number of copies of a polynucleotide that encodes a polypeptide having the Cytochrome P450 reductase activity. In some embodiments, the genetic alteration places a promoter and/or 5'UTR in functional connection with a polynucleotide that encodes a polypeptide having the Cytochrome P450 reductase activity. In certain embodiments the monooxygenase reductase activity is selected from the group consisting of cytochrome P450:NADPH P450 reductase (B. megaterium), NADPH cytochrome P450 reductase (CPR; C. tropicalis strain ATCC750), NADPH cytochrome P450 reductase A (e.g., CPRA; C. tropicalis strain ATCC20336), and NADPH cytochrome P450 reductase B (CPRB; C. tropicalis strain ATCC20336). In some embodiments the monooxygenase reductase activity is provided by a polypeptide encoded by a polynucleotide of any one of SEQ ID NOs: 23-26.

In certain embodiments, the genetic alteration that blocks beta oxidation activity disrupts acyl-CoA oxidase activity. In some embodiments, the genetic alteration disrupts POX4 and/or POX5 activity. In certain embodiments, the genetic alteration disrupts a polynucleotide that encodes a polypeptide having the acyl-CoA oxidase activity. In some embodiments, the genetic alteration disrupts a promoter and/or 5'UTR in functional connection with a polynucleotide that encodes a polypeptide having the acyl-CoA oxidase activity.

In some embodiments a genetic alteration that increases beta oxidation activity increases acyl-CoA oxidase activity. In certain embodiments, the genetic alteration that increases beta oxidation activity adds or increases the number of copies of a polynucleotide encoding an acyl-CoA oxidase activity. In some embodiments, the genetic alteration increases the activity of a promoter and/or 5'UTR in functional connection with a polynucleotide encoding an acyl-CoA oxidase activity. In certain embodiments, the genetic alteration adds or increases the number of copies of a polynucleotide encoding an acyl-CoA oxidase activity and/or increases the activity of a promoter and/or 5'UTR in functional connection with a polynucleotide encoding an acyl-CoA oxidase activity.

In some embodiments, the feedstock comprises a 6-carbon sugar. In certain embodiments, the feedstock comprises a 5-carbon sugar. In some embodiments, the feedstock comprises a fatty acid, and in certain embodiments the feedstock comprises a mixture of fatty acids. In some embodiments, the feedstock comprises a triacylglyceride. In certain embodiments, the adipic acid is produced at a level of about 80% or more of theoretical yield. In some embodiments, the amount of adipic acid produced is detected. In certain embodiments, the adipic acid produced is isolated (e.g., partially or completely purified). In some embodiments, the culture conditions comprise fermenting the engineered microorganism.

Provided also herein are engineered microorganisms in contact with a feedstock. In some embodiments, the feedstock includes a saccharide. In certain embodiments, the saccharide is a monosaccharide, polysaccharide, or a mixture of a monosaccharide and polysaccharide. In some embodiments, the feedstock includes a paraffin. In certain embodiments, the paraffin is a saturated paraffin, unsaturated paraffin, substituted paraffin, branched paraffin, linear paraffin, or combination thereof.

In some embodiments, the paraffin includes about 1 to about 60 carbon atoms (e.g., between about 1 carbon atom, about 2 carbon atoms, about 3 carbon atoms, about 4 carbon atoms, about 5 carbon atoms, about 6 carbon atoms, about 7 carbon atoms, about 8 carbon atoms, about 9 carbon atoms, about 10 carbon atoms, about 12 carbon atoms, about 14 carbon atoms, about 16 carbon atoms, about 18 carbon atoms, about 20 carbon atoms, about 22 carbon atoms, about 24 carbon atoms, about 26 carbon atoms, about 28 carbon atoms, about 30 carbon atoms, about 32 carbon atoms, about 34 carbon atoms, about 36 carbon atoms, about 38 carbon atoms, about 40 carbon atoms, about 42 carbon atoms, about 44 carbon atoms, about 46 carbon atoms, about 48 carbon atoms, about 50 carbon atoms, about 52 carbon atoms, about 54 carbon atoms, about 56 carbon atoms, about 58 carbon atoms and about 60 carbon atoms). In certain embodiments, the paraffin is in a mixture of paraffins. In some embodiments, the paraffins in the mixture of paraffins have a mean number of carbon atoms of about 8 carbon atoms to about 18 carbon atoms (e.g., about 8 carbon atoms, about 9 carbon atoms, about 10 carbon atoms, about 11 carbon atoms, about 12 carbon atoms, about 13 carbon atoms, about 14 carbon atoms, about 15 carbon atoms, about 16 carbon atoms, about 17 carbon atoms or about 18 carbon atoms). In certain embodiments, the paraffin is in a wax, and in some embodiments, the paraffin is in an oil. In some embodiments, the paraffin contains one or more fatty acids. In certain embodiments, the paraffin is from a petroleum product, and in some embodiments, the petroleum product is a petroleum distillate. In certain embodiments, the paraffin is from a plant or plant product.

Also provided herein, is an isolated polynucleotide selected from the group including a polynucleotide having a nucleotide sequence 96% or more (e.g., 96% or more, 97% or more, 98% or more, 99% or more, or 100%) identical to the nucleotide sequence of SEQ ID NO: 1, a polynucleotide having a nucleotide sequence that encodes a polypeptide of SEQ ID NO: 8, and a polynucleotide having a portion of a nucleotide sequence 96% or more identical to the nucleotide sequence of SEQ ID NO: 1 and encodes a polypeptide having fatty alcohol oxidase activity.

Also provided herein, is an isolated polynucleotide selected from the group including a polynucleotide having a nucleotide sequence 98% or more (e.g., 98% or more, 99% or more, or 100%) identical to the nucleotide sequence of SEQ ID NO: 2, a polynucleotide having a nucleotide sequence that encodes a polypeptide of SEQ ID NO:10, and a polynucleotide having a portion of a nucleotide sequence 98% or more identical to the nucleotide sequence of SEQ ID NO: 2 and encodes a polypeptide having fatty alcohol oxidase activity.

Also provided herein, is an isolated polynucleotide selected from the group including a polynucleotide having a nucleotide sequence 95% or more (e.g., 95% or more, 96% or more, 97% or more, 98% or more, 99% or more, or 100%) identical to the nucleotide sequence of SEQ ID NO: 3, a polynucleotide having a nucleotide sequence that encodes a polypeptide of SEQ ID NO: 9, and a polynucleotide having a portion of a nucleotide sequence 95% or more identical to the nucleotide sequence of SEQ ID NO: 3 and encodes a polypeptide having fatty alcohol oxidase activity.

Also provided herein, is an isolated polynucleotide selected from the group including a polynucleotide having a nucleotide sequence 83% or more (e.g., 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 100%) identical to the nucleotide sequence of SEQ ID NO: 4, a polynucleotide having a nucleotide sequence that encodes a polypeptide of SEQ ID NO: 11, and a polynucleotide having a portion of a nucleotide sequence 83% or more identical to the nucleotide sequence of SEQ ID NO: 3 and encodes a polypeptide having fatty alcohol oxidase activity.

Also provided herein, is an isolated polynucleotide selected from the group including a polynucleotide having a nucleotide sequence 82% or more (e.g., 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, or 100%) identical to the nucleotide sequence of SEQ ID NO: 5, a polynucleotide having a nucleotide sequence that encodes a polypeptide of SEQ ID NO: 12, and a polynucleotide having a portion of a nucleotide sequence 82% or more identical to the nucleotide sequence of SEQ ID NO: 3 and encodes a polypeptide having fatty alcohol oxidase activity.

Also provided herein, is an isolated polynucleotide having a polynucleotide identical to the polynucleotide of SEQ ID NO: 13, or fragments thereof and encodes a polypeptide having monooxygenase activity. Also provided herein, is an isolated polynucleotide having a polynucleotide 96% or more identical to the polynucleotide of SEQ ID NO: 14 or 15, or fragments thereof and encodes a polypeptide having monooxygenase activity. Also provided herein, is an isolated polynucleotide having a polynucleotide 96% or more identical to the polynucleotide of SEQ ID NO 16 or 17, or fragments thereof and encodes a polypeptide having monooxygenase activity. Also provided herein, is an isolated polynucleotide having a polynucleotide 94% or more identical to the polynucleotide of SEQ ID NO 18 or 19, or fragments thereof and encodes a polypeptide having monooxygenase activity. Also provided herein, is an isolated polynucleotide having a polynucleotide 95% or more identical to the polynucleotide of SEQ ID NO 20 or 21, or fragments thereof and encodes a polypeptide having monooxygenase activity. Also provided herein, is an isolated polynucleotide comprising a nucleotide sequence of any one of SEQ ID NOs: 23 to 26, or fragment thereof that encodes a polypeptide having monooxygenase reductase activity.

Also provided herein, is an isolated polynucleotide (i) comprising a nucleotide sequence identical to the nucleotide sequence of SEQ ID NO: 27 or fragment thereof, that encodes a polypeptide, or (ii) a polynucleotide that encodes a polypeptide of SEQ ID NO: 28, the polypeptide having lipase activity. Also provided herein is a polypeptide having an amino acid sequence identical to the polypeptide of SEQ ID NO: 29, the polypeptide having lipase activity.

Also provided herein, is an isolated polynucleotide having a nucleotide sequence identical to the nucleotide sequence of SEQ ID NO: 30 or fragments thereof and encodes a polypeptide having acetyl-CoA carboxylase activity. Also provided herein, is an isolated polynucleotide selected from the group including polynucleotides having a nucleotide sequence identical to the nucleotide sequence of any one of SEQ ID NOs: 31 and 32, or fragments thereof and encodes a polypeptide having fatty acid synthase activity.

Also provided herein, is an isolated polynucleotide having a nucleotide sequence identical to the nucleotide sequence of SEQ ID NO: 33 or fragments thereof and encoding a polypeptide having glucose-6-phosphate dehydrogenase activity. Also provided herein is a polypeptide having an amino acid sequence identical to the polypeptide of SEQ ID NO: 34, the polypeptide having glucose-6-phosphate dehydrogenase activity.

Also provided herein, is an isolated polynucleotide selected from the group including a polynucleotide having a nucleotide sequence 96% or more (e.g., 96% or more, 97% or more, 98% or more, 99% or more, or 100%) identical to the nucleotide sequence of SEQ ID NO: 42 or 44, a polynucleotide having a nucleotide sequence that encodes a polypeptide having an amino acid sequence 98% or more (e.g., 98% or more, 99% or more, or 100%) identical to the amino acid sequence of SEQ ID NO: 43 or 45, and a polynucleotide having a portion of a nucleotide sequence 96% or more identical to the nucleotide sequence of SEQ ID NO: 42 or 44 and encodes a polypeptide having acyl-CoA hydrolase activity.

Also provided herein, is an isolated polynucleotide having a polynucleotide sequence identical to the polynucleotide sequence of SEQ ID NO: 45, or fragments thereof and encodes a polypeptide of SEQ ID NO:46 having acyl-CoA thioesterase activity.

We also describe an engineered microorganism capable of producing adipic acid, the microorganism including genetic alterations resulting in commitment of molecular pathways in directions for production of adipic acid, the pathways and directions include: (i) fatty acid synthesis pathway in the direction of acetyl CoA to long-chain fatty acids, and away from generation of biomass and/or carbon storage molecules (e.g., starch, lipids, triacylglycerides) and/or utilization of fatty acids for energy, (ii) omega oxidation pathway in the direction of long-chain fatty acids to diacids and (iii) beta oxidation pathway in the direction of diacids to adipic acid. Also provided herein, is an engineered microorganism capable of producing adipic acid, which microorganism comprises genetic alterations resulting in three or more increased activities, relative to the microorganism not containing the genetic alterations, selected from the group consisting of acetyl CoA carboxylase activity, fatty acid synthase activity, monooxygenase activity, monooxygenase reductase activity, acyl-CoA hydrolase activity, acyl-CoA thioesterase activity and acyl-CoA oxidase activity.

Also provided herein, is an engineered microorganism capable of producing adipic acid, the microorganism including genetic alterations resulting in commitment of molecular pathways in directions for production of adipic acid, the pathways and directions include: (i) hexanoic acid synthesis pathway in the direction of acetyl CoA to hexanoic acid, and (ii) omega oxidation pathway in the direction of hexanoic acid to adipic acid. Provided also herein, is an engineered microorganism capable of producing adipic acid, which microorganism comprises genetic alterations resulting in three or more increased activities, relative to the microorganism not containing the genetic alterations, selected from the group consisting of acetyl CoA carboxylase activity, acyl-CoA hydrolase activity, acyl-CoA thioesterase activity, hexanoate synthase activity, monooxygenase activity and monooxygenase reductase activity.

Provided also herein, is an engineered microorganism capable of producing adipic acid, the microorganism includes genetic alterations resulting in commitment of molecular pathways in directions for production of adipic acid, the pathways and directions include: (i) gluconeogenesis pathway in the direction of triacyl glycerides to 6-phosphoglucono-lactone and nicotinamide adenine dinucleotide phosphate (NADPH), (ii) omega oxidation pathway in the direction of fatty acids to diacids, (iii) beta oxidation pathway in the direction of diacids to adipic acid and (iv) fatty acid synthesis pathway in the direction of acetyl CoA to fatty acids. Also provided herein, is an engineered microorganism capable of producing adipic acid, which microorganism comprises genetic alterations resulting in three or more increased activities, relative to the microorganism not containing the genetic alterations, selected from the group consisting of lipase activity, glucose-6-phosphate dehydrogenase activity, fatty acid synthase activity, monooxygenase activity, monooxygenase reductase activity, acyl-CoA hydrolase activity, acyl-CoA thioesterase activity and acyl-CoA oxidase activity.

In some embodiments, an engineered microorganism includes an increased activity, relative to the microorganism not containing the genetic alterations, independently in each pathway. In certain embodiments, an engineered microorganism includes an increased acetyl CoA carboxylase activity in the fatty acid synthesis pathway. In some embodiments, an engineered microorganism includes an increased fatty acid synthase activity in the fatty acid synthesis pathway. In certain embodiments, an engineered microorganism includes an increased acyl-CoA hydrolase activity in the fatty acid synthesis pathway. In some embodiments, an engineered microorganism includes an increased acyl-CoA thioesterase activity in the fatty acid synthesis pathway. In certain embodiments, an engineered microorganism includes an increased monooxygenase activity. In some embodiments, an engineered microorganism includes an increased monooxygenase reductase activity. In certain embodiments, an engineered microorganism includes an increased acyl-CoA oxidase activity.

In certain embodiments, an engineered microorganism includes an increased acetyl CoA carboxylase activity in the hexanoic acid synthesis pathway. In some embodiments, an engineered microorganism includes increased hexanoate synthase activity in the hexanoic acid synthesis pathway. In certain embodiments, an engineered microorganism includes increased monooxygenase activity in the omega oxidation pathway. In some embodiments, an engineered microorganism includes increased monooxygenase reductase activity in the omega oxidation pathway.

In some embodiments, an engineered microorganism includes an increased lipase activity in the gluconeogenesis pathway. In certain embodiments, an engineered microorganism includes an increased glucose-6-phosphate dehydrogenase activity in the gluconeogenesis pathway. In some embodiments, an engineered microorganism includes an increased acyl-CoA oxidase activity in the beta oxidation pathway. In certain embodiments, an engineered microorganism includes an increased acyl-CoA hydrolase and/or an increased acyl-CoA thioesterase in the fatty acid synthesis pathway

In some embodiments, one or more enzymes or proteins can provide an activity (e.g., single subunit protein, multi subunit protein). In certain embodiments, 2 or more activities can be provided by one or more enzymes or proteins (e.g., multifunction single protein, enzyme complex). In some embodiments, each of the increased activities independently is provided by an enzyme encoded by a gene endogenous to the microorganism. In certain embodiments, each of the increased activities independently is provided by an enzyme encoded by a gene exogenous to the microorganism. In some embodiments, each of the increased activities independently is provided by an increased amount of an enzyme from a yeast. In certain embodiments, the microorganism is a C. tropicalis strain, and in some embodiments, the microorganism is a C. viswanithii strain. In certain embodiments, the microorganism is a Yarrowia yeast, and in some embodiments, the microorganism is a Yarrowia lipolytica strain.

In some embodiments, each one of the increased activities independently results from increasing the copy number of a gene that encodes an enzyme that provides the activity. In certain embodiments, each one of the increased activities independently results from inserting a promoter in functional proximity to a gene that encodes an enzyme that provides the activity. In some embodiments, the gene is in plasmid nucleic acid, and in certain embodiments, the gene is in genomic nucleic acid of the microorganism.

In some embodiments, the acetyl CoA carboxylase activity is provided by an increased amount of an enzyme comprising (i) the amino acid sequence encoded by SEQ ID NO: 30, (ii) an amino acid sequence 90% or more identical to (i), or (iii) an amino acid sequence that includes 1 to 10 amino acid substitutions, insertions or deletions with respect to (i).

In certain embodiments, the fatty acid synthase activity is provided by an increased amount of a FAS1-encoded enzyme, a FAS2-encoded enzyme, or FAS1-encoded enzyme and FAS2-encoded enzyme. In some embodiments, the FAS1-encoded enzyme comprises (i) the amino acid sequence encoded by SEQ ID NO: 32, (ii) an amino acid sequence 90% or more identical to (i), or (iii) an amino acid sequence that includes 1 to 10 amino acid substitutions, insertions or deletions with respect to (i). In certain embodiments, the FAS2-encoded enzyme comprises (i) the amino acid sequence encoded by SEQ ID NO: 31, (ii) an amino acid sequence 90% or more identical to (i), or (iii) an amino acid sequence that includes 1 to 10 amino acid substitutions, insertions or deletions with respect to (i).

In some embodiments, the monooxygenase activity is provided by an increased amount of a cytochrome P450 enzyme. In certain embodiments, the monooxygenase activity is provided by an exogenous cytochrome P450 enzyme. In some embodiments, the exogenous cytochrome P450 enzyme is from Bacillus megaterium. In certain embodiments, the exogenous cytochrome P450 enzyme comprises (i) the amino acid sequence of SEQ ID NO: 41, (ii) an amino acid sequence 90% or more identical to (i), or (iii) an amino acid sequence that includes 1 to 10 amino acid substitutions, insertions or deletions with respect to (i).

In some embodiments, two or more endogenous cytochrome P450 enzymes are expressed in increased amounts. In certain embodiments, all endogenous cytochrome P450 enzymes are expressed in increased amounts. In some embodiments, the endogenous cytochrome P450 enzymes comprise (i) an amino acid sequence encoded by a polynucleotide selected from the group consisting of SEQ ID NO: 13, 14, 15, 16, 17, 18, 19, 20, 21, and 22, (ii) an amino acid sequence 90% or more identical to (i), or (iii) an amino acid sequence that includes 1 to 10 amino acid substitutions, insertions or deletions with respect to (i).

In certain embodiments, the monooxygenase reductase activity is provided by an increased amount of an enzyme comprising (i) the amino acid sequence encoded by any one of SEQ ID NOS: 23 to 26, (ii) an amino acid sequence 90% or more identical to (i), or (iii) an amino acid sequence that includes 1 to 10 amino acid substitutions, insertions or deletions with respect to (i). In some embodiments, the monooxygenase reductase activity is provided by an increased amount of a cytochrome P450:NADPH P450 reductase-encoded enzyme, a CPR-encoded enzyme, a CPRA-encoded enzyme, a CPRB-encoded enzyme, or a cytochrome P450:NADPH P450 reductase-encoded enzyme, a CPR-encoded enzyme, a CPRA-encoded enzyme, and/or a CPRB-encoded enzyme. In certain embodiments, the cytochrome P450:NADPH P450 reductase-encoded enzyme comprises (i) the amino acid sequence of SEQ ID NO: 41 (ii) an amino acid sequence 90% or more identical to (i), or (iii) an amino acid sequence that includes 1 to 10 amino acid substitutions, insertions or deletions with respect to (i). In some embodiments, the CPR-, CPRA and/or CPRB encoded enzymes comprise (i)an amino acid sequence encoded by any one of SEQ ID NOS: 24 to 26, (ii) an amino acid sequence 90% or more identical to (i), or (iii) an amino acid sequence that includes 1 to 10 amino acid substitutions, insertions or deletions with respect to (i).

In some embodiments, the acyl-CoA oxidase activity is provided by an increased amount of a POX5-encoded enzyme. In certain embodiments, the POX4-encoded enzyme comprises (i) the amino acid sequence of SEQ ID NO: 39, (ii) an amino acid sequence 90% or more identical to (i), or (iii) an amino acid sequence that includes 1 to 10 amino acid substitutions, insertions or deletions with respect to (i). In some embodiments, the POX5-encoded enzyme comprises (i) the amino acid sequence of SEQ ID NO: 40, (ii) an amino acid sequence 90% or more identical to (i), or (iii) an amino acid sequence that includes 1 to 10 amino acid substitutions, insertions or deletions with respect to (i). In certain embodiments, the microorganism lacks an enzyme providing an acyl-CoA oxidase activity. In certain embodiments, the enzyme is a POX4-encoded enzyme or a POX5-encoded enzyme. Also described herein is a POX4 polynucleotide that encodes the POX4-encoded enzyme comprises (i) the polynucleotide of SEQ ID NO: 37, (ii) a polynucleotide 90% or more identical to (i), or (iii) polynucleotide that includes 1 to 10 nucleotide substitutions, insertions or deletions with respect to (i). In some embodiments, the POX5 polynucleotide that encodes the POX5-encoded enzyme comprises (i) the polynucleotide of SEQ ID NO: 38, (ii) a polynucleotide 90% or more identical to (i), or (iii) a polynucleotide that includes 1 to 10 amino acid substitutions, insertions or deletions with respect to (i). In certain embodiments, the microorganism lacks an enzyme providing an acyl-CoA oxidase activity, and sometimes lacks a POX4-encoded enzyme or a POX5-encoded enzyme.

In certain embodiments, the hexanoate synthase activity is provided by an increased amount of a HEXA-encoded protein, a HEXB-encoded protein, or HEXA-encoded protein and HEXB-encoded protein. In some embodiments, the HEXA-encoded protein comprises (i) the amino acid sequence encoded by SEQ ID NO: 35, (ii) an amino acid sequence 90% or more identical to (i), or (iii) an amino acid sequence that includes 1 to 10 amino acid substitutions, insertions or deletions with respect to (i). In certain embodiments, the HEXB-encoded protein comprises (i) the amino acid sequence encoded by SEQ ID NO: 36, (ii) an amino acid sequence 90% or more identical to (i), or (iii) an amino acid sequence that includes 1 to 10 amino acid substitutions, insertions or deletions with respect to (i).

In some embodiments, the lipase activity is provided by an increased amount of an enzyme comprising (i) the amino acid sequences of SEQ ID NO: 28 or 29, (ii) an amino acid sequence 90% or more identical to (i), or (iii) an amino acid sequence that includes 1 to 10 amino acid substitutions, insertions or deletions with respect to (i). In some embodiments, the lipase activity is provided by an increased amount of an enzyme encoded by a polynucleotide comprising (i) the polynucleotide of SEQ ID NO: 27, (ii) a polynucleotide 90% or more identical to (i), or (iii) a polynucleotide that includes 1 to 10 nucleotide substitutions, insertions or deletions with respect to (i).

In certain embodiments, the glucose-6-phosphate dehydrogenase activity is provided by an increased amount of an enzyme comprising (i) the amino acid sequence of SEQ ID NO: 34, (ii) an amino acid sequence 90% or more identical to (i), or (iii) an amino acid sequence that includes 1 to 10 amino acid substitutions, insertions or deletions with respect to (i). In some embodiments, the glucose-6-phosphate dehydrogenase activity is provided by an increased amount of an enzyme encoded by a polynucleotide comprising (i) the polynucleotide of SEQ ID NO: 33, (ii) a polynucleotide 90% or more identical to (i), or (iii) a polynucleotide that includes 1 to 10 nucleotide substitutions, insertions or deletions with respect to (i).

In some embodiments, the acyl-CoA hydrolase activity is provided by an increased amount of an enzyme comprising (i) the amino acid sequence encoded by SEQ ID NO: 43 or 45, (ii) an amino acid sequence 98% or more identical to (i), or (iii) an amino acid sequence that includes 1 to 10 amino acid substitutions, insertions or deletions with respect to (i).

In some embodiments, the acyl-CoA thioesterase activity is provided by an increased amount of an enzyme comprising (i) the amino acid sequence encoded by SEQ ID NO: 47, or (ii) an amino acid sequence that includes 1 to 10 amino acid substitutions, insertions or deletions with respect to (i).

In certain embodiments, an expression vector includes a polynucleotide sequence or expresses an amino acid sequence of any one of SEQ ID NOS: 1 to 59. In some embodiments, an integration vector includes a polynucleotide sequence or expresses an amino acid sequence of any one of SEQ ID NOS: 1 to 59. In certain embodiments, a microorganism includes an expression vector, an integration vector, or an expression vector and an integration vector that includes a polynucleotide sequence of SEQ ID NOS: 1 to 59. In some embodiments, a culture includes a microorganism that includes an expression vector, an integration vector, or an expression vector and an integration vector that includes a polynucleotide sequence or expresses an amino acid sequence of any one of SEQ ID NOS: 1 to 59. In certain embodiments, a fermentation device includes a microorganism that includes an expression vector, an integration vector, or an expression vector and an integration vector that includes a polynucleotide sequence or expresses an amino acid sequence of any one of SEQ ID NOS: 1 to 59. In some embodiments an integration vector is used to disrupt a polynucleotide sequence. Also provided herein is a polypeptide or a polypeptide encoded by a polynucleotide sequence of any one of SEQ ID NOS: 1 to 47 or produced by an expression vector that includes a polynucleotide sequence of, or expresses an amino acid sequence of any one of SEQ ID NOS: 1 to 47. Provided also herein is an antibody that specifically binds to a polypeptide of, or is encoded by a polynucleotide sequence or expresses an amino acid sequence of any one of SEQ ID NOS: 1 to 59 or produced by an expression vector that includes a polynucleotide sequence or expresses an amino acid sequence of any one of SEQ ID NOS: 1 to 59.

Also provided herein is a method for producing adipic acid, the method including culturing an engineered microorganism described herein under conditions in which adipic acid is produced. In some embodiments, the culture conditions include fermentation conditions. In certain embodiments, the culture conditions include introduction of biomass. In some embodiments, the culture conditions include introduction of a feedstock comprising glucose. In certain embodiments, the culture conditions include introduction of a feedstock comprising hexane. In some embodiments, the culture conditions include introduction of a feedstock comprising an oil.

In certain embodiments, the adipic acid is produced with a yield of greater than about 0.15 grams per gram of the glucose, hexane or oil. In some embodiments, the adipic acid is produced at between about 25% and about 100% of maximum theoretical yield of any introduced feedstock (e.g., about 25%, about 30%, about 35%, about 40%, about 45%, about 50%, about 55%, about 60%, about 65%, about 70%, about 75%, about 80%, about 85%, about 90%, about 95%, about 99%, or about 100% of theoretical maximum yield). In certain embodiments, the adipic acid is produced in a concentration range of between about 50 g/L to about 1000g/L of culture media (e.g., about 50 g/L, about 55 g/L, about 60 g/L, about 65 g/L, about 70 g/L, about 75 g/L, about 80 g/L, about 85 g/L, about 90 g/L, about 95 g/L, about 100 g/L, about 110 g/L, about 120 g/L, about 130 g/L, about 140 g/L, about 150 g/L, about 160 g/L, about 170 g/L, about 180 g/L, about 190 g/L, about 200 g/L, about 225 g/L, about 250 g/L, about 275 g/L, about 300 g/L, about 325 g/L, about 350 g/L, about 375 g/L, about 400 g/L, about 425 g/L, about 450 g/L, about 475 g/L, about 500 g/L, about 550 g/L, about 600 g/L, about 650 g/L, about 700 g/L, about 750 g/L, about 800 g/L, about 850 g/L, about 900 g/L, about 950 g/L, or about 1000 g/L).

In some embodiments, the adipic acid is produced at a rate of between about 0.5 g/L/hour to about 5 g/L/hour (e.g., about 0.5 g/L/hour, about 0.6 g/L/hour, about 0.7 g/L/hour, about 0.8 g/L/hour, about 0.9 g/L/hour, about 1.0 g/L/hour, about 1.1 g/L/hour, about 1.2 g/L/hour, about 1.3 g/L/hour, about 1.4 g/L/hour, about 1.5 g/L/hour, about 1.6 g/L/hour, about 1.7 g/L/hour, about 1.8 g/L/hour, about 1.9 g/L/hour, about 2.0 g/L/hour, about 2.25 g/L/hour, about 2.5 g/L/hour, about 2.75 g/L/hour, about 3.0 g/L/hour, about 3.25 g/L/hour, about 3.5 g/L/hour, about 3.75 g/L/hour, about 4.0 g/L/hour, about 4.25 g/L/hour, about 4.5 g/L/hour, about 4.75 g/L/hour, or about 5.0 g/L/hour.) In certain, embodiments, the engineered organism comprises between about a 5-fold to about a 500-fold increase in adipic acid production when compared to wild-type or partially engineered organisms of the same strain, under identical fermentation conditions (e.g., about a 5-fold increase, about a 10-fold increase, about a 15-fold increase, about a 20-fold increase, about a 25-fold increase, about a 30-fold increase, about a 35-fold increase, about a 40-fold increase, about a 45-fold increase, about a 50-fold increase, about a 55-fold increase, about a 60-fold increase, about a 65-fold increase, about a 70-fold increase, about a 75-fold increase, about a 80-fold increase, about a 85-fold increase, about a 90-fold increase, about a 95-fold increase, about a 100-fold increase, about a 125-fold increase, about a 150-fold increase, about a 175-fold increase, about a 200-fold increase, about a 250-fold increase, about a 300-fold increase, about a 350-fold increase, about a 400-fold increase, about a 450-fold increase, or about a 500-fold increase).

In some embodiments, the method includes purifying the adipic acid from the cultured microorganisms. In certain embodiments, the method includes modifying the adipic acid, thereby producing modified adipic acid. In some embodiments, the method includes placing the cultured microorganisms, the adipic acid or the modified adipic acid in a container, and in certain embodiments, the method includes shipping the container.

In some embodiments, provided is a chimera exhibiting a fatty acid synthase and/or hexanoate synthase activity. In some embodiments, the chimera is encoded by a nucleotide sequence that includes a donor sequence in a base sequence. In certain embodiments, a donor sequence replaces a sequence earlier excised from the base sequence (excised sequence). There can be one or more donor sequences, and optionally one or more excised sequences, in a particular polynucleotide that encodes a chimera. In certain embodiments, the base sequence and donor sequence are from fatty acid synthase polynucleotides from different organisms. In some embodiments, each fatty acid synthase polynucleotide independently is obtained from a fungus or yeast (e.g., Candida yeast (e.g., C. tropicalis, C. viswanithii)). In certain embodiments, the donor sequence and/or base sequence is from a hexanoate synthase subunit A or B (e.g., HEXA, HEXB), such as a HEXA or HEXB sequence described herein. In some embodiments, the donor sequence or base sequence is from a fatty acid synthase gene of a fungus or yeast (e.g., Candida (e.g., C. tropicalis, C. viswanithii).

In certain chimera embodiments, the excised sequence and/or the donor sequence encodes a functional polypeptide, or portion thereof, that adds malonyl units to a growing fatty acid chain, and/or removes a grown fatty acid chain (e.g., palmitoyl fatty acid or derivative) from the polypeptide or portion thereof. In some embodiments, the donor sequence and/or excised sequence encodes a malonyl-palmitoyl transferase domain (MPT domain) from a hexanoate synthase gene or fatty acid synthase gene. In certain embodiments, the donor sequence and/or excised sequence encodes all or part of the functional polypeptide or domain described above, and optionally includes one or more additional nucleotides or stretches of contiguous polynucleotides. In some embodiments, the donor sequence or excised sequence is about 900 contiguous nucleotides to about 1500 contiguous nucleotides in length, and sometimes about 1200 contiguous nucleotides in length. In certain embodiments, the base sequence and/or donor sequence independently are (i) identical to a native sequence, (ii) 90% or more identical to a native sequence, or (iii) include 1 to 10 insertions, deletions or substitutions with respect to a native sequence. In some embodiments, the native donor sequence is from HEXB and the native base sequence is from FAS1. Examples of each of these sequences are provided herein.

In some chimera embodiments, the donor sequence and/or excised sequence encodes a ketoacyl synthase domain (KS domain) from a hexanoate synthase gene or fatty acid synthase gene. In certain embodiments, the donor sequence and/or excised sequence encodes all or part of the domain described above, and optionally includes one or more additional nucleotides or stretches of contiguous polynucleotides. In some embodiments, the donor sequence or excised sequence is about 900 contiguous nucleotides to about 1700 contiguous nucleotides in length, and sometimes about 1350 contiguous nucleotides in length. In certain embodiments, the base sequence and/or donor sequence independently are (i) identical to a native sequence, (ii) 90% or more identical to a native sequence, or (iii) include 1 to 10 insertions, deletions or substitutions with respect to a native sequence. In some embodiments, the native donor sequence is from HEXA and the native base sequence is from FAS2. Examples of each of these sequences are provided herein.

In certain chimera embodiments, donor sequences from FAS1 are used to fill in regions of a base HexB sequence that are not present in HexB. Donor sequences may be selected, in some embodiments, based upon an alignment of HexB and FAS1 sequences and identifying sequences present in FAS1 that do not align with, and/or appear to be inserted with respect to, HexB. In some embodiments, the donor sequence is about 100 contiguous nucleotides or less from FAS1. In certain embodiments, the base sequence and/or donor sequence independently are (i) identical to a native sequence from HexB and FAS1, respectively, (ii) 90% or more identical to the native sequence, or (iii) include 1 to 10 insertions, deletions or substitutions with respect to the native sequence.

Provided also herein are genetically modified microorganisms including one or more increased activities, with respect to the activity level in an unmodified or parental strain, which increased activities are chosen from: a monooxygenase activity, a monooxygenase reductase activity, an acyl-CoA oxidase activity, an acyl-CoA hydrolase activity, an acyl-CoA thioesterase activity and combinations of the forgoing. In some embodiments, the monooxygenase activity includes a cytochrome P450 A19 (e.g., CYP52A19) activity. In certain embodiments, the monooxygenase activity is a cytochrome P450 A19 (e.g., CYP52A19) activity. In some embodiments, the monooxygenase reductase activity includes one or more activities selected from CPR, CPRA, CPRB, and combinations of the foregoing.

In certain embodiments, the acyl-CoA oxidase activity includes a POX5 activity. In some embodiments, the acyl-CoA oxidase activity is a POX5 activity. In certain embodiments, the acyl-CoA hydrolase activity includes one or more activities selected from ACHA activity, ACHB activity, and ACHA activity and ACHB activity. In some embodiments, the acyl-CoA thioesterase activity includes a TESA activity.

In some embodiments, the one or more increased activities are three or more increased activities. In certain embodiments, the one or more increased activities are four or more increased activities, and in some embodiments, the one or more increased activities are five or more increased activities. In certain embodiments, the three or more increased activities include an increased monooxygenase activity, an increased monooxygenase reductase activity, and an increased acyl-CoA hydrolase activity. In some embodiments, the three or more increased activities include an increased monooxygenase activity, an increased monooxygenase reductase activity, and an increased acyl-CoA thioesterase activity. In certain embodiments, the four or more increased activities include an increased monooxygenase activity, an increased monooxygenase reductase activity, an increased acyl-CoA oxidase activity and an increased acyl-CoA hydrolase activity. In some embodiments, the four or more increased activities include an increased monooxygenase activity, an increased monooxygenase reductase activity, an increased acyl-CoA hydrolase activity and an increased acyl-CoA thioesterase activity. In certain embodiments, the five or more increased activities include an increased monooxygenase activity, an increased monooxygenase reductase activity, an increased acyl-CoA oxidase activity, and increased acyl-CoA thioesterase activity and an increased acyl-CoA hydrolase activity.

In some embodiments, genetically modified microorganisms further include one or more reduced activities, with respect to the activity level in an unmodified or parental strain, which reduced activities are chosen from: acyl-CoA synthetase activity, long chain acyl-CoA synthetase activity, acyl-CoA sterol acyl transferase activity, acyltransferase activity, and combinations of the foregoing. In certain embodiments, the acyl-CoA synthetase activity includes an ACS1 activity. The long chain acyl-CoA synthetase activity includes a FAT1 activity. In certain embodiments, the acyl-CoA sterol acyl transferase activity includes one or more activities selected from an ARE1 activity, an ARE2 activity, and an ARE1 activity and an ARE2 activity. In some embodiments, the acyltransferase activity is a diacylglycerol acyltransferase activity, and in certain embodiments, the diacylglycerol acyltransferase activity includes one or more activities selected from a DGA1 activity, a LRO1 activity and a DGA1 activity and a LRO1 activity.

In certain embodiments, the one or more reduced activities is three or more reduced activities. In some embodiments, the three or more reduced activities are four or more reduced activities. In certain embodiments, the three or more reduced activities are five or more reduced activities. In some embodiments, genetically modified microorganisms include a reduced ACS1 activity, a reduced FAT1 activity, a reduced ARE1 activity, a reduced ARE2 activity, a reduced DGA1 activity and a reduced LRO1 activity.

Also provided herein, is a method for preparing a microorganism that produces adipic acid, which includes: (a) introducing one or more genetic modifications to a host organism that add or increase one or more activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, acyl-CoA hydrolase activity, acyl-CoA thioesterase activity, monooxygenase activity, and monooxygenase reductase activity, thereby producing engineered microorganisms, and (b) selecting for engineered microorganisms that produce adipic acid. In some embodiments a method for preparing a microorganism that produces adipic acid further includes selecting for engineered microorganisms having one or more detectable and/or increased activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, acyl-CoA hydrolase activity, acyl-CoA thioesterase activity, monooxygenase activity, and monooxygenase reductase activity, relative to the host microorganism. In certain embodiments, the method includes introducing a genetic modification that reduces one or more activities selected from acyl-CoA oxidase, acyl-CoA synthetase activity, long chain acyl-CoA synthetase activity, acyl-CoA sterol acyl transferase activity, acyltransferase activity, and 6-hydroxyhexanoic acid conversion activity, thereby producing engineered microorganisms, and selecting for engineered microorganisms having one or more reduced activities selected from acyl-CoA oxidase, acyl-CoA synthetase activity, long chain acyl-CoA synthetase activity, acyl-CoA sterol acyl transferase activity, acyltransferase activity, and 6-hydroxyhexanoic acid conversion activity relative to the host microorganism.

Provided also herein is a method for producing adipic acid, including: contacting an engineered microorganism with a feedstock including one or more sugars, cellulose, fatty acids, triacylglycerides or combinations of the forgoing, wherein the engineered microorganism includes: (a) a genetic alteration that partially blocks beta oxidation activity, (b) a genetic alteration that adds or increases a monooxygenase activity, (c) a genetic alteration that adds or increases a monooxygenase reductase activity, and (d) a genetic alteration that adds or increases an acyl-CoA hydrolase and/or an acyl-CoA thioesterase activity, and culturing the engineered microorganism under conditions in which adipic acid is produced. In some embodiments, the engineered microorganism further includes one or more genetic alterations that reduce an activity selected from an acyl-CoA oxidase activity, an acyl-CoA synthetase activity, a long chain acyl-CoA synthetase activity, an acyl-CoA sterol acyl transferase activity, and an acyltransferase activity.

In certain embodiments, the acyltransferase activity is a diacyl-glycerol acyltransferase activity. In some embodiments, the engineered microorganism includes a heterologous polynucleotide encoding a polypeptide having acyl-CoA thioesterase activity. In certain embodiments, the heterologous polynucleotide independently is selected from a bacterium. In some embodiments, the bacterium is an Enteric bacterium, and in certain embodiments, the Enteric bacterium is E. coli.

In certain embodiments, the engineered microorganism includes a heterologous polynucleotide encoding a polypeptide having acyl-CoA thioesterase activity, and the polynucleotide sequence has been codon optimized for expression in C. tropicalis or C. viswanithii. In some embodiments, the genetic alteration that partially blocks beta oxidation activity reduces or eliminates POX4 activity. In certain embodiments, the genetic alteration that adds or increases monooxygenase activity, increases CYP52A19 activity. In some embodiments, the genetic alteration that adds or increases monooxygenase reductase activity increases a CPR activity, a CPRA activity, a CPRB activity, or combinations thereof.

In some embodiments, the genetic alteration that adds or increases acyl-CoA hydrolase activity increases an ACHA activity, an ACHB activity or an ACHA activity and an ACHB activity. In certain embodiments, the genetic alteration that adds or increases acyl-CoA thioesterase activity adds an E. coli derived TESA activity. In some embodiments, the genetic alteration that reduces an acyl-CoA synthetase activity, reduces or eliminates an ACS1 activity. In certain embodiments, the genetic alteration that reduces a long chain acyl-CoA synthetase activity, reduces or eliminates an FAT1 activity. In some embodiments, the genetic alteration that reduces an acyl-CoA sterol acyl transferase activity, reduces or eliminates an ARE1 activity, an ARE2 activity, or an ARE1 activity and an ARE2 activity. In certain embodiments, the genetic alteration that reduces an acyltransferase activity, reduces or eliminates a DGA1 activity, a LRO1 activity or a DGA1 activity and a LRO1 activity.

In certain embodiments, the maximum theoretical yield (Yₘₐₓ) is about 0.6 grams of adipic acid produced per gram of coconut oil added, the percentage of Yₘₐₓ for the engineered microorganism under conditions in which adipic acid is produced is calculated as (%Yₘₐₓ) = Y_{p/s} / Yₘₐₓ *100, where (Y_{p/s}) = [adipic acid (g/L] * final volume of culture in flask (L)] / [feedstock added to flask (g)]. In some embodiments, the engineered microorganism produces adipic acid at about 10% to about 100% of maximum theoretical yield.

Also provided herein is an isolated polynucleotide selected from the group consisting of: a polynucleotide having a nucleotide sequence 96% or more identical to the nucleotide sequence of SEQ ID NO: 42 or 44: a polynucleotide having a nucleotide sequence that encodes a polypeptide having an amino acid sequence 98% or more identical to the amino acid sequence of SEQ ID NO: 43 or 45; and a polynucleotide having a portion of a nucleotide sequence 96% or more identical to the nucleotide sequence of SEQ ID NO: 42 or 44 and encodes a polypeptide having acyl-coA hydrolase activity. Provided also herein is an isolated polynucleotide selected from the group consisting of: a polynucleotide having a nucleotide sequence identical to the nucleotide sequence of SEQ ID NO: 46: a polynucleotide having a nucleotide sequence that encodes a polypeptide of SEQ ID NO: 47; and a polynucleotide having a portion of a nucleotide sequence identical to the nucleotide sequences of SEQ ID NO: 46 and encodes a polypeptide having acyl-CoA thioesterase activity. Also provided herein are expression vectors including a polynucleotide sequence 96% or more identical to the nucleotide sequence of SEQ ID NOS: 42 and 44. Provided also herein are expression vectors including a polynucleotide having the nucleotide sequence of SEQ ID NO: 46. Provided also herein are integration vectors including a polynucleotide sequence 96% or more identical to the nucleotide sequence of SEQ ID NOS: 42 and 44. Provided also herein are integration vectors including a polynucleotide having the nucleotide sequence of SEQ ID NO: 46.

Also provided herein are microorganisms including an expression vector and/or an integration vector including a polynucleotide sequence 96% or more identical to the nucleotide sequence of SEQ ID NOS: 42 and 44. Provided also here are microorganisms including an expression vector and/or an integration vector including a polynucleotide having the nucleotide sequence of SEQ ID NO: 46. Provided also herein is a culture including a microorganism that includes an expression vector and/or integration described herein. Also provided herein is a fermentation device including a microorganism that includes an expression vector and/or integration described herein. Also provided in some embodiments are antibodies that specifically bind to a polypeptide produced from an expression vector described herein. Provided also in some embodiments are polypeptides encoded by a polynucleotide SEQ ID 42, 44, and 46, expressed by an engineered microorganism.

Also provided herein is an engineered microorganism capable of producing adipic acid, which microorganism includes genetic alterations resulting in one or more increased activities and further resulting in commitment of molecular pathways in directions for production of adipic acid, which pathways and directions include: (i) fatty acid synthesis pathway in the direction of acetyl CoA to long-chain fatty acids and away from synthesis or generation of biomass and/or carbon storage molecules, (ii) omega oxidation pathway in the direction of long-chain fatty acids to diacids and (iii) beta oxidation pathway in the direction of diacids to adipic acid. In some embodiments, carbon storage molecules include storage starches, storage lipids and combinations thereof. In certain embodiments, an engineered microorganism includes an increased activity, relative to the microorganism not containing the genetic alterations, independently in each pathway.

In some embodiments, an engineered microorganism includes an increased monooxygenase activity. In certain embodiments, an engineered microorganism includes an increased monooxygenase reductase activity. In some embodiments, an engineered microorganism includes an increased acyl-CoA oxidase activity. In certain embodiments, an engineered microorganism includes an increased acetyl CoA carboxylase activity in the fatty acid synthesis pathway. In some embodiments, an engineered microorganism includes an increased fatty acid synthase activity in the fatty acid synthesis pathway. In certain embodiments, an engineered microorganism includes an increased acyl-CoA hydrolase activity in the fatty acid synthesis pathway.

In some embodiments, the increased activities independently is provided by an enzyme encoded by a gene endogenous to the microorganism. In certain embodiments, each of the increased activities independently is provided by an increased amount of an enzyme from a yeast.

In certain embodiments, an engineered microorganism includes an added acyl-CoA thioesterase activity in the fatty acid synthesis pathway. In some embodiments, the added activity independently is provided by an enzyme encoded by a gene exogenous to the microorganism. In certain embodiments an engineered microorganism further includes one or more reduced activities selected from an acyl-CoA oxidase activity, an acyl-CoA synthetase activity, a long chain acyl-CoA synthetase activity, an acyl-CoA sterol acyl transferase activity, and an acyltransferase activity. In some embodiments, the acyltransferase activity is a diacyl-glycerol acyltransferase activity.

Provided also herein is an engineered yeast including genetic modifications that (a) reduce an acyl-CoA oxidase activity and (b) reduce an acyl-CoA synthetase activity, reduce a long chain acyl-CoA synthetase activity, or reduce an acyl-CoA synthetase activity and reduce a long chain acyl-CoA synthetase activity. In some embodiments, an engineered yeast strain further includes a genetic modification that partially blocks beta oxidation. In certain embodiments, the genetic modification reduces the expression level of a polypeptide having an acyl-CoA oxidase activity. The reduced acyl-CoA oxidase activity is a reduced POX4 activity. In certain embodiments, the reduced POX4 activity results from a reduction in the level of expression of a polypeptide including (i) the amino acid sequence of SEQ ID NO: 39, (ii) an amino acid sequence 90% or more identical to (i), or (iii) an amino acid sequence that includes 1 to 10 amino acid substitutions, insertions or deletions with respect to (i).

In certain embodiments, an engineered yeast strain further includes genetic modifications that reduce the long chain acyl-CoA synthetase activity and do not reduce the acyl-CoA synthetase activity. In some embodiments, the reduced long chain acyl-CoA synthetase activity is a FAT1 activity. In certain embodiments, the reduced FAT1 activity results from a reduction in the level of expression of a polypeptide including (i) the amino acid sequence of SEQ ID NO: 51, (ii) an amino acid sequence 90% or more identical to (i), or (iii) an amino acid sequence that includes 1 to 10 amino acid substitutions, insertions or deletions with respect to (i). In some embodiments, an engineered yeast strain further includes genetic modifications that reduce the acyl-CoA synthetase activity and do not reduce the long chain acyl-CoA synthetase activity. In certain embodiments, the reduced acyl-CoA synthetase activity is an ACS1 activity. In some embodiments, the reduced ACS1 activity results from a reduction in the level of expression of a polypeptide including (i) the amino acid sequence of SEQ ID NO: 49, (ii) an amino acid sequence 90% or more identical to (i), or (iii) an amino acid sequence that includes 1 to 10 amino acid substitutions, insertions or deletions with respect to (i). In certain embodiments, an engineered yeast strain further includes genetic modifications that reduce the long chain acyl-CoA synthetase activity and the acyl-CoA synthetase activity. In some embodiments, the reduced ACS1 activity results from a reduction in the level of expression of a polypeptide including (i) the amino acid sequence of SEQ ID NO: 49, (ii) an amino acid sequence 90% or more identical to (i), or (iii) an amino acid sequence that includes 1 to 10 amino acid substitutions, insertions or deletions with respect to (i), and the reduced FAT1 activity results from a reduction in the level of expression of a polypeptide including (iv) the amino acid sequence of SEQ ID NO: 51, (v) an amino acid sequence 90% or more identical to (iv), or (vi) an amino acid sequence that includes 1 to 10 amino acid substitutions, insertions or deletions with respect to (iv).

In some embodiments, an engineered yeast strain further includes one or more genetic modifications that increase one or more activities, with respect to the activity level in an unmodified or parental strain, which increased activities are chosen from: an acyl-CoA oxidase activity, one or more monooxygenase activities, a monooxygenase reductase activity and an acyl-CoA hydrolase activity. In certain embodiments, a genetic modification that increases an acyl-CoA oxidase activity increases a POX5 activity. In some embodiments, the POX5 activity is provided by an increased amount of an enzyme including (i) the amino acid sequence of SEQ ID NO: 40, (ii) an amino acid sequence 90% or more identical to (i), or (iii) an amino acid sequence that includes 1 to 10 amino acid substitutions, insertions or deletions with respect to (i). In certain embodiments, a genetic modification that increases an acyl-CoA hydrolase activity increases an ACH activity. In some embodiments, the ACH activity is an ACHA activity, an ACHB activity or an ACHA and an ACHB activity. In certain embodiments, the ACHA activity is provided by an increased amount of an enzyme including (i) the amino acid sequence of SEQ ID NO: 43, (ii) an amino acid sequence 90% or more identical to (i), or (iii) an amino acid sequence that includes 1 to 10 amino acid substitutions, insertions or deletions with respect to (i), and the ACHB activity is provided by an increased amount of an enzyme including (iv) the amino acid sequence of SEQ ID NO: 45, (v) an amino acid sequence 90% or more identical to (iv), or (vi) an amino acid sequence that includes 1 to 10 amino acid substitutions, insertions or deletions with respect to (iv).

In some embodiments, the one or more monooxygenase activities includes one or more cytochrome P450 activities chosen from: a CYP52A13 activity, a CYP52A14 activity, a CYP52A15 activity, a CYP52A16 activity, and a CYP52A19 activity. In certain embodiments, the CYP52A13 activity is provided by an increased amount of an enzyme including (i) the amino acid sequence of SEQ ID NO: 63, (ii) an amino acid sequence 90% or more identical to (i), or (iii) an amino acid sequence that includes 1 to 10 amino acid substitutions, insertions or deletions with respect to (i). In some embodiments, the CYP52A14 activity is provided by an increased amount of an enzyme including (i) the amino acid sequence of SEQ ID NO: 65, (ii) an amino acid sequence 90% or more identical to (i), or (iii) an amino acid sequence that includes 1 to 10 amino acid substitutions, insertions or deletions with respect to (i). In certain embodiments, the CYP52A15 activity is provided by an increased amount of an enzyme including (i) the amino acid sequence of SEQ ID NO: 67, (ii) an amino acid sequence 90% or more identical to (i), or (iii) an amino acid sequence that includes 1 to 10 amino acid substitutions, insertions or deletions with respect to (i). In some embodiments, the CYP52A16 activity is provided by an increased amount of an enzyme including (i) the amino acid sequence of SEQ ID NO: 69, (ii) an amino acid sequence 90% or more identical to (i), or (iii) an amino acid sequence that includes 1 to 10 amino acid substitutions, insertions or deletions with respect to (i). In certain embodiments, the CYP52A19 activity is provided by an increased amount of an enzyme including (i) the amino acid sequence of SEQ ID NO: 75, (ii) an amino acid sequence 90% or more identical to (i), or (iii) an amino acid sequence that includes 1 to 10 amino acid substitutions, insertions or deletions with respect to (i).

In certain embodiments, a genetic modification that increases a monooxygenase reductase activity increases a CPR activity. In some embodiments, the CPR activity is a CPRB activity. In certain embodiments, the CPRB activity is provided by an increased amount of an enzyme including (i) the amino acid sequence of SEQ ID NO: 81, (ii) an amino acid sequence 90% or more identical to (i), or (iii) an amino acid sequence that includes 1 to 10 amino acid substitutions, insertions or deletions with respect to (i).

In some embodiments, the one or more increased activities are three or more increased activities. In certain embodiments, the three or more increased activities are four or more increased activities. In some embodiments, the three or more increased activities are five or more increased activities. In certain embodiments, the three or more increased activities are six or more increased activities. In some embodiments, the three or more increased activities include an increased monooxygenase activity, an increased monooxygenase reductase activity, and an increased acyl-CoA oxidase activity. In certain embodiments, the four or more increased activities include an increased monooxygenase activity, an increased monooxygenase reductase activity, an increase acyl-CoA oxidase, and an increased acyl-CoA hydrolase activity. In some embodiments, the five or more increased activities include at least two increased monooxygenase activities, an increased monooxygenase reductase activity, an increased acyl-CoA oxidase activity, and an increased acyl-CoA hydrolase activity. In certain embodiments, the six or more increased activities include at least two increased monooxygenase activities, an increased monooxygenase reductase activity, an increased acyl-CoA oxidase activity, and at least two increased acyl-CoA hydrolase activities.

In certain embodiments, the one or more increased activities independently is provided by an increased amount of an enzyme from a yeast. In some embodiments, each of the one or more increased activities independently results from increasing the copy number of a gene that encodes an enzyme that provides the activity. In certain embodiments, the one or more increased activities independently results from inserting a promoter in functional proximity to a gene that encodes an enzyme that provides the activity.

In some embodiments, the yeast is a Candida yeast. Genetically modified yeast can be derived from an ancestral yeast cell line chosen from: ATCC 20362, ATCC 8862, ATCC 18944, ATCC 20228, ATCC 76982, LGAM S(7)1, ATCC 20336, ATCC20913, SU-2 (ura3-/ura3-), ATCC 20962, ATCC 24690, ATCC 38163, H5343, ATCC 8661, ATCC 8662, ATCC 9773, ATCC 15586, ATCC 16617, ATCC 16618, ATCC 18942, ATCC 18943, ATCC 18944, ATCC 18945, ATCC 20114, ATCC 20177, ATCC 20182, ATCC 20225, ATCC 20226, ATCC 20228, ATCC 20237, ATCC 20255, ATCC 20287, ATCC 20297, ATCC 20306, ATCC 20315, ATCC 20320, ATCC 20324, ATCC 20341, ATCC 20346, ATCC 20348, ATCC 20362, ATCC 20363, ATCC 20364, ATCC 20372, ATCC 20373, ATCC 20383, ATCC 20390, ATCC 20400, ATCC 20460, ATCC 20461, ATCC 20462, ATCC 20496, ATCC 20510, ATCC 20628, ATCC 20688, ATCC 20774, ATCC 20775, ATCC 20776, ATCC 20777, ATCC 20778, ATCC 20779, ATCC 20780, ATCC 20781, ATCC 20794, ATCC 20795, ATCC 20875, ATCC 22421, ATCC 22422, ATCC 22423, ATCC 22969, ATCC 32338, ATCC 32339, ATCC 32340, ATCC 32341, ATCC 32342, ATCC 32343, ATCC 32935, ATCC 34017, ATCC 34018, ATCC 34088, ATCC 34922, ATCC 38295, ATCC 42281, ATCC 44601, ATCC 46025, ATCC 46026, ATCC 46027, ATCC 46028, ATCC 46067, ATCC 46068, ATCC 46069, ATCC 46070, ATCC 46330, ATCC 46482, ATCC 46483, ATCC 46484, ATCC 48436, ATCC 60594, ATCC 62385, ATCC 64042, ATCC 74234, ATCC 76598, ATCC 76861, ATCC 76862, ATCC 90716, ATCC 90806, ATCC 90811, ATCC 90812, ATCC 90813, ATCC 90814, ATCC 90903, ATCC 90904, ATCC 90905, ATCC 96028, ATCC 201089, ATCC 201241, ATCC 201242, ATCC 201243, ATCC 201244, ATCC 201245, ATCC 201246, ATCC 201247, ATCC 201248, ATCC 201249, ATCC 201847, ATCC MYA-165, ATCC MYA-166, ATCC MYA-2613, and ATCC MYA-4467.

Certain embodiments are described further in the following description, examples, claims and drawings.

### Brief Description of the Drawings

The drawings illustrate embodiments of the technology and are not limiting. For clarity and ease of illustration, the drawings are not made to scale and, in some instances, various aspects may be shown exaggerated or enlarged to facilitate an understanding of particular embodiments.

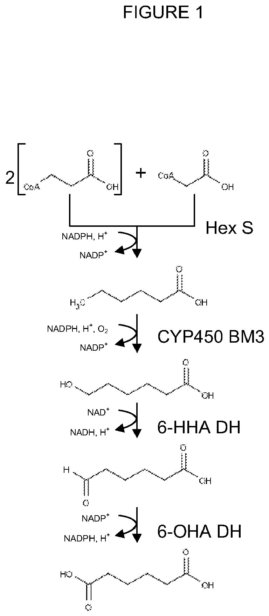

Figure 1 depicts a metabolic pathway for making adipic acid. The pathway can be engineered into a eukaryotic microorganism to generate a microorganism capable of producing adipic acid.

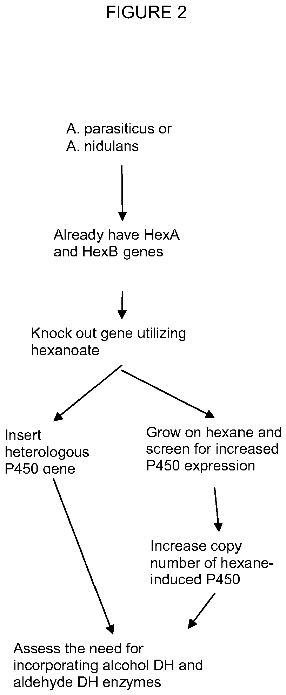

Figure 2 depicts an embodiment for a method of generating an adipic acid producing microorganism. The method comprises expressing one or more genes catalyzing the omega oxidation of fatty acids to dicarboxylic acids in a host microorganism that produces hexanoate. In the method depicted, the host organism, for example A. parasiticus or A. nidulans, endogenously includes HEXA and HEXB (or STCJ and STCK) genes. In one embodiment the method comprises knocking out or otherwise disabling the gene coding for diversion of hexanoate into an endogenous pathway such as mycotoxin production. Certain embodiments of the method further comprise inserting a heterologous cytochrome P450 gene. Some embodiments of the method comprise growing the culture on hexane and screening for increased P450 expression. The copy number of hexanoate induced P450 may in certain embodiments be increased. In some embodiments the microorganism may be altered to increase the flux of six carbon substrate through the final two oxidation steps.

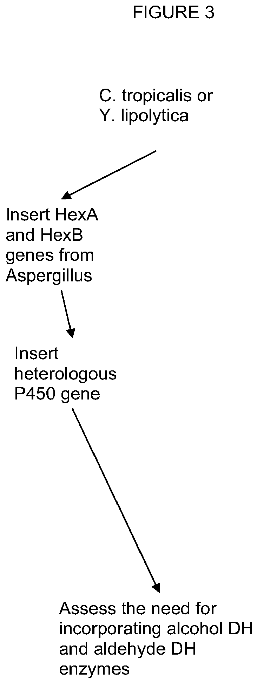

Figure 3 depicts an embodiment for a method of generating an adipic acid producing organism. The method comprises expressing one or more genes encoding hexanoate synthase in a host microorganism that produces dicarboxylic acids via an omega-oxidation pathway. Such microorganisms may include, without limitation, C. tropicalis, C. viswanithii and C. maltosa. As depicted, the method comprises inserting HEXA and HEXB genes into the host microorganism. The genes may be isolated from Aspergillus, or another appropriate organism. In some embodiments, the genes are synthesized from an alternative sequence as described herein to produce the amino acid sequence of the donor mircroorganism enzyme through a non-standard translation mechanism of C. tropicalis. In some embodiments the method comprises inserting a heterologous cytochrome P450 gene into the host organism. In certain embodiments the microorganism may be altered to increase the flux of a six-carbon substrate through the final two oxidation steps.

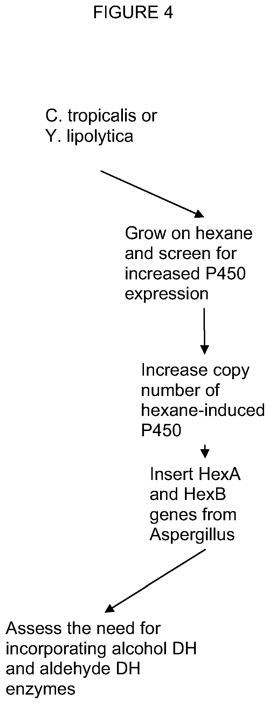

Figure 4 depicts an embodiment of a method for generating an adipic acid producing organism. The method comprises expressing one or more genes encoding hexanoate synthase in a host microorganism that produces dicarboxylic acids via an omega-oxidation pathway. The microorganisms can include, without limitation, C. tropicalis, C. viswanithii and C. maltosa. In some embodiments, the method comprises growing a host microorganism on hexane and screening for increased P450 expression. In certain embodiments, copy number of hexane-induced P450 may be increased. HEXA and HEXB genes may be inserted into the host microorganism. In certain embodiments, the host microorganism may be altered to increase the flux of a six-carbon substrate through the final two oxidation steps.

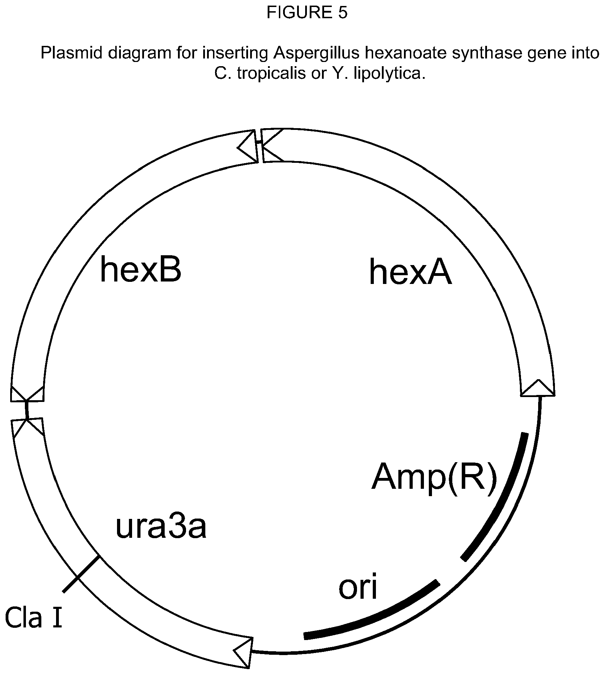

Figure 5 depicts a plasmid diagram for inserting Aspergillus hexanoate synthase genes HEXA and HEXB into C. tropicalis or Y. lipolytica.

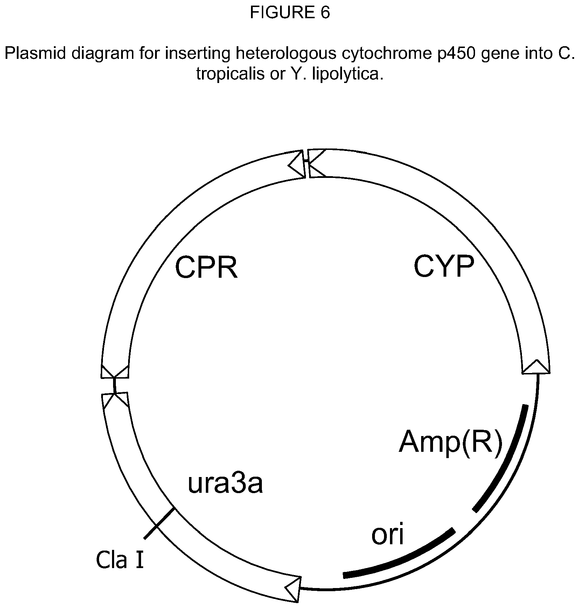

Figure 6 depicts a plasmid diagram for inserting a heterologous cytochrome P450 monooxygenase gene and cytochrome P450 reductase gene into C. tropicalis or Y. lipolytica.

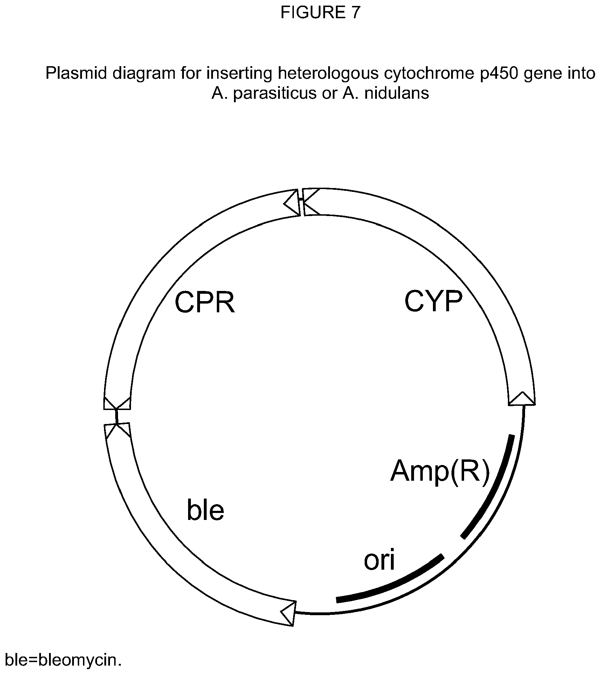

Figure 7 depicts a plasmid diagram for inserting a heterologous cytochrome P450 monooxygenase gene and cytochrome P450 reductase gene into A. parasiticus or A. nidulans.

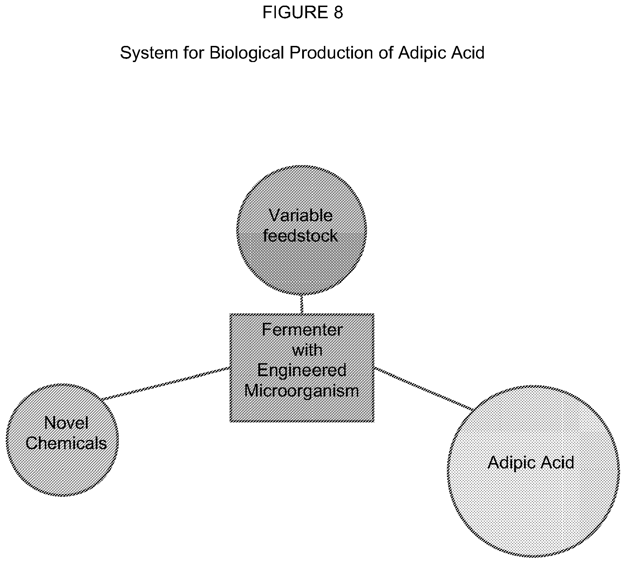

Figure 8 depicts a system for biological production of a target product. As depicted, a fermenter is populated with microorganisms engineered for target product production. A flexible feedstock supplies the fermenter with an energy and nutrition source for the microorganisms. In some embodiments the feedstock comprises a sugar. In certain embodiments the feedstock comprises fatty acids. The feedstock may also include biomass, industrial waste products and other sources of carbon. Vitamins, minerals, enzymes and other growth or production enhancers may be added to the feedstock. In certain embodiments the fermentation produces adipic acid. The fermentation process may produce other novel chemicals.

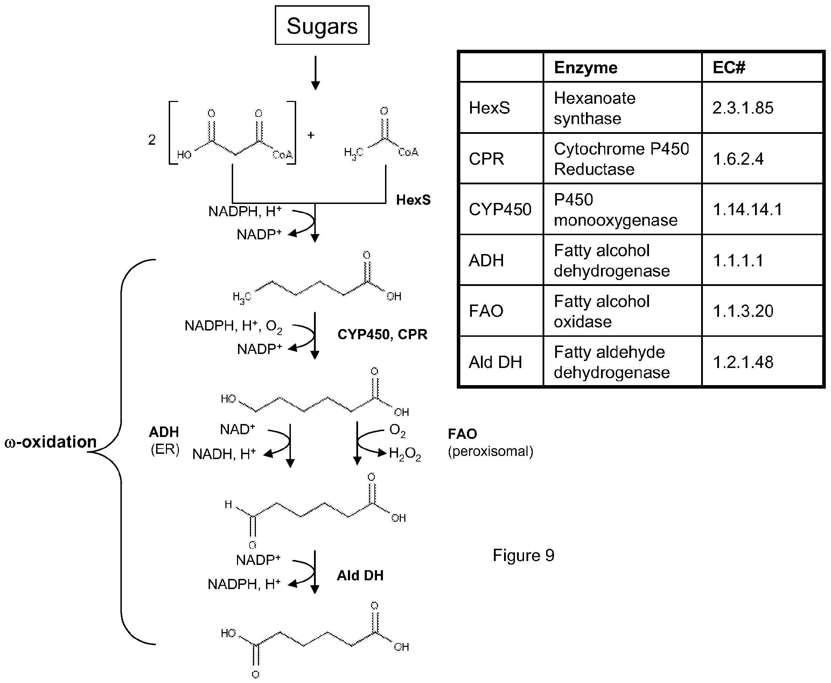

Figure 9 depicts a metabolic pathway for making adipic acid from saccharide or polysaccharide carbon sources, similar to the pathway depicted in Figure 1, with additional activities that aid in metabolism of, or enhance metabolism of, pathway intermediates, thereby potentially increasing the yield of adipic acid. The additional activities are a monooxygenase reductase activity (cytochrome P450 reductase or CPR) and a fatty alcohol oxidase activity (FAO). Part, or all, of the pathway can be engineered into a eukaryotic microorganism to generate a microorganism capable of producing adipic acid.

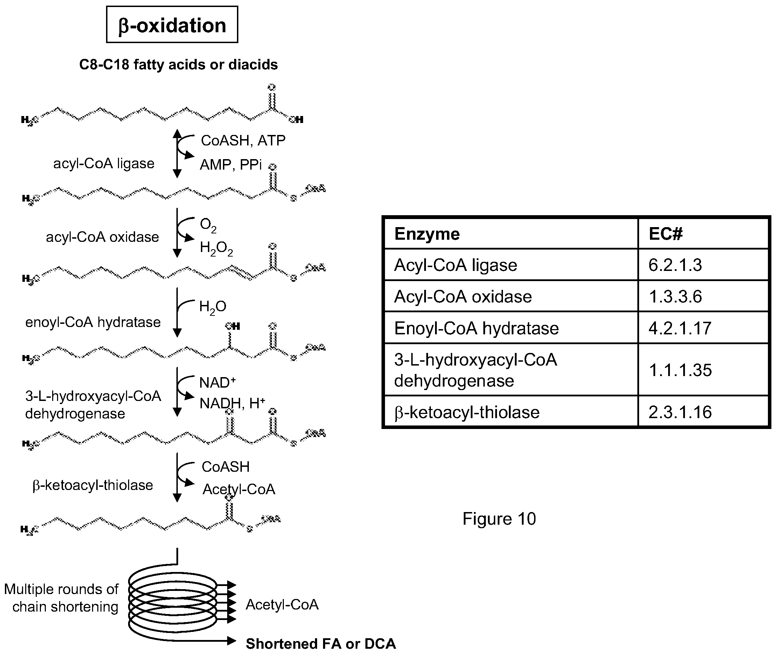

Figure 10 depicts a non-limiting example of a metabolic pathway for making adipic acid from paraffins, fats, oils, fatty acids or dicarboxylic acids, as described in Figure 2. Part, or all, of the pathway can be engineered (e.g., added, altered to increase or decrease copy number, or increase or decrease promoter activity, depending on the desired effect) into a microorganism, depending on the activities already present in the host organism, to generate a microorganism capable of producing adipic acid.

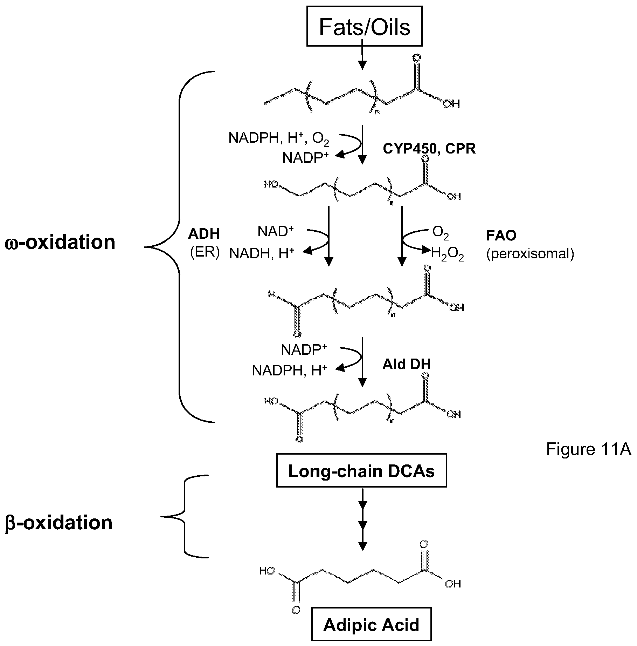

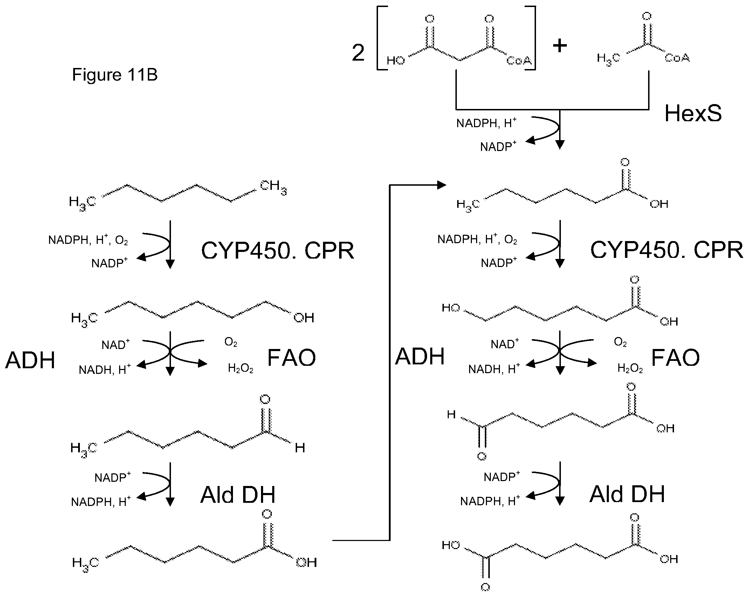

Figure 11A and 11B depict omega and beta oxidation pathways useful for producing adipic acid from various carbon sources. Adipic acid can be produced from paraffins, fats, oils and intermediates of sugar metabolism, using omega oxidation, as shown in Figure 11A. Adipic acid also can be produced from long chain fatty acids or dicarboxylic acids using beta oxidation, as shown in Figure 11A. Figure 11B shows a common intermediate from the metabolism of fats and sugars entering the omega oxidation pathway to ultimately produce adipic acid.

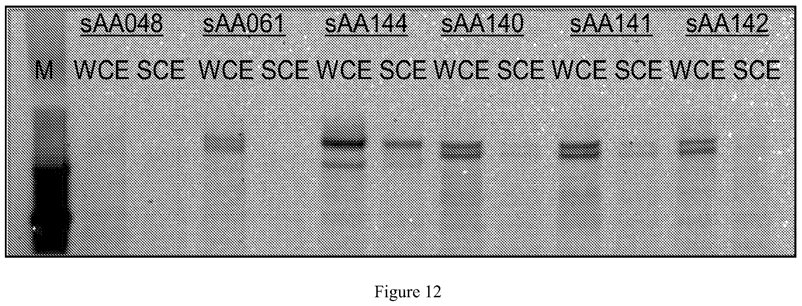

Figure 12 shows results of immunodetection of 6xHis-tagged proteins expressed in S. cerevisiae BY4742. Strains sAA061, sAA140, sAA141, sAA142 contain 6xHis-tagged HEXA and HEXB proteins. Strain sAA144 contains 6xHis-tagged STCJ and STCK proteins. Strain sAA048 contains only vectors p425GPD and p426GPD.

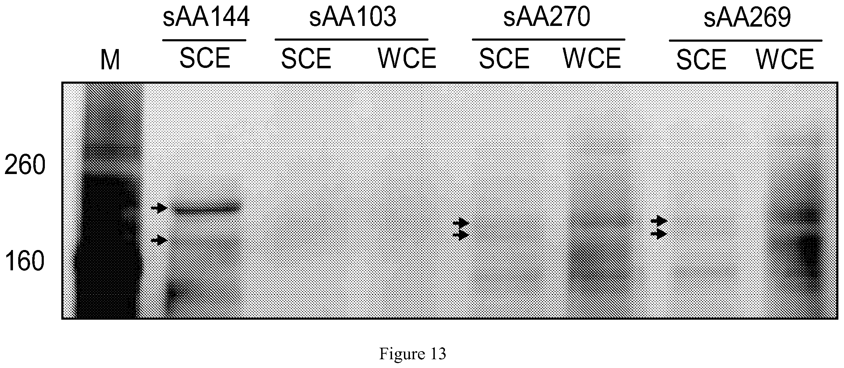

Figure 13 shows results of immunodetection of 6xHis-tagged proteins expressed in either S. cerevisiae (sAA144) or in C. tropicalis (sAA103, sAA270, sAA269). 6xHis tagged HEXA and HEXB expressed in strains sAA269 and sAA270 are indicated with arrows. 6xHis tagged STCJ and STCK from strain sAA144 were included as a positive control. Strain sAA103 is the parent strain for sAA269 and sAA270 and does not contain integrated vectors for the expression of 6xHis-tagged HEXA and HEXB.

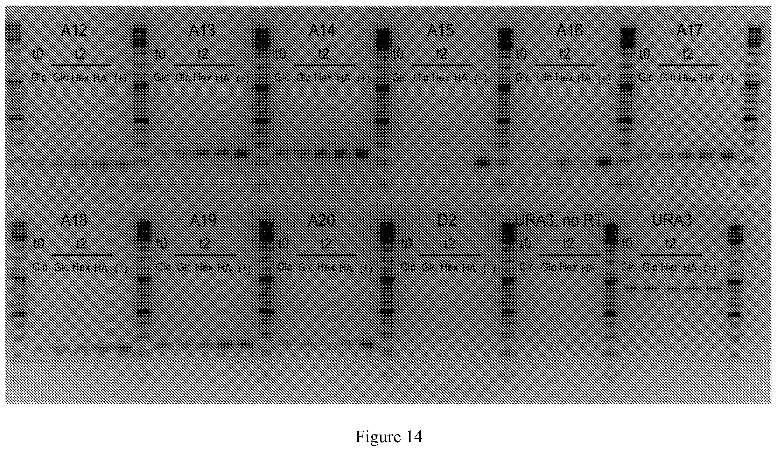

Figure 14 shows results of RT-PCR from cultures of C. tropicalis strain sAA003 exposed to glucose only (Glc), hexane only (Hex), or hexanoic acid only (HA). PCR products of A15 and A16 alleles show hexane and hexanoic acid specific induction.

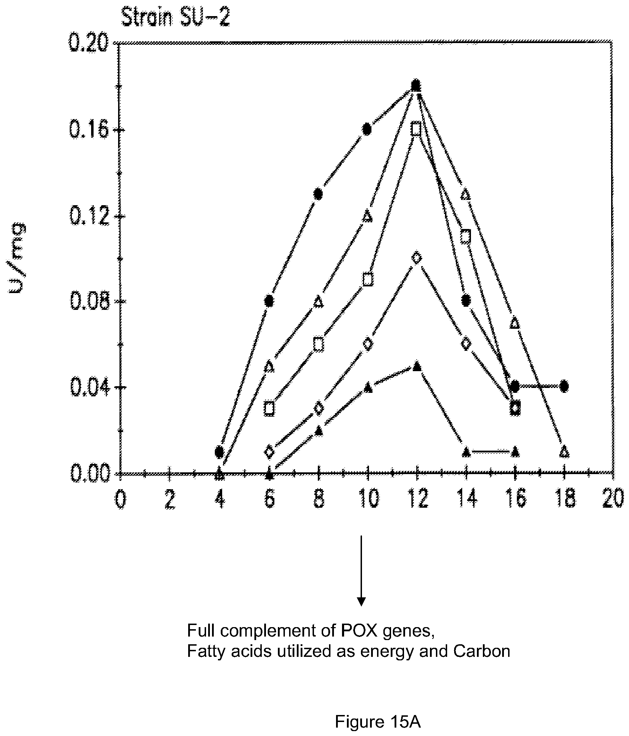

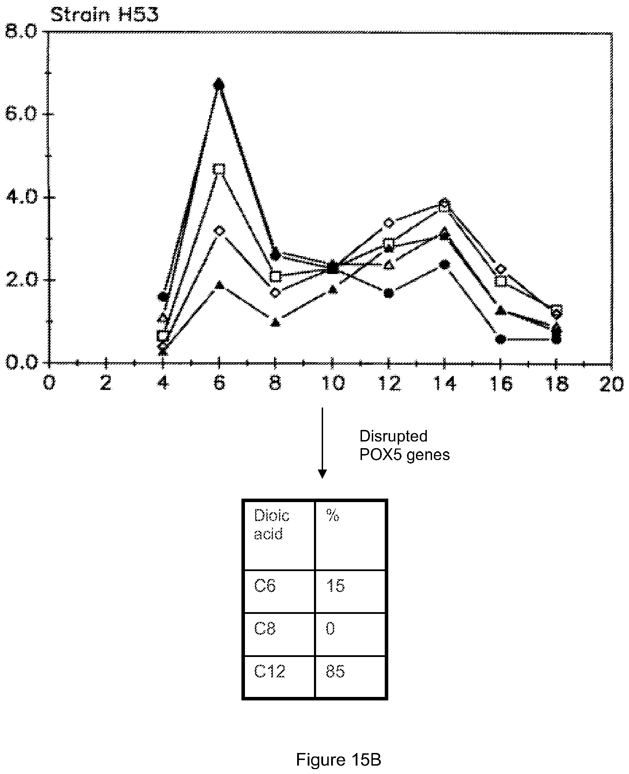

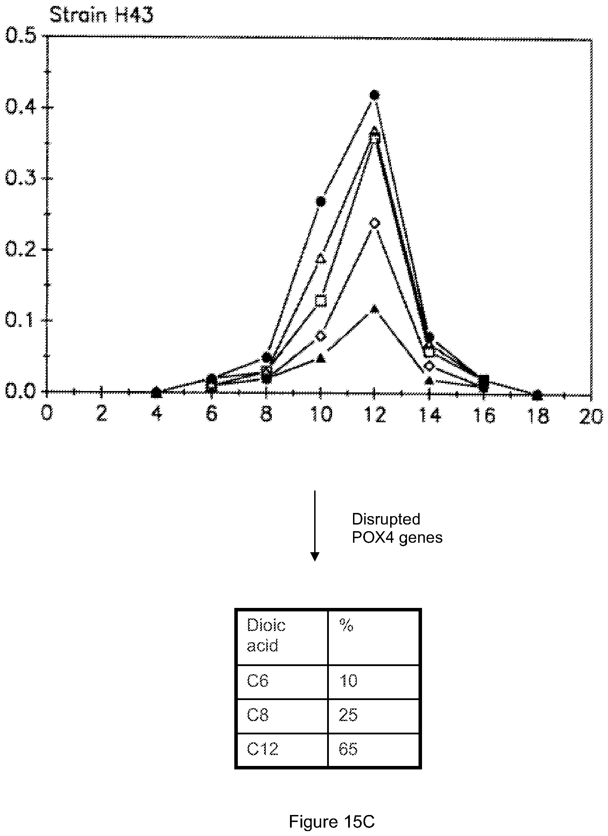

Figures 15A-15C illustrate results of acyl-CoA oxidase (POX) enzymatic activity assays on substrates of various carbon lengths, using acyl-CoA enzyme preparations from Candida tropicalis strains with no POX genes disrupted (see Figure 15A), POX4 genes disrupted (see Figure 15C) or POX5 genes disrupted (see Figure 15B). Experimental results and conditions are given in the Detailed Description and Examples sections.

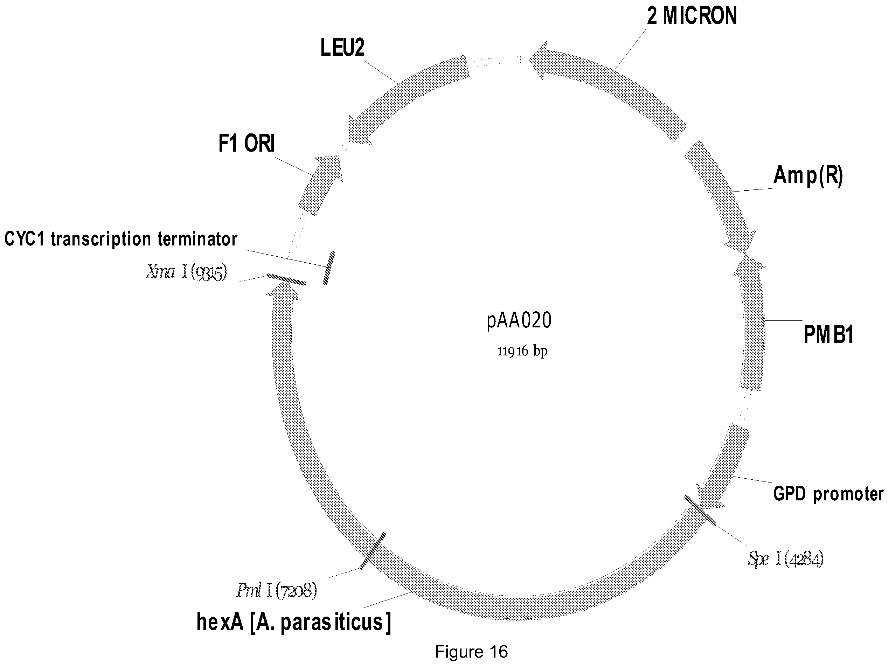

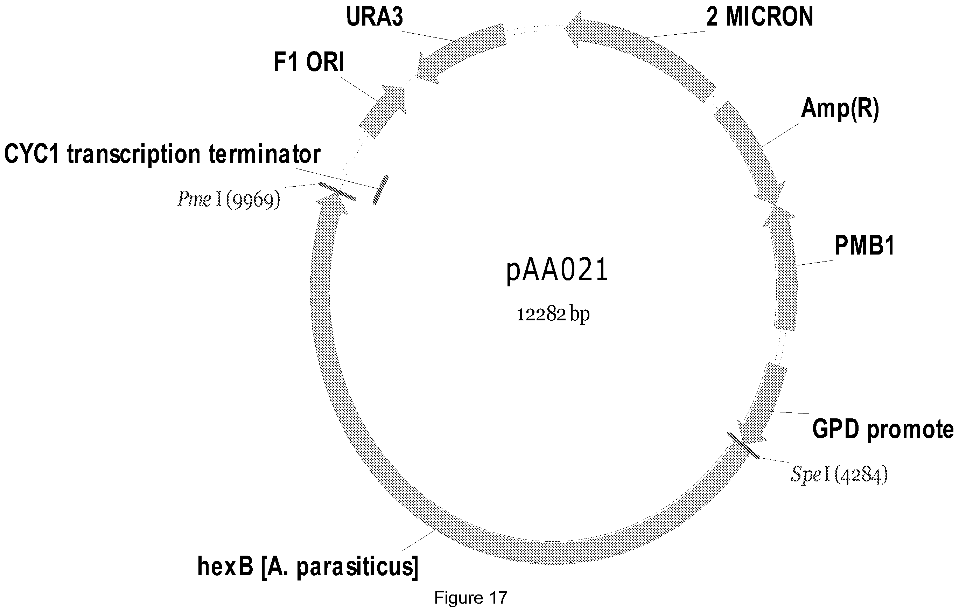

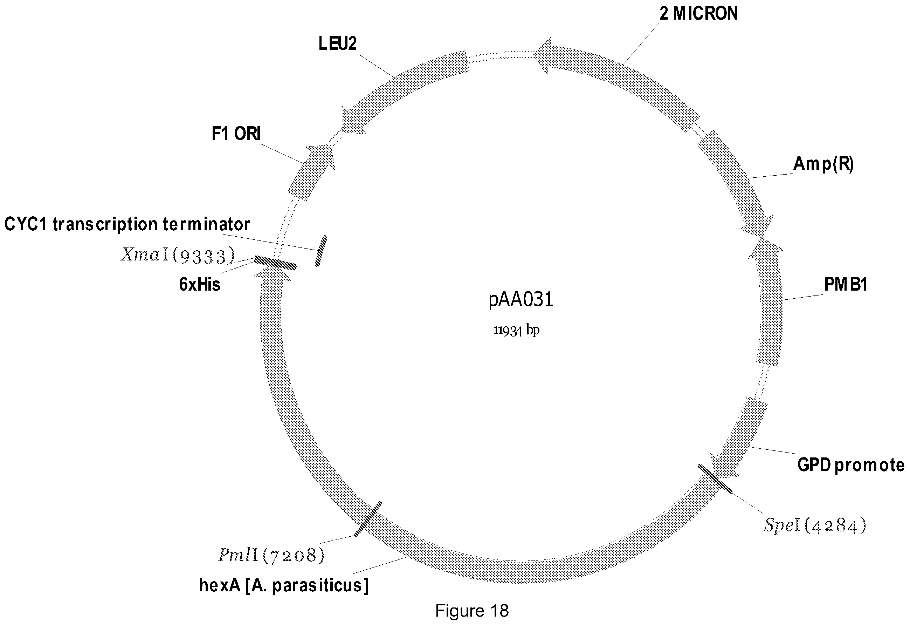

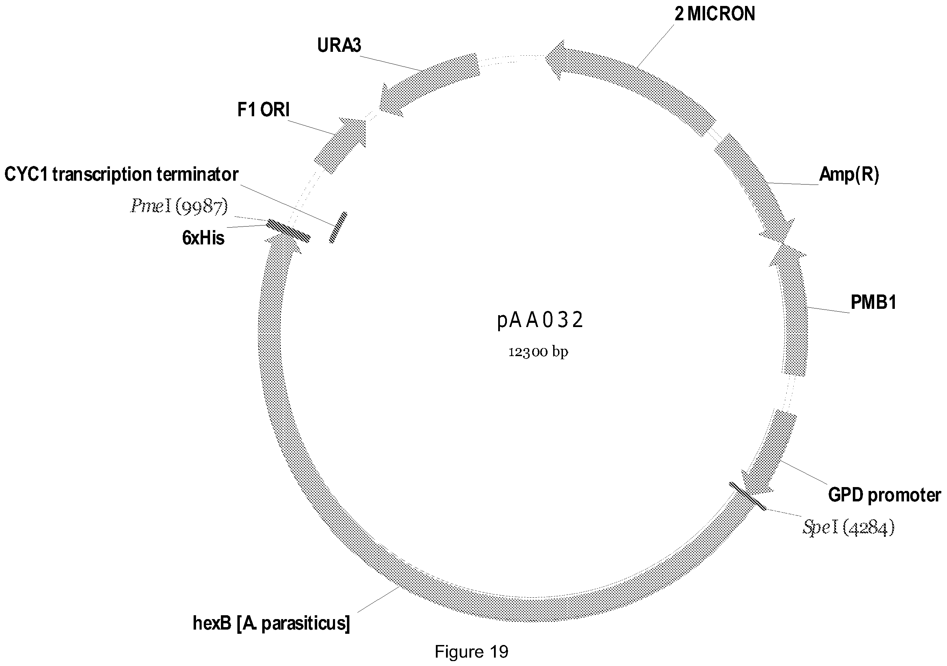

Figures 16-34 illustrate various plasmids for cloning, expression, or integration of various activities described herein, into a host organism or engineered organism. Figure 16 depicts a plasmid diagram for inserting a heterologous HEXA gene into S. cerevisiae. Figure 17 depicts a plasmid diagram for inserting a heterologous HEXB gene into S. cerevisiae. Figure 18 depicts a plasmid diagram for inserting a heterologous HEXA-6xHis gene into S. cerevisiae. Figure 19 depicts a plasmid diagram for inserting a heterologous HEXB-6xHis gene into S. cerevisiae.

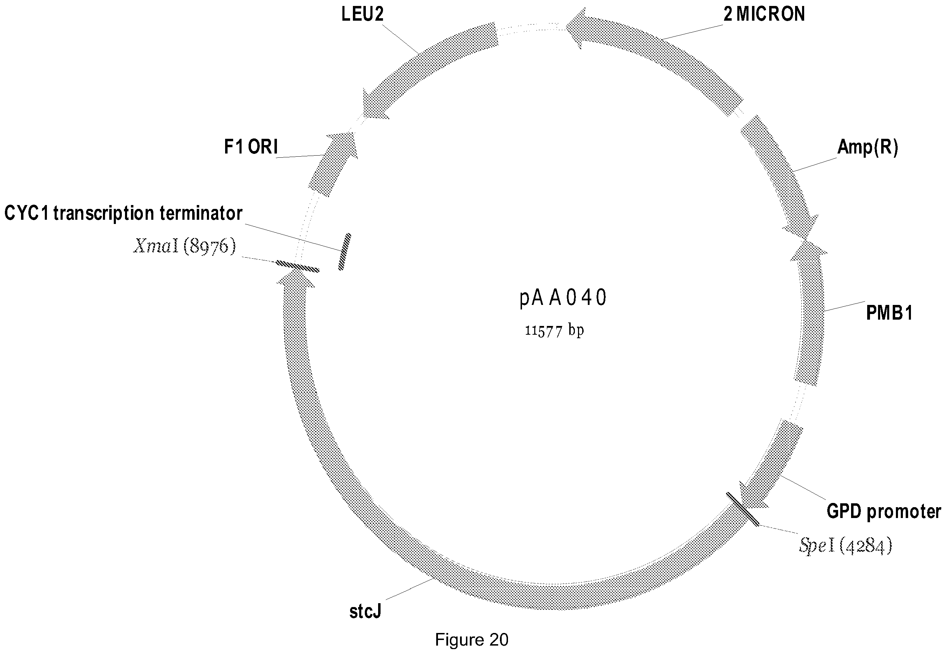

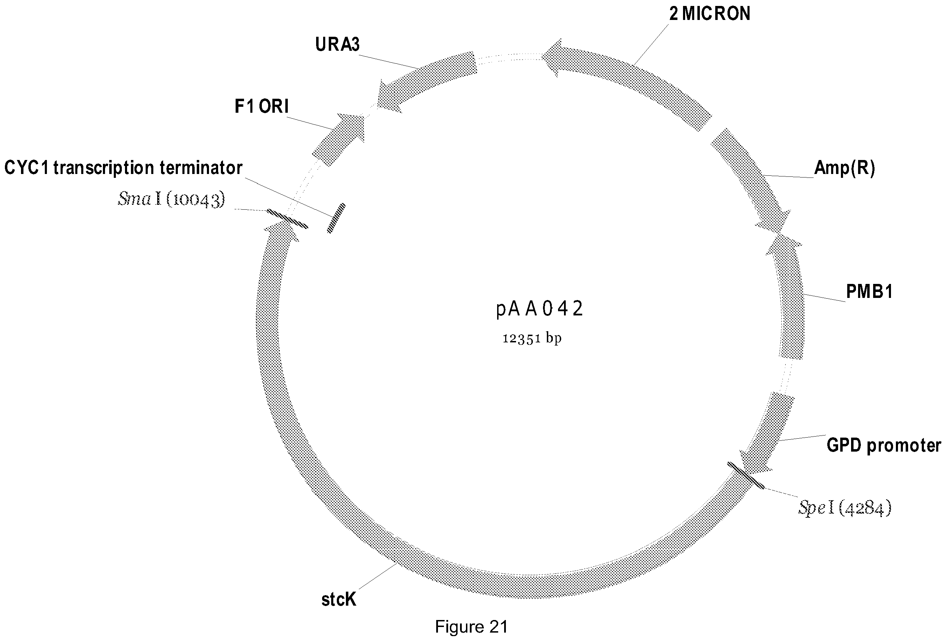

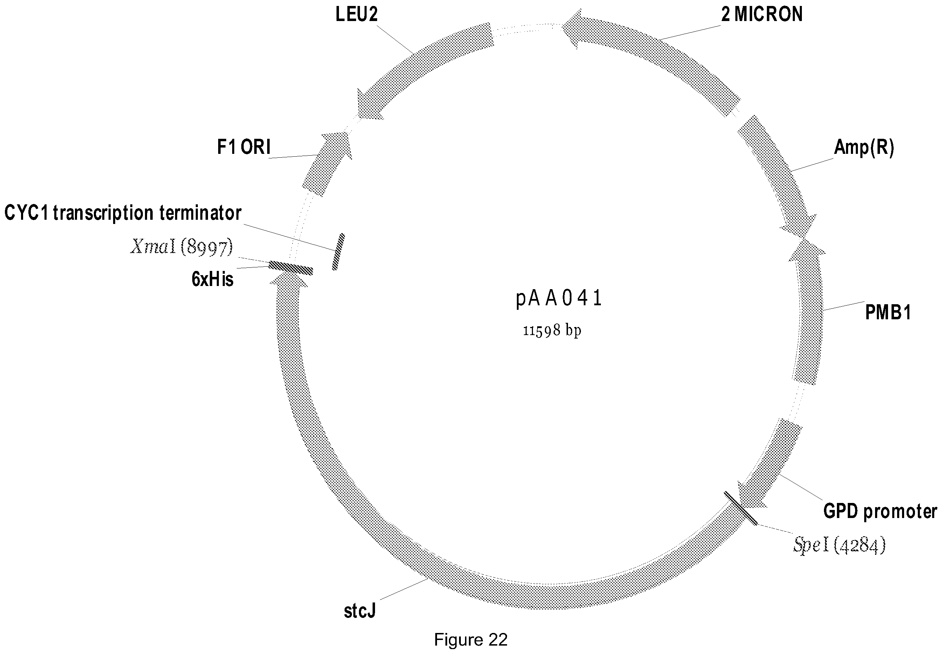

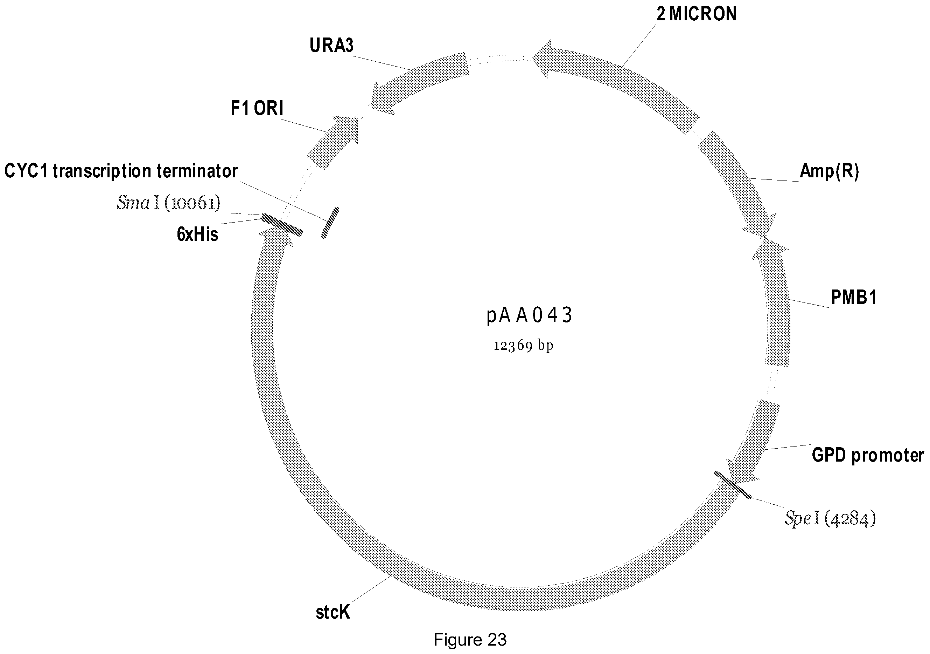

Figure 20 depicts a plasmid diagram for inserting a heterologous STCJ gene into S. cerevisiae. Figure 21 depicts a plasmid diagram for inserting a heterologous STCK gene into S. cerevisiae. Figure 22 depicts a plasmid diagram for inserting a heterologous STCJ-6xHis gene into S. cerevisiae. Figure 23 depicts a plasmid diagram for inserting a heterologous STCK-6xHis gene into S. cerevisiae.

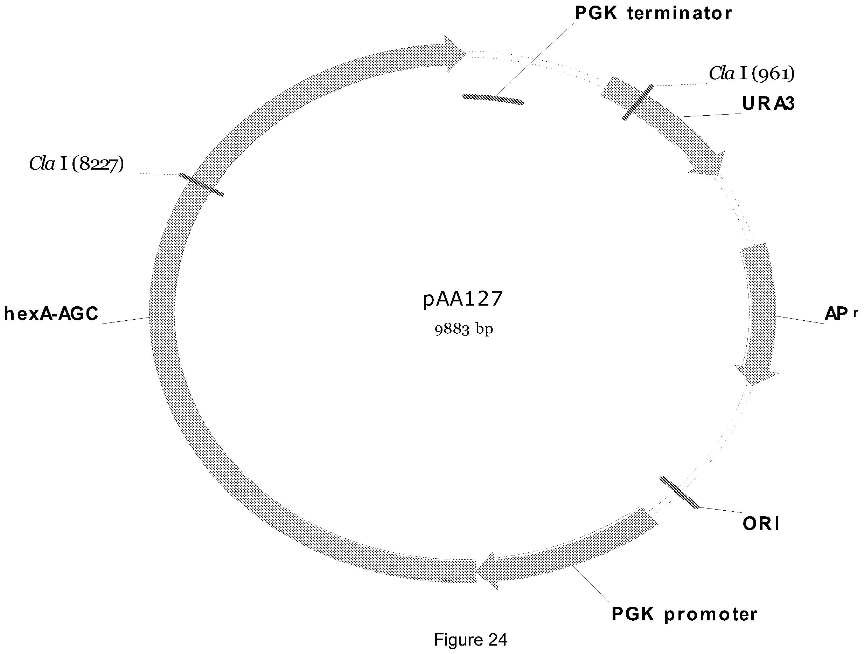

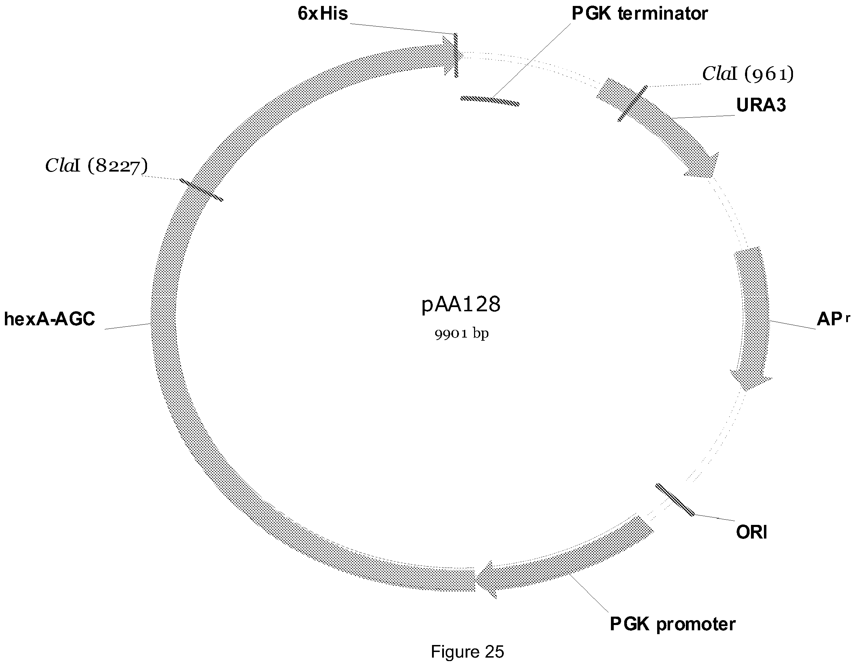

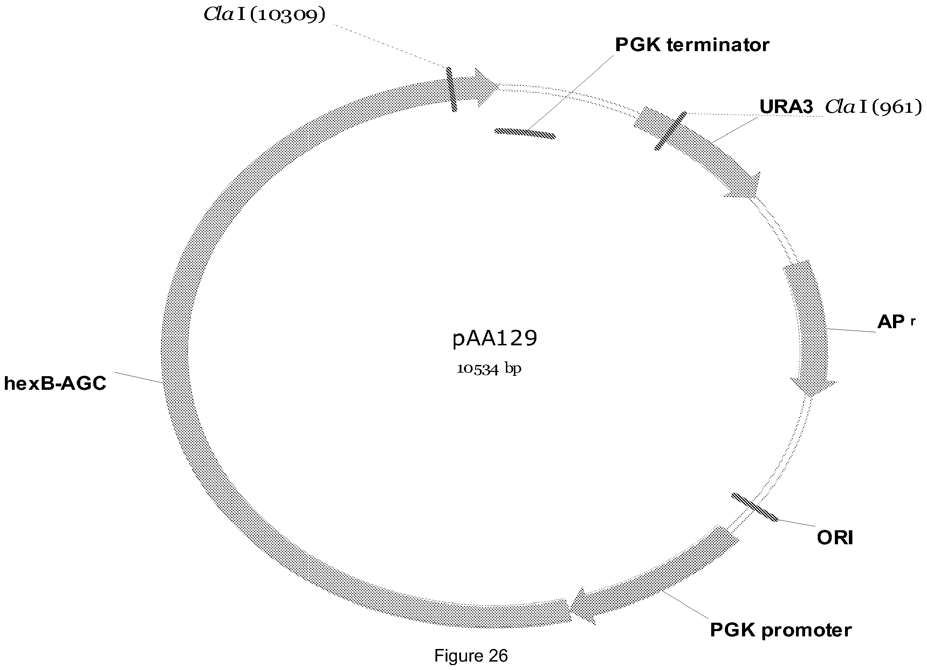

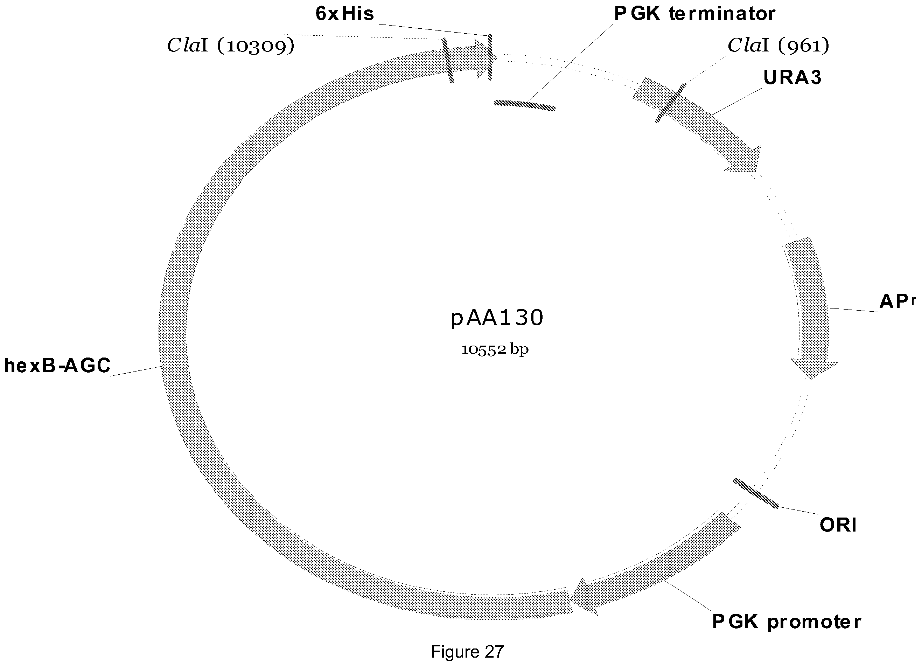

Figure 24 depicts a plasmid diagram for inserting a heterologous alternative genetic code (AGC) HEXA gene into C. tropicalis. Figure 25 depicts a plasmid diagram for inserting a heterologous AGC-HEXB gene into C. tropicalis. Figure 26 depicts a plasmid diagram for inserting a heterologous AGC-HEXA-6xHis gene into C. tropicalis. Figure 27 depicts a plasmid diagram for inserting a heterologous AGC-HEXB-6xHis gene into C. tropicalis.

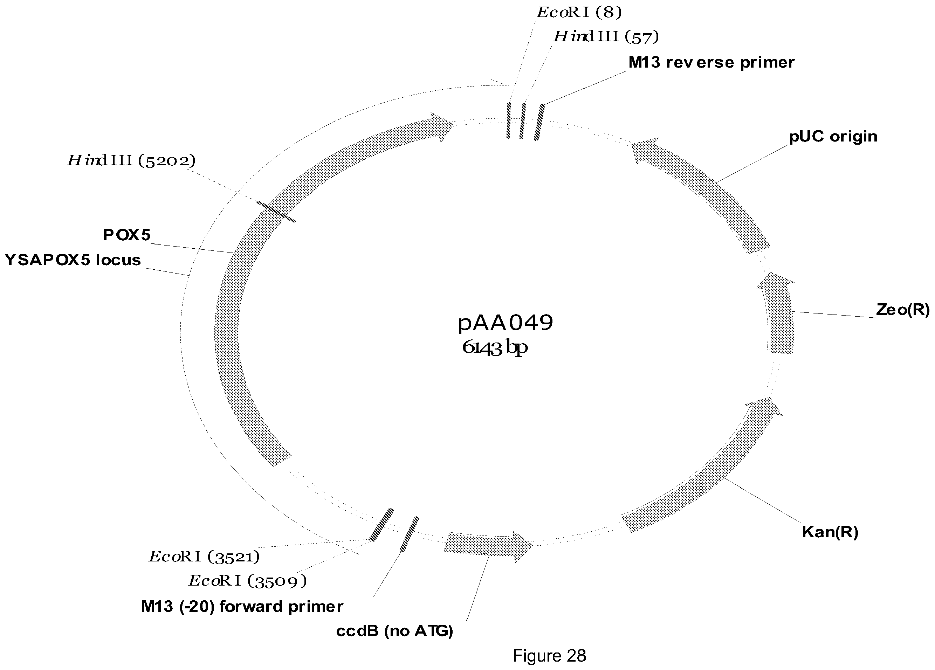

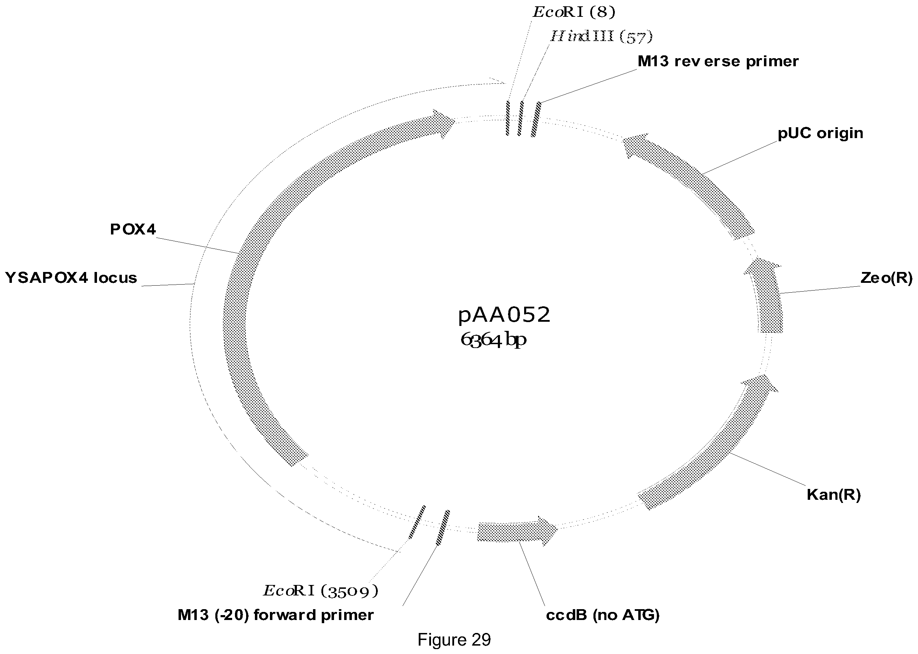

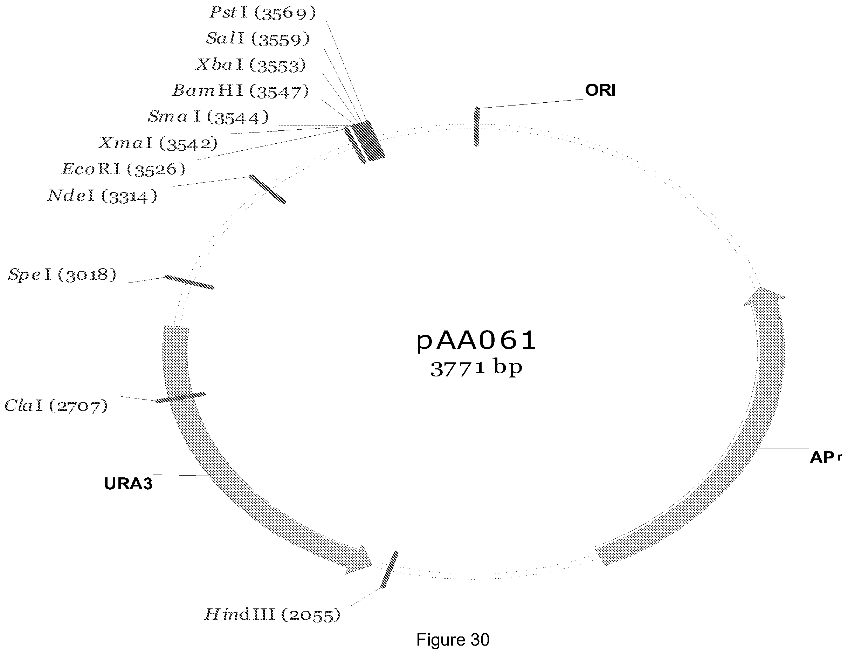

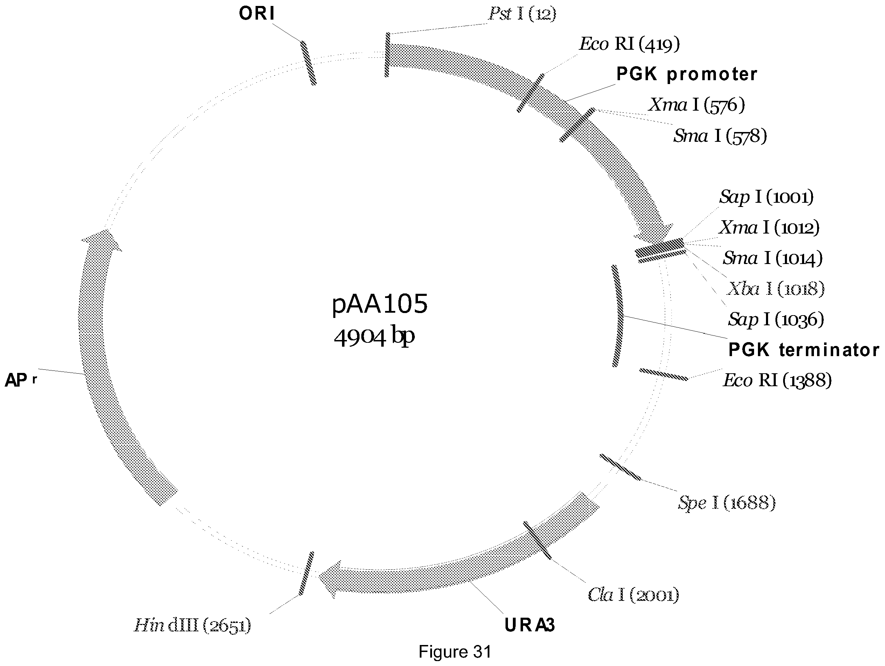

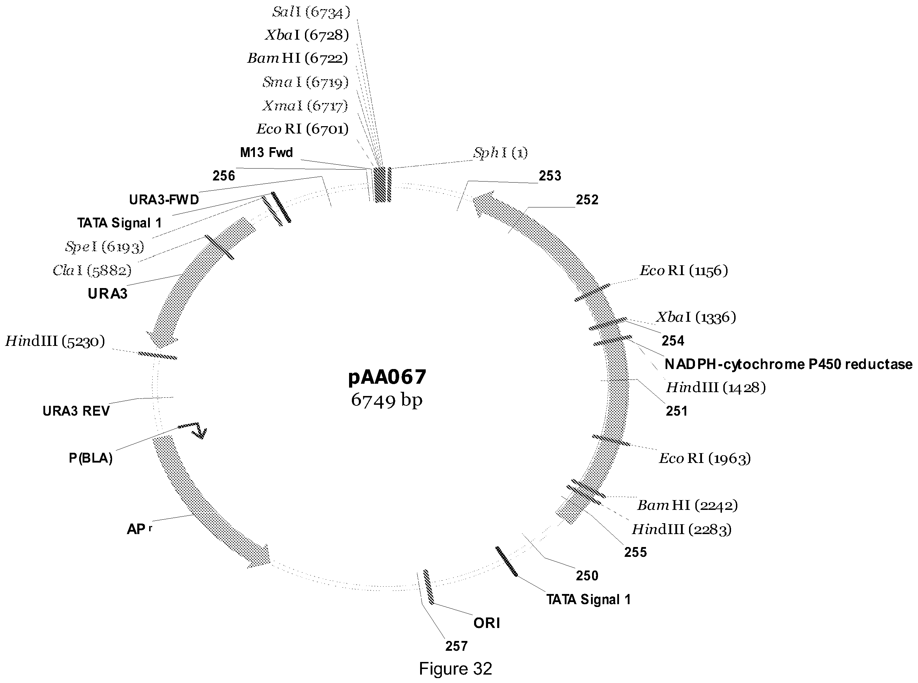

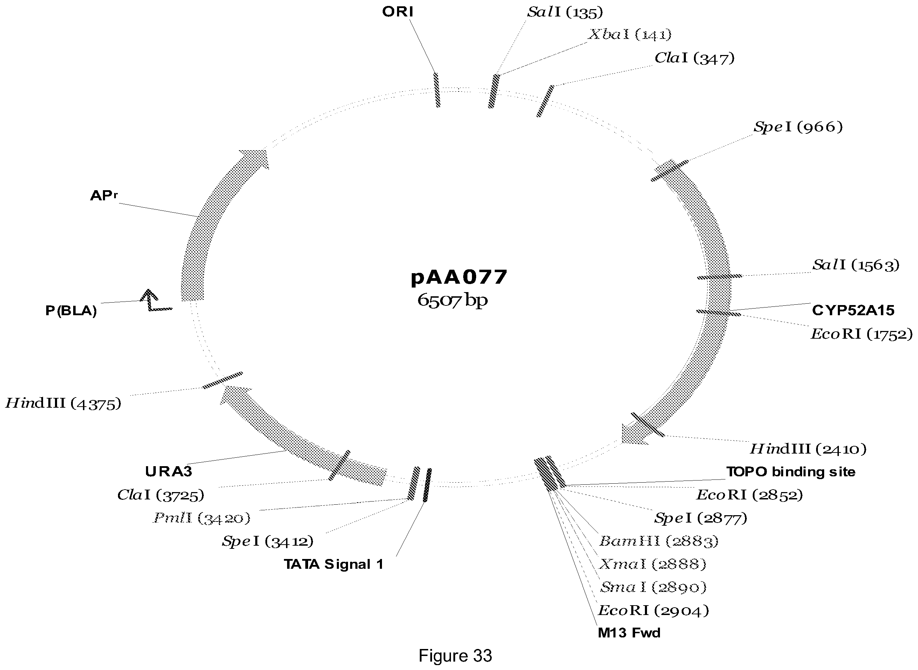

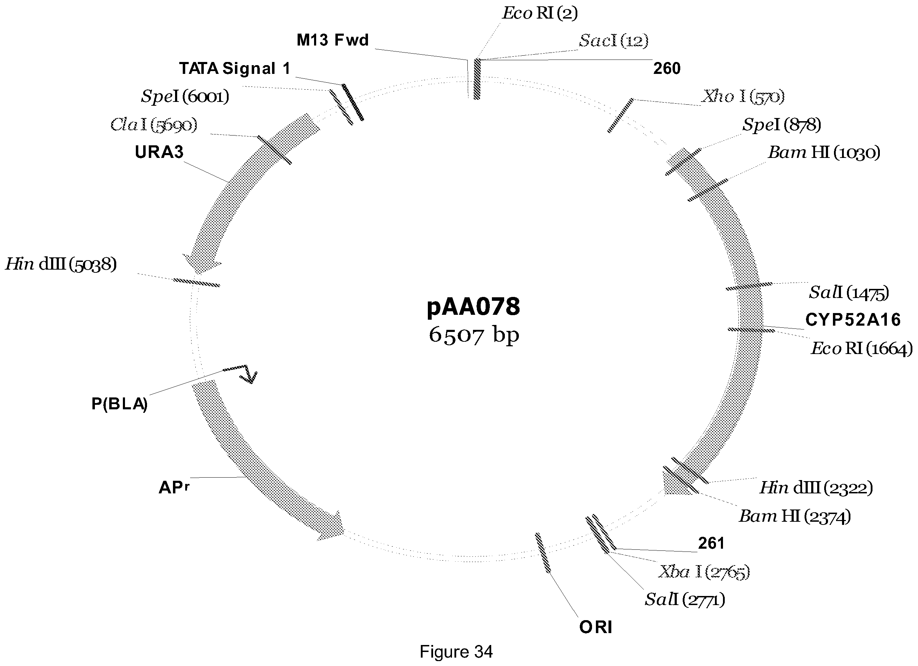

Figure 28 depicts a diagram of a plasmid used for cloning the POX5 gene from C. tropicalis. Figure 29 depicts a diagram of a plasmid used for cloning the POX4 gene from C. tropicalis. Figure 30 illustrates a plasmid constructed for use of URA selection in C. tropicalis. Figure 31 depicts a plasmid containing the PGK promoter and terminator from C. tropicalis. Figure 32 depicts a plasmid used for integration of the CPR gene in C. tropicalis. Figure 33 depicts a plasmid used for integration of the CYP52A15 gene in C. tropicalis. Figure 34 depicts a plasmid used for integration of the CYP52A16 gene in C. tropicalis.

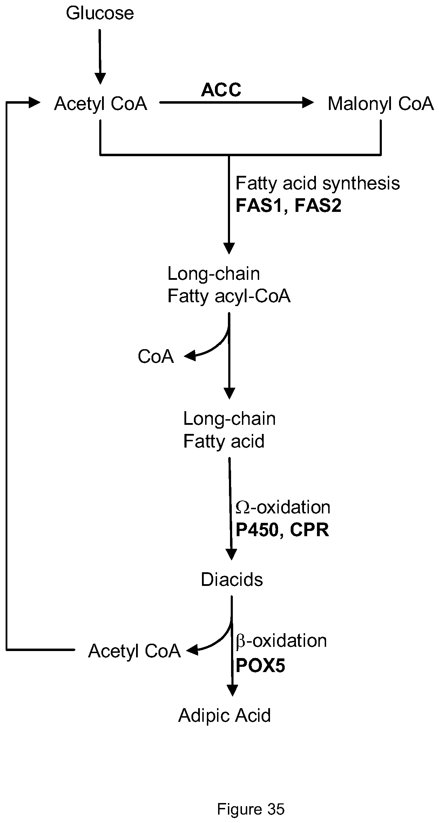

Figure 35 depicts a metabolic pathway for making adipic acid from saccharide or polysaccharide carbon sources, similar to the pathways depicted in Figures 1 and 9, with additional activities that aid in metabolism of, or enhance metabolism of, pathway intermediates, thereby potentially increasing the yield of adipic acid. The additional activities are an acetyl-CoA carboxylase activity (ACC), a fatty acid synthase (FAS1, FAS2), a monooxygenase (P450) activity, a monooxygenase reductase activity (CPR) and an acyl-CoA oxidase activity (POX5). Part, or all, of the pathway can be engineered into a eukaryotic microorganism to generate a microorganism capable of producing adipic acid.

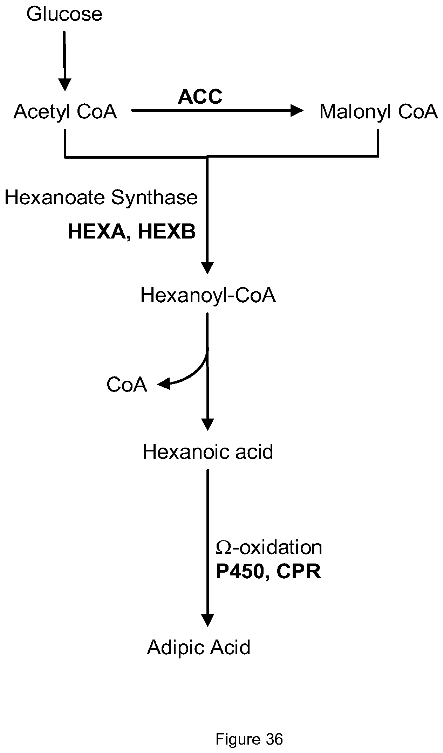

Figure 36 depicts a metabolic pathway for making adipic acid from saccharide or polysaccharide carbon sources, similar to the pathways depicted in Figures 1 and 9, with additional activities that aid in metabolism of, or enhance metabolism of, pathway intermediates, thereby potentially increasing the yield of adipic acid. The additional activities are an acetyl-CoA carboxylase activity (ACC), a hexanoate synthase (HEXA, HEXB), a monooxygenase (P450) activity, and a monooxygenase reductase activity (CPR). Part, or all, of the pathway can be engineered into a eukaryotic microorganism to generate a microorganism capable of producing adipic acid.

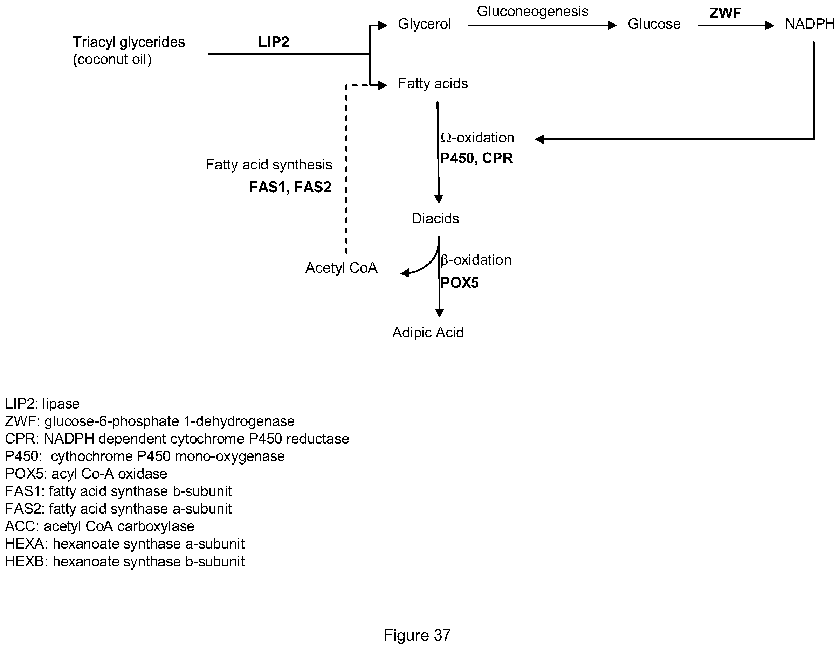

Figure 37 depicts a non-limiting example of a metabolic pathway for making adipic acid from paraffins, fats, oils, fatty acids or dicarboxylic acids, as described in Figures 2 and 10, with additional activities that aid in metabolism of, or enhance metabolism of, pathway intermediates, thereby potentially increasing the yield of adipic acid. The additional activities are a lipase, a fatty acid synthase, a glucose-6-phosphate dehydrogenase (ZWF1), a monooxygenase (P450) activity, and a monooxygenase reductase activity (CPR). Part, or all, of the pathway can be engineered (e.g., added, altered to increase or decrease copy number, or increase or decrease promoter activity, depending on the desired effect) into a microorganism, depending on the activities already present in the host organism, to generate a microorganism capable of producing adipic acid.

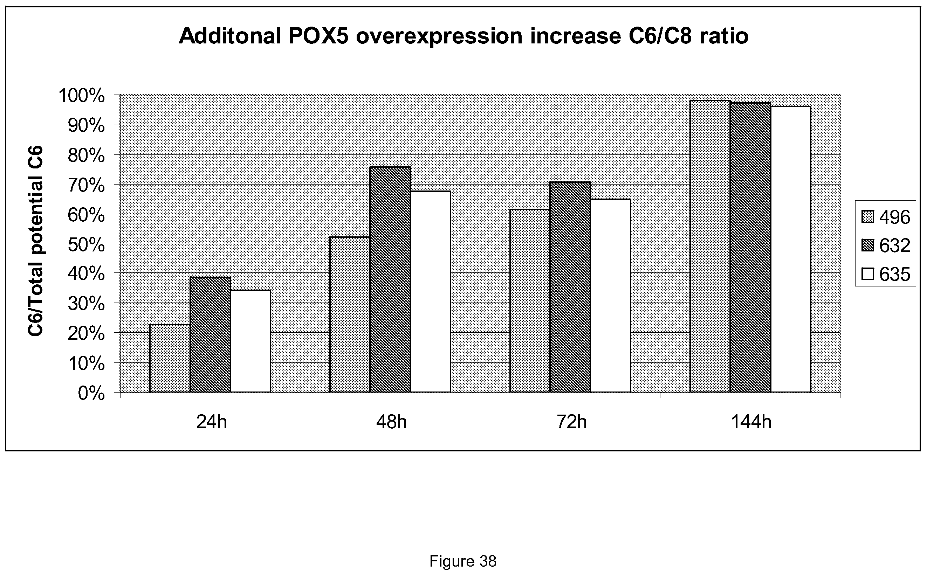

Figure 38 graphically illustrates the ratio of C6 diacids/(C6 + C8 diacids) produced in yeast cultures grown in shake flasks using coconut oil as the feedstock (e.g., carbon source). Experimental results and conditions are given in Example 32.

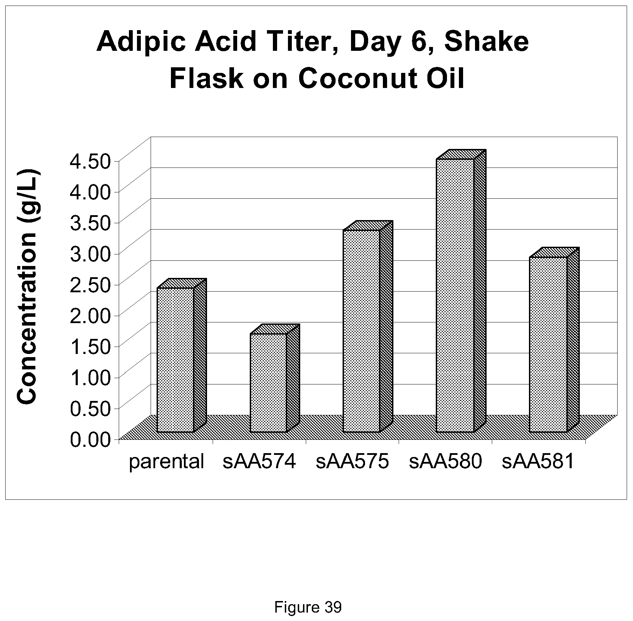

Figure 39 graphically illustrates the effect of increased lipase activity on the conversion of coconut oil to adipic acid. Experimental results and conditions are given in Example 33.

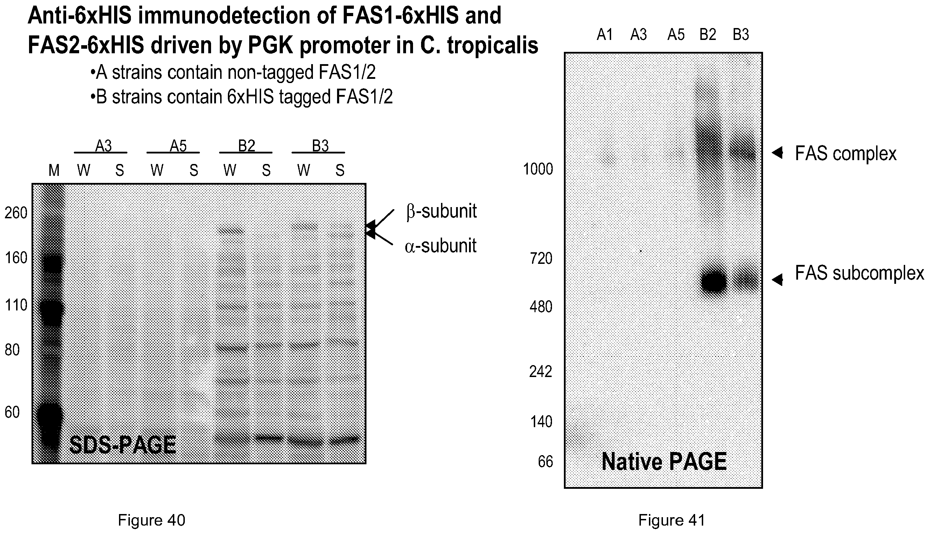

Figure 40 shows the results of immunodetection of over expressed polypeptides coding FAS2 and FAS1 activities after denaturing polyacrylamide gel electrophoresis (SDS-PAGE). Figure 41 shows the results of immunodetection of over expressed polypeptides coding FAS2 and FAS1 activities after native polyacrylamide gel electrophoresis (Native-PAGE). Experimental results and conditions are given in Example 35.

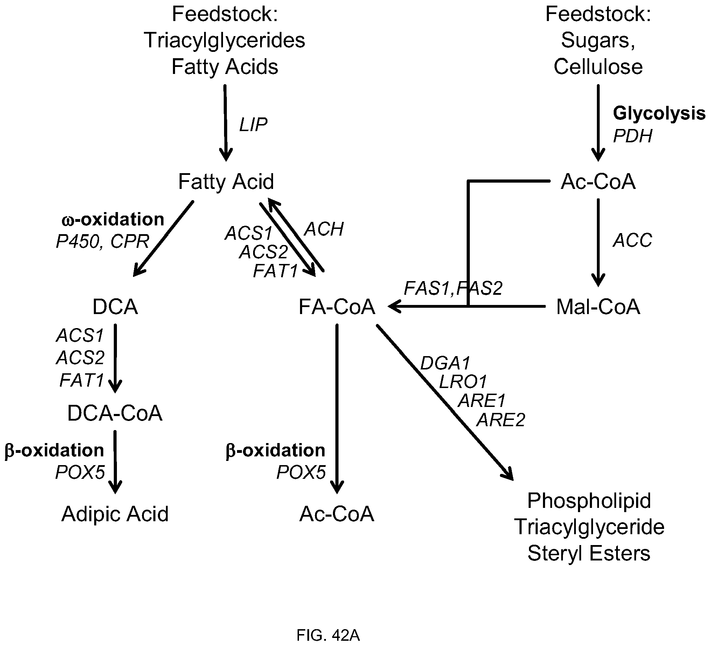

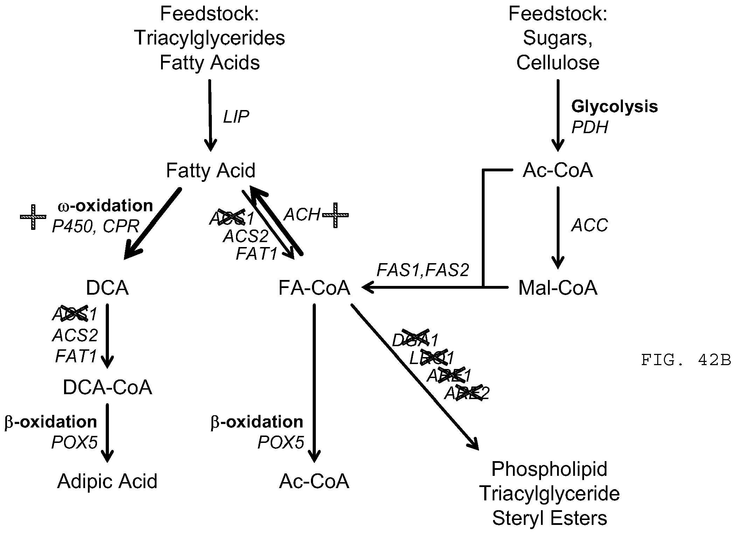

FIG. 42A depicts naturally occurring metabolic pathways which together combine to produce adipic acid, acetyl-CoA, and phospholipids, triacylglycerides and steryl esters from a number of different feedstocks (e.g., triacylglycerides, fatty acids, sugars, cellulose). Production of adipic acid is accompanied by the production of energy and carbon dioxide. Production of acetyl-CoA is accompanied by the production of biomass, energy and carbon dioxide. Production of phospholipids, triacylglycerides and steryl esters is accompanied by the production of biomass and carbon storage moieties (e.g., triacylglycerides and steryl esters). FIG. 42B depicts modifications to the various metabolic pathways, illustrated in FIG. 42A, which alter the host organism's carbon flux towards the production of adipic acid through increased fatty acid production, increased omega oxidation and increased beta oxidation. The altered activities are highlighted by a + for activities that are added or increased and by an X for activities that are reduced or eliminated.

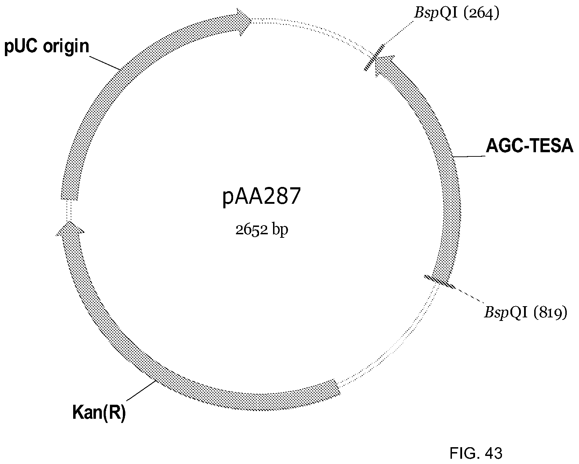

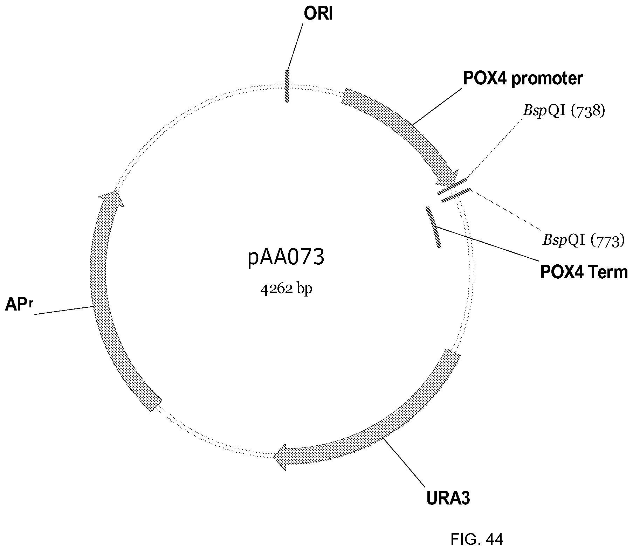

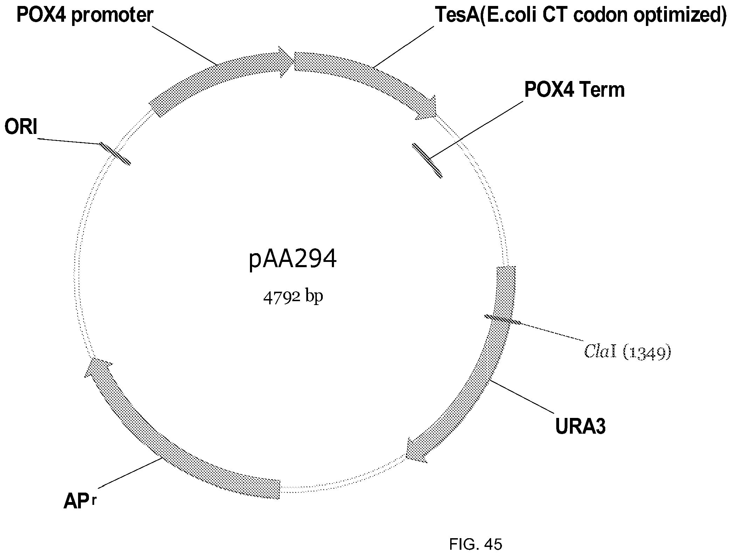

FIG. 43 illustrates a plasmid used for cloning the TESA gene from E. coli, which also was codon optimized for proper functionality in C. tropicalis. The codon optimization included altering CTG codons which are translated differently in C. tropicalis with respect to E. coli, as noted herein. FIG. 44 depicts a plasmid used to donate the POX4 promoter which is used to drive transcription of various genes described herein. For example, the POX4 promoter is the promoter that drives transcription of the TESA gene included in the plasmid shown in FIG. 43. FIG. 45 depicts a plasmid used for integration of the codon optimized TESA gene into C. tropicalis. The plasmid depicted in FIG. 45 is assembled from pieces of the plasmids depicted in FIGS. 43 and 44. See Example 40 for experimental details and results.

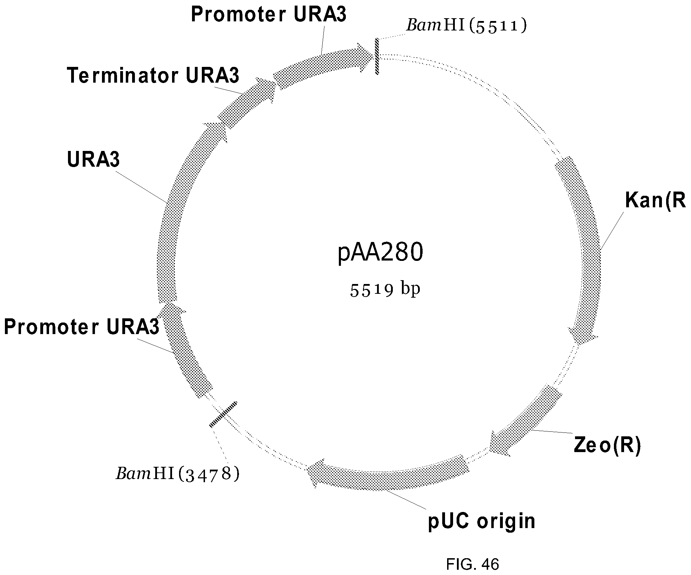

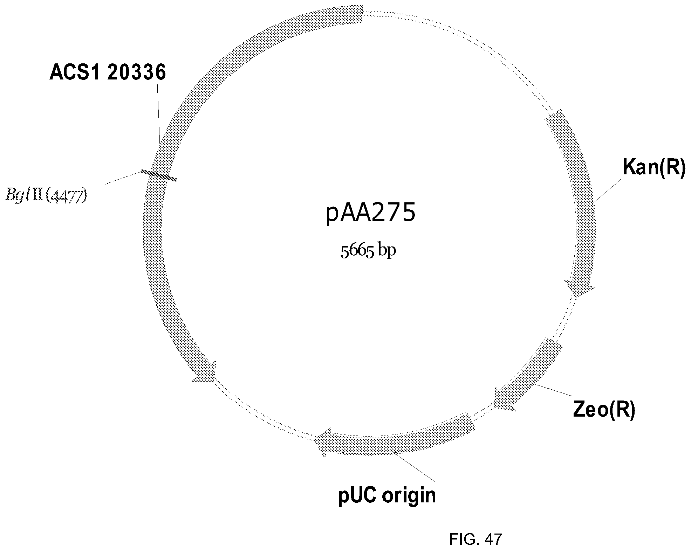

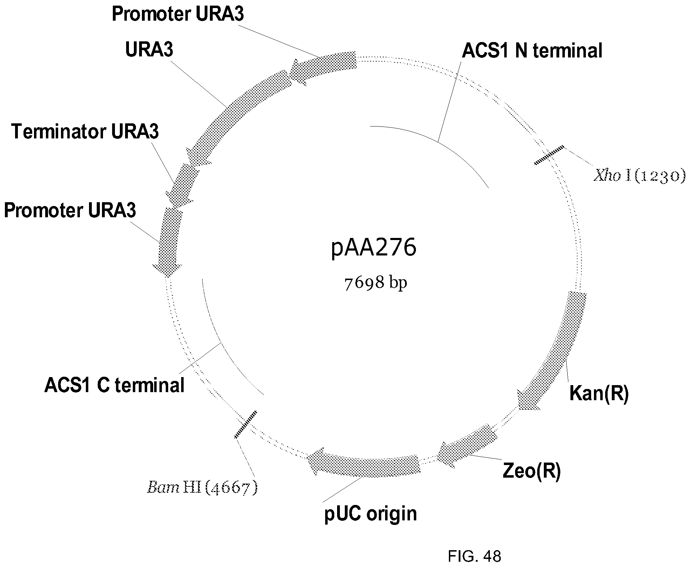

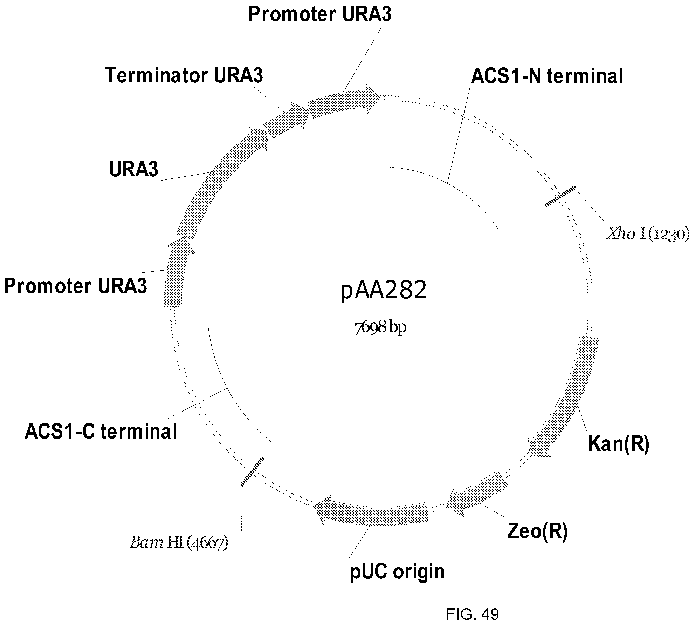

FIG. 46 depicts a plasmid used to donate a "knockout cassette", for disrupting various genes described herein. FIG. 47 depicts a plasmid used for cloning the ACS1 gene from C. tropicalis, for use in generating an ACS1 knockout construct. FIGS. 48 and 49 depict the ACS1 knockout constructs generated from pieces of the plasmids depicted in FIGS. 46 and 47. The difference between the constructs illustrated in FIGS. 48 and 49 is the orientation of the URA3 cassette (e.g., Promoter URA3-URA3-Terminator URA3-Promoter URA3). See Example 43 for experimental details and results.

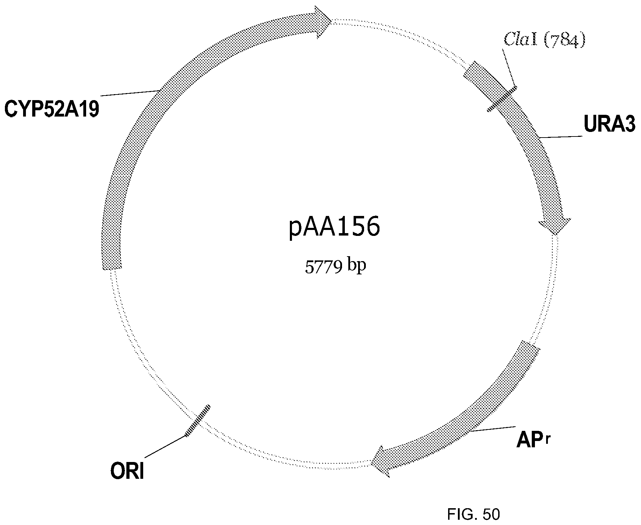

FIG. 50 illustrates the plasmid used to amplify the number of copies of cytochrome P450 A19 (e.g., CYP52A19). See Examples 48 and 49 for experimental details and results.

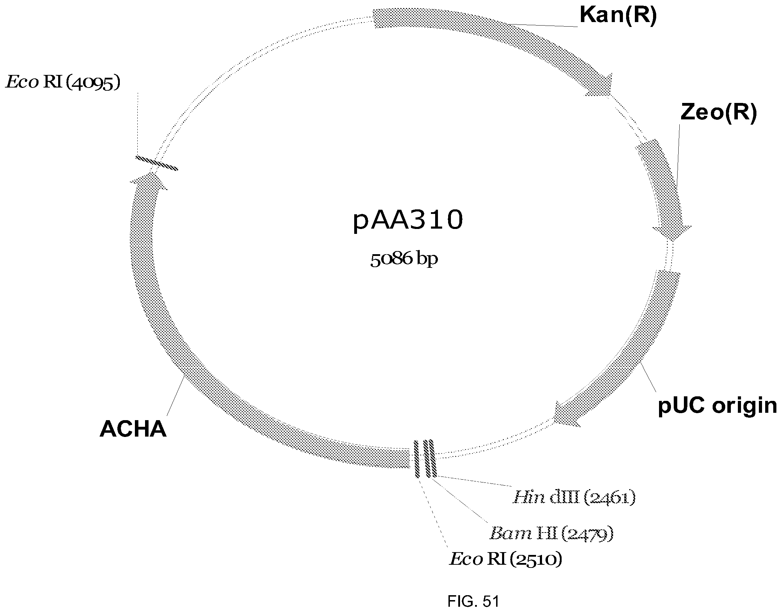

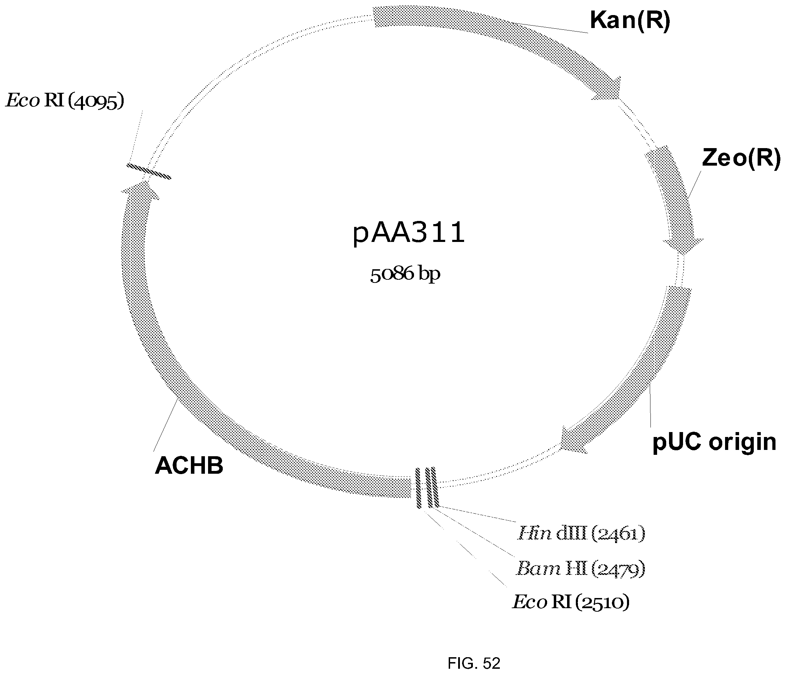

FIG. 51 illustrates a plasmid used for cloning the ACHA allele from C. tropicalis. FIG. 52 illustrates a plasmid used for cloning ACHB from C. tropicalis. See Example 37 for experimental details.

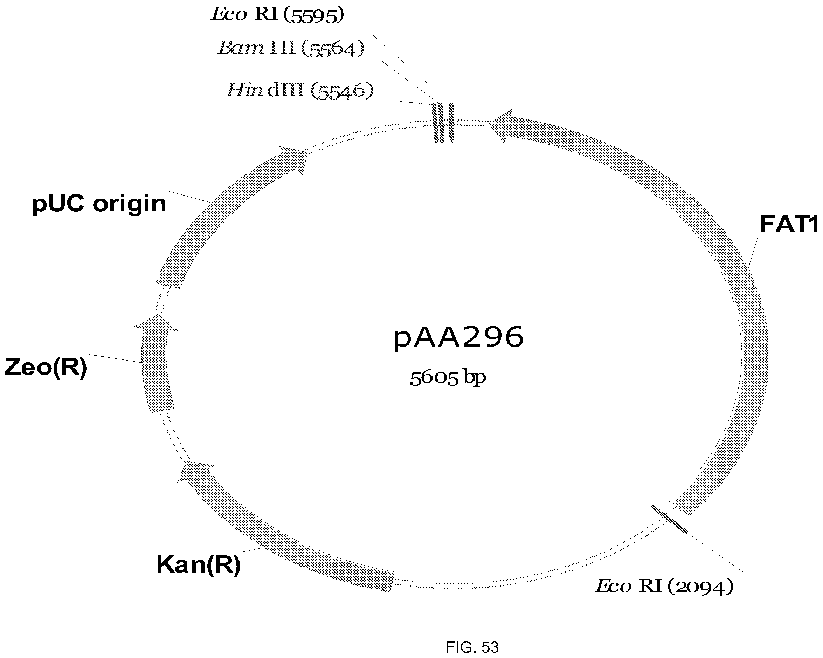

FIG 53. illustrates a plasmid used for cloning the FAT1 gene from C. tropicalis. See Example 38 for experimental details. The cloned FAT1 DNA sequence are used to construct FAT1 "knock out" constructs.

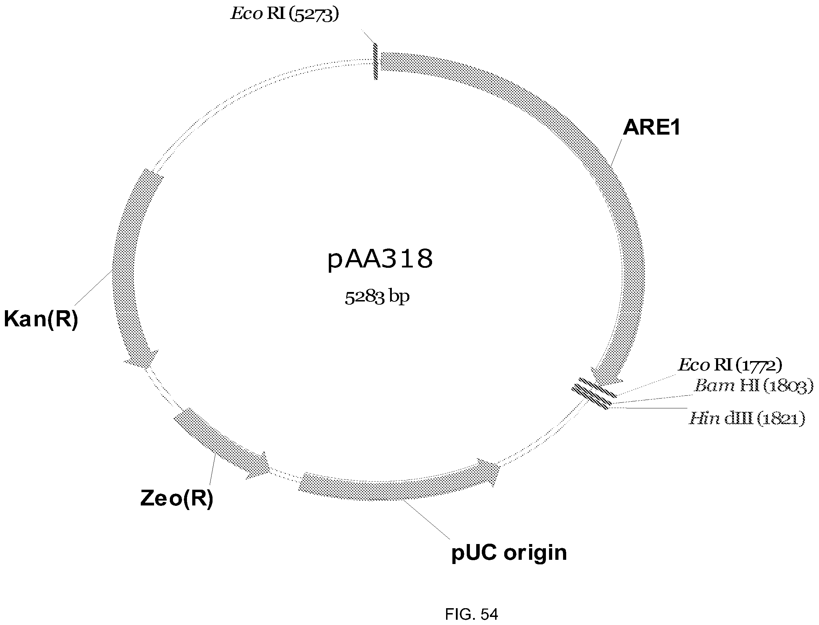

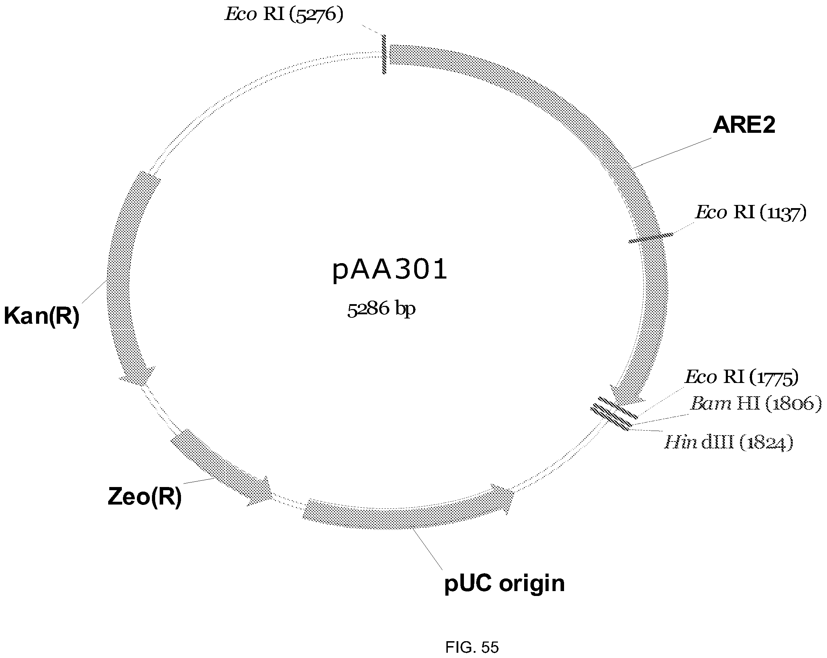

FIG. 54 illustrates a plasmid used for cloning the ARE1 gene from C. tropicalis. FIG. 55 illustrates a plasmid used for cloning the ARE2 gene from C. tropicalis. See Example 39 for experimental details. The cloned ARE1 and ARE2 DNA sequence are used to construct ARE1 and ARE2 "knock out" constructs.

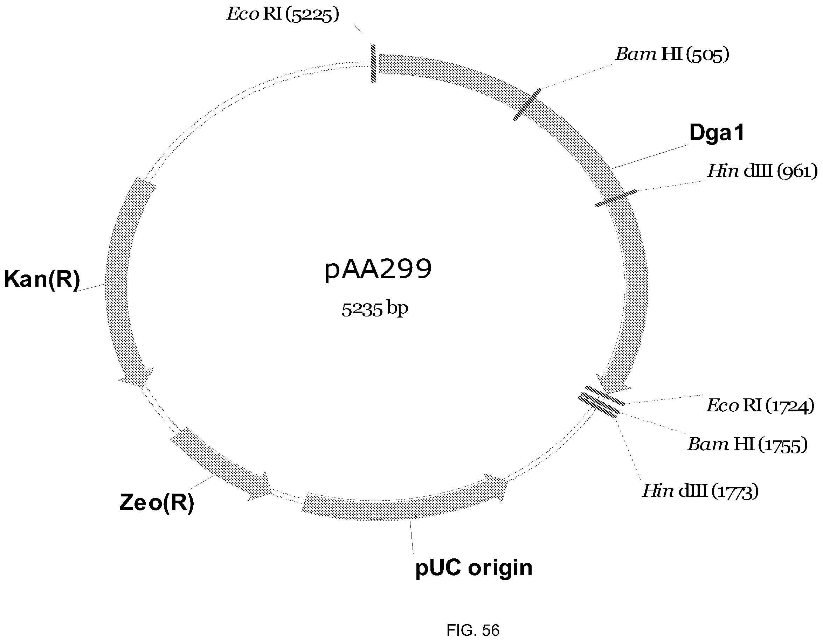

FIG. 56 illustrates a plasmid used for cloning the DGA1 gene from C. tropicalis. See Example 41 for experimental details. The cloned DGA1 DNA sequence are used to construct DGA1 "knock out" constructs.

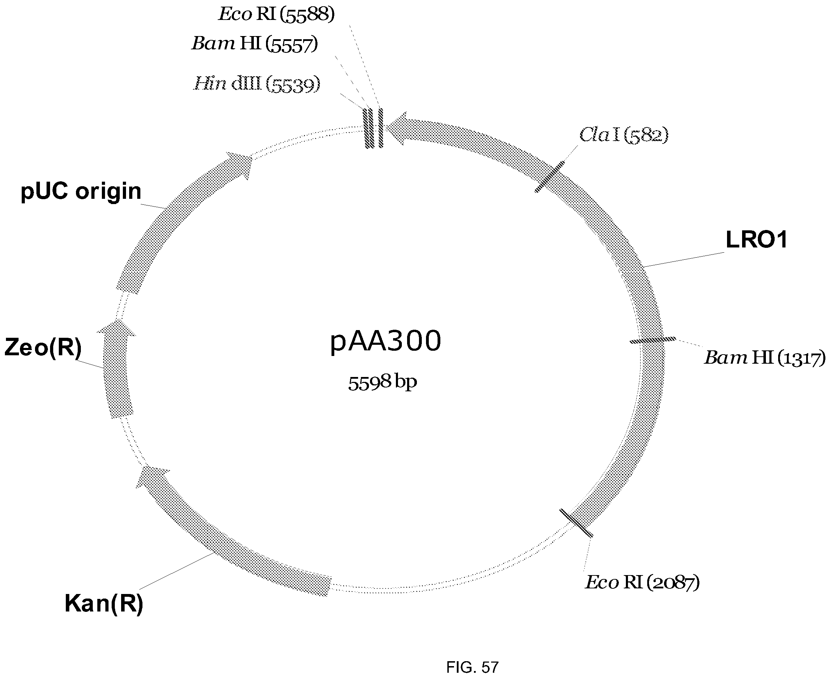

FIG. 57 illustrates a plasmid used for cloning the LRO1 gene from C. tropicalis. See Example 42 for experimental details. The cloned LRO1 DNA sequence are used to construct LRO1 "knock out" constructs.

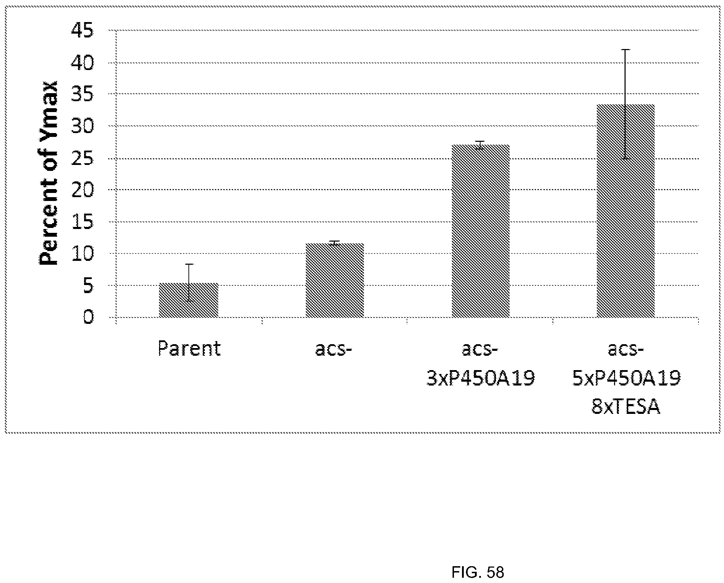

FIG. 58 graphically illustrates the percent of theoretical maximum yield for production of adipic acid from various parental and engineered strains of C. tropicalis. See Example 50 for experimental details and results.

### Detailed Description

Adipic acid is a six-carbon organic molecule that is a chemical intermediate in manufacturing processes used to make certain polyamides, polyurethanes and plasticizers, all of which have wide applications in producing items such as carpets, coatings, adhesives, elastomers, food packaging, and lubricants, for example. Some large-scale processes for making adipic acid include (i) liquid phase oxidation of ketone alcohol oil (KA oil); (ii) air oxidation/hydration of cyclohexane with boric acid to make cyclohexanol, followed by oxidation with nitric acid; and (iii) hydrocyanation of butadiene to a pentenenitrile mixture, followed by hydroisomerization of adiponitrile, followed by hydrogenation. Each of the latter processes requires use of noxious chemicals and/or solvents, some require high temperatures, and all require significant energy input. In addition, some of the processes emit toxic byproducts (such as nitrous oxide) and give rise to environmental concerns.

Provided herein are methods for producing adipic acid and other organic chemical intermediates using biological systems. Such production systems may have significantly less environmental impact and could be economically competitive with current manufacturing systems. Thus, provided herein are methods for manufacturing adipic acid by engineered microorganisms. In some embodiments microorganisms are engineered to contain at least one heterologous gene encoding an enzyme, where the enzyme is a member of a novel pathway engineered into the microorganism. In certain embodiments, an organism may be selected for elevated activity of a native enzyme.

### Microorganisms

A microorganism selected often is suitable for genetic manipulation and often can be cultured at cell densities useful for industrial production of a target product. A microorganism selected often can be maintained in a fermentation device.

The term "engineered microorganism" as used herein refers to a modified microorganism that includes one or more activities distinct from an activity present in a microorganism utilized as a starting point (hereafter a "host microorganism"). An engineered microorganism includes a heterologous polynucleotide in some embodiments, and in certain embodiments, an engineered organism has been subjected to selective conditions that alter an activity, or introduce an activity, relative to the host microorganism. Thus, an engineered microorganism has been altered directly or indirectly by a human being. A host microorganism sometimes is a native microorganism, and at times is a microorganism that has been engineered to a certain point. An engineered microorganism can be derived from any one of the following cell lines: ATCC 20362, ATCC 8862, ATCC 18944, ATCC 20228, ATCC 76982, LGAM S(7)1, ATCC 20336, ATCC20913, SU-2 (ura3-/ura3-), ATCC 20962, ATCC 24690, ATCC 38163, H5343, ATCC 8661, ATCC 8662, ATCC 9773, ATCC 15586, ATCC 16617, ATCC 16618, ATCC 18942, ATCC 18943, ATCC 18944, ATCC 18945, ATCC 20114, ATCC 20177, ATCC 20182, ATCC 20225, ATCC 20226, ATCC 20228, ATCC 20237, ATCC 20255, ATCC 20287, ATCC 20297, ATCC 20306, ATCC 20315, ATCC 20320, ATCC 20324, ATCC 20341, ATCC 20346, ATCC 20348, ATCC 20362, ATCC 20363, ATCC 20364, ATCC 20372, ATCC 20373, ATCC 20383, ATCC 20390, ATCC 20400, ATCC 20460, ATCC 20461, ATCC 20462, ATCC 20496, ATCC 20510, ATCC 20628, ATCC 20688, ATCC 20774, ATCC 20775, ATCC 20776, ATCC 20777, ATCC 20778, ATCC 20779, ATCC 20780, ATCC 20781, ATCC 20794, ATCC 20795, ATCC 20875, ATCC 22421, ATCC 22422, ATCC 22423, ATCC 22969, ATCC 32338, ATCC 32339, ATCC 32340, ATCC 32341, ATCC 32342, ATCC 32343, ATCC 32935, ATCC 34017, ATCC 34018, ATCC 34088, ATCC 34922, ATCC 38295, ATCC 42281, ATCC 44601, ATCC 46025, ATCC 46026, ATCC 46027, ATCC 46028, ATCC 46067, ATCC 46068, ATCC 46069, ATCC 46070, ATCC 46330, ATCC 46482, ATCC 46483, ATCC 46484, ATCC 48436, ATCC 60594, ATCC 62385, ATCC 64042, ATCC 74234, ATCC 76598, ATCC 76861, ATCC 76862, ATCC 90716, ATCC 90806, ATCC 90811, ATCC 90812, ATCC 90813, ATCC 90814, ATCC 90903, ATCC 90904, ATCC 90905, ATCC 96028, ATCC 201089, ATCC 201241, ATCC 201242, ATCC 201243, ATCC 201244, ATCC 201245, ATCC 201246, ATCC 201247, ATCC 201248, ATCC 201249, ATCC 201847, ATCC MYA-165, ATCC MYA-166, ATCC MYA-2613, and ATCC MYA-4467. That is, an engineered microorganism described herein is generated from one or more of the aforementioned ancestral cell lines. An engineered microorganism of the invention described herein is generated from any one of the aforementioned Candida yeast ancestral cell lines.

In some embodiments an engineered microorganism is a single cell organism, often capable of dividing and proliferating. A microorganism can include one or more of the following features: aerobe, anaerobe, filamentous, non-filamentous, monoploid, dipoid, auxotrophic and/or non-auxotrophic. In certain embodiments, an engineered microorganism is a prokaryotic microorganism (e.g., bacterium), and in certain embodiments, an engineered microorganism is a non-prokaryotic microorganism. In some embodiments, an engineered microorganism is a eukaryotic microorganism (e.g., yeast, fungi, amoeba).

Any suitable yeast may be selected as a host microorganism, engineered microorganism or source for a heterologous polynucleotide. Yeast include, but are not limited to, Yarrowia yeast (e.g., Y. lipolytica (formerly classified as Candida lipolytica)), Candida yeast (e.g., C. revkaufi, C. pulcherrima, C. tropicalis, C. utilis, C. viswanithii), Rhodotorula yeast (e.g., R. glutinus, R. graminis), Rhodosporidium yeast (e.g., R. toruloides), Saccharomyces yeast (e.g., S. cerevisiae, S. bayanus, S. pastorianus, S. carlsbergensis), Cryptococcus yeast, Trichosporon yeast (e.g., T. pullans, T. cutaneum), Pichia yeast (e.g., P. pastoris) and Lipomyces yeast (e.g., L. starkeyii, L. lipoferus). In some embodiments, a yeast is a Y. lipolytica strain that includes, but is not limited to, ATCC20362, ATCC8862, ATCC18944, ATCC20228, ATCC76982 and LGAM S(7)1 strains (Papanikolaou S., and Aggelis G., Bioresour. Technol. 82(1):43-9 (2002)). In certain embodiments, a yeast is a C. tropicalis strain, a C. viswanithii strain, a Y. lipolytica strain or a yeast strain that includes, but is not limited to, ATCC20336, ATCC20913, SU-2 (ura3-/ura3-), ATCC20962, H5343 (beta oxidation blocked; US Patent No. 5648247) ATCC 20362, ATCC 8862, ATCC 18944, ATCC 20228, ATCC 76982, LGAM S(7)1, ATCC 8661, ATCC 8662, ATCC 9773, ATCC 15586, ATCC 16617, ATCC 16618, ATCC 18942, ATCC 18943, ATCC 18944, ATCC 18945, ATCC 20114, ATCC 20177, ATCC 20182, ATCC 20225, ATCC 20226, ATCC 20228, ATCC 20237, ATCC 20255, ATCC 20287, ATCC 20297, ATCC 20306, ATCC 20315, ATCC 20320, ATCC 20324, ATCC 20341, ATCC 20346, ATCC 20348, ATCC 20362, ATCC 20363, ATCC 20364, ATCC 20372, ATCC 20373, ATCC 20383, ATCC 20390, ATCC 20400, ATCC 20460, ATCC 20461, ATCC 20462, ATCC 20496, ATCC 20510, ATCC 20628, ATCC 20688, ATCC 20774, ATCC 20775, ATCC 20776, ATCC 20777, ATCC 20778, ATCC 20779, ATCC 20780, ATCC 20781, ATCC 20794, ATCC 20795, ATCC 20875, ATCC 22421, ATCC 22422, ATCC 22423, ATCC 22969, ATCC 32338, ATCC 32339, ATCC 32340, ATCC 32341, ATCC 32342, ATCC 32343, ATCC 32935, ATCC 34017, ATCC 34018, ATCC 34088, ATCC 34922, ATCC 38295, ATCC 42281, ATCC 44601, ATCC 46025, ATCC 46026, ATCC 46027, ATCC 46028, ATCC 46067, ATCC 46068, ATCC 46069, ATCC 46070, ATCC 46330, ATCC 46482, ATCC 46483, ATCC 46484, ATCC 48436, ATCC 60594, ATCC 62385, ATCC 64042, ATCC 74234, ATCC 76598, ATCC 76861, ATCC 76862, ATCC 90716, ATCC 90806, ATCC 90811, ATCC 90812, ATCC 90813, ATCC 90814, ATCC 90903, ATCC 90904, ATCC 90905, ATCC 96028, ATCC 201089, ATCC 201241, ATCC 201242, ATCC 201243, ATCC 201244, ATCC 201245, ATCC 201246, ATCC 201247, ATCC 201248, ATCC 201249, ATCC 201847, ATCC MYA-165, ATCC MYA-166, ATCC MYA-2613, and ATCC MYA-4467.

Any suitable fungus may be selected as a host microorganism, engineered microorganism or source for a heterologous polynucleotide. Non-limiting examples of fungi include, but are not limited to Aspergillus fungi (e.g., A. parasiticus, A. nidulans), Thraustochytrium fungi, Schizochytrium fungi and Rhizopus fungi (e.g., R. arrhizus, R. oryzae, R. nigricans), and the aforementioned yeast strains. In some embodiments, a fungus is one of the aforementioned yeast strains, an A. parasiticus strain that includes, but is not limited to, strain ATCC24690, and in certain embodiments, a fungus is an A. nidulans strain that includes, but is not limited to, strain ATCC38163.

Any suitable prokaryote may be selected as a host microorganism, engineered microorganism or source for a heterologous polynucleotide. A Gram negative or Gram positive bacteria may be selected. Examples of bacteria include, but are not limited to, Bacillus bacteria (e.g., B. subtilis, B. megaterium), Acinetobacter bacteria, Norcardia baceteria, Xanthobacter bacteria, Escherichia bacteria (e.g., E. coli (e.g., strains DH10B, Stbl2, DH5-alpha, DB3, DB3.1), DB4, DB5, JDP682 and ccdA-over (e.g., U.S. Application No. 09/518,188))), Streptomyces bacteria, Erwinia bacteria, Klebsiella bacteria, Serratia bacteria (e.g., S. marcessans), Pseudomonas bacteria (e.g., P. aeruginosa), Salmonella bacteria (e.g., S. typhimurium, S. typhi), Megasphaera bacteria (e.g., Megasphaera elsdenii). Bacteria also include, but are not limited to, photosynthetic bacteria (e.g., green non-sulfur bacteria (e.g., Choroflexus bacteria (e.g., C. aurantiacus), Chloronema bacteria (e.g., C. gigateum)), green sulfur bacteria (e.g., Chlorobium bacteria (e.g., C. limicola), Pelodictyon bacteria (e.g., P. luteolum), purple sulfur bacteria (e.g., Chromatium bacteria (e.g., C. okenii)), and purple non-sulfur bacteria (e.g., Rhodospirillum bacteria (e.g., R. rubrum), Rhodobacter bacteria (e.g., R. sphaeroides, R. capsulatus), and Rhodomicrobium bacteria (e.g., R. vanellii)).

Cells from non-microbial organisms can be utilized as a host microorganism, engineered microorganism or source for a heterologous polynucleotide. Examples of such cells, include, but are not limited to, insect cells (e.g., Drosophila (e.g., D. melanogaster), Spodoptera (e.g., S. frugiperda Sf9 or Sf21 cells) and Trichoplusa (e.g., High-Five cells); nematode cells (e.g., C. elegans cells); avian cells; amphibian cells (e.g., Xenopus laevis cells); reptilian cells; and mammalian cells (e.g., NIH3T3, 293, CHO, COS, VERO, C127, BHK, Per-C6, Bowes melanoma and HeLa cells).

Microorganisms or cells used as host organisms or source for a heterologous polynucleotide are commercially available. Microoganisms and cells described herein, and other suitable microorganisms and cells are available, for example, from Invitrogen Corporation, (Carlsbad, CA), American Type Culture Collection (Manassas, Virginia), and Agricultural Research Culture Collection (NRRL; Peoria, Illinois).

Host microorganisms and engineered microorganisms may be provided in any suitable form. For example, such microorganisms may be provided in liquid culture or solid culture (e.g., agar-based medium), which may be a primary culture or may have been passaged (e.g., diluted and cultured) one or more times. Microorganisms also may be provided in frozen form or dry form (e.g., lyophilized). Microorganisms may be provided at any suitable concentration.

### Carbon Processing Pathways and Activities

Figures 1, 9, 35, 36, 42A and 42B depict embodiments of a biological pathways for making adipic acid, using a sugar as the carbon source starting material. Any suitable sugar can be used as the feedstock for the organism, (e.g., 6-carbon sugars (e.g., glucose, fructose), 5-carbon sugars (e.g., xylose), the like or combinations thereof). The sugars are initially metabolized using naturally occurring and/or engineered pathways to yield malonyl CoA, which is depicted as the molecule entering the omega oxidation pathway shown in Figure 9. Malonyl-CoA sometimes is generated by the activity of an acyl-CoA carboxylase activity (e.g., ACC), as depicted in Figures 35 and 36, and sometimes is formed by the metabolism of sugars or paraffins, yielding Malonyl-CoA as a direct or indirect metabolic product.

An acetyl-CoA carboxylase activity (e.g., EC 6.4.1.2) catalyzes the irreversible carboxylation of acetyl-CoA to produce malonyl-CoA through its two catalytic activities, biotin carboxylase and carboxyltransferase. The reaction can be represented as;

ATP + acetyl-CoA + HCO3- = ADP + phosphate + malonyl-CoA

Production of malonyl-CoA is the committed step in fatty acid biosynthesis. Acetyl-CoA carboxylase activity sometimes is present in a variety of organisms (e.g., prokaryotes, plants, algae) as a large multi-subunit protein, and often located in the endoplasmic reticulum of eukaryotes. Acetyl-CoA carboxylase activity in some plants also can carboxylate propanoyl-CoA and butanoyl-CoA. ACC sometimes is also referred to as "acetyl-CoA:carbon-dioxide ligase (ADP-forming)" and "acetyl coenzyme A carboxylase". The reverse activity (e.g., decarboxylation of malonyl-CoA) is carried out by a separate enzyme, malonyl-CoA decarboxylase. In some embodiments, to further increase carbon flux through a particular reaction or through a metabolic pathway, one or more reverse activities in the pathway can be altered to inhibit the back conversion of a desired product into its starting reactants. In certain embodiments, a malonyl-CoA decarboxylase activity is reduced or eliminated to further increase the carbon flux through an acetyl-CoA carboxylase activity in the direction of malonyl-CoA production.

An ACC activity in yeast may be amplified by over-expression of the ACC gene by any suitable method. Non-limiting examples of methods suitable to amplify or over express ACC include amplifying the number of ACC genes in yeast following transformation with a high-copy number plasmid (e.g., such as one containing a 2u origin of replication), integration of multiple copies of ACC into the yeast genome, over-expression of the ACC gene directed by a strong promoter, the like or combinations thereof. The ACC gene may be native to C. tropicalis or C. viswanithii, for example, or it may be obtained from a heterologous source.

A fatty acid synthase (e.g., FAS) activity catalyzes a series of decarboxylative Claisen condensation reactions from acetyl-CoA and malonyl-CoA. Without being limited by any theory, it is believed that following each round of elongation the beta keto group is reduced to the fully saturated carbon chain by the sequential action of a ketoreductase activity, a dehydratase activty, and an enol reductase activity. In the case of Type I FAS's, the growing fatty acid chain is carried between these active sites while attached covalently to the phosphopantetheine prosthetic group of an acyl carrier protein (ACP), and is released by the action of a thioesterase (TE) upon reaching a carbon chain length of 16 (e.g., palmitic acid). Thus, a fatty acid synthase activity comprises a collection of activities (e.g., an enzymatic system) that perform functions associated with the production of fatty acids. Therefore, the terms "fatty acid synthase activity", "fatty acid synthase", "FAS", and "FAS activity", as used herein refer to a collection of activities, or an enzymatic system, that perform functions associated with the production of fatty acids. In some embodiments, the collection of activities is found in a multifunctional, multi-subunit protein complex (e.g., Type I FAS activity).

Fatty acid synthases typically produce fatty acids with longer chain carbon chain lengths, however fatty acids with carbon chain lengths of 6C or 8C often are found in organisms. In some instances, the shorter fatty acids are the result of metabolic activities (e.g., beta-oxidation) that shorten the carbon chain length to a desired shorter number of carbon units. However, in certain instances, shorter chain fatty acids are produced directly by the activity of a specialized fatty acid synthase activity, hexanoate synthase.

As depicted in Figures 1, 9, and 36, the enzyme hexanoate synthase converts two molecules of malonyl-CoA and one molecule of acetyl-CoA to one molecule of hexanoic acid. As depicted in Figure 36, fatty acid synthase converts malonyl-CoA to a long chain fatty acyl-CoA by repeated condensation with acetyl-CoA. In some embodiments a cytochrome P450 enzyme converts hexanoic acid to 6-hydroxyhexanoic acid, which may be oxidized to 6-oxohexanoic acid via 6-hydroxyhexanoic acid dehydrogenase, or fatty alcohol oxidase. 6-oxohexanoic acid may be converted to adipic acid by 6-oxohexanoic acid dehydrogenase. In certain embodiments, a cytochrome P450 enzyme converts medium-, or long-chain fatty acids to dicarboxylic acids (e.g., diacids), which may be further metabolized by natural or engineered pathways described herein to yield adipic acid.

A fatty acid synthase enzyme (FAS) is coded by fatty acid synthase subunit alpha (FAS2) and fatty acid synthase subunit beta (FAS1) genes. In some embodiments, the FAS enzyme is endogenous to the host microorganism. Fatty acid synthase activity in yeast may be amplified by over-expression of the FAS2 and FAS1 genes by any suitable method. Non-limiting examples of methods suitable to amplify or over express FAS2 and FAS1 genes include amplifying the number of FAS2 and FAS1 genes in yeast following transformation with a high-copy number plasmid (e.g., such as one containing a 2u origin of replication), integration of multiple copies of FAS2 and FAS1 genes into the yeast genome, over-expression of the FAS2 and FAS1 genes directed by a strong promoter, the like or combinations thereof. The FAS2 and FAS1 genes may be native to C. tropicalis or C. viswanithii, for example, or they may be obtained from a heterologous source.

A specialized fatty acid synthase enzyme, hexanoate synthase (HexS) is coded by hexonate synthase subunit alpha (HEXA) and hexanoate synthase subunit beta (HEXB) genes. In some embodiments, the HexS enzyme is endogenous to the host microorganism. In certain embodiments, HEXA and HEXB genes may be isolated from a suitable organism (e.g., Aspergillus parasiticus). In some embodiments, HEXA and HEXB orthologs, such as STCJ and STCK, also may be isolated from suitable organisms (e.g., Aspergillus nidulans).

Hexanoate is omega-hydroxylated by the activity of cytochrome P450 enzymes, thereby generating a six carbon alcohol, in some embodiments. In certain embodiments, a cytochrome P450 activity can be increased by increasing the number of copies of a cytochrome P450 gene (e.g., 2, 3, 4, 5, 6, 7, 8, 9, 10, 15, 20, 25 or more copies of the gene), by increasing the activity of a promoter that regulates transcription of a cytochrome P450 gene, or by increasing the number of copies of a cytochrome P450 gene and increasing the activity of a promoter that regulates transcription of a cytochrome P450 gene, thereby increasing the production of target product (e.g., adipic acid) via increased activity of one or more cytochrome P450 enzymes. In some embodiments, a cytochrome P450 enzyme is endogenous to the host microorganism. In certain embodiments, the cytochrome P450 gene is isolated from Bacillus megaterium and codes for a single subunit, soluble, cytoplasmic enzyme. Soluble or membrane bound cytochrome P450 from certain host organisms is specific for 6-carbon substrates and may be used in some embodiments. Cytochrome P450 is reduced by the activity of cytochrome P450 reductase (CPR), thereby recycling cytochrome P450 to allow further enzymatic activity. In certain embodiments, the CPR enzyme is endogenous to the host microorganism. In some embodiments, host CPR activity can be increased by increasing the number of copies of a CPR gene (e.g., 2, 3, 4, 5, 6, 7, 8, 9, 10, 15, 20, 25 or more copies of the gene), by increasing the activity of a promoter that regulates transcription of a CPR gene, or by increasing the number of copies of a CPR gene and increasing the activity of a promoter that regulates transcription of a CPR gene, thereby increasing the production of target product (e.g., adipic acid) via increased recycling of cytochrome P450. In certain embodiments, the promoter can be a heterologous promoter (e.g., endogenous or exogenous promoter). In some embodiments, the CPR gene is heterologous and exogenous and can be isolated from any suitable organism. Non-limiting examples of organisms from which a CPR gene can be isolated include C. tropicalis, C. viswanithii, S. cerevisiae and Bacillus megaterium.

Oxidation of the alcohol to an aldehyde may be performed by an enzyme in the fatty alcohol oxidase family (e.g., 6-hydroxyhexanoic acid dehydrogenase, omega hydroxyl fatty acid dehydrogenase), or an enzyme in the aldehyde dehydrogenase family (e.g., 6-oxohexanoic acid dehydrogenase, omega oxo fatty acid dehydrogenase). The enzyme 6-oxohexanoic acid dehydrogenase or omega oxo fatty acid dehydrogenase may oxidize the aldehyde to the carboxylic acid adipic acid. In some embodiments, the enzymes 6-hydroxyhexanoic acid dehydrogenase, omega hydroxyl fatty acid dehydrogenase, fatty alcohol oxidase, 6-oxohexanoic acid dehydrogenase, or omega oxo fatty acid dehydrogenase, exist in a host organism. Flux through these two steps may sometimes be augmented by increasing the copy number of the enzymes, or by increasing the activity of the promoter transcribing the genes. In some embodiments alcohol and aldehyde dehydrogenases specific for six carbon substrates may be isolated from another organism, for example Acinetobacter, Candida, Saccharomyces or Pseudomonas and inserted into the host organism.

Figures 10 and 37 depict embodiments of biological pathways for making adipic acid, using fats, oils, dicarboxylic acids, paraffins (e.g., linear, branched, substituted, saturated, unsaturated, the like and combinations thereof), fatty alcohols, fatty acids, or the like, as the carbon source starting material. Any suitable fatty alcohol, fatty acid, paraffin, dicarboxylic acid, fat or oil can be used as the feedstock for the organism, (e.g., hexane, hexanoic acid, oleic acid, coconut oil, the like or combinations thereof). Carbon sources with longer chain lengths (e.g., 8 carbons or greater in length) can be metabolized using naturally occurring and/or engineered pathways to yield molecules that can be further metabolized using the beta oxidation pathway shown in Figure 10 and the lower portion of Figures 11A and 37. In some embodiments, the activities in the pathway shown in Figure 10 and 37 also can be engineered (e.g., as described herein) to enhance metabolism and target product formation.

In certain embodiments, one or more activities in one or more metabolic pathways can be engineered to increase carbon flux through the engineered pathways to produce a desired product (e.g., adipic acid). The engineered activities can be chosen to allow increased production of metabolic intermediates that can be utilized in one or more other engineered pathways to achieve increased production of a desired product with respect to the unmodified host organism. This "carbon flux management" can be optimized for any chosen feedstock, by engineering the appropriate activities in the appropriate pathways. A non-limiting example is given herein using an oil (e.g. coconut oil) based feedstock (see Figure 37). The engineered activities increase the production of adipic acid through the increased activities in a number of pathways (e.g., fatty acid degradation, fatty acid synthesis, gluconeogenesis, pentose phosphate pathway, beta oxidation, omega oxidation). The pathways utilized in the non-limiting examples presented herein were chosen to maximize the production of adipic acid by regenerating or utilizing metabolic byproducts to internally generate additional carbon sources that can be further metabolized to produce adipic acid. The process of "carbon flux management" through engineered pathways produces adipic acid at a level and rate closer to the calculated maximum theoretical yield for any given feedstock, in certain embodiments. The terms "theoretical yield" or "maximum theoretical yield" as used herein refer to the yield of product of a chemical or biological reaction that would be formed if the reaction went to completion. Theoretical yield is based on the stoichiometry of the reaction and ideal conditions in which starting material is completely consumed, undesired side reactions do not occur, the reverse reaction does not occur, and there no losses in the work-up procedure. The overall yield of product depends on the limiting reagent. In the embodiment depicted in Figure 37, carbon flux management is achieved by the production of adipic acid from fatty acids liberated from triacylglcyerides, and synthesis of glucose through gluconeogenesis which is subsequently converted to adipic acid through the engineered pentose phosphate pathway, omega oxidation pathway and beta oxidation pathway, as described herein.

Fats, oils, paraffins and the like frequently contain triacylglycerides that can be converted into one or more products useful for producing adipic acid utilizing engineered microorganisms described herein. Triacylglycerides can be converted into glycerol and fatty acids by a lipase activity, as shown in Figure 37. Lipases catalyze the hydrolysis of ester chemical bonds in water-insoluble lipid (e.g., fats, oils, paraffins) substrates. The generalized reaction can be represented by;

triacylglycerol + H₍₂₎O <=> diacylglycerol + a carboxylate

Lipase activity often is associated with activiation of other activities or pathways, by its involvement with protein lipoylation. Certain lipases are involved in the modification of mitochondrial enzymes. Increasing lipase activity in an engineered microorganism can enhance the utilization of fats, oils, paraffins and the like as feedstocks for production of adipic acid by increasing overall carbon flux through the native and engineered pathways of a host microorganism.

Fatty acids cleaved from a glycerol backbone can be further metabolized directly, or indirectly via utilization in synthesis pathways that yield products that subsequently can be metabolized, by native and/or engineered (i) omega oxidation pathways, (ii) beta oxidation pathways, (iii) fatty acid synthase pathways, (iv) hexanoate synthase pathways, or (v) combinations thereof, described herein and shown in Figure 37. In some embodiments, the pathway by which a fatty acid is further directly or indirectly metabolized into adipic acid, is determined by fatty acid chain length.
The glycerol backbone also can be further metabolized (e.g., directly and/or indirectly) to yield adipic acid, by entry into the gluconeogenesis pathway to yield glucose. To further increase production of adipic acid, metabolism of the increased carbon flux through gluconeogenesis can be enhanced by increasing glucose-6-phosphate dehydrogenase activity. Glucose-6-phosphate dehydrogenase (EC 1.1.1.49) catalyzes the first step of the pentose phosphate pathway, and is encoded by the C. tropicalis gene, ZWF. The reaction for the first step in the PPP pathway is; D-glucose 6-phosphate + NADP+ = D-glucono-1,5-lactone 6-phosphate + NADPH + H+

This reaction is irreversible and rate-limiting for efficient fermentation of sugar via the Entner-Doudoroff pathway. The enzyme regenerates NADPH from NADP+ and is important both for maintaining cytosolic levels of NADPH and protecting yeast against oxidative stress. Glucoses-6-phosphate expression in yeast is constitutive, and the activity is inhibited by NADPH such that processes that decrease the cytosolic levels of NADPH stimulate the oxidative branch of the pentose phosphate pathway. Amplification of glucose-6-phosphate dehydrogenase activity in yeast may be desirable to increase the proportion of glucose-6-phosphate converted to 6-phosphoglucono-lactone and thereby improve conversion of sugar (e.g., glucose) to adipic acid via metabolism into products that can be further metabolized by native and/or engineered (i) omega oxidation pathways, (ii) beta oxidation pathways, (iii) fatty acid synthase pathways, (iv) hexanoate synthase pathways, or (v) combinations thereof, described herein, and shown in Figure 37.

Glucose-6-phosphate dehydrogenase (EC 1.1.1.49) activity in yeast may be amplified by over-expression of the ZWF gene by any suitable method. Non-limiting examples of methods suitable to amplify or over express ZWF include amplifying the number of ZWF genes in yeast following transformation with a high-copy number plasmid (e.g., such as one containing a 2u origin of replication), integration of multiple copies of ZWF into the yeast genome, over-expression of the ZWF gene directed by a strong promoter, the like or combinations thereof. The ZWF gene may be native to C. tropicalis or C. viswanithii, for example, or it may be obtained from a heterologous source.

Depicted in the first step of the reaction in Figure 10, the enzyme acyl-CoA ligase converts a long chain fatty alcohol, fatty acid or dicarboxylic acid and 1 molecule of acetyl-CoA into an acyl-CoA derivative of the long chain fatty alcohol, fatty acid or dicarboxylic acid with the conversion of ATP to AMP and inorganic phosphate. The term "beta oxidation pathway" as used herein, refers to a series of enzymatic activities utilized to metabolize fatty alcohols, fatty acids, or dicarboxylic acids. The activities utilized to metabolize fatty alcohols, fatty acids, or dicarboxylic acids include, but are not limited to, acyl-CoA ligase activity, acyl-CoA oxidase activity, acyl-CoA hydrolase, acyl-CoA thioesterase enoyl-CoA hydratase activity, 3-hydroxyacyl-CoA dehydrogenase activity and acetyl-CoA C-acyltransferase activity. The term "beta oxidation activity" refers to any of the activities in the beta oxidation pathway utilized to metabolize fatty alcohols, fatty acids or dicarboxylic acids.

The term "omega oxidation activity" refers to any of the activities in the omega oxidation pathway utilized to metabolize fatty alcohols, fatty acids, dicarboxylic acids, or sugars.

In certain embodiments, an acyl-CoA ligase enzyme converts a long chain fatty alcohol, fatty acid or dicarboxylic acid into the acyl-CoA derivative, which may be oxidized to yield a trans-2,3-dehydroacyl-CoA derivative, by the activity of Acyl CoA oxidase (e.g., also known as acyl-CoA oxidoreductase and fatty acyl-coenzyme A oxidase). The trans-2,3-dehydroacyl-CoA derivative long chain fatty alcohol, fatty acid or dicarboxylic acid may be further converted to 3-hydroxyacyl-CoA by the activity of enoyl-CoA hydratase. 3-hydroxyacyl-CoA can be converted to 3-oxoacyl-CoA by the activity of 3-hydroxyacyl-CoA dehydrogenase. 3-oxoacyl-CoA may be converted to an acyl-CoA molecule, shortened by 2 carbons and an acetyl-CoA, by the activity of Acetyl-CoA C-acyltransferase (e.g., also known as beta-ketothiolase and β-ketothiolase). In some embodiments, acyl-CoA molecules may be repeatedly shortened by beta oxidation until a desired carbon chain length is generated (e.g., 6 carbons, adipic acid). The shortened fatty acid can be further processed using omega oxidation to yield adipic acid.

An acyl-CoA ligase enzyme sometimes is encoded by the host organism and can be added to generate an engineered organism. In some embodiments, host acyl-CoA ligase activity can be increased by increasing the number of copies of an acyl-CoA ligase gene, by increasing the activity of a promoter that regulates transcription of an acyl-CoA ligase gene, or by increasing the number copies of the gene and by increasing the activity of a promoter that regulates transcription of the gene, thereby increasing production of target product (e.g., adipic acid) due to increased carbon flux through the pathway. In certain embodiments, the acyl-CoA ligase gene can be isolated from any suitable organism. Non-limiting examples of organisms that include, or can be used as donors for, acyl-CoA ligase enzymes include Candida, Saccharomyces, or Yarrowia.

An enoyl-CoA hydratase enzyme catalyzes the addition of a hydroxyl group and a proton to the unsaturated β-carbon on a fatty-acyl CoA and sometimes is encoded by the host organism and sometimes can be added to generate an engineered organism. In certain embodiments, the enoyl-CoA hydratase activity is unchanged in a host or engineered organism. In some embodiments, the host enoyl-CoA hydratase activity can be increased by increasing the number of copies of an enoyl-CoA hydratase gene, by increasing the activity of a promoter that regulates transcription of an enoyl-CoA hydratase gene, or by increasing the number copies of the gene and by increasing the activity of a promoter that regulates transcription of the gene, thereby increasing the production of target product (e.g., adipic acid) due to increased carbon flux through the pathway. In certain embodiments, the enoyl-CoA hydratase gene can be isolated from any suitable organism. Non-limiting examples of organisms that include, or can be used as donors for, enoyl-CoA hydratase enzymes include Candida, Saccharomyces, or Yarrowia.

3-hydroxyacyl-CoA dehydrogenase enzyme catalyzes the formation of a 3-ketoacyl-CoA by removal of a hydrogen from the newly formed hydroxyl group created by the activity of enoyl-CoA hydratase. In some embodiments, the activity is encoded by the host organism and sometimes can be added or increased to generate an engineered organism. In certain embodiments, the 3-hydroxyacyl-CoA activity is unchanged in a host or engineered organism. In some embodiments, the host 3-hydroxyacyl-CoA dehydrogenase activity can be increased by increasing the number of copies of a 3-hydroxyacyl-CoA dehydrogenase gene, by increasing the activity of a promoter that regulates transcription of a 3-hydroxyacyl-CoA dehydrogenase gene, or by increasing the number copies of the gene and by increasing the activity of a promoter that regulates transcription of the gene, thereby increasing production of target product (e.g., adipic acid) due to increased carbon flux through the pathway. In certain embodiments, the 3-hydroxyacyl-CoA dehydrogenase gene can be isolated from any suitable organism. Non-limiting examples of organisms that include, or can be used as donors for, 3-hydroxyacyl-CoA dehydrogenase enzymes include Candida, Saccharomyces, or Yarrowia.

An Acetyl-CoA C-acyltransferase (e.g., β-ketothioiase) enzyme catalyzes the formation of a fatty acyl-CoA shortened by 2 carbons by cleavage of the 3-ketoacyl-CoA by the thiol group of another molecule of CoA. The thiol is inserted between C-2 and C-3, which yields an acetyl CoA molecule and an acyl CoA molecule that is two carbons shorter. An Acetyl-CoA C-acyltransferase sometimes is encoded by the host organism and sometimes can be added to generate an engineered organism. In certain embodiments, the acetyl-CoA C-acyltransferase activity is unchanged in a host or engineered organism. In some embodiments, the host acetyl-CoA C-acyltransferase activity can be increased by increasing the number of copies of an acetyl-CoA C-acyltransferase gene, or by increasing the activity of a promoter that regulates transcription of an acetyl-CoA C-acyltransferase gene, thereby increasing the production of target product (e.g., adipic acid) due to increased carbon flux through the pathway. In certain embodiments, the acetyl-CoA C-acyltransferase gene can be isolated from any suitable organism. Non-limiting examples of organisms that include, or can be used as donors for, acetyl-CoA C-acyltransferase enzymes include Candida, Saccharomyces, or Yarrowia.

FIGS. 42A and 42B illustrate pathways that can be manipulated to produce adipic acid from sugars, cellulose, triacylglycerides and fatty acids. Illustrated in FIG. 42A are various activities normally active in a host organism, whereas FIG. 42B illustrates activities, that when manipulated, direct the flow of carbon in the host organism towards the production of adipic acid through increased fatty acid production and increased omega and beta oxidation activities. As shown in FIG. 42B, activities that are increased or added are shown with a "+" and activities that are reduced or eliminated are shown with a "X". In addition to directing the flow of carbon towards the production of adipic acid through increased fatty acid production and increased omega and beta oxidation activities, the altered activities in the pathways illustrated in FIGS. 42A and 42B direct the flow of carbon away from the production of biomass and carbon storage molecules (e.g., starch, lipids, triacylglycerides, the like, combinations thereof) and away from the utilization of fatty acids for energy. Increased activities shown in FIGS. 42A and 42B (e.g., monooxygenase (e.g., P450), monooxygenase reductase (e.g., CPR), and thioesterase (e.g., ACH and TESA)) are described herein.

A microorganism may be modified and engineered to include or regulate one or more activities in an adipic acid pathway. The term "activity" as used herein refers to the functioning of a microorganism's natural or engineered biological pathways to yield various products including adipic acid and its precursors. Adipic acid producing activity can be provided by any non-mammalian source in certain embodiments. Such sources include, without limitation, eukaryotes such as yeast and fungi and prokaryotes such as bacteria. In some embodiments, a reverse activity in a pathway described herein can be altered (e.g., disrupted, reduced) to increase carbon flux through a beta oxidation pathway, an omega oxidation pathway, or a beta oxidation and omega oxidation pathway, towards the production of target product (e.g., adipic acid). In some embodiments, a genetic modification disrupts an activity in the beta oxidation pathway, or disrupts a polynucleotide that encodes a polypeptide that carries out a forward reaction in the beta oxidation pathway, which renders beta oxidation activity undetectable. The term "undetectable" as used herein refers to an amount of an analyte that is below the limits of detection, using detection methods or assays known (e.g., described herein). In certain embodiments, the genetic modification partially reduces beta oxidation activity. The term "partially reduces beta oxidation activity" as used here refers to a level of activity in an engineered organism that is lower than the level of activity found in the host or starting organism.

An activity within an engineered microorganism provided herein can include one or more (e.g., 1, 2, 3, 4, 5, 6, 7, 8, 9, 10, 11, 12, 13, 14 or all) of the following activities: 6-oxohexanoic acid dehydrogenase activity; 6-hydroxyhexanoic acid dehydrogenase activity; hexanoate synthase activity; cytochrome P450 activity; cytochrome P450 reductase activity; fatty alcohol oxidase activity; acyl-CoA ligase activity, acyl-CoA oxidase activity; enoyl-CoA hydratase activity, 3-hydroxyacyl-CoA dehydrogenase activity, fatty acid synthase activity, lipase activity, acyl-CoA carboxylase activity, glucose-6-phosphate dehydrogenase, and thioesterase activity (e.g., acyl-CoA hydrolase, acyl-CoA thioesterase, acetyl-CoA C-acyltransferase, beta-ketothiolase). In certain embodiments, one or more (e.g., 1, 2, 3, 4, 5, 6, 7, 8, 9, 10 or all) of the foregoing activities is altered by way of a genetic modification. In some embodiments, one or more (e.g., 1, 2, 3, 4, 5, 6, 7, 8, 9, 10, 11, 12, 13, 14 or all) of the foregoing activities is altered by way of (i) adding a heterologous polynucleotide that encodes a polypeptide having the activity, and/or (ii) altering or adding a regulatory sequence that regulates the expression of a polypeptide having the activity.

The term "6-oxohexanoic acid dehydrogenase activity" as used herein refers to conversion of 6-oxohexanoic acid to adipic acid. The 6-oxohexanoic acid dehydrogenase activity can be provided by a polypeptide. In some embodiments, the polypeptide is encoded by a heterologous nucleotide sequence introduced to a host microorganism. In certain embodiments, an endogenous polypeptide having the 6-oxohexanoic acid dehydrogenase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Nucleic acid sequences conferring 6-oxohexanoic acid dehydrogenase activity can be obtained from a number of sources, including Actinobacter, Norcardia, Pseudomonas and Xanthobacter bacteria. Examples of an amino acid sequence of a polypeptide having 6-oxohexanoic acid dehydrogenase activity, and a nucleotide sequence of a polynucleotide that encodes the polypeptide, are presented herein. Presence, absence or amount of 6-oxohexanoic acid dehydrogenase activity can be detected by any suitable method known in the art. For an example of a detection method for alcohol oxidase or alcohol dehydrogenase activity (see Appl. Environ. Microbiol. 70: 4872). In some embodiments, 6-oxohexanoic acid dehydrogenase activity is not altered in a host microorganism, and in certain embodiments, the activity is added or increased in the engineered microorganism relative to the host microorganism.

The term "omega oxo fatty acid dehydrogenase activity" as used herein refers to conversion of an omega oxo fatty acid to a dicarboxylic acid. The omega oxo fatty acid dehydrogenase activity can be provided by a polypeptide. In some embodiments, the polypeptide is encoded by a heterologous nucleotide sequence introduced to a host microorganism. In certain embodiments, an endogenous polypeptide having the omega oxo fatty acid dehydrogenase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Nucleic acid sequences conferring omega oxo fatty acid dehydrogenase activity can be obtained from a number of sources, including Actinobacter, Norcardia, Pseudomonas and Xanthobacter bacteria. Examples of an amino acid sequence of a polypeptide having omega oxo fatty acid dehydrogenase activity and a nucleotide sequence of a polynucleotide that encodes the polypeptide, are presented herein. Presence, absence or amount of omega oxo fatty acid dehydrogenase activity can be detected by any suitable method known in the art. In some embodiments, omega oxo fatty acid dehydrogenase activity is not altered in a host microorganism, and in certain embodiments, the activity is added or increased in the engineered microorganism relative to the host microorganism.

The term "6-hydroxyhexanoic acid dehydrogenase activity" as used herein refers to conversion of 6-hydroxyhexanoic acid to 6-oxohexanoic acid. The 6-hydroxyhexanoic acid dehydrogenase activity can be provided by a polypeptide. In some embodiments, the polypeptide is encoded by a heterologous nucleotide sequence introduced to a host microorganism. In certain embodiments, an endogenous polypeptide having the 6-hydroxyhexanoic acid dehydrogenase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Nucleic acid sequences conferring 6-hydroxohexanoic acid dehydrogenase activity can be obtained from a number of sources, including Actinobacter, Norcardia, Pseudomonas, and Xanthobacter. Examples of an amino acid sequence of a polypeptide having 6-hydroxyhexanoic acid dehydrogenase activity, and a nucleotide sequence of a polynucleotide that encodes the polypeptide, are presented herein. Presence, absence or amount of 6-hydroxyhexanoic acid dehydrogenase activity can be detected by any suitable method known in the art. An example of such a method is described in Methods in Enzymology, 188: 176. In some embodiments, 6-hydroxyhexanoic acid dehydrogenase activity is not altered in a host microorganism, and in certain embodiments, the activity is added or increased in the engineered microorganism relative to the host microorganism.

The term "omega hydroxyl fatty acid dehydrogenase activity" as used herein refers to conversion of an omega hydroxyl fatty acid to an omega oxo fatty acid. The omega hydroxyl fatty acid dehydrogenase activity can be provided by a polypeptide. In some embodiments, the polypeptide is encoded by a heterologous nucleotide sequence introduced to a host microorganism. In certain embodiments, an endogenous polypeptide having the omega hydroxyl fatty acid dehydrogenase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Nucleic acid sequences conferring omega hydroxyl fatty acid dehydrogenase activity can be obtained from a number of sources, including Actinobacter, Norcardia, Pseudomonas and Xanthobacter bacteria. Examples of an amino acid sequence of a polypeptide having omega hydroxyl fatty acid dehydrogenase activity and a nucleotide sequence of a polynucleotide that encodes the polypeptide are presented herein. Presence, absence or amount of omega hydroxyl fatty acid dehydrogenase activity can be detected by any suitable method known in the art. In some embodiments, omega hydroxyl fatty acid dehydrogenase activity is not altered in a host microorganism, and in certain embodiments, the activity is added or increased in the engineered microorganism relative to the host microorganism.

The term "lipase activity" as used herein refers to the hydrolysis of triacylglycerol to produce a diacylglycerol and a fatty acid anion. The lipase activity can be provided by a polypeptide. In certain embodiments, an endogenous polypeptide having the lipase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Examples of a nucleotide sequence of a polynucleotide that encodes a polypeptide having lipase activity, and amino acid sequences that code a lipase activity are presented herein. In some embodiments, lipase activity is not altered in a host microorganism, and in certain embodiments, the activity is added or increased in the engineered microorganism relative to the host microorganism. Presence, absence or amount of lipase activity can be detected by any suitable method known in the art, including western blot analysis.

The term "glucose-6-phosphate dehydrogenase activity" as used herein refers to the conversion of D-glucose 6-phosphate into D-glucono-1,5-lactone 6-phosphate. Glucose-6-phosphate dehydrogenase is an activity that forms part of the pentose phosphate pathway. Glycerol backbones liberated from fatty acids by a lipase activity can ultimately be converted to glucose by the action of the gluconeogenesis pathway, where glycerol is first converted to dihydroxyacetone phosphate. Glucose can be preferentially metabolized by the pentose phosphate pathway by increasing one or more activities in the pentose phosphate pathway (e.g., glucose-6-phosphate dehydrogenase). In addition to increasing the conversion of D-glucose 6-phosphate into D-glucono-1,5-lactone 6-phosphate, increasing the level of glucose-6-phosphate dehydrogenase activity also may yield advantageous benefits due to the additional reducing power generated by the increased activity of glucose-6-phosphate dehydrogenase. In some embodiments, increasing the activity of glucose-6-phosphate dehydrogenase increases the activity of a gluconeogenesis pathway, a pentose phosphate pathway or a gluconeogenesis pathway and a pentose phosphate pathway due to a forward biased increased carbon flux through the pathways.

A glucose-6-phosphate dehydrogenase (G6PD) activity may be provided by an enzyme. The glucose-6-phosphate dehydrogenase activity can be provided by a polypeptide. In certain embodiments, an endogenous polypeptide having the glucose-6-phosphate dehydrogenase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Examples of a nucleotide sequence of a polynucleotide that encodes a polypeptide having glucose-6-phosphate dehydrogenase activity, is presented herein. In some embodiments, glucose-6-phosphate dehydrogenase activity is not altered in a host microorganism, and in certain embodiments, the activity is added or increased in the engineered microorganism relative to the host microorganism. Presence, absence or amount of glucose-6-phosphate dehydrogenase activity can be detected by any suitable method known in the art including western blot analysis

The term "acetyl-CoA carboxylase activity" as used herein refers to the irreversible carboxylation of acetyl-CoA to produce malonyl-CoA. Acetyl-CoA carboxylase activity may be provided by an enzyme that includes one or two subunits, depending on the source organism. The acetyl-CoA carboxylase synthase activity can be provided by a polypeptide. In certain embodiments, an endogenous polypeptide having the acetyl-CoA carboxylase activity is identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). An example of a nucleotide sequence of a polynucleotide that encodes a polypeptide having acetyl-CoA carboxylase activity, is presented herein. In some embodiments, acetyl-CoA carboxylase activity is not altered in a host microorganism, and in certain embodiments, the activity is added or increased in the engineered microorganism relative to the host microorganism. Presence, absence or amount of acetyl-CoA carboxylase activity can be detected by any suitable method known in the art including western blot analysis

The term "fatty acid synthase activity" as used herein refers to conversion of acetyl-CoA and malonyl-CoA to fatty acids. Fatty acid synthase activity may be provided by an enzyme that includes one or two subunits (referred to hereafter as "subunit alpha" and/or "subunit beta"). The fatty acid synthase activity can be provided by a polypeptide. In certain embodiments, endogenous polypeptides having the fatty acid synthase activity are identified in the host microorganism, and the host microorganism is genetically altered to increase the amount of the polypeptide produced (e.g., a heterologous promoter is introduced in operable linkage with a polynucleotide that encodes the polypeptide; the copy number of a polynucleotide that encodes the polypeptide is increased (e.g., by introducing a plasmid that includes the polynucleotide)). Examples of a nucleotide sequence of a polynucleotide that encodes a polypeptide having fatty acid synthase activity, is presented herein. In some embodiments, fatty acid synthase activity is not altered in a host microorganism, and in certain embodiments, the activity is added or increased in the engineered microorganism relative to the host microorganism. Presence, absence or amount of fatty acid synthase activity can be detected by any suitable method known in the art, including western blot analysis.

The term "hexanoate synthase activity" as used herein refers to conversion of acetyl-CoA and malonyl-CoA to hexanoic acid. Hexanoate synthase activity may be provided by an enzyme that includes one or two subunits (referred to hereafter as "subunit A" and/or "subunit B"). The hexanoate synthase activity can be provided by a polypeptide. In some embodiments, the polypeptide is encoded by a heterologous nucleotide sequence introduced to a host microorganism. Nucleic acid sequences conferring hexanoate synthase activity can be obtained from a number of sources, including Aspergillus parasiticus, for example. Examples of an amino acid sequence of a polypeptide having hexanoate synthase activity, and a nucleotide sequence of a polynucleotide that encodes the polypeptide, are presented herein. In some embodiments, hexanoate synthase activity is not altered in a host microorganism, and in certain embodiments, the activity is added or increased in the engineered microorganism relative to the host microorganism.

Presence, absence or amount of hexanoate synthase activity can be detected by any suitable method known in the art. An example of such a method is described in Hexanoate synthase + thioesterase (Chemistry and Biology 9: 981-988). Briefly, an indicator strain may be prepared. An indicator strain may be Bacillus subtilis containing a reporter gene (beta-galactosidase, green fluorescent protein, etc.) under control of the promoter regulated by LiaR, for example. An indicator strain also may be Candida tropicalis or Candida viswanithii containing either the LiaR regulatable promoter from Bacillus subtilis or the alkane inducible promoter for the native gene for the peroxisomal 3-ketoacyl coenzyme A thiolase gene (CT-T3A), for example. Mutants with an improved functionality of HexS, thereby producing more hexanoic acid, can be plated onto a lawn of indicator strain. Upon incubation and growth of both the test mutant and the indicator strain, the appearance of a larger halo, which correlates to the induction of the reporter strain compared to control strains, indicates a mutant with improved activity. In alternative approach, mutants are grown in conditions favoring production of hexanoyl CoA or hexanoic acid and lysed. Cell lysates are treated with proteases which may release hexanoic acid from the PKS. Clarified lysates may be spotted onto lawns of indicator strains to assess improved production. In another alternative approach, indicator strains are grown under conditions suitable to support expression of the reporter gene when induced by hexanoic acid. Dilutions of a known concentration of hexanoic acid are used to determine a standard curve. Lysates of the test strain grown under conditions favoring production of hexanoic acid are prepared and dilutions of the lysate added to the indicator strain. Indicator strains with lysates are placed under identical conditions as used to determine the standard curve. The lysate dilutions that minimally support induction can be used to determine, quantitatively, the amount produced when compared to the standard curve.

The term "monooxygenase activity" as used herein refers to inserting one atom of oxygen from O₂ into an organic substrate (RH) and reducing the other oxygen atom to water. In some embodiments, monooxygenase activity refers to incorporation of an oxygen atom onto a six-carbon organic substrate. In certain embodiments, monooxygenase activity refers to conversion of hexanoate to 6-hydroxyhexanoic acid. Monooxygenase activity can be provided by any suitable polypeptide, such as a cytochrome P450 polypeptide (hereafter "CYP450") in certain embodiments. Nucleic acid sequences conferring CYP450 activity can be obtained from a number of sources, including Bacillus megaterium and may be induced in organisms including but not limited to Candida tropicalis, Candida viswanithii, Yarrowia lipolytica, Aspergillus nidulans, and Aspergillus parasiticus. Examples of oligonucleotide sequences utilized to isolate a polynucleotide sequence encoding a polypeptide having CYP450 activity (e.g., CYP52A12 polynucleotide, a CYP52A13 polynucleotide, a CYP52A14 polynucleotide, a CYP52A15 polynucleotide, a CYP52A16 polynucleotide, a CYP52A17 polynucleotide, a CYP52A18 polynucleotide, a CYP52A19 polynucleotide, a CYP52A20 polynucleotide, a CYP52D2 polynucleotide, and/or a BM3 polynucleotide) are presented herein. In some embodiments, monooxygenase activity is not altered in a host microorganism, and in certain embodiments, the activity is added or increased in the engineered microorganism relative to the host microorganism. In some embodiments, the altered monooxygenase activity is an endogenous activity, and in certain embodiments, the altered monooxygenase activity is an exogenous activity. In some embodiments, the exogenous activity is a single polypeptide with both monooxygenase and monooxygenase reductase activities (e.g., B. megaterium cytochrome P450:NADPH P450 reductase).

Presence, absence or amount of cytochrome P450 activity can be detected by any suitable method known in the art. For example, detection can be performed by assaying a reaction containing cytochrome P450 (CYP52A family) and NADPH - cytochrome P450 reductase (see Appl Environ Microbiol 69: 5983 and 5992). Briefly, cells are grown under standard conditions and harvested for production of microsomes, which are used to detect CYP activity. Microsomes are prepared by lysing cells in Tris-buffered sucrose (10mM Tris-HCl pH 7.5, 1mM EDTA, 0.25M sucrose). Differential centrifugation is performed first at 25,000xg then at 100,000xg to pellet cell debris then microsomes, respectively. The microsome pellet is resuspended in 0.1 M phosphate buffer (pH 7.5), 1 mM EDTA to a final concentration of approximately 10mg protein/mL. A reaction mixture containing approximately 0.3mg microsomes, 0.1 mM sodium hexanoate, 0.7mM NADPH, 50mM Tris-HCl pH 7.5 in 1 mL is initiated by the addition of NADPH and incubated at 37°C for 10 minutes. The reaction is terminated by addition of 0.25mL 5M HCl and 0.25mL 2.5ug/mL 10-hydroxydecanoic acid is added as an internal standard (3.3 nmol). The mixture is extracted with 4.5mL diethyl ether under NaCl-saturated conditions. The organic phase is transferred to a new tube and evaporated to dryness. The residue is dissolved in acetonitrile containing 10mM 3-bromomethyl-7-methoxy-1,4-benzoxazin-2-one (BrMB) and 0.1mL of 15mg/mL 18-crown-6 in acetonitril saturated with K₂CO₃. The solution is incubated at 40°C for 30 minutes before addition of 0.05mL 2% acetic acid. The fluorescently labeled omega-hydroxy fatty acids are resolved via HPLC with detection at 430nm and excitation at 355nm (Yamada et al., 1991, AnalBiochem 199: 132-136). Optionally, specifically induced CYP gene(s) may be detected by Northern blotting and/or quantitative RT-PCR. (Craft et al., 2003, App. Environ. Micro. 69: 5983-5991).

The term "monooxygenase reductase activity" as used herein refers to the transfer of an electron from NAD(P)H, FMN, or FAD by way of an electron transfer chain, reducing the ferric heme iron of cytochrome P450 to the ferrous state. The term "monooxygenase reductase activity" as used herein also can refer to the transfer of a second electron via the electron transport system, reducing a dioxygen adduct to a negatively charged peroxo group. In some embodiments, a monooxygenase activity can donate electrons from the two-electron donor NAD(P)H to the heme of cytochrome P450 (e.g., monooxygenase activity) in a coupled two-step reaction in which NAD(P)H can bind to the NAD(P)H-binding domain of the polypeptide having the monooxygenase reductase activity and electrons are shuttled from NAD(P)H through FAD and FMN to the heme of the monooxygenase activity, thereby regenerating an active monooxygenase activity (e.g., cytochrome P450). Monooxygenase reductase activity can be provided by any suitable polypeptide, such as a cytochrome P450 reductase polypeptide (hereafter "CPR") in certain embodiments. Nucleic acid sequences conferring CPR activity can be obtained from and/or induced in a number of sources, including but not limited to Bacillus megaterium, Candida tropicalis, Candida viswanithii, Yarrowia lipolytica, Aspergillus nidulans, and Aspergillus parasiticus. Examples of oligonucleotide sequences utilized to isolate a polynucleotide sequence encoding a polypeptide having CPR activity are presented herein. In some embodiments, monooxygenase reductase activity is not altered in a host microorganism, and in certain embodiments, the activity is added or increased in the engineered microorganism relative to the host microorganism. In some embodiments, the altered monooxygenase reductase activity is an endogenous activity, and in certain embodiments, the altered monooxygenase reductase activity is an exogenous activity. In some embodiments, the exogenous activity is a single polypeptide with both monooxygenase and monooxygenase reductase activities (e.g., B. megaterium cytochrome P450:NADPH P450 reductase).

Presence, absence or amount of CPR activity can be detected by any suitable method known in the art. For example, an engineered microorganism having an increased number of genes encoding a CPR activity, relative to the host microorganism, could be detected using quantitative nucleic acid detection methods (e.g., southern blotting, PCR, primer extension, the like and combinations thereof). An engineered microorganism having increased expression of genes encoding a CPR activity, relative to the host microorganism, could be detected using quantitative expression based analysis (e.g., RT-PCR, western blot analysis, northern blot analysis, the like and combinations thereof). Alternately, an enzymatic assay can be used to detect Cytochrome P450 reductase activity, where the enzyme activity alters the optical absorbance at 550 nanometers of a substrate solution (Masters, B.S.S., Williams, C.H., Kamin, H. (1967) Methods in Enzymology, X, 565-573).

The term "fatty alcohol oxidase activity" as used herein refers to inserting one atom of oxygen from O₂ into an organic substrate and reducing the other oxygen atom to peroxide. Fatty alcohol oxidase activity sometimes also is referred to as "long-chain-alcohol oxidase activity", "long-chain-alcohol:oxygen oxidoreductase activity", "fatty alcohol:oxygen oxidoreductase activity" and "long-chain fatty acid oxidase activity". In some embodiments, fatty alcohol oxidase activity refers to incorporation of an oxygen atom onto a six-carbon organic substrate. In certain embodiments, fatty alcohol oxidase activity refers to the conversion of 6-hydroxyhexanoic acid into 6-oxohexanoic acid. In some embodiments, fatty alcohol oxidase activity refers to the conversion of an omega hydroxyl fatty acid into an omega oxo fatty acid. A Fatty alcohol oxidase (FAO) activity can be provided by any suitable polypeptide, such as a fatty alcohol oxidase peptide, a long-chain-alcohol oxidase peptide, a long-chain-alcohol:oxygen oxidoreductase peptide, a fatty alcohol:oxygen oxidoreductase peptide and a long-chain fatty acid oxidase peptide. Nucleic acid sequences conferring FAO activity can be obtained from a number of sources, including but not limited to Candida tropicalis, Candida viswanithii, Candida cloacae, Yarrowia lipolytica, and Arabidopsis thaliana. Examples of amino acid sequences of polypeptides having FAO activity, and nucleotide sequences of polynucleotides that encode the polypeptides, are presented herein. In some embodiments, fatty alcohol oxidase activity is not altered in a host microorganism, and in certain embodiments, the activity is added or increased in the engineered microorganism relative to the host microorganism.

Presence, absence or amount of FAO activity can be detected by any suitable method known in the art. For example, an engineered microorganism having an increased number of genes encoding an FAO activity, relative to the host microorganism, could be detected using quantitative nucleic acid detection methods (e.g., southern blotting, PCR, primer extension, the like and combinations thereof). An engineered microorganism having increased expression of genes encoding an FAO activity, relative to the host microorganism, could be detected using quantitative expression based analysis (e.g., RT-PCR, western blot analysis, northern blot analysis, the like and combinations thereof). Alternately, an enzymatic assay can be used to detect fatty alcohol oxidase activity as described in Eirich et al, 2004, or as modified in the Examples herein.

The term "acyl-CoA oxidase activity" as used herein refers to the oxidation of a long chain fatty-acyl-CoA to a trans-2,3-dehydroacyl-CoA fatty alcohol. In some embodiments, the acyl-CoA activity is from a peroxisome. In certain embodiments, the acyl-CoA oxidase activity is a peroxisomal acyl-CoA oxidase (POX) activity, carried out by a POX polypeptide. In some embodiments the acyl-CoA oxidase activity is encoded by the host organism and sometimes can be altered to generate an engineered organism. Acyl-CoA oxidase activity is encoded by the POX4 and POX5 genes of C. tropicalis or C. viswanithii in some embodiments. In certain embodiments, endogenous acyl-CoA oxidase activity can be increased. In some embodiments, acyl-CoA oxidase activity of the POX4 polypeptide or the POX5 polypeptide can be altered independently of each other (e.g., increase activity of POX4 alone, POX5 alone, increase one and disrupt the other, and the like). Increasing the activity of one POX activity, while disrupting the activity of another POX activity, may alter the specific activity of acyl-CoA oxidase with respect to carbon chain length, while maintaining or increasing overall flux through the beta oxidation pathway, in certain embodiments.

Figures 15A-15C graphically illustrate the units of acyl-CoA oxidase activity expressed as units (U) per milligram of protein (Y axis) in various strains of Candida tropicalis induced by feedstocks of specific chain length (Picataggio et al. 1991 Molecular and Cellular Biology 11: 4333-4339). Isolated protein was assayed for acyl-CoA oxidase activity using carbon chains of various length (X axis). The X and Y axes in Figures 15A -15C represent substantially similar data. Figure 15A illustrates acyl-CoA oxidase activity as measured in a strain having a full complement of POX genes (e.g., POX4 and POX5 are active). Figure 15B illustrates acyl-CoA oxidase activity as measured in a strain having a disrupted POX5 gene. The activity encoded by the functional POX4 gene exhibits a higher specific activity for acyl-CoA molecules with shorter carbon chain lengths (e.g., less than 10 carbons). The results of the POX5 disrupted strain also are presented numerically in the table in Figure 15B. Figure 15C illustrates acyl-CoA oxidase activity as measured in a strain having a disrupted POX4 gene. The activity encoded by the functional POX5 gene exhibits a narrow peak of high specific activity for acyl-CoA molecules 12 carbons in length, with a lower specific activity for molecules 10 carbons in length. The results of the POX4 disrupted strain are presented numerically in the table in Figure 15C.

In certain embodiments, host acyl-CoA oxidase activity of one of the POX genes can be increased by genetically altering (e.g., increasing) the amount of the polypeptide produced (e.g., a strongly transcribed or constitutively expressed heterologous promoter is introduced in operable linkage with a polynucleotide that encodes the polypeptide; the copy number of a polynucleotide that encodes the polypeptide is increased (e.g., by introducing a plasmid that includes the polynucleotide, integration of additional copies in the host genome)). In some embodiments, the host acyl-CoA oxidase activity can be decreased by disruption (e.g., knockout, insertion mutagenesis, the like and combinations thereof) of an acyl-CoA oxidase gene, or by decreasing the activity of the promoter (e.g., addition of repressor sequences to the promoter or 5'UTR) which transcribes an acyl-CoA oxidase gene.

A noted above, disruption of nucleotide sequences encoding POX4, POX 5, or POX4 and POX5 sometimes can alter pathway efficiency, specificity and/or specific activity with respect to metabolism of carbon chains of different lengths (e.g., carbon chains including fatty alcohols, fatty acids, paraffins, dicarboxylic acids of between about 1 and about 60 carbons in length). In some embodiments, the nucleotide sequence of POX4, POX5, or POX4 and POX5 is disrupted with a URA3 nucleotide sequence encoding a selectable marker, and introduced to a host microorganism, thereby generating an engineered organism deficient in POX4, POX5 or POX4 and POX5 activity. Nucleic acid sequences encoding POX4 and POX5 can be obtained from a number of sources, including Candida tropicalis, for example. Examples of POX4 and POX5 amino acid sequences and nucleotide sequences of polynucleotides that encode the polypeptides, are presented herein. Example 32 describes experiments conducted to amplify the activity encoded by the POX5 gene.

Presence, absence or amount of POX4 and/or POX5 activity can be detected by any suitable method known in the art, for example, using enzymatic assays as described in Shimizu et al, 1979, and as described herein in the Examples. Alternatively, nucleic acid sequences representing native and/or disrupted POX4 and POX5 sequences also can be detected using nucleic acid detection methods (e.g., PCR, primer extension, nucleic acid hybridization, the like and combinations thereof), or quantitative expression based analysis (e.g., RT-PCR, western blot analysis, northern blot analysis, the like and combinations thereof), where the engineered organism exhibits decreased RNA and/or polypeptide levels as compared to the host organism.

The term "a genetic modification that results in increased fatty acid synthesis" as used herein refers to a genetic alteration of a host microorganism that increases an endogenous activity and/or adds a heterologous activity that metabolically synthesizes fatty acids and/or imports fatty acids into the microorganism from an external source (e.g., a feedstock, culture medium, environment, the like and combinations thereof). In some embodiments, an endogenous activity that converts fatty acyl-CoA to fatty acid is increased. In certain embodiments, a thioesterase activity is added or increased. Such alterations can advantageously increase yields of end products, such as adipic acid.

The term "thioesterase activity" as used herein refers to removal of Coenzyme A from hexanoate. The term "thioesterase activity" as used herein also refers to the removal of Coenzyme A from an activated fatty acid (e.g., fatty-acyl-CoA). A Non-limiting example of an enzyme with thioesterase activity includes acyl-CoA hydrolase (e.g., EC 3.1.2.20; also referred to as acyl coenzyme A thioesterase, acyl-CoA thioesterase, acyl coenzyme A hydrolase, thioesterase B, thioesterase II, lecithinase B, lysophopholipase L1, acyl-CoA thioesterase 1, and acyl-CoA thioesterase). Thioesterases that remove Coenzyme A from fatty-acyl-CoA molecules catalyze the reaction,

acyl-CoA + H₂O CoA + a carboxylate,

where the carboxylate often is a fatty acid. The released Coenzyme A can then be reused for other cellular activities.

The thioesterase activity can be provided by a polypeptide. In certain embodiments, the polypeptide is an endogenous nucleotide sequence that is increased in copy number, operably linked to a heterologous and/or endogenous promoter, or increased in copy number and operably linked to a heterologous and/or endogenous promoter. In some embodiments, the polypeptide is encoded by a heterologous nucleotide sequence introduced to a host microorganism. Nucleic acid sequences conferring thioesterase activity can be obtained from a number of sources, including C. tropicalis (e.g., see SEQ ID NOS: 42 and 44), C. viswanithii, E. coli (e.g., see SEQ ID NO: 46) and Cuphea lanceolata. Examples of such polypeptides include, without limitation, acyl-CoA hydrolase (e.g., ACHA and ACHB, see SEQ ID NOS: 43 and 45)) from C. tropicalis, acyl-CoA thioesterase (e.g., TESA, see SEQ ID NO: 47) from E. coli, and acyl-(ACP) thioesterase type B from Cuphea lanceolata, encoded by the nucleotide sequences referenced by accession number CAB60830 at the World Wide Web Uniform Resource Locator (URL) ncbi.nlm.nih.gov of the National Center for Biotechnology Information (NCBI).

Presence, absence or amount of thioesterase activity can be detected by any suitable method known in the art. An example of such a method is described Chemistry and Biology 9: 981-988. In some embodiments, thioesterase activity is not altered in a host microorganism, and in certain embodiments, the activity is added or increased in the engineered microorganism relative to the host microorganism. In some embodiments, a polypeptide having thioesterase activity is linked to another polypeptide (e.g., a hexanoate synthase A or hexanoate synthase B polypeptide). A non-limiting example of an amino acid sequence (one letter code sequence) for a polypeptide having thioesterase activity is provided hereafter:

MVAAAATSAFFPVPAPGTSPKPGKSGNWPSSLSPTFKPKSIPNAGFQVKANASAHP
KANGSAVNLKSGSLNTQEDTSSSPPPRAFLNQLPDWSMLLTAITTVFVAAEKQWTM
LDRKSKRPDMLVDSVGLKSIVRDGLVSRQSFLIRSYEIGADRTASIETLMNHLQET
SINHCKSLGLLNDGFGRTPGMCKNDLIWVLTKMQIMVNRYPTWGDTVEINTWFSQS
GKIGMASDWLISDCNTGEILIRATSVWAMMNQKTRRFSRLPYEVRQELTPHFVDSP
HVIEDNDQKLHKFDVKTGDSIRKGLTPRWNDLDVNQHVSNVKYIGWILESMPIEVL
ETQELCSLTVEYRRECGMDSVLESVTAVDPSENGGRSQYKHLLRLEDGTDIVKSRT
EWRPKNAGTNGAISTSTAKTSNGNSAS

Additional examples of polynucleotide sequences encoding thioesterase activities, and polypeptides having thioesterase activity are provided in Example 51 (see SEQ ID NOS: 42-47).

The term "a genetic modification that results in substantial hexanoate usage by monooxygenase activity" as used herein refers to a genetic alteration of a host microorganism that reduces an endogenous activity that converts hexanoate to another product. In some embodiments, an endogenous activity that converts hexanoate to a toxin (e.g., in fungus) is reduced. In certain embodiments, a polyketide synthase activity is reduced. Such alterations can advantageously increase yields of end products, such as adipic acid.

The term "polyketide synthase activity" as used herein refers to the alteration of hexanoic acid by the polyketide synthase enzyme (PKS) as a step in the production of other products including mycotoxin. The PKS activity can be provided by a polypeptide. Examples of such polypeptides include, without limitation, an Aspergillus parasiticus enzyme referenced by accession number AAS66004 at the World Wide Web Uniform Resource Locator (URL) ncbi.nlm.nih.gov of the National Center for Biotechnology Information (NCBI). In certain embodiments, a PKS enzyme uses hexanoic acid generated by hexanoate synthase as a substrate and a component of the Aspergillus NorS multienzyme complex, a closely associated gene cluster involved in the synthesis of various products including mytoxin. Accordingly, a PKS activity sometimes is altered to free hexanoic acid for an engineered adipic acid pathway. In some embodiments PKS activity is diminished or blocked. In certain embodiments the PKS enzyme is engineered to substitute thioesterase activity for PKS activity. Presence, absence, or amount of PKS activity can be detected by any suitable method known in the art, such as that described in Watanabe C and Townsend C (2002) Initial characterization of a type I fatty acid synthase and polyketide synthase multienzyme complex NorS in the biosynthesis of aflatoxin B1. Chemistry and Biology 9: 981-988. A non-limiting example of an amino acid sequence (one letter code sequence) of a polypeptide having polyketide synthase activity is provided hereafter:

MAQSRQLFLFGDQTADFVPKLRSLLSVQDSPILAAFLDQSHYVVRAQMLQSMNTVD
HKLARTADLRQMVQKYVDGKLTPAFRTALVCLCQLGCFIREYEESGNMYPQPSDSY
VLGFCMGSLAAVAVSCSRSLSELLPIAVQTVLIAFRLGLCALEMRDRVDGCSDDRG
DPWSTIVWGLDPQQARDQIEVFCRTTNVPQTRRPWISCISKNAITLSGSPSTLRAF
CAMPQMAQHRTAPIPICLPAHNGALFTQADITTILDTTPTTPWEQLPGQIPYISHV
TGNVVQTSNYRDLIEVALSETLLEQVRLDLVETGLPRLLQSRQVKSVTIVPFLTRM
NETMSNILPDSFISTETRTDTGRAIPASGRPGAGKCKLAIVSMSGRFPESPTTESF
WDLLYKGLDVCKEVPRRRWDINTHVDPSGKARNKGATKWGCWLDFSGDFDPRFFGI
SPKEAPQMDPAQRMALMSTYEAMERAGLVPDTTPSTQRDRIGVFHGVTSNDWMETN
TAQNIDTYFITGGNRGFIPGRINFCFEFAGPSYTNDTACSSSLAAIHLACNSLWRG
DCDTAVAGGTNMIYTPDGHTGLDKGFFLSRTGNCKPYDDKADGYCRAEGVGTVFIK
RLEDALADNDPILGVILDAKTNHSAMSESMTRPHVGAQIDNMTAALNTTGLHPNDF
SYIEMHGTGTQVGDAVEMESVLSVFAPSETARKADQPLFVGSAKANVGHGEGVSGV
TSLIKVLMMMQHDTIPPHCGIKPGSKINRNFPDLGARNVHIAFEPKPWPRTHTPRR
VLINNFSAAGGNTALIVEDAPERHWPTEKDPRSSHIVALSAHVGASMKTNLERLHQ
YLLKNPHTDLAQLSYTTTARRWHYLHRVSVTGASVEEVTRKLEMAIQNGDGVSRPK
SKPKILFAFTGQGSQYATMGKQVYDAYPSFREDLEKFDRLAQSHGFPSFLHVCTSP
KGDVEEMAPVVVQLAITCLQMALTNLMTSFGIRPDVTVGHSLGEFAALYAAGVLSA
SDVVYLVGQRAELLQERCQRGTHAMLAVKATPEALSQWIQDHDCEVACINGPEDTV
LSGTTKNVAEVQRAMTDNGIKCTLLKLPFAFHSAQVQPILDDFEALAQGATFAKPQ
LLILSPLLRTEIHEQGVVTPSYVAQHCRHTVDMAQALRSAREKGLIDDKTLVIELG
PKPLISGMVKMTLGDKISTLPTLAPNKAIWPSLQKILTSVYTGGWDINWKKYHAPF
ASSQKVVDLPSYGWDLKDYYIPYQGDWCLHRHQQDCKCAAPGHEIKTADYQVPPES

TPHRPSKLDPSKEAFPEIKTTTTLHRVVEETTKPLGATLVVETDISRKDVNGLARG
HLVDGIPLCTPSFYADIAMQVGQYSMQRLRAGHPGAGAIDGLVDVSDMVVDKALVP
HGKGPQLLRTTLTMEWPPKAAATTRSAKVKFATYFADGKLDTEHASCTVRFTSDAQ
LKSLRRSVSEYKTHIRQLHDGHAKGQFMRYNRKTGYKLMSSMARFNPDYMLLDYLV
LNEAENEAASGVDFSLGSSEGTFAAHPAHVDAITQVAGFAMNANDNVDIEKQVYVN
HGWDSFQIYQPLDNSKSYQVYTKMGQAKENDLVHGDVVVLDGEQIVAFFRGLTLRS
VPRGALRVVLQTTVKKADRQLGFKTMPSPPPPTTTMPISPYKPANTQVSSQAIPAE
ATHSHTPPQPKHSPVPETAGSAPAAKGVGVSNEKLDAVMRVVSEESGIALEELTDD
SNFADMGIDSLSSMVIGSRFREDLGLDLGPEFSLFIDCTTVRALKDFMLGSGDAGS
GSNVEDPPPSATPGINPETDWSSSASDSIFASEDHGHSSESGADTGSPPALDLKPY
CRPSTSVVLQGLPMVARKTLFMLPDGGGSAFSYASLPRLKSDTAVVGLNCPYARDP
ENMNCTHGAMIESFCNEIRRRQPRGPYHLGGWSSGGAFAYVVAEALVNQGEEVHSL
IIIDAPIPQAMEQLPRAFYEHCNSIGLFATQPGASPDGSTEPPSYLIPHFTAVVDV
MLDYKLAPLHARRMPKVGIVWAADTVMDERDAPKMKGMHFMIQKRTEFGPDGWDTI
MPGASFDIVRADGANHFTLMQKEHVSIISDLIDRVMA

The terms "a genetic modification that reduces 6-hydroxyhexanoic acid conversion" or "a genetic modification that reduces omega hydroxyl fatty acid conversion" as used herein refer to genetic alterations of a host microorganism that reduce an endogenous activity that converts 6-hydroxyhexanoic acid to another product. In some embodiments, an endogenous 6-hydroxyhexanoic acid dehydrogenase activity is reduced. Such alterations can advantageously increase the amount of 6-hydroxyhexanoic acid, which can be purified and further processed.

The term "a genetic modification that reduces beta-oxidation activity" as used herein refers to a genetic alteration of a host microorganism that reduces an endogenous activity that oxidizes a beta carbon of carboxylic acid containing organic molecules. In certain embodiments, the organic molecule is a six carbon molecule, and sometimes contains one or two carboxylic acid moieties located at a terminus of the molecule (e.g., adipic acid). Such alterations can advantageously increase yields of end products, such as adipic acid.

The term "a genetic modification that results in increased fatty acid synthesis" as used herein also refers to a genetic alteration of a host microorganism that reduces an endogenous activity that converts fatty acids into fatty-acyl-CoA intermediates. In some embodiments, an endogenous activity that converts fatty acids into fatty-acyl-CoA intermediates is reduced. In certain embodiments, an acyl-CoA synthetase activity is reduced. Such alterations can advantageously increase yields of end products, such as adipic acid.

Fatty acids can be converted into fatty-acyl-CoA intermediates by the activity of an acyl-CoA synthetase (e.g., ACS1, ACS2; EC 6.2.1.3; also referred to as acyl-CoA synthetase, acyl-CoA ligase), in many organisms. Acyl-CoA synthetase has two isoforms encoded by ACS1 and ACS2, respectively, in C. tropicalis (e.g., homologous to FAA1, FAA2, FAA3 and FAA4 in S. cerevisiae). Acyl-CoA synthetase is a member of the ligase class of enzymes and catalyzes the reaction,

ATP + Fatty Acid + CoA <=> AMP + Pyrophosphate + Fatty-Acyl-CoA.

Fatty acids and Coenzyme A often are utilized in the activation of fatty acids to fatty-acyl-CoA intermediates for entry into various cellular processes. Without being limited by theory, it is believed that reduction in the amount of fatty-acyl-CoA available for various cellular processes can increase the amount of fatty acids available for conversion into adipic acid by other engineered pathways in the same host organism (e.g., omega oxidation pathway, beta oxidation pathway, omega oxidation pathway and beta oxidation pathway). Acyl-CoA synthetase can be inactivated by any suitable means. Described herein are gene knockout methods suitable for use to disrupt the nucleotide sequence that encodes a polypeptide having ACS1 activity. A nucleotide sequence of ACS1 is provided in Example 51, SEQ ID NO: 48. An example of an integration/disruption construct, configured to generate a deletion mutant for ACS1 is described in Example 43.

The presence, absence or amount of acyl-CoA synthetase activity can be detected by any suitable method known in the art. Non-limiting examples of suitable detection methods include enzymatic assays (e.g., Lageweg et al "A Fluorometric Assay for Acyl-CoA Synthetase Activity", Analytical Biochemistry, 197(2):384-388 (1991)), PCR based assays (e.g., qPCR, RTPCR), immunological detection methods (e.g., antibodies specific for acyl-CoA synthetase), the like and combinations thereof.

The term "a genetic modification that results in increased fatty acid synthesis" as used herein also refers to a genetic alteration of a host microorganism that reduces an endogenous activity that converts long chain and very long chain fatty acids into activated fatty-acyl-CoA intermediates. In some embodiments, an endogenous activity that converts long chain and very long chain fatty acids into activated fatty-acyl-CoA intermediates is reduced. In certain embodiments, a long chain acyl-coA synthetase activity is reduced. Such alterations can advantageously increase yields of end products, such as adipic acid.

Long chain fatty acids (e.g., C12-C18 chain lengths) and very long chain fatty acids (e.g., C20-C26) often are activated and/or transported by the thioesterification activity of a long-chain acyl-CoA synthetase (e.g., FAT1; EC EC 6.2.1.3; also referred to as long-chain fatty acid-CoA ligase, acyl-CoA synthetase; fatty acid thiokinase (long chain); acyl-activating enzyme; palmitoyl-CoA synthase; lignoceroyl-CoA synthase; arachidonyl-CoA synthetase; acyl coenzyme A synthetase; acyl-CoA ligase; palmitoyl coenzyme A synthetase; thiokinase; palmitoyl-CoA ligase; acyl-coenzyme A ligase; fatty acid CoA ligase; long-chain fatty acyl coenzyme A synthetase; oleoyl-CoA synthetase; stearoyl-CoA synthetase; long chain fatty acyl-CoA synthetase; long-chain acyl CoA synthetase; fatty acid elongase; LCFA synthetase; pristanoyl-CoA synthetase; ACS3; long-chain acyl-CoA synthetase I; long-chain acyl-CoA synthetase II; fatty acyl-coenzyme A synthetase; long-chain acyl-coenzyme A synthetase; and acid:CoA ligase (AMP-forming)), in some organisms. Fatty acids also can be transported into the host organism from feedstocks by the activity of long chain acyl-CoA synthetase.

Long-chain acyl-CoA synthetase catalyzes the reaction,

ATP + a long-chain carboxylic acid + CoA = AMP + diphosphate + an acyl-CoA,

where "an acyl-CoA" refers to a fatty-acyl-CoA molecule. As noted herein, activation of fatty acids is often necessary for entry of fatty acids into various cellular processes (e.g., as an energy source, as a component for membrane formation and/or remodeling, as carbon storage molecules). Deletion mutants of FAT1 have been shown to accumulate very long chain fatty acids and exhibit decreased activation of these fatty acids. Without being limited by theory, it is believed that reduction in the activity of long-chain acyl-CoA synthetase may reduce the amount of long chain fatty acids converted into fatty-acyl-CoA intermediates, thereby increasing the amount of fatty acids available for conversion into adipic acid by other engineered pathways in the same host organism (e.g., omega oxidation pathway, beta oxidation pathway, omega oxidation pathway and beta oxidation pathway). Long-chain-acyl-CoA synthetase activity can be reduced or inactivated by any suitable means. Described herein are gene knockout methods suitable for disrupting the nucleotide sequence that encodes the polypeptide having FAT1 activity. The nucleotide sequence of FAT1 is provided in Example 51, SEQ ID NO: 50. DNA vectors suitable for use in constructing "knockout" constructs are described herein.

The presence, absence or amount of long-chain-acyl-CoA synthetase activity can be detected by any suitable method known in the art. Non-limiting examples of suitable detection methods include enzymatic assays, binding assays (e.g., Erland et al, Analytical Biochemistry 295(1):38-44 (2001)), PCR based assays (e.g., qPCR, RT-PCR), immunological detection methods (e.g., antibodies specific for long-chain-acyl-CoA synthetase), the like and combinations thereof.

The term "a genetic modification that results in increased fatty acid synthesis" as used herein also refers to a genetic alteration of a host microorganism that reduces an endogenous activity that converts cholesterol into cholesterol esters. In some embodiments, an endogenous activity that converts cholesterol into cholesterol esters is reduced. In certain embodiments, an acyl-CoA sterol acyltransferase activity is reduced. Such alterations can advantageously increase yields of end products, such as adipic acid.

Cholesterol can be converted into a cholesterol-ester by the activity of acyl-CoA sterol acyltransferase (e.g., ARE1, ARE2; EC 2.3.1.26; also referred to as sterol O-acyltransferase; cholesterol acyltransferase; sterol-ester synthase; sterol-ester synthetase; sterol-ester synthase; acyl coenzyme A-cholesterol-O-acyltransferase; acyl-CoA:cholesterol acyltransferase; ACAT; acylcoenzyme A:cholesterol O-acyltransferase; cholesterol ester synthase; cholesterol ester synthetase; and cholesteryl ester synthetase), in many organisms. Without being limited by any theory, cholesterol esterification may be involved in directing cholesterol away from incorporation into cell membranes and towards storage forms of lipids. Acyl-CoA sterol acyltransferase catalyzes the reaction,

acyl-CoA + cholesterol = CoA + cholesterol ester.

The esterification of cholesterol is believed to limit its solubility in cell membrane lipids and thus promotes accumulation of cholesterol ester in the fat droplets (e.g., a form of carbon storage molecule) within cytoplasm. Therefore, without being limited by any theory esterification of cholesterol may cause the accumulation of lipid storage molecules, and disruption of the activity of acyl-CoA sterol acyltransferase may cause an increase in acyl-CoA levels that can be converted into adipic acid by other engineered pathways in the same host organism (e.g., omega oxidation pathway, beta oxidation pathway, omega oxidation pathway and beta oxidation pathway). Acyl-CoA sterol acyltransferase can be inactivated by any suitable means. Described herein are gene knockout methods suitable for disrupting nucleotide sequences that encode polypeptides having ARE1 activity, ARE2 activity or ARE1 activity and ARE2 activity. The nucleotide sequences of ARE1 and ARE2 are provided in Example 51, SEQ ID NOS: 52 and 54. DNA vectors suitable for use in constructing "knockout" constructs are described herein.
The presence, absence or amount of acyl-CoA sterol acyltransferase activity can be detected by any suitable method known in the art. Non-limiting examples of suitable detection methods include enzymatic assays (e.g., Chen et al, Plant Physiology 145:974-984 (2007)), binding assays, PCR based assays (e.g., qPCR, RT-PCR), immunological detection methods (e.g., antibodies specific for long-chain-acyl-CoA synthetase), the like and combinations thereof.

The term "a genetic modification that results in increased fatty acid synthesis" as used herein also refers to a genetic alteration of a host microorganism that reduces an endogenous activity that catalyzes diacylglycerol esterification (e.g., addition of acyl group to a diacylglycerol to form a triacylglycerol). In some embodiments, an endogenous activity that converts diacyglycerol into triacylglycerol is reduced. In certain embodiments, an acyltransferase activity is reduced. In some embodiments a diacylglycerol acyltransferase activity is reduced. In some embodiments a diacylglycerol acyltransferase (e.g., DGA1) activity and an acyltransferase (e.g., LRO1) activity are reduced. Such alterations can advantageously increase yields of end products, such as adipic acid.

Diacylglycerol can be converted into triacylglycerol by the activity of diacylglycerol acyltransferase (e.g., DGA1; EC2.3.1.20; also referred to as diglyceride acyltransferase; 1,2-diacylglycerol acyltransferase; diacylglycerol acyltransferase; diglyceride O-acyltransferase; palmitoyl-CoA-sn-1,2-diacylglycerol acyltransferase; acyl-CoA:1,2-diacylglycerol O-acyltransferase and acyl-CoA:1,2-diacyl-sn-glycerol O-acyltransferase), in many organisms. Diacylglycerol acyltransferase catalyzes the reaction,

Acyl-CoA + 1,2-diacyl-sn-glycerol = CoA + triacylglycerol,

and is generally considered the terminal and only committed step in triglyceride synthesis. The product of the DGA1 gene in yeast normally is localized to lipid particles.

In addition to the diacylglycerol esterification activity described for DGA1, many organisms also can generate triglycerides by the activity of other acyltransferase activities, non-limiting examples of which include lecithin-cholesterol acyltransferase activity (e.g., LRO1; EC 2.3.1.43; also referred to as phosphatidylcholine-sterol O-acyltransferase activity; lecithin-cholesterol acyltransferase activity; phospholipid-cholesterol acyltransferase activity; LCAT (lecithin-cholesterol acyltransferase) activity; lecithin:cholesterol acyltransferase activity; and lysolecithin acyltransferase activity) and phospholipid:diacylglycerol acyltransferase (e.g., EC 2.3.1.158; also referred to as PDAT activity and phospholipid:1,2-diacyl-sn-glycerol O-acyltransferase activity). Acyltransferases of the families EC 2.3.1.43 and EC 2.3.1.58 catalyze the general reaction,

phospholipid + 1,2-diacylglycerol = lysophospholipid + triacylglycerol.

Triacylglycerides often are utilized as carbon (e.g., fatty acid or lipid) storage molecules. Without being limited by any theory, it is believe that reducing the activity of acytransferases may reduce the conversion of diacylglycerol to triacylglycerol, which may cause increased accumulation of fatty acid, in conjunction with additional genetic modifications (e.g., lipase to further remove fatty acids from the glycerol backbone) that can be converted into adipic acid by other engineered pathways in the same host organism (e.g., omega oxidation pathway, beta oxidation pathway, omega oxidation pathway and beta oxidation pathway). Acyltransferases can be inactivated by any suitable means. Described herein are gene knockout methods suitable for disrupting nucleotide sequences that encode polypeptides having DGA1 activity, LRO1 activity or DGA1 activity and LRO1 activity. The nucleotide sequence of DGA1 is provided in Example 51, SEQ ID NO: 56. The nucleotide sequence of LRO1 is provided in Example 51, SEQ ID NO: 58. DNA vectors suitable for use in constructing "knockout" constructs are described herein.

The presence, absence or amount of acyltransferase activity can be detected by any suitable method known in the art. Non-limiting examples of suitable detection methods include enzymatic assays (e.g., Geelen, Analytical Biochemistry 322(2):264-268 (2003), Dahlqvist et al, PNAS 97(12):6487-6492 (2000)), binding assays, PCR based assays (e.g., qPCR, RT-PCR), immunological detection methods (e.g., antibodies specific for a DGA1 or LRO1 acyltransferase), the like and combinations thereof.

### Polynucleotides and Polypeptides

A nucleic acid (e.g., also referred to herein as nucleic acid reagent, target nucleic acid, target nucleotide sequence, nucleic acid sequence of interest or nucleic acid region of interest) can be from any source or composition, such as DNA, cDNA, gDNA (genomic DNA), RNA, siRNA (short inhibitory RNA), RNAi, tRNA or mRNA, for example, and can be in any form (e.g., linear, circular, supercoiled, single-stranded, double-stranded, and the like). A nucleic acid can also comprise DNA or RNA analogs (e.g., containing base analogs, sugar analogs and/or a non-native backbone and the like). It is understood that the term "nucleic acid" does not refer to or infer a specific length of the polynucleotide chain, thus polynucleotides and oligonucleotides are also included in the definition. Deoxyribonucleotides include deoxyadenosine, deoxycytidine, deoxyguanosine and deoxythymidine. For RNA, the uracil base is uridine.

A nucleic acid sometimes is a plasmid, phage, autonomously replicating sequence (ARS), centromere, artificial chromosome, yeast artificial chromosome (e.g., YAC) or other nucleic acid able to replicate or be replicated in a host cell. In certain embodiments a nucleic acid can be from a library or can be obtained from enzymatically digested, sheared or sonicated genomic DNA (e.g., fragmented) from an organism of interest. In some embodiments, nucleic acid subjected to fragmentation or cleavage may have a nominal, average or mean length of about 5 to about 10,000 base pairs, about 100 to about 1,000 base pairs, about 100 to about 500 base pairs, or about 10, 15, 20, 25, 30, 35, 40, 45, 50, 55, 60, 65, 70, 75, 80, 85, 90, 95, 100, 200, 300, 400, 500, 600, 700, 800, 900, 1000, 2000, 3000, 4000, 5000, 6000, 7000, 8000, 9000 or 10000 base pairs. Fragments can be generated by any suitable method in the art, and the average, mean or nominal length of nucleic acid fragments can be controlled by selecting an appropriate fragment-generating procedure by the person of ordinary skill. In some embodiments, the fragmented DNA can be size selected to obtain nucleic acid fragments of a particular size range.

Nucleic acid can be fragmented by various methods known to the person of ordinary skill, which include without limitation, physical, chemical and enzymic processes. Examples of such processes are described in U.S. Patent Application Publication No. 20050112590 (published on May 26, 2005, entitled "Fragmentation-based methods and systems for sequence variation detection and discovery," naming Van Den Boom et al.). Certain processes can be selected by the person of ordinary skill to generate non-specifically cleaved fragments or specifically cleaved fragments. Examples of processes that can generate non-specifically cleaved fragment sample nucleic acid include, without limitation, contacting sample nucleic acid with apparatus that expose nucleic acid to shearing force (e.g., passing nucleic acid through a syringe needle; use of a French press); exposing sample nucleic acid to irradiation (e.g., gamma, x-ray, UV irradiation; fragment sizes can be controlled by irradiation intensity); boiling nucleic acid in water (e.g., yields about 500 base pair fragments) and exposing nucleic acid to an acid and base hydrolysis process.

Nucleic acid may be specifically cleaved by contacting the nucleic acid with one or more specific cleavage agents. The term "specific cleavage agent" as used herein refers to an agent, sometimes a chemical or an enzyme that can cleave a nucleic acid at one or more specific sites. Specific cleavage agents often will cleave specifically according to a particular nucleotide sequence at a particular site. Examples of enzymic specific cleavage agents include without limitation endonucleases (e.g., DNase (e.g., DNase I, II); RNase (e.g., RNase E, F, H, P); Cleavase™ enzyme; Taq DNA polymerase; E. coli DNA polymerase I and eukaryotic structure-specific endonucleases; murine FEN-1 endonucleases; type I, II or III restriction endonucleases such as Acc I, Afl III, Alu I, Alw44 I, Apa I, Asn I, Ava I, Ava II, BamH I, Ban II, Bcl I, Bgl I. Bgl II, Bln I, Bsm I, BssH II, BstE II, Cfo I, Cla I, Dde I, Dpn I, Dra I, EclX I, EcoR I, EcoR I, EcoR II, EcoR V, Hae II, Hae II, Hind II, Hind III, Hpa I, Hpa II, Kpn I, Ksp I, Mlu I, MluN I, Msp I, Nci I, Nco I, Nde I, Nde II, Nhe I, Not I, Nru I, Nsi I, Pst I, Pvu I, Pvu II, Rsa I, Sac I, Sal I, Sau3A I, Sca I, ScrF I, Sfi I, Sma I, Spe I, Sph I, Ssp I, Stu I, Sty I, Swa I, Taq I, Xba I, Xho I); glycosylases (e.g., uracil-DNA glycolsylase (UDG), 3-methyladenine DNA glycosylase, 3-methyladenine DNA glycosylase II, pyrimidine hydrate-DNA glycosylase, FaPy-DNA glycosylase, thymine mismatch-DNA glycosylase, hypoxanthine-DNA glycosylase, 5-Hydroxymethyluracil DNA glycosylase (HmUDG), 5-Hydroxymethylcytosine DNA glycosylase, or 1,N6-etheno-adenine DNA glycosylase); exonucleases (e.g., exonuclease III); ribozymes, and DNAzymes. Sample nucleic acid may be treated with a chemical agent, or synthesized using modified nucleotides, and the modified nucleic acid may be cleaved. In non-limiting examples, sample nucleic acid may be treated with (i) alkylating agents such as methylnitrosourea that generate several alkylated bases, including N3-methyladenine and N3-methylguanine, which are recognized and cleaved by alkyl purine DNA-glycosylase; (ii) sodium bisulfite, which causes deamination of cytosine residues in DNA to form uracil residues that can be cleaved by uracil N-glycosylase; and (iii) a chemical agent that converts guanine to its oxidized form, 8-hydroxyguanine, which can be cleaved by formamidopyrimidine DNA N-glycosylase. Examples of chemical cleavage processes include without limitation alkylation, (e.g., alkylation of phosphorothioate-modified nucleic acid); cleavage of acid lability of P3'-N5'-phosphoroamidate-containing nucleic acid; and osmium tetroxide and piperidine treatment of nucleic acid.

As used herein, the term "complementary cleavage reactions" refers to cleavage reactions that are carried out on the same nucleic acid using different cleavage reagents or by altering the cleavage specificity of the same cleavage reagent such that alternate cleavage patterns of the same target or reference nucleic acid or protein are generated. In certain embodiments, nucleic acids of interest may be treated with one or more specific cleavage agents (e.g., 1, 2, 3, 4, 5, 6, 7, 8, 9, 10 or more specific cleavage agents) in one or more reaction vessels (e.g., nucleic acid of interest is treated with each specific cleavage agent in a separate vessel).

A nucleic acid suitable for use in the embodiments described herein sometimes is amplified by any amplification process known in the art (e.g., PCR, RT-PCR and the like). Nucleic acid amplification may be particularly beneficial when using organisms that are typically difficult to culture (e.g., slow growing, require specialize culture conditions and the like). The terms "amplify", "amplification", "amplification reaction", or "amplifying" as used herein refer to any in vitro processes for multiplying the copies of a target sequence of nucleic acid. Amplification sometimes refers to an "exponential" increase in target nucleic acid. However, "amplifying" as used herein can also refer to linear increases in the numbers of a select target sequence of nucleic acid, but is different than a one-time, single primer extension step. In some embodiments, a limited amplification reaction, also known as pre-amplification, can be performed. Pre-amplification is a method in which a limited amount of amplification occurs due to a small number of cycles, for example 10 cycles, being performed. Pre-amplification can allow some amplification, but stops amplification prior to the exponential phase, and typically produces about 500 copies of the desired nucleotide sequence(s). Use of pre-amplification may also limit inaccuracies associated with depleted reactants in standard PCR reactions.

In some embodiments, a nucleic acid reagent sometimes is stably integrated into the chromosome of the host organism, or a nucleic acid reagent can be a deletion of a portion of the host chromosome, in certain embodiments (e.g., genetically modified organisms, where alteration of the host genome confers the ability to selectively or preferentially maintain the desired organism carrying the genetic modification). Such nucleic acid reagents (e.g., nucleic acids or genetically modified organisms whose altered genome confers a selectable trait to the organism) can be selected for their ability to guide production of a desired protein or nucleic acid molecule. When desired, the nucleic acid reagent can be altered such that codons encode for (i) the same amino acid, using a different tRNA than that specified in the native sequence, or (ii) a different amino acid than is normal, including unconventional or unnatural amino acids (including detectably labeled amino acids). As described herein, the term "native sequence" refers to an unmodified nucleotide sequence as found in its natural setting (e.g., a nucleotide sequence as found in an organism).

A nucleic acid or nucleic acid reagent can comprise certain elements often selected according to the intended use of the nucleic acid. Any of the following elements can be included in or excluded from a nucleic acid reagent. A nucleic acid reagent, for example, may include one or more or all of the following nucleotide elements: one or more promoter elements, one or more 5' untranslated regions (5'UTRs), one or more regions into which a target nucleotide sequence may be inserted (an "insertion element"), one or more target nucleotide sequences, one or more 3' untranslated regions (3'UTRs), and one or more selection elements. A nucleic acid reagent can be provided with one or more of such elements and other elements may be inserted into the nucleic acid before the nucleic acid is introduced into the desired organism. In some embodiments, a provided nucleic acid reagent comprises a promoter, 5'UTR, optional 3'UTR and insertion element(s) by which a target nucleotide sequence is inserted (i.e., cloned) into the nucleotide acid reagent. In certain embodiments, a provided nucleic acid reagent comprises a promoter, insertion element(s) and optional 3'UTR, and a 5' UTR/target nucleotide sequence is inserted with an optional 3'UTR. The elements can be arranged in any order suitable for expression in the chosen expression system (e.g., expression in a chosen organism, or expression in a cell free system, for example), and in some embodiments a nucleic acid reagent comprises the following elements in the 5' to 3' direction: (1) promoter element, 5'UTR, and insertion element(s); (2) promoter element, 5'UTR, and target nucleotide sequence; (3) promoter element, 5'UTR, insertion element(s) and 3'UTR; and (4) promoter element, 5'UTR, target nucleotide sequence and 3'UTR.

A promoter element typically is required for DNA synthesis and/or RNA synthesis. A promoter element often comprises a region of DNA that can facilitate the transcription of a particular gene, by providing a start site for the synthesis of RNA corresponding to a gene. Promoters generally are located near the genes they regulate, are located upstream of the gene (e.g., 5' of the gene), and are on the same strand of DNA as the sense strand of the gene, in some embodiments. In some embodiments, a promoter element can be isolated from a gene or organism and inserted in functional connection with a polynucleotide sequence to allow altered and/or regulated expression. A non-native promoter (e.g., promoter not normally associated with a given nucleic acid sequence) used for expression of a nucleic acid often is referred to as a heterologous promoter. In certain embodiments, a heterologous promoter and/or a 5'UTR can be inserted in functional connection with a polynucleotide that encodes a polypeptide having a desired activity as described herein. The terms "operably linked" and "in functional connection with" as used herein with respect to promoters, refer to a relationship between a coding sequence and a promoter element. The promoter is operably linked or in functional connection with the coding sequence when expression from the coding sequence via transcription is regulated, or controlled by, the promoter element. The terms "operably linked" and "in functional connection with" are utilized interchangeably herein with respect to promoter elements.

A promoter often interacts with a RNA polymerase. A polymerase is an enzyme that catalyses synthesis of nucleic acids using a preexisting nucleic acid reagent. When the template is a DNA template, an RNA molecule is transcribed before protein is synthesized. Enzymes having polymerase activity suitable for use in the present methods include any polymerase that is active in the chosen system with the chosen template to synthesize protein. In some embodiments, a promoter (e.g., a heterologous promoter) also referred to herein as a promoter element, can be operably linked to a nucleotide sequence or an open reading frame (ORF). Transcription from the promoter element can catalyze the synthesis of an RNA corresponding to the nucleotide sequence or ORF sequence operably linked to the promoter, which in turn leads to synthesis of a desired peptide, polypeptide or protein.

Promoter elements sometimes exhibit responsiveness to regulatory control. Promoter elements also sometimes can be regulated by a selective agent. That is, transcription from promoter elements sometimes can be turned on, turned off, up-regulated or down-regulated, in response to a change in environmental, nutritional or internal conditions or signals (e.g., heat inducible promoters, light regulated promoters, feedback regulated promoters, hormone influenced promoters, tissue specific promoters, oxygen and pH influenced promoters, promoters that are responsive to selective agents (e.g., kanamycin) and the like, for example). Promoters influenced by environmental, nutritional or internal signals frequently are influenced by a signal (direct or indirect) that binds at or near the promoter and increases or decreases expression of the target sequence under certain conditions.

Non-limiting examples of selective or regulatory agents that can influence transcription from a promoter element used in embodiments described herein include, without limitation, (1) nucleic acid segments that encode products that provide resistance against otherwise toxic compounds (e.g., antibiotics); (2) nucleic acid segments that encode products that are otherwise lacking in the recipient cell (e.g., essential products, tRNA genes, auxotrophic markers); (3) nucleic acid segments that encode products that suppress the activity of a gene product; (4) nucleic acid segments that encode products that can be readily identified (e.g., phenotypic markers such as antibiotics (e.g., β-lactamase), β-galactosidase, green fluorescent protein (GFP), yellow fluorescent protein (YFP), red fluorescent protein (RFP), cyan fluorescent protein (CFP), and cell surface proteins); (5) nucleic acid segments that bind products that are otherwise detrimental to cell survival and/or function; (6) nucleic acid segments that otherwise inhibit the activity of any of the nucleic acid segments described in Nos. 1-5 above (e.g., antisense oligonucleotides); (7) nucleic acid segments that bind products that modify a substrate (e.g., restriction endonucleases); (8) nucleic acid segments that can be used to isolate or identify a desired molecule (e.g., specific protein binding sites); (9) nucleic acid segments that encode a specific nucleotide sequence that can be otherwise non-functional (e.g., for PCR amplification of subpopulations of molecules); (10) nucleic acid segments that, when absent, directly or indirectly confer resistance or sensitivity to particular compounds; (11) nucleic acid segments that encode products that either are toxic or convert a relatively non-toxic compound to a toxic compound (e.g., Herpes simplex thymidine kinase, cytosine deaminase) in recipient cells; (12) nucleic acid segments that inhibit replication, partition or heritability of nucleic acid molecules that contain them; and/or (13) nucleic acid segments that encode conditional replication functions, e.g., replication in certain hosts or host cell strains or under certain environmental conditions (e.g., temperature, nutritional conditions, and the like). In some embodiments, the regulatory or selective agent can be added to change the existing growth conditions to which the organism is subjected (e.g., growth in liquid culture, growth in a fermentor, growth on solid nutrient plates and the like for example).

In some embodiments, regulation of a promoter element can be used to alter (e.g., increase, add, decrease or substantially eliminate) the activity of a peptide, polypeptide or protein (e.g., enzyme activity for example). For example, a microorganism can be engineered by genetic modification to express a nucleic acid reagent that can add a novel activity (e.g., an activity not normally found in the host organism) or increase the expression of an existing activity by increasing transcription from a homologous or heterologous promoter operably linked to a nucleotide sequence of interest (e.g., homologous or heterologous nucleotide sequence of interest), in certain embodiments. In some embodiments, a microorganism can be engineered by genetic modification to express a nucleic acid reagent that can decrease expression of an activity by decreasing or substantially eliminating transcription from a homologous or heterologous promoter operably linked to a nucleotide sequence of interest, in certain embodiments.

In some embodiments the activity can be altered using recombinant DNA and genetic techniques known to the artisan. Methods for engineering microorganisms are further described herein. Tables herein provide non-limiting lists of yeast promoters that are up-regulated by oxygen, yeast promoters that are down-regulated by oxygen, yeast transcriptional repressors and their associated genes, DNA binding motifs as determined using the MEME sequence analysis software. Potential regulator binding motifs can be identified using the program MEME to search intergenic regions bound by regulators for overrepresented sequences. For each regulator, the sequences of intergenic regions bound with p-values less than 0.001 were extracted to use as input for motif discovery. The MEME software was run using the following settings: a motif width ranging from 6 to 18 bases, the "zoops" distribution model, a 6^{th} order Markov background model and a discovery limit of 20 motifs. The discovered sequence motifs were scored for significance by two criteria: an E-value calculated by MEME and a specificity score. The motif with the best score using each metric is shown for each regulator. All motifs presented are derived from datasets generated in rich growth conditions with the exception of a previously published dataset for epitope-tagged Gal4 grown in galactose.

In some embodiments, the altered activity can be found by screening the organism under conditions that select for the desired change in activity. For example, certain microorganisms can be adapted to increase or decrease an activity by selecting or screening the organism in question on a media containing substances that are poorly metabolized or even toxic. An increase in the ability of an organism to grow a substance that is normally poorly metabolized would result in an increase in the growth rate on that substance, for example. A decrease in the sensitivity to a toxic substance might be manifested by growth on higher concentrations of the toxic substance, for example. Genetic modifications that are identified in this manner sometimes are referred to as naturally occurring mutations or the organisms that carry them can sometimes be referred to as naturally occurring mutants. Modifications obtained in this manner are not limited to alterations in promoter sequences. That is, screening microorganisms by selective pressure, as described above, can yield genetic alterations that can occur in non-promoter sequences, and sometimes also can occur in sequences that are not in the nucleotide sequence of interest, but in a related nucleotide sequences (e.g., a gene involved in a different step of the same pathway, a transport gene, and the like). Naturally occurring mutants sometimes can be found by isolating naturally occurring variants from unique environments, in some embodiments.

In addition to the regulated promoter sequences, regulatory sequences, and coding polynucleotides provided herein, a nucleic acid reagent may include a polynucleotide sequence 80% or more identical to the foregoing (or to the complementary sequences). That is, a nucleotide sequence that is at least 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more identical to a nucleotide sequence described herein can be utilized. The term "identical" as used herein refers to two or more nucleotide sequences having substantially the same nucleotide sequence when compared to each other. One test for determining whether two nucleotide sequences or amino acids sequences are substantially identical is to determine the percent of identical nucleotide sequences or amino acid sequences shared.

Calculations of sequence identity can be performed as follows. Sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is sometimes 30% or more, 40% or more, 50% or more, often 60% or more, and more often 70% or more, 80% or more, 90% or more, or 100% of the length of the reference sequence. The nucleotides or amino acids at corresponding nucleotide or polypeptide positions, respectively, are then compared among the two sequences. When a position in the first sequence is occupied by the same nucleotide or amino acid as the corresponding position in the second sequence, the nucleotides or amino acids are deemed to be identical at that position. The percent identity between the two sequences is a function of the number of identical positions shared by the sequences, taking into account the number of gaps, and the length of each gap, introduced for optimal alignment of the two sequences.

Comparison of sequences and determination of percent identity between two sequences can be accomplished using a mathematical algorithm. Percent identity between two amino acid or nucleotide sequences can be determined using the algorithm of Meyers & Miller, CABIOS 4: 11-17 (1989), which has been incorporated into the ALIGN program (version 2.0), using a PAM120 weight residue table, a gap length penalty of 12 and a gap penalty of 4. Also, percent identity between two amino acid sequences can be determined using the Needleman & Wunsch, J. Mol. Biol. 48: 444-453 (1970) algorithm which has been incorporated into the GAP program in the GCG software package (available at the http address www.gcg.com), using either a Blossum 62 matrix or a PAM250 matrix, and a gap weight of 16, 14, 12, 10, 8, 6, or 4 and a length weight of 1, 2, 3, 4, 5, or 6. Percent identity between two nucleotide sequences can be determined using the GAP program in the GCG software package (available at http address www.gcg.com), using a NWSgapdna.CMP matrix and a gap weight of 40, 50, 60, 70, or 80 and a length weight of 1, 2, 3, 4, 5, or 6. A set of parameters often used is a Blossum 62 scoring matrix with a gap open penalty of 12, a gap extend penalty of 4, and a frameshift gap penalty of 5.

Sequence identity can also be determined by hybridization assays conducted under stringent conditions. As use herein, the term "stringent conditions" refers to conditions for hybridization and washing. Stringent conditions are known to those skilled in the art and can be found in Current Protocols in Molecular Biology, John Wiley & Sons, N.Y., 6.3.1-6.3.6 (1989). Aqueous and nonaqueous methods are described in that reference and either can be used. An example of stringent hybridization conditions is hybridization in 6X sodium chloride/sodium citrate (SSC) at about 45ºC, followed by one or more washes in 0.2X SSC, 0.1% SDS at 50ºC. Another example of stringent hybridization conditions are hybridization in 6X sodium chloride/sodium citrate (SSC) at about 45ºC, followed by one or more washes in 0.2X SSC, 0.1% SDS at 55ºC. A further example of stringent hybridization conditions is hybridization in 6X sodium chloride/sodium citrate (SSC) at about 45ºC, followed by one or more washes in 0.2X SSC, 0.1% SDS at 60ºC. Often, stringent hybridization conditions are hybridization in 6X sodium chloride/sodium citrate (SSC) at about 45ºC, followed by one or more washes in 0.2X SSC, 0.1% SDS at 65ºC. More often, stringency conditions are 0.5M sodium phosphate, 7% SDS at 65ºC, followed by one or more washes at 0.2X SSC, 1% SDS at 65ºC.

As noted above, nucleic acid reagents may also comprise one or more 5' UTR's, and one or more 3'UTR's. A 5' UTR may comprise one or more elements endogenous to the nucleotide sequence from which it originates, and sometimes includes one or more exogenous elements. A 5' UTR can originate from any suitable nucleic acid, such as genomic DNA, plasmid DNA, RNA or mRNA, for example, from any suitable organism (e.g., virus, bacterium, yeast, fungi, plant, insect or mammal). The artisan may select appropriate elements for the 5' UTR based upon the chosen expression system (e.g., expression in a chosen organism, or expression in a cell free system, for example). A 5' UTR sometimes comprises one or more of the following elements known to the artisan: enhancer sequences (e.g., transcriptional or translational), transcription initiation site, transcription factor binding site, translation regulation site, translation initiation site, translation factor binding site, accessory protein binding site, feedback regulation agent binding sites, Pribnow box, TATA box, -35 element, E-box (helix-loop-helix binding element), ribosome binding site, replicon, internal ribosome entry site (IRES), silencer element and the like. In some embodiments, a promoter element may be isolated such that all 5' UTR elements necessary for proper conditional regulation are contained in the promoter element fragment, or within a functional subsequence of a promoter element fragment.

A 5 'UTR in the nucleic acid reagent can comprise a translational enhancer nucleotide sequence. A translational enhancer nucleotide sequence often is located between the promoter and the target nucleotide sequence in a nucleic acid reagent. A translational enhancer sequence often binds to a ribosome, sometimes is an 18S rRNA-binding ribonucleotide sequence (i.e., a 40S ribosome binding sequence) and sometimes is an internal ribosome entry sequence (IRES). An IRES generally forms an RNA scaffold with precisely placed RNA tertiary structures that contact a 40S ribosomal subunit via a number of specific intermolecular interactions. Examples of ribosomal enhancer sequences are known and can be identified by the artisan (e.g., Mignone et al., Nucleic Acids Research 33: D141-D146 (2005); Paulous et al., Nucleic Acids Research 31: 722-733 (2003); Akbergenov et al., Nucleic Acids Research 32: 239-247 (2004); Mignone et al., Genome Biology 3(3): reviews0004.1-0001.10 (2002); Gallie, Nucleic Acids Research 30: 3401-3411 (2002); Shaloiko et al., http address www.interscience.wiley.com, DOI: 10.1002/bit.20267; and Gallie et al., Nucleic Acids Research 15: 3257-3273 (1987)).

A translational enhancer sequence sometimes is a eukaryotic sequence, such as a Kozak consensus sequence or other sequence (e.g., hydroid polyp sequence, GenBank accession no. U07128). A translational enhancer sequence sometimes is a prokaryotic sequence, such as a Shine-Dalgarno consensus sequence. In certain embodiments, the translational enhancer sequence is a viral nucleotide sequence. A translational enhancer sequence sometimes is from a 5' UTR of a plant virus, such as Tobacco Mosaic Virus (TMV), Alfalfa Mosaic Virus (AMV); Tobacco Etch Virus (ETV); Potato Virus Y (PVY); Turnip Mosaic (poty) Virus and Pea Seed Borne Mosaic Virus, for example. In certain embodiments, an omega sequence about 67 bases in length from TMV is included in the nucleic acid reagent as a translational enhancer sequence (e.g., devoid of guanosine nucleotides and includes a 25 nucleotide long poly (CAA) central region).

A 3' UTR may comprise one or more elements endogenous to the nucleotide sequence from which it originates and sometimes includes one or more exogenous elements. A 3' UTR may originate from any suitable nucleic acid, such as genomic DNA, plasmid DNA, RNA or mRNA, for example, from any suitable organism (e.g., a virus, bacterium, yeast, fungi, plant, insect or mammal). The artisan can select appropriate elements for the 3' UTR based upon the chosen expression system (e.g., expression in a chosen organism, for example). A 3' UTR sometimes comprises one or more of the following elements known to the artisan: transcription regulation site, transcription initiation site, transcription termination site, transcription factor binding site, translation regulation site, translation termination site, translation initiation site, translation factor binding site, ribosome binding site, replicon, enhancer element, silencer element and polyadenosine tail. A 3' UTR often includes a polyadenosine tail and sometimes does not, and if a polyadenosine tail is present, one or more adenosine moieties may be added or deleted from it (e.g., about 5, about 10, about 15, about 20, about 25, about 30, about 35, about 40, about 45 or about 50 adenosine moieties may be added or subtracted).

In some embodiments, modification of a 5' UTR and/or a 3' UTR can be used to alter (e.g., increase, add, decrease or substantially eliminate) the activity of a promoter. Alteration of the promoter activity can in turn alter the activity of a peptide, polypeptide or protein (e.g., enzyme activity for example), by a change in transcription of the nucleotide sequence(s) of interest from an operably linked promoter element comprising the modified 5' or 3' UTR. For example, a microorganism can be engineered by genetic modification to express a nucleic acid reagent comprising a modified 5' or 3' UTR that can add a novel activity (e.g., an activity not normally found in the host organism) or increase the expression of an existing activity by increasing transcription from a homologous or heterologous promoter operably linked to a nucleotide sequence of interest (e.g., homologous or heterologous nucleotide sequence of interest), in certain embodiments. In some embodiments, a microorganism can be engineered by genetic modification to express a nucleic acid reagent comprising a modified 5' or 3' UTR that can decrease the expression of an activity by decreasing or substantially eliminating transcription from a homologous or heterologous promoter operably linked to a nucleotide sequence of interest, in certain embodiments.

A nucleotide reagent sometimes can comprise a target nucleotide sequence. A "target nucleotide sequence" as used herein encodes a nucleic acid, peptide, polypeptide or protein of interest, and may be a ribonucleotide sequence or a deoxyribonucleotide sequence. A target nucleic acid sometimes is an untranslated ribonucleic acid and sometimes is a translated ribonucleic acid. An untranslated ribonucleic acid may include, but is not limited to, a small interfering ribonucleic acid (siRNA), a short hairpin ribonucleic acid (shRNA), other ribonucleic acid capable of RNA interference (RNAi), an antisense ribonucleic acid, or a ribozyme. A translatable target nucleotide sequence (e.g., a target ribonucleotide sequence) sometimes encodes a peptide, polypeptide or protein, which are sometimes referred to herein as "target peptides," "target polypeptides" or "target proteins."

Any peptides, polypeptides or proteins, or an activity catalyzed by one or more peptides, polypeptides or proteins may be encoded by a target nucleotide sequence and may be selected by a user. Representative proteins include enzymes (e.g., glucose-6-phosphate dehydrogenase, lipase, fatty acid synthase acetyl-CoA carboxylase, acyl-CoA oxidase, hexanoate synthase, thioesterase, monooxygenase, monooxygenase reductase, fatty alcohol oxidase, 6-oxohexanoic acid deydrogenase, 6-hydroxyhexanoic acid dehydrogenase and the like, for example), antibodies, serum proteins (e.g., albumin), membrane bound proteins, hormones (e.g., growth hormone, erythropoietin, insulin, etc.), cytokines, etc., and include both naturally occurring and exogenously expressed polypeptides. Representative activities (e.g., enzymes or combinations of enzymes which are functionally associated to provide an activity) include hexanoate synthase activity, thioesterase activity, monooxygenase activity, 6-oxohexanoic acid deydrogenase activity, 6-hydroxyhexanoic acid dehydrogenase activity, beta-oxidation activity and the like, for example. The term "enzyme" as used herein refers to a protein which can act as a catalyst to induce a chemical change in other compounds, thereby producing one or more products from one or more substrates.

Specific polypeptides (e.g., enzymes) useful for embodiments described herein are listed herein. The term "protein" as used herein refers to a molecule having a sequence of amino acids linked by peptide bonds. This term includes fusion proteins, oligopeptides, peptides, cyclic peptides, polypeptides and polypeptide derivatives, whether native or recombinant, and also includes fragments, derivatives, homologs, and variants thereof. A protein or polypeptide sometimes is of intracellular origin (e.g., located in the nucleus, cytosol, or interstitial space of host cells in vivo) and sometimes is a cell membrane protein in vivo. In some embodiments (described above, and in further detail hereafter in Engineering and Alteration Methods), a genetic modification can result in a modification (e.g., increase, substantially increase, decrease or substantially decrease) of a target activity.

A translatable nucleotide sequence generally is located between a start codon (AUG in ribonucleic acids and ATG in deoxyribonucleic acids) and a stop codon (e.g., UAA (ochre), UAG (amber) or UGA (opal) in ribonucleic acids and TAA, TAG or TGA in deoxyribonucleic acids), and sometimes is referred to herein as an "open reading frame" (ORF). A translatable nucleotide sequence (e.g., ORF) sometimes is encoded differently in one organism (e.g., most organisms encode CTG as leucine) than in another organism (e.g., C. tropicalis encodes CTG as serine). In some embodiments, a translatable nucleotide sequence is altered to correct alternate genetic code (e.g., codon usage) differences between a nucleotide donor organism and an nucleotide recipient organism (e.g., engineered organism). In certain embodiments, a translatable nucleotide sequence is altered to improve; (i) codon usage, (ii) transcriptional efficiency, (iii) translational efficiency, (iv) the like, and combinations thereof.

A nucleic acid reagent sometimes comprises one or more ORFs. An ORF may be from any suitable source, sometimes from genomic DNA, mRNA, reverse transcribed RNA or complementary DNA (cDNA) or a nucleic acid library comprising one or more of the foregoing, and is from any organism species that contains a nucleic acid sequence of interest, protein of interest, or activity of interest. Non-limiting examples of organisms from which an ORF can be obtained include bacteria, yeast, fungi, human, insect, nematode, bovine, equine, canine, feline, rat or mouse, for example.

A nucleic acid reagent sometimes comprises a nucleotide sequence adjacent to an ORF that is translated in conjunction with the ORF and encodes an amino acid tag. The tag-encoding nucleotide sequence is located 3' and/or 5' of an ORF in the nucleic acid reagent, thereby encoding a tag at the C-terminus or N-terminus of the protein or peptide encoded by the ORF. Any tag that does not abrogate in vitro transcription and/or translation may be utilized and may be appropriately selected by the artisan. Tags may facilitate isolation and/or purification of the desired ORF product from culture or fermentation media.

A tag sometimes specifically binds a molecule or moiety of a solid phase or a detectable label, for example, thereby having utility for isolating, purifying and/or detecting a protein or peptide encoded by the ORF. In some embodiments, a tag comprises one or more of the following elements: FLAG (e.g., DYKDDDDKG), V5 (e.g., GKPIPNPLLGLDST), c-MYC (e.g., EQKLISEEDL), HSV (e.g., QPELAPEDPED), influenza hemaglutinin, HA (e.g., YPYDVPDYA), VSV-G (e.g., YTDIEMNRLGK), bacterial glutathione-S-transferase, maltose binding protein, a streptavidin- or avidin-binding tag (e.g., pcDNA™6 BioEase™ Gateway® Biotinylation System (Invitrogen)), thioredoxin, β-galactosidase, VSV-glycoprotein, a fluorescent protein (e.g., green fluorescent protein or one of its many color variants (e.g., yellow, red, blue)), a polylysine or polyarginine sequence, a polyhistidine sequence (e.g., His6) or other sequence that chelates a metal (e.g., cobalt, zinc, copper), and/or a cysteine-rich sequence that binds to an arsenic-containing molecule. In certain embodiments, a cysteine-rich tag comprises the amino acid sequence CC-Xn-CC, wherein X is any amino acid and n is 1 to 3, and the cysteine-rich sequence sometimes is CCPGCC. In certain embodiments, the tag comprises a cysteine-rich element and a polyhistidine element (e.g., CCPGCC and His6).

A tag often conveniently binds to a binding partner. For example, some tags bind to an antibody (e.g., FLAG) and sometimes specifically bind to a small molecule. For example, a polyhistidine tag specifically chelates a bivalent metal, such as copper, zinc and cobalt; a polylysine or polyarginine tag specifically binds to a zinc finger; a glutathione S-transferase tag binds to glutathione; and a cysteine-rich tag specifically binds to an arsenic-containing molecule. Arsenic-containing molecules include LUMIO™ agents (Invitrogen, California), such as FlAsH™ (EDT2[4',5'-bis(1,3,2-dithioarsolan-2-yl)fluorescein-(1,2-ethanedithiol)2]) and ReAsH reagents (e.g., U.S. Patent 5,932,474 to Tsien et al., entitled "Target Sequences for Synthetic Molecules;" U.S. Patent 6,054,271 to Tsien et al., entitled "Methods of Using Synthetic Molecules and Target Sequences;" U.S. Patents 6,451,569 and 6,008,378; published U.S. Patent Application 2003/0083373, and published PCT Patent Application WO 99/21013, all to Tsien et al. and all entitled "Synthetic Molecules that Specifically React with Target Sequences"). Such antibodies and small molecules sometimes are linked to a solid phase for convenient isolation of the target protein or target peptide.

A tag sometimes comprises a sequence that localizes a translated protein or peptide to a component in a system, which is referred to as a "signal sequence" or "localization signal sequence" herein. A signal sequence often is incorporated at the N-terminus of a target protein or target peptide, and sometimes is incorporated at the C-terminus. Examples of signal sequences are known to the artisan, are readily incorporated into a nucleic acid reagent, and often are selected according to the organism in which expression of the nucleic acid reagent is performed. A signal sequence in some embodiments localizes a translated protein or peptide to a cell membrane. Examples of signal sequences include, but are not limited to, a nucleus targeting signal (e.g., steroid receptor sequence and N-terminal sequence of SV40 virus large T antigen); mitochondrial targeting signal (e.g., amino acid sequence that forms an amphipathic helix); peroxisome targeting signal (e.g., C-terminal sequence in YFG from S.cerevisiae); and a secretion signal (e.g., N-terminal sequences from invertase, mating factor alpha, PHO5 and SUC2 in S.cerevisiae; multiple N-terminal sequences of B. subtilis proteins (e.g., Tjalsma et al., Microbiol.Molec. Biol. Rev. 64: 515-547 (2000)); alpha amylase signal sequence (e.g., U.S. Patent No. 6,288,302); pectate lyase signal sequence (e.g., U.S. Patent No. 5,846,818); precollagen signal sequence (e.g., U.S. Patent No. 5,712,114); OmpA signal sequence (e.g., U.S. Patent No. 5,470,719); lam beta signal sequence (e.g., U.S. Patent No. 5,389,529); B. brevis signal sequence (e.g., U.S. Patent No. 5,232,841); and P. pastoris signal sequence (e.g., U.S. Patent No. 5,268,273)).

A tag sometimes is directly adjacent to the amino acid sequence encoded by an ORF (i.e., there is no intervening sequence) and sometimes a tag is substantially adjacent to an ORF encoded amino acid sequence (e.g., an intervening sequence is present). An intervening sequence sometimes includes a recognition site for a protease, which is useful for cleaving a tag from a target protein or peptide. In some embodiments, the intervening sequence is cleaved by Factor Xa (e.g., recognition site I (E/D)GR), thrombin (e.g., recognition site LVPRGS), enterokinase (e.g., recognition site DDDDK), TEV protease (e.g., recognition site ENLYFQG) or PreScission™ protease (e.g., recognition site LEVLFQGP), for example.

An intervening sequence sometimes is referred to herein as a "linker sequence," and may be of any suitable length selected by the artisan. A linker sequence sometimes is about 1 to about 20 amino acids in length, and sometimes about 5 to about 10 amino acids in length. The artisan may select the linker length to substantially preserve target protein or peptide function (e.g., a tag may reduce target protein or peptide function unless separated by a linker), to enhance disassociation of a tag from a target protein or peptide when a protease cleavage site is present (e.g., cleavage may be enhanced when a linker is present), and to enhance interaction of a tag/target protein product with a solid phase. A linker can be of any suitable amino acid content, and often comprises a higher proportion of amino acids having relatively short side chains (e.g., glycine, alanine, serine and threonine).

A nucleic acid reagent sometimes includes a stop codon between a tag element and an insertion element or ORF, which can be useful for translating an ORF with or without the tag. Mutant tRNA molecules that recognize stop codons (described above) suppress translation termination and thereby are designated "suppressor tRNAs." Suppressor tRNAs can result in the insertion of amino acids and continuation of translation past stop codons (e.g., U.S. Patent Application No. 60/587,583, filed July 14, 2004, entitled "Production of Fusion Proteins by Cell-Free Protein Synthesis,"; Eggertsson, et al., (1988) Microbiological Review 52(3):354-374, and Engleerg-Kukla, et al. (1996) in Escherichia coli and Salmonella Cellular and Molecular Biology, Chapter 60, pps 909-921, Neidhardt, et al. eds., ASM Press, Washington, DC). A number of suppressor tRNAs are known, including but not limited to, supE, supP, supD, supF and supZ suppressors, which suppress the termination of translation of the amber stop codon; supB, gIT, supL, supN, supC and supM suppressors, which suppress the function of the ochre stop codon and glyT, trpT and Su-9 suppressors, which suppress the function of the opal stop codon. In general, suppressor tRNAs contain one or more mutations in the anti-codon loop of the tRNA that allows the tRNA to base pair with a codon that ordinarily functions as a stop codon. The mutant tRNA is charged with its cognate amino acid residue and the cognate amino acid residue is inserted into the translating polypeptide when the stop codon is encountered. Mutations that enhance the efficiency of termination suppressors (i.e., increase stop codon read-through) have been identified. These include, but are not limited to, mutations in the uar gene (also known as the prfA gene), mutations in the ups gene, mutations in the sueA, sueB and sueC genes, mutations in the rpsD (ramA) and rpsE (spcA) genes and mutations in the rplL gene.

Thus, a nucleic acid reagent comprising a stop codon located between an ORF and a tag can yield a translated ORF alone when no suppressor tRNA is present in the translation system, and can yield a translated ORF-tag fusion when a suppressor tRNA is present in the system. Suppressor tRNA can be generated in cells transfected with a nucleic acid encoding the tRNA (e.g., a replication incompetent adenovirus containing the human tRNA-Ser suppressor gene can be transfected into cells, or a YAC containing a yeast or bacterial tRNA suppressor gene can be transfected into yeast cells, for example). Vectors for synthesizing suppressor tRNA and for translating ORFs with or without a tag are available to the artisan (e.g., Tag-On-Demand™ kit (Invitrogen Corporation, California); Tag-On-Demand™ Suppressor Supernatant Instruction Manual, Version B, 6 June 2003, at http address www.invitrogen.com/content/sfs/ manuals/tagondemand _supernatant_man.pdf; Tag-On-Demand™ Gateway® Vector Instruction Manual, Version B, 20 June, 2003 at http address www.invitrogen.com/content/sfs/ manuals/tagondemand_vectors_man.pdf; and Capone et al., Amber, ochre and opal suppressor tRNA genes derived from a human serine tRNA gene. EMBO J. 4:213, 1985).

Any convenient cloning strategy known in the art may be utilized to incorporate an element, such as an ORF, into a nucleic acid reagent. Known methods can be utilized to insert an element into the template independent of an insertion element, such as (1) cleaving the template at one or more existing restriction enzyme sites and ligating an element of interest and (2) adding restriction enzyme sites to the template by hybridizing oligonucleotide primers that include one or more suitable restriction enzyme sites and amplifying by polymerase chain reaction (described in greater detail herein). Other cloning strategies take advantage of one or more insertion sites present or inserted into the nucleic acid reagent, such as an oligonucleotide primer hybridization site for PCR, for example, and others described herein. In some embodiments, a cloning strategy can be combined with genetic manipulation such as recombination (e.g., recombination of a nucleic acid reagent with a nucleic acid sequence of interest into the genome of the organism to be modified, as described further herein). In some embodiments, the cloned ORF(s) can produce (directly or indirectly) adipic acid, by engineering a microorganism with one or more ORFs of interest, which microorganism comprises one or more altered activities selected from the group consisting of 6-oxohexanoic acid dehydrogenase activity, 6-hydroxyhexanoic acid dehydrogenase activity, glucose-6-phosphate dehydrogenase activity, hexanoate synthase activity, lipase activity, fatty acid synthase activity, acetyl CoA carboxylase activity, monooxygenase activity and monooxygenase reductase activity.

In some embodiments, the nucleic acid reagent includes one or more recombinase insertion sites. A recombinase insertion site is a recognition sequence on a nucleic acid molecule that participates in an integration/recombination reaction by recombination proteins. For example, the recombination site for Cre recombinase is loxP, which is a 34 base pair sequence comprised of two 13 base pair inverted repeats (serving as the recombinase binding sites) flanking an 8 base pair core sequence (e.g., Figure 1 of Sauer, B., Curr. Opin. Biotech. 5:521-527 (1994)). Other examples of recombination sites include attB, attP, attL, and attR sequences, and mutants, fragments, variants and derivatives thereof, which are recognized by the recombination protein λ Int and by the auxiliary proteins integration host factor (IHF), FIS and excisionase (Xis) (e.g., U.S. Patent Nos. 5,888,732; 6,143,557; 6,171,861; 6,270,969; 6,277,608; and 6,720,140; U.S. Patent Appln. Nos. 09/517,466, filed March 2, 2000, and 09/732,914, filed August 14, 2003, and in U.S. patent publication no. 2002-0007051-A1; Landy, Curr. Opin. Biotech. 3:699-707 (1993)).

Examples of recombinase cloning nucleic acids are in Gateway® systems (Invitrogen, California), which include at least one recombination site for cloning a desired nucleic acid molecule in vivo or in vitro. In some embodiments, the system utilizes vectors that contain at least two different site-specific recombination sites, often based on the bacteriophage lambda system (e.g., att1 and att2), and are mutated from the wild-type (att0) sites. Each mutated site has a unique specificity for its cognate partner att site (i.e., its binding partner recombination site) of the same type (for example attB1 with attP1, or attL1 with attR1) and will not cross-react with recombination sites of the other mutant type or with the wild-type att0 site. Different site specificities allow directional cloning or linkage of desired molecules thus providing desired orientation of the cloned molecules. Nucleic acid fragments flanked by recombination sites are cloned and subcloned using the Gateway® system by replacing a selectable marker (for example, ccdB) flanked by att sites on the recipient plasmid molecule, sometimes termed the Destination Vector. Desired clones are then selected by transformation of a ccdB sensitive host strain and positive selection for a marker on the recipient molecule. Similar strategies for negative selection (e.g., use of toxic genes) can be used in other organisms such as thymidine kinase (TK) in mammals and insects.

A recombination system useful for engineering yeast is outlined briefly. The system makes use of the URA3 gene (e.g., for S. cerevisieae and C. albicans, for example) or URA4 and URA5 genes (e.g., for S. pombe, for example) and toxicity of the nucleotide analogue 5-Fluoroorotic acid (5-FOA). The URA3 or URA4 and URA5 genes encode orotine-5'-monophosphate (OMP) dicarboxylase. Yeast with an active URA3 or URA4 and URA5 gene (phenotypically Ura+) convert 5-FOA to fluorodeoxyuridine, which is toxic to yeast cells. Yeast carrying a mutation in the appropriate gene(s) or having a knock out of the appropriate gene(s) can grow in the presence of 5-FOA, if the media is also supplemented with uracil.

A nucleic acid engineering construct can be made which may comprise the URA3 gene or cassette (for S. cerevisieae), flanked on either side by the same nucleotide sequence in the same orientation. The URA3 cassette comprises a promoter, the URA3 gene and a functional transcription terminator. Target sequences which direct the construct to a particular nucleic acid region of interest in the organism to be engineered are added such that the target sequences are adjacent to and abut the flanking sequences on either side of the URA3 cassette. Yeast can be transformed with the engineering construct and plated on minimal media without uracil. Colonies can be screened by PCR to determine those transformants that have the engineering construct inserted in the proper location in the genome. Checking insertion location prior to selecting for recombination of the ura3 cassette may reduce the number of incorrect clones carried through to later stages of the procedure. Correctly inserted transformants can then be replica plated on minimal media containing 5-FOA to select for recombination of the URA3 cassette out of the construct, leaving a disrupted gene and an identifiable footprint (e.g., nucleic acid sequence) that can be use to verify the presence of the disrupted gene. The technique described is useful for disrupting or "knocking out" gene function, but also can be used to insert genes or constructs into a host organisms genome in a targeted, sequence specific manner.

In certain embodiments, a nucleic acid reagent includes one or more topoisomerase insertion sites. A topoisomerase insertion site is a defined nucleotide sequence recognized and bound by a site-specific topoisomerase. For example, the nucleotide sequence 5'-(C/T)CCTT-3' is a topoisomerase recognition site bound specifically by most poxvirus topoisomerases, including vaccinia virus DNA topoisomerase I. After binding to the recognition sequence, the topoisomerase cleaves the strand at the 3'-most thymidine of the recognition site to produce a nucleotide sequence comprising 5'-(C/T)CCTT-PO4-TOPO, a complex of the topoisomerase covalently bound to the 3' phosphate via a tyrosine in the topoisomerase (e.g., Shuman, J. Biol. Chem. 266:11372-11379, 1991; Sekiguchi and Shuman, Nucl. Acids Res. 22:5360-5365, 1994; U.S. Pat. No. 5,766,891; PCT/US95/16099; and PCT/US98/12372). In comparison, the nucleotide sequence 5'-GCAACTT-3' is a topoisomerase recognition site for type IA E. coli topoisomerase III. An element to be inserted often is combined with topoisomerase-reacted template and thereby incorporated into the nucleic acid reagent (e.g., World Wide Web URL invitrogen.com/downloads/F-13512_Topo_Flyer.pdf; World Wide Web URL invitrogen.com/content/sfs/brochures/ 710_021849%20_B_TOPOCloning_bro.pdf; TOPO TA Cloning® Kit and Zero Blunt® TOPO® Cloning Kit product information).

A nucleic acid reagent sometimes contains one or more origin of replication (ORI) elements. In some embodiments, a template comprises two or more ORIs, where one functions efficiently in one organism (e.g., a bacterium) and another functions efficiently in another organism (e.g., a eukaryote, like yeast for example). In some embodiments, an ORI may function efficiently in one species (e.g., S. cerevisieae, for example) and another ORI may function efficiently in a different species (e.g., S. pombe, for example). A nucleic acid reagent also sometimes includes one or more transcription regulation sites.

A nucleic acid reagent can include one or more selection elements (e.g., elements for selection of the presence of the nucleic acid reagent, and not for activation of a promoter element which can be selectively regulated). Selection elements often are utilized using known processes to determine whether a nucleic acid reagent is included in a cell. In some embodiments, a nucleic acid reagent includes two or more selection elements, where one functions efficiently in one organism and another functions efficiently in another organism. Examples of selection elements include, but are not limited to, (1) nucleic acid segments that encode products that provide resistance against otherwise toxic compounds (e.g., antibiotics); (2) nucleic acid segments that encode products that are otherwise lacking in the recipient cell (e.g., essential products, tRNA genes, auxotrophic markers); (3) nucleic acid segments that encode products that suppress the activity of a gene product; (4) nucleic acid segments that encode products that can be readily identified (e.g., phenotypic markers such as antibiotics (e.g., β-lactamase), β-galactosidase, green fluorescent protein (GFP), yellow fluorescent protein (YFP), red fluorescent protein (RFP), cyan fluorescent protein (CFP), and cell surface proteins); (5) nucleic acid segments that bind products that are otherwise detrimental to cell survival and/or function; (6) nucleic acid segments that otherwise inhibit the activity of any of the nucleic acid segments described in Nos. 1-5 above (e.g., antisense oligonucleotides); (7) nucleic acid segments that bind products that modify a substrate (e.g., restriction endonucleases); (8) nucleic acid segments that can be used to isolate or identify a desired molecule (e.g., specific protein binding sites); (9) nucleic acid segments that encode a specific nucleotide sequence that can be otherwise non-functional (e.g., for PCR amplification of subpopulations of molecules); (10) nucleic acid segments that, when absent, directly or indirectly confer resistance or sensitivity to particular compounds; (11) nucleic acid segments that encode products that either are toxic or convert a relatively non-toxic compound to a toxic compound (e.g., Herpes simplex thymidine kinase, cytosine deaminase) in recipient cells; (12) nucleic acid segments that inhibit replication, partition or heritability of nucleic acid molecules that contain them; and/or (13) nucleic acid segments that encode conditional replication functions, e.g., replication in certain hosts or host cell strains or under certain environmental conditions (e.g., temperature, nutritional conditions, and the like).

A nucleic acid reagent is of any form useful for in vivo transcription and/or translation. A nucleic acid sometimes is a plasmid, such as a supercoiled plasmid, sometimes is a yeast artificial chromosome (e.g., YAC), sometimes is a linear nucleic acid (e.g., a linear nucleic acid produced by PCR or by restriction digest), sometimes is single-stranded and sometimes is double-stranded. A nucleic acid reagent sometimes is prepared by an amplification process, such as a polymerase chain reaction (PCR) process or transcription-mediated amplification process (TMA). In TMA, two enzymes are used in an isothermal reaction to produce amplification products detected by light emission (see, e.g., Biochemistry 1996 Jun 25;35(25):8429-38 and http address www.devicelink.com/ivdt/archive/00/11/007.html). Standard PCR processes are known (e.g., U. S. Patent Nos. 4,683,202; 4,683,195; 4,965,188; and 5,656,493), and generally are performed in cycles. Each cycle includes heat denaturation, in which hybrid nucleic acids dissociate; cooling, in which primer oligonucleotides hybridize; and extension of the oligonucleotides by a polymerase (i.e., Taq polymerase). An example of a PCR cyclical process is treating the sample at 95°C for 5 minutes; repeating forty-five cycles of 95°C for 1 minute, 59°C for 1 minute, 10 seconds, and 72°C for 1 minute 30 seconds; and then treating the sample at 72°C for 5 minutes. Multiple cycles frequently are performed using a commercially available thermal cycler. PCR amplification products sometimes are stored for a time at a lower temperature (e.g., at 4°C) and sometimes are frozen (e.g., at -20°C) before analysis.

In some embodiments, a nucleic acid reagent, protein reagent, protein fragment reagent or other reagent described herein is isolated or purified. The term "isolated" as used herein refers to material removed from its original environment (e.g., the natural environment if it is naturally occurring, or a host cell if expressed exogenously), and thus is altered "by the hand of man" from its original environment. The term "purified" as used herein with reference to molecules does not refer to absolute purity. Rather, "purified" refers to a substance in a composition that contains fewer substance species in the same class (e.g., nucleic acid or protein species) other than the substance of interest in comparison to the sample from which it originated. "Purified," if a nucleic acid or protein for example, refers to a substance in a composition that contains fewer nucleic acid species or protein species other than the nucleic acid or protein of interest in comparison to the sample from which it originated. Sometimes, a protein or nucleic acid is "substantially pure," indicating that the protein or nucleic acid represents at least 50% of protein or nucleic acid on a mass basis of the composition. Often, a substantially pure protein or nucleic acid is at least 75% on a mass basis of the composition, and sometimes at least 95% on a mass basis of the composition.

### Engineering and Alteration Methods

Methods and compositions (e.g., nucleic acid reagents) described herein can be used to generate engineered microorganisms. As noted above, the term "engineered microorganism" as used herein refers to a modified organism that includes one or more activities distinct from an activity present in a microorganism utilized as a starting point for modification (e.g., host microorganism or unmodified organism). Engineered microorganisms typically arise as a result of a genetic modification, usually introduced or selected for, by one of skill in the art using readily available techniques. Non-limiting examples of methods useful for generating an altered activity include, introducing a heterologous polynucleotide (e.g., nucleic acid or gene integration, also referred to as "knock in"), removing an endogenous polynucleotide, altering the sequence of an existing endogenous nucleic acid sequence (e.g., site-directed mutagenesis), disruption of an existing endogenous nucleic acid sequence (e.g., knock outs and transposon or insertion element mediated mutagenesis), selection for an altered activity where the selection causes a change in a naturally occurring activity that can be stably inherited (e.g., causes a change in a nucleic acid sequence in the genome of the organism or in an epigenetic nucleic acid that is replicated and passed on to daughter cells), PCR-based mutagenesis, and the like. The term "mutagenesis" as used herein refers to any modification to a nucleic acid (e.g., nucleic acid reagent, or host chromosome, for example) that is subsequently used to generate a product in a host or modified organism. Non-limiting examples of mutagenesis include, deletion, insertion, substitution, rearrangement, point mutations, suppressor mutations and the like. Mutagenesis methods are known in the art and are readily available to the artisan. Non-limiting examples of mutagenesis methods are described herein and can also be found in Maniatis, T., E. F. Fritsch and J. Sambrook (1982) Molecular Cloning: a Laboratory Manual; Cold Spring Harbor Laboratory, Cold Spring Harbor, N.Y. Another non-limiting example of mutagenesis can be conducted using a Stratagene (San Diego, CA) "QuickChange" kit according to the manufacturer's instructions.

The term "genetic modification" as used herein refers to any suitable nucleic acid addition, removal or alteration that facilitates production of a target product (e.g., adipic acid, 6-hydroxyhexanoic acid) in an engineered microorganism. Genetic modifications include, without limitation, insertion of one or more nucleotides in a native nucleic acid of a host organism in one or more locations, deletion of one or more nucleotides in a native nucleic acid of a host organism in one or more locations, modification or substitution of one or more nucleotides in a native nucleic acid of a host organism in one or more locations, insertion of a non-native nucleic acid into a host organism (e.g., insertion of an autonomously replicating vector), and removal of a non-native nucleic acid in a host organism (e.g., removal of a vector).

The term "heterologous polynucleotide" as used herein refers to a nucleotide sequence not present in a host microorganism in some embodiments. In certain embodiments, a heterologous polynucleotide is present in a different amount (e.g., different copy number) than in a host microorganism, which can be accomplished, for example, by introducing more copies of a particular nucleotide sequence to a host microorganism (e.g., the particular nucleotide sequence may be in a nucleic acid autonomous of the host chromosome or may be inserted into a chromosome). A heterologous polynucleotide is from a different organism in some embodiments, and in certain embodiments, is from the same type of organism but from an outside source (e.g., a recombinant source).

In some embodiments, an organism engineered using the methods and nucleic acid reagents described herein can produce adipic acid. In certain embodiments, an engineered microorganism described herein that produces adipic acid may comprise one ore more altered activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, hexanoate synthase activity, lipase activity, fatty acid synthase activity, acetyl CoA carboxylase activity, monooxygenase activity and monooxygenase reductase activity. In some embodiments, an engineered microorganism as described herein may comprise a genetic modification that adds or increases the 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity and monooxygenase reductase activity.

In certain embodiments, an engineered microorganism described herein can comprise an altered thioesterase activity. In some embodiments, the engineered microorganism may comprise a genetic alteration that adds or increases a thioesterase activity. In some embodiments, the engineered microorganism comprising a genetic alteration that adds or increases a thioesterase activity, may further comprise a heterologous polynucleotide encoding a polypeptide having thioesterase activity.

The term "altered activity" as used herein refers to an activity in an engineered microorganism that is added or modified relative to the host microorganism (e.g., added, increased, reduced, inhibited or removed activity). An activity can be altered by introducing a genetic modification to a host microorganism that yields an engineered microorganism having added, increased, reduced, inhibited or removed activity.

An added activity often is an activity not detectable in a host microorganism. An increased activity generally is an activity detectable in a host microorganism that has been increased in an engineered microorganism. An activity can be increased to any suitable level for production of a target product (e.g., adipic acid, 6-hydroxyhexanoic acid), including but not limited to less than 2-fold (e.g., about 10% increase to about 99% increase; about 20%, 30%, 40%, 50%, 60%, 70%, 80%, 90% increase), 2-fold, 3-fold, 4-fold, 5-fold, 6-fold, 7-fold, 8-fold, 9-fold, of 10-fold increase, or greater than about 10-fold increase. A reduced or inhibited activity generally is an activity detectable in a host microorganism that has been reduced or inhibited in an engineered microorganism. An activity can be reduced to undetectable levels in some embodiments, or detectable levels in certain embodiments. An activity can be decreased to any suitable level for production of a target product (e.g., adipic acid, 6-hydroxyhexanoic acid), including but not limited to less than 2-fold (e.g., about 10% decrease to about 99% decrease; about 20%, 30%, 40%, 50%, 60%, 70%, 80%, 90% decrease), 2-fold, 3-fold, 4-fold, 5-fold, 6-fold, 7-fold, 8-fold, 9-fold, of 10-fold decrease, or greater than about 10-fold decrease.

An altered activity sometimes is an activity not detectable in a host organism and is added to an engineered organism. An altered activity also may be an activity detectable in a host organism and is increased in an engineered organism. An activity may be added or increased by increasing the number of copies of a polynucleotide that encodes a polypeptide having a target activity, in some embodiments. In certain embodiments an activity can be added or increased by inserting into a host microorganism a heterologous polynucleotide that encodes a polypeptide having the added activity. In certain embodiments, an activity can be added or increased by inserting into a host microorganism a heterologous polynucleotide that is (i) operably linked to another polynucleotide that encodes a polypeptide having the added activity, and (ii) up regulates production of the polynucleotide. Thus, an activity can be added or increased by inserting or modifying a regulatory polynucleotide operably linked to another polynucleotide that encodes a polypeptide having the target activity. In certain embodiments, an activity can be added or increased by subjecting a host microorganism to a selective environment and screening for microorganisms that have a detectable level of the target activity. Examples of a selective environment include, without limitation, a medium containing a substrate that a host organism can process and a medium lacking a substrate that a host organism can process.

An altered activity sometimes is an activity detectable in a host organism and is reduced, inhibited or removed (i.e., not detectable) in an engineered organism. An activity may be reduced or removed by decreasing the number of copies of a polynucleotide that encodes a polypeptide having a target activity, in some embodiments. In some embodiments, an activity can be reduced or removed by (i) inserting a polynucleotide within a polynucleotide that encodes a polypeptide having the target activity (disruptive insertion), and/or (ii) removing a portion of or all of a polynucleotide that encodes a polypeptide having the target activity (deletion or knock out, respectively). In certain embodiments, an activity can be reduced or removed by inserting into a host microorganism a heterologous polynucleotide that is (i) operably linked to another polynucleotide that encodes a polypeptide having the target activity, and (ii) down regulates production of the polynucleotide. Thus, an activity can be reduced or removed by inserting or modifying a regulatory polynucleotide operably linked to another polynucleotide that encodes a polypeptide having the target activity.

An activity also can be reduced or removed by (i) inhibiting a polynucleotide that encodes a polypeptide having the activity or (ii) inhibiting a polynucleotide operably linked to another polynucleotide that encodes a polypeptide having the activity. A polynucleotide can be inhibited by a suitable technique known in the art, such as by contacting an RNA encoded by the polynucleotide with a specific inhibitory RNA (e.g., RNAi, siRNA, ribozyme). An activity also can be reduced or removed by contacting a polypeptide having the activity with a molecule that specifically inhibits the activity (e.g., enzyme inhibitor, antibody). In certain embodiments, an activity can be reduced or removed by subjecting a host microorganism to a selective environment and screening for microorganisms that have a reduced level or removal of the target activity.

In some embodiments, an untranslated ribonucleic acid, or a cDNA can be used to reduce the expression of a particular activity or enzyme. For example, a microorganism can be engineered by genetic modification to express a nucleic acid reagent that reduces the expression of an activity by producing an RNA molecule that is partially or substantially homologous to a nucleic acid sequence of interest which encodes the activity of interest. The RNA molecule can bind to the nucleic acid sequence of interest and inhibit the nucleic acid sequence from performing its natural function, in certain embodiments. In some embodiments, the RNA may alter the nucleic acid sequence of interest which encodes the activity of interest in a manner that the nucleic acid sequence of interest is no longer capable of performing its natural function (e.g., the action of a ribozyme for example).

In certain embodiments, nucleotide sequences sometimes are added to, modified or removed from one or more of the nucleic acid reagent elements, such as the promoter, 5'UTR, target sequence, or 3'UTR elements, to enhance, potentially enhance, reduce, or potentially reduce transcription and/or translation before or after such elements are incorporated in a nucleic acid reagent. In some embodiments, one or more of the following sequences may be modified or removed if they are present in a 5'UTR: a sequence that forms a stable secondary structure (e.g., quadruplex structure or stem loop stem structure (e.g., EMBL sequences X12949, AF274954, AF139980, AF152961, S95936, U194144, AF116649 or substantially identical sequences that form such stem loop stem structures)); a translation initiation codon upstream of the target nucleotide sequence start codon; a stop codon upstream of the target nucleotide sequence translation initiation codon; an ORF upstream of the target nucleotide sequence translation initiation codon; an iron responsive element (IRE) or like sequence; and a 5' terminal oligopyrimidine tract (TOP, e.g., consisting of 5-15 pyrimidines adjacent to the cap). A translational enhancer sequence and/or an internal ribosome entry site (IRES) sometimes is inserted into a 5'UTR (e.g., EMBL nucleotide sequences J04513, X87949, M95825, M12783, AF025841, AF013263, AF006822, M17169, M13440, M22427, D14838 and M17446 and substantially identical nucleotide sequences).
An AU-rich element (ARE, e.g., AUUUA repeats) and/or splicing junction that follows a non-sense codon sometimes is removed from or modified in a 3'UTR. A polyadenosine tail sometimes is inserted into a 3'UTR if none is present, sometimes is removed if it is present, and adenosine moieties sometimes are added to or removed from a polyadenosine tail present in a 3'UTR. Thus, some embodiments are directed to a process comprising: determining whether any nucleotide sequences that increase, potentially increase, reduce or potentially reduce translation efficiency are present in the elements, and adding, removing or modifying one or more of such sequences if they are identified. Certain embodiments are directed to a process comprising: determining whether any nucleotide sequences that increase or potentially increase translation efficiency are not present in the elements, and incorporating such sequences into the nucleic acid reagent.

In some embodiments, an activity can be altered by modifying the nucleotide sequence of an ORF. An ORF sometimes is mutated or modified (for example, by point mutation, deletion mutation, insertion mutation, PCR based mutagenesis and the like) to alter, enhance or increase, reduce, substantially reduce or eliminate the activity of the encoded protein or peptide. The protein or peptide encoded by a modified ORF sometimes is produced in a lower amount or may not be produced at detectable levels, and in other embodiments, the product or protein encoded by the modified ORF is produced at a higher level (e.g., codons sometimes are modified so they are compatible with tRNA's preferentially used in the host organism or engineered organism). To determine the relative activity, the activity from the product of the mutated ORF (or cell containing it) can be compared to the activity of the product or protein encoded by the unmodified ORF (or cell containing it).

In some embodiments, an ORF nucleotide sequence sometimes is mutated or modified to alter the triplet nucleotide sequences used to encode amino acids (e.g., amino acid codon triplets, for example). Modification of the nucleotide sequence of an ORF to alter codon triplets sometimes is used to change the codon found in the original sequence to better match the preferred codon usage of the organism in which the ORF or nucleic acid reagent will be expressed. The codon usage, and therefore the codon triplets encoded by a nucleic acid sequence, in bacteria may be different from the preferred codon usage in eukaryotes, like yeast or plants for example. Preferred codon usage also may be different between bacterial species. In certain embodiments an ORF nucleotide sequences sometimes is modified to eliminate codon pairs and/or eliminate mRNA secondary structures that can cause pauses during translation of the mRNA encoded by the ORF nucleotide sequence. Translational pausing sometimes occurs when nucleic acid secondary structures exist in an mRNA, and sometimes occurs due to the presence of codon pairs that slow the rate of translation by causing ribosomes to pause. In some embodiments, the use of lower abundance codon triplets can reduce translational pausing due to a decrease in the pause time needed to load a charged tRNA into the ribosome translation machinery. Therefore, to increase transcriptional and translational efficiency in bacteria (e.g., where transcription and translation are concurrent, for example) or to increase translational efficiency in eukaryotes (e.g., where transcription and translation are functionally separated), the nucleotide sequence of a nucleotide sequence of interest can be altered to better suit the transcription and/or translational machinery of the host and/or genetically modified microorganism. In certain embodiments, slowing the rate of translation by the use of lower abundance codons, which slow or pause the ribosome, can lead to higher yields of the desired product due to an increase in correctly folded proteins and a reduction in the formation of inclusion bodies.

Codons can be altered and optimized according to the preferred usage by a given organism by determining the codon distribution of the nucleotide sequence donor organism and comparing the distribution of codons to the distribution of codons in the recipient or host organism. Techniques described herein (e.g., site directed mutagenesis and the like) can then be used to alter the codons accordingly. Comparisons of codon usage can be done by hand, or using nucleic acid analysis software commercially available to the artisan.

Modification of the nucleotide sequence of an ORF also can be used to correct codon triplet sequences that have diverged in different organisms. For example, certain yeast (e.g., C. tropicalis and C. maltosa) use the amino acid triplet CUG (e.g., CTG in the DNA sequence) to encode serine. CUG typically encodes leucine in most organisms. In order to maintain the correct amino acid in the resultant polypeptide or protein, the CUG codon must be altered to reflect the organism in which the nucleic acid reagent will be expressed. Thus, if an ORF from a bacterial donor is to be expressed in either Candida yeast strain mentioned above, the heterologous nucleotide sequence must first be altered or modified to the appropriate leucine codon. Therefore, in some embodiments, the nucleotide sequence of an ORF sometimes is altered or modified to correct for differences that have occurred in the evolution of the amino acid codon triplets between different organisms. In some embodiments, the nucleotide sequence can be left unchanged at a particular amino acid codon, if the amino acid encoded is a conservative or neutral change in amino acid when compared to the originally encoded amino acid.

In some embodiments, an activity can be altered by modifying translational regulation signals, like a stop codon for example. A stop codon at the end of an ORF sometimes is modified to another stop codon, such as an amber stop codon described above. In some embodiments, a stop codon is introduced within an ORF, sometimes by insertion or mutation of an existing codon. An ORF comprising a modified terminal stop codon and/or internal stop codon often is translated in a system comprising a suppressor tRNA that recognizes the stop codon. An ORF comprising a stop codon sometimes is translated in a system comprising a suppressor tRNA that incorporates an unnatural amino acid during translation of the target protein or target peptide. Methods for incorporating unnatural amino acids into a target protein or peptide are known, which include, for example, processes utilizing a heterologous tRNA/synthetase pair, where the tRNA recognizes an amber stop codon and is loaded with an unnatural amino acid (e.g., World Wide Web URL iupac.org/news/prize/2003/wang.pdf).

Depending on the portion of a nucleic acid reagent (e.g., Promoter, 5' or 3' UTR, ORI, ORF, and the like) chosen for alteration (e.g., by mutagenesis, introduction or deletion, for example) the modifications described above can alter a given activity by (i) increasing or decreasing feedback inhibition mechanisms, (ii) increasing or decreasing promoter initiation, (iii) increasing or decreasing translation initiation, (iv) increasing or decreasing translational efficiency, (v) modifying localization of peptides or products expressed from nucleic acid reagents described herein, or (vi) increasing or decreasing the copy number of a nucleotide sequence of interest, (vii) expression of an anti-sense RNA, RNAi, siRNA, ribozyme and the like. In some embodiments, alteration of a nucleic acid reagent or nucleotide sequence can alter a region involved in feedback inhibition (e.g., 5' UTR, promoter and the like). A modification sometimes is made that can add or enhance binding of a feedback regulator and sometimes a modification is made that can reduce, inhibit or eliminate binding of a feedback regulator.

In certain embodiments, alteration of a nucleic acid reagent or nucleotide sequence can alter sequences involved in transcription initiation (e.g., promoters, 5' UTR, and the like). A modification sometimes can be made that can enhance or increase initiation from an endogenous or heterologous promoter element. A modification sometimes can be made that removes or disrupts sequences that increase or enhance transcription initiation, resulting in a decrease or elimination of transcription from an endogenous or heterologous promoter element.

In some embodiments, alteration of a nucleic acid reagent or nucleotide sequence can alter sequences involved in translational initiation or translational efficiency (e.g., 5' UTR, 3' UTR, codon triplets of higher or lower abundance, translational terminator sequences and the like, for example). A modification sometimes can be made that can increase or decrease translational initiation, modifying a ribosome binding site for example. A modification sometimes can be made that can increase or decrease translational efficiency. Removing or adding sequences that form hairpins and changing codon triplets to a more or less preferred codon are non-limiting examples of genetic modifications that can be made to alter translation initiation and translation efficiency.

In certain embodiments, alteration of a nucleic acid reagent or nucleotide sequence can alter sequences involved in localization of peptides, proteins or other desired products (e.g., adipic acid, for example). A modification sometimes can be made that can alter, add or remove sequences responsible for targeting a polypeptide, protein or product to an intracellular organelle, the periplasm, cellular membranes, or extracellularly. Transport of a heterologous product to a different intracellular space or extracellularly sometimes can reduce or eliminate the formation of inclusion bodies (e.g., insoluble aggregates of the desired product).

In some embodiments, alteration of a nucleic acid reagent or nucleotide sequence can alter sequences involved in increasing or decreasing the copy number of a nucleotide sequence of interest. A modification sometimes can be made that increases or decreases the number of copies of an ORF stably integrated into the genome of an organism or on an epigenetic nucleic acid reagent. Non-limiting examples of alterations that can increase the number of copies of a sequence of interest include, adding copies of the sequence of interest by duplication of regions in the genome (e.g., adding additional copies by recombination or by causing gene amplification of the host genome, for example), cloning additional copies of a sequence onto a nucleic acid reagent, or altering an ORI to increase the number of copies of an epigenetic nucleic acid reagent. Non-limiting examples of alterations that can decrease the number of copies of a sequence of interest include, removing copies of the sequence of interest by deletion or disruption of regions in the genome, removing additional copies of the sequence from epigenetic nucleic acid reagents, or altering an ORI to decrease the number of copies of an epigenetic nucleic acid reagent.

In certain embodiments, increasing or decreasing the expression of a nucleotide sequence of interest can also be accomplished by altering, adding or removing sequences involved in the expression of an anti-sense RNA, RNAi, siRNA, ribozyme and the like. The methods described above can be used to modify expression of anti-sense RNA, RNAi, siRNA, ribozyme and the like.

The methods and nucleic acid reagents described herein can be used to generate genetically modified microorganisms with altered activities in cellular processes involved in adipic acid synthesis. In some embodiments, an engineered microorganism described herein may comprise a heterologous polynucleotide encoding a polypeptide having 6-oxohexanoic acid dehydrogenase activity, and in certain embodiments, an engineered microorganism described herein may comprise a heterologous polynucleotide encoding a polypeptide having omega oxo fatty acid dehydrogenase activity. In some embodiments, an engineered microorganism described herein may comprise a heterologous polynucleotide encoding a polypeptide having 6-hydroxyhexanoic acid dehydrogenase activity, and in certain embodiments, an engineered microorganism described herein may comprise a heterologous polynucleotide encoding a polypeptide having omega hydroxyl fatty acid dehydrogenase activity. In some embodiments, the heterologous polynucleotide can be from a bacterium. In some embodiments, the bacterium can be an Acinetobacter, Nocardia, Pseudomonas or Xanthobacter bacterium.

In certain embodiments, an engineered microorganism described herein may comprise a heterologous polynucleotide encoding a polypeptide having hexanoate synthase subunit A activity. In some embodiments, an engineered microorganism described herein may comprise a heterologous polynucleotide encoding a polypeptide having hexanoate synthase subunit B activity. In certain embodiments, the heterologous polynucleotide independently is selected from a fungus. In some embodiments, the fungus can be an Aspergillus fungus. In certain embodiments, the Aspergillus fungus is A. parasiticus.

In some embodiments, an engineered microorganism described herein may comprise a heterologous polynucleotide encoding a polypeptide having monooxygenase activity. In certain embodiments, the heterologous polynucleotide can be from a bacterium. In some embodiments, the bacterium can be a Bacillus bacterium. In certain embodiments, the Bacillus bacterium is B. megaterium.

In some embodiments, an engineered microorganism described herein may comprise a genetic modification that results in primary hexanoate usage by monooxygenase activity. In certain embodiments, the genetic modification can reduce a polyketide synthase activity. In some embodiments, the engineered microorganism can be a eukaryote. In certain embodiments, the eukaryote can be a yeast. In some embodiments, the eukaryote may be a fungus. In certain embodiments, the yeast can be a Candida yeast. In some embodiments, the Candida yeast may be C. troplicalis. In certain embodiments, the yeast can be a Yarrowia yeast. In some embodiments the Yarrowia yeast may be Y. lipolytica. In certain embodiments, the fungus can be an Aspergillus fungus. In some embodiments, the Aspergillus fungus may be A. parasiticus or A. nidulans.

In certain embodiments, an engineered microorganism described herein may comprise a genetic modification that reduces 6-hydroxyhexanoic acid conversion. In some embodiments, the genetic modification can reduce 6-hydroxyhexanoic acid dehydrogenase activity. In certain embodiments, an engineered microorganism described herein may comprise a genetic modification that reduces beta-oxidation activity. In some embodiments, the genetic modification can reduce a target activity described herein.

Engineered microorganisms that produce adipic acid, as described herein, can comprise an altered monooxygenase activity, in certain embodiments. In some embodiments, the engineered microorganism described herein may comprise a genetic modification that alters the monooxygenase activity. In certain embodiments, the genetic modification can result in substantial hexanoate usage by the monooxygenase activity. In some embodiments, the genetic modification can reduce a polyketide synthase activity. In certain embodiments, the engineered microorganism described herein can comprise a heterologous polynucleotide encoding a polypeptide having monooxygenase activity. In certain embodiments, the heterologous polynucleotide can be from a bacterium. In some embodiments, the bacterium can be a Bacillus bacterium. In certain embodiments, the Bacillus bacterium is B. megaterium.
In some embodiments, the engineered microorganism described herein may comprise an altered hexanoate synthase activity. In certain embodiments, the altered hexanoate synthase activity is an altered hexanoate synthase subunit A activity, altered hexanoate synthase subunit B activity, or altered hexanoate synthase subunit A activity and altered hexanoate synthase subunit B activity. In some embodiments, the engineered microorganism may comprise a genetic alteration that adds or increases hexanoate synthase activity. In certain embodiments, the engineered microorganism may comprise a heterologous polynucleotide encoding a polypeptide having hexanoate synthase activity. In some embodiments, the heterologous polynucleotide can be from a fungus. In certain embodiments, the fungus can be an Aspergillus fungus. In some embodiments, the Aspergillus fungus is A. parasiticus.

Engineered microorganisms that produce adipic acid, as described herein, can comprise an altered thioesterase activity, in certain embodiments. In some embodiments, the engineered microorganism may comprise a genetic modification that adds or increases the thioesterase activity. In certain embodiments, the engineered microorganism may comprise a heterologous polynucleotide encoding a polypeptide having thioesterase activity.

In some embodiments, the engineered microorganism with an altered thioesterase activity may comprise an altered 6-oxohexanoic acid dehydrogenase activity, or an altered omega oxo fatty acid dehydrogenase activity. In certain embodiments, the engineered microorganism with an altered thioesterase activity may comprise a genetic modification that adds or increases 6-oxohexanoic acid dehydrogenase activity, or a genetic modification that adds or increases omega oxo fatty acid dehydrogenase activity. In some embodiments, the engineered microorganism may comprise a heterologous polynucleotide encoding a polypeptide having altered 6-oxohexanoic acid dehydrogenase activity, and in some embodiments, the engineered microorganism may comprise a heterologous polynucleotide encoding a polypeptide having altered omega oxo fatty acid dehydrogenase activity. In certain embodiments, the heterologous polynucleotide can be from a bacterium. In some embodiments, the bacterium can be an Acinetobacter, Nocardia, Pseudomonas or Xanthobacter bacterium.

Engineered microorganisms that produce adipic acid, as described herein, can comprise an altered 6-hydroxyhexanoic acid dehydrogenase activity, in certain embodiments, and in some embodiments, can comprise an altered omega hydroxyl fatty acid dehydrogenase activity. In certain embodiments, the engineered microorganism may comprise a genetic modification that adds or increases the 6-hydroxyhexanoic acid dehydrogenase activity and in some embodiments the engineered microorganism may comprise a genetic modification that adds or increases the omega hydroxyl fatty acid dehydrogenase activity. In certain embodiments, the engineered microorganism may comprise a heterologous polynucleotide encoding a polypeptide having altered 6-hydroxyhexanoic acid dehydrogenase activity, and in some embodiments, the engineered microorganism may comprise a heterologous polynucleotide encoding a polypeptide having altered omega hydroxyl fatty acid dehydrogenase activity. In some embodiments, the heterologous polynucleotide is from a bacterium. In certain embodiments, the bacterium can be an Acinetobacter, Nocardia, Pseudomonas or Xanthobacter bacterium. In some embodiments, the Candida yeast may be C. troplicalis. In certain embodiments, the yeast can be a Yarrowia yeast. In some embodiments the Yarrowia yeast may be Y. lipolytica. In certain embodiments, the fungus can be an Aspergillus fungus. In some embodiments, the Aspergillus fungus may be A. parasiticus or A. nidulans.

In some embodiments, an engineered microorganism as described above may comprise a genetic modification that reduces 6-hydroxyhexanoic acid conversion. In certain embodiments, the genetic modification can reduce 6-hydroxyhexanoic acid dehydrogenase activity. In some embodiments the genetic may reduce beta-oxidation activity. In certain embodiments, the genetic modification may reduce a target activity described herein.

Engineered microorganisms can be prepared by altering, introducing or removing nucleotide sequences in the host genome or in stably maintained epigenetic nucleic acid reagents, as noted above. The nucleic acid reagents use to alter, introduce or remove nucleotide sequences in the host genome or epigenetic nucleic acids can be prepared using the methods described herein or available to the artisan.

Nucleic acid sequences having a desired activity can be isolated from cells of a suitable organism using lysis and nucleic acid purification procedures described in a known reference manual (e.g., Maniatis, T., E. F. Fritsch and J. Sambrook (1982) Molecular Cloning: a Laboratory Manual; Cold Spring Harbor Laboratory, Cold Spring Harbor, N.Y.) or using commercially available cell lysis and DNA purification reagents and kits. In some embodiments, nucleic acids used to engineer microorganisms can be provided for conducting methods described herein after processing of the organism containing the nucleic acid. For example, the nucleic acid of interest may be extracted, isolated, purified or amplified from a sample (e.g., from an organism of interest or culture containing a plurality of organisms of interest, like yeast or bacteria for example). The term "isolated" as used herein refers to nucleic acid removed from its original environment (e.g., the natural environment if it is naturally occurring, or a host cell if expressed exogenously), and thus is altered "by the hand of man" from its original environment. An isolated nucleic acid generally is provided with fewer non-nucleic acid components (e.g., protein, lipid) than the amount of components present in a source sample. A composition comprising isolated sample nucleic acid can be substantially isolated (e.g., about 90%, 91%, 92%, 93%, 94%, 95%, 96%, 97%, 98%, 99% or greater than 99% free of non-nucleic acid components). The term "purified" as used herein refers to sample nucleic acid provided that contains fewer nucleic acid species than in the sample source from which the sample nucleic acid is derived. A composition comprising sample nucleic acid may be substantially purified (e.g., about 90%, 91 %, 92%, 93%, 94%, 95%, 96%, 97%, 98%, 99% or greater than 99% free of other nucleic acid species). The term "amplified" as used herein refers to subjecting nucleic acid of a cell, organism or sample to a process that linearly or exponentially generates amplicon nucleic acids having the same or substantially the same nucleotide sequence as the nucleotide sequence of the nucleic acid in the sample, or portion thereof. As noted above, the nucleic acids used to prepare nucleic acid reagents as described herein can be subjected to fragmentation or cleavage.

Amplification of nucleic acids is sometimes necessary when dealing with organisms that are difficult to culture. Where amplification may be desired, any suitable amplification technique can be utilized. Non-limiting examples of methods for amplification of polynucleotides include, polymerase chain reaction (PCR); ligation amplification (or ligase chain reaction (LCR)); amplification methods based on the use of Q-beta replicase or template-dependent polymerase (see US Patent Publication Number US20050287592); helicase-dependant isothermal amplification (Vincent et al., "Helicase-dependent isothermal DNA amplification". EMBO reports 5 (8): 795-800 (2004)); strand displacement amplification (SDA); thermophilic SDA nucleic acid sequence based amplification (3SR or NASBA) and transcription-associated amplification (TAA). Non-limiting examples of PCR amplification methods include standard PCR, AFLP-PCR, Allele-specific PCR, Alu-PCR, Asymmetric PCR, Colony PCR, Hot start PCR, Inverse PCR (IPCR), In situ PCR (ISH), Intersequence-specific PCR (ISSR-PCR), Long PCR, Multiplex PCR, Nested PCR, Quantitative PCR, Reverse Transcriptase PCR (RT-PCR), Real Time PCR, Single cell PCR, Solid phase PCR, combinations thereof, and the like. Reagents and hardware for conducting PCR are commercially available.

Protocols for conducting the various type of PCR listed above are readily available to the artisan. PCR conditions can be dependent upon primer sequences, target abundance, and the desired amount of amplification, and therefore, one of skill in the art may choose from a number of PCR protocols available (see, e.g., U.S. Pat. Nos. 4,683,195 and 4,683,202; and PCR Protocols: A Guide to Methods and Applications, Innis et al., eds, 1990. PCR often is carried out as an automated process with a thermostable enzyme. In this process, the temperature of the reaction mixture is cycled through a denaturing region, a primer-annealing region, and an extension reaction region automatically. Machines specifically adapted for this purpose are commercially available. A non-limiting example of a PCR protocol that may be suitable for embodiments described herein is, treating the sample at 95ºC for 5 minutes; repeating forty-five cycles of 95ºC for 1 minute, 59ºC for 1 minute, 10 seconds, and 72ºC for 1 minute 30 seconds; and then treating the sample at 72ºC for 5 minutes. Additional PCR protocols are described in the example section. Multiple cycles frequently are performed using a commercially available thermal cycler. Suitable isothermal amplification processes known and selected by the person of ordinary skill in the art also may be applied, in certain embodiments. In some embodiments, nucleic acids encoding polypeptides with a desired activity can be isolated by amplifying the desired sequence from an organism having the desired activity using oligonucleotides or primers designed based on sequences described herein.

Amplified, isolated and/or purified nucleic acids can be cloned into the recombinant DNA vectors described in Figures herein or into suitable commercially available recombinant DNA vectors. Cloning of nucleic acid sequences of interest into recombinant DNA vectors can facilitate further manipulations of the nucleic acids for preparation of nucleic acid reagents, (e.g., alteration of nucleotide sequences by mutagenesis, homologous recombination, amplification and the like, for example). Standard cloning procedures (e.g., enzymic digestion, ligation, and the like) are known (e.g., described in Maniatis, T., E. F. Fritsch and J. Sambrook (1982) Molecular Cloning: a Laboratory Manual; Cold Spring Harbor Laboratory, Cold Spring Harbor, N.Y.).

In some embodiments, nucleic acid sequences prepared by isolation or amplification can be used, without any further modification, to add an activity to a microorganism and thereby create a genetically modified or engineered microorganism. In certain embodiments, nucleic acid sequences prepared by isolation or amplification can be genetically modified to alter (e.g., increase or decrease, for example) a desired activity. In some embodiments, nucleic acids, used to add an activity to an organism, sometimes are genetically modified to optimize the heterologous polynucleotide sequence encoding the desired activity (e.g., polypeptide or protein, for example). The term "optimize" as used herein can refer to alteration to increase or enhance expression by preferred codon usage. The term optimize can also refer to modifications to the amino acid sequence to increase the activity of a polypeptide or protein, such that the activity exhibits a higher catalytic activity as compared to the "natural" version of the polypeptide or protein.

Nucleic acid sequences of interest can be genetically modified using methods known in the art. Mutagenesis techniques are particularly useful for small scale (e.g., 1, 2, 5, 10 or more nucleotides) or large scale (e.g., 50, 100, 150, 200, 500, or more nucleotides) genetic modification. Mutagenesis allows the artisan to alter the genetic information of an organism in a stable manner, either naturally (e.g., isolation using selection and screening) or experimentally by the use of chemicals, radiation or inaccurate DNA replication (e.g., PCR mutagenesis). In some embodiments, genetic modification can be performed by whole scale synthetic synthesis of nucleic acids, using a native nucleotide sequence as the reference sequence, and modifying nucleotides that can result in the desired alteration of activity. Mutagenesis methods sometimes are specific or targeted to specific regions or nucleotides (e.g., site-directed mutagenesis, PCR-based site-directed mutagenesis, and in vitro mutagenesis techniques such as transplacement and in vivo oligonucleotide site-directed mutagenesis, for example). Mutagenesis methods sometimes are non-specific or random with respect to the placement of genetic modifications (e.g., chemical mutagenesis, insertion element (e.g., insertion or transposon elements) and inaccurate PCR based methods, for example).

Site directed mutagenesis is a procedure in which a specific nucleotide or specific nucleotides in a DNA molecule are mutated or altered. Site directed mutagenesis typically is performed using a nucleic acid sequence of interest cloned into a circular plasmid vector. Site-directed mutagenesis requires that the wild type sequence be known and used a platform for the genetic alteration. Site-directed mutagenesis sometimes is referred to as oligonucleotide-directed mutagenesis because the technique can be performed using oligonucleotides which have the desired genetic modification incorporated into the complement a nucleotide sequence of interest. The wild type sequence and the altered nucleotide are allowed to hybridize and the hybridized nucleic acids are extended and replicated using a DNA polymerase. The double stranded nucleic acids are introduced into a host (e.g., E. coli, for example) and further rounds of replication are carried out in vivo. The transformed cells carrying the mutated nucleic acid sequence are then selected and/or screened for those cells carrying the correctly mutagenized sequence. Cassette mutagenesis and PCR-based site-directed mutagenesis are further modifications of the site-directed mutagenesis technique. Site-directed mutagenesis can also be performed in vivo (e.g., transplacement "pop-in pop-out", In vivo site-directed mutagenesis with synthetic oligonucleotides and the like, for example).

PCR-based mutagenesis can be performed using PCR with oligonucleotide primers that contain the desired mutation or mutations. The technique functions in a manner similar to standard site-directed mutagenesis, with the exception that a thermocycler and PCR conditions are used to replace replication and selection of the clones in a microorganism host. As PCR-based mutagenesis also uses a circular plasmid vector, the amplified fragment (e.g., linear nucleic acid molecule) containing the incorporated genetic modifications can be separated from the plasmid containing the template sequence after a sufficient number of rounds of thermocycler amplification, using standard electrophorectic procedures. A modification of this method uses linear amplification methods and a pair of mutagenic primers that amplify the entire plasmid. The procedure takes advantage of the E. coli Dam methylase system which causes DNA replicated in vivo to be sensitive to the restriction endonucleases Dpnl. PCR synthesized DNA is not methylated and is therefore resistant to Dpnl. This approach allows the template plasmid to be digested, leaving the genetically modified, PCR synthesized plasmids to be isolated and transformed into a host bacteria for DNA repair and replication, thereby facilitating subsequent cloning and identification steps. A certain amount of randomness can be added to PCR-based sited directed mutagenesis by using partially degenerate primers.

Recombination sometimes can be used as a tool for mutagenesis. Homologous recombination allows the artisan to specifically target regions of known sequence for insertion of heterologous nucleotide sequences using the host organisms natural DNA replication and repair enzymes. Homologous recombination methods sometimes are referred to as "pop in pop out" mutagenesis, transplacement, knock out mutagenesis or knock in mutagenesis. Integration of a nucleic acid sequence into a host genome is a single cross over event, which inserts the entire nucleic acid reagent (e.g., pop in). A second cross over event excises all but a portion of the nucleic acid reagent, leaving behind a heterologous sequence, often referred to as a "footprint" (e.g., pop out). Mutagenesis by insertion (e.g., knock in) or by double recombination leaving behind a disrupting heterologous nucleic acid (e.g., knock out) both server to disrupt or "knock out" the function of the gene or nucleic acid sequence in which insertion occurs. By combining selectable markers and/or auxotrophic markers with nucleic acid reagents designed to provide the appropriate nucleic acid target sequences, the artisan can target a selectable nucleic acid reagent to a specific region, and then select for recombination events that "pop out" a portion of the inserted (e.g., "pop in") nucleic acid reagent.

Such methods take advantage of nucleic acid reagents that have been specifically designed with known target nucleic acid sequences at or near a nucleic acid or genomic region of interest. Popping out typically leaves a "foot print" of left over sequences that remain after the recombination event. The left over sequence can disrupt a gene and thereby reduce or eliminate expression of that gene. In some embodiments, the method can be used to insert sequences, upstream or downstream of genes that can result in an enhancement or reduction in expression of the gene. In certain embodiments, new genes can be introduced into the genome of a host organism using similar recombination or "pop in" methods. An example of a yeast recombination system using the ura3 gene and 5-FOA were described briefly above and further detail is presented herein.

A method for modification is described in Alani et al., "A method for gene disruption that allows repeated use of URA3 selection in the construction of multiply disrupted yeast strains", Genetics 116(4):541-545 August 1987. The original method uses a Ura3 cassette with 1000 base pairs (bp) of the same nucleotide sequence cloned in the same orientation on either side of the URA3 cassette. Targeting sequences of about 50 bp are added to each side of the construct. The double stranded targeting sequences are complementary to sequences in the genome of the host organism. The targeting sequences allow site-specific recombination in a region of interest. The modification of the original technique replaces the two 1000 bp sequence direct repeats with two 200 bp direct repeats. The modified method also uses 50 bp targeting sequences. The modification reduces or eliminates recombination of a second knock out into the 1000 bp repeat left behind in a first mutagenesis, therefore allowing multiply knocked out yeast. Additionally, the 200 bp sequences used herein are uniquely designed, self-assembling sequences that leave behind identifiable footprints. The technique used to design the sequences incorporate design features such as low identity to the yeast genome, and low identity to each other. Therefore a library of the self-assembling sequences can be generated to allow multiple knockouts in the same organism, while reducing or eliminating the potential for integration into a previous knockout. As noted above, the URA3 cassette makes use of the toxicity of 5-FOA in yeast carrying a functional URA3 gene. Uracil synthesis deficient yeast are transformed with the modified URA3 cassette, using standard yeast transformation protocols, and the transformed cells are plated on minimal media minus uracil. In some embodiments, PCR can be used to verify correct insertion into the region of interest in the host genome, and certain embodiments the PCR step can be omitted. Inclusion of the PCR step can reduce the number of transformants that need to be counter selected to "pop out" the URA3 cassette. The transformants (e.g., all or the ones determined to be correct by PCR, for example) can then be counter-selected on media containing 5-FOA, which will select for recombination out (e.g., popping out) of the URA3 cassette, thus rendering the yeast ura3 deficient again, and resistant to 5-FOA toxicity. Targeting sequences used to direct recombination events to specific regions are presented herein. A modification of the method described above can be used to integrate genes in to the chromosome, where after recombination a functional gene is left in the chromosome next to the 200bp footprint.

In some embodiments, other auxotrophic or dominant selection markers can be used in place of URA3 (e.g., an auxotrophic selectable marker), with the appropriate change in selection media and selection agents. Auxotrophic selectable markers are used in strains deficient for synthesis of a required biological molecule (e.g., amino acid or nucleoside, for example). Non-limiting examples of additional auxotrophic markers include; HIS3, TRP1, LEU2, LEU2-d, and LYS2. Certain auxotrophic markers (e.g., URA3 and LYS2) allow counter selection to select for the second recombination event that pops out all but one of the direct repeats of the recombination construct. HIS3 encodes an activity involved in histidine synthesis. TRP1 encodes an activity involved in tryptophan synthesis. LEU2 encodes an activity involved in leucine synthesis. LEU2-d is a low expression version of LEU2 that selects for increased copy number (e.g., gene or plasmid copy number, for example) to allow survival on minimal media without leucine. LYS2 encodes an activity involved in lysine synthesis, and allows counter selection for recombination out of the LYS2 gene using alpha-amino adipate (α-amino adipate).

Dominant selectable markers are useful because they also allow industrial and/or prototrophic strains to be used for genetic manipulations. Additionally, dominant selectable markers provide the advantage that rich medium can be used for plating and culture growth, and thus growth rates are markedly increased. Non-limiting examples of dominant selectable markers include; Tn903 kan^{r}, Cm^{r}, Hyg^{r}, CUP1, and DHFR. Tn903 kan^{r} encodes an activity involved in kanamycin antibiotic resistance (e.g., typically neomycin phosphotransferase II or NPTII, for example). Cm^{r} encodes an activity involved in chloramphenicol antibiotic resistance (e.g., typically chloramphenicol acetyl transferase or CAT, for example). Hyg^{r} encodes an activity involved in hygromycin resistance by phosphorylation of hygromycin B (e.g., hygromycin phosphotransferase, or HPT). CUP1 encodes an activity involved in resistance to heavy metal (e.g., copper, for example) toxicity. DHFR encodes a dihydrofolate reductase activity which confers resistance to methotrexate and sulfanilamde compounds.

In contrast to site-directed or specific mutagenesis, random mutagenesis does not require any sequence information and can be accomplished by a number of widely different methods. Random mutagenesis often is used to create mutant libraries that can be used to screen for the desired genotype or phenotype. Non-limiting examples of random mutagenesis include; chemical mutagenesis, UV-induced mutagenesis, insertion element or transposon-mediated mutagenesis, DNA shuffling, error-prone PCR mutagenesis, and the like.

Chemical mutagenesis often involves chemicals like ethyl methanesulfonate (EMS), nitrous acid, mitomycin C, N-methyl-N-nitrosourea (MNU), diepoxybutane (DEB), 1, 2, 7, 8- diepoxyoctane (DEO), methyl methane sulfonate (MMS), N-methyl- N'-nitro-N-nitrosoguanidine (MNNG), 4-nitroquinoline 1-oxide (4-NQO), 2-methyloxy-6-chloro-9(3-[ethyl-2-chloroethyl]-aminopropylamino)-acridinedihydrochloride (ICR-170), 2-amino purine (2AP), and hydroxylamine (HA), provided herein as non-limiting examples. These chemicals can cause base-pair subsitutions, frameshift mutations, deletions, transversion mutations, transition mutations, incorrect replication, and the like. In some embodiments, the mutagenesis can be carried out in vivo. Sometimes the mutagenic process involves the use of the host organisms DNA replication and repair mechanisms to incorporate and replicate the mutagenized base or bases.

Another type of chemical mutagenesis involves the use of base-analogs. The use of base-analogs cause incorrect base pairing which in the following round of replication is corrected to a mismatched nucleotide when compared to the starting sequence. Base analog mutagenesis introduces a small amount of non-randomness to random mutagenesis, because specific base analogs can be chose which can be incorporated at certain nucleotides in the starting sequence. Correction of the mispairing typically yields a known substitution. For example, Bromodeoxyuridine (BrdU) can be incorporated into DNA and replaces T in the sequence. The host DNA repair and replication machinery can sometime correct the defect, but sometimes will mispair the BrdU with a G. The next round of replication then causes a G-C transversion from the original A-T in the native sequence.

Ultra violet (UV) induced mutagenesis is caused by the formation of thymidine dimers when UV light irradiates chemical bonds between two adjacent thymine residues. Excision repair mechanism of the host organism correct the lesion in the DNA, but occasionally the lesion is incorrectly repaired typically resulting in a C to T transition.

Insertion element or transposon-mediated mutagenesis makes use of naturally occurring or modified naturally occurring mobile genetic elements. Transposons often encode accessory activities in addition to the activities necessary for transposition (e.g., movement using a transposase activity, for example). In many examples, transposon accessory activities are antibiotic resistance markers (e.g., see Tn903 kan^{r} described above, for example). Insertion elements typically only encode the activities necessary for movement of the nucleic acid sequence. Insertion element and transposon mediated mutagenesis often can occur randomly, however specific target sequences are known for some transposons. Mobile genetic elements like IS elements or Transposons (Tn) often have inverted repeats, direct repeats or both inverted and direct repeats flanking the region coding for the transposition genes. Recombination events catalyzed by the transposase cause the element to remove itself from the genome and move to a new location, leaving behind a portion of an inverted or direct repeat. Classic examples of transposons are the "mobile genetic elements" discovered in maize. Transposon mutagenesis kits are commercially available which are designed to leave behind a 5 codon insert (e.g., Mutation Generation System kit, Finnzymes, World Wide Web URL finnzymes.us, for example). This allows the artisan to identify the insertion site, without fully disrupting the function of most genes.

DNA shuffling is a method which uses DNA fragments from members of a mutant library and reshuffles the fragments randomly to generate new mutant sequence combinations. The fragments are typically generated using DNasel, followed by random annealing and re-joining using self priming PCR. The DNA overhanging ends, from annealing of random fragments, provide "primer" sequences for the PCR process. Shuffling can be applied to libraries generated by any of the above mutagenesis methods.

Error prone PCR and its derivative rolling circle error prone PCR uses increased magnesium and manganese concentrations in conjunction with limiting amounts of one or two nucleotides to reduce the fidelity of the Taq polymerase. The error rate can be as high as 2% under appropriate conditions, when the resultant mutant sequence is compared to the wild type starting sequence. After amplification, the library of mutant coding sequences must be cloned into a suitable plasmid.

Although point mutations are the most common types of mutation in error prone PCR, deletions and frameshift mutations are also possible. There are a number of commercial error-prone PCR kits available, including those from Stratagene and Clontech (e.g., World Wide Web URL strategene.com and World Wide Web URL clontech.com, respectively, for example). Rolling circle error-prone PCR is a variant of error-prone PCR in which wild-type sequence is first cloned into a plasmid, then the whole plasmid is amplified under error-prone conditions.

As noted above, organisms with altered activities can also be isolated using genetic selection and screening of organisms challenged on selective media or by identifying naturally occurring variants from unique environments. For example, 2-Deoxy-D-glucose is a toxic glucose analog. Growth of yeast on this substance yields mutants that are glucose-deregulated. A number of mutants have been isolated using 2-Deoxy-D-glucose including transport mutants, and mutants that ferment glucose and galactose simultaneously instead of glucose first then galactose when glucose is depleted. Similar techniques have been used to isolate mutant microorganisms that can metabolize plastics (e.g., from landfills), petrochemicals (e.g., from oil spills), and the like, either in a laboratory setting or from unique environments.

Similar methods can be used to isolate naturally occurring mutations in a desired activity when the activity exists at a relatively low or nearly undetectable level in the organism of choice, in some embodiments. The method generally consists of growing the organism to a specific density in liquid culture, concentrating the cells, and plating the cells on various concentrations of the substance to which an increase in metabolic activity is desired. The cells are incubated at a moderate growth temperature, for 5 to 10 days. To enhance the selection process, the plates can be stored for another 5 to 10 days at a low temperature. The low temperature sometimes can allow strains that have gained or increased an activity to continue growing while other strains are inhibited for growth at the low temperature. Following the initial selection and secondary growth at low temperature, the plates can be replica plated on higher or lower concentrations of the selection substance to further select for the desired activity.

A native, heterologous or mutagenized polynucleotide can be introduced into a nucleic acid reagent for introduction into a host organism, thereby generating an engineered microorganism. Standard recombinant DNA techniques (restriction enzyme digests, ligation, and the like) can be used by the artisan to combine the mutagenized nucleic acid of interest into a suitable nucleic acid reagent capable of (i) being stably maintained by selection in the host organism, or (ii) being integrating into the genome of the host organism. As noted above, sometimes nucleic acid reagents comprise two replication origins to allow the same nucleic acid reagent to be manipulated in bacterial before final introduction of the final product into the host organism (e.g., yeast or fungus for example). Standard molecular biology and recombinant DNA methods are known (e.g., described in Maniatis, T., E. F. Fritsch and J. Sambrook (1982) Molecular Cloning: a Laboratory Manual; Cold Spring Harbor Laboratory, Cold Spring Harbor, N.Y.).

Nucleic acid reagents can be introduced into microorganisms using various techniques. Non-limiting examples of methods used to introduce heterologous nucleic acids into various organisms include; transformation, transfection, transduction, electroporation, ultrasound-mediated transformation, particle bombardment and the like. In some instances the addition of carrier molecules (e.g., bis-benzimdazolyl compounds, for example, see US Patent 5595899) can increase the uptake of DNA in cells typically though to be difficult to transform by conventional methods. Conventional methods of transformation are known (e.g., described in Maniatis, T., E. F. Fritsch and J. Sambrook (1982) Molecular Cloning: a Laboratory Manual; Cold Spring Harbor Laboratory, Cold Spring Harbor, N.Y.).

### Culture, Production and Process Methods

Engineered microorganisms often are cultured under conditions that optimize yield of a target molecule (e.g., six-carbon target molecule). Non-limiting examples of such target molecules are adipic acid and 6-hydroxyhexanoic acid. Culture conditions often optimize activity of one or more of the following activities: 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase activity, hexanoate synthase activity, lipase activity, fatty acid synthase activity, acetyl CoA carboxylase activity, monooxygenase activity, monooxygenase reductase activity, fatty alcohol oxidase, acyl-CoA ligase, acyl-CoA oxidase, enoyl-CoA hydratase, 3-hydroxyacyl-CoA dehydrogenase, and/or acetyl-CoA C-acyltransferase activities. In general, non-limiting examples of conditions that may be optimized include the type and amount of carbon source, the type and amount of nitrogen source, the carbon-to-nitrogen ratio, the oxygen level, growth temperature, pH, length of the biomass production phase, length of target product accumulation phase, and time of cell harvest.

Culture media generally contain a suitable carbon source. Carbon sources useful for culturing microorganisms and/or fermentation processes sometimes are referred to as feedstocks. The term "feedstock" as used herein refers to a composition containing a carbon source that is provided to an organism, which is used by the organism to produce energy and metabolic products useful for growth. A feedstock may be a natural substance, a "man-made substance," a purified or isolated substance, a mixture of purified substances, a mixture of unpurified substances or combinations thereof. A feedstock often is prepared by and/or provided to an organism by a person, and a feedstock often is formulated prior to administration to the organism. A carbon source may include, but is not limited to including, one or more of the following substances: monosaccharides (e.g., also referred to as "saccharides," which include 6-carbon sugars (e.g., glucose, fructose), 5-carbon sugars (e.g., xylose and other pentoses) and the like), disaccharides (e.g., lactose, sucrose), oligosaccharides (e.g., glycans, homopolymers of a monosaccharide), polysaccharides (e.g., starch, cellulose, heteropolymers of monosaccharides or mixtures thereof), sugar alcohols (e.g., glycerol), and renewable feedstocks (e.g., cheese whey permeate, cornsteep liquor, sugar beet molasses, barley malt).

A carbon source also may include a metabolic product that can be used directly as a metabolic substrate in an engineered pathway described herein, or indirectly via conversion to a different molecule using engineered or native biosynthetic pathways in an engineered microorganism. In some embodiments, a carbon source may include glycerol backbones generated by the action of an engineered pathway including at least a lipase activity. In certain embodiments, metabolic pathways can be preferentially biased towards production of a desired product by increasing the levels of one or more activities in one or more metabolic pathways having and/or generating at least one common metabolic and/or synthetic substrate. In some embodiments, a metabolic byproduct (e.g., glycerol) of an engineered activity (e.g., lipase activity) can be used in one or more metabolic pathways selected from gluconeogenesis, pentose phosphate pathway, glycolysis, fatty acid synthesis, beta oxidation, and omega oxidation, to generate a carbon source that can be converted to adipic acid.

Carbon sources also can be selected from one or more of the following non-limiting examples: paraffin (e.g., saturated paraffin, unsaturated paraffin, substituted paraffin, linear paraffin, branched paraffin, or combinations thereof); alkanes (e.g., hexane), alkenes or alkynes, each of which may be linear, branched, saturated, unsaturated, substituted or combinations thereof (described in greater detail below); linear or branched alcohols (e.g., hexanol); fatty acids (e.g., about 1 carbon to about 60 carbons, including free fatty acids, soap stock, for example); esters of fatty acids; monoglycerides; diglycerides; triglycerides, phospholipids. Non-limiting commercial sources of products for preparing feedstocks include plants or plant products (e.g., vegetable oils (e.g., almond oil, canola oil, cocoa butter, coconut oil, corn oil, cottonseed oil, flaxseed oil, grape seed oil, illipe, olive oil, palm oil, palm olein, palm kernel oil, safflower oil, peanut oil, soybean oil, sesame oil, shea nut oil, sunflower oil walnut oil, the like and combinations thereof) and animal fats (e.g., beef tallow, butterfat, lard, cod liver oil). A carbon source may include a petroleum product and/or a petroleum distillate (e.g., diesel, fuel oils, gasoline, kerosene, paraffin wax, paraffin oil, petrochemicals).

The term "paraffin" as used herein refers to the common name for alkane hydrocarbons, independent of the source (e.g., plant derived, petroleum derived, chemically synthesized, fermented by a microorganism), or carbon chain length. A carbon source sometimes comprises a paraffin, and in some embodiments, a paraffin is predominant in a carbon source (e.g., about 75%, 80%, 85%, 90% or 95% paraffin). A paraffin sometimes is saturated (e.g., fully saturated), sometimes includes one or more unsaturations (e.g., about 1, 2, 3, 4, 5, 6, 7, 8, 9, 10 unsaturations) and sometimes is substituted with one or more non-hydrogen substituents. Non-limiting examples of non-hydrogen substituents include halo, acetyl, =O, =N-CN, =N-OR, =NR, OR, NR₂, SR, SO₂R, SO₂NR₂, NRSO₂R, NRCONR₂, NRCOOR, NRCOR, CN, COOR, CONR₂, OOCR, COR, and NO₂, where each R is independently H, C1-C8 alkyl, C2-C8 heteroalkyl, C1-C8 acyl, C2-C8 heteroacyl, C2-C8 alkenyl, C2-C8 heteroalkenyl, C2-C8 alkynyl, C2-C8 heteroalkynyl, C6-C10 aryl, or C5-C10 heteroaryl, and each R is optionally substituted with halo, =O, =N-CN, =N-OR', =NR', OR', NR'2, SR', SO₂R', SO₂NR'₂, NR'SO₂R' NR'CONR'₂, NR'COOR', NR'COR', CN, COOR', CONR'₂, OOCR', COR', and NO₂, where each R' is independently H, C1-C8 alkyl, C2-C8 heteroalkyl, C1-C8 acyl, C2-C8 heteroacyl, C6-C10 aryl or C5-C10 heteroaryl.

In some embodiments a feedstock is selected according to the genotype and/or phenotype of the engineered microorganism to be cultured. For example, a feedstock rich in 12-carbon fatty acids, 12-carbon dicarboxylic acids or 12-carbon paraffins, or a mixture of 10-carbon and 12-carbon compounds can be useful for culturing yeast strains harboring an alteration that partially blocks beta oxidation by disrupting POX4 activity, as described herein. Non-limiting examples of carbon sources having 10 and/or 12 carbons include fats (e.g., coconut oil, palm kernel oil), paraffins (e.g., alkanes, alkenes, or alkynes) having 10 or 12 carbons, (e.g., decane, dodecane (also referred to as adakane12, bihexyl, dihexyl and duodecane), alkene and alkyne derivatives), fatty acids (decanoic acid, dodecanoic acid), fatty alcohols (decanol, dodecanol), the like, non-toxic substituted derivatives or combinations thereof.

A carbon source sometimes comprises an alkyl, alkenyl or alkynyl compound or molecule (e.g., a compound that includes an alkyl, alkenyl or alkynyl moiety (e.g., alkane, alkene, alkyne)). In certain embodiments, an alkyl, alkenyl or alkynyl molecule, or combination thereof, is predominant in a carbon source (e.g., about 75%, 80%, 85%, 90% or 95% of such molecules). As used herein, the terms "alkyl," "alkenyl" and "alkynyl" include straight-chain (referred to herein as "linear"), branched-chain (referred to herein as "non-linear"), cyclic monovalent hydrocarbyl radicals, and combinations of these, which contain only C and H atoms when they are unsubstituted. Non-limiting examples of alkyl moieties include methyl, ethyl, isobutyl, cyclohexyl, cyclopentylethyl, 2-propenyl, 3-butynyl, and the like. An alkyl that contains only C and H atoms and is unsubstituted sometimes is referred to as "saturated." An alkenyl or alkynyl generally is "unsaturated" as it contains one or more double bonds or triple bonds, respectively. An alkenyl can include any number of double bonds, such as 1, 2, 3, 4 or 5 double bonds, for example. An alkynyl can include any number of triple bonds, such as 1, 2, 3, 4 or 5 triple bonds, for example. Alkyl, alkenyl and alkynyl molecules sometimes contain between about 2 to about 60 carbon atoms (C). For example, an alkyl, alkenyl and alkynyl molecule can include about 1 carbon atom, about 2 carbon atoms, about 3 carbon atoms, about 4 carbon atoms, about 5 carbon atoms, about 6 carbon atoms, about 7 carbon atoms, about 8 carbon atoms, about 9 carbon atoms, about 10 carbon atoms, about 12 carbon atoms, about 14 carbon atoms, about 16 carbon atoms, about 18 carbon atoms, about 20 carbon atoms, about 22 carbon atoms, about 24 carbon atoms, about 26 carbon atoms, about 28 carbon atoms, about 30 carbon atoms, about 32 carbon atoms, about 34 carbon atoms, about 36 carbon atoms, about 38 carbon atoms, about 40 carbon atoms, about 42 carbon atoms, about 44 carbon atoms, about 46 carbon atoms, about 48 carbon atoms, about 50 carbon atoms, about 52 carbon atoms, about 54 carbon atoms, about 56 carbon atoms, about 58 carbon atoms or about 60 carbon atoms. In some embodiments, paraffins can have a mean number of carbon atoms of between about 8 to about 18 carbon atoms (e.g., about 8 carbon atoms, about 9 carbon atoms, about 10 carbon atoms, about 11 carbon atoms, about 12 carbon atoms, about 13 carbon atoms, about 14 carbon atoms, about 15 carbon atoms, about 16 carbon atoms, about 17 carbon atoms and about 18 carbon atoms). A single group can include more than one type of multiple bond, or more than one multiple bond. Such groups are included within the definition of the term "alkenyl" when they contain at least one carbon-carbon double bond, and are included within the term "alkynyl" when they contain at least one carbon-carbon triple bond. Alkyl, alkenyl and alkynyl molecules include molecules that comprise an alkyl, alkenyl and/or alkynyl moiety, and include molecules that consist of an alkyl, alkenyl or alkynyl moiety (i.e., alkane, alkene and alkyne molecules).

Alkyl, alkenyl and alkynyl substituents sometimes contain 1-20C (alkyl) or 2-20C (alkenyl or alkynyl). They can contain about 8-14C or about 10-14C in some embodiments. A single group can include more than one type of multiple bond, or more than one multiple bond. Such groups are included within the definition of the term "alkenyl" when they contain at least one carbon-carbon double bond, and are included within the term "alkynyl" when they contain at least one carbon-carbon triple bond.

Alkyl, alkenyl and alkynyl groups or compounds sometimes are substituted to the extent that such substitution can be synthesized and can exist. Typical substituents include, but are not limited to, halo, acetyl, =O, =N-CN, =N-OR, =NR, OR, NR₂, SR, SO₂R, SO₂NR₂, NRSO₂R, NRCONR₂, NRCOOR, NRCOR, CN, COOR, CONR₂, OOCR, COR, and NO₂, where each R is independently H, C1-C8 alkyl, C2-C8 heteroalkyl, C1-C8 acyl, C2-C8 heteroacyl, C2-C8 alkenyl, C2-C8 heteroalkenyl, C2-C8 alkynyl, C2-C8 heteroalkynyl, C6-C11 aryl, or C5-C11 heteroaryl, and each R is optionally substituted with halo, =O, =N-CN, =N-OR', =NR', OR', NR'₂, SR', SO₂R', SO₂NR'₂, NR'SO₂R', NR'CONR'₂, NR'COOR', NR'COR', CN, COOR', CONR'₂, OOCR', COR', and NO₂, where each R' is independently H, C1-C8 alkyl, C2-C8 heteroalkyl, C1-C8 acyl, C2-C8 heteroacyl, C6-C10 aryl or C5-C10 heteroaryl. Alkyl, alkenyl and alkynyl groups can also be substituted by C1-C8 acyl, C2-C8 heteroacyl, C6-C10 aryl or C5-C10 heteroaryl, each of which can be substituted by the substituents that are appropriate for the particular group.

"Acetylene" or "acetyl" substituents are 2-10C alkynyl groups that are optionally substituted, and are of the formula -C=C-Ri, where Ri is H or C1-C8 alkyl, C2-C8 heteroalkyl, C2-C8 alkenyl, C2-C8 heteroalkenyl, C2-C8 alkynyl, C2-C8 heteroalkynyl, C1-C8 acyl, C2-C8 heteroacyl, C6-C10 aryl, C5-C10 heteroaryl, C7-C12 arylalkyl, or C6-C12 heteroarylalkyl, and each Ri group is optionally substituted with one or more substituents selected from halo, =O, =N-CN, =N-OR', =NR', OR', NR'2, SR', SO₂R', SO₂NR'₂, NR'SO₂R', NR'CONR'₂, NR'COOR', NR'COR', CN, COOR', CONR'₂, OOCR', COR', and NO₂, where each R' is independently H, C1-C6 alkyl, C2-C6 heteroalkyl, C1-C6 acyl, C2-C6 heteroacyl, C6-C10 aryl, C5-C10 heteroaryl, C7-12 arylalkyl, or C6-12 heteroarylalkyl, each of which is optionally substituted with one or more groups selected from halo, C1-C4 alkyl, C1-C4 heteroalkyl, C1-C6 acyl, C1-C6 heteroacyl, hydroxy, amino, and =O; and where two R' can be linked to form a 3-7 membered ring optionally containing up to three heteroatoms selected from N, O and S. In some embodiments, Ri of -C=C-Ri is H or Me.

A carbon source sometimes comprises a heteroalkyl, heteroalkenyl and/or heteroalkynyl molecule or compound (e.g., comprises heteroalkyl, heteroalkenyl and/or heteroalkynyl moiety (e.g., heteroalkane, heteroalkene or heteroalkyne)). "Heteroalkyl", "heteroalkenyl", and "heteroalkynyl" and the like are defined similarly to the corresponding hydrocarbyl (alkyl, alkenyl and alkynyl) groups, but the 'hetero' terms refer to groups that contain one to three O, S or N heteroatoms or combinations thereof within the backbone; thus at least one carbon atom of a corresponding alkyl, alkenyl, or alkynyl group is replaced by one of the specified heteroatoms to form a heteroalkyl, heteroalkenyl, or heteroalkynyl group. The typical and sizes for heteroforms of alkyl, alkenyl and alkynyl groups are generally the same as for the corresponding hydrocarbyl groups, and the substituents that may be present on the heteroforms are the same as those described above for the hydrocarbyl groups. For reasons of chemical stability, it is also understood that, unless otherwise specified, such groups do not include more than two contiguous heteroatoms except where an oxo group is present on N or S as in a nitro or sulfonyl group.

The term "alkyl" as used herein includes cycloalkyl and cycloalkylalkyl groups and compounds, the term "cycloalkyl" may be used herein to describe a carbocyclic non-aromatic compound or group that is connected via a ring carbon atom, and "cycloalkylalkyl" may be used to describe a carbocyclic non-aromatic compound or group that is connected to a molecule through an alkyl linker. Similarly, "heterocyclyl" may be used to describe a non-aromatic cyclic group that contains at least one heteroatom as a ring member and that is connected to the molecule via a ring atom, which may be C or N; and "heterocyclylalkyl" may be used to describe such a group that is connected to another molecule through a linker. The sizes and substituents that are suitable for the cycloalkyl, cycloalkylalkyl, heterocyclyl, and heterocyclylalkyl groups are the same as those described above for alkyl groups. As used herein, these terms also include rings that contain a double bond or two, as long as the ring is not aromatic.

A carbon source sometimes comprises an acyl compound or moiety (e.g., compound comprising an acyl moiety). As used herein, "acyl" encompasses groups comprising an alkyl, alkenyl, alkynyl, aryl or arylalkyl radical attached at one of the two available valence positions of a carbonyl carbon atom, and heteroacyl refers to the corresponding groups where at least one carbon other than the carbonyl carbon has been replaced by a heteroatom chosen from N, O and S. Thus heteroacyl includes, for example, -C(=O)OR and -C(=O)NR₂ as well as -C(=O)-heteroaryl.

Acyl and heteroacyl groups are bonded to any group or molecule to which they are attached through the open valence of the carbonyl carbon atom. Typically, they are C1-C8 acyl groups, which include formyl, acetyl, pivaloyl, and benzoyl, and C2-C8 heteroacyl groups, which include methoxyacetyl, ethoxycarbonyl, and 4-pyridinoyl. The hydrocarbyl groups, aryl groups, and heteroforms of such groups that comprise an acyl or heteroacyl group can be substituted with the substituents described herein as generally suitable substituents for each of the corresponding component of the acyl or heteroacyl group.

A carbon source sometimes comprises one or more aromatic moieties and/or heteroaromatic moieties. "Aromatic" moiety or "aryl" moiety refers to a monocyclic or fused bicyclic moiety having the well-known characteristics of aromaticity; examples include phenyl and naphthyl. Similarly, "heteroaromatic" and "heteroaryl" refer to such monocyclic or fused bicyclic ring systems which contain as ring members one or more heteroatoms selected from O, S and N. The inclusion of a heteroatom permits aromaticity in 5 membered rings as well as 6 membered rings. Typical heteroaromatic systems include monocyclic C5-C6 aromatic groups such as pyridyl, pyrimidyl, pyrazinyl, thienyl, furanyl, pyrrolyl, pyrazolyl, thiazolyl, oxazolyl, and imidazolyl and the fused bicyclic moieties formed by fusing one of these monocyclic groups with a phenyl ring or with any of the heteroaromatic monocyclic groups to form a C8-C10 bicyclic group such as indolyl, benzimidazolyl, indazolyl, benzotriazolyl, isoquinolyl, quinolyl, benzothiazolyl, benzofuranyl, pyrazolopyridyl, quinazolinyl, quinoxalinyl, cinnolinyl, and the like. Any monocyclic or fused ring bicyclic system which has the characteristics of aromaticity in terms of electron distribution throughout the ring system is included in this definition. It also includes bicyclic groups where at least the ring which is directly attached to the remainder of the molecule has the characteristics of aromaticity. Typically, the ring systems contain 5-12 ring member atoms. The monocyclic heteroaryls sometimes contain 5-6 ring members, and the bicyclic heteroaryls sometimes contain 8-10 ring members.

Aryl and heteroaryl moieties may be substituted with a variety of substituents including C1-C8 alkyl, C2-C8 alkenyl, C2-C8 alkynyl, C5-C12 aryl, C1-C8 acyl, and heteroforms of these, each of which can itself be further substituted; other substituents for aryl and heteroaryl moieties include halo, OR, NR₂, SR, SO₂R, SO₂NR₂, NRSO₂R, NRCONR₂, NRCOOR, NRCOR, CN, COOR, CONR₂, OOCR, COR, and NO₂, where each R is independently H, C1-C8 alkyl, C2-C8 heteroalkyl, C2-C8 alkenyl, C2-C8 heteroalkenyl, C2-C8 alkynyl, C2-C8 heteroalkynyl, C6-C10 aryl, C5-C10 heteroaryl, C7-C12 arylalkyl, or C6-C12 heteroarylalkyl, and each R is optionally substituted as described above for alkyl groups. The substituent groups on an aryl or heteroaryl group may be further substituted with the groups described herein as suitable for each type of such substituents or for each component of the substituent. Thus, for example, an arylalkyl substituent may be substituted on the aryl portion with substituents typical for aryl groups, and it may be further substituted on the alkyl portion with substituents as typical or suitable for alkyl groups.

Similarly, "arylalkyl" and "heteroarylalkyl" refer to aromatic and heteroaromatic ring systems, which are stand-alone molecules (e.g., benzene or substituted benzene, pyridine or substituted pyridine), or which are bonded to an attachment point through a linking group such as an alkylene, including substituted or unsubstituted, saturated or unsaturated, cyclic or acyclic linkers. A linker often is C1-C8 alkyl or a hetero form thereof. These linkers also may include a carbonyl group, thus making them able to provide substituents as an acyl or heteroacyl moiety. An aryl or heteroaryl ring in an arylalkyl or heteroarylalkyl group may be substituted with the same substituents described above for aryl groups. An arylalkyl group sometimes includes a phenyl ring optionally substituted with the groups defined above for aryl groups and a C1-C4 alkylene that is unsubstituted or is substituted with one or two C1-C4 alkyl groups or heteroalkyl groups, where the alkyl or heteroalkyl groups can optionally cyclize to form a ring such as cyclopropane, dioxolane, or oxacyclopentane. Similarly, a heteroarylalkyl group often includes a C5-C6 monocyclic heteroaryl group optionally substituted with one or more of the groups described above as substituents typical on aryl groups and a C1-C4 alkylene that is unsubstituted. A heteroarylalkyl group sometimes is substituted with one or two C1-C4 alkyl groups or heteroalkyl groups, or includes an optionally substituted phenyl ring or C5-C6 monocyclic heteroaryl and a C1-C4 heteroalkylene that is unsubstituted or is substituted with one or two C1-C4 alkyl or heteroalkyl groups, where the alkyl or heteroalkyl groups can optionally cyclize to form a ring such as cyclopropane, dioxolane, or oxacyclopentane.

Where an arylalkyl or heteroarylalkyl group is described as optionally substituted, the substituents may be on the alkyl or heteroalkyl portion or on the aryl or heteroaryl portion of the group. The substituents optionally present on the alkyl or heteroalkyl portion sometimes are the same as those described above for alkyl groups, and the substituents optionally present on the aryl or heteroaryl portion often are the same as those described above for aryl groups generally.

"Arylalkyl" groups as used herein are hydrocarbyl groups if they are unsubstituted, and are described by the total number of carbon atoms in the ring and alkylene or similar linker. Thus a benzyl group is a C7-arylalkyl group, and phenylethyl is a C8-arylalkyl.

"Heteroarylalkyl" as described above refers to a moiety comprising an aryl group that is attached through a linking group, and differs from "arylalkyl" in that at least one ring atom of the aryl moiety or one atom in the linking group is a heteroatom selected from N, O and S. The heteroarylalkyl groups are described herein according to the total number of atoms in the ring and linker combined, and they include aryl groups linked through a heteroalkyl linker; heteroaryl groups linked through a hydrocarbyl linker such as an alkylene; and heteroaryl groups linked through a heteroalkyl linker. Thus, for example, C7-heteroarylalkyl includes pyridylmethyl, phenoxy, and N-pyrrolylmethoxy.

"Alkylene" as used herein refers to a divalent hydrocarbyl group. Because an alkylene is divalent, it can link two other groups together. An alkylene often is referred to as -(CH₂)ₙ- where n can be 1-20, 1-10, 1-8, or 1-4, though where specified, an alkylene can also be substituted by other groups, and can be of other lengths, and the open valences need not be at opposite ends of a chain. Thus -CH(Me)- and -C(Me)₂- may also be referred to as alkylenes, as can a cyclic group such as cyclopropan-1,1-diyl. Where an alkylene group is substituted, the substituents include those typically present on alkyl groups as described herein.

In some embodiments, a feedstock includes a mixture of carbon sources, where each carbon source in the feedstock is selected based on the genotype of the engineered microorganism. In certain embodiments, a mixed carbon source feedstock includes one or more carbon sources selected from sugars, cellulose, fatty acids, triacylglycerides, paraffins, the like and combinations thereof.

Nitrogen may be supplied from an inorganic (e.g., (NH.sub.4).sub.2SO.sub.4) or organic source (e.g., urea or glutamate). In addition to appropriate carbon and nitrogen sources, culture media also can contain suitable minerals, salts, cofactors, buffers, vitamins, metal ions (e.g., Mn.sup.+2, Co.sup.+2, Zn.sup.+2, Mg.sup.+2) and other components suitable for culture of microorganisms. Engineered microorganisms sometimes are cultured in complex media (e.g., yeast extract-peptone-dextrose broth (YPD)). In some embodiments, engineered microorganisms are cultured in a defined minimal media that lacks a component necessary for growth and thereby forces selection of a desired expression cassette (e.g., Yeast Nitrogen Base (DIFCO Laboratories, Detroit, Mich.)). Culture media in some embodiments are common commercially prepared media, such as Yeast Nitrogen Base (DIFCO Laboratories, Detroit, Mich.). Other defined or synthetic growth media may also be used and the appropriate medium for growth of the particular microorganism are known. A variety of host organisms can be selected for the production of engineered microorganisms. Non-limiting examples include yeast (e.g., Candida tropicalis (e.g., ATCC20336, ATCC20913, ATCC20962), Candida viswanithii, Yarrowia lipolytica (e.g., ATCC20228)) and filamentous fungi (e.g., Aspergillus nidulans (e.g., ATCC38164) and Aspergillus parasiticus (e.g., ATCC 24690)). In specific embodiments, yeast are cultured in YPD media (10 g/L Bacto Yeast Extract, 20 g/L Bacto Peptone, and 20 g/L Dextrose). Filamentous fungi, in particular embodiments, are grown in CM (Complete Medium) containing 10 g/L Dextrose, 2 g/L Bacto Peptone, 1 g/L Bacto Yeast Extract, 1 g/L Casamino acids, 50 mL /L 20X Nitrate Salts (120 g/L NaNO₃, 10.4 g/L KCI, 10.4 g/L MgSO₄·7 H₂O), 1 mL/L 1000X Trace Elements (22 g/L ZnSO₄·7 H₂O, 11 g/L H₃BO₃, 5 g/L MnCl₂·7 H₂O, 5 g/L FeSO₄·7 H₂O, 1.7 g/L CoCl₂·6 H₂O, 1.6 g/L CuSO₄·5 H₂O, 1.5 g/L Na₂MoO₄·2 H₂O, and 50 g/L Na₄EDTA), and 1 mL/L Vitamin Solution (100 mg each of Biotin, pyridoxine, thiamine, riboflavin, p-aminobenzoic acid, and nicotinic acid in 100 mL water).

A suitable pH range for the fermentation often is between about pH 4.0 to about pH 8.0, where a pH in the range of about pH 5.5 to about pH 7.0 sometimes is utilized for initial culture conditions. Depending on the host organism, culturing may be conducted under aerobic or anaerobic conditions, where microaerobic conditions sometimes are maintained. A two-stage process may be utilized, where one stage promotes microorganism proliferation and another state promotes production of target molecule. In a two-stage process, the first stage may be conducted under aerobic conditions (e.g., introduction of air and/or oxygen) and the second stage may be conducted under anaerobic conditions (e.g., air or oxygen are not introduced to the culture conditions). In some embodiments, the first stage may be conducted under anaerobic conditions and the second stage may be conducted under aerobic conditions. In certain embodiments, a two-stage process may include two more organisms, where one organism generates an intermediate product (e.g., hexanoic acid produced by Megasphera spp.) in one stage and another organism processes the intermediate product into a target product (e.g., adipic acid) in another stage, for example.

A variety of fermentation processes may be applied for commercial biological production of a target product. In some embodiments, commercial production of a target product from a recombinant microbial host is conducted using a batch, fed-batch or continuous fermentation process, for example.

A batch fermentation process often is a closed system where the media composition is fixed at the beginning of the process and not subject to further additions beyond those required for maintenance of pH and oxygen level during the process. At the beginning of the culturing process the media is inoculated with the desired organism and growth or metabolic activity is permitted to occur without adding additional sources (i.e., carbon and nitrogen sources) to the medium. In batch processes the metabolite and biomass compositions of the system change constantly up to the time the culture is terminated. In a typical batch process, cells proceed through a static lag phase to a high-growth log phase and finally to a stationary phase, wherein the growth rate is diminished or halted. Left untreated, cells in the stationary phase will eventually die.

A variation of the standard batch process is the fed-batch process, where the carbon source is continually added to the fermentor over the course of the fermentation process. Fed-batch processes are useful when catabolite repression is apt to inhibit the metabolism of the cells or where it is desirable to have limited amounts of carbon source in the media at any one time. Measurement of the carbon source concentration in fed-batch systems may be estimated on the basis of the changes of measurable factors such as pH, dissolved oxygen and the partial pressure of waste gases (e.g., CO.sub.2).

Batch and fed-batch culturing methods are known in the art. Examples of such methods may be found in Thomas D. Brock in Biotechnology: A Textbook of Industrial Microbiology, 2.sup.nd ed., (1989) Sinauer Associates Sunderland, Mass. and Deshpande, Mukund V., Appl. Biochem. Biotechnol., 36:227 (1992).

In continuous fermentation process a defined media often is continuously added to a bioreactor while an equal amount of culture volume is removed simultaneously for product recovery. Continuous cultures generally maintain cells in the log phase of growth at a constant cell density. Continuous or semi-continuous culture methods permit the modulation of one factor or any number of factors that affect cell growth or end product concentration. For example, an approach may limit the carbon source and allow all other parameters to moderate metabolism. In some systems, a number of factors affecting growth may be altered continuously while the cell concentration, measured by media turbidity, is kept constant. Continuous systems often maintain steady state growth and thus the cell growth rate often is balanced against cell loss due to media being drawn off the culture. Methods of modulating nutrients and growth factors for continuous culture processes, as well as techniques for maximizing the rate of product formation, are known and a variety of methods are detailed by Brock, supra.

In some embodiments involving fermentation, the fermentation can be carried out using two or more microorganisms (e.g., host microorganism, engineered microorganism, isolated naturally occurring microorganism, the like and combinations thereof), where a feedstock is partially or completely utilized by one or more organisms in the fermentation (e.g., mixed fermentation), and the products of cellular respiration or metabolism of one or more organisms can be further metabolized by one or more other organisms to produce a desired target product (e.g., adipic acid, hexanoic acid). In certain embodiments, each organism can be fermented independently and the products of cellular respiration or metabolism purified and contacted with another organism to produce a desired target product. In some embodiments, one or more organisms are partially or completely blocked in a metabolic pathway (e.g., beta oxidation, omega oxidation, the like or combinations thereof), thereby producing a desired product that can be used as a feedstock for one or more other organisms. Any suitable combination of microorganisms can be utilized to carry out mixed fermentation or sequential fermentation. A non-limiting example of an organism combination and feedstock suitable for use in mixed fermentations or sequential fermentations where the fermented media from a first organism is used as a feedstock for a second organism is the use of long chain dicarboxylic acids as a fermentation media for Megasphaera elsdenii to produce hexanoic acid, and Candida tropicalis engineered as described herein to produce adipic acid from the hexanoic acid produced by Megasphaera elsdenii. Megasphaera elsdenii is a facultative anaerobe. Without being limited by theory, it is believe that Megasphaera elsdenii naturally accumulates hexanoic acid as a result of anaerobic respiration. Candida tropicalis can grow aerobically and anaerobically. In some embodiments, the hexanoic acid produced by Megasphaera elsdenii can be utilized as a feedstock for Candida tropicalis to produce adipic acid. In certain embodiments, the Megasphaera produced hexanoic acid is purified (e.g., partially, completely) prior to being used as a feedstock for C. tropicalis.

In various embodiments adipic acid is isolated or purified from the culture media or extracted from the engineered microorganisms. In some embodiments, fermentation of feedstocks by methods described herein can produce a target product (e.g., adipic acid) at a level of about 80% or more of theoretical yield (e.g., 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more of theoretical yield). The term "theoretical yield" as used herein refers to the amount of product that could be made from a starting material if the reaction is 100% complete. Theoretical yield is based on the stoichiometry of a reaction and ideal conditions in which starting material is completely consumed, undesired side reactions do not occur, the reverse reaction does not occur, and there are no losses in the work-up procedure. Culture media may be tested for target product (e.g., adipic acid) concentration and drawn off when the concentration reaches a predetermined level. Detection methods are known in the art, including but not limited to those set forth in B Stieglitz and P J Weimer, Novel microbial screen for detection of 1,4-butanediol, ethylene glycol, and adipic acid, Appl Environ Microbiol. 198. Target product (e.g., adipic acid) may be present at a range of levels as described herein.

A target product sometimes is retained within an engineered microorganism after a culture process is completed, and in certain embodiments, the target product is secreted out of the microorganism into the culture medium. For the latter embodiments, (i) culture media may be drawn from the culture system and fresh medium may be supplemented, and/or (ii) target product may be extracted from the culture media during or after the culture process is completed. Engineered microorganisms may be cultured on or in solid, semi-solid or liquid media. In some embodiments media is drained from cells adhering to a plate. In certain embodiments, a liquid-cell mixture is centrifuged at a speed sufficient to pellet the cells but not disrupt the cells and allow extraction of the media, as known in the art. The cells may then be resuspended in fresh media. Target product may be purified from culture media according to known methods, such as those described in U.S. Pat. No. 6,787,669 and U.S. Pat. No. 5,296,639, for example.

In certain embodiments, target product is extracted from the cultured engineered microorganisms. The micoorganism cells may be concentrated through centrifugation at a speed sufficient to shear the cell membranes. In some embodiments, the cells may be physically disrupted (e.g., shear force, sonication) or chemically disrupted (e.g., contacted with detergent or other lysing agent). The phases may be separated by centrifugation or other method known in the art and target product may be isolated according to known methods.

Commercial grade target product sometimes is provided in substantially pure form (e.g., 90% pure or greater, 95% pure or greater, 99% pure or greater or 99.5% pure or greater). In some embodiments, target product may be modified into any one of a number of downstream products. For example, adipic acid may be polycondensed with hexamethylenediamine to produce nylon. Nylon may be further processed into fibers for applications in carpeting, automobile tire cord and clothing. Adipic acid is also used for manufacturing plasticizers, lubricant components and polyester polyols for polyurethane systems. Food grade adipic acid is used as a gelling aid, acidulant, leavening and buffering agent. Adipic acid has two carboxylic acid (-COOH) groups, which can yield two kinds of salts. Its derivatives, acyl halides, anhydrides, esters, amides and nitriles, are used in making downstream products such as flavoring agents, internal plasticizers, pesticides, dyes, textile treatment agents, fungicides, and pharmaceuticals through further reactions of substitution, catalytic reduction, metal hydride reduction, diborane reduction, keto formation with organometallic reagents, electrophile bonding at oxygen, and condensation.

Target product may be provided within cultured microbes containing target product, and cultured microbes may be supplied fresh or frozen in a liquid media or dried. Fresh or frozen microbes may be contained in appropriate moisture-proof containers that may also be temperature controlled as necessary. Target product sometimes is provided in culture medium that is substantially cell-free. In some embodiments target product or modified target product purified from microbes is provided, and target product sometimes is provided in substantially pure form. In certain embodiments crystallized or powdered target product is provided. Crystalline adipic acid is a white powder with a melting point of 360°F and may be transported in a variety of containers including one ton cartons, drums, 50 pound bags and the like.

In certain embodiments, a target product (e.g., adipic acid, 6-hydroxyhexanoic acid) is produced with a yield of about 0.30 grams of target product, or greater, per gram of glucose added during a fermentation process (e.g., about 0.31 grams of target product per gram of glucose added, or greater; about 0.32 grams of target product per gram of glucose added, or greater; about 0.33 grams of target product per gram of glucose added, or greater; about 0.34 grams of target product per gram of glucose added, or greater; about 0.35 grams of target product per gram of glucose added, or greater; about 0.36 grams of target product per gram of glucose added, or greater; about 0.37 grams of target product per gram of glucose added, or greater; about 0.38 grams of target product per gram of glucose added, or greater; about 0.39 grams of target product per gram of glucose added, or greater; about 0.40 grams of target product per gram of glucose added, or greater; about 0.41 grams of target product per gram of glucose added, or greater; 0.42 grams of target product per gram of glucose added, or greater; 0.43 grams of target product per gram of glucose added, or greater; 0.44 grams of target product per gram of glucose added, or greater; 0.45 grams of target product per gram of glucose added, or greater; 0.46 grams of target product per gram of glucose added, or greater; 0.47 grams of target product per gram of glucose added, or greater; 0.48 grams of target product per gram of glucose added, or greater; 0.49 grams of target product per gram of glucose added, or greater; 0.50 grams of target product per gram of glucose added, or greater; 0.51 grams of target product per gram of glucose added, or greater; 0.52 grams of target product per gram of glucose added, or greater; 0.53 grams of target product per gram of glucose added, or greater; 0.54 grams of target product per gram of glucose added, or greater; 0.55 grams of target product per gram of glucose added, or greater; 0.56 grams of target product per gram of glucose added, or greater; 0.57 grams of target product per gram of glucose added, or greater; 0.58 grams of target product per gram of glucose added, or greater; 0.59 grams of target product per gram of glucose added, or greater; 0.60 grams of target product per gram of glucose added, or greater; 0.61 grams of target product per gram of glucose added, or greater; 0.62 grams of target product per gram of glucose added, or greater; 0.63 grams of target product per gram of glucose added, or greater; 0.64 grams of target product per gram of glucose added, or greater; 0.65 grams of target product per gram of glucose added, or greater; 0.66 grams of target product per gram of glucose added, or greater; 0.67 grams of target product per gram of glucose added, or greater; 0.68 grams of target product per gram of glucose added, or greater; 0.69 or 0.70 grams of target product per gram of glucose added or greater).

### Examples

The examples set forth below illustrate certain embodiments and do not limit the technology. Certain examples set forth below utilize standard recombinant DNA and other biotechnology protocols known in the art. Many such techniques are described in detail in Maniatis, T., E. F. Fritsch and J. Sambrook (1982) Molecular Cloning: a Laboratory Manual; Cold Spring Harbor Laboratory, Cold Spring Harbor, N.Y. DNA mutagenesis can be accomplished using the Stratagene (San Diego, CA) "QuickChange" kit according to the manufacturer's instructions.

### Example 1: Cloning Hexanoate Synthase ("HexS") Subunit Genes

Total RNA from Aspergillus parasiticus was prepared using the RiboPure™ (Ambion, Austin, TX) kit for yeast. The genes encoding the two subunits of hexanoate synthase (referred to as "hexA" and "hex B" were isolated from this total RNA using the 2-step RT-PCR method with Superscript III reverse transcriptase (Life Technologies, Carlsbad, CA) and the fragments were gel purified. The primers used for each RT-PCR reaction are as follows:

**HexA Aspergillus parasiticus primers**

| | |
|---|---|
| SP_HexA_Apar_1_1149 | ATGGTCATCCAAGGGAAGAGATTGGCCGCCTCCTCTATTCAGC |
| AS_P_HexA_Apar_1_1149 | GTAGGCGTCACAGGAAAGACTGCGTACCA |
| SP_HexA_Apar_941_2270 | TATCACCAATGCTGGATGTAAAGAAGTCGCG |
| ASP_HexA_Apar_941_2270 | AATTGGGCTAGGAAACCGGGGATGC |
| SP_HexA_Apar_2067_3016 | CGGTCTAATGACGGCGCATGATATCATAGCCGAAACGGTCGAG |
| ASP_HexA_Apar_2067_3016 | ACTTGGCTGGAGTCCATCCCTTCGGCA |
| SP_HexA_Apar_2812_4181 | CTGCCCGAGTTTGAAGTATCTCAACTTACCGCCGACGCCATG |
| ASP_HexA_Apar_2812_4181 | TGAGACGCGCTGCGCAGGGC |
| SP_HexA_Apar_3975_5016 | CGAGGTGATCGAGACGCAGATGC |
| ASP_HexA_Apar_3975_5016 | TTATGAAGCACCAGACATCAGCCCCAGC |

**HexB Aspergillus parasiticus primers**

| | |
|---|---|
| SP_HexB_Apar_1_1166 | ATGGGTTCCGTTAGTAGGGAACATGAGTCAATC |
| ASP_HexB_Apar_1_1166 | GTTCCTTGTGTGAGCTCCTGAATAAGACTGCATG |
| SP_HexB_Apar_962_2042 | CCATCAAAATCCCCCTCTATCACACGGGCACTGGGAGCAAC |
| ASP_HexB_Apar_962_2042 | CCCACGCCTTGCGCATCTATAATCAGG |
| SP_HexB_Apar_1837_3527 | TGTCCGAATATTCTCCTCGTTGTAGGTAGTGGATT |
| ASP_HexB_Apar_1837_3527 | GCAGTAGTCGATAGGTACACATCCTTGGGGGTTCCATGACTGC |
| SP_HexB_Apar_3322_4460 | AGAGGATCAAGGCATTATACATGAGTCTGTGGAACTTGGGCTTTCC |
| ASP_HexB_Apar_3322_4460 | TTCCCCGTCCTCCATGGCCTTATGC |
| SP_HexB_Apar_4256_5667 | GGCCTTTGCGCGATACGCTGGTCTCTCGGGTCCCAT |
| ASP_HexB_Apar_4256_5667 | TCACGCCATTTGTTGAAGCAGGGAATG |

Each of the fragments was inserted separately into the plasmid pCRBlunt II (Life Technologies, Carlsbad, CA) such that there were four hexA plasmids, each with a different hexA gene fragment, and five hex B plasmids each with a different hexB gene fragment. Each hexA and hexB fragment was sequence verified, after which the fragments were PCR cloned from each plasmid. Overlap PCR was then used to create the full length hexA and hexB genes. The hexA gene was inserted into the vector p425GPD which has a LEU2 selectable marker and a glyceraldehyde 3-phosphate dehydrogenase promoter (American Type Culture Collection) and the hexB full length gene was inserted into p426GPD which has a URA3 selectable marker and a glyceraldehyde 3-phosphate dehydrogenase promoter (American Type Culture Collection).

### Example 2: Transformation of Saccharomyces cerevisiae with HexA and HexB Genes

Saccharomyces cereviseae cells (strain BY4742, ATCC Accession Number 201389) were grown in standard YPD (10g Yeast Extract, 20g Bacto-Peptone, 20g Glucose, 1 L total) media at about 30 degrees Celsius for about 3 days. The plasmids containing the hexA and hexB genes were co-transformed into the Saccharomyces cerevisiae. Transformation was accomplished using the Zymo kit (Catalog number T2001; Zymo Research Corp., Orange, CA 92867) using 1µg plasmid DNA and cultured on SC drop out media with glucose (minus uracil and minus leucine) (20g glucose; 2.21g SC (-URA,-LEU) dry mix, 6.7g Yeast Nitrogen Base, 1 L total) for 2-3 days at about 30°C.

SC(-URA) mix contains:

| | |
|---|---|
| 0.4g | Adenine hemisulfate |
| 3.5g | Arginine |
| 1g | Glutamic Acid |
| 0.433g | Histidine |
| 0.4g | Myo-Inositol |
| 5.2g | Isoleucine |
| 0.9g | Lysine |
| 1.5g | Methionine |
| 0.8g | Phenylalanine |
| 1.1g | Serine |
| 1.2g | Threonine |
| 0.8g | Tryptophan |
| 0.2g | Tyrosine |
| 1.2g | Valine |

When needed:

| | |
|---|---|
| 0.263g | Leucine |
| 0.2g | Uracil |

Co-transformants were selected and established as liquid cultures in YPD media under standard conditions.

### Example 3: Production of Synthetic HexA and HexB Genes

Synthetic hexaonoate synthase subunit genes were designed for use in Candida tropicalis. This organism uses an alternate genetic code in which the codon "CTG" encodes serine instead of leucine. Therefore, all "CTG" codons were replaced with the codon "TTG" to ensure that these genes, when translated by C. tropicalis, would generate polypeptides with amino acid sequences identical to the wild type polypeptides found in A. parasiticus. Due to the large size of each subunit, each was synthesized as four fragments, and each fragment was inserted into the vector pUC57. PCR was used to clone each fragment, and overlap extension PCR was then used to generate each full length gene.

The sequence of the synthetic gene for each hexanoate synthase subunit is set forth below. The synthetic gene encoding the hexA subunit is referred to as hexA-AGC ("Alternate Genetic Code") and the synthetic gene encoding the hexB subunit is referred to as hexB-AGC.
hexA-AGC for Candida tropicalis SEQ ID NO: 35 
atggtcatccaaggaagagattggccgcctcctctattcagcttctcgcaagctc
gttagacgcgaagaagctttgttatgagtatgacgagaggcaagccccaggtgtaa
cccaaatcaccgaggaggcgcctacagagcaaccgcctctctctacccctccctcg
ctaccccaaacgcccaatatttcgcctataagtgcttcaaagatcgtgatcgacga
tgtggcgctatctcgagtgcaaattgttcaggctcttgttgccagaaagttgaaga
cggcaattgctcagcttcctacatcaaagtcaatcaaagagttgtcgggtggtcgg
tcttctttgcagaacgagctcgtgggggatatacacaacgagttcagctccatccc
ggatgcaccagagcagatcttgttgcgggactttggcgacgccaacccaacagtgc
aattggggaaaacgtcctccgcggcagttgccaaactaatctcgtccaagatgcct
agtgacttcaacgccaacgctattcgagcccacctagcaaacaagtggggtctagg
acccttgcgacaaacagcggtgttgctctacgccattgcgtcagaaccccccatcgc
gtttagcttcatcgagcgcagcggaagagtactgggacaacgtgtcatccatgtac
gccgaatcgtgtggcatcaccctccgcccgagacaagacactatgaatgaagatgc
tatggcatcgtcggcgattgatccggctgtggtagccgagttttccaaggggcacc
gtaggctcggagttcaacagttccaagcgctagcagaatacttacaaattgatttg
tcggggtctcaagcctctcagtcggatgctttggtggcggaacttcagcagaaagt
cgatctctggacggccgaaatgacccccgagtttctcgccgggatatcaccaatgt
tggatgtaaagaagtcgcgacgctatggctcgtggtggaacatggcacggcaggat
gtcttggccttctatcgccgtccttcctacagtgaattcgtggacgacgccttggc
cttcaaagtttttctcaatcgtctctgtaaccgagctgatgaggccctcctcaaca
tggtacgcagtctttcctgtgacgcctacttcaagcaaggttctttgcccggatat
catgccgcctcgcgactccttgagcaggccatcacatccacagtggcggattgccc
gaaggcacgcctcattctcccggcggtggcccccacaccaccattacaaaggacg
gcacgattgaatacgcggaggcaccgcgccagggagtgagtggtcccactgcgtac
atccagtctctccgccaaggcgcatctttcattggtctcaagtcagccgacgtcga
tactcagagcaacttgaccgacgctttgcttgacgccatgtgcttagcactccata
atggaatctcgtttgttggtaaaacctttttggtgacggagcgggtcaggggtca
ataggagcgggagtggtgcgtctattgttagagggaggagcccgagtattggtgac
gacgagcagggagccggcgacgacatccagatacttccagcagatgtacgataatc
 
acggtgcgaagttctccgagttgcgggtagttccttgcaatctagccagcgcccaa
gattgcgaagggttgatccggcacgtctacgatccccgtgggctaaattgggattt
ggatgccatccttcccttcgctgccgcgtccgactacagcaccgagatgcatgaca
ttcggggacagagcgagttgggccaccggctaatgttggtcaatgtcttccgcgtg
ttggggcatatcgtccactgtaaacgagatgccggggttgactgccatccgacgca
ggtgttgttgccattgtcgccaaatcacggcatcttcggtggcgatgggatgtatc
cggagtcaaagctagcccttgagagcttgttccatcgcatccgatcagagtcttgg
tcagaccagttatctatatgcggcgttcgtatcggttggacccggtcgaccggtct
aatgacggcgcatgatatcatagccgaaacggtcgaggaacacggaatacgcacat
tttccgtggccgagatggcactcaacatagccatgttgttaaccccgactttgtg
gcccattgtgaagatggacctttggatgccgatttcaccggcagcttgggaacatt
gggtagcatccccggtttcctagcccaattgcaccagaaagtccagttggcagccg
aggtgatccgtgccgtgcaggccgaggatgagcatgagagattcttgtctccggga
acaaaacctaccttgcaagcacccgtggccccaatgcacccccgcagtagccttcg
tgtaggctatccccgtctccccgattatgagcaagagattcgcccgttgtccccac
ggttggaaaggttgcaagatccggccaatgctgtggtggtggtcgggtactcggag
ttggggccatggggtagcgcgcgattacggtgggaaatagagagccaggccagtg
gacttcagccggttatgtcgaacttgcctggttgatgaacctcatccgccacgtca
acgatgaatcctacgtcggctgggtggatactcagaccggaaagccagtgcgggat
ggcgagatccaggcattgtacggggaccacattgacaaccacaccggtatccgtcc
tatccagtccacctcgtacaacccagagcgcatggaggtcttgcaggaggtcgctg
tcgaggaggatttgcccgagtttgaagtatctcaacttaccgccgacgccatgcgt
ctccgccatggagctaacgtttccatccgccccagtggaaatcccgacgcatgcca
cgtgaagcttaaacgaggcgctgttatccttgttcccaagacagttccctttgttt
ggggatcgtgtgccggtgagttgccgaaggatggactccagccaagtacggcatc
cctgagaacctaattcatcaggtcgaccccgtcacgctctatacaatttgctgcgt
ggcggaggcattttacagtgccggtataactcaccctcttgaggtctttcgacaca
ttcacctctcggaactaggcaactttatcggatcctccatgggtgggccgacgaag
actcgtcagctctaccgagatgtctacttcgaccatgagattccgtcggatgtttt
gcaagacacttatctcaacacacctgctgcctgggttaatatgctactccttggct
gcacggggccgatcaaaactcccgtcggcgcatgtgccaccggggtcgagtcgatc
gattccggctacgagtcaatcatggcgggcaagacaaagatgtgtcttgtgggtgg
ctacgacgatttgcaggaggaggcatcgtatggattcgcacaacttaaggccacgg
tcaacgttgaagaggagatcgcctgcggtcgacagccctcggagatgtcgcgcccc
atggctgagagtcgtgctggctttgtcgaggcgcatggctgcggtgtacagttgtt
gtgtcgaggtgacatcgccttgcaaatgggtcttcctatctatgcggtcattgcca
gctcagccatggccgccgacaagatcggttcctcggtgccagcaccggccagggc
attctaagcttctcccgtgagcgcgctcgatccagtatgatatccgtcacgtcgcg
cccgagtagccgtagcagcacatcatctgaagtctcggacaaatcatccttgacct
caatcacctcaatcagcaatcccgctcctcgtgcacaacgcgcccgatccaccact
gatatggctccgttgcgagcagcgcttgcgacttgggggttgactatcgacgactt
ggatgtggcctcattgcacggcacctcgacgcgcggtaacgatctcaatgagcccg
aggtgatcgagacgcagatgcgccatttaggtcgcactcctggccgccccttgtgg
gccatctgccaaaagtcagtgacgggacaccctaaagcccagcggccgcatggat
gctcaatggatgcttgcaagtattggactcggggttggtgccgggcaaccgcaatc
ttgacacgttggacgaggccttgcgcagcgcgtctcatctctgcttccctacgcgc
accgtgcagctacgtgaggtcaaggcattcttgttgacctcatttggcttcggaca
gaagggggggccaagtcgtcggcgttgcccccaagtacttctttgctacgctcccc
gccccgaggttgagggctactatcgcaaggtgagggttcgaaccgaggcgggtgat
cgcgcctacgccgcggcggtcatgtcgcaggcggtggtgaagatccagacgcaaaa
cccgtacgacgagccggatgcccccgcatttttctcgatcccttggcacgtatct
cccaggatccgtcgacggccagtatcggtttcgttccgatgccactcccgccctc
gatgatgatgctttgccacctcccggcgaacccaccgagctagtgaaggcatctc
ctccgcctggatcgaggagaaggtgcgaccgcatatgtctcccggcggcacggtgg
gcgtggacttggttcctctcgcctccttcgacgcatacaagaatgccatctttgtt
gagcgcaattatacggtaagggagcgcgattgggctgaaaagagtgcggatgtgcg
cgcggcctatgccagtcggtggtgtgcaaaagaggcggtgttcaaatgtctccaga
cacattcacaggcgcgggggcagccatgaaagagattgagatcgagcatggaggt
aacggcgcaccgaaagtcaagctccggggtgctgcgcaaacagcggcgcggcaacg
aggattggaaggagtgcaattgagcatcagctatggcgacgatgcggtgatagcgg
tggcgttggggttgatgtctggtgcttcataa
hexB-AGC for Candida tropicalis SEQ ID NO: 36

atgggttccgttagtagggaacatgagtcaatccccatccaggccgcccagagagg
cgctgcccggatctgcgctgcttttggaggtcaagggtctaacaatttggacgtgt
taaaaggtctattggagttatacaagcggtatggcccagatttggatgagctacta
gacgtggcatccaacacgctttcgcagttggcatcttcccctgctgcaatagacgt
ccacgaaccctggggtttcgacctccgacaatggttgaccacaccggaggttgctc
ctagcaaagaaattcttgccttgccaccacgaagctttcccttaaatacgttactt
agcttggcgctctattgtgcaacttgtcgagagcttgaacttgatcctgggcaatt
tcgatccctccttcatagttccacggggcattcccaaggcatattggcggcggtgg
ccatcacccaagccgagagctggccaacctttatgacgcctgcaggacggtgctc
cagatctctttctggattggactcgaggcttacctcttcactccatcctccgccgc
ctcggatgccatgatccaagattgcatcgaacatggcgagggccttctttcctcaa
tgctaagtgtctccgggctctcccgctcccaagttgagcgagtaattgagcacgtc
aataaaggctcggagaatgcaaccgatgggttcacttggccttggttaactccca
cgaaaagttcgtcttagcggaccacctcaatccttatggccgtttgtcttcatg
tccgacggatcagagcagacaatgacctcgaccagtcgcgtatcttgttccgcaac
cgaaagcctatagtggatatattatttcttcccatatccgcaccatttcacacacc
gtacttggacggtgttcaagatcgcgttatcgaggctttgagctctgcttcgttgg
ctctccattccatcaaaatcccctctatcacacggcactgggagcaacctacaa
gaactacaaccacatcagctaatcccgactcttatccgcgccattaccgtggacca
attggactggccgttggtttgccgggcttgaacgcaacgcacgtgttggactttg
gacctggacaaacatgcagtcttattcaggagctcacacaaggaacaggtgtatca
gtgatccagttgactactcaatcggaccaaaacccgttggaggccatttggcggc
agtgaactgggaggccgagtttggcttacgacttcatgccaatgtccacggtgcag
ctaaattgcacaaccgtatgacaacattgcttggaagcctcctgtgatggtagcc
ggaatgacacctactacggtgcgctgggactttgtcgctgccgttgctcaagctgg
ataccacgtcgaattggctggtggtggctaccacgcagagcgccagttcgaggccg
agattcggcgcttggcaactgccatcccagcagatcatggcatcacctgcaatctc
ctctacgccaagcctacgactttttcctggcagatctctgtcatcaaggatttggt
gcgccagggagttcccgtggaaggaatcaccatcggcgccggcatcccttctccgg
aggtcgtccaagaatgtgtacagtccatcggactcaagcacatctcattcaagcct
gggtctttcgaagccattcaccaagtcatacagatcgcgcgtacccatcctaactt
tttgatcgggttgcaatggaccgcaggacgaggggggaggacatcattcctggaag
acttccatggacctatttggcaacctacgctcaaatccgatcatgtccgaatatt
ctcctcgttgtaggtagtggattcggtggaggcccggacacgtttccctacctcac
gggccaatgggcccaggcctttggctatccatgcatgcccttcgacggagtgttgc
tcggcagtcgcatgatggtggctcgggaagcccatacgtcagcccaggcaaaacgc
ttgattatagatgcgcaaggcgtgggagatgcagattggcacaagtctttcgatga
gcctaccggcggcgtagtgacggtcaactcggaattcggtcaacctatccacgttc
tagctactcgcggagtgatgttgtggaaagaactcgacaaccgggtcttttcaatc
aaagacacttctaagcgcttagaatatttgcgcaaccaccggcaagaaattgtgag
ccgtcttaacgcagactttgcccgtccctggtttgccgttgacggacacggacaga
atgtggagttggaggacatgacctacctcgaggttctccgccgtttgtgcgatctc
acgtatgtttcccaccagaagcgatgggtagatccatcatatcgaatattattgtt
ggacttcgttcatttgcttcgagaacgattccaatgcgctattgacaaccccggcg
aatatccactcgacatcatcgtccgggtggaagagagcttgaaggataaagcatac
cgcacgctttatccagaagatgtctctcttctaatgcatttgttcagccgacgtga
catcaagcccgtaccattcatccccaggttggatgagcgttttgagacctggttta
aaaaagactcattgtggcaatccgaagatgtggaggcggtaattggacaggacgtc
cagcgaatcttcatcattcaagggcctatggccgttcagtactcaatatccgacga
tgagtctgttaaagacattttacacaatatttgtaatcattacgtggaggctctac
aggctgattcaagagaaacttctatcggcgatgtacactcgatcacgcaaaaacct
ctcagcgcgtttcctggctcaaagtgacgacaaatagggtccaaggctctataa
gttcgagaaagtaggagcagtccccgaaatggacgttctttttgagcatattgtcg
gattgtcgaagtcatgggctcggacatgtttgatgagtaaatcggtctttagggac
ggttctcgtttgcataaccccattcgcgccgcactccagctccagcgcggcgacac
catcgaggtgcttttaacagcagactcggaaattcgcaagattcgacttatttcac
ccacgggggatggtggatccacttctaaggtcgtattagagatagtctctaacgac
ggacaaagagttttcgccaccttggcccctaacatcccactcagccccgagcccag
cgtcgtcttttgcttcaaggtcgaccagaagccgaatgagtggacccttgaggagg
atgcgtctggccgggcagagaggatcaaggcattatacatgagtttgtggaacttg
ggctttccgaacaaggcctctgttttgggtcttaattcgcaattcacgggagaaga
attgatgatcacaacggacaagattcgtgatttcgaaagggtattgcggcaaacca
gtcctcttcagttgcagtcatggaacccccaaggatgtgtacctatcgactactgc

gtggtcatcgcctggtctgctcttaccaagcctttgatggtctcctctttgaaatg
cgacctcttggatttgctccacagcgctataagcttccactatgctccatctgtca
aaccattgcgggtgggcgatattgtcaaaacctcatcccgtatcctagcggtctcg
gtgagacctaggggaactatgttgacggtgtcggcggacattcagcgccagggaca
acatgtagtcactgtcaaatcagatttctttctcggaggccccgttttggcatgtg
aaacccctttcgaactcactgaggagcctgaaatggttgtccatgtcgactctgaa
gtgcgccgtgctatttacacagccgcaagtggctcatgcgagaagatcgcgcgct
agatttgctagggaggcagctcctcttcagattaaagagcgaaaaattgttcaggc
cagacggccagctagcattgttacaggtaacaggttccgtgttcagctacagcccc
gatgggtcaacgacagcattcggtcgcgtatacttcgaaagcgagtcttgtacagg
gaacgtggtgatggacttcttgcaccgctacggtgcacctcggcgcagttgttgg
agttgcaacatcccgggtggacggcacctctactgtggcagtaagaggtcctcga
cgcagccaatcctacgcacgcgtctccctcgatcataatcccatccatgtttgtcc
ggcctttgcgcgatacgctggtctctcgggtcccattgtccatgggatggaaacct
ctgccatgatgcgcagaattgccgaatggccatcggagatgcagaccggtctcgg
ttccggagctggcatatcaccttgcaagcacccgtccaccccaacgaccctttgcg
ggtggagttgcagcataaggccatggaggacggggaaatggttttgaaagtacaag
catttaacgaaaggacggaagaacgcgtagcggaggcagatgcccatgttgagcag
gaaactacggcttacgtcttctgtggccagggcagtcaacgacaggggatggaat
ggacttgtacgtcaactgtccggaggctaaagcgttgtgggctcgcgccgacaagc
atttgtgggagaaatatgggttctccatcttgcacattgtgcaaaacaaccctcca
gccctcactgttcactttggcagccagcgagggcgccgtattcgtgccaactattt
gcgcatgatgggacagccaccgatagatggtagacatccgcccatattgaaggat
tgacgcggaattcgacctcgtacaccttctcctattcccaggggttgttgatgtcc
acccagttcgcccagcccgcattggcgttgatggaaatggctcagttcgaatggct
caaagcccagggagtcgttcagaaggtgcgcggttcgcgggacattcgttgggag
aatatgccgcccttggagcttgtgcttccttcctctcatttgaagatctcatatct
ctcatcttttatcggggcttgaagatgcagaatgcgttgccgcgcgatgccaacgg
ccacaccgactatggaatgttggctgccgatccatcgcggataggaaaaggtttcg
aggaagcgagtttgaaatgtcttgtccatatcattcaacaggagaccggctggttc
gtggaagtcgtcaactacaacatcaactcgcagcaatacgtctgtgcaggccattt
ccgagccctttggatgttgggtaagatatgcgatgacctttcatgccaccctcaac
cggagactgttgaaggccaagagctacggccatggtctggaagcatgtcccgacg
gtggagcaggtgccccgcgaggatcgcatggaacgaggtcgagcgaccattccgtt
gccggggatcgatatcccataccattcgaccatgttacgagggagattgagcctt
atcgtgaatatttgtctgaacgtatcaaggtgggggatgtgaagccgtgcgaattg
gtgggacgctggatccctaatgttgttggccagcctttctccgtcgataagtctta
cgttcagttggtgcacggcatcacaggtagtcctcggcttcattccttgcttcaac
aaatggcgtga

### Example 4: Transformation of C. tropicalis with the Synthetic Hexanoate Synthase Subunit Genes

Candida tropicalis cells (ATCC number 20962) and cultured under standard conditions in YPD medium at 30 degrees Celsius. The synthetic genes encoding hexA and hexB are amplified using standard PCR amplification techniques. A linear DNA construct comprising, from 5' to 3', the TEF (transcription elongation factor) promoter, the hexA-AGC gene, the TEF promoter, the hexB-AGC gene, and the URA3 marker. Each end of this construct is designed to contain a mini-URA-Blaster for integration of the construct into C. tropicalis genomic DNA (Alani E, Cao L, Kleckner N. A method for gene disruption that allows repeated use of URA3 selection in the construction of multiply disrupted yeast strains. Genetics. 1987 Aug;116 (4):541-545).

The construct is amplified using standard techniques. Transformation of C. tropicalis cells with this linear construct is accomplished using standard electroporation techniques such as those set forth in U.S. Patent No. 5,648,247 or 5,204,252. Transformants are selected by plating and growing the transformed cells on SC-URA media as described above in which only transformants will survive. To remove the URA cassette, the confirmed strain is then replated onto SC complete media containing 5-Fluoroorotic Acid (5-FOA) and confirmed for the loss of the URA cassette.

### Example 5: Assay of Cytochrome P450 with Activity on Six Carbon Chains in C. tropicalis

Cultures of C. tropicalis are cultured in YPD media to late log phase and then exposed to hexane exposed to various concentrations of hexane up to about 0.1 percent (v/v) induce the expression of the cytochrome p450 gene having activity specific for six carbon substrates. After about 2 hours exposure to the hexane solution, cells were harvested and RNA isolated using techniques described above. The specifically induced gene may be detected by Northern blotting and/or quantitative RT-PCR.

Cells to be analyzed for cytochrome P450 activity are grown under standard conditions and harvested for the production of microsomes. Microsomes were prepared by lysing cells in Tris-buffered sucrose (10mM Tris-HCl pH 7.5, 1 mM EDTA, 0.25M sucrose). Differential centrifugation is performed first at 25,000xg then at 100,000xg to pellet cell debris then microsomes, respectively. The microsome pellet is resuspended in 0.1 M phosphate buffer (pH 7.5), 1 mM EDTA to a final concentration of approximately 10mg protein/mL.

A reaction mixture containing approximately 0.3mg microsomes, 0.1 mM sodium hexanoate, 0.7mM NADPH, 50mM Tris-HCl pH 7.5 in 1 mL is initiated by the addition of NADPH and incubated at 37°C for 10 minutes. The reaction is terminated by addition of 0.25mL 5M HCl and 0.25mL 2.5ug/mL 10-hydroxydecanoic acid is added as an internal standard (3.3 nmol). The mixture is extracted with 4.5mL diethyl ether under NaCl-saturated conditions. The organic phase is transferred to a new tube and evaporated to dryness. The residue is dissolved in acetonitrile containing 10mM 3-bromomethyl-7-methoxy-1,4-benzoxazin-2-one (BrMB) and 0.1mL of 15mg/mL 18-crown-6 in acetonitril saturated with K₂CO₃. The solution is incubated at 40 °C for 30 minutes before addition of 0.05mL 2% acetic acid. The fluorescently labeled omega-hydroxy fatty acids are resolved via HPLC with detection at 430nm and excitation at 355nm.

### Example 6: Examples of Polynucleotide Regulators

Provided in the tables hereafter are non-limiting examples of regulator polynucleotides that can be utilized in embodiments herein. Such polynucleotides may be utilized in native form or may be modified for use herein. Examples of regulatory polynucleotides include those that are regulated by oxygen levels in a system (e.g., upregulated or downregulated by relatively high oxygen levels or relatively low oxygen levels).

**Regulated Yeast Promoters - Up-regulated by oxygen**

| **ORF name** | **Gene name** | **Relative mRNA level (Aerobic)** | **Relative mRNA level (Anaerobic)** | **Ratio** |
|---|---|---|---|---|
| YPL275W | | 4389 | 30 | 219.5 |
| YPL276W | | 2368 | 30 | 118.4 |
| YDR256C | CTA1 | 2076 | 30 | 103.8 |
| YHR096C | HXT5 | 1846 | 30 | 72.4 |
| YDL218W | | 1189 | 30 | 59.4 |
| YCR010C | | 1489 | 30 | 48.8 |
| YOR161C | | 599 | 30 | 29.9 |
| YPL200W | | 589 | 30 | 29.5 |
| YGR110W | | 1497 | 30 | 27 |
| YN L237W | YTP1 | 505 | 30 | 25.2 |
| YBR116C | | 458 | 30 | 22.9 |
| YOR348C | PUT4 | 451 | 30 | 22.6 |
| YBR117C | TKL2 | 418 | 30 | 20.9 |
| YLL052C | | 635 | 30 | 20 |
| YNL195C | | 1578 | 30 | 19.4 |
| YPR193C | | 697 | 30 | 15.7 |
| YDL222C | | 301 | 30 | 15 |
| YN L335W | | 294 | 30 | 14.6 |
| YPL036W | PMA2 | 487 | 30 | 12.8 |
| YML122C | | 206 | 30 | 10.3 |
| YGR067C | | 236 | 30 | 10.2 |
| YPR192W | | 204 | 30 | 10.2 |
| YNL014W | | 828 | 30 | 9.8 |
| YFL061W | | 256 | 30 | 9.1 |
| YNR056C | | 163 | 30 | 8.1 |
| YOR186W | | 153 | 30 | 7.6 |
| YDR222W | | 196 | 30 | 6.5 |
| YOR338W | | 240 | 30 | 6.3 |
| YPR200C | | 113 | 30 | 5.7 |
| YMR018W | | 778 | 30 | 5.2 |
| YOR364W | | 123 | 30 | 5.1 |
| YN L234W | | 93 | 30 | 4.7 |
| YNR064C | | 85 | 30 | 4.2 |
| YGR213C | RTA1 | 104 | 30 | 4 |
| YCL064C | CHA1 | 80 | 30 | 4 |
| YOL154W | | 302 | 30 | 3.9 |
| YPR150W | | 79 | 30 | 3.9 |
| YPR196W | MAL63 | 30 | 30 | 3.6 |
| YDR420W | HKR1 | 221 | 30 | 3.5 |
| YJL216C | | 115 | 30 | 3.5 |
| YNL270C | ALP1 | 67 | 30 | 3.3 |
| YHL016C | DUR3 | 224 | 30 | 3.2 |
| YOL131W | | 230 | 30 | 3 |
| YOR077W | RTS2 | 210 | 30 | 3 |
| YDR536W | STL1 | 55 | 30 | 2.7 |
| YNL150W | | 78 | 30 | 2.6 |
| YHR212C | | 149 | 30 | 2.4 |
| YJL108C | | 106 | 30 | 2.4 |
| YGR069W | | 49 | 30 | 2.4 |
| YDR106W | | 60 | 30 | 2.3 |
| YNR034W | SOL1 | 197 | 30 | 2.2 |
| YEL073C | | 104 | 30 | 2.1 |
| YOL141W | | 81 | 30 | 1.8 |

**Regulated Yeast Promoters - Down-regulated by oxygen**

| **ORF name** | **Gene name** | **Relative mRNA level (Aerobic)** | **Relative mRNA level (Anaerobic)** | **Ratio** |
|---|---|---|---|---|
| YJR047C | ANB1 | 30 | 4901 | 231.1 |
| YMR319C | FET4 | 30 | 1159 | 58 |
| YPR194C | | 30 | 982 | 49.1 |
| YIR019C | STA1 | 30 | 981 | 22.8 |
| YHL042W | | 30 | 608 | 12 |
| YHR210C | | 30 | 552 | 27.6 |
| YHR079B | SAE3 | 30 | 401 | 2.7 |
| YGL162W | STO1 | 30 | 371 | 9.6 |
| YHL044W | | 30 | 334 | 16.7 |
| YOL015W | | 30 | 320 | 6.1 |
| YCLX07W | | 30 | 292 | 4.2 |
| YIL013C | PDR11 | 30 | 266 | 10.6 |
| YDR046C | | 30 | 263 | 13.2 |
| YBR040W | FIG1 | 30 | 257 | 12.8 |
| YLR040C | | 30 | 234 | 2.9 |
| YOR255W | | 30 | 231 | 11.6 |
| YOL014W | | 30 | 229 | 11.4 |
| YAR028W | | 30 | 212 | 7.5 |
| YER089C | | 30 | 201 | 6.2 |
| YFL012W | | 30 | 193 | 9.7 |
| YDR539W | | 30 | 187 | 3.4 |
| YHL043W | | 30 | 179 | 8.9 |
| YJR162C | | 30 | 173 | 6 |
| YMR165C | SMP2 | 30 | 147 | 3.5 |
| YER106W | | 30 | 145 | 7.3 |
| YDR541C | | 30 | 140 | 7 |
| YCRX07W | | 30 | 138 | 3.3 |
| YHR048W | | 30 | 137 | 6.9 |
| YCL021W | | 30 | 136 | 6.8 |
| YOL160W | | 30 | 136 | 6.8 |
| YCRX08W | | 30 | 132 | 6.6 |
| YMR057C | | 30 | 109 | 5.5 |
| YDR540C | | 30 | 83 | 4.2 |
| YOR378W | | 30 | 78 | 3.9 |
| YBR085W | AAC3 | 45 | 1281 | 28.3 |
| YER188W | | 47 | 746 | 15.8 |
| YLL065W | GIN11 | 50 | 175 | 3.5 |
| YDL241W | | 58 | 645 | 11.1 |
| YBR238C | | 59 | 274 | 4.6 |
| YCR048W | ARE1 | 60 | 527 | 8.7 |
| YOL165C | | 60 | 306 | 5.1 |
| YNR075W | | 60 | 251 | 4.2 |
| YJL213W | | 60 | 250 | 4.2 |
| YPL265W | DIP5 | 61 | 772 | 12.7 |
| YDL093W | PMT5 | 62 | 353 | 5.7 |
| YKR034W | DAL80 | 63 | 345 | 5.4 |
| YKR053C | | 66 | 1268 | 19.3 |
| YJR147W | | 68 | 281 | 4.1 |

**Known and putative DNA binding motifs**

| **Regulator** | **Known Consensus Motif** | |
|---|---|---|
| Abf1 | TCRNNNNNNACG | |
| Cbf1 | RTCACRTG | |
| Gal4 | CGGNNNNNNNNNNNCCG | |
| Gcn4 | TGACTCA | |
| Gcr1 | CTTCC | |
| Hap2 | CCAATNA | |
| Hap3 | CCAATNA | |
| Hap4 | CCAATNA | |
| Hsf1 | GAANNTTCNNGAA | |
| Ino2 | ATGTGAAA | |
| Mata(A1) | TGATGTANNT | |
| Mcm1 | CCNNNWWRGG | |
| Mig1 | WWWWSYGGGG | |
| Pho4 | CACGTG | |
| Rap1 | RMACCCANNCAYY | |
| Reb1 | CGGGTRR | |
| Ste12 | TGAAACA | |
| Swi4 | CACGAAA | |
| Swi6 | CACGAAA | |
| Yap1 | TTACTAA | |
| | | |

| **Putative DNA Binding Motifs** | | |
|---|---|---|
| **Regulator** | **Best Motif (scored by E-value)** | **Best Motif (scored by Hypergeometric)** |
| Abf1 | TYCGT--R-ARTGAYA | TYCGT--R-ARTGAYA |
| Ace2 | RRRAARARAA-A-RARAA | GTGTGTGTGTGTGTG |
| Adr1 | A-AG-GAGAGAG-GGCAG | YTSTYSTT-TTGYTWTT |
| Arg80 | T--CCW-TTTKTTTC | GCATGACCATCCACG |
| Arg81 | AAAAARARAAAARMA | GSGAYARMGGAMAAAAA |
| Aro80 | YKYTYTTYTT----KY | TRCCGAGRYW-SSSGCGS |
| Ash1 | CGTCCGGCGC | CGTCCGGCGC |
| Azf1 | GAAAAAGMAAAAAAA | AARWTSGARG-A--CSAA |
| Bas1 | TTTTYYTTYTTKY-TY-T | CS-CCAATGK--CS |
| Cad1 | CATKYTTTTTTKYTY | GCT-ACTAAT |
| Cbf1 | CACGTGACYA | CACGTGACYA |
| Cha4 | CA---ACACASA-A | CAYAMRTGY-C |
| Cin5 | none | none |
| Crz1 | GG-A-A--AR-ARGGC- | TSGYGRGASA |
| Cup9 | TTTKYTKTTY-YTTTKTY | K-C-C---SCGCTACKGC |
| Dal81 | WTTKTTTTTYTTTTT-T | SR-GGCMCGGC-SSG |
| Dal82 | TTKTTTTYTTC | TACYACA-CACAWGA |
| Dig1 | AAA--RAA-GARRAA-AR | CCYTG-AYTTCW-CTTC |
| Dot6 | GTGMAK-MGRA-G-G | GTGMAK-MGRA-G-G |
| Fhl1 | -TTWACAYCCRTACAY-Y | -TTWACAYCCRTACAY-Y |
| Fkh1 | TTT-CTTTKYTT-YTTTT | AAW-RTAAAYARG |
| Fkh2 | AAARA-RAAA-AAAR-AA | GG-AAWA-GTAAACAA |
| Fzf1 | CACACACACACACACAC | SASTKCWCTCKTCGT |
| Gal4 | TTGCTTGAACGSATGCCA | TTGCTTGAACGSATGCCA |
| Gal4 (Gal) | YCTTTTTTTTYTTYYKG | CGGM---CW-Y--CCCG |
| Gat1 | none | none |
| Gat3 | RRSCCGMCGMGRCGCGCS | RGARGTSACGCAKRTTCT |
| Gcn4 | AAA-ARAR-RAAAARRAR | TGAGTCAY |
| Gcr1 | GGAAGCTGAAACGYMWRR | GGAAGCTGAAACGYMWRR |
| Gcr2 | GGAGAGGCATGATGGGGG | AGGTGATGGAGTGCTCAG |
| Gln3 | CT-CCTTTCT | GKCTRR-RGGAGA-GM |
| Grf10 | GAAARRAAAAAAMRMARA | -GGGSG-T-SYGT-CGA |
| Gts1 | G-GCCRS--TM | AG-AWGTTTTTGWCAAMA |
| Haa1 | none | none |
| Hal9 | TTTTTTYTTTTY-KTTTT | KCKSGCAGGCWTTKYTCT |
| Hap2 | YTTCTTTTYT-Y-C-KT- | G-CCSART-GC |
| Hap3 | T-SYKCTTTTCYTTY | SGCGMGGG--CC-GACCG |
| Hap4 | STT-YTTTY-TTYTYYYY | YCT-ATTSG-C-GS |
| Hap5 | YK-TTTWYYTC | T-TTSMTT-YTTTCCK-C |
| Hir1 | AAAA-A-AARAR-AG | CCACKTKSGSCCT-S |
| Hir2 | WAAAAAAGAAAA-AAAAR | CRSGCYWGKGC |
| Hms1 | AAA-GG-ARAM | -AARAAGC-GGGCAC-C |
| Hsf1 | TYTTCYAGAA--TTCY | TYTTCYAGAA--TTCY |
| Ime4 | CACACACACACACACACA | CACACACACACACACACA |
| Ino2 | TTTYCACATGC | SCKKCGCKSTSSTTYAA |
| Ino4 | G--GCATGTGAAAA | G--GCATGTGAAAA |
| Ixr1 | GAAAA-AAAAAAAARA-A | CTTTTTTTYYTSGCC |
| Leu3 | GAAAAARAARAA-AA | GCCGGTMMCGSYC-- |
| Mac1 | YTTKT--TTTTTYTYTTT | A--TTTTTYTTKYGC |
| Mal13 | GCAG-GCAGG | AAAC-TTTATA-ATACA |
| Mal33 | none | none |
| Mata1 | GCCC-C | CAAT-TCT-CK |
| Mbp1 | TTTYTYKTTT-YYTTTTT | G-RR-A-ACGCGT-R |
| Mcm1 | TTTCC-AAW-RGGAAA | TTTCC-AAW-RGGAAA |
| Met31 | YTTYYTTYTTTTYTYTTC | |
| Met4 | MTTTTTYTYTYTTC | |
| Mig1 | TATACA-AGMKRTATATG | |
| Mot3 | TMTTT-TY-CTT-TTTW K | |
| Msn1 | KT--TTWTTATTCC-C | |
| Msn2 | ACCACC | |
| Msn4 | R--AAAA-RA-AARAAAT | |
| Mss11 | IIIIIIIICWCTTTKYC | |
| Ndd1 | TTTY-YTKTTTY-YTTYT | |
| Nrg1 | TTY--TTYTT-YTTTYYY | |
| Pdr1 | T-YGTGKRYGT-YG | |
| Phd1 | TTYYYTTTTTYTTTTYTT | |
| Pho4 | GAMAAAAAARAAAAR | |
| Put3 | CYCGGGAAGCSAMM-CCG | |
| Rap1 | GRTGYAYGGRTGY | |
| Rcs1 | KMAARAAAAARAAR | |
| Reb1 | RTTACCCGS | |
| Rfx1 | AYGRAAAARARAAAARAA | |
| Rgm1 | GGAKSCC-TTTY-GMRTA | |
| Rgt1 | CCCTCC | |
| Rim101 | GCGCCGC | |
| Rlm1 | TTTTC-KTTTYTTTTTC | |
| Rme1 | ARAAGMAGAAARRAA | |
| Rox1 | YTTTTCTTTTY-TTTTT | |
| Rph1 | ARRARAAAGG- | |
| Rtg1 | YST-YK-TYTT-CTCCCM | |
| Rtg3 | GARA-AAAAR-RAARAAA | |
| Sfl1 | CY--GGSSA-C | |
| Sfp1 | CACACACACACACAYA | |
| Sip4 | CTTYTWTTKTTKTSA | |
| Skn7 | YTTYYYTYTTTYTYYTTT | |
| Sko1 | none | |
| Smp1 | AMAAAAARAARWARA-AA | |
| Sok2 | ARAAAARRAAAAAG-RAA | |
| Stb1 | RAARAAAAARCMRSRAAA | |
| Ste12 | TTYTKTYTY-TYYKTTTY | |
| Stp1 | GAAAAMAA-AAAAA-AAA | |
| Stp2 | YAA-ARAARAAAAA-AAM | |
| Sum1 | TY-TTTTTTYTTTTT-TK | |
| Swi4 | RAARAARAAA-AA-R-AA | |
| Swi5 | CACACACACACACACACA | |
| Swi6 | RAARRRAAAAA-AAAMAA | |
| Thi2 | GCCAGACCTAC | |
| Uga3 | GG-GGCT | |
| Yap1 | TTYTTYTTYTTTY-YTYT | |
| Yap3 | none | |
| Yap5 | YKSGCGCGYCKCGKCGGS | |
| Yap6 | TTTTYYTTTTYYYYKTT | |
| Yap7 | none | |
| Yfl044c | TTCTTKTYYTTTT | |
| Yjl206c | TTYTTTTYTYYTTTYTTT | |
| Zap1 | TTGCTTGAACGGATGCCA | |
| Zms1 | MG-MCAAAAATAAAAS | |

**Transcriptional repressors**

| **Associated Gene(s)** | **Description(s)** |
|---|---|
| WHI5 | Repressor of G1 transcription that binds to SCB binding factor (SBF) at SCB target promoters in early G1; phosphorylation of Whi5p by the CDK, Cln3p/Cdc28p relieves repression and promoter binding by Whi5; periodically expressed in G1 |
| TUP1 | General repressor of transcription, forms complex with Cyc8p, involved in the establishment of repressive chromatin structure through interactions with histones H3 and H4, appears to enhance expression of some genes |
| ROX1 | Heme-dependent repressor of hypoxic genes; contains an HMG domain that is responsible for DNA bending activity |
| SFL1 | Transcriptional repressor and activator; involved in repression of flocculation-related genes, and activation of stress responsive genes; negatively regulated by cAMP-dependent protein kinase A subunit Tpk2p |
| RIM101 | Transcriptional repressor involved in response to pH and in cell wall construction; required for alkaline pH-stimulated haploid invasive growth and sporulation; activated by proteolytic processing; similar to A. nidulans PacC |
| RDR1 | Transcriptional repressor involved in the control of multidrug resistance; negatively regulates expression of the PDR5 gene; member of the Gal4p family of zinc cluster proteins |
| SUM1 | Transcriptional repressor required for mitotic repression of middle sporulation-specific genes; also acts as general replication initiation factor; involved in telomere maintenance, chromatin silencing; regulated by pachytene checkpoint |
| XBP1 | Transcriptional repressor that binds to promoter sequences of the cyclin genes, CYS3, and SMF2; expression is induced by stress or starvation during mitosis, and late in meiosis; member of the Swi4p/Mbp1p family; potential Cdc28p substrate |
| NRG2 | Transcriptional repressor that mediates glucose repression and negatively regulates filamentous growth; has similarity to Nrg1p |
| NRG1 | Transcriptional repressor that recruits the Cyc8p-Tup1 p complex to promoters; mediates glucose repression and negatively regulates a variety of processes including filamentous growth and alkaline pH response |
| CUP9 | Homeodomain-containing transcriptional repressor of PTR2, which encodes a major peptide transporter; imported peptides activate ubiquitin-dependent proteolysis, resulting in degradation of Cup9p and de-repression of PTR2 transcription |
| YOX1 | Homeodomain-containing transcriptional repressor, binds to Mcm1p and to early cell cycle boxes (ECBs) in the promoters of cell cycle-regulated genes expressed in M/G1 phase; expression is cell cycle-regulated; potential Cdc28p substrate |
| RFX1 | Major transcriptional repressor of DNA-damage-regulated genes, recruits repressors Tup1p and Cyc8p to their promoters; involved in DNA damage and replication checkpoint pathway; similar to a family of mammalian DNA binding RFX1-4 proteins |
| MIG3 | Probable transcriptional repressor involved in response to toxic agents such as hydroxyurea that inhibit ribonucleotide reductase; phosphorylation by Snf1 p or the Mec1 p pathway inactivates Mig3p, allowing induction of damage response genes |
| RGM1 | Putative transcriptional repressor with proline-rich zinc fingers; overproduction impairs cell growth |
| YHP1 | One of two homeobox transcriptional repressors (see also Yox1p), that bind to Mcm1p and to early cell cycle box (ECB) elements of cell cycle regulated genes, thereby restricting ECB-mediated transcription to the M/G1 interval |
| HOS4 | Subunit of the Set3 complex, which is a meiotic-specific repressor of sporulation specific genes that contains deacetylase activity; potential Cdc28p substrate |
| CAF20 | Phosphoprotein of the mRNA cap-binding complex involved in translational control, repressor of cap-dependent translation initiation, competes with elF4G for binding to elF4E |
| SAP1 | Putative ATPase of the AAA family, interacts with the Sin1p transcriptional repressor in the two-hybrid system |
| SET3 | Defining member of the SET3 histone deacetylase complex which is a meiosis-specific repressor of sporulation genes; necessary for efficient transcription by RNAPII; one of two yeast proteins that contains both SET and PHD domains |
| RPH1 | JmjC domain-containing histone demethylase which can specifically demethylate H3K36 tri- and dimethyl modification states; transcriptional repressor of PHR1; Rph1p phosphorylation during DNA damage is under control of the MEC1-RAD53 pathway |
| YMR181C | Protein of unknown function; mRNA transcribed as part of a bicistronic transcript with a predicted transcriptional repressor RGM1/YMR182C; mRNA is destroyed by nonsense-mediated decay (NMD); YMR181C is not an essential gene |
| YLR345W | Similar to 6-phosphofructo-2-kinase/fructose-2,6-bisphosphatase enzymes responsible for the metabolism of fructoso-2,6-bisphosphate; mRNA expression is repressed by the Rfx1p-Tup1p-Ssn6p repressor complex; YLR345W is not an essential gene |
| MCM1 | Transcription factor involved in cell-type-specific transcription and pheromone response; plays a central role in the formation of both repressor and activator complexes |
| PHR1 | DNA photolyase involved in photoreactivation, repairs pyrimidine dimers in the presence of visible light; induced by DNA damage; regulated by transcriptional repressor Rph1p |
| HOS2 | Histone deacetylase required for gene activation via specific deacetylation of lysines in H3 and H4 histone tails; subunit of the Set3 complex, a meiotic-specific repressor of sporulation specific genes that contains deacetylase activity |
| RGT1 | Glucose-responsive transcription factor that regulates expression of several glucose transporter (HXT) genes in response to glucose; binds to promoters and acts both as a transcriptional activator and repressor |
| SRB7 | Subunit of the RNA polymerase II mediator complex; associates with core polymerase subunits to form the RNA polymerase II holoenzyme; essential for transcriptional regulation; target of the global repressor Tup1p |
| GAL11 | Subunit of the RNA polymerase II mediator complex; associates with core polymerase subunits to form the RNA polymerase II holoenzyme; affects transcription by acting as target of activators and repressors |

**Transcriptional activators**

| **Associated Gene(s)** | **Description(s)** |
|---|---|
| SKT5 | Activator of Chs3p (chitin synthase III), recruits Chs3p to the bud neck via interaction with Bni4p; has similarity to Shc1p, which activates Chs3p during sporulation |
| MSA1 | Activator of G1-specific transcription factors, MBF and SBF, that regulates both the timing of G1-specific gene transcription, and cell cycle initiation; potential Cdc28p substrate |
| AMA1 | Activator of meiotic anaphase promoting complex (APC/C); Cdc20p family member; required for initiation of spore wall assembly; required for Clb1p degradation during meiosis |
| STB5 | Activator of multidrug resistance genes, forms a heterodimer with Pdr1 p; contains a Zn(II)2Cys6 zinc finger domain that interacts with a PDRE (pleotropic drug resistance element) in vitro; binds Sin3p in a two-hybrid assay |
| RRD2 | Activator of the phosphotyrosyl phosphatase activity of PP2A,peptidyl-prolyl cis/trans-isomerase; regulates G1 phase progression, the osmoresponse, microtubule dynamics; subunit of the Tap42p-Pph21 p-Rrd2p complex |
| BLM10 | Proteasome activator subunit; found in association with core particles, with and without the 19S regulatory particle; required for resistance to bleomycin, may be involved in protecting against oxidative damage; similar to mammalian PA200 |
| SHC1 | Sporulation-specific activator of Chs3p (chitin synthase III), required for the synthesis of the chitosan layer of ascospores; has similarity to Skt5p, which activates Chs3p during vegetative growth; transcriptionally induced at alkaline pH |
| NDD1 | Transcriptional activator essential for nuclear division; localized to the nucleus; essential component of the mechanism that activates the expression of a set of late-S-phase-specific genes |
| IMP2' | Transcriptional activator involved in maintenance of ion homeostasis and protection against DNA damage caused by bleomycin and other oxidants, contains a C-terminal leucine-rich repeat |
| LYS14 | Transcriptional activator involved in regulation of genes of the lysine biosynthesis pathway; requires 2-aminoadipate semialdehyde as co-inducer |
| MSN1 | Transcriptional activator involved in regulation of invertase and glucoamylase expression, invasive growth and pseudohyphal differentiation, iron uptake, chromium accumulation, and response to osmotic stress; localizes to the nucleus |
| HAA1 | Transcriptional activator involved in the transcription of TPO2, YRO2, and other genes putatively encoding membrane stress proteins; involved in adaptation to weak acid stress |
| UGA3 | Transcriptional activator necessary for gamma-aminobutyrate (GABA)-dependent induction of GABA genes (such as UGA1, UGA2, UGA4); zinc-finger transcription factor of the Zn(2)-Cys(6) binuclear cluster domain type; localized to the nucleus |
| GCR1 | Transcriptional activator of genes involved in glycolysis; DNA-binding protein that interacts and functions with the transcriptional activator Gcr2p |
| GCR2 | Transcriptional activator of genes involved in glycolysis; interacts and functions with the DNA-binding protein Gcr1p |
| GAT1 | Transcriptional activator of genes involved in nitrogen catabolite repression; contains a GATA-1-type zinc finger DNA-binding motif; activity and localization regulated by nitrogen limitation and Ure2p |
| GLN3 | Transcriptional activator of genes regulated by nitrogen catabolite repression (NCR), localization and activity regulated by quality of nitrogen source |
| PUT3 | Transcriptional activator of proline utilization genes, constitutively binds PUT1 and PUT2 promoter sequences and undergoes a conformational change to form the active state; has a Zn(2)-Cys(6) binuclear cluster domain |
| ARR1 | Transcriptional activator of the basic leucine zipper (bZIP) family, required for transcription of genes involved in resistance to arsenic compounds |
| PDR3 | Transcriptional activator of the pleiotropic drug resistance network, regulates expression of ATP-binding cassette (ABC) transporters through binding to cis-acting sites known as PDREs (PDR responsive elements) |
| MSN4 | Transcriptional activator related to Msn2p; activated in stress conditions, which results in translocation from the cytoplasm to the nucleus; binds DNA at stress response elements of responsive genes, inducing gene expression |
| MSN2 | Transcriptional activator related to Msn4p; activated in stress conditions, which results in translocation from the cytoplasm to the nucleus; binds DNA at stress response elements of responsive genes, inducing gene expression |
| PHD1 | Transcriptional activator that enhances pseudohyphal growth; regulates expression of FLO11 an adhesin required for pseudohyphal filament formation; similar to StuA, an A. nidulans developmental regulator; potential Cdc28p substrate |
| FHL1 | Transcriptional activator with similarity to DNA-binding domain of Drosophila forkhead but unable to bind DNA in vitro; required for rRNA processing; isolated as a suppressor of splicing factor prp4 |
| VHR1 | Transcriptional activator, required for the vitamin H-responsive element (VHRE) mediated induction of VHT1 (Vitamin H transporter) and BIO5 (biotin biosynthesis intermediate transporter) in response to low biotin concentrations |
| CDC20 | Cell-cycle regulated activator of anaphase-promoting complex/cyclosome (APC/C), which is required for metaphase/anaphase transition; directs ubiquitination of mitotic cyclins, Pds1p, and other anaphase inhibitors; potential Cdc28p substrate |
| CDH1 | Cell-cycle regulated activator of the anaphase-promoting complex/cyclosome (APC/C), which directs ubiquitination of cyclins resulting in mitotic exit; targets the APC/C to specific substrates including Cdc20p, Ase1p, Cin8p and Fin1p |
| AFT2 | Iron-regulated transcriptional activator; activates genes involved in intracellular iron use and required for iron homeostasis and resistance to oxidative stress; similar to Aft1 p |
| MET4 | Leucine-zipper transcriptional activator, responsible for the regulation of the sulfur amino acid pathway, requires different combinations of the auxiliary factors Cbf1 p, Met28p, Met31 p and Met32p |
| CBS2 | Mitochondrial translational activator of the COB mRNA; interacts with translating ribosomes, acts on the COB mRNA 5'-untranslated leader |
| CBS1 | Mitochondrial translational activator of the COB mRNA; membrane protein that interacts with translating ribosomes, acts on the COB mRNA 5'-untranslated leader |
| CBP6 | Mitochondrial translational activator of the COB mRNA; phosphorylated |
| PET111 | Mitochondrial translational activator specific for the COX2 mRNA; located in the mitochondrial inner membrane |
| PET494 | Mitochondrial translational activator specific for the COX3 mRNA, acts together with Pet54p and Pet122p; located in the mitochondrial inner membrane |
| PET122 | Mitochondrial translational activator specific for the COX3 mRNA, acts together with Pet54p and Pet494p; located in the mitochondrial inner membrane |
| RRD1 | Peptidyl-prolyl cis/trans-isomerase, activator of the phosphotyrosyl phosphatase activity of PP2A; involved in G1 phase progression, microtubule dynamics, bud morphogenesis and DNA repair; subunit of the Tap42p-Sit4p-Rrd1p complex |
| YPR196W | Putative maltose activator |
| POG1 | Putative transcriptional activator that promotes recovery from pheromone induced arrest; inhibits both alpha-factor induced G1 arrest and repression of CLN1 and CLN2 via SCB/MCB promoter elements; potential Cdc28p substrate; SBF regulated |
| MSA2 | Putative transcriptional activator, that interacts with G1-specific transcription factor, MBF and G1-specific promoters; ortholog of Msa2p, an MBF and SBF activator that regulates G1-specific transcription and cell cycle initiation |
| PET309 | Specific translational activator for the COX1 mRNA, also influences stability of intron-containing COX1 primary transcripts; localizes to the mitochondrial inner membrane; contains seven pentatricopeptide repeats (PPRs) |
| TEA1 | Ty1 enhancer activator required for full levels of Ty enhancer-mediated transcription; C6 zinc cluster DNA-binding protein |
| PIP2 | Autoregulatory oleate-specific transcriptional activator of peroxisome proliferation, contains Zn(2)-Cys(6) cluster domain, forms heterodimer with Oaf1p, binds oleate response elements (OREs), activates beta-oxidation genes |
| CHA4 | DNA binding transcriptional activator, mediates serine/threonine activation of the catabolic L-serine (L-threonine) deaminase (CHA1); Zinc-finger protein with Zn[2]-Cys[6] fungal-type binuclear cluster domain |
| SFL1 | Transcriptional repressor and activator; involved in repression of flocculation-related genes, and activation of stress responsive genes; negatively regulated by cAMP-dependent protein kinase A subunit Tpk2p |
| RDS2 | Zinc cluster transcriptional activator involved in conferring resistance to ketoconazole |
| CAT8 | Zinc cluster transcriptional activator necessary for derepression of a variety of genes under non-fermentative growth conditions, active after diauxic shift, binds carbon source responsive elements |
| ARO80 | Zinc finger transcriptional activator of the Zn2Cys6 family; activates transcription of aromatic amino acid catabolic genes in the presence of aromatic amino acids |
| SIP4 | C6 zinc cluster transcriptional activator that binds to the carbon source-responsive element (CSRE) of gluconeogenic genes; involved in the positive regulation of gluconeogenesis; regulated by Snf1p protein kinase; localized to the nucleus |
| SPT10 | Putative histone acetylase, sequence-specific activator of histone genes, binds specifically and highly cooperatively to pairs of UAS elements in core histone promoters, functions at or near the TATA box |
| MET28 | Basic leucine zipper (bZIP) transcriptional activator in the Cbf1p-Met4p-Met28p complex, participates in the regulation of sulfur metabolism |
| GCN4 | Basic leucine zipper (bZIP) transcriptional activator of amino acid biosynthetic genes in response to amino acid starvation; expression is tightly regulated at both the transcriptional and translational levels |
| CAD1 | AP-1-like basic leucine zipper (bZIP) transcriptional activator involved in stress responses, iron metabolism, and pleiotropic drug resistance; controls a set of genes involved in stabilizing proteins; binds consensus sequence TTACTAA |
| INO2 | Component of the heteromeric Ino2p/Ino4p basic helix-loop-helix transcription activator that binds inositol/choline-responsive elements (ICREs), required for derepression of phospholipid biosynthetic genes in response to inositol depletion |
| THI2 | Zinc finger protein of the Zn(II)2Cys6 type, probable transcriptional activator of thiamine biosynthetic genes |
| SWI4 | DNA binding component of the SBF complex (Swi4p-Swi6p), a transcriptional activator that in concert with MBF (Mbp1-Swi6p) regulates late G1-specific transcription of targets including cyclins and genes required for DNA synthesis and repair |
| HAP5 | Subunit of the heme-activated, glucose-repressed Hap2/3/4/5 CCAAT-binding complex, a transcriptional activator and global regulator of respiratory gene expression; required for assembly and DNA binding activity of the complex |
| HAP3 | Subunit of the heme-activated, glucose-repressed Hap2p/3p/4p/5p CCAAT-binding complex, a transcriptional activator and global regulator of respiratory gene expression; contains sequences contributing to both complex assembly and DNA binding |
| HAP2 | Subunit of the heme-activated, glucose-repressed Hap2p/3p/4p/5p CCAAT-binding complex, a transcriptional activator and global regulator of respiratory gene expression; contains sequences sufficient for both complex assembly and DNA binding |
| HAP4 | Subunit of the heme-activated, glucose-repressed Hap2p/3p/4p/5p CCAAT-binding complex, a transcriptional activator and global regulator of respiratory gene expression; provides the principal activation function of the complex |
| YML037C | Putative protein of unknown function with some characteristics of a transcriptional activator; may be a target of Dbf2p-Mob1p kinase; GFP-fusion protein co-localizes with clathrin-coated vesicles; YML037C is not an essential gene |
| TRA1 | Subunit of SAGA and NuA4 histone acetyltransferase complexes; interacts with acidic activators (e.g., Gal4p) which leads to transcription activation; similar to human TRRAP, which is a cofactor for c-Myc mediated oncogenic transformation |
| YLL054C | Putative protein of unknown function with similarity to Pip2p, an oleate-specific transcriptional activator of peroxisome proliferation; YLL054C is not an essential gene |
| RTG2 | Sensor of mitochondrial dysfunction; regulates the subcellular location of Rtg1p and Rtg3p, transcriptional activators of the retrograde (RTG) and TOR pathways; Rtg2p is inhibited by the phosphorylated form of Mks1p |
| YBR012C | Dubious open reading frame, unlikely to encode a functional protein; expression induced by iron-regulated transcriptional activator Aft2p |
| JEN1 | Lactate transporter, required for uptake of lactate and pyruvate; phosphorylated; expression is derepressed by transcriptional activator Cat8p during respiratory growth, and repressed in the presence of glucose, fructose, and mannose |
| MRP1 | Mitochondrial ribosomal protein of the small subunit; MRP1 exhibits genetic interactions with PET122, encoding a COX3-specific translational activator, and with PET123, encoding a small subunit mitochondrial ribosomal protein |
| MRP17 | Mitochondrial ribosomal protein of the small subunit; MRP17 exhibits genetic interactions with PET122, encoding a COX3-specific translational activator |
| TPI1 | Triose phosphate isomerase, abundant glycolytic enzyme; mRNA half-life is regulated by iron availability; transcription is controlled by activators Reb1p, Gcr1p, and Rap1p through binding sites in the 5' non-coding region |
| PKH3 | Protein kinase with similarity to mammalian phosphoinositide-dependent kinase 1 (PDK1) and yeast Pkh1p and Pkh2p, two redundant upstream activators of Pkc1p; identified as a multicopy suppressor of a pkh1 pkh2 double mutant |
| YGL079W | Putative protein of unknown function; green fluorescent protein (GFP)-fusion protein localizes to the endosome; identified as a transcriptional activator in a high-throughput yeast one-hybrid assay |
| TFB1 | Subunit of TFIIH and nucleotide excision repair factor 3 complexes, required for nucleotide excision repair, target for transcriptional activators |
| PET123 | Mitochondrial ribosomal protein of the small subunit; PET123 exhibits genetic interactions with PET122, which encodes a COX3 mRNA-specific translational activator |
| MHR1 | Protein involved in homologous recombination in mitochondria and in transcription regulation in nucleus; binds to activation domains of acidic activators; required for recombination-dependent mtDNA partitioning |
| MCM1 | Transcription factor involved in cell-type-specific transcription and pheromone response; plays a central role in the formation of both repressor and activator complexes |
| EGD1 | Subunit beta1 of the nascent polypeptide-associated complex (NAC) involved in protein targeting, associated with cytoplasmic ribosomes; enhances DNA binding of the Gal4p activator; homolog of human BTF3b |
| STE5 | Pheromone-response scaffold protein; binds Ste11 p, Ste7p, and Fus3p kinases, forming a MAPK cascade complex that interacts with the plasma membrane and Ste4p-Ste18p; allosteric activator of Fus3p that facilitates Ste7p-mediated activation |
| RGT1 | Glucose-responsive transcription factor that regulates expression of several glucose transporter (HXT) genes in response to glucose; binds to promoters and acts both as a transcriptional activator and repressor |
| TYE7 | Serine-rich protein that contains a basic-helix-loop-helix (bHLH) DNA binding motif; binds E-boxes of glycolytic genes and contributes to their activation; may function as a transcriptional activator in Ty1-mediated gene expression |
| VMA13 | Subunit H of the eight-subunit V1 peripheral membrane domain of the vacuolar H+-ATPase (V-ATPase), an electrogenic proton pump found throughout the endomembrane system; serves as an activator or a structural stabilizer of the V-ATPase |
| GAL11 | Subunit of the RNA polymerase II mediator complex; associates with core polymerase subunits to form the RNA polymerase II holoenzyme; affects transcription by acting as target of activators and repressors |
| VAC14 | Protein involved in regulated synthesis of Ptdlns(3,5)P(2), in control of trafficking of some proteins to the vacuole lumen via the MVB, and in maintenance of vacuole size and acidity; interacts with Fig4p; activator of Fab1p |

### Example 7: Cloning of HEXA and HEXB genes

Aspergillus parasiticus (ATCC 24690) cultures were grown in malt extract broth media (15 g/L malt extract broth, Difco) with shaking at 25 °C for 3 days. A. parasiticus pellets were transferred to a 1.5mL tube to provide a volume of pellets equal to approximately 500uL. The mycelia were frozen in a dry ice ethanol bath, transferred to a mortar and pestle, and ground into a fine powder. The powder was placed in a 1.5mL tube with approximately 500uL 0.7mm Zirconia beads, and total RNA was prepared using a Ribopure Plant Kit (Ambion), according to manufacturer's recommendations.

First strand synthesis of cDNA was performed with gene-specific primers oAA0031 (for HEXA) and oAA0041 (for HEXB) in a reaction containing 0.2uL of gene-specific primer (10uM), 300ng total RNA, 1.0uL dNTP (10mM and sterile water to bring the volume to 13uL. The total RNA/primer mixtures were heated at 65°C for 5 minutes then cooled on ice for 5 minutes before the addition of 4uL 5X First strand buffer, 1 uL 0.1 M DTT, 1 uL H20, and 1 uL Superscript III RT (Invitrogen). First strand synthesis reactions were incubated at 55°C for 1 hour, followed by inactivation of the enzyme at 70°C for 15 minutes and cooling of the reactions to 4°C. The primers utilized for isolation of HEXA and HEXB genes were configured to independently amplify the HEXA and HEXB genes in fragments, having fragment lengths in the range of between about 1.0 kilobases (kb) to about 1.6kb, with approximately 200bp of overlapping sequence between the fragments. The sequences are shown in the tables below.

| Oligonucleotides for cloning of HEXA DNA fragments | | | |
|---|---|---|---|
| Oligos | Sequence | HEXA sequence | PCR product (bp) |
| oAA0022 | ATGGTCATCCAAGGGAAGAGATTGGCCGCCTCCTCTATTCAGC | 1-1149 | 1149 |
| oAA0023 | GTAGGCGTCACAGGAAAGACTGCGTACCA | | |
| oAA0024 | TATCACCAATGCTGGATGTAAAGAAGTCGCG | 941-2270 | 1330 |
| oAA0025 | AATTGGGCTAGGAAACCGGGGATGC | | |
| oAA0026 | CGGTCTAATGACGGCGCATGATATCATAGCCGAAACGGTCGAG | 2067-3016 | 950 |
| oAA0027 | ACTTGGCTGGAGTCCATCCCTTCGGCA | | |
| oAA0028 | CTGCCCGAGTTTGAAGTATCTCAACTTACCGCCGACGCCATG | 2812-4181 | 1370 |
| oAA0029 | TGAGACGCGCTGCGCAGGGC | | |
| oAA0030 | CGAGGTGATCGAGACGCAGATGC | 3975-5016 | 1042 |
| oAA0031 | TTATGAAGCACCAGACATCAGCCCCAGC | | |
| oAA0046 | gtactagtaaaaaaATGGTCATCCAAGGGAAGAGATTGGCCGCCT CCTCTATTCAGC | 1-5016 | 5041 |
| oAA0047 | gtcccgggctaTTATGAAGCACCAGACATCAGCCCCAGC | | |
| oAA0051 | tacccgggctattagtgatggtggtgatggtgTGAAGCACCAGAC ATCAGCCCCAGC | 1-5016 | 5062 |

| Oligonucleotides for cloning of HEXB DNA fragments | | | |
|---|---|---|---|
| Oligos | Sequence | HEXB sequence | PCR product (bp) |
| oAA0032 | ATGGGTTCCGTTAGTAGGGAACATGAGTCAATC | 1-1166 | 1166 |
| oAA0033 | GTTCCTTGTGTGAGCTCCTGAATAAGACTGCATG | | |
| oAA0034 | CCATCAAAATCCCCCTCTATCACACGGGCACTGGGAGCAAC | 962-2042 | 1081 |
| oAA0035 | CCCACGCCTTGCGCATCTATAATCAGG | | |
| oAA0036 | TGTCCGAATATTCTCCTCGTTGTAGGTAGTGGATT | 1837-3527 | 1691 |
| oAA0037 | GCAGTAGTCGATAGGTACACATCCTTGGGGGTTCCATGACTGC | | |
| oAA0038 | AGAGGATCAAGGCATTATACATGAGTCTGTGGAACTTGGGCTTTC C | 3323-4461 | 1139 |
| oAA0039 | TTCCCCGTCCTCCATGGCCTTATGC | | |
| oAA0040 | GGCCTTTGCGCGATACGCTGGTCTCTCGGGTCCCAT | 4256-5667 | 1412 |
| oAA0041 | TCACGCCATTTGTTGAAGCAGGGAATG | | |
| oAA0048 | gtactagtaaaaaaATGGGTTCCGTTAGTAGGGAACATGAGTCAA TC | 1-5667 | 5694 |
| oAA0049 | gtgtttaaacctaTCACGCCATTTGTTGAAGCAGGGAATG | | |
| oAA0111 | ggtttaaacctatcagtgatggtggtgatggtgCGCCATTTGTTG AAGCAGGGAATGAA | 1-5667 | 5714 |

HEXA and HEXB gene fragments were PCR amplified using the cDNA generated above by the addition of 5uL 10X Pfu reaction buffer, 1.OuL dNTPs (1 mM), 1.OuL Sense and Antisense Primer Mix (1 out), 1.OuL Pfu Ultra Fusion HS (Agilent), 2.OuL cDNA, 40uL sterile H20. Thermocycling parameters used to amplify the HEXA and HEXB genes were 94°C for 5 minutes, 40 cycles of 94°C 30 seconds, 62°C 40 seconds, 72°C 4 minutes, followed by 72°C 10 minutes and a 4°C hold. PCR products of the correct size were gel purified and ligated into pCR-Blunt II-TOPO (Invitrogen), transformed into competent TOP10 E. coli cells (Invitrogen) and clones containing PCR inserts were sequenced to confirm correct DNA sequence.

DNA fragments of HEXA and HEXB were PCR amplified using the sequence-confirmed fragments in pCR-Bluntll as template in order to produce overlapping DNA fragments covering the entire sequence of both HEXA and HEXB. The overlapping DNA fragments for each gene were combined in a 50uL overlap extension PCR reaction containing each DNA fragment at 0.2nM, sense and antisense primers at 0.2uM each, 1X Pfu reaction buffer, 1.0uL Pfu Ultra Fusion HS polymerase, and 0.2mM dNTPs. Unique restriction sites were incorporated into the sense and antisense primers to allow for cloning the HEXA and HEXB genes into p425GPD and p426GPD respectively. For HEXA the restriction sites were Spel / Smal and for HEXB the restriction sites were Spel / Pmel. Ligation of the HEXA and HEXB genes into p425GPD and p426GPD resulted in plasmids pAA020 and pAA021 respectively. Variants of the HEXA and HEXB genes that incorporated C-terminal 6xHis tags were constructed by using an antisense primer encoding a 6xHis sequence. Ligation of the HEXA-6xHis and HEXB-6xHis genes into p425GPD and p426GPD resulted in plasmids pAA031 and pAA032, respectively. Vectors pAA020, pAA021, pAA031 and pAA032 were used to demonstrate protein expression in S. cerevisiae, as shown in Figures 11 and 12.

### Example 8: Cloning of STCJ and STCK genes

Total RNA was prepared from Aspergillus nidulans (ATCC 38163), as described in Example 1. First strand synthesis of cDNA was performed with gene-specific primers oAA0008 (for STCJ) and oAA0021 (for STCK) in a reaction containing 0.2uL of gene-specific primer (10uM), 300ng total RNA, 1.0uL dNTP (10mM and sterile water to bring the volume to 13uL. The total RNA/primer mixtures were heated at 65°C for 5 minutes then cooled on ice for 5 minutes before the addition of 4uL 5X First strand buffer, 1 uL 0.1 M DTT, 1 uL H20, and 1 uL Superscript III RT (Invitrogen). First strand synthesis reactions were incubated at 55°C for 1 hour, followed by inactivation of the enzyme at 70°C for 15 minutes and cooling of the reactions to 4°C. Primers design strategies substantially similar to those described herein were used to amplify the STCJ and STCK genes in fragments in the range of between about 1.1 kb to about 1.6kb, with approximately 200bp of overlapping sequence between the fragments. The primers used to amplify the STCJ and STCK genes are shown in the tables below.

| Oligonucleotides for cloning of STCJ DNA fragments | | | |
|---|---|---|---|
| Oligos | Sequence | STCJ sequence | PCR product (bp) |
| oAA0001 | ATGACCCAAAAGACTATACAGCAGGTCCCAAGA | 1-1290 | 1290 |
| oAA0002 | TATGGTGCATCGAATGTTGTTTGCCTGG | | |
| oAA0009 | AAAATGCGTGAGCACTTTGTCCAGCGC | 1021-2506 | 1486 |
| oAA0004 | CGACGTAATTGACGTTGTCAACATGCCG | | |
| oAA0005 | CATCTCGGGTTCCCATCACTCCCTGAGTATGAC | 2284-3424 | 1141 |
| oAA0006 | GACAAAGAAGCTGGACACCGCAGCCTTGGGATTCCACGAAC | | |
| oAA0007 | GATCTGCCTTGTCGGTGGCTATGACGACCTTCAGCCTGAGGAGTCA | 3234-4680 | 1447 |
| oAA0008 | TTAACGGATGATAGAGGCCAACGGCCAAAGACACCACTTGCGTACA C | | |
| oAA0126 | cacacaactagtaaaaaaATGACCCAAAAGACTATACAGCAGGTCC CAAGA | 1-4680 | 4710 |
| oAA0127 | tgtgtgcccgggTTAACGGATGATAGAGGCCAACGGCCAAAGACAC CACTTGCGTACAC | | |
| oAA0154 | tacccgggctattagtgatggtggtgatggtgACGGATGATAGAGG CCAAC | 1-4680 | 4730 |

| Oligonucleotides for cloning of STCK DNA fragments | | | |
|---|---|---|---|
| Oligos | Sequence | STCK sequence | PCR product (bp) |
| oAA0012 | ATGACTCCATCACCGTTTCTCGATGCTGT | 1-1110 | 1110 |
| oAA0013 | CACATGGGTAGCATCGTTCATTGCCCAACACAAAGCGGGCCAGTTA ACTC | | |
| oAA0014 | GTCGAGCTAAGAGTGACTGATGCCATTGGC | 901-2510 | 1610 |
| oAA0015 | CGTAATTCAGCTTCTGAACCTGAGCCCAGG | | |
| oAA0016 | CTTTGCCCGGCCGTGGTTCGC | 2301-3555 | 1255 |
| oAA0017 | CCCCCAAGCTCGACAACGGGC | | |
| oAA0018 | TTCTCAAAATGCACCGGACTGATTACTTGGA | 3350-4682 | 1333 |
| oAA0019 | CCCATTCCTCTCTCCTGCGTGCCCTGGCCGGTAAAGACGTAT | | |
| oAA0020 | CCCTCCTTCGATGGACTTGTCCGGGCAAACGACCGGTTGCGAATGG AGAT | 4477-5745 | 1268 |
| oAA0021 | CTACCTATTCTCTTCAACCCGCCGTAACAGC | | |
| oAA0128 | cacacaactagtaaaaaaATGACTCCATCACCGTTTCTCGATGCTG T | 1-5745 | 5775 |
| oAA0129 | tgtgtgcccgggCTACCTATTCTCTTCAACCCGCCGTAACAGC | | |
| oAA0170 | tgtgtgcccgggctatcagtgatggtggtgatggtgCCTATTCTCT TCAAC | 1-5745 | 5796 |

STCJ and STCK fragments were amplified using the cDNA prepared above in PCR reactions containing 5uL 10X Pfu reaction buffer, 1.0uL dNTPs (10mM 1.0uL Sense and Antisense Primer Mix (10uM), 1.0uL Pfu Ultra Fusion HS (Agilent), 2.0uL cDNA, 40uL sterile H20. Thermocycling parameters used were 94°C for 5 minutes, 40 cycles of 94°C 30 seconds, 62°C 40 seconds, 72°C 4 minutes, followed by 72°C 10 minutes and a 4°C hold. PCR products of the correct size were gel purified and ligated into pCR-Blunt II-TOPO (Invitrogen), transformed into competent TOP10 E. coli cells (Invitrogen). PCR inserts were sequenced to confirm the correct DNA sequence. DNA fragments of STCJ and STCK were PCR amplified using the sequence-confirmed fragments in pCR-Bluntll as template in order to produce overlapping DNA fragments covering the entire sequence of both STCJ and STCK. The overlapping DNA fragments for each gene were combined in a 50uL overlap extension PCR reaction containing each DNA fragment at 0.2nM, sense and antisense primers at 0.2uM each, 1X Pfu reaction buffer, 1.0uL Pfu Ultra Fusion HS polymerase, and 0.2mM dNTPs.

Sense and antisense primers were designed to incorporate unique restriction sites for cloning the STCJ and STCK genes into p425GPD and p426GPD respectively. For STCJ the restriction sites were Spel / Xmal and for STCK the restriction sites were Spel / Smal. Ligation of the STCJ and STCK genes into p425GPD and p426GPD resulted in plasmids pAA040 and pAA042 respectively. Variants of the STCJ and STCK genes that incorporated C-terminal 6xHis tags were constructed by using an antisense primer encoding a 6xHis sequence. Ligation of the STCJ-6xHis and STCK-6xHis genes into p425GPD and p426GPD resulted in plasmids pAA041 and pAA043. Vectors pAA040, pAA0421 pAA042 and pAA043 were used to demonstrate protein expression in S. cerevisiae, as shown in Figures 11 and 12.

### Example 9: Design and cloning of HEXA and HEXB genes for C. tropicalis alternate genetic code

The HEXA and HEXB genes contain multiple CTG codons, which normally code for leucine. However, certain organisms, Candida tropicalis for example, translate CTG as serine. DNA sequences for HEXA and HEXB were prepared that replaced all CTG codons with TTG codons, which is translated as leucine in C. tropicalis. The TTG codon was chosen due to it being the most frequently used leucine codon in C. tropicalis. The alternate genetic code (AGC) HEXA and HEXB genes were synthesized as equal size fragments with 200bp overlaps and ligated into pUC57 vector (Integrated DNA Technologies). DNA fragments of AGC-HEXA and AGC-HEXB were PCR amplified using the fragments in pUC57 as template in order to produce overlapping DNA fragments covering the entire sequence of both AGC-HEXA and AGC-HEXB.

The overlapping DNA fragments for each gene were combined in a 50uL overlap extension PCR reaction containing each DNA fragment at 0.2nM, sense and antisense primers at 0.2uM each, 1X Pfu reaction buffer, 1.0uL Pfu Ultra Fusion HS polymerase, and 0.2mM dNTPs. Sense and antisense primers incorporated unique Sapl restriction sites for cloning the AGC-HEXA and AGC-HEXB genes into pAA105 resulting in plasmids pAA127 and pAA129 respectively. Gene variants of AGC-HEXA and AGC-HEXB that contained C-terminal 6xHis tags were ligated into pAA105 resulting in plasmids pAA128 and pAA130 respectively. The alternate genetic code primers used to alter leucine codons for C. tropicalis expression of HEXA and HEXB are shown in the tables below.

| Oligonucleotides for cloning of AGC-HEXA DNA fragments | | | |
|---|---|---|---|
| Oligos | Sequence | AGC-HEXA sequence | PCR product (bp) |
| oAA0383 | cacacagctcttctagaATGGTCATCCAAGGGAAGAG | 1-1404 | 1421 |
| oAA0055 | AGTATCGACGTCGGCTGACTTGAGACCA | | |
| oAA0056 | CCATCACATCCACAGTGGCGG | 1205-2609 | 1405 |
| oAA0057 | AACCAGGCAAGTTCGACATAACCGGC | | |
| oAA0058 | GTAGGCTATCCCCGTCTCCCCGATTATG | 2410-3814 | 1405 |
| oAA0059 | TGATTGAGGTCAAGGATGATTTGTCCGAGA | | |
| oAA0060 | TCTTCCTATCTATGCGGTCATTGCCAGCT | 3615-5016 | 1419 |
| oAA0384 | cacacagctcttcctttTTATGAAGCACCAGACATCAAC | | |
| oAA0385 | cacacagctcttcctttttagtgatggtggtgatggtgTGAAGCAC CAGACATCAACCCCAACG | 1-5016 | 5071 |

| Oligonucleotides for cloning of AGC-HEXB DNA fragments | | | |
|---|---|---|---|
| Oligos | Sequence | AGC-HEXB sequence | PCR product (bp) |
| oAA0386 | cacacagctcttctagaATGGGTTCCGTTAGTAGGGA | 1-1566 | 1583 |
| oAA0064 | CAAATCCTTGATGACAGAGATCTGCCAGGA | | |
| oAA0065 | GCTGGGACTTTGTCGCTGCCGTTGCTCAAGCTGGAT | 1367-2933 | 1567 |
| oAA0066 | ACTGCTCCTACTTTCTCGAACTTATAGAGCCCTTG | | |
| oAA0067 | ATATCCGACGATGAGTCTGT | 2734-4299 | 1566 |
| oAA0068 | ATGGACAATGGGACCCGAGA | | |
| oAA0069 | GGACTTCTTGCACCGCTACG | 4101-5667 | 1584 |
| oAA0387 | cacacagctcttcctttTCACGCCATTTGTTGAAGCAAAG | | |
| oAA0388 | cacacagctcttccttttcagtgatggtggtgatggtgCGCCATTT GTTGAAGCA | 1-5667 | 5692 |

### Example 10: Transformation of S. cerevisiae procedure

Competent cells of S. cerevisiae strain BY4742 were prepared using the Frozen-EZ Yeast Transformation II Kit (Zymo Research) following manufacturer's instructions. 50uL aliquots of competent cells were stored at -80 °C until use. Competent cells were transformed by the addition of 0.5-1.0ug of intact plasmid DNA as instructed by the Frozen-EZ Yeast Transformation II Kit (Zymo Research). Selection for transformants was performed by plating on selective media; SC-URA (for p426-based vectors) or SC-LEU (for p425-based vectors).

### Example 11: Transformation of C. tropicalis procedure

5mL YPD start cultures were inoculated with a single colony of C. tropicalis and incubated overnight at 30°C, with shaking at about 200rpm. The following day, fresh 25mL YPD cultures, containing 0.05% Antifoam B, were inoculated to an initial OD₆₀₀ₙₘ of 0.4 and the culture incubated at 30°C, with shaking at about 200rpm until an OD₆₀₀ₙₘ of 1.0-2.0 was reached. Cells were pelleted by centrifugation at 1,000 x *g,* 4°C for 10 minutes. Cells were washed by resuspending in 10mL sterile water, pelleted, resuspended in 1 mL sterile water and transferred to a 1.5mL microcentrifuge tube. The cells were then washed in 1 mL sterile TE/LiOAC solution, pH 7.5, pelleted, resuspended in 0.25mL TE/LiOAC solution and incubated with shaking at 30°C for 30 minutes.

The cell solution was divided into 50uL aliquots in 1.5mL tubes to which was added 5-8ug of linearized DNA and 5uL of carrier DNA (boiled and cooled salmon sperm DNA, 10mg/mL). 300uL of sterile PEG solution (40% PEG 3500, 1X TE, 1X LiOAC) was added, mixed thoroughly and incubated at 30°C for 60 minutes with gentle mixing every 15 minutes. 40uL of DMSO was added, mixed thoroughly and the cell solution was incubated at 42°C for 15 minutes. Cells were then pelleted by centrifugation at 1,000 x *g* 30 seconds, resuspended in 500uL of YPD media and incubated at 30 °C with shaking at about 200rpm for 2 hours. Cells were then pelleted by centrifugation and resuspended in 1 mL 1X TE, cells were pelleted again, resuspended in 0.2mL 1X TE and plated on selective media. Plates were incubated at 30 °C for growth of transformants.

### Example 12: HEXA and HEXB expression in S. cerevisiae

Plasmids pAA031 and pAA032 were transformed into competent BY4742 S. cerevisiae cells independently and in combination. Selection for transformants containing pAA031 was performed on SC-LEU plates. Selection for transformants containing pAA032 was performed on SC-URA plates. Selection for transformants containing both pAA031 and pAA032 was performed on SC-URA-LEU plates. Single colonies were used to inoculate 5mL of SC drop out media and grown overnight at 30 °C, with shaking as described herein. Cells from 3mL of overnight culture were harvested by centrifugation at 12,000rpm for 2 minutes. Cell pellets were incubated at -80 °C until frozen.

Approximately 500uL of cold 0.7mm zirconia beads (Ambion) were added on top of the frozen cell pellets. Yeast lysis buffer (50mM Tris pH 8.0, 0.1% Triton X100, 0.5mM EDTA, 1X ProCEASE protease inhibitors [G Biosciences]) was added to fill the tube leaving as little air in the tube as possible, the tubes were placed on ice during manipulations. Cells were broken using three, 2 minute cycles in a Bead Beater (BioSpec) with 1 minute rests on ice between cycles. 200uL of whole cell extract (WCE) was removed to a new tube and the remainder of the whole cell extract was centrifuged at 16,000 x *g,* 4 °C for 15 minutes to pellet insoluble debris. The supernatant was removed to a new tube as the soluble cell extract (SCE). The protein content in the soluble cell extract was determined by Bradford assay (Pierce). A volume of SCE containing 50ug of protein (and the same volume WCE) was precipitated by the addition of 4 volumes of cold 100% acetone. After centrifugation at 16,000 x *g,* and 4 °C for 15 minutes, the supernatant was carefully removed and the pellet washed with 200uL of cold 80% acetone and centrifuged again. The supernatant again was carefully removed and the cell pellets air dried for 5 minutes.

Protein pellets were then resuspended in 1X LDS sample buffer containing 50mM DTT (Invitrogen) by incubating at 70 °C, with shaking at about 1200rpm. After brief centrifugation and cooling to room temperature, samples (20ug) were separated by SDS PAGE and transferred to nitrocellulose for immunodetection with mouse anti-6xHis antibodies (Abcam). Incubation in 1:5,000 primary antibody was performed overnight at room temperature, incubation in 1:5,000 donkey anti-mouse HRP conjugate secondary antibody was performed for 3 hours at room temperature, and detection was performed with SuperSignal West Pico chemiluminescent substrate (Pierce). Multiple clones displayed soluble expression of both HEXA and HEXB subunits of hexanoate synthase. As shown in Figure 11, a substantial portion of the expressed protein fractionated with the insoluble pellet. Strains sAA061, sAA140, sAA141, sAA142 contained 6xHis-tagged HEXA and HEXB proteins. Strain sAA048 contained only vectors p425GPD and p426GPD.

### Example 13: STCJ and STCK expression in S. cerevisiae

Plasmids pAA041 and pAA043 were cotransformed into competent BY4742 S. cerevisiae. Selection for transformants containing both pAA041 and pAA043 was performed on SC-URA-LEU plates. Culture growth, cell extract preparation, SDS PAGE, and immunodetection were performed as described herein. One clone displayed soluble expression of both STCJ and STCK subunits. As shown in Figure 11, a substantial portion of the expressed protein fractionated with the insoluble pellet. Strain sAA144 contained 6xHis-tagged STCJ and STCK proteins. Strain sAA048 contained only vectors p425GPD and p426GPD.

### Example 14: HEXA and HEXB expression in C. tropicalis

Plasmids pAA128 and pAA130 were linearized using Clal, and cotransformed into competent sAA103 cells (ura3/ura3, pox4::ura3/pox4::ura3, pox5::ura3/pox5::ura3). The Clal recognition sites in the HEXA and HEXB ORF's are blocked due to overlapping dam methylation. Selection for transformants containing integrated vector DNA was performed on SC-URA plates. Confirmation of vector integration was performed by PCR using HEXA and HEXB specific primers. Transformants that were PCR positive for both HEXA and HEXB were selected for analysis of target protein expression. Overnight culture growth was performed as described herein. Fresh 5mL YPD cultures were inoculated from the overnight cultures to an initial OD₆₀₀ₙₘ of 0.4 and incubated until the OD₆₀₀ₙₘ reached ∼5-8, at which point the culture was harvested. Cell extract preparation, SDS PAGE, and immunodetection were performed as described herein. Strains sAA269 and sAA270 contained plasmids pAA128 and pAA130 integrated into the genome for expression of 6xHis-tagged HEXA and HEXB proteins. Both strains displayed soluble expression of 6xHis-tagged HEXA and HEXB subunits as shown in Figure 12. 6xHis tagged HEXA and HEXB expressed in strains sAA269 and sAA270 are indicated with arrows. 6xHis tagged STCJ and STCK from strain sAA144 were included as a positive control. Strain sAA103 is the parent strain for sAA269 and sAA270 and does not contain integrated vectors for the expression of 6xHis-tagged HEXA and HEXB.

### Example 15: Procedure for recycling of the URA3 marker

C. tropicalis has a limited number of selectable marker, as compared to S. cerevisiae, therefore, the URA3 marker is "recycled" to allow multiple rounds of selection using URA3. To reutilize the URA3 marker for subsequent engineering of C. tropicalis, a single colony having the Ura⁺ phenotype was inoculated into 3 mL YPD and grown overnight at 30°C with shaking. The overnight culture was then harvested by centrifugation and resuspended in 1 mL YNB+YE (6.7 g/L Yeast Nitrogen Broth, 3g/L Yeast Extract). The resuspended cells were then serially diluted in YNB+YE and 100 uL aliquots plated on YPD plates (incubation overnight at 30 °C) to determine titer of the original suspension. Additionally, triplicate 100 uL aliquots of the undiluted suspension were plated on SC Dextrose (Bacto Agar 20g/L, Uracil 0.3 g/L, Dextrose 20 g/L, Yeast Nitrogen Broth 6.7 g/L, Amino Acid Dropout Mix 2.14 g/L) and 5-FOA.at 3 different concentrations (0.5, 0.75, 1 mg/mL).

Plates were incubated for at least 5 days at 30 °C. Colonies arising on the SC Dextrose + 5-FOA plates were resuspended in 50 uL sterile, distilled water and 5 uL utilized to streak on to YPD and SC-URA (SC Dextrose medium without Uracil) plates. Colonies growing only on YPD and not on SC-URA plates were then inoculated into 3 mL YPD and grown overnight at 30 °C with shaking. Overnight cultures were harvested by centrifugation and resuspended in 1.5 mL YNB (6.7 g/L Yeast Nitrogen Broth). The resuspended cells were serially diluted in YNB and 100 uL aliquots plated on YPD plates and incubation overnight at 30 °C to determine initial titer. 1 mL of each undiluted cell suspension also was plated on SC-URA and incubated for up to 7 days at 30 °C. Colonies on the SC-URA plates are revertants and the isolate with the lowest reversion frequency (< 10⁻⁷) was used for subsequent strain engineering.

### Example 16: Omega oxidation of hexane and hexanoic acid to adipic acid

Starter cultures of strain sAA003 were grown in YPD_{2.0} (1% yeast extract, 2% peptone, 2% dextrose) overnight as described. Starter cultures were used to inoculate 100mL of fresh YPD_{2.0} to an initial OD₆₀₀ₙₘ of 0.4 and incubated overnight at 30°C, with shaking at about 200rpm. The 100mL culture was pelleted by centrifugation at 4,000 x *g,* 23°C for 10 minutes and resuspended in 100mL fresh YPD_{0.1} media (1% yeast extract, 1% peptone, 0.1% dextrose). The culture was divided into 4 x 25mL cultures to which were added either 1% hexane, 0.05% hexanoic acid, 1.0% hexanoic acid, or no other carbon source. Strain sAA003 is completely blocked in β-oxidation, therefore fermentation tested the ability of the β-oxidation pathway to oxidize C6 substrates. Samples were taken at 24, 48, and 72 hours and analyzed by LC-MS (Scripps Center for Mass Spectrometry) using published methods for the detection of adipic acid (Cheng et al., 2000). The data for the 72 hour time-point, shown in the table below demonstrates that strain sAA003 was able to oxidize both hexanoic acid and hexane to adipic acid. The results also indicate that the 1% hexanoic acid level was toxic to the cells leading to no production of adipic acid over background levels.

| **Oxidation of hexane and hexanoic acid to adipic acid** | | |
|---|---|---|
| **Time (h)** | **MEDIA** | **Adipic acid (mg/L)** |
| 0 | YPD_{0.1} | 0.000 |
| 72 | YPD_{0.1} | 0.005 |
| 72 | YPD_{0.1}+ 0.05% Hexanoic Acid | 0.406 |
| 72 | YPD_{0.1} + 1% Hexanoic Acid | 0.003 |
| 72 | YPD_{0.1} + 1% Hexane | 0.091 |

### Example 17: Identification of P450 alleles induced by exposure to hexane or hexanoic acid

350 mL cultures grown overnight in YNB-Salts + 2.0% Glucose (6.7 g/L Yeast Nitrogen Broth, 3.0 g/L Yeast Extract, 3.0 g/L ammonium sulfate, 3.0 g/L monopotassium phosphate, 0.5 g/L sodium chloride, and 20 g/L dextrose) were inoculated from a 3 mL overnight culture of YPD (1% Yeast Extract, 2% Peptone, 2% Dextrose), and used for RNA preparation. Cultures were harvested by centrifugation. Each pellet was resuspended in 100 mL of YNB-Salts medium with no glucose. A 1 mL aliquot was taken for RNA isolation as a time=0 control. To each 100 mL suspension, a different inducer was added, 1% glucose, 1% hexane or 0.05% hexanoic acid and aliquoted as two 50 mL portions into 250 mL baffled flasks and incubated for 2 or 4 hours at 30 °C with shaking. At 2 hours and 4 hours, one flask for each inducer was harvested by centrifugation and resuspended in its own spent media in order to collapse culture foam. 1 mL samples were isolated by centrifugation of 1 mL of each culture and RNA prepared using the RiboPure-Yeast Kit, according to the manufacturer's directions, with an additional extraction of the initial RNA preparations with 1 volume of Chloroform:Isoamyl Alcohol (24:1) to the aqueous phase after lysis and extraction with Phenol:Chloroform:Isoamyl Alcohol (25:24:1).

Each RNA preparation was further purified by precipitation with ethanol and treatment with DNase I, again according to manufacturers' recommendations. All RNA preparations were shown to be free of contaminating genomic DNA by electrophoresis and by failure to prime a PCR product of the URA3 gene. First strand synthesis reactions were completed for each RNA preparation using Superscript III Reverse Transcriptase (Invitrogen), as described herein. Reactions for each sample consisted of 1 uL oAA0542 (polyT 10 uM), 1 uL dNTP mix (10 mM each), 1 µg RNA in 13 uL sterile, distilled water. The RNA/primer mix was heated to 65 °C for 5 minutes, and on ice for 1 minute. Primers were generated that amplified a substantially unique area of each cytochrome P450 and are shown in the table below.

PCR reactions were performed on the 2 hour induced cDNA samples and compared to the Time = 0 and genomic DNA controls. PCR reactions for each cDNA and primer pair combination consisted of 0.5 uL template, sense and antisense primers at 0.4 uM each, 1X Taq DNA polymerase Buffer (New England Biolabs), 0.1 uL Taq DNA polymerase, and 0.2mM dNTPs and sterile, distilled water to 25 uL. Cycling parameters used were 95 °C for 5 minutes, 30 cycles of 95 °C 30 seconds, 50 °C 40 seconds, 72 °C 2 minutes, followed by 72°C 5 minutes and a 4°C hold. PCR reactions were electrophoresed on 1.2% agarose gels to identify differential expression due to the inducer used. Several P450s displayed increased induction in the presence of hexane or hexanoic acid, however the results were not quantitative. Two Cytochrome P450's, CYP52A15 and CYP52A16, showed induction only in the presence of hexane and hexanoic acid and not in the presence of glucose, as shown in Figure 13. The primers used for PCR analysis of induced expression are shown in the table below.

| **Oligonucleotides for identification of P450 DNA fragments** | | | | |
|---|---|---|---|---|
| **Oligos** | **Sequence** | **P450** | **P450 sequence** | **PCR product (bp)** |
| oAA0082 | gattactgcagcaatattaatcttc | CYP52A12 | 60-249 | 190 |
| oAA0083 | gtcgaaaacttcatcggcaaag | | | |
| oAA0084 | cacgatattatcgccacatacttc | CYP52A13 | 10-256 | 247 |
| oAA0085 | cgggacgatcgagatcgtggatacg | | | |
| oAA0086 | caggatattatcgccacatacatc | CYP52A14 | 10-256 | 247 |
| oAA0087 | ctggacgattgagcgcttggatacg | | | |
| oAA0088 | cgtcttctccatcgtttgcccaagag | CYP52A15 | 5-199 | 195 |
| oAA0089 | ggtccctgacaaagttaccgagtg | | | |
| oAA0090 | cgtcttctccatcgtttgctcaggag | CYP52A16 | 5-199 | 195 |
| oAA0091 | gatccaacacgacgttaccgaqcg | | | |
| oAA0092 | ggtatgtcgttgtgccagtgttg | CYP52A17 | 26-248 | 223 |
| oAA0093 | cccacgcttgggttcttggagtggtc | | | |
| oAA0094 | ggtatattgttgtgcctgtpttg | CYP52A18 | 26-248 | 223 |
| oAA0095 | ccgacgcttgggttcttggagctgtc | | | |
| oAA0096 | ggaaggatgaggtggtgcaptac | CYP52A19 | 1217-1458 | 242 |
| oAA0097 | gtcttgtgacaagtttggaaactc | | | |
| oAA0098 | gaaagaatgaggtggtgcaatac | CYP52A20 | 1217-1458 | 242 |
| oAA0099 | gtcctgtgacaagctagggaattc | | | |
| oAA0104 | ctatcgtgggatgtgatctgtgtcg | CYP52D2 | 19-231 | 213 |
| oAA0105 | ctcgaatctcttgacactgaactcg | | | |

### Example 18: Cloning and Analysis of C. tropicalis Fatty alcohol oxidase (FAO) alleles

### Isolation of fatty alcohol oxidase genes from C. tropicalis

C. tropicalis (ATCC20336) fatty alcohol oxidase genes were isolated by PCR amplification using primers generated to amplify the sequence region covering promoter, fatty alcohol oxidase gene (FAO) and terminator of the FAO1 sequence (GenBank accession number of FAO1 AY538780). The primers used to amplify the fatty alcohol oxidase nucleotide sequences from C. tropicalis strain ATCC20336, are showing in the table below.

| **Oligonucleotides for cloning FAO alleles** | |
|---|---|
| Oligo | Sequence |
| oAA0144 | AACGACAAGATTAGATTGGTTGAGA |
| oAA0145 | GTCGAGTTTGAAGTGTGTGTCTAAG |
| oAA0268 | AGATCTCATATGGCTCCATTTTTGCCCGACCAGGTCGACTACAAACACGTC |
| oAA0269 | ATCTGGATCCTCATTACTACAACTTGGCTTTGGTCTTCAAGGAGTCTGCCAAACCTAAC |
| oAA0282 | ACATCTGGATCCTCATTACTACAACTTGGCCTTGGTCT |
| oAA0421 | CACACAGCTCTTCTAGAATGGCTCCATTTTTGCCCGACCAGGTCGAC |
| oAA0422 | CACACAGCTCTTCCTTTCTACAACTTGGCTTTGGTCTTCAAGGAGTCTGC |
| oAA0429 | GTCTACTGATTCCCCTTTGTC |
| oAA0281 | TTCTCGTTGTACCCGTCGCA |

PCR reactions contained 25uL 2X master mix, 1.5 uL of oAA0144 and oAA0145 (10uM), 3.0uL genomic DNA, and 19uL sterile H₂0. Thermocycling parameters used were 98 °C for 2 minutes, 35 cycles of 98 °C 20 seconds, 52 °C 20 seconds, 72 °C 1 minute, followed by 72 °C 5 minutes and a 4 °C hold. PCR products of the correct size were gel purified, ligated into pCR-Blunt II-TOPO (Invitrogen) and transformed into competent TOP10 E. coli cells (Invitrogen). Clones containing PCR inserts were sequenced to confirm correct DNA sequence. Four FAO alleles were identified from sequence analysis and designated as FAO-13, FAO-17, FAO-18 and FAO-20. The sequence of the clone designated FAO-18 had a sequence that was substantially identical to the sequence of FAO1 from GenBank. The resulting plasmids of the four alleles were designated pAA083, pAA084, pAA059 and pAA085, respectively. Sequence identity comparisons of FAO genes isolated as described herein are shown in the tables below.

| **DNA sequence identity** | | | | | | | |
|---|---|---|---|---|---|---|---|
| | FAO1 | FAO-18 | FAO-17 | FAO-13 | FAO-20 | FAO2a | FAO2b |
| FAO1 | 100 | 100 | 98 | 96 | 95 | 83 | 82 |
| FAO-18 | | 100 | 98 | 96 | 95 | 83 | 82 |
| FAO-17 | | | 100 | 98 | 98 | 83 | 82 |
| FAO-13 | | | | 100 | 99 | 83 | 83 |
| FAO-20 | | | | | 100 | 83 | 83 |
| FAO2a | | | | | | 100 | 96 |
| FAO2b | | | | | | | 100 |

| **Protein sequence identity** | | | | | | | |
|---|---|---|---|---|---|---|---|
| | FASO1 | FAO-18 | FAO-17 | FAO-13 | FAO-20 | FAO2a | FAO2b |
| FAO1 | 100 | 100 | 99 | 98 | 98 | 81 | 80 |
| FAO-18 | | 100 | 99 | 98 | 98 | 81 | 80 |
| FAO-17 | | | 100 | 99 | 99 | 82 | 81 |
| FAO-13 | | | | 100 | 99 | 82 | 81 |
| FAO-20 | | | | | 100 | 82 | 81 |
| FAO2a | | | | | | 100 | 97 |
| FAO2b | | | | | | | 100 |

| **Amino acid differences in FAO alleles** | | | | | | | | | | |
|---|---|---|---|---|---|---|---|---|---|---|
| | 32 | 75 | 89 | 179 | 185 | 213 | 226 | 352 | 544 | 590 |
| FAO1 | E | M | G | L | Y | T | R | H | S | P |
| FAO-13 | Q | T | A | L | Y | A | K | Q | A | A |
| FAO-20 | Q | T | A | M | D | A | K | Q | A | A |

### Expression of FAO alleles in E. coli

To determine the levels of FAO enzyme activity with respect to various carbon sources, the four isolated FAO alleles were further cloned and over-expressed in E. coli. The FAOs were amplified using the plasmids mentioned above as DNA template by PCR with primers oAA0268 and oAA0269 for FAO-13 and FAO-20 and oAA0268 and oAA0282 for FAO-17 and FAO-18, using conditions as described herein. PCR products of the correct size were gel purified and ligated into pET11a vector between Ndel and BamHI sites and transformed into BL21 (DE3) E. coli cells. The colonies containing corresponding FAOs were confirmed by DNA sequencing. Unmodified pET11 a vector also was transformed into BL21 (DE3) cells, as a control. The resulting strains and plasmids were designated sAA153 (pET11 a), sAA154 (pAA079 containing FAO-13), sAA155 (pAA080 containing FAO-17), sAA156 (pAA081 containing FAO-18) and sAA157 (pAA082 containing FAO-20), respectively. The strains and plasmids were used for FAO over-expression in E. coli. One colony of each strain was transferred into 5 mL of LB medium containing 100 µg/mL ampicillin and grown overnight at 37 °C, 200 rpm. The overnight culture was used to inoculate a new culture to OD₆₀₀ₙₘ 0.2 in 25 ml LB containing 100 µg/ml ampicillin. Cells were induced at OD₆₀₀ₙₘ 0.8 with 0.3 mM IPTG for 3 hours and harvested by centrifugation at 4 °C 1,050xg for 10 minutes. The cell pellet was stored at -20 °C.

### Expression of FAOs in C. tropicalis

Two alleles, FAO-13 and FAO-20, were chosen for amplification in C. tropicalis based on their substrate specificity profile, as determined from enzyme assays of soluble cell extracts of E. coli with over expressed FAOs. DNA fragments containing FAO-13 and FAO-20 were amplified using plasmids pAA079 and pAA082 as DNA templates, respectively, by PCR with primers oAA0421 and oAA0422. PCR products of the correct sizes were gel purified and ligated into pCR-Blunt II-TOPO (Invitrogen), transformed into competent TOP10 E. coli cells (Invitrogen) and clones containing FAO inserts were sequenced to confirm correct DNA sequence. Plasmids containing FAO-13 and FAO-20 were digested with Sapl and ligated into vector pAA105, which includes the C. tropicalis PGK promoter and terminator. The resulting plasmids were confirmed by restriction digestion and DNA sequencing and designated as pAA115 (FAO-13) and pAA116 (FAO-20), respectively. Plasmids pAA115 and pAA116 were linearized with Spel, transformed into competent C. tropicalis Ura strains sAA002 (SU-2, ATCC20913) and sAA103. The integration of FAO-13 and FAO-20 was confirmed by colony PCR using primers oAA0429 and oAA0281. The resulting strains were designated as sAA278 (pAA115 integrated in strain sAA002), sAA280 (pAA116 integrated in sAA002), sAA282 (pAA115 integrated in sAA103), and sAA284 (pAA116 integrated in sAA103), and were used for fatty alcohol oxidase over-expression in C. tropicalis.

One colony of each strain was inoculated into 5 ml YPD and grown overnight as described herein. The overnight culture was used to inoculate a new 25 mL YPD culture to about OD₆₀₀ₙₘ 0.5. FAO over-expression was regulated by the PGK promoter/terminator, induced with glucose in the medium and expressed constitutively. Strains sAA002 and sAA103 (e.g., untransformed starting strains) were included as negative controls for FAO over-expression. Cells were harvested at early log phase (OD₆₀₀ₙₘ = in the range of between about 3 to about 5) by centrifugation at 4 °C for 10 minutes at 1,050 x *g.* Cell pellets were stored at -20 °C.

### Cell extract preparation from E. coli

Cell pellets from 25 mL of FAO expressing E. coli cultures were resuspended in 10 mL phosphate-glycerol buffer containing 50 mM potassium phosphate buffer (pH7.6), 20% glycerol, 1 mM Phenylmethylsulfonyl fluoride (PMSF), 2uL Benzonase 25U/uL, 20uL 10mg/mL lysozyme. The cells were then lysed by incubation at room temperature for 50 minutes on a rotating shaker, and the cell suspension centrifuged for 30 minutes at 4°C using 15,000 x *g* for. The supernatant was aliquoted in 1.5 ml microcentrifuge tubes and stored at -20 °C for FAO enzyme activity assays.

### Cell extract preparation from C. tropicalis

Frozen C. tropicalis cell pellets were resuspended in 1.2 ml of phosphate-glycerol buffer containing 50 mM potassium phosphate buffer (pH7.6), 20% glycerol, 1 mM Phenylmethylsulfonyl fluoride (PMSF). Resuspended cells were transferred to 1.5mL screw-cap tubes containing about 500uL of zirconia beads on ice. The cells were lysed with a Bead Beater (Biospec) using 2 minute pulses and 1 minute rest intervals on ice. The process was repeated 3 times. The whole cell extract was then transferred to a new 1.5 ml tube and centrifuged at 16,000 x *g* for 15 minutes at 4°C. The supernatant was transferred into a new tube and used for FAO enzyme activity assays.

### Protein concentration determination

Protein concentration of the cell extracts was determined using the Bradford Reagent following manufacturers' recommendations (Cat# 23238, Thermo scientific).

### FAO enzyme activity assay

FAO enzyme activity assays were performed using a modification of Eirich et al., 2004). The assay utilizes a two-enzyme coupled reaction (e.g., FAO and horse radish peroxidase (HRP)) and can be monitored by spectrophotometry. 1-Dodecanol was used as a standard substrate for fatty alcohol oxidase enzymatic activity assays. FAO oxidizes the dodecanol to dodecanal while reducing molecular oxygen to hydrogen peroxide simultaneously. HRP reduces (2,2'-azino-bis 3-ethylbenzthiazoline-6-sulfonic acid; ABTS) in the two-enzyme coupled reaction, where the electron obtained from oxidizing hydrogen peroxide to ABTS, which can be measured by spectrometry at 405 nm. The assay was modified using aminotriazole (AT) to prevent the destruction of H₂O₂ by endogenous catalase, thus eliminating the need for microsomal fractionation. The final reaction mixture (1.0 mL) for FAO enzyme assay consisted of 500 µL of 200 mM HEPES buffer, pH 7.6; 50 µL of a 10 mg/mL ABTS solution in deionized water; 10 µL of 5 mM solution of dodecanol in acetone; 40 µL of 1 M AT and 5 µL of a 2 mg/mL horseradish peroxidase solution in 50 mM potassium phosphate buffer, pH 7.6. Reaction activity was measured by measuring light absorbance at 405 nm for 10 minutes at room temperature after adding the extract. The amount of extract added to the reaction mixture was varied so that the activity fell within the range of 0.2 to 1.0 ΔA₄₀₅ₙₘ/min. The actual amounts of extract used were about 1.69 U/mg for E. coli expressed FAO-13, 0.018U/mg for E. coli expressed FAO-17, 0.35U/mg for E. coli expressed FAO-18 (e.g., FAO1), 0.47U/mg E. coli expressed FAO-20, 0.036U/mg C. tropicalis (strain sAA278) expressed FAO-13, 0.016U/mg C. tropicalis (strain sAA282) expressed FAO-13, 0.032U/mg C. tropicalis (strain sAA280) expressed FAO-20 and 0.029U/mg C. tropicalis (strain sAA284) expressed FAO-20. FAO activity was reported as activity units/mg of total protein (1 unit = 1 µmole substrate oxidized/min). An extinction coefficient at 405 nm of 18.4 was used for ABTS and was equivalent to 0.5 mM oxidized substrate. The results of the activity assays are shown in the tables below.

| **FAO activity (units/mg total protein) on primary alcohols** | | | | | | | | |
|---|---|---|---|---|---|---|---|---|
| | 1-Butanol | 1-Pentanol | 1-Hexanol | 1-Octanol | 1-Decanol | 1-Dodecanol | 1-Tetradecanol | Hexadecanol |
| FAO-13 | 0.01 | 0.09 | 1.17 | 82.67 | 70.94 | 100 | 79.35 | 58.88 |
| FAO-17 | 0.72 | 0.26 | 1.06 | 66.23 | 22.00 | 100 | 47.86 | 60.98 |
| FAO-18 | 0.07 | 0.11 | 0.26 | 60.56 | 54.56 | 100 | 114.47 | 50.65 |
| FAO-20 | 0.07 | 0.11 | 0.91 | 55.96 | 74.57 | 100 | 89.52 | 42.59 |

| **FAO activity (units/mg total protein) on omega hydroxy fatty acids** | | | | | |
|---|---|---|---|---|---|
| | 1-Dodecanol | 6-OH-HA | 10-OH-DA | 12-OH-DDA | 16-OH-HDA |
| FAO-13 | 100 | 4.18 | 4.14 | 6.87 | 8.57 |
| FAO-17 | 100 | 1.18 | 0.00 | 0.59 | 0.94 |
| FAO-18 | 100 | 0.00 | 0.00 | 4.87 | 2.94 |
| FAO-20 | 100 | 0.03 | 0.04 | 2.25 | 7.46 |

### Example 19: Construction of C. tropicalis shuttle vector pAA061

Vector pAA061 was constructed from a pUC19 backbone to harbor the selectable marker URA3 from C. tropicalis strain ATCC20336 as well as modifications to allow insertion of C. tropicalis promoters and terminators. A 1,507bp DNA fragment containing the promoter, ORF, and terminator of URA3 from C. tropicalis ATCC20336 was amplified using primers oAA0124 and oAA0125, shown in the table below. The URA3 PCR product was digested with Ndel / Mlul and ligated into the 2,505bp fragment of pUC19 digested with Ndel / BsmBl (an Mlul compatible overhang was produced by BsmBl). In order to replace the lac promoter with a short 21 bp linker sequence, the resulting plasmid was digested with Sphl / Sapl and filled in with a linker produced by annealing oligos oAA0173 and oAA0174. The resulting plasmid was named pAA061, and is shown in Figure 30.

| **Oligonucleotides for construction of pAA061** | | |
|---|---|---|
| Oligos | Sequence | PCR product (bp) |
| oAA0124 | cacacacatatgCGACGGGTACAACGAGAATT | 1507 |
| oAA0125 | cacacaacgcgtAGACGAAGCCGTTCTTCAAG | |
| oAA0173 | ATGATCTGCCATGCCGAACTC | 21 (linker) |
| oAA0174 | AGCGAGTTCGGCATGGCAGATCATCATG | |

### Example 20: Cloning of C. tropicalis PGK promoter and terminator

Vector pAA105 was constructed from base vector pAA061 to include the phosphoglycerate kinase (PGK) promoter and terminator regions from C. tropicalis ATCC20336 with an intervening multiple cloning site (MCS) for insertion of open reading frames (ORF's). The PGK promoter region was amplified by PCR using primers oAA0347 and oAA0348, shown in the table below. The 1,029bp DNA fragment containing the PGK promoter was digested with restriction enzymes Pstl / Xmal. The PGK terminator region was amplified by PCR using primers oAA0351 and oAA0352, also shown in the table below. The 396bp DNA fragment containing the PGK terminator was digested with restriction enzymes Xmal / EcoRI. The 3,728bp Pstl / EcoRI DNA fragment from pAA061 was used in a three piece ligation reaction with the PGK promoter and terminator regions to produce pAA105. The sequence between the PGK promoter and terminator contains restriction sites for incorporating ORF's to be controlled by the functionally linked constitutive PGK promoter.

| **Oligonucleotides for cloning *C. tropicalis* PGK promoter and terminator** | | |
|---|---|---|
| Oligos | Sequence | PCR product (bp) |
| oAA0347 | CACACACTGCAGTTGTCCAATGTAATAATTTT | 1028 |
| oAA0348 | CACACATCTAGACCCGGGCTCTTCTTCTGAATAGGCAATTGATA AACTTACTTATC | |
| oAA0351 | GAGCCCGGGTCTAGATGTGTGCTCTTCCAAAGTACGGTGTTGTT GACA | 396 |
| oAA0352 | CACACACATATGAATTCTGTACTGGTAGAGCTAAATT | |

### Example 21: Cloning of the POX4 locus

Primers oAA0138 and oAA0141 (shown in the table below) were generated to amplify the entire sequence of NCBI accession number M12160 for the YSAPOX4 locus from genomic DNA prepared from C. tropicalis strain ATCC20336. The 2,845bp PCR product was cloned into the vector, pCR-Bluntll-TOPO (Invitrogen), sequenced and designated pAA052, and is shown in Figure 29.

| **Oligonucleotides for cloning of *POX4*** | | |
|---|---|---|
| Oligos | Sequence | PCR product (bp) |
| oAA0138 | GAGCTCCAATTGTAATATTTCGGG | 2845 |
| oAA0141 | GTCGACCTAAATTCGCAACTATCAA | |

### Example 22: Cloning of the POX5 locus

Primers oAA0179 and oAA0182 (shown in the table below) were generated to amplify the entire sequence of NCBI accession number M12161 for the YSAPOX5 locus from genomic DNA prepared from C. tropicalis strain ATCC20336. The 2,624bp PCR product was cloned into the vector, pCR-Bluntll-TOPO (Invitrogen), sequenced and designated pAA049, and is shown in Figure 28.

| **Oligonucleotides for cloning of *POX5*** | | |
|---|---|---|
| Oligos | Sequence | PCR product (bp) |
| oAA0179 | GAATTCACATGGCTAATTTGGCCTCGGTTCCACAACGCACTCAGC ATTAAAAA | 2624 |
| oAA0182 | GAGCTCCCCTGCAAACAGGGAAACACTTGTCATCTGATTT | |

### Example 23: Construction of strain sAA105 and sAA106

Functional POX4 alleles were restored in C. tropicalis strain sAA003 (ATCC20962; ura3/ura3, pox4::ura3/pox4::ura3, pox5::ura3/pox5::URA3) by transformation of sAA003 with POX4 linear DNA to replace the ura3-disrupted loci with functional alleles. A 2,845bp DNA fragment was amplified by PCR using primers oAA0138 and oAA0141 (described in Example 21) that contained the POX4 ORF as well as 531 bp upstream and 184bp downstream of the ORF, using plasmid pAA052 as template. The purified PCR product was used to transform competent sAA003 cells which were plated on YNB-agar plates supplemented with hexadecane vapor as the carbon source (e.g., by placing a filter paper soaked with hexadecane in the lid of the inverted petri dish) and incubated at 30°C for 4-5 days. Colonies growing on hexadecane as the sole carbon source were restreaked onto YPD-agar and incubated at 30°C. Single colonies were grown in YPD cultures and used for the preparation of genomic DNA.

PCR analysis of the genomic DNA prepared from the transformants was performed with oligos oAA0138 and oAA0141. An URA3-disrupted POX4 would produce a PCR product of 5,045bp, while a functional POX4 would produce a PCR product of 2,845bp. In strain sAA105 only one PCR product was amplified with a size of 2,845bp indicating that both POX4 alleles had been functionally restored. In strain sAA106 PCR products of both 2,845bp and 5,045bp were amplified indicating that one POX4 allele had been functionally restored while the other POX4 allele remained disrupted by URA3. The resultant strain genotypes were: sAA105 (ura3/ura3, POX4/POX4, pox5::ura3/pox5::URA3) and sAA106 (ura3/ura3, POX4/pox4::ura3, pox5::ura3/pox5::URA3).

### Example 24: Construction of strain sAA 152

Functional POX5 alleles were restored in C. tropicalis strain sAA103 (ura3/ura3, pox4::ura3/pox4::ura3, pox5::ura3/pox5::ura3) by transformation of sAA103 with Pmll-linearized plasmid pAA086 (containing the POX5 promoter, gene, terminator and a URA3 marker). Selection of transformants was performed by plating on SC-URA agar plates. Verification of plasmid integration was performed by PCR with primers oAA179 and oAA182 (described in Example 22). Integration of the plasmid was shown by a PCR product of 2,584bp indicating the presence of a functional POX5 allele. Other POX5 alleles in sAA152 were disrupted with an ura3 gene increasing the PCR product size for nonfunctional alleles to 4,734bp. Genotype for strain sAA152 is ura3/ura3, pox4::ura3/pox4::ura3, pox5::ura3/pox5::ura3, ura3::POX5,URA3).

### Example 25: Construction of strain sAA232

Functional POX5 alleles were restored in C. tropicalis strain sAA003 by transformation of sAA003 with POX5 linear DNA to replace the URA3-disrupted loci with a functional allele. A 2,584bp DNA fragment was amplified by PCR using primers oAA0179 and oAA0182 (described in Example 22) that contained the POX5 ORF as well as 456bp upstream and 179bp downstream of the ORF using plasmid pAA049 as template. The purified PCR product was used to transform competent sAA003 cells which were plated on SC+URA+5FOA plates and incubated at 30 °C for 3-4 days. Colonies were restreaked onto YPD-agar and incubated at 30 °C. Single colonies were grown in YPD cultures and used for the preparation of genomic DNA. PCR analysis of the genomic DNA prepared from the transformants was performed with oligos oAA0179 and oAA0182. An ura3-disrupted POX5 would produce a PCR product of 4,784bp while a functional POX5 would produce a PCR product of 2,584bp. In strain sAA232 PCR products of both 2,584bp and 4,784bp were amplified indicating that one POX5 allele had been functionally restored while the other POX5 allele remained disrupted by ura3. The resultant genotype of strain sAA232 is ura3/ura3, pox4::ura3/pox4::ura3, pox5::ura3/POX5. 5-FOA selection restored the POX5 allele that had been disrupted with the functional URA3 leaving the sAA232 strain Ura⁻.

### Example 26: Construction of strain sAA235

Functional POX5 alleles were restored in C. tropicalis strain sAA003 by transformation of sAA003 with POX5 linear DNA to replace the URA3-disrupted loci with a functional allele. A 2,584bp DNA fragment was amplified by PCR using primers oAA0179 and oAA0182 (described in Example 22) that contained the POX5 ORF as well as 456bp upstream and 179bp downstream of the ORF using plasmid pAA049 as template. The purified PCR product was used to transform competent sAA003 cells which were plated on YNB-agar plates supplemented with dodecane vapor as the carbon source (e.g., by placing a filter paper soaked with dodecane in the lid of the inverted petri dish) and incubated at 30 °C for 4-5 days. Colonies growing on dodecane as the sole carbon source were restreaked onto YPD-agar and incubated at 30 °C. Single colonies were grown in YPD cultures and used for the preparation of genomic DNA. PCR analysis of the genomic DNA prepared from the transformants was performed with oligos oAA0179 and oAA0182. An ura3-disrupted POX5 would produce a PCR product of 4,784bp while a functional POX5 would produce a PCR product of 2,584bp. In strain sAA235 a PCR product of 2,584bp was amplified indicating that both POX5 alleles had been functionally restored. An unintended consequence of the selection strategy (YNB-agar with dodecane) was that the cells reverted back to an Ura⁺ phenotype. Without being limited by any theory, it is believed the absence of uracil in the solid media and the replacement of the only functional URA3 forced the cells to mutate one of the other ura3 loci back to a functional allele. Therefore the genotype of the strain sAA235 is believed to be URA3/ura3, pox4::ura3/pox4::ura3, POX5/POX5. Verification of which of the loci is the functional URA3 is underway.

### Example 27: Construction of strains with amplified CPR and CYP52 genes

Strains having an increased number of copies of cytochrome P450 reductase (CPR) and/or for cytochrome P450 monooxygenase (CYP52) genes were constructed to determine how over expression of CPR and CYP52 affected diacid production.

### Cloning and integration of the CPR gene.

A 3,019bp DNA fragment encoding the CPR promoter, ORF, and terminator from C. tropicalis ATCC750 was amplified by PCR using primers oAA0171 and oAA0172 (see table below) incorporating unique Sapl and Sphl sites. The amplified DNA fragment was cut with the indicated restriction enzymes and ligated into plasmid pAA061, shown in Figure 30, to produce plasmid pAA067, shown in Figure. 32. Plasmid pAA067 was linearized with Clal and transformed into C. tropicalis Ura strain sAA103 (ura3/ura3, pox4::ura3/pox4::ura3, pox5::ura3/pox5::ura3). Transformations were performed with plasmid pAA067 alone and in combination with plasmids harboring the CYP52A15 or CYP52A16 genes, described below.

### Cloning and integration of CYP52A15 gene.

A 2,842bp DNA fragment encoding the CYP52A15 promoter, ORF, and terminator from C. tropicalis ATCC20336 was amplified by PCR using primers oAA0175 and oAA0178 (see table below) and cloned into pCR-Bluntll-TOPO for DNA sequence verification. The cloned CYP52A15 DNA fragment was isolated by restriction digest with Xbal / BamHl (2,742bp) and ligated into plasmid pAA061, shown in Figure 30, to produce plasmid pAA077, shown in Figure 33. Plasmid pAA077 was linearized with Pmll and transformed into C. tropicalis Ura⁻ strain sAA103 (ura3/ura3, pox4::ura3/pox4::ura3, pox5::ura3/pox5::ura3). pAA077 was cotransformed with plasmid pAA067 harboring the CPR gene.

### Cloning and integration of CYP52A16 gene.

A 2,728bp DNA fragment encoding the CYP52A16 promoter, ORF, and terminator from C. tropicalis ATCC20336 was amplified by PCR using primers oAA0177 and oAA0178 (see table below) and cloned into pCR-Bluntll-TOPO for DNA sequence verification. The cloned CYP52A16 DNA fragment was amplified with primers oAA0260 and oAA0261 (see table below) which incorporated unique Sacl / Xbal restriction sites. The amplified DNA fragment was digested with Sacl and Xbal restriction enzymes and ligated into plasmid pAA061 to produce plasmid pAA078, shown in Figure 34. Plasmid pAA078 was linearized with Clal and transformed into C. tropicalis Ura⁻ strain sAA103 (ura3/ura3, pox4::ura3/pox4::ura3, pox5::ura3/pox5::ura3). pAA078 was cotransformed with plasmid pAA067 harboring the CPR gene.

| **Oligonucleotides for cloning of CPR, CYP52A15 and CYP52A16** | | |
|---|---|---|
| Oligos | Sequence | PCR product (bp) |
| oAA0171 | cacctcgctcttccAGCTGTCATGTCTATTCAATGCTTCGA | 3019 |
| oAA0172 | cacacagcatgcTAATGTTTATATCGTTGACGGTGAAA | |
| oAA0175 | cacaaagcggaagagcAAATTTTGTATTCTCAGTAGGATTT CATC | 2842 |
| oAA0178 | cacacagcatgCAAACTTAAGGGTGTTGTAGATATCCC | |
| oAA0177 | cacacacccgggATCGACAGTCGATTACGTAATCCATATT ATTT | 2772 |
| oAA0178 | cacacagcatgCAAACTTAAGGGTGTTGTAGATATCCC | |
| oAA0260 | cacacagagctcACAGTCGATTACGTAATCCAT | 2772 |
| oAA0261 | cacatctagaGCATGCAAACTTAAGGGTGTTGTA | |

### Preparation of genomic DNA.

Genomic DNA was prepared from transformants for PCR verification and for Southern blot analysis. Isolated colonies were inoculated into 3 mL YPD and grown overnight at 30 °C with shaking. Cells were pelleted by centrifugation. To each pellet, 200 uL Breaking Buffer (2% Triton X-100, 1% SDS, 100 mM NaCl, 10 mM Tris pH 8 and, 1 mM EDTA) was added, and the pellet resuspended and transferred to a fresh tube containing 200 uL 0.5 mm Zirconia/Silica Beads. 200 uL Phenol:Chloroform:Isoamyl Alcohol (25:24:1) was added to each tube, followed by vortexing for 1 minute. Sterile distilled water was added (200 uL) to each tube and the tubes were centrifuged at 13000 rpm for 10 minutes. The aqueous layer was ethanol precipitated and washed with 70% ethanol. The pellet was resuspended in 100-200 µl 10 mM Tris, after drying. Genomic DNA preparation for southern blot analysis was performed using the same procedure on 25 mL cultures for each colony tested.

### Characterization of strains with amplified CPR and CYP52 genes.

Verification of integrants was performed by PCR using primers oAA0252 and oAA0256 (CPR), oAA0231 and oAA0281 (CYP52A15), and oAA242 and oAA0257 (CYP52A16). The primers used for verification are shown in the table below.

| **Oligonucleotides for PCR verification of *CPR, CYP52A15 and CYP52A16*** | | |
|---|---|---|
| Oligos | Sequence | PCR product (bp) |
| oAA0252 | TTAATGCCTTCTCAAGACAA | 743 |
| oAA0256 | GGTTTTCCCAGTCACGACGT | |
| oAA0231 | CCTTGCTAATTTTCTTCTGTATAGC | 584 |
| oAA0281 | TTCTCGTTGTACCCGTCGCA | |
| oAA0242 | CACACAACTTCAGAGTTGCC | 974 |
| oAA0257 | TCGCCACCTCTGACTTGAGC | |

Southern blot analysis was used to determine the copy number of the CPR, CYP52A15 and CYP52A15 genes. Biotinylated DNA probes were prepared with gene specific oligonucleotides using the NEBlot Phototope Kit from New England BioLabs (Catalog #N7550S) on PCR products generated from each gene target as specified in the table below. Southern Hybridizations were performed using standard methods (e.g., Sambrook, J. and Russell, D. W. (2001) Molecular Cloning: A Laboratory Manual, (3rd ed.), pp. 6.33-6.64. Cold Spring Harbor Laboratory Press). Detection of hybridized probe was performed using the Phototope-Star Detection Kit from New England BioLabs (Catalog #N7020S). Copy number was determined by densitometry of the resulting bands.

| **Oligonucleotides for Probe Template PCR of *CPR, CYP52A15 and CYP52A16*** | | | | |
|---|---|---|---|---|
| Oligos | Sequence | Gene | Template | PCR product (bp) |
| oAA0250 | AATTGAACATCAGAAGAGGA | CPR | pAA067 | 1313 |
| oAA0254 | CCTGAAATTTCCAAATGGTGTCTAA | | | |
| oAA0227 | TTTTTTGTGCGCAAGTACAC | CYP52A15 | pAA077 | 905 |
| oAA0235 | CAACTTGACGTGAGAAACCT | | | |
| oAA0239 | AGATGCTCGTTTTACACCCT | CYP52A16 | pAA078 | 672 |
| oAA0247 | ACACAGCTTTGATGTTCTCT | | | |

### Example 28: Strain evaluation of partially β-oxidation blocked strains

### Fermentation of methyl laurate feedstock.

5mL starter cultures, in SP92 media (6.7g/L Difco yeast nitrogen base, 3.0g/L Difco yeast extract, 3.0g/L ammonium sulfate, 1.0g/L potassium phosphate monobasic, 1.0g/L potassium phosphate dibasic, 75g/L dextrose) were incubated overnight at 30 °C, with shaking and used to inoculate flasks containing 25mL of SP92 media to an initial OD₆₀₀ₙₘ of about 0.4. Cultures were incubated approximately 18 hours at 30 °C, with shaking at about 200rpm. Cells were pelleted by centrifugation at 4 °C for 10 minutes at 4,000 x *g*, then resuspended in SP92-D media (6.7g/L Difco yeast nitrogen base, 3.0g/L Difco yeast extract, 3.0g/L ammonium sulfate, 1.0g/L potassium phosphate monobasic, 1.0g/L potassium phosphate dibasic) supplemented with 0.1 % dextrose and 2% methyl laurate. Incubation of the cultures continued at 30 °C, with shaking and samples were taken for analysis of fatty acids and diacids by gas chromatography (GC).

Sample for GC were prepared by adding 0.8mL of 6.0M HCl to 1 mL of whole culture samples and the samples were stored at 4 °C to await processing. Samples were processed by incubating in a 60 °C water bath for 5 minutes, after which 4.0 mL of MTBE was added to the 1.8 mL acidified whole culture samples and vortexed for 20 seconds. The phases were allowed to separate for 10 min at room temperature. 1 mL of the MTBE phase was drawn and dried with sodium sulfate. Aliquots of the MTBE phase were derivatized with BSTFA reagent (Regis Technologies Inc.) and analyzed by GC equipped with a Flame Ionization Detector. The results of the gas chromatography are shown in the table below.

| **Fatty acid and Diacid profile (g/L) in Methyl Laurate fermentation** | | | | | | |
|---|---|---|---|---|---|---|
| **Strain** | **Time (h)** | **C12 Acid** | **C12 Diacid** | **C10 Diacid** | **C8 Diacid** | **C6 Diacid** |
| sAA105 | 0 | 0.00 | 0.00 | 0.00 | 0.00 | 0.00 |
| | 24 | 0.05 | 0.00 | 0.00 | 0.07 | 0.42 |
| | 48 | 0.00 | 0.00 | 0.00 | 0.00 | 0.00 |
| sAA106 | 0 | 0.00 | 0.00 | 0.00 | 0.00 | 0.00 |
| | 24 | 2.92 | 1.29 | 0.15 | 0.58 | 0.37 |
| | 48 | 0.04 | 0.02 | 0.00 | 0.00 | 0.01 |
| sAA152 | 0 | 0.02 | 0.00 | 0.00 | 0.00 | 0.00 |
| | 24 | 0.58 | 0.55 | 0.07 | 0.43 | 0.03 |
| | 48 | 0.00 | 0.03 | 0.00 | 0.05 | 0.58 |
| sAA003 | 0 | 0.00 | 0.00 | 0.00 | 0.00 | 0.00 |
| | 24 | 1.96 | 0.41 | 0.00 | 0.00 | 0.00 |
| | 48 | 1.43 | 0.47 | 0.00 | 0.00 | 0.00 |

### Fermentation of methyl myristate and oleic acid feedstocks.

Fermentations were performed essentially as described for methyl laurate feedstock except that 2% methyl myristate or 2% oleic acid was substituted for the 2% methyl laurate. The results of the gas chromatography are shown in the tables below.

| **Fatty acid and Diacid profile (g/L) in Methyl Myristate fermentation** | | | | | | | |
|---|---|---|---|---|---|---|---|
| **Strain** | **Time (h)** | **C14 Acid** | **C14 Diacid** | **C12 Diacid** | **C10 Diacid** | **C8 Diacid** | **C6 Diacid** |
| sAA105 | 0 | 0.02 | 0.00 | 0.00 | 0.00 | 0.00 | 0.00 |
| | 24 | 0.02 | 0.00 | 0.00 | 0.00 | 0.00 | 0.29 |
| | 48 | 0.01 | 0.00 | 0.00 | 0.00 | 0.00 | 0.03 |
| sAA106 | 0 | 0.01 | 0.00 | 0.00 | 0.00 | 0.00 | 0.00 |
| | 24 | 0.02 | 0.00 | 0.00 | 0.00 | 0.08 | 1.71 |
| | 48 | 0.01 | 0.00 | 0.00 | 0.00 | 0.00 | 0.04 |
| sAA232 | 0 | 0.01 | 0.00 | 0.00 | 0.00 | 0.00 | 0.00 |
| | 24 | 0.01 | 0.00 | 0.00 | 0.00 | 0.59 | 0.26 |
| | 48 | 0.01 | 0.00 | 0.00 | 0.00 | 0.35 | 0.47 |
| sAA235 | 0 | 0.01 | 0.00 | 0.00 | 0.00 | 0.00 | 0.00 |
| | 24 | 0.02 | 0.00 | 0.00 | 0.00 | 0.25 | 0.38 |
| | 48 | 0.01 | 0.00 | 0.00 | 0.00 | 0.04 | 0.66 |
| sAA003 | 0 | 0.02 | 0.01 | 0.01 | 0.00 | 0.00 | 0.00 |
| | 24 | 0.55 | 0.25 | 0.00 | 0.00 | 0.00 | 0.00 |
| | 48 | 0.49 | 0.38 | 0.00 | 0.00 | 0.00 | 0.00 |

| **Diacid profile (g/L) in Oleic acid fermentation** | | | | | | |
|---|---|---|---|---|---|---|
| **Strain** | **Time (h)** | **C14 Diacid** | **C12 Diacid** | **C10 Diacid** | **C8 Diacid** | **C6 Diacid** |
| sAA105 | 0 | 0.00 | 0.00 | 0.00 | 0.00 | 0.00 |
| | 24 | 0.00 | 0.00 | 0.00 | 0.00 | 0.03 |
| | 48 | 0.00 | 0.00 | 0.00 | 0.00 | 0.01 |
| sAA106 | 0 | 0.00 | 0.00 | 0.00 | 0.00 | 0.00 |
| | 24 | 0.00 | 0.00 | 0.00 | 0.00 | 1.48 |
| | 48 | 0.00 | 0.00 | 0.00 | 0.00 | 0.42 |
| sAA232 | 0 | 0.00 | 0.00 | 0.00 | 0.00 | 0.00 |
| | 24 | 0.00 | 0.00 | 0.00 | 0.00 | 0.09 |
| | 48 | 0.00 | 0.00 | 0.00 | 0.00 | 0.10 |
| sAA235 | 0 | 0.00 | 0.00 | 0.00 | 0.00 | 0.00 |
| | 24 | 0.00 | 0.00 | 0.00 | 0.00 | 0.10 |
| | 48 | 0.00 | 0.00 | 0.00 | 0.00 | 0.11 |

Fermentations also were performed using coconut oil as a feed stock. Coconut oil contains a mixture of fatty acids of different carbon chain lengths. The percent composition of fatty acids, by weight, is about 6% capric acid (C10:0, where 0 refers to the number of double or unsaturated bonds), about 47% lauric acid (C12:0), about 18% myristic acid (C14:0), about 9% palmitic acid (C16:0). About 3% stearic acid (C18:0), about 6% oleic acid (C18:1, where 1 refers to the number of double bonds), and about 2% linoleic acid (omega-6 fatty acid, C18:2). In some embodiments, palm kernel oil can be substituted for coconut oil. Palm kernel oil has a distribution of fatty acids similar to that of coconut oil. Fermentations and GC were carried out essentially as described herein with the exception of feedstock used. The result of fermentations performed using coconut oil as a feedstock are presented below.

| **Diacid profile (g/L) in Coconut Oil fermentation** | | | | | | |
|---|---|---|---|---|---|---|
| **Strain** | **Time (h)** | **C14 Diacid** | **C12 Diacid** | **C10 Diacid** | **C8 Diacid** | **C6 Diacid** |
| sAA105 | 0 | 0.00 | 0.00 | 0.00 | 0.00 | 0.00 |
| | 24 | 0.00 | 0.00 | 0.00 | 0.00 | 0.00 |
| | 48 | 0.00 | 0.00 | 0.00 | 0.00 | 0.00 |
| sAA106 | 0 | 0.00 | 0.00 | 0.00 | 0.00 | 0.00 |
| | 24 | 0.00 | 0.00 | 0.00 | 0.00 | 0.01 |
| | 48 | 0.00 | 0.00 | 0.00 | 0.00 | 0.00 |
| sAA152 | 0 | 0.00 | 0.00 | 0.00 | 0.00 | 0.00 |
| | 24 | 0.00 | 0.00 | 0.00 | 0.00 | 0.43 |
| | 48 | 0.00 | 0.00 | 0.00 | 0.00 | 0.45 |
| sAA232 | 0 | 0.00 | 0.00 | 0.00 | 0.00 | 0.00 |
| | 24 | 0.00 | 0.00 | 0.00 | 0.00 | 0.41 |
| | 48 | 0.00 | 0.00 | 0.00 | 0.00 | 0.58 |
| sAA235 | 0 | 0.00 | 0.00 | 0.00 | 0.00 | 0.00 |
| | 24 | 0.00 | 0.00 | 0.00 | 0.00 | 0.43 |
| | 48 | 0.00 | 0.00 | 0.00 | 0.00 | 0.76 |

### Example 29: Strain evaluation of completely β-oxidation blocked strains

Fermentations also were performed using methyl myristate as a feed stock. Fermentations and GC were carried out essentially as described herein with the exception of feedstock used. The result of fermentations performed using coconut oil as a feedstock are presented below.

| **C14 Diacid production in strains with amplified CPR, CYP52A15, and/or CYP52A16** | | | | |
|---|---|---|---|---|
| Strain | C14 diacid, 72h (g/L) | CPR | A15 | A16 |
| sAA003 | 0.98 | 2 | 1 | 1 |
| sAA318 | 1.19 | 3 | 1 | 1 |
| sAA239 | 2.75 | 3 | 1 | 3 |
| sAA319 | 1.37 | 7 | 1 | 1 |
| sAA238 | 1.93 | 7 | 2 | 1 |

### Example 30: Nucleic acid and amino acid sequences of novel fatty alcohol oxidase genes

As noted above, novel fatty alcohol oxidase genes were identified and cloned. The nucleotide and amino acid sequences of the novel sequences are presented herein. Nucleotide and amino acid sequence identity comparison are shown in Example 18.

### Nucleotide Sequences

FAO-13 (SEQ ID NO:1) 
atggctccatttttgcccgaccaggtcgactacaaacacgtcgacacccttatgttattatgtgacggatcatccacga
aaccaccgtcgaccaaatcaaagacgttattgctcctgacttccctgctgacaagtacgaagagtacgtcaggacattca
ccaaaccctccgaaaccccagggttcagggaaaccgtctacaacacagtcaacgcaaacaccacggacgcaatccaccag
ttcattatcttgaccaatgttttggcatccagggtcttggctccagctttgaccaactcgttgacgcctatcaaggacat
gagcttggaagaccgtgaaaaattgttggcctcgtggcgcgactccccaatcgctgccaaaaggaagttgttcaggttgg
tttctacgcttaccttggtcacgttcacgagattggccaatgagttgcatttgaaagccattcattatccaggaagagaa
gaccgtgaaaaggcttatgaaacccaggagattgaccctttaagtaccagttttggaaaaaccgaagttttacggcgc
tgagttgtacttgccagatattgatgtgatcattattggatctggtgccggtgctggtgttgtggcccacactttggcca
acgatggcttcaagagtttggttttggaaaagggcaaatactttagcaactccgagttgaactttgatgacaaggacggc
gttcaagaattataccaaagtggaggtactttgactacagtcaaccaacagttgtttgttcttgctggttccacttttgg
tggcggtaccactgtcaattggtcagcctgtcttaagacgccattcaaggtgcgtaaggaatggtatgatgagtttggtg
ttgactttgctgctgatgaagcatacgataaagcgcaggattatgtttggcagcaaatgggagcttctaccgaaggcatc
acccactctttggctaacgagattattattgaaggtggtaagaaattaggttacaaggccaaggtattagaccaaaacag
cggtggtcatcctcagcacagatgcggtttctgttatttgggctgtaagcacggtatcaagcagggttctgttaataact
ggtttagagacgcagctgcccacggttcccagttcatgcaacaggttagagttttgcaaatacttaacaagaaggggatc
gcttacggtatcttgtgtgaggatgttgtaaccggcgccaagttcaccattactggccccaaaaagtttgttgttgctgc
cggtgctttgaacactccatctgtgttggtcaactccggcttcaagaacaagaacatcggtaagaacttaactttgcacc
cagtttctgtcgtgtttggtgatttggcaaagacgttcaagcagaccacttccacaactccatcatgactgccctttgt
tcagaagccgctgatttagacggcaagggccatggatgcagaattgaaaccatcttgaacgctccattcatccaggcttc
attcttaccatggagaggtagtaacgaggctagacgagacttgttgcgttacaacaacatggtggcgatgttgctcctta
gtcgtgacaccaccagtggttccgtttctgctcatccaaccaaacctgaagctttggttgtcgagtacgacgtgaacaag
tttgacagaaactcgatcttgcaggcattgttggtcactgctgacttgttgtatatccaaggtgccaagagaatccttag
tccacaggcatgggtgccaattttgaatccgacaagccaaaggataagagatcaatcaaggacgaggactatgtcgaat
ggagagccaaggttgccaagattcctttcgacacctacggctcaccttatggttcggcacatcaaatgtcttcttgccgt
atgtcaggtaagggtcctaaatacggtgctgttgacaccgatggtagattgtttgaatgttcgaatgtttatgttgccga
tgcaagtcttttgccaactgcaagcggtgccaaccctatggtcaccaccatgactcttgccagacatgttgcgttaggtt
tggcagactccttgaagaccaaagccaagttgtag
FAO-17 (SEQ ID NO:2) 
atggctccatttttgcccgaccaggtcgactacaaacacgtcgacacccttatgttattatgtgacggatcatccacga
aaccaccgtggacgaaatcaaagacgtcattgcccctgacttccccgccgacaaatacgaggagtacgtcaggacattca
ccaaaccctccgaaaccccagggttcagggaaaccgtctacaacaccgtcaacgcaaacaccatggatgcaatccaccag
ttcattatcttgaccaatgttttgggatcaagggtcttggcaccagctttgaccaactcgttgactcctatcaaggacat
 
gagcttggaagaccgtgaaaagttgttagcctcgtggcgtgactcccctattgctgctaaaaggaagttgttcaggttgg
tttctacgcttaccttggtcacgttcacgagattggccaatgagttgcatttgaaagccattcattatccaggaagagaa
gaccgtgaaaaggcttatgaaacccaggagattgacccttttaagtaccagttttggaaaaaccgaagttttacggcgc
tgagttgtacttgccagatattgatgtgatcattattggatctggtgccggtgctggtgttgtggcccacactttggcca
acgatggcttcaagagtttggttttggaaaagggcaaatactttagcaactccgagttgaactttgatgacaaggacggc
gttcaagaattataccaaagtggaggtactttgactacagtcaaccaacagttgtttgttcttgctggttccactttgg
tggcggtaccactgtcaattggtcagcctgtcttaagacgccattcaaggtgcgtaaggaatggtatgatgagtttggtg
ttgactttgctgctgatgaagcatacgataaagcgcaggattatgtttggcagcaaatggagcttctaccgaaggcatc
acccactctttggctaacgagattattattgaaggtggtaagaaattaggttacaaggccaaggtattagaccaaaacag
cggtggtcatcctcagcacagatgcggtttctgttatttgggttgtaagcacggtatcaagcagggctctgttaataact
ggtttagagacgcagctgcccacggttctcagttcatgcaacaggttagagttttgcaaatccttaacaagaagggcatc
gcttatggtatcttgtgtgaggatgttgtaaccggtgccaagttcaccattactggcccaaaaagtttgttgttgccgc
cggcgccttaaacactccatctgtgttggtcaactccggattcaagaacaagaacatcggtaagaacttaactttgcatc
cagtttctgtcgtgtttggtgatttggcaaagacgttcaagcagaccacttccacaactccatcatgactgccctttgt
tcagaagccgctgatttagacggcaagggccatggatgcagaattgaaaccatcttgaacgctccattcatccaggcttc
attcttaccatggagaggtagtaacgaggctagacgagacttgttgcgttacaacaacatggtggcgatgttgctcctta
gtcgtgacaccaccagtggttccgtttctgctcatccaaccaaacctgaagctttggttgtcgagtacgacgtgaacaag
tttgacagaaactcgatcttgcaggcattgttggtcactgctgacttgttgtatatccaaggtgccaagagaatccttag
tccacaggcatgggtgccaattttgaatccgacaagccaaaggataagagatcaatcaaggacgaggactatgtcgaat
ggagagccaaggttgccaagattcctttcgacacctacggctcaccttatggttcggcacatcaaatgtcttcttgccgt
atgtcaggtaagggtcctaaatacggtgctgttgacaccgatggtagattgtttgaatgttcgaatgtttatgttgccga
tgcaagtcttttgccaactgcaagcggtgccaaccctatggtcaccaccatgactcttgcaagacatgttgcgttaggtt
tggcagactccttgaagaccaaggccaagttgtag
FAO-20 (SEQ ID NO:3) 
atggctccatttttgcccgaccaggtcgactacaaacacgtcgacacccttatgttattatgtgacggatcatccacga
aaccaccgtcgaccaaatcaaagacgttattgctcctgacttccctgctgacaagtacgaagagtacgtcaggacattca
ccaaaccctccgaaaccccagggttcagggaaaccgtctacaacacagtcaacgcaaacaccacggacgcaatccaccag
ttcattatcttgaccaatgttttggcatccagggtcttggctccagctttgaccaactcgttgacgcctatcaaggacat
gagcttggaagaccgtgaaaaattgttggcctcgtggcgcgactccccaatcgctgccaaaaggaaattgttcaggttgg
tttccacgcttaccttggttactttcacgagattggccaatgagttgcatttgaaagccattcactatccaggaagagaa
gaccgtgaaaaggcttatgaaacccaggagattgaccctttcaagtaccagtttatggaaaagccaaagtttgacggcgc
tgagttgtacttgccagatattgatgttatcattattggatctggtgccggtgctggtgttgtggcccacactttggcca
acgatggcttcaagagtttggttttggaaaagggcaaatactttagcaactccgagttgaactttgatgacaaggacggc
gttcaagaattataccaaagtggaggtactttgactacagtcaaccaacagttgtttgttcttgctggttccacttttgg
tggcggtaccactgtcaattggtcagcctgtcttaagacgccattcaaggtgcgtaaggaatggtatgatgagtttggtg
ttgactttgctgctgatgaagcatacgataaagcgcaggattatgtttggcagcaaatgggagcttctaccgaaggcatc
acccactctttggctaacgagattattattgaaggtggtaagaaattaggttacaaggccaaggtattagaccaaaacag
cggtggtcatcctcagcacagatgcggtttctgttatttgggctgtaagcacggtatcaagcagggttctgttaataact
ggtttagagacgcagctgcccacggttcccagttcatgcaacaggttagagttttgcaaatacttaacaagaaggggatc
gcttacggtatcttgtgtgaggatgttgtaaccggcgccaagttcaccattactggccccaaaaagtttgttgttgctgc
cggtgctttgaacactccatctgtgttggtcaactccggcttcaagaacaagaacatcggtaagaacttaactttgcacc
cagtttctgtcgtgtttggtgatttggcaaagacgttcaagcagaccacttccacaactccatcatgactgccctttgt
tcagaagccgctgatttagacggcaagggccatggatgcagaattgaaaccatcttgaacgctccattcatccaggcttc
attcttaccatggagaggtagtaacgaggctagacgagacttgttgcgttacaacaacatggtggcgatgttgctcctta
gtcgtgacaccaccagtggttccgtttctgctcatccaaccaaacctgaagctttggttgtcgagtacgacgtgaacaag
tttgacagaaactcgatcttgcaggcattgttggtcactgctgacttgttgtatatccaaggtgccaagagaatccttag
tccacaggcatgggtgccaattttgaatccgacaagccaaaggataagagatcaatcaaggacgaggactatgtcgaat
ggagagccaaggttgccaagattcctttcgacacctacggctcaccttatggttcggcacatcaaatgtcttcttgccgt
atgtcaggtaagggtcctaaatacggtgctgttgacaccgatggtagattgtttgaatgttcgaatgtttatgttgccga
tgcaagtcttttgccaactgcaagcggtgccaaccctatggtcaccaccatgactcttgccagacatgttgcgttaggtt
tggcagactccttgaagaccaaagccaagttgtag
FAO2a (SEQ ID NO:4) 
atgaataccttcttgccagacgtgctcgaatacaaacacgtcgacaccctttttgttattgtgtgacggatcatccacga
aaccacagtcgatcagatcaaggacgccattgctcccgacttccctgaggaccagtacgaggagtatctcaagaccttca
ccaagccatctgagacccctgggttcagagaagccgtctacgacacgatcaacgccaccccaaccgatgccgtgcacatg
tgtattgtcttgaccaccgcattggactccagaatcttggcccccacgttgaccaactcgttgacgcctatcaaggatat
gaccttgaaggagcgtgaacaattgttggcctcttggcgtgattccccgattgcggcaaagagaagattgttcagattga
tttcctcgcttaccttgacgacgtttacgagattggccagcgaattgcacttgaaagccatccactaccctggcagagac
ttgcgtgaaaaggcgtatgaaacccaggtggttgaccctttcaggtacctgtttatggagaaaccaaagtttgacggcgc
cgaattgtacttgccagatatcgacgtcatcatcattggatcaggcgccggtgctggtgtcatggcccacactctcgcca
acgacgggttcaagaccttggttttggaaaagggaaagtatttcagcaactccgagttgaactttaatgacgctgatggc
 
gtgaaagagttgtaccaaggtaaaggtgctttggccaccaccaatcagcagatgtttattcttgccggttccactttggg
cggtggtaccactgtcaactggtctgcttgccttaaaacaccatttaaagtgcgtaaggagtggtacgacgagtttggtc
ttgaatttgctgccgatgaagcctacgacaaagcgcaggattatgtttggaaacaaatgggtgcttcaacagatggaatc
actcactccttggccaacgaagttgtggttgaaggaggtaagaagttgggctacaagagcaaggaaattgagcagaacaa
cggtggccaccctgaccacccatgtggtttctgttacttgggctgtaagtacggtattaaacagggttctgtgaataact
ggtttagagacgcagctgcccacgggtccaagttcatgcaacaagtcagagttgtgcaaatcctcaacaagaatggcgtc
gcttatggtatcttgtgtgaggatgtcgaaaccggagtcaggttcactattagtggccccaaaaagtttgttgtttctgc
tggttctttgaacacgccaactgtgttgaccaactccggattcaagaacaagcacattggtaagaacttgacgttgcacc
cagtttccaccgtgtttggtgactttggcagagacgtgcaagccgaccatttccacaaatctattatgacttcgctttgt
tacgaggttgctgacttggacggcaagggccacggatgcagaatcgaaaccatcttgaacgctccattcatccaagcttc
tttgttgccatggagaggaagtgacgaggtcagaagagacttgttgcgttacaacaacatggtggccatgttgcttatca
cgcgtgataccaccagtggttcagtttctgctgacccaaagaagcccgacgctttgattgtcgactatgagattaacaag
tttgacaagaatgccatcttgcaagctttcttgatcacttccgacatgttgtacattgaaggtgccaagagaatcctcag
tccacagccatgggtgccaatctttgagtcgaacaagccaaaggagcaaagaacgatcaaggacaaggactatgttgagt
ggagagccaaggctgctaagatacctttcgacacctacggttctgcatatgggtccgcacatcaaatgtccacctgtcgt
atgtccggaaagggtcctaaatacggtgctgttgatactgatggtagattgtttgaatgttcgaatgtctatgttgctga
tgctagtgttttgcctactgccagcggtgccaacccaatgatatccaccatgacctttgctagacagattgcgttaggtt
tggctgactccttgaagaccaaacccaagttgtag
FAO2b (SEQ ID NO:5)

atgaataccttcttgccagacgtgctcgaatacaaacacgtcgatacccttttgttattatgtgacggatcatccacga
aaccacagtcgaccagatcagggacgccattgctcccgacttccctgaagaccagtacgaggagtatctcaagaccttca
ccaagccatctgagacccctgggttcagagaagccgtctacgacacgatcaacagcaccccaaccgaggctgtgcacatg
tgtattgtattgaccaccgcattggactcgagaatcttggcccccacgttgaccaactcgttgacgcctatcaaggatat
gaccttgaaagagcgtgaacaattgttggctgcctggcgtgattccccgatcgcggccaagagaagattgttcagattga
tttcctcacttaccttgacgacctttacgagattggccagcgacttgcacttgagagccatccactaccctggcagagac
ttgcgtgaaaaggcatatgaaacccaggtggttgaccctttcaggtacctgtttatggaaaaaccaaagtttgacggcac
cgagttgtacttgccagatatcgacgtcatcatcattggatccggtgccggtgctggtgtcatggcccacactttagcca
acgacgggtacaagaccttggttttggaaaagggaaagtatttcagcaactccgagttgaactttaatgatgccgatggt
atgaaagagttgtaccaaggtaaatgtgcgttgaccaccacgaaccagcagatgtttattcttgccggttccactttggg
cggtggtaccactgttaactggtctgcttgtcttaaaacaccatttaaagtgcgtaaggagtggtacgacgagtttggtc
ttgaatttgctgccgacgaagcctacgacaaagcacaagactatgtttggaaacaaatggcgcttctaccgaaggaatc
actcactctttggcgaacgcggttgtggttgaaggaggtaagaagttgggttacaagagcaaggaaatcgagcagaacaa
tggtggccatcctgaccacccctgtggtttctgttacttgggctgtaagtacggtattaagcagggttctgtgaataact
ggtttagagacgcagctgcccacgggtccaagttcatgcaacaagtcagagttgtgcaaatcctccacaataaaggcgtc
gcttatggcatcttgtgtgaggatgtcgagaccggagtcaaattcactatcagtggcccccaaaaagtttgttgtttctgc
aggttctttgaacacgccaacggtgttgaccaactccggattcaagaacaaacacatcggtaagaacttgacgttgcacc
cagtttcgaccgtgtttggtgactttggcagagacgtgcaagccgaccatttccacaaatctattatgacttcgctctgt
tacgaagtcgctgacttggacggcaagggccacggatgcagaatcgagaccatcttgaacgctccattcatccaagcttc
tttgttgccatggagaggaagcgacgaggtcagaagagacttgttgcgttacaacaacatggtggccatgttgcttatca
cccgtgacaccaccagtggttcagtttctgctgacccaaagaagcccgacgctttgattgtcgactatgacatcaacaag
tttgacaagaatgccatcttgcaagctttcttgatcacctccgacatgttgtacatcgaaggtgccaagagaatcctcag
tccacaggcatgggtgccaatctttgagtcgaacaagccaaaggagcaaagaacaatcaaggacaaggactatgtcgaat
ggagagccaaggctgccaagatacctttcgacacctacggttctgcctatgggtccgcacatcaaatgtccacctgtcgt
atgtccggaaagggtcctaaatacggcgccgttgataccgatggtagattgtttgaatgttcgaatgtctatgttgctga
tgctagtgttttgcctactgccagcggtgccaacccaatgatctccaccatgacgtttgctagacagattgcgttaggtt
tggctgactctttgaagaccaaacccaagttgtag

In addition to the novel FAO genes isolated, a sequence substantially identical to the sequence used for primer design, described above, also was isolated. The nucleotide sequence of the gene is presented below.
FAO-18 (SEQ ID NO: 6)

atggctccatttttgcccgaccaggtcgactacaaacacgtcgacacccttatgttattatgtgacggatcatccacga
aaccaccgtggacgaaatcaaagacgtcattgcccctgacttccccgccgacaaatacgaggagtacgtcaggacattca
ccaaaccctccgaaaccccagggttcagggaaaccgtctacaacaccgtcaacgcaaacaccatggatgcaatccaccag
ttcattatcttgaccaatgttttgggatcaagggtcttggcaccagctttgaccaactcgttgactcctatcaaggacat
gagcttggaagaccgtgaaaagttgttagcctcgtggcgtgactcccctattgctgctaaaaggaagttgttcaggttgg

tttctacgcttaccttggtcacgttcacgagattggccaatgagttgcatttgaaagccattcattatccaggaagagaa
gaccgtgaaaaggcttatgaaacccaggagattgacccttttaagtaccagtttttggaaaaaccgaagttttacggcgc
tgagttgtacttgccagatattgatgtgatcattattggatctggggccggtgctggtgtcgtggcccacactttgacca
acgacggcttcaagagtttggttttggaaaagggcagatactttagcaactccgagttgaactttgatgacaaggacggg
gttcaagaattataccaaagtggaggtactttgaccaccgtcaaccagcagttgtttgttcttgctggttccacttttgg
tggtggtaccactgtcaattggtcggcctgtcttaaaacgccattcaaggtgcgtaaggaatggtatgatgagtttggcg
ttgactttgctgccgatgaagcctacgacaaagcacaggattatgtttggcagcaaatgggagcttctaccgaaggcatc
acccactctttggctaacgagattattattgaaggtggcaagaaattaggttacaaggccaaggtattagaccaaaacag
cggtggtcatcctcatcacagatgcggtttctgttatttgggttgtaagcacggtatcaagcagggctctgttaataact
ggtttagagacgcagctgcccacggttctcagttcatgcaacaggttagagttttgcaaatccttaacaagaaggcatc
gcttatggtatcttgtgtgaggatgttgtaaccggtgccaagttcaccattactggccccaaaaagtttgttgttgccgc
cggcgccttaaacactccatctgtgttggtcaactccggattcaagaacaagaacatcggtaagaacttaactttgcatc
cagtttctgtcgtgtttggtgatttggcaaagacgttcaagcagatcacttccacaactccatcatgactgctctttgt
tcagaagccgctgatttagacggcaagggtcatggatgcagaattgaaaccatcttgaacgctccattcatccaggcttc
attcttaccatggagaggtagtaacgaggctagacgagacttgttgcgttacaacaacatggtggccatgttacttctta
gtcgtgataccaccagtggttccgtttcgtcccatccaactaaacctgaagcattagttgtcgagtacgacgtgaacaag
tttgacagaaactccatcttgcaggcattgttggtcactgctgacttgttgtacattcaaggtgccaagagaatccttag
tccccaaccatgggtgccaatttttgaatccgacaagccaaaggataagagatcaatcaaggacgaggactatgtcgaat
ggagagccaaggttgccaagattccttttgacacctacggctcgccttatggttcggcgcatcaaatgtcttcttgtcgt
atgtcaggtaagggtcctaaatacggtgctgttgataccgatggtagattgtttgaatgttcgaatgtttatgttgctga
cgctagtcttttgccaactgctagcggtgctaatcctatggtcaccaccatgactcttgcaagacatgttgcgttaggtt
tggcagactccttgaagaccaaggccaagttgtag

### Clustal nucleotide sequence alignments

CLUSTAL 2.0.12 multiple sequence alignment

FAO-13 ATGGCTCCATTTTTGCCCGACCAGGTCGACTACAAACACGTCGACACCCTTATGTTATTA 60
FAO-20 ATGGCTCCATTTTTGCCCGACCAGGTCGACTACAAACACGTCGACACCCTTATGTTATTA 60
FAO-17 ATGGCTCCATTTTTGCCCGACCAGGTCGACTACAAACACGTCGACACCCTTATGTTATTA 60
FAO-18 ATGGCTCCATTTTTGCCCGACCAGGTCGACTACAAACACGTCGACACCCTTATGTTATTA 60
FAO2a ATGAATACCTTCTTGCCAGACGTGCTCGAATACAAACACGTCGACACCCTTTTGTTATTG 60
FAO2b ATGAATACCTTCTTGCCAGACGTGCTCGAATACAAACACGTCGATACCCTTTTGTTATTA 60
*** * * ** ***** *** * **** ************** ****** *******

FAO-13 TGTGACGGGATCATCCACGAAACCACCGTCGACCAAATCAAAGACGTTATTGCTCCTGAC 120
FAO-20 TGTGACGGGATCATCCACGAAACCACCGTCGACCAAATCAAAGACGTTATTGCTCCTGAC 120
FAO-17 TGTGACGGGATCATCCACGAAACCACCGTGGACGAAATCAAAGACGTCATTGCCCCTGAC 120
FAO-18 TGTGACGGGATCATCCACGAAACCACCGTGGACGAAATCAAAGACGTCATTGCCCCTGAC 120
FAO2a TGTGACGGGATCATCCACGAAACCACAGTCGATCAGATCAAGGACGCCATTGCTCCCGAC 120
FAO2b TGTGACGGGATCATCCACGAAACCACAGTCGACCAGATCAGGGACGCCATTGCTCCCGAC 120
************************** ** ** * **** **** ***** ** ***

FAO-13 TTCCCTGCTGACAAGTACGAAGAGTACGTCAGGACATTCACCAAACCCTCCGAAACCCCA 180
FAO-20 TTCCCTGCTGACAAGTACGAAGAGTACGTCAGGACATTCACCAAACCCTCCGAAACCCCA 180
FAO-17 TTCCCCGCCGACAAATACGAGGAGTACGTCAGGACATTCACCAAACCCTCCGAAACCCCA 180
FAO-18 TTCCCCGCCGACAAATACGAGGAGTACGTCAGGACATTCACCAAACCCTCCGAAACCCCA 180
FAO2a TTCCCTGAGGACCAGTACGAGGAGTATCTCAAGACCTTCACCAAGCCATCTGAGACCCCT 180
FAO2b TTCCCTGAAGACCAGTACGAGGAGTATCTCAAGACCTTCACCAAGCCATCTGAGACCCCT 180
***** * *** * ***** ***** *** *** ******** ** ** ** *****

FAO-13 GGGTTCAGGGAAACCGTCTACAACACAGTCAACGCAAACACCACGGACGCAATCCACCAG 240
FAO-20 GGGTTCAGGGAAACCGTCTACAACACAGTCAACGCAAACACCACGGACGCAATCCACCAG 240
FAO-17 GGGTTCAGGGAAACCGTCTACAACACCGTCAACGCAAACACCATGGATGCAATCCACCAG 240
FAO-18 GGGTTCAGGGAAACCGTCTACAACACCGTCAACGCAAACACCATGGATGCAATCCACCAG 240
FAO2a GGGTTCAGAGAAGCCGTCTACGACACGATCAACGCCACCCCAACCGATGCCGTGCACATG 240
FAO2b GGGTTCAGAGAAGCCGTCTACGACACGATCAACAGCACCCCAACCGAGGCTGTGCACATG 240
******** *** ******** **** ***** * * * * ** ** * *** *

FAO-13 TTCATTATCTTGACCAATGTTTTGGCATCCAGGGTCTTGGCTCCAGCTTTGACCAACTCG 300
FAO-20 TTCATTATCTTGACCAATGTTTTGGCATCCAGGGTCTTGGCTCCAGCTTTGACCAACTCG 300
FAO-17 TTCATTATCTTGACCAATGTTTTGGGATCAAGGGTCTTGGCACCAGCTTTGACCAACTCG 300
FAO-18 TTCATTATCTTGACCAATGTTTTGGGATCAAGGGTCTTGGCACCAGCTTTGACCAACTCG 300
FAO2a TGTATTGTCTTGACCACCGCATTGGACTCCAGAATCTTGGCCCCCACGTTGACCAACTCG 300
FAO2b TGTATTGTATTGACCACCGCATTGGACTCGAGAATCTTGGCCCCCACGTTGACCAACTCG 300
* *** * ******* * **** ** ** ******* ** * ***********

FAO-13          TTGACGCCTATCAAGGACATGAGCTTGGAAGACCGTGAAAAATTGTTGGCCTCGTGGCGC 360
FAO-20          TTGACGCCTATCAAGGACATGAGCTTGGAAGACCGTGAAAAATTGTTGGCCTCGTGGCGC 360
FAO-17          TTGACTCCTATCAAGGACATGAGCTTGGAAGACCGTGAAAAGTTGTTAGCCTCGTGGCGT 360
FAO-18          TTGACTCCTATCAAGGACATGAGCTTGGAAGACCGTGAAAAGTTGTTAGCCTCGTGGCGT 360
FAO2a           TTGACGCCTATCAAGGATATGACCTTGAAGGAGCGTGAACAATTGTTGGCCTCTTGGCGT 360
FAO2b           TTGACGCCTATCAAGGATATGACCTTGAAAGAGCGTGAACAATTGTTGGCTGCCTGGCGT 360
                ***** *********** **** **** * ** ****** * ***** **   * *****

FAO-13          GACTCCCCAATCGCTGCCAAAAGGAAGTTGTTCAGGTTGGTTTCTACGCTTACCTTGGTC 420
FAO-20          GACTCCCCAATCGCTGCCAAAAGGAAATTGTTCAGGTTGGTTTCCACGCTTACCTTGGTT 420
FAO-17          GACTCCCCTATTGCTGCTAAAAGGAAGTTGTTCAGGTTGGTTTCTACGCTTACCTTGGTC 420
FAO-18          GACTCCCCTATTGCTGCTAAAAGGAAGTTGTTCAGGTTGGTTTCTACGCTTACCTTGGTC 420
FAO2a           GATTCCCCGATTGCGGCAAAGAGAAGATTGTTCAGATTGATTTCCTCGCTTACCTTGACG 420
FAO2b           GATTCCCCGATCGCGGCCAAGAGAAGATTGTTCAGATTGATTTCCTCACTTACCTTGACG 420
                ** ***** ** ** ** ** ** *  ******** *** ****  * *********

FAO-13          ACGTTCACGAGATTGGCCAATGAGTTGCATTTGAAAGCCATTCATTATCCAGGAAGAGAA 480
FAO-20          ACTTTCACGAGATTGGCCAATGAGTTGCATTTGAAAGCCATTCACTATCCAGGAAGAGAA 480
FAO-17          ACGTTCACGAGATTGGCCAATGAGTTGCATTTGAAAGCCATTCATTATCCAGGAAGAGAA 480
FAO-18          ACGTTCACGAGATTGGCCAATGAGTTGCATTTGAAAGCCATTCATTATCCAGGAAGAGAA 480
FAO2a           ACGTTTACGAGATTGGCCAGCGAATTGCACTTGAAAGCCATCCACTACCCTGGCAGAGAC 480
FAO2b           ACCTTTACGAGATTGGCCAGCGACTTGCACTTGAGAGCCATCCACTACCCTGGCAGAGAC 480
                ** ** *************  ** ***** **** ****** ** ** ** ** *****

FAO-13          GACCGTGAAAAGGCTTATGAAACCCAGGAGATTGACCCTTTTAAGTACCAGTTTTTGGAA 540
FAO-20          GACCGTGAAAAGGCTTATGAAACCCAGGAGATTGACCCTTTCAAGTACCAGTTTATGGAA 540
FAO-17          GACCGTGAAAAGGCTTATGAAACCCAGGAGATTGACCCTTTTAAGTACCAGTTTTTGGAA 540
FAO-18          GACCGTGAAAAGGCTTATGAAACCCAGGAGATTGACCCTTTTAAGTACCAGTTTTTGGAA 540
FAO2a           TTGCGTGAAAAGGCGTATGAAACCCAGGTGGTTGACCCTTTCAGGTACCTGTTTATGGAG 540
FAO2b           TTGCGTGAAAAGGCATATGAAACCCAGGTGGTTGACCCTTTCAGGTACCTGTTTATGGAA 540
                   *********** ************* * ********** * ***** **** ****

FAO-13          AAACCGAAGTTTTACGGCGCTGAGTTGTACTTGCCAGATATTGATGTGATCATTATTGGA 600
FAO-20          AAGCCAAAGTTTGACGGCGCTGAGTTGTACTTGCCAGATATTGATGTTATCATTATTGGA 600
FAO-17          AAACCGAAGTTTTACGGCGCTGAGTTGTACTTGCCAGATATTGATGTGATCATTATTGGA 600
FAO-18          AAACCGAAGTTTTACGGCGCTGAGTTGTACTTGCCAGATATTGATGTGATCATTATTGGA 600
FAO2a           AAACCAAAGTTTGACGGCGCCGAATTGTACTTGCCAGATATCGACGTCATCATCATTGGA 600
FAO2b           AAACCAAAGTTTGACGGCACCGAGTTGTACTTGCCAGATATCGACGTCATCATCATTGGA 600
                ** ** ****** ***** * ** ***************** ** ** ***** ******

FAO-13          TCTGGTGCCGGTGCTGGTGTTGTGGCCCACACTTTGGCCAACGATGGCTTCAAGAGTTTG 660
FAO-20          TCTGGTGCCGGTGCTGGTGTTGTGGCCCACACTTTGGCCAACGATGGCTTCAAGAGTTTG 660
FAO-17          TCTGGTGCCGGTGCTGGTGTTGTGGCCCACACTTTGGCCAACGATGGCTTCAAGAGTTTG 660
FAO-18          TCTGGGGCCGGTGCTGGTGTCGTGGCCCACACTTTGACCAACGACGGCTTCAAGAGTTTG 660
FAO2a           TCAGGCGCCGGTGCTGGTGTCATGGCCCACACTCTCGCCAACGACGGGTTCAAGACCTTG 660
FAO2b           TCCGGTGCCGGTGCTGGTGTCATGGCCCACACTTTAGCCAACGACGGGTACAAGACCTTG 660
                ** ** **************  *********** *  ******* ** * *****  ***

FAO-13 GTTTTGGAAAAGGGCAAATACTTTAGCAACTCCGAGTTGAACTTTGATGACAAGGACGGC 720
FAO-20 GTTTTGGAAAAGGGCAAATACTTTAGCAACTCCGAGTTGAACTTTGATGACAAGGACGGC 720
FAO-17 GTTTTGGAAAAGGGCAAATACTTTAGCAACTCCGAGTTGAACTTTGATGACAAGGACGGC 720
FAO-18 GTTTTGGAAAAGGGCAGATACTTTAGCAACTCCGAGTTGAACTTTGATGACAAGGACGGG 720
FAO2a GTTTTGGAAAAGGGAAAGTATTTCAGCAACTCCGAGTTGAACTTTAATGACGCTGATGGC 720
FAO2b GTTTTGGAAAAGGGAAAGTATTTCAGCAACTCCGAGTTGAACTTTAATGATGCCGATGGT 720
************** *  ** ** ********************* ****    ** **

FAO-13 GTTCAAGAATTATACCAAAGTGGAGGTACTTTGACTACAGTCAACCAACAGTTGTTTGTT 780
FAO-20 GTTCAAGAATTATACCAAAGTGGAGGTACTTTGACTACAGTCAACCAACAGTTGTTTGTT 780
FAO-17 GTTCAAGAATTATACCAAAGTGGAGGTACTTTGACTACAGTCAACCAACAGTTGTTTGTT 780
FAO-18 GTTCAAGAATTATACCAAAGTGGAGGTACTTTGACCACCGTCAACCAGCAGTTGTTTGTT 780
FAO2a GTGAAAGAGTTGTACCAAGGTAAAGGTGCTTTGGCCACCACCAATCAGCAGATGTTTATT 780
FAO2b ATGAAAGAGTTGTACCAAGGTAAATGTGCGTTGACCACCACGAACCAGCAGATGTTTATT 780
 *  **** ** ****** **  * ** * *** * **    ** ** *** ***** **

FAO-13 CTTGCTGGTTCCACTTTTGGTGGCGGTACCACTGTCAATTGGTCAGCCTGTCTTAAGACG 840
FAO-20 CTTGCTGGTTCCACTTTTGGTGGCGGTACCACTGTCAATTGGTCAGCCTGTCTTAAGACG 840
FAO-17 CTTGCTGGTTCCACTTTTGGTGGCGGTACCACTGTCAATTGGTCAGCCTGTCTTAAGACG 840
FAO-18 CTTGCTGGTTCCACTTTTGGTGGTGGTACCACTGTCAATTGGTCGGCCTGTCTTAAAACG 840
FAO2a CTTGCCGGTTCCACTTTGGGCGGTGGTACCACTGTCAACTGGTCTGCTTGCCTTAAAACA 840
FAO2b CTTGCCGGTTCCACTTTGGGCGGTGGTACCACTGTTAACTGGTCTGCTTGTCTTAAAACA 840
***** *********** ** ** *********** ** ***** ** ** ***** **

FAO-13 CCATTCAAGGTGCGTAAGGAATGGTATGATGAGTTTGGTGTTGACTTTGCTGCTGATGAA 900
FAO-20 CCATTCAAGGTGCGTAAGGAATGGTATGATGAGTTTGGTGTTGACTTTGCTGCTGATGAA 900
FAO-17 CCATTCAAGGTGCGTAAGGAATGGTATGATGAGTTTGGTGTTGACTTTGCTGCTGATGAA 900
FAO-18 CCATTCAAGGTGCGTAAGGAATGGTATGATGAGTTTGGCGTTGACTTTGCTGCCGATGAA 900
FAO2a CCATTTAAAGTGCGTAAGGAGTGGTACGACGAGTTTGGTCTTGAATTTGCTGCCGATGAA 900
FAO2b CCATTTAAAGTGCGTAAGGAGTGGTACGACGAGTTTGGTCTTGAATTTGCTGCCGACGAA 900
***** ** *********** ***** ** ********  **** ******** ** ***

FAO-13 GCATACGATAAAGCGCAGGATTATGTTTGGCAGCAAATGGGAGCTTCTACCGAAGGCATC 960
FAO-20 GCATACGATAAAGCGCAGGATTATGTTTGGCAGCAAATGGGAGCTTCTACCGAAGGCATC 960
FAO-17 GCATACGATAAAGCGCAGGATTATGTTTGGCAGCAAATGGGAGCTTCTACCGAAGGCATC 960
FAO-18 GCCTACGACAAAGCACAGGATTATGTTTGGCAGCAAATGGGAGCTTCTACCGAAGGCATC 960
FAO2a GCCTACGACAAAGCGCAGGATTATGTTTGGAAACAAATGGGTGCTTCAACAGATGGAATC 960
FAO2b GCCTACGACAAAGCACAAGACTATGTTTGGAAACAAATGGGCGCTTCTACCGAAGGAATC 960
** ***** ***** ** ** ********* * ******** ***** ** ** ** ***

FAO-13 ACCCACTCTTTGGCTAACGAGATTATTATTGAAGGTGGTAAGAAATTAGGTTACAAGGCC 1020
FAO-20 ACCCACTCTTTGGCTAACGAGATTATTATTGAAGGTGGTAAGAAATTAGGTTACAAGGCC 1020
FAO-17 ACCCACTCTTTGGCTAACGAGATTATTATTGAAGGTGGTAAGAAATTAGGTTACAAGGCC 1020
FAO-18 ACCCACTCTTTGGCTAACGAGATTATTATTGAAGGTGGCAAGAAATTAGGTTACAAGGCC 1020
FAO2a ACTCACTCCTTGGCCAACGAAGTTGTGGTTGAAGGAGGTAAGAAGTTGGGCTACAAGAGC 1020
FAO2b ACTCACTCTTTGGCGAACGCGGTTGTGGTTGAAGGAGGTAAGAAGTTGGGTTACAAGAGC 1020
** ***** ***** ****   ** *  ******* ** ***** ** ** ******  *

FAO-13 AAGGTATTAGACCAAAACAGCGGTGGTCATCCTCAGCACAGATGCGGTTTCTGTTATTTG 1080
FAO-20 AAGGTATTAGACCAAAACAGCGGTGGTCATCCTCAGCACAGATGCGGTTTCTGTTATTTG 1080
FAO-17 AAGGTATTAGACCAAAACAGCGGTGGTCATCCTCAGCACAGATGCGGTTTCTGTTATTTG 1080
FAO-18 AAGGTATTAGACCAAAACAGCGGTGGTCATCCTCATCACAGATGCGGTTTCTGTTATTTG 1080
FAO2a AAGGAAATTGAGCAGAACAACGGTGGCCACCCTGACCACCCATGTGGTTTCTGTTACTTG 1080
FAO2b AAGGAAATCGAGCAGAACAATGGTGGCCATCCTGACCACCCCTGTGGTTTCTGTTACTTG 1080
**** * * ** ** ****  ***** ** *** * ***   ** *********** ***

FAO-13 GGCTGTAAGCACGGTATCAAGCAGGGTTCTGTTAATAACTGGTTTAGAGACGCAGCTGCC 1140
FAO-20 GGCTGTAAGCACGGTATCAAGCAGGGTTCTGTTAATAACTGGTTTAGAGACGCAGCTGCC 1140
FAO-17 GGTTGTAAGCACGGTATCAAGCAGGGCTCTGTTAATAACTGGTTTAGAGACGCAGCTGCC 1140
FAO-18 GGTTGTAAGCACGGTATCAAGCAGGGCTCTGTTAATAACTGGTTTAGAGACGCAGCTGCC 1140
FAO2a GGCTGTAAGTACGGTATTAAACAGGGTTCTGTGAATAACTGGTTTAGAGACGCAGCTGCC 1140
FAO2b GGCTGTAAGTACGGTATTAAGCAGGGTTCTGTGAATAACTGGTTTAGAGACGCAGCTGCC 1140
** ****** ******* ** ***** ***** ***************************

FAO-13 CACGGTTCCCAGTTCATGCAACAGGTTAGAGTTTTGCAAATACTTAACAAGAAGGGGATC 1200
FAO-20 CACGGTTCCCAGTTCATGCAACAGGTTAGAGTTTTGCAAATACTTAACAAGAAGGGGATC 1200
FAO-17 CACGGTTCTCAGTTCATGCAACAGGTTAGAGTTTTGCAAATCCTTAACAAGAAGGGCATC 1200
FAO-18 CACGGTTCTCAGTTCATGCAACAGGTTAGAGTTTTGCAAATCCTTAACAAGAAGGGCATC 1200
FAO2a CACGGGTCCAAGTTCATGCAACAAGTCAGAGTTGTGCAAATCCTCAACAAGAATGGCGTC 1200
FAO2b CACGGGTCCAAGTTCATGCAACAAGTCAGAGTTGTGCAAATCCTCCACAATAAAGGCGTC 1200
***** **  ************* ** ****** ******* **  **** ** **  **

FAO-13 GCTTACGGTATCTTGTGTGAGGATGTTGTAACCGGCGCCAAGTTCACCATTACTGGCCCC 1260
FAO-20 GCTTACGGTATCTTGTGTGAGGATGTTGTAACCGGCGCCAAGTTCACCATTACTGGCCCC 1260
FAO-17 GCTTATGGTATCTTGTGTGAGGATGTTGTAACCGGTGCCAAGTTCACCATTACTGGCCCC 1260
FAO-18 GCTTATGGTATCTTGTGTGAGGATGTTGTAACCGGTGCCAAGTTCACCATTACTGGCCCC 1260
FAO2a GCTTATGGTATCTTGTGTGAGGATGTCGAAACCGGAGTCAGGTTCACTATTAGTGGCCCC 1260
FAO2b GCTTATGGCATCTTGTGTGAGGATGTCGAGACCGGAGTCAAATTCACTATCAGTGGCCCC 1260
***** ** ***************** *  ***** * **  ***** ** * *******

FAO-13 AAAAAGTTTGTTGTTGCTGCCGGTGCTTTGAACACTCCATCTGTGTTGGTCAACTCCGGC 1320
FAO-20 AAAAAGTTTGTTGTTGCTGCCGGTGCTTTGAACACTCCATCTGTGTTGGTCAACTCCGGC 1320
FAO-17 AAAAAGTTTGTTGTTGCCGCCGGCGCCTTAAACACTCCATCTGTGTTGGTCAACTCCGGA 1320
FAO-18 AAAAAGTTTGTTGTTGCCGCCGGCGCCTTAAACACTCCATCTGTGTTGGTCAACTCCGGA 1320
FAO2a AAAAAGTTTGTTGTTTCTGCTGGTTCTTTGAACACGCCAACTGTGTTGACCAACTCCGGA 1320
FAO2b AAAAAGTTTGTTGTTTCTGCAGGTTCTTTGAACACGCCAACGGTGTTGACCAACTCCGGA 1320
*************** * ** **  * ** ***** *** * ******  *********

FAO-13 TTCAAGAACAAGAACATCGGTAAGAACTTAACTTTGCACCCAGTTTCTGTCGTGTTTGGT 1380
FAO-20 TTCAAGAACAAGAACATCGGTAAGAACTTAACTTTGCACCCAGTTTCTGTCGTGTTTGGT 1380
FAO-17 TTCAAGAACAAGAACATCGGTAAGAACTTAACTTTGCATCCAGTTTCTGTCGTGTTTGGT 1380
FAO-18 TTCAAGAACAAGAACATCGGTAAGAACTTAACTTTGCATCCAGTTTCTGTCGTGTTTGGT 1380
FAO2a TTCAAGAACAAGCACATTGGTAAGAACTTGACGTTGCACCCAGTTTCCACCGTGTTTGGT 1380
FAO2b TTCAAGAACAAACACATCGGTAAGAACTTGACGTTGCACCCAGTTTCGACCGTGTTTGGT 1380
***********  **** *********** ** ***** ********   **********

FAO-13 GATTTTGGCAAAGACGTTCAAGCAGACCACTTCCACAACTCCATCATGACTGCCCTTTGT 1440
FAO-20 GATTTTGGCAAAGACGTTCAAGCAGACCACTTCCACAACTCCATCATGACTGCCCTTTGT 1440
FAO-17 GATTTTGGCAAAGACGTTCAAGCAGACCACTTCCACAACTCCATCATGACTGCCCTTTGT 1440
FAO-18 GATTTTGGCAAAGACGTTCAAGCAGATCACTTCCACAACTCCATCATGACTGCTCTTTGT 1440
FAO2a GACTTTGGCAGAGACGTGCAAGCCGACCATTTCCACAAATCTATTATGACTTCGCTTTGT 1440
FAO2b GACTTTGGCAGAGACGTGCAAGCCGACCATTTCCACAAATCTATTATGACTTCGCTCTGT 1440
** ******* ****** ***** ** ** ******** ** ** ****** * ** ***

FAO-13 TCAGAAGCCGCTGATTTAGACGGCAAGGGCCATGGATGCAGAATTGAAACCATCTTGAAC 1500
FAO-20 TCAGAAGCCGCTGATTTAGACGGCAAGGGCCATGGATGCAGAATTGAAACCATCTTGAAC 1500
FAO-17 TCAGAAGCCGCTGATTTAGACGGCAAGGGCCATGGATGCAGAATTGAAACCATCTTGAAC 1500
FAO-18 TCAGAAGCCGCTGATTTAGACGGCAAGGGTCATGGATGCAGAATTGAAACCATCTTGAAC 1500
FAO2a TACGAGGTTGCTGACTTGGACGGCAAGGGCCACGGATGCAGAATCGAAACCATCTTGAAC 1500
FAO2b TACGAAGTCGCTGACTTGGACGGCAAGGGCCACGGATGCAGAATCGAGACCATCTTGAAC 1500
*  ** *  ***** ** *********** ** *********** ** ************

FAO-13 GCTCCATTCATCCAGGCTTCATTCTTACCATGGAGAGGTAGTAACGAGGCTAGACGAGAC 1560
FAO-20 GCTCCATTCATCCAGGCTTCATTCTTACCATGGAGAGGTAGTAACGAGGCTAGACGAGAC 1560
FAO-17 GCTCCATTCATCCAGGCTTCATTCTTACCATGGAGAGGTAGTAACGAGGCTAGACGAGAC 1560
FAO-18 GCTCCATTCATCCAGGCTTCATTCTTACCATGGAGAGGTAGTAACGAGGCTAGACGAGAC 1560
FAO2a GCTCCATTCATCCAAGCTTCTTTGTTGCCATGGAGAGGAAGTGACGAGGTCAGAAGAGAC 1560
FAO2b GCTCCATTCATCCAAGCTTCTTTGTTGCCATGGAGAGGAAGCGACGAGGTCAGAAGAGAC 1560
************** ***** ** ** *********** **  ******  *** *****

FAO-13 TTGTTGCGTTACAACAACATGGTGGCGATGTTGCTCCTTAGTCGTGACACCACCAGTGGT 1620
FAO-20 TTGTTGCGTTACAACAACATGGTGGCGATGTTGCTCCTTAGTCGTGACACCACCAGTGGT 1620
FAO-17 TTGTTGCGTTACAACAACATGGTGGCGATGTTGCTCCTTAGTCGTGACACCACCAGTGGT 1620
FAO-18 TTGTTGCGTTACAACAACATGGTGGCCATGTTACTTCTTAGTCGTGATACCACCAGTGGT 1620
FAO2a TTGTTGCGTTACAACAACATGGTGGCCATGTTGCTTATCACGCGTGATACCACCAGTGGT 1620
FAO2b TTGTTGCGTTACAACAACATGGTGGCCATGTTGCTTATCACCCGTGACACCACCAGTGGT 1620
************************** ***** **  * *  ***** ************

FAO-13 TCCGTTTCTGCTCATCCAACCAAACCTGAAGCTTTGGTTGTCGAGTACGACGTGAACAAG 1680
FAO-20 TCCGTTTCTGCTCATCCAACCAAACCTGAAGCTTTGGTTGTCGAGTACGACGTGAACAAG 1680
FAO-17 TCCGTTTCTGCTCATCCAACCAAACCTGAAGCTTTGGTTGTCGAGTACGACGTGAACAAG 1680
FAO-18 TCCGTTTCGTCCCATCCAACTAAACCTGAAGCATTAGTTGTCGAGTACGACGTGAACAAG 1680
FAO2a TCAGTTTCTGCTGACCCAAAGAAGCCCGACGCTTTGATTGTCGACTATGAGATTAACAAG 1680
FAO2b TCAGTTTCTGCTGACCCAAAGAAGCCCGACGCTTTGATTGTCGACTATGACATCAACAAG 1680
** *****  *  * ****  ** ** ** ** **  ******* ** **  * ******

FAO-13 TTTGACAGAAACTCGATCTTGCAGGCATTGTTGGTCACTGCTGACTTGTTGTATATCCAA 1740
FAO-20 TTTGACAGAAACTCGATCTTGCAGGCATTGTTGGTCACTGCTGACTTGTTGTATATCCAA 1740
FAO-17 TTTGACAGAAACTCGATCTTGCAGGCATTGTTGGTCACTGCTGACTTGTTGTATATCCAA 1740
FAO-18 TTTGACAGAAACTCCATCTTGCAGGCATTGTTGGTCACTGCTGACTTGTTGTACATTCAA 1740
FAO2a TTTGACAAGAATGCCATCTTGCAAGCTTTCTTGATCACTTCCGACATGTTGTACATTGAA 1740
FAO2b TTTGACAAGAATGCCATCTTGCAAGCTTTCTTGATCACCTCCGACATGTTGTACATCGAA 1740
*******  **  * ******** ** ** *** ****   * *** ******* **  **

FAO-13          GGTGCCAAGAGAATCCTTAGTCCACAGGCATGGGTGCCAATTTTTGAATCCGACAAGCCA 1800
FAO-20          GGTGCCAAGAGAATCCTTAGTCCACAGGCATGGGTGCCAATTTTTGAATCCGACAAGCCA 1800
FAO-17          GGTGCCAAGAGAATCCTTAGTCCACAGGCATGGGTGCCAATTTTTGAATCCGACAAGCCA 1800
FAO-18          GGTGCCAAGAGAATCCTTAGTCCCCAACCATGGGTGCCAATTTTTGAATCCGACAAGCCA 1800
FAO2a           GGTGCCAAGAGAATCCTCAGTCCACAGCCATGGGTGCCAATCTTTGAGTCGAACAAGCCA 1800
FAO2b           GGTGCCAAGAGAATCCTCAGTCCACAGGCATGGGTGCCAATCTTTGAGTCGAACAAGCCA 1800
                ***************** ***** **  ************* ***** **  ********

FAO-13          AAGGATAAGAGATCAATCAAGGACGAGGACTATGTCGAATGGAGAGCCAAGGTTGCCAAG 1860
FAO-20          AAGGATAAGAGATCAATCAAGGACGAGGACTATGTCGAATGGAGAGCCAAGGTTGCCAAG 1860
FAO-17          AAGGATAAGAGATCAATCAAGGACGAGGACTATGTCGAATGGAGAGCCAAGGTTGCCAAG 1860
FAO-18          AAGGATAAGAGATCAATCAAGGACGAGGACTATGTCGAATGGAGAGCCAAGGTTGCCAAG 1860
FAO2a           AAGGAGCAAAGAACGATCAAGGACAAGGACTATGTTGAGTGGAGAGCCAAGGCTGCTAAG 1860
FAO2b           AAGGAGCAAAGAACAATCAAGGACAAGGACTATGTCGAATGGAGAGCCAAGGCTGCCAAG 1860
                *****   * *** * ********* ********** ** ************* *** ***

FAO-13          ATTCCTTTCGACACCTACGGCTCACCTTATGGTTCGGCACATCAAATGTCTTCTTGCCGT 1920
FAO-20          ATTCCTTTCGACACCTACGGCTCACCTTATGGTTCGGCACATCAAATGTCTTCTTGCCGT 1920
FAO-17          ATTCCTTTCGACACCTACGGCTCACCTTATGGTTCGGCACATCAAATGTCTTCTTGCCGT 1920
FAO-18          ATTCCTTTTGACACCTACGGCTCGCCTTATGGTTCGGCGCATCAAATGTCTTCTTGTCGT 1920
FAO2a           ATACCTTTCGACACCTACGGTTCTGCATATGGGTCCGCACATCAAATGTCCACCTGTCGT 1920
FAO2b           ATACCTTTCGACACCTACGGTTCTGCCTATGGGTCCGCACATCAAATGTCCACCTGTCGT 1920
                ** ***** *********** **   * ***** ** ** ***********   * ** ***

FAO-13          ATGTCAGGTAAGGGTCCTAAATACGGTGCTGTTGACACCGATGGTAGATTGTTTGAATGT 1980
FAO-20          ATGTCAGGTAAGGGTCCTAAATACGGTGCTGTTGACACCGATGGTAGATTGTTTGAATGT 1980
FAO-17          ATGTCAGGTAAGGGTCCTAAATACGGTGCTGTTGACACCGATGGTAGATTGTTTGAATGT 1980
FAO-18          ATGTCAGGTAAGGGTCCTAAATACGGTGCTGTTGATACCGATGGTAGATTGTTTGAATGT 1980
FAO2a           ATGTCCGGAAAGGGTCCTAAATACGGTGCTGTTGATACTGATGGTAGATTGTTTGAATGT 1980
FAO2b           ATGTCCGGAAAGGGTCCTAAATACGGCGCCGTTGATACCGATGGTAGATTGTTTGAATGT 1980
                ***** ** ***************** ** ***** ** ********************

FAO-13          TCGAATGTTTATGTTGCCGATGCAAGTCTTTTGCCAACTGCAAGCGGTGCCAACCCTATG 2040
FAO-20          TCGAATGTTTATGTTGCCGATGCAAGTCTTTTGCCAACTGCAAGCGGTGCCAACCCTATG 2040
FAO-17          TCGAATGTTTATGTTGCCGATGCAAGTCTTTTGCCAACTGCAAGCGGTGCCAACCCTATG 2040
FAO-18          TCGAATGTTTATGTTGCTGACGCTAGTCTTTTGCCAACTGCTAGCGGTGCTAATCCTATG 2040
FAO2a           TCGAATGTCTATGTTGCTGATGCTAGTGTTTTGCCTACTGCCAGCGGTGCCAACCCAATG 2040
FAO2b           TCGAATGTCTATGTTGCTGATGCTAGTGTTTTGCCTACTGCCAGCGGTGCCAACCCAATG 2040
                ******** ******** ** ** *** ******* ***** ******** ** ** ***

FAO-13          GTCACCACCATGACTCTTGCCAGACATGTTGCGTTAGGTTTGGCAGACTCCTTGAAGACC 2100
FAO-20          GTCACCACCATGACTCTTGCCAGACATGTTGCGTTAGGTTTGGCAGACTCCTTGAAGACC 2100
FAO-17          GTCACCACCATGACTCTTGCAAGACATGTTGCGTTAGGTTTGGCAGACTCCTTGAAGACC 2100
FAO-18          GTCACCACCATGACTCTTGCAAGACATGTTGCGTTAGGTTTGGCAGACTCCTTGAAGACC 2100
FAO2a           ATATCCACCATGACCTTTGCTAGACAGATTGCGTTAGGTTTGGCTGACTCCTTGAAGACC 2100
FAO2b           ATCTCCACCATGACGTTTGCTAGACAGATTGCGTTAGGTTTGGCTGACTCTTTGAAGACC 2100
                 *  **********  **** *****  *************** ***** *********

FAO-13          AAAGCCAAGTTGTAG 2115
FAO-20          AAAGCCAAGTTGTAG 2115
FAO-17          AAGGCCAAGTTGTAG 2115
FAO-18          AAGGCCAAGTTGTAG 2115
FAO2a           AAACCCAAGTTGTAG 2115
FAO2b           AAACCCAAGTTGTAG 2115
                **  ***********

### Amino acid sequences

FAO-1 - SEQ ID NO:7 
MAPFLPDQVDYKHVDTLMLLCDGIIHETTVDEIKDVIAPDFPADKYEEYVRTFTKPSETPGFRETVYNTVNANTMDAIHQ
FIILTNVLGSRVLAPALTNSLTPIKDMSLEDREKLLASWRDSPIAAKRKLFRLVSTLTLVTFTRLANELHLKAIHYPGRE
DREKAYETQEIDPFKYQFLEKPKFYGAELYLPDIDVIIIGSGAGAGVVAHTLTNDGFKSLVLEKGRYFSNSELNFDDKDG
VQELYQSGGTLTTVNQQLFVLAGSTFGGGTTVNWSACLKTPFKVRKEWYDEFGVDFAADEAYDKAQDYVWQQMGASTEGI
THSLANEIIIEGGKKLGYKAKVLDQNSGGHPHHRCGFCYLGCKHGIKQGSVNNWFRDAAAHGSQFMQQVRVLQILNKKGI
AYGILCEDVVTGAKFTITGPKKFVVAAGALNTPSVLVNSGFKNKNIGKNLTLHPVSVVFGDFGKDVQADHFHNSIMTALC
SEAADLDGKGHGCRIETILNAPFIQASFLPWRGSNEARRDLLRYNNMVAMLLLSRDTTSGSVSSHPTKPEALVVEYDVNK
FDRNSILQALLVTADLLYIQGAKRILSPQPWVPIFESDKPKDKRSIKDEDYVEWRAKVAKIPFDTYGSPYGSAHQMSSCR
MSGKGPKYGAVDTDGRLFECSNVYVADASLLPTASGANPMVTTMTLARHVALGLADSLKTKAKL
FAO-13 - SEQ ID NO:8 
MAPFLPDQVDYKHVDTLMLLCDGIIHETTVDQIKDVIAPDFPADKYEEYVRTFTKPSETPGFRETVYNTVNANTTDAIHQ
FIILTNVLASRVLAPALTNSLTPIKDMSLEDREKLLASWRDSPIAAKRKLFRLVSTLTLVTFTRLANELHLKAIHYPGRE
DREKAYETQEIDPFKYQFLEKPKFYGAELYLPDIDVIIIGSGAGAGVVAHTLANDGFKSLVLEKGKYFSNSELNFDDKDG
VQELYQSGGTLTTVNQQLFVLAGSTFGGGTTVNWSACLKTPFKVRKEWYDEFGVDFAADEAYDKAQDYVWQQMGASTEGI
THSLANEIIIEGGKKLGYKAKVLDQNSGGHPQHRCGFCYLGCKHGIKQGSVNNWFRDAAAHGSQFMQQVRVLQILNKKGI
AYGILCEDVVTGAKFTITGPKKFVVAAGALNTPSVLVNSGFKNKNIGKNLTLHPVSVVFGDFGKDVQADHFHNSIMTALC
SEAADLDGKGHGCRIETILNAPFIQASFLPWRGSNEARRDLLRYNNMVAMLLLSRDTTSGSVSAHPTKPEALVVEYDVNK
FDRNSILQALLVTADLLYIQGAKRILSPQAWVPIFESDKPKDKRSIKDEDYVEWRAKVAKIPFDTYGSPYGSAHQMSSCR
MSGKGPKYGAVDTDGRLFECSNVYVADASLLPTASGANPMVTTMTLARHVALGLADSLKTKAKL
FAO-20 - SEQ ID NO:9 
MAPFLPDQVDYKHVDTLMLLCDGIIHETTVDQIKDVIAPDFPADKYEEYVRTFTKPSETPGFRETVYNTVNANTTDAIHQ
FIILTNVLASRVLAPALTNSLTPIKDMSLEDREKLLASWRDSPIAAKRKLFRLVSTLTLVTFTRLANELHLKAIHYPGRE
DREKAYETQEIDPFKYQFMEKPKFDGAELYLPDIDVIIIGSGAGAGVVAHTLANDGFKSLVLEKGKYFSNSELNFDDKDG
VQELYQSGGTLTTVNQQLFVLAGSTFGGGTTVNWSACLKTPFKVRKEWYDEFGVDFAADEAYDKAQDYVWQQMGASTEGI
THSLANEIIIEGGKKLGYKAKVLDQNSGGHPQHRCGFCYLGCKHGIKQGSVNNWFRDAAAHGSQFMQQVRVLQILNKKGI
AYGILCEDVVTGAKFTITGPKKFVVAAGALNTPSVLVNSGFKNKNIGKNLTLHPVSVVFGDFGKDVQADHFHNSIMTALC
SEAADLDGKGHGCRIETILNAPFIQASFLPWRGSNEARRDLLRYNNMVAMLLLSRDTTSGSVSAHPTKPEALVVEYDVNK
FDRNSILQALLVTADLLYIQGAKRILSPQAWVPIFESDKPKDKRSIKDEDYVEWRAKVAKIPFDTYGSPYGSAHQMSSCR
MSGKGPKYGAVDTDGRLFECSNVYVADASLLPTASGANPMVTTMTLARHVALGLADSLKTKAKL
FAO-17 - SEQ ID NO: 10 
MAPFLPDQVDYKHVDTLMLLCDGIIHETTVDEIKDVIAPDFPADKYEEYVRTFTKPSETPGFRETVYNTVNANTMDAIHQ
FIILTNVLGSRVLAPALTNSLTPIKDMSLEDREKLLASWRDSPIAAKRKLFRLVSTLTLVTFTRLANELHLKAIHYPGRE
DREKAYETQEIDPFKYQFLEKPKFYGAELYLPDIDVIIIGSGAGAGVVAHTLANDGFKSLVLEKGKYFSNSELNFDDKDG
VQELYQSGGTLTTVNQQLFVLAGSTFGGGTTVNWSACLKTPFKVRKEWYDEFGVDFAADEAYDKAQDYVWQQMGASTEGI
THSLANEIIIEGGKKLGYKAKVLDQNSGGHPQHRCGFCYLGCKHGIKQGSVNNWFRDAAAHGSQFMQQVRVLQILNKKGI
AYGILCEDVVTGAKFTITGPKKFVVAAGALNTPSVLVNSGFKNKNIGKNLTLHPVSVVFGDFGKDVQADHFHNSIMTALC
SEAADLDGKGHGCRIETILNAPFIQASFLPWRGSNEARRDLLRYNNMVAMLLLSRDTTSGSVSAHPTKPEALVVEYDVNK
FDRNSILQALLVTADLLYIQGAKRILSPQAWVPIFESDKPKDKRSIKDEDYVEWRAKVAKIPFDTYGSPYGSAHQMSSCR
MSGKGPKYGAVDTDGRLFECSNVYVADASLLPTASGANPMVTTMTLARHVALGLADSLKTKAKl
FAO-2a - SEQ ID NO: 11 
MNTFLPDVLEYKHVDTLLLLCDGIIHETTVDQIKDAIAPDFPEDQYEEYLKTFTKPSETPGFREAVYDTINATPTDAVHM
CIVLTTALDSRILAPTLTNSLTPIKDMTLKEREQLLASWRDSPIAAKRRLFRLISSLTLTTFTRLASELHLKAIHYPGRD
LREKAYETQVVDPFRYSFMEKPKFDGAELYLPDIDVIIIGSGAGAGVMAHTLANDGFKTLVLEKGKYFSNSELNFNDADG
VKELYQGKGALATTNQQMFILAGSTLGGGTTVNWSACLKTPFKVRKEWYDEFGLEFAADEAYDKAQDYVWKQMGASTDGI
THSLANEVVVEGGKKLGYKSKEIEQNNGGHPDHPCGFCYLGCKYGIKQGSVNNWFRDAAAHGSKFMQQVRVVQILNKNGV
AYGILCEDVETGVRFTISGPKKFVVSAGSLNTPTVLTNSGFKNKHIGKNLTLHPVSTVFGDFGRDVQADHFHKSIMTSLC
YEVADLDGKGHGCRIETILNAPFIQASLLPWRGSDEVRRDLLRYNNMVAMLLITRDTTSGSVSADPKKPDALIVDYEINK
 
FDKNAILQAFLITSDMLYIEGAKRILSPQPWVPIFESNKPKEQRTIKDKDYVEWRAKAAKIPFDTYGSAYGSAHQMSTCR
MSGKGPKYGAVDTDGRLFECSNVYVADASVLPTASGANPMISTMTFARQIALGLADSLKTKPKL
FAO-2b - SEQ ID NO:12

MNTFLPDVLEYKHVDTLLLLCDGIIHETTVDQIRDAIAPDFPEDQYEEYLKTFTKPSETPGFREAVYDTINSTPTEAVHM
CIVLTTALDSRILAPTLTNSLTPIKDMTLKEREQLLAAWRDSPIAAKRRLFRLISSLTLTTFTRLASDLHLRAIHYPGRD
LREKAYETQVVDPFRYSFMEKPKFDGTELYLPDIDVIIIGSGAGAGVMAHTLANDGYKTLVLEKGKYFSNSELNFNDADG
MKELYQGKCALTTTNQQMFILAGSTLGGGTTVNWSACLKTPFKVRKEWYDEFGLEFAADEAYDKAQDYVWKQMGASTEGI
THSLANAVVVEGGKKLGYKSKEIEQNNGGHPDHPCGFCYLGCKYGIKQGSVNNWFRDAAAHGSKFMQQVRVVQILHNKGV
AYGILCEDVETGVKFTISGPKKFVVSAGSLNTPTVLTNSGFKNKHIGKNLTLHPVSTVFGDFGRDVQADHFHKSIMTSLC
YEVADLDGKGHGCRIETILNAPFIQASLLPWRGSDEVRRDLLRYNNMVAMLLITRDTTSGSVSADPKKPDALIVDYDINK
FDKNAILQAFLITSDMLYIEGAKRILSPQAWVPIFESNKPKEQRTIKDKDYVEWRAKAAKIPFDTYGSAYGSAHQMSTCR
MSGKGPKYGAVDTDGRLFECSNVYVADASVLPTASGANPMISTMTFARQIALGLADSLKTKPKL

### Clustal amino acid sequence alignments

FAO-13 MAPFLPDQVDYKHVDTLMLLCDGIIHETTVDQIKDVIAPDFPADKYEEYVRTFTKPSETP 60
FAO-20 MAPFLPDQVDYKHVDTLMLLCDGIIHETTVDQIKDVIAPDFPADKYEEYVRTFTKPSETP 60
FAO-1 MAPFLPDQVDYKHVDTLMLLCDGIIHETTVDEIKDVIAPDFPADKYEEYVRTFTKPSETP 60
*******************************:****************************

FAO-13 GFRETVYNTVNANTTDAIHQFIILTNVLASRVLAPALTNSLTPIKDMSLEDREKLLASWR 120
FAO-20 GFRETVYNTVNANTTDAIHQFIILTNVLASRVLAPALTNSLTPIKDMSLEDREKLLASWR 120
FAO-1 GFRETVYNTVNANTMDAIHQFIILTNVLGSRVLAPALTNSLTPIKDMSLEDREKLLASWR 120
************** *************.*******************************

FAO-13 DSPIAAKRKLFRLVSTLTLVTFTRLANELHLKAIHYPGREDREKAYETQEIDPFKYQFLE 180
FAO-20 DSPIAAKRKLFRLVSTLTLVTFTRLANELHLKAIHYPGREDREKAYETQEIDPFKYQFME 180
FAO-1 DSPIAAKRKLFRLVSTLTLVTFTRLANELHLKAIHYPGREDREKAYETQEIDPFKYQFLE 180
**********************************************************:*

FAO-13 KPKFYGAELYLPDIDVIIIGSGAGAGVVAHTLANDGFKSLVLEKGKYFSNSELNFDDKDG 240
FAO-20 KPKFDGAELYLPDIDVIIIGSGAGAGVVAHTLANDGFKSLVLEKGKYFSNSELNFDDKDG 240
FAO-1 KPKFYGAELYLPDIDVIIIGSGAGAGVVAHTLTNDGFKSLVLEKGRYFSNSELNFDDKDG 240
**** ***************************:************:**************

FAO-13 VQELYQSGGTLTTVNQQLFVLAGSTFGGGTTVNWSACLKTPFKVRKEWYDEFGVDFAADE 300
FAO-20 VQELYQSGGTLTTVNQQLFVLAGSTFGGGTTVNWSACLKTPFKVRKEWYDEFGVDFAADE 300
FAO-1 VQELYQSGGTLTTVNQQLFVLAGSTFGGGTTVNWSACLKTPFKVRKEWYDEFGVDFAADE 300
************************************************************

FAO-13 AYDKAQDYVWQQMGASTEGITHSLANEIIIEGGKKLGYKAKVLDQNSGGHPQHRCGFCYL 360
FAO-20 AYDKAQDYVWQQMGASTEGITHSLANEIIIEGGKKLGYKAKVLDQNSGGHPQHRCGFCYL 360
FAO-1 AYDKAQDYVWQQMGASTEGITHSLANEIIIEGGKKLGYKAKVLDQNSGGHPHHRCGFCYL 360
***************************************************:********

FAO-13 GCKHGIKQGSVNNWFRDAAAHGSQFMQQVRVLQILNKKGIAYGILCEDVVTGAKFTITGP 420
FAO-20 GCKHGIKQGSVNNWFRDAAAHGSQFMQQVRVLQILNKKGIAYGILCEDVVTGAKFTITGP 420
FAO-1 GCKHGIKQGSVNNWFRDAAAHGSQFMQQVRVLQILNKKGIAYGILCEDVVTGAKFTITGP 420
************************************************************

FAO-13 KKFVVAAGALNTPSVLVNSGFKNKNIGKNLTLHPVSVVFGDFGKDVQADHFHNSIMTALC 480
FAO-20 KKFVVAAGALNTPSVLVNSGFKNKNIGKNLTLHPVSVVFGDFGKDVQADHFHNSIMTALC 480
FAO-1 KKFVVAAGALNTPSVLVNSGFKNKNIGKNLTLHPVSVVFGDFGKDVQADHFHNSIMTALC 480
************************************************************

FAO-13 SEAADLDGKGHGCRIETILNAPFIQASFLPWRGSNEARRDLLRYNNMVAMLLLSRDTTSG 540
FAO-20 SEAADLDGKGHGCRIETILNAPFIQASFLPWRGSNEARRDLLRYNNMVAMLLLSRDTTSG 540
FAO-1 SEAADLDGKGHGCRIETILNAPFIQASFLPWRGSNEARRDLLRYNNMVAMLLLSRDTTSG 540
************************************************************

FAO-13 SVSAHPTKPEALVVEYDVNKFDRNSILQALLVTADLLYIQGAKRILSPQAWVPIFESDKP 600
FAO-20 SVSAHPTKPEALVVEYDVNKFDRNSILQALLVTADLLYIQGAKRILSPQAWVPIFESDKP 600
FAO-1 SVSSHPTKPEALVVEYDVNKFDRNSILQALLVTADLLYIQGAKRILSPQPWVPIFESDKP 600
***:**********************************************.*********

FAO-13 KDKRSIKDEDYVEWRAKVAKIPFDTYGSPYGSAHQMSSCRMSGKGPKYGAVDTDGRLFEC 660
FAO-20 KDKRSIKDEDYVEWRAKVAKIPFDTYGSPYGSAHQMSSCRMSGKGPKYGAVDTDGRLFEC 660
FAO-1 KDKRSIKDEDYVEWRAKVAKIPFDTYGSPYGSAHQMSSCRMSGKGPKYGAVDTDGRLFEC 660
************************************************************

FAO-13 SNVYVADASLLPTASGANPMVTTMTLARHVALGLADSLKTKAKL 704
FAO-20 SNVYVADASLLPTASGANPMVTTMTLARHVALGLADSLKTKAKL 704
FAO-1 SNVYVADASLLPTASGANPMVTTMTLARHVALGLADSLKTKAKL 704
********************************************

### Example 31: Addition and/or Amplification of Monooxygenase and Monooxygenase reductase activities.

Cytochrome P450's often catalyze a monooxygenase reaction, e.g., insertion of one atom of oxygen into an organic substrate (RH) while the other oxygen atom is reduced to water:

RH + O₂ + 2H+ + 2e- → ROH + H₂O

The substrates sometimes are of a homogeneous carbon chain length. Enzymes with monooxygenase activity sometimes recognize substrates of specific carbon chain lengths, or a subgroup of carbon chain lengths with respect to organic substrates of homogenous carbon chain length. Addition of novel cytochrome activities (e.g., B. megaterium BM3) and/or amplification of certain or all endogenous or heterologous monooxygenase activities (e.g., CYP52A12 polynucleotide, CYP52A13 polynucleotide, CYP52A14 polynucleotide, CYP52A15 polynucleotide, CYP52A16 polynucleotide, CYP52A17 polynucleotide, CYP52A18 polynucleotide, CYP52A19 polynucleotide, CYP52A20 polynucleotide, CYP52D2 polynucleotide, BM3 polynucleotide) can contribute to an overall increase in carbon flux through native and/or engineered metabolic pathways, in some embodiments. In certain embodiments, adding a novel monooxygenase or increasing certain or all endogenous or heterologous monooxygenase activities can increase the flux of substrates of specific carbon chain length or subgroups of substrates with mixtures of specific carbon chain lengths. In some embodiments, the selection of a monooxygenase activity for amplification in an engineered strain is related to the feedstock utilized for growth of the engineered strain, pathways for metabolism of the chosen feedstock and the desire end product (e.g., adipic acid).

Strains engineered to utilize plant-based oils for conversion to adipic acid, would benefit by having one or more monooxygenase activities with substrate specificity that matches the fatty acid chain-length distribution of the oil. For example, the most prevalent fatty acid in coconut oil is lauric acid (12 carbons long), therefore, the monooxygenase activity chosen for a coconut oil-utilizing strain would have a substrate preference for C12 fatty acids. For strains engineered to utilize other plant based oils with different fatty acid chain-length distributions it would be desirable to amplify a monooxygenase activity that has a matching substrate preference. In some embodiments, a genetic modification that alters monooxygenase activity increases the activity of one or more monooxygenase activities with a substrate preference for fatty acids prevalent in coconut oil. In certain embodiments, the genetic modification increases the activity of a monooxygenase activity with a preference for C12 fatty acids.

Strains engineered to utilize glucose for conversion to adipic acid, would benefit by choosing a monooxygenase activity that can utilize the distribution of chain lengths produced by fatty acid synthase (FAS). For strains engineered to utilize the long chain fatty acids produced by FAS, it often is desirable to add and/or amplify one or more monooxygenase activities preferring long chain fatty acids. For strains engineered to utilize a mutant FAS that produces medium- or short-chain fatty acids, or a specialized FAS (e.g., hexanoate synthase), it often is desirable to add and/or amplify one or more monooxygenase activities preferring medium- or short-chain fatty acids. In some embodiments, a genetic modification that alters monooxygenase activity increases the activity of one or more monooxygenase activities with a preference for the long chain fatty acids produced by fatty acid synthase activity (e.g., FAS). In certain embodiments, the genetic modification increases the activity of a monooxygenase activity with a preference for the medium-or short-chain fatty acids produced by a mutant or specialized FAS.

As mentioned previously, the enzymes that carry out the monooxygenase activity are reduced by the activity of monooxygenase reductase, thereby regenerating the enzyme. Selection of a CPR for amplification in an engineered strain depends upon which P450 is amplified, in some embodiments. A particular CPR may interact preferentially with one or more monooxygenase activities, in some embodiments, but not well with other monooxygenases. A monooxygenase reductase from C. tropicalis strain ATCC750, two monooxygenase reductase activities from C. tropicalis strain ATCC20336 and a monooxygenase reductase activity from Bacillus megaterium are being evaluated for activity with the added and/or amplified monooxygenases described herein. Provided in the tables below are nucleotide sequences used to add or amplify monooxygenase and monooxygenase reductase activities.

Monooxygenase activity nucleotide sequences

| SEQ ID NO | Description | Sequence |
|---|---|---|
| SEQ ID NO: 13 | cytochrome P450 A12 (CYP52A12) | atggccacacaagaaatcatcgattctgtacttccgtacttgaccaaatgg tacactgtgattactgcagcagtattagtcttccttatctccacaaacatc aagaactacgtcaaggcaaagaaattgaaatgtgtcgatccaccatacttg aaggatgccggtctcactggtattctgtctttgatcgccgccatcaaggcc aagaacgacggtagattggctaactttgccgatgaagttttcgacgagtac ccaaaccacaccttctacttgtctgttgccggtgctttgaagattgtcatg actgttgacccagaaaacatcaaggctgtcttggccacccaattcactgac ttctccttgggtaccagacacgcccactttgctcctttgttgggtgacggt atcttcaccttggacggagaaggttggaagcactccagagctatgttgaga |
| | | ccacagtttgctagagaccagattggacacgttaaagccttggaaccacac atccaaatcatggctaagcagatcaagttgaaccagggaaagactttcgat atccaagaattgttctttagatttaccgtcgacaccgctactgagttcttg tttggtgaatccgttcactccttgtacgatgaaaaattgggcatcccaact ccaaacgaaatcccaggaagagaaaactttgccgctgctttcaacgtttcc caacactacttggccaccagaagttactcccagactttttactttttgacc aaccctaaggaattcagagactgtaacgccaaggtccaccacttggccaag tactttgtcaacaaggccttgaactttactcctgaagaactcgaagagaaa tccaagtccggttacgttttcttgtacgaattggttaagcaaaccagagat ccaaaggtcttgcaagatcaattgttgaacattatggttgccggaagagac accactgccggtttgttgtcctttgctttgtttgaattggctagacaccca gagatgtggtccaagttgagagaagaaatcgaagttaactttggtgttggt gaagactcccgcgttgaagaaattaccttcgaagccttgaagagatgtgaa tacttgaaggctatccttaacgaaaccttgcgtatgtacccatctgttcct gtcaactttagaaccgccaccagagacaccactttgccaagaggtggtggt gctaacggtaccgacccaatctacattcctaaaggctccactgttgcttac gttgtctacaagacccaccgtttggaagaatactacggtaaggacgctaac gacttcagaccagaaagatggtttgaaccatctactaagaagttgggctgg gcttatgttccattcaacggtggtccaagagtctgcttgggtcaacaattc gccttgactgaagcttcttatgtgatcactagattggcccagatgtttgaa actgtctcatctgatccaggtctcgaataccctccaccaaagtgtattcac ttgaccatgagtcacaacgatggtgtctttgtcaagatgtaa |
| SEQ ID NO: 14 | cytochrome P450 A13 (CYP52A13) | atgactgtacacgatattatcgccacatacttcaccaaatggtacgtgata gtaccactcgctttgattgcttatagagtcctcgactacttctatggcaga tacttgatgtacaagcttggtgctaaaccattttccagaaacagacagac ggctgtttcggattcaaagctccgcttgaattgttgaagaagaagagcgac ggtaccctcatagacttcacactccagcgtatccacgatctcgatcgtccc gatatcccaactttcacattcccggtcttttccatcaaccttgtcaatacc cttgagccggagaacatcaaggccatcttggccactcagttcaacgatttc tccttgggtaccagacactcgcactttgctcctttgttgggtgatggtatc tttacgttggatggcgccggctggaagcacagcagatctatgttgagacca cagtttgccagagaacagatttcccacgtcaagttgttggagccacacgtt caggtgttcttcaaacacgtcagaaaggcacagggcaagactttgacatc caggaattgtttttcagattgaccgtcgactccgccaccgagtttttgttt ggtgaatccgttgagtccttgagagatgaatctatcggcatgtccatcaat gcgcttgactttgacggcaaggctggctttgctgatgcttttaactattcg cagaattatttggcttcgagagcggttatgcaacaattgtactgggtgttg aacgggaaaaagtttaaggagtgcaacgctaaagtgcacaagtttgctgac tactacgtcaacaaggctttggacttgacgcctgaacaattggaaaagcag gatggttatgtgtttttgtacgaattggtcaagcaaaccagagacaagcaa gtgttgagagaccaattgttgaacatcatggttgctggtagagacaccacc gccggtttgttgtcgtttgttttctttgaattggccagaaacccagaagtt accaacaagttgagagaagaaattgaggacaagtttggactcggtgagaat gctagtgttgaagacatttcctttgagtcgttgaagtcctgtgaatacttg aaggctgttctcaacgaaaccttgagattgtacccatccgtgccacagaat ttcagagttgccaccaagaacactaccctcccaagaggtggtggtaaggac gggttgtctcctgttttggtgagaaagggtcagaccgttatttacggtgtc tacgcagcccacagaaacccagctgtttacggtaaggacgctcttgagttt agaccagagagatggtttgagccagagacaaagaagcttggctgggccttc ctcccattcaacggtggtccaagaatctgtttgggacagcagtttgccttg acagaagcttcgtatgtcactgtcaggttgctccaggagtttgcacacttg tctatggacccagacaccgaatatccacctaagaaaatgtcgcatttgacc atgtcgcttttcgacggtgccaatattgagatgtattag |
| SEQ ID NO: 15 | cytochrome P450 A14 (CYP52A14) | atgactgcacaggatattatcgccacatacatcaccaaatggtacgtgata gtaccactcgctttgattgcttatagggtcctcgactacttttacggcaga tacttgatgtacaagcttggtgctaaaccgtttttccagaaacaaacagac ggttatttcggattcaaagctccacttgaattgttaaaaaagaagagtgac ggtaccctcatagacttcactctcgagcgtatccaagcgctcaatcgtcca gatatcccaacttttacattcccaatcttttccatcaaccttatcagcacc cttgagccggagaacatcaaggctatcttggccacccagttcaacgatttc tccttgggcaccagacactcgcactttgctcctttgttgggcgatggtatc tttaccttggacggtgccggctggaagcacagcagatctatgttgagacca cagtttgccagagaacagatttcccacgtcaagttgttggagccacacatg caggtgttcttcaagcacgtcagaaaggcacagggcaagacttttgacatc |
| | | caagaattgtttttcagattgaccgtcgactccgccactgagtttttgttt ggtgaatccgttgagtccttgagagatgaatctattgggatgtccatcaat gcacttgactttgacggcaaggctggctttgctgatgcttttaactactcg cagaactatttggcttcgagagcggttatgcaacaattgtactgggtgttg aacggaaaaagtttaaggagtgcaacgctaaagtgcacaagtttgctgac tattacgtcagcaaggctttggacttgacacctgaacaattggaaaagcag gatggttatgtgttcttgtacgagttggtcaagcaaaccagagacaggcaa gtgttgagagaccagttgttgaacatcatggttgccggtagagacaccacc gccggtttgttgtcgtttgttttctttgaattggccagaaacccagaggtg accaacaagttgagagaagaaatcgaggacaagtttggtcttggtgagaat gctcgtgttgaagacatttcctttgagtcgttgaagtcatgtgaatacttg aaggctgttctcaacgaaactttgagattgtacccatccgtgccacagaat ttcagagttgccaccaaaaacactacccttccaagggggaggtggtaaggac gggttatctcctgttttggtcagaaagggtcaaaccgttatgtacggtgtc tacgctgcccacagaaacccagctgtctacggtaaggacgcccttgagttt agaccagagaggtggtttgagccagagacaaagaagcttggctgggccttc cttccattcaacggtggtccaagaatttgcttgggacagcagtttgccttg acagaagcttcgtatgtcactgtcagattgctccaagagtttggacacttg tctatggaccccaacaccgaatatccacctaggaaaatgtcgcatttgacc atgtcccttttcgacggtgccaacattgagatgtattag |
| SEQ ID NO: 16 | cytochrome P450 A15 (CYP52A15) | atgtcgtcttctccatcgtttgcccaagaggttctcgctaccactagtcct tacatcgagtactttcttgacaactacaccagatggtactacttcatacct ttggtgcttctttcgttgaactttataagtttgctccacacaaggtacttg gaacgcaggttccacgccaagccactcggtaactttgtcaggaccctacg tttggtatcgctactccgttgcttttgatctacttgaagtcgaaaggtacg gtcatgaagtttgcttggggcctctggaacaacaagtacatcgtcagagac ccaaagtacaagacaactgggctcaggattgttggcctcccattgattgaa accatggacccagagaacatcaaggctgttttggctactcagttcaatgat ttctctttgggaaccagacacgatttcttgtactccttgttgggtgacggt attttcaccttggacggtgctggctggaaacatagtagaactatgttgaga ccacagtttgctagagaacaggtttctcacgtcaagttgttggagccacac gttcaggtgttcttcaagcacgttagaaagcaccgcggtcaaacgttcgac atccaagaattgttcttcaggttgaccgtcgactccgccaccgagttcttg tttggtgagtctgctgaatccttgagggacgaatctattggattgacccca accaccaaggatttcgatggcagaagagatttcgctgacgctttcaactat tcgcagacttaccaggcctacagattttgttgcaacaaatgtactggatc ttgaatggctcggaattcagaaagtcgattgctgtcgtgcacaagtttgct gaccactatgtgcaaaaggctttggagttgaccgacgatgacttgcagaaa caagacggctatgtgttcttgtacgagttggctaagcaaaccagagaccca aaggtcttgagagaccagttattgaacattttggttgccggtagagacacg accgccggtttgttgtcatttgttttctacgagttgtcaagaaaccctgag gtgtttgctaagttgagagaggaggtggaaaacagatttggactcggtgaa gaagctcgtgttgaagagatctcgtttgagtccttgaagtcttgtgagtac ttgaaggctgtcatcaatgaaaccttgagattgtacccatcggttccacac aactttagagttgctaccagaaacactaccctcccaagaggtggtggtgaa gatggatactcgccaattgtcgtcaagaagggtcaagttgtcatgtacact gttattgctacccacagagacccaagtatctacggtgccgacgctgacgtc ttcagaccagaaagatggtttgaaccagaaactagaaagttgggctgggca tacgttccattcaatggtggtccaagaatctgtttgggtcaacagtttgcc ttgaccgaagcttcatacgtcactgtcagattgctccaggagtttgcacac ttgtctatggacccagacaccgaatatccaccaaaattgcagaacaccttg accttgtcgctctttgatggtgctgatgttagaatgtactaa |
| SEQ ID NO: 17 | cytochrome P450 A16 (CYP52A16) | atgtcgtcttctccatcgtttgctcaggaggttctcgctaccactagtcct tacatcgagtactttcttgacaactacaccagatggtactacttcatccct ttggtgcttctttcgttgaacttcatcagcttgctccacacaaagtacttg gaacgcaggttccacgccaagccgctcggtaacgtcgtgttggatcctacg tttggtatcgctactccgttgatcttgatctacttaaagtcgaaaggtaca gtcatgaagtttgcctggagcttctggaacaacaagtacattgtcaaagac ccaaagtacaagaccactggccttagaattgtcggcctcccattgattgaa accatagacccagagaacatcaaagctgtgttggctactcagttcaacgat ttctccttgggaactagacacgatttcttgtactccttgttgggcgatggt atttttaccttggacggtgctggctggaaacacagtagaactatgttgaga ccacagtttgctagagaacaggtttcccacgtcaagttgttggaaccacac gttcaggtgttcttcaagcacgttagaaaacaccgcggtcagacttttgac |
| | | atccaagaattgttcttcagattgaccgtcgactccgccaccgagttcttg tttggtgagtctgctgaatccttgagagacgactctgttggtttgacccca accaccaaggatttcgaaggcagaggagatttcgctgacgctttcaactac tcgcagacttaccaggcctacagattttgttgcaacaaatgtactggatt ttgaatggcgcggaattcagaaagtcgattgccatcgtgcacaagtttgct gaccactatgtgcaaaaggctttggagttgaccgacgatgacttgcagaaa caagacggctatgtgttcttgtacgagttggctaagcaaactagagaccca aaggtcttgagagaccagttgttgaacattttggttgccggtagagacacg accgccggtttgttgtcgtttgtgttctacgagttgtcgagaaaccctgaa gtgtttgccaagttgagagaggaggtggaaaacagatttggactcggcgaa gaggctcgtgttgaagagatctcttttgagtccttgaagtcctgtgagtac ttgaaggctgtcatcaatgaagccttgagattgtacccatctgttccacac aacttcagagttgccaccagaaacactacccttccaagaggcggtggtaaa gacggatgctcgccaattgttgtcaagaagggtcaagttgtcatgtacact gtcattggtacccacagagacccaagtatctacggtgccgacgccgacgtc ttcagaccagaaagatggttcgagccagaaactagaaagttgggctgggca tatgttccattcaatggtggtccaagaatctgtttgggtcagcagtttgcc ttgactgaagcttcatacgtcactgtcagattgctccaagagtttggaaac ttgtccctggatccaaacgctgagtacccaccaaaattgcagaacaccttg accttgtcactctttgatggtgctgacgttagaatgttctaa |
| SEQ ID NO: 18 | cytochrome P450 A17 (CYP52A17) | atgattgaacaactcctagaatattggtatgtcgttgtgccagtgttgtac atcatcaaacaactccttgcatacacaaagactcgcgtcttgatgaaaaag ttgggtgctgctccagtcacaaacaagttgtacgacaacgctttcggtatc gtcaatggatggaaggctctccagttcaagaaagagggcagggctcaagag tacaacgattacaagtttgaccactccaagaacccaagcgtgggcacctac gtcagtattcttttcggcaccaggatcgtcgtgaccaaagatccagagaat atcaaagctatttggcaacccagtttggtgatttttctttgggcaagagg cacactctttttaagcctttgttaggtgatgggatcttcacattggacggc gaaggctggaagcacagcagagccatgttgagaccacagtttgccagagaa caagttgctcatgtgacgtcgttggaaccacacttccagttgttgaagaag catattcttaagcacaagggtgaatactttgatatccaggaattgttcttt agatttaccgttgattcggccacggagttcttatttggtgagtccgtgcac tccttaaaggacgaatctattggtatcaaccaagacgatatagattttgct ggtagaaaggactttgctgagtcgttcaacaaagcccaggaatacttggct attagaaccttggtgcagacgttctactggttggtcaacaacaaggagttt agagactgtaccaagctggtgcacaagttcaccaactactatgttcagaaa gctttggatgctagcccagaagagcttgaaaagcaaagtgggtatgtgttc ttgtacgagcttgtcaagcagacaagagaccccaatgtgttgcgtgaccag tctttgaacatcttgttggccggaagagacaccactgctgggttgttgtcg tttgctgtctttgagttggccagacacccagagatctgggccaagttgaga gaggaaattgaacaacagtttggtcttggagaagactctcgtgttgaagag attacctttgagagcttgaagagatgtgagtacttgaaagcgttccttaat gaaaccttgcgtatttacccaagtgtcccaagaaacttcagaatcgccacc aagaacacgacattgccaagggggcggtggttcagacggtacctcgccaatc ttgatccaaaaggagaagctgtgtcgtatggtatcaactctactcatttg gaccctgtctattacggccctgatgctgctgagttcagaccagagagatgg tttgagccatcaaccaaaaagctcggctgggcttacttgccattcaacggt ggtccaagaatctgtttgggtcagcagtttgccttgacggaagctggctat gtgttggttagattggtgcaagagttctcccacgttaggctggacccagac gaggtgtacccgccaaagaggttgaccaacttgaccatgtgtttgcaggat ggtgctattgtcaagtttgactag |
| SEQ ID NO: 19 | cytochrome P450 A18 (CYP52A18) | atgattgaacaaatcctagaatattggtatattgttgtgcctgtgttgtac atcatcaaacaactcattgcctacagcaagactcgcgtcttgatgaaacag ttgggtgctgctccaatcacaaaccagttgtacgacaacgttttcggtatc gtcaacggatggaaggctctccagttcaagaaagagggcagagctcaagag tacaacgatcacaagtttgacagctccaagaacccaagcgtcggcacctat gtcagtattctttttggcaccaagattgtcgtgaccaaggatccagagaat atcaaagctattttggcaacccagtttggcgatttttctttgggcaagaga cacgctctttttaaacctttgttaggtgatgggatcttcaccttggacggc gaaggctggaagcatagcagatccatgttaagaccacagtttgccagagaa caagttgctcatgtgacgtcgttggaaccacacttccagttgttgaagaag catatccttaaacacaagggtgagtactttgatatccaggaattgttcttt agatttactgtcgactcggccacggagttcttatttggtgagtccgtgcac tccttaaaggacgaaactatcggtatcaaccaagacgatatagattttgct |
| | | ggtagaaaggactttgctgagtcgttcaacaaagcccaggagtatttgtct attagaattttggtgcagaccttctactggttgatcaacaacaaggagttt agagactgtaccaagctggtgcacaagtttaccaactactatgttcagaaa gctttggatgctaccccagaggaacttgaaaagcaaggcgggtatgtgttc ttgtatgagcttgtcaagcagacgagagaccccaaggtgttgcgtgaccag tctttgaacatcttgttggcaggaagagacaccactgctgggttgttgtcc tttgctgtgtttgagttggccagaaacccacacatctgggccaagttgaga gaggaaattgaacagcagtttggtcttggagaagactctcgtgttgaagag attacctttgagagcttgaagagatgtgagtacttgaaagcgttccttaac gaaaccttgcgtgtttacccaagtgtcccaagaaacttcagaatcgccacc aagaatacaacattgccaagggggtggtggtccagacggtacccagccaatc ttgatccaaaaggagaaggtgtgtcgtatggtatcaactctacccactta gatcctgtctattatggccctgatgctgctgagttcagaccagagagatgg tttgagccatcaaccagaaagctcggctgggcttacttgccattcaacggt gggccacgaatctgtttgggtcagcagtttgccttgaccgaagctggttac gttttggtcagattggtgcaagagttctcccacattaggctggacccagat gaagtgtatccaccaaagaggttgaccaacttgaccatgtgtttgcaggat ggtgctattgtcaagtttgactag |
| SEQ ID NO: 20 | cytochrome P450 A19 (CYP52A19) | atgctcgatcagatcttacattactggtacattgtcttgccattgttggcc attatcaaccagatcgtggctcatgtcaggaccaattatttgatgaagaaa ttgggtgctaagccattcacacacgtccaacgtgacgggtggttgggcttc aaattcggccgtgaattcctcaaagcaaaaagtgctgggagactggttgat ttaatcatctcccgtttccacgataatgaggacactttctccagctatgct tttggcaaccatgtggtgttcaccagggaccccgagaatatcaaggcgctt ttggcaacccagtttggtgatttttcattgggcagcagggtcaagttcttc aaaccattattggggtacggtatcttcacattggacgccgaaggctggaag cacagcagagccatgttgagaccacagtttgccagagaacaagttgctcat gtgacgtcgttggaaccacacttccagttgttgaagaagcatatccttaaa cacaagggtgagtactttgatatccaggaattgttctttagatttactgtc gactcggccacggagttcttatttggtgagtccgtgcactccttaaaggac gaggaaattggctacgacacgaaagacatgtctgaagaaagacgcagattt gccgacgcgttcaacaagtcgcaagtctacgtggccaccagagttgcttta cagaacttgtactggttggtcaacaacaaagagttcaaggagtgcaatgac attgtccacaagtttaccaactactatgttcagaaagccttggatgctacc ccagaggaacttgaaaagcaaggcgggtatgtgttcttgtatgagcttgtc aagcagacgagagaccccaaggtgttgcgtgaccagtctttgaacatcttg ttggcaggaagagacaccactgctgggttgttgtcctttgctgtgtttgag ttggccagaaacccacacatctgggccaagttgagagaggaaattgaacag cagtttggtcttggagaagactctcgtgttgaagagattacctttgagagc ttgaagagatgtgagtacttgaaggccgtgttgaacgaaactttgagatta cacccaagtgtcccaagaaacgcaagatttgcgattaaagacacgacttta ccaagaggcggtggccccaacggcaaggatcctatcttgatcaggaaggat gaggtggtgcagtactccatctcggcaactcagacaaatcctgcttattat ggcgccgatgctgctgattttagaccggaaagatggtttgaaccatcaact agaaacttgggatgggctttcttgccattcaacggtggtccaagaatctgt ttgggacaacagtttgctttgactgaagccggttacgttttggttagactt gttcaggagtttccaaacttgtcacaagaccccgaaaccaagtacccacca cctagattggcacacttgacgatgtgcttgtttgacggtgcacacgtcaag atgtcatag |
| SEQ ID NO: 21 | cytochrome P450 A20 (CYP52A20) | atgctcgaccagatcttccattactggtacattgtcttgccattgttggtc attatcaagcagatcgtggctcatgccaggaccaattatttgatgaagaag ttgggcgctaagccattcacacatgtccaactagacgggtggtttggcttc aaatttggccgtgaattcctcaaagctaaaagtgctgggaggcaggttgat ttaatcatctcccgtttccacgataatgaggacactttctccagctatgct tttggcaaccatgtggtgttcaccagggaccccgagaatatcaaggcgctt ttggcaacccagtttggtgatttttcattgggaagcagggtcaaattcttc aaaccattgttggggtacggtatcttcaccttggacggcgaaggctggaag cacagcagagccatgttgagaccacagtttgccagagagcaagttgctcat gtgacgtcgttggaaccacatttccagttgttgaagaagcatattcttaag cacaagggtgaatactttgatatccaggaattgttctttagatttaccgtt gattcagcgacggagttcttatttggtgagtccgtgcactccttaagggac gaggaaattggctacgatacgaaggacatggctgaagaaagacgcaaattt gccgacgcgttcaacaagtcgcaagtctatttgtccaccagagttgcttta cagacattgtactggttggtcaacaacaaagagttcaaggagtgcaacgac |
| | | attgtccacaagttcaccaactactatgttcagaaagccttggatgctacc ccagaggaacttgaaaaacaaggcgggtatgtgttcttgtacgagcttgcc aagcagacgaaagaccccaatgtgttgcgtgaccagtctttgaacatcttg ttggctggaagggacaccactgctgggttgttgtcctttgctgtgtttgag ttggccaggaacccacacatctgggccaagttgagagaggaaattgaatca cactttgggctgggtgaggactctcgtgttgaagagattacctttgagagc ttgaagagatgtgagtacttgaaagccgtgttgaacgaaacgttgagatta cacccaagtgtcccaagaaacgcaagatttgcgattaaagacacgacttta ccaagaggcggtggccccaacggcaaggatcctatcttgatcagaaagaat gaggtggtgcaatactccatctcggcaactcagacaaatcctgcttattat ggcgccgatgctgctgattttagaccggaaagatggtttgagccatcaact agaaacttgggatgggcttacttgccattcaacggtggtccaagaatctgc ttgggacaacagtttgctttgaccgaagccggttacgttttggttagactt gttcaggaattccctagcttgtcacaggaccccgaaactgagtacccacca cctagattggcacacttgacgatgtgcttgtttgacggggcatacgtcaag atgcaatag |
| SEQ ID NO: 22 | cytochrome P450 D2 (CYP52D2) | atggctatatctagtttgctatcgtgggatgtgatctgtgtcgtcttcatt tgcgtttgtgtttatttcgggtatgaatattgttatactaaatacttgatg cacaaacatggcgctcgagaaatcgagaatgtgatcaacgatgggttcttt gggttccgcttacctttgctactcatgcgagccagcaatgaggccgactt atcgagttcagtgtcaagagattcgagtcggcgccacatccacagaacaag acattggtcaaccgggcattgagcgttcctgtgatactcaccaaggaccca gtgaatatcaaagcgatgctatcgacccagtttgatgacttttcccttggg ttgagactacaccagtttgcgccgttgttggggaaaggcatctttactttg gacggcccagagtggaagcagagccgatctatgttgcgtccgcaatttgcc aaagatcgggtttctcatatcctggatctagaaccgcatttgtgttgctt cggaagcacattgatggccacaatggagactacttcgacatccaggagctc tacttccggttctcgatggatgtggcgacgggtttttgtttggcgagtct gtggggtcgttgaaagacgaagatgcgaggttcctggaagcattcaatgag tcgcagaagtatttggcaactagggcaacgttgcacgagttgtactttctt tgtgacgggtttaggtttcgccagtacaacaaggttgtgcgaaagttctgc agccagtgtgtccacaaggcgttagatgttgcaccggaagacaccagcgag tacgtgtttctccgcgagttggtcaaacacactcgagatcccgttgtttta caagaccaagcgttgaacgtcttgcttgctggacgcgacaccaccgcgtcg ttattatcgtttgcaacatttgagctagcccggaatgaccacatgtggagg aagctacgagaggaggttatcctgacgatgggaccgtccagtgatgaaata accgtggccgggttgaagagttgccgttacctcaaagcaatcctaaacgaa actcttcgactatacccaagtgtgcctaggaacgcgagatttgctacgagg aatacgacgcttcctcgtggcggaggtccagatggatcgtttccgatttg ataagaaagggccagccagtggggtatttcatttgtgctacacacttgaat gagaaggtatatggaatgatagccatgtgtttcgaccggagagatggct gcgttagagggcaagagtttggctggtcgtatcttccattcaacggcggc ccgagaagctgccttggtcagcagtttgcaatccttgaagcttcgtatgtt ttggctcgattgacacagtgctacacgacgatacagcttagaactaccgag tacccaccaaagaaactcgttcatctcacgatgagtcttctcaacggggtg tacatccgaactagaacttga |

A comparison of the amino acid sequence identities of the monooxygenase activities is presented in the table below. The black colored boxes indicate regions of 100% identity. Many of the monooxygenase activities shown in the table below have a closely related "sibling" sequence.

| **Protein Sequence Identity** | | | | | | | | | | |
|---|---|---|---|---|---|---|---|---|---|---|
| | CYP52A12 | CYP52A17 | CYP52A18 | CYP52A19 | CYP52A20 | CYP52A13 | CYP52A14 | CYP52A15 | CYP52A16 | CYP52D2 |
| CYP52A12 | | 62 | 61 | 60 | 60 | 59 | 59 | 55 | 55 | 51 |
| CYP52A17 | | | 94 | 73 | 73 | 62 | 62 | 60 | 59 | 54 |
| CYP52A18 | | | | 74 | 74 | 61 | 61 | 60 | 59 | 54 |
| CYP52A19 | | | | | 95 | 64 | 63 | 59 | 58 | 54 |
| CYP52A20 | | | | | | 64 | 63 | 59 | 59 | 55 |
| CYP52A13 | | | | | | | 96 | 68 | 66 | 53 |
| CYP52A14 | | | | | | | | 68 | 67 | 53 |
| CYP52A15 | | | | | | | | | 96 | 50 |
| CYP52A16 | | | | | | | | | | 49 |
| CYP52D2 | | | | | | | | | | |

Monooxygenase reductase activity nucleotide sequences

| SEQ ID NO | Description | Sequence |
|---|---|---|
| SEQ ID NO:23 | cytochrome P450:NADPH P450 reductase (Bacillus megaterium) nucleotide | atgacaattaaagaaatgcctcagccaaaaacgtttggagagcttaaaaa tttaccgttattaaacacagataaaccggttcaagctttgatgaaaattg cggatgaattaggagaaatctttaaattcgaggcgcctggtcgtgtaacg cgctacttatcaagtcagcgtctaattaaagaagcatgcgatgaatcacg ctttgataaaaacttaagtcaagcgcttaaatttgtacgtgattttgcag gagacgggttatttacaagctggacgcatgaaaaaaattggaaaaaagcg cataatatcttacttccaagcttcagtcagcaggcaatgaaaggctatca tgcgatgatggtcgatatcgccgtgcagcttgttcaaaagtgggagcgtc taaatgcagatgagcatattgaagtaccggaagacatgacacgtttaacg cttgatacaattggtctttgcggctttaactatcgctttaacagcttta ccgagatcagcctcatccatttattacaagtatggtccgtgcactggatg aagcaatgaacaagctgcagcgagcaaatccagacgacccagcttatgat gaaaacaagcgccagtttcaagaagatatcaaggtgatgaacgacctagt agataaaattattgcagatcgcaaagcaagcggtgaacaaagcgatgatt tattaacgcatatgctaaacggaaaagatccagaaacgggtgagccgctt gatgacgagaacattcgctatcaaattattacattcttaattgcgggaca cgaaacaacaagtggtcttttatcatttgcgctgtatttcttagtgaaaa atccacatgtattacaaaaagcagcagaagaagcagcacgagttctagta gatcctgttccaagctacaaacaagtcaaacagcttaaatatgtcggcat ggtcttaaacgaagcgctgcgcttatggccaactgctcctgcgttttccc tatatgcaaaagaagatacggtgcttggaggagaatatcctttagaaaaa ggcgacgaactaatggttctgattcctcagcttcaccgtgataaaacaat ttggggagacgatgtggaagagttccgtccagagcgttttgaaaatccaa gtgcgattccgcagcatgcgtttaaaccgtttggaaacggtcagcgtgcg tgtatcggtcagcagttcgctcttcatgaagcaacgctggtacttggtat gatgctaaaacactttgactttgaagatcatacaaactacgagctggata ttaaagaaactttaacgttaaaacctgaaggctttgtggtaaaagcaaaa tcgaaaaaaattccgcttggcggtattccttcacctagcactgaacagtc tgctaaaaaagtacgcaaaaaggcagaaaacgctcataatacgccgctgc ttgtgctatacggttcaaatatgggaacagctgaaggaacggcgcgtgat ttagcagatattgcaatgagcaaaggatttgcaccgcaggtcgcaacgct tgattcacacgccggaaatcttccgcgcgaaggagctgtattaattgtaa cggcgtcttataacggtcatccgcctgataacgcaaagcaatttgtcgac tggttagaccaagcgtctgctgatgaagtaaaaggcgttcgctactccgt atttggatgcggcgataaaaactgggctactacgtatcaaaaagtgcctg cttttatcgatgaaacgcttgccgctaaaggggcagaaaacatcgctgac |
| | | cgcggtgaagcagatgcaagcgacgactttgaaggcacatatgaagaatg gcgtgaacatatgtggagtgacgtagcagcctactttaacctcgacattg aaaacagtgaagataataaatctactctttcacttcaatttgtcgacagc gccgcggatatgccgcttgcgaaaatgcacggtgcgttttcaacgaacgt cgtagcaagcaaagaacttcaacagccaggcagtgcacgaagcacgcgac atcttgaaattgaacttccaaaagaagcttcttatcaagaaggagatcat ttaggtgttattcctcgcaactatgaaggaatagtaaaccgtgtaacagc aaggttcggcctagatgcatcacagcaaatccgtctggaagcagaagaag aaaaattagctcatttgccactcgctaaaacagtatccgtagaagagctt ctgcaatacgtggagcttcaagatcctgttacgcgcacgcagcttcgcgc aatggctgctaaaacggtctgcccgccgcataaagtagagcttgaagcct tgcttgaaaagcaagcctacaaagaacaagtgctggcaaaacgtttaaca atgcttgaactgcttgaaaaatacccggcgtgtgaaatgaaattcagcga atttatcgcccttctgccaagcatacgcccgcgctattactcgatttctt catcacctcgtgtcgatgaaaaacaagcaagcatcacggtcagcgttgtc tcaggagaagcgtggagcggatatggagaatataaaggaattgcgtcgaa ctatcttgccgagctgcaagaaggagatacgattacgtgctttatttcca caccgcagtcagaatttacgctgccaaaagaccctgaaacgccgcttatc atggtcggaccgggaacaggcgtcgcgccgtttagaggctttgtgcaggc gcgcaaacagctaaaagaacaaggacagtcacttggagaagcacatttat acttcggctgccgttcacctcatgaagactatctgtatcaagaagagctt gaaaacgcccaaagcgaaggcatcattacgcttcataccgcttttctcg catgccaaatcagccgaaaacatacgttcagcacgtaatggaacaagacg gcaagaaattgattgaacttcttgatcaaggagcgcacttctatatttgc ggagacggaagccaaatggcacctgccgttgaagcaacgcttatgaaaag ctatgctgacgttcaccaagtgagtgaagcagacgctcgcttatggctgc agcagctagaagaaaaaggccgatacgcaaaagacgtgtgggctgggtaa |
| SEQ ID NO:24 | NADPH cytochrome P450 reductase, CPR (C. tropicalis strain ATCC750) | atggcattagataagttagatttatatgttattataacattggtggttgc aattgcagcttattttgcaaagaatcagtttcttgaccaacaacaagata ccgggttccttaatactgatagtggagatggtaattcaagagatatctta caagctttgaagaagaacaataaaaatacgttattattatttggatccca aacaggtacagcagaagattatgccaacaaattgtcaagagaattgcatt caagatttggtttgaaaaccatggttgctgatttcgctgattatgatttc gaaaacttcggagatattactgaagatatcttggtttttctttattgttgc tacttatggtgaaggtgaaccaaccgataatgctgacgaatttcacactt ggttgactgaagaagctgacaccttgagtactttgaaatatactgttttt ggtttgggtaattcaacttatgaattcttcaatgctattggtagaaaatt tgacagattgttgggagaaaaaggtggtgacagatttgctgaatacggtg aaggtgacgatggtactggtactttagatgaagatttcttggcctggaag gataacgtgtttgattccttaaagaatgatttgaattttgaagaaaaaga gttgaaatacgaaccaaatgttaaattgactgaaagagatgatttatctg gcaatgatccagatgtctccttgggtgaaccaaatgtcaaatacattaaa tctgaaggtgttgacttaactaaaggtccatttgatcatactcatccatt tttggctagaattgttaaaactaaagaattgtttacttctgaagacagac attgtgttcatgttgaatttgatatttctgaatcaaacttgaaatatacc accggtgatcatcttgcaatctggccatctaactctgatgaaaacattaa gcaatttgccaaatgttttggtttagaagacaaacttgatactgttattg aattgaaagctttggattccacttattccatcccattccctaatccaatc acttatggagctgttattagacaccatttggaaatttcaggtcctgtttc tagacaattttcttatctattgctggatttgcccctgatgaagaaacta aaaagtcatttactagaattggtggtgataagcaagaatttgctagtaaa gtcacccgtagaaaattcaacattgccgatgctttattatttgcttccaa caacagaccatggtccgatgttccattcgaattccttattgaaaatgtcc aacacttaactcctcgttattactccatttcttcttcctcattaagtgaa aagcaaaccattaatgttactgctgttgttgaagccgaagaagaagctga tggaagaccagttactggtgttgtcaccaacttgttgaagaatattgaaa ttgaacaaaacaaaactggtgaaaccccaatggttcattatgatttgaat ggtccaagaggcaaatttagcaagttcagattgccagttcacgttagaag atctaatttcaaattaccaaagaatagcactaccccagttattttgattg gtccaggtaccggtgttgcaccattgagaggttttgttagagaaagagtt caacaagttaaaaatggtgttaatgttggtaagactgtattgttttatgg atgtagaaattccgaacaagatttcttgtacaaacaagaatggagtgaat atgcctcagtattgggagaaaatttcgaaatgtttaatgccttctcaaga caagatccaactaagaaagtttatgttcaagataagattttagaaaatag |
| | | tgctcttgttgatgagttattatctagtggagcaattatttatgtttgtg gtgatgccagtagaatggctagagatgttcaagctgcaattgccaagatt gttgccaaaagtagagatatccacgaagataaagctgctgaattggttaa atcttggaaagttcaaaatagataccaagaagatgtctggtaa |
| SEQ ID NO:25 | NADPH cytochrome P450 reductase A, CPRA (C. tropicalis strain ATCC20336) | atggctttagacaagttagatttgtatgtcatcataacattggtggtcgc tgtagccgcctattttgctaagaaccagttccttgatcagccccaggaca ccgggttcctcaacacggacagcggaagcaactccagagacgtcttgctg acattgaagaagaataataaaaacacgttgttgtttgggtcccagac gggtacggcagaagattacgccaacaaattgtccagagaattgcactcca gatttggcttgaaaacgatggttgcagatttcgctgattacgattgggat aacttcggagatatcaccgaagacatcttggtgtttttcattgttgccac ctatggtgagggtgaacctaccgataatgccgacgagttccacacctggt tgactgaagaagctgacactttgagtaccttgaaatacaccgtgttcggg ttgggtaactccacgtacgagttcttcaatgccattggtagaaagtttga cagattgttgagcgagaaaggtggtgacaggtttgctgaatacgctgaag gtgatgacggtactggcaccttggacgaagatttcatggcctggaaggac aatgtctttgacgccttgaagaatgatttgaactttgaagaaaaggaatt gaagtacgaaccaaacgtgaaattgactgagagagacgacttgtctgctg ctgactcccaagtttccttgggtgagccaaacaagaagtacatcaactcc gagggcatcgacttgaccaagggtccattcgaccacacccacccatactt ggccagaatcaccgagacgagagagttgttcagctccaaggacagacact gtatccacgttgaatttgacatttctgaatcgaacttgaaatacaccacc ggtgaccatctagctatctggccatccaactccgacgaaaacattaagca atttgccaagtgtttcggattggaagataaactcgacactgttattgaat tgaaggcgttggactccacttacaccatcccattcccaaccccaattacc tacggtgctgtcattagacaccatttagaaatctccggtccagtctcgag acaattcttttgtcaattgctgggtttgctcctgatgaagaaacaaaga aggcttttaccagacttggtggtgacaagcaagaattcgccgccaaggtc acccgcagaaagttcaacattgccgatgccttgttatattcctccaacaa cgctccatggtccgatgttccttttgaattccttattgaaaacgttccac acttgactccacgttactactccatttcgtcttcgtcattgagtgaaaag caactcatcaacgttactgcagttgttgaagccgaagaagaagctgatgg cagaccagtcactggtgttgtcaccaacttgttgaagaacgttgaaattg tgcaaaacaagactggcgaaaagccacttgtccactacgatttgagcggc ccaagaggcaagttcaacaagttcaagttgccagtgcatgtgagaagatc caactttaagttgccaaagaactccaccaccccagttatcttgattggtc caggtactggtgttgccccattgagaggttttgtcagagaaagagttcaa caagtcaagaatggtgtcaatgttggcaagactttgttgttttatggttg cagaaactccaacgaggacttttttgtacaagcaagaatggccgagtacg cttctgttttgggtgaaaactttgagatgttcaatgccttctccagacaa gacccatccaagaaggtttacgtccaggataagattttagaaaacagcca acttgtgcacgagttgttgactgaaggtgccattatctacgtctgtggtg atgccagtagaatggctagagacgtgcagaccacaatttccaagattgtt gctaaaagcagagaaattagtgaagacaaggctgctgaattggtcaagtc ctggaaggtccaaaatagataccaagaagatgtttggtag |
| SEQ ID NO:26 | NADPH cytochrome P450 reductase B, CPRB (C. tropicalis strain ATCC20336) | atggctttagacaagttagatttgtatgtcatcataacattggtggtcgc tgtggccgcctattttgctaagaaccagttccttgatcagccccaggaca ccgggttcctcaacacggacagcggaagcaactccagagacgtcttgctg acattgaagaagaataataaaaacacgttgttgttgtttgggtcccagac cggtacggcagaagattacgccaacaaattgtcaagagaattgcactcca gatttggcttgaaaaccatggttgcagatttcgctgattacgattgggat aacttcggagatatcaccgaagatatcttggtgtttttcatcgttgccac ctacggtgagggtgaacctaccgacaatgccgacgagttccacacctggt tgactgaagaagctgacactttgagtactttgagatataccgtgttcggg ttgggtaactccacctacgagttcttcaatgctattggtagaaagtttga cagattgttgagtgagaaaggtggtgacagatttgctgaatatgctgaag gtgacgacggcactggcaccttggacgaagatttcatggcctggaaggat aatgtctttgacgccttgaagaatgacttgaactttgaagaaaaggaatt gaagtacgaaccaaacgtgaaattgactgagagagatgacttgtctgctg ccgactcccaagtttccttgggtgagccaaacaagaagtacatcaactcc gagggcatcgacttgaccaagggtccattcgaccacacccacccatactt ggccaggatcaccgagaccagagagttgttcagctccaaggaaagacact gtattcacgttgaatttgacatttctgaatcgaacttgaaatacaccacc ggtgaccatctagccatctggccatccaactccgacgaaaacatcaagca |
| | | atttgccaagtgtttcggattggaagataaactcgacactgttattgaat tgaaggcattggactccacttacaccattccattcccaactccaattact tacggtgctgtcattagacaccatttagaaatctccggtccagtctcgag acaattctttttgtcgattgctgggtttgctcctgatgaagaaacaaaga agactttcaccagacttggtggtgacaaacaagaattcgccaccaaggtt acccgcagaaagttcaacattgccgatgccttgttatattcctccaacaa cactccatggtccgatgttccttttgagttccttattgaaaacatccaac acttgactccacgttactactccatttcttcttcgtcgttgagtgaaaaa caactcatcaatgttactgcagtcgttgaggccgaagaagaagccgatgg cagaccagtcactggtgttgttaccaacttgttgaagaacattgaaattg cgcaaaacaagactggcgaaaagccacttgttcactacgatttgagcggc ccaagaggcaagttcaacaagttcaagttgccagtgcacgtgagaagatc caactttaagttgccaaagaactccaccaccccagttatcttgattggtc caggtactggtgttgccccattgagaggtttcgttagagaaagagttcaa caagtcaagaatggtgtcaatgttggcaagactttgttgttttatggttg cagaaactccaacgaggactttttgtacaagcaagaatgggccgagtacg cttctgttttgggtgaaaactttgagatgttcaatgccttctctagacaa gacccatccaagaaggtttacgtccaggataagattttagaaaacagcca acttgtgcacgaattgttgaccgaaggtgccattatctacgtctgtggtg acgccagtagaatggccagagacgtccagaccacgatctccaagattgtt gccaaaagcagagaaatcagtgaagacaaggccgctgaattggtcaagtc ctggaaagtccaaaatagataccaagaagatgtttggtag |
| SEQ ID NO:41 | cytochrome P450:NADPH P450 reductase (Bacillus megaterium) amino acid [P450 activity shown in italics, P450 reductase activity shown in normal font] | mtikempqpktfgelknlpllntdkpvqalmkiadelgeifkfeapgrvt rylssqrlikeacdesrfdknlsqalkfvrdfagdglftswtheknwkka hnillpsfsqqamkgyhammvdiavqlvqkwerlnadehievpedmtrlt ldtiglcgfnyrfnsfyrdqphpfitsmvrasdeamnksqranpddpayd enkrqfqedikvmndlvdkiiadrkasgeqsddllthmlngkdpetgepl ddeniryqiitfliaghettsgllsfasyflvknphvlqkaaeeaarvlv dpvpsykqvkqlkyvgmvlneasrlwptapafslyakedtvlggeyplek gdelmvsipqlhrdktiwgddveefrperfenpsaipqhafkpfgngqra cigqqfalheatsvlgmmlkhfdfedhtnyesdiketltlkpegfvvkak skkiplggipspsteqsakkvrkkaenahntpslvlygsnmgtaegtard ladiamskgfapqvatldshagnlpregavlivtasynghppdnakqfvd wldqasadevkgvrysvfgcgdknwattyqkvpafidetlaakgaeniad rgeadasddfegtyeewrehmwsdvaayfnldiensednkstlslqfvds aadmplakmhgafstnvvaskelqqpgsarstrhleielpkeasyqegdh lgviprnyegivnrvtarfgldasqqirseaeeeklahlplaktvsveel sqyvelqdpvtrtqlramaaktvcpphkveleallekqaykeqvsakrlt mleslekypacemkfsefialspsirpryysisssprvdekqasitvsvv sgeawsgygeykgiasnylaesqegdtitcfistpqseftspkdpetpli mvgpgtgvapfrgfvqarkqlkeqgqslgeahlyfgcrsphedysyqeel enaqsegiitlhtafsrmpnqpktyvqhvmeqdgkklielldqgahfyic gdgsqmapaveatlmksyadvhqvseadarlwsqqleekgryakdvwag* |

### Example 32: Amplification of Selected Beta Oxidation Activities

As noted previously, beta oxidation of fatty acids involves two acyl CoA oxidase activities, encoded by POX4 and POX5. Figures 15A-15C presented data regarding distribution of fatty acids present in yeast strains that were wild type with respect to acyl CoA oxidase activity (e.g., POX4:POX5, see Figure 15A), disrupted for POX5 (e.g., POX4:pox5), or disrupted for POX4 (e.g., pox4:POX5). Wild-type C. tropicalis has two copies of POX4 and two copies of POX5. The POX4 enzyme has broad substrate specificity (see Figure 15B), whereas the POX5 enzyme (see Figure 15C) has a narrow substrate specificity with regard to fatty acid chain length. A partially beta oxidation disrupted strain was generated that contained no POX4 activity (both alleles are disrupted), and two functional copies of POX5, as described herein. The results of experiments conducted with the pox4:pox4/POX5:POX5 strain indicated that since the activity of the POX5 enzyme drops off dramatically with fatty acids shorter than C10, the beta-oxidation products of the POX5 strain are the C8 diacid and C6 diacid (adipic). Additionally, if the strain is starved for carbon (e.g., the only possible source of energy is the C8 diacid), it will convert the C8 diacid to the C6 diacid. Data illustrating that amplification of the number of POX 5 genes in the engineered organism to Increase beta oxidation activity, increases the C6/C8 ratio, is presented below and shown in Figure 38.

### Construction and shake flask evaluation of POX5 amplified strains

### Plasmid pAA166 (P_{POX4}POX5T_{POX4})

A PCR product containing the nucleotide sequence of POX5 was amplified from C. tropicalis 20336 genomic DNA using primers oAA540 and oAA541. The PCR product was gel purified and ligated into pCR-Blunt II-TOPO (Invitrogen), transformed into competent TOP10 E. coli cells (Invitrogen) and clones containing PCR inserts were sequenced to confirm correct DNA sequence. One such plasmid was designated, pAA165. Plasmid pAA165 was digested with BspQI and a 2-kb fragment was isolated. Plasmid pAA073 which contained a POX4 promoter and POX4 terminator was also digested with BspQI and gel purified. The isolated fragments were ligated together to generate plasmid pAA166. Plasmid pAA166 contains a P_{POX4}POX5T_{POX4} fragment.

### Plasmid pAA204 (thiolase deletion construct)

A PCR product containing the nucleotide sequence of a short-chain thiolase (e.g., acetyl-coA acetyltransferase) was amplified from C. tropicalis 20336 genomic DNA using primers oAA640 and oAA641. The PCR product was gel purified and ligated into pCR-Blunt II-TOPO (Invitrogen), transformed into competent TOP10 E. coli cells (Invitrogen) and clones containing PCR inserts were sequenced to confirm correct DNA sequence. One such plasmid was designated, pAA184. A URA3 PCR product was amplified from pAA061 using primers oAA660 and oAA661. The PCR product was gel purified and ligated into pCR-Blunt II-TOPO (Invitrogen), transformed as described and clones containing PCR inserts were sequenced to confirm the correct DNA sequence. One such plasmid was designated pAA192. Plasmid pAA184 was digested with Bglll/Sall and gel purified. Plasmid pAA192 was digested with Bglll/Sall and a 1.5 kb fragment was gel purified. The isolate fragments were ligated together to create pAA199. An alternative P_{URA3} PCR product was amplified from plasmid pAA061 using primers oAA684 and oAA685. The PCR product was gel purified and ligated into pCR-Blunt II-TOPO (Invitrogen), transformed as described and clones containing PCR inserts were sequenced. One such plasmid was designated, pAA201. Plasmid pAA199 was digested with Sall and gel purified. Plasmid pAA201 was digested with Sall and a 0.43 kb P_{URA3} was gel purified. The isolated fragments were ligated to create plasmid pAA204 that contains a direct repeat of P_{URA3}.

### Plasmid pAA221 (P_{POX4}POX5T_{POX4} in thiolase deletion construct)

A PCR product containing the nucleotide sequence of P_{POX4}POX5T_{POX4} was amplified from plasmid pAA166 DNA using primers oAA728 and oAA729. The PCR product was gel purified and ligated into pCR-Blunt II-TOPO, transformed as described and clones containing PCR inserts were sequenced to confirm the sequence of the insert. One such plasmid was designated, pAA220. Plasmid pAA204 was digested with BglII, treated with shrimp alkaline phosphatase (SAP), and a 6.5 kb fragment was gel purified. Plasmid pAA220 was digested with BglII and a 2.7 kb fragment containing P_{POX4}POX5T_{POX4} was gel purified. The isolated fragments were ligated to create plasmid pAA221.

### Strain sAA617 (P_{POX4}POX5T_{POX4} in sAA451)

Strain sAA451 is a ura-, partially β-oxidation blocked strain (ura3/ura3 pox4a::ura3/pox4b::ura3 POX5/POX5). Plasmid pAA221 was digested with EcoRI to release a DNA fragment containing P_{POX4}POX5T_{POX4} in a thiolase deletion construct. The DNA was column purified and transformed to strain sAA451 to plate on SCD-ura plate. After two days, colonies were streaked out on YPD plates, single colonies selected and again streaked out on YPD plates. Single colonies were selected from the second YPD plates and characterized by colony PCR. The insertion of P_{POX4}POX5T_{POX4} in strain sAA451, disrupting the short-chain thiolase gene, was confirmed by PCR and one such strain was designated sAA617.

### Strain sAA620

Strain sAA617 was grown overnight on YPD medium and plated on SCD+URA+5-FOA, to select for loop-out of URA3. Colonies were streaked out onto YPD plates twice as described for strain sAA617, and single colonies characterized by colony PCR. The loop-out of URA3 by direct repeats of PURA3 was confirmed by PCR. One such strain was designated sAA620. Strain sAA620 has one additional copy of POX5 under control of the POX4 promoter.

### Plasmid pAA156

A PCR product containing the nucleotide sequence of CYP52A19 was amplified from C. tropicalis strain 20336 genomic DNA, using primers oAA525 and oAA526. The PCR product was gel purified and ligated into pCR-Blunt II-TOPO, transformed as described, and clones containing PCR inserts were sequenced to confirm correct DNA sequence. One such plasmid was designated, pAA144. Plasmid pAA144 was digested with BspQl and a 1.7-kb fragment was isolated. Plasmid pAA073, which includes a POX4 promoter and POX4 terminator, also was digested with BspQl and gel purified. The isolated fragments were ligated together to generate plasmid, pAA156. Plasmid pAA156 included P_{POX4}CYP52A19T_{POX4} fragment and URA3.

### Strain sAA496

Plasmid pAA156 was digested with Clal and column purified. Strain sAA451 was transformed with this linearized DNA and plated on SCD-ura plate. Colonies were checked for CYP52A19 integration. Colonies positive for plasmid integration were further analyzed by qPCR to determine the number of copies of CYP52A19 integrated. One such strain, designated sAA496 contained about 13 copies of the monooxygenase activity encoded by CYP52A19.

### Strains sAA632 and sAA635

Strain sAA620 was transformed with linearized pAA156 DNA and plated on SCD-ura plates. Several colonies were checked for CYP52A19 integration. Colonies positive for plasmid integration were further analyzed by qPCR to determine the number of copies of CYP52A19 integrated. One such strain, designated sAA632 contained about 27 copies of the monooxygenase activity encoded by CYP52A19. Another strain, designated sAA635, contained about 12 copies of the monooxygenase activity encoded by CYP52A19.

### Shake flask characterization of sAA496, sAA632 and sAA635

250mL glass bottom-baffled flasks containing 50mL of SP92 media were inoculated with a 5mL overnight YPD culture (OD = 0.4). After 24h incubation at 30°C, with shaking at 250rpm (2" throw incubator), sterile antifoam B was added to a final concentration of 0.1 % to dissipate foam. The cells were centrifuged and the cell pellet resuspended in 20mL of TB-lowN media (yeast nitrogen base without amino acids and without ammonium sulfate, 1.7g/L; yeast extract, 3.0g/L; potassium phosphate monobasic, 1.0g/L; potassium phosphate dibasic, 1.0g/L). 1.6mL coconut oil containing 20uL sterile antifoam B was added to start adipic acid production in cultures grown at 30°C, with shaking. Samples were taken every 24 hour for gas chromatographic (GC) analysis.

Shown in the table below and in Figure 38, are the diacid profiles for strains described herein, at various time points. Strains with an additional copy of the POX5 gene show an increased proportion of C6 in the C6 + C8 diacid pool at early time points of the analysis. An increased proportion of C6 at early time points may indicate that the additional copy of POX5 in these strains increases beta-oxidation activity, allowing chain shortening of the coconut oil feedstock to the C6 diacid rather than to the C8 diacid. At the 144h time point, the strain without an additional copy of POX5 has the same proportion of C6 in the C6 + C8 diacid pool.

| **Strain name** | **time** | **C6 (g/L)** | **C6+C8 (g/L)** | **C6/(C6+C8)** |
|---|---|---|---|---|
| sAA496 | 24h | 0.23 | 1.11 | 20% |
| sAA632 | 24h | 0.39 | 1.14 | 34% |
| sAA635 | 24h | 0.34 | 0.98 | 35% |
| sAA496 | 48h | 1.4 | 2.69 | 52% |
| sAA632 | 48h | 2.34 | 3.09 | 76% |
| sAA635 | 48h | 1.86 | 2.75 | 68% |
| sAA496 | 72h | 2.6 | 4.24 | 61% |
| sAA632 | 72h | 3.6 | 5.1 | 71% |
| sAA635 | 72h | 2.78 | 4.27 | 65% |
| sAA496 | 144h | 7.70 | 7.86 | 98% |
| sAA632 | 144h | 8.55 | 8.79 | 97% |
| sAA635 | 144h | 7.92 | 8.23 | 96% |

Strains described herein (e.g., partially beta-oxidation blocked, increased monooxygenase activity (e.g., SEQ ID NO: 20 amplified) and increased POX5 activity) have produced greater than 50g/L of adipic acid under fermentation conditions using coconut oil as the feedstock. In some embodiments, strains described herein have 1 or more, 5 or more, 10 or more, 15 or more, 20 or more, or 25 or more additional copies a nucleotide sequences encoding a monooxygenase activity, a monooxygenase reductase activity, and/or a POX5 activity as compared to a strain native for monooxygenase activity, monooxygenase reductase activity, and/or POX5 activity. In certain embodiments, strains described herein have 2 times or more, 5 times or more, 10 times or more, 15 times or more, 20 times or more, or 25 times or more monooxygenase activity, monooxygenase reductase activity, and/or a POX5 activity, as compared to a strain native for monooxygenase activity, monooxygenase reductase activity, and/or POX5 activity.

The polynucleotide sequences of the POX4 and POX5 genes isolated as described herein are presented below as SEQ ID NOS: 37 and 38, respectively. The amino acid sequences encoded by the polynucleotides of SEQ ID NOS 37 and 38, are presented as SEQ ID NOS: 39 and 40, respectively.

| SEQ ID NO | Description | Sequence |
|---|---|---|
| SEQ ID NO:37 | acyl CoA oxidase, POX4 (C. tropicalis strain ATCC20336) nucleotide | ATGACTTTTACAAAGAAAAACGTTAGTGTATCACAAGGTCCTGACCC TAGATCATCCATCCAAAAGGAAAGAGACAGCTCCAAATGGAACCCTC AACAAATGAACTACTTCTTGGAAGGCTCCGTCGAAAGAAGTGAGTTG ATGAAGGCTTTGGCCCAACAAATGGAAAGAGACCCAATCTTGTTCAC AGACGGCTCCTACTACGACTTGACCAAGGACCAACAAAGAGAATTGA CCGCCGTCAAGATCAACAGAATCGCCAGATACAGAGAACAAGAATCC ATCGACACTTTCAACAAGAGATTGTCCTTGATTGGTATCTTTGACCC ACAGGTCGGTACCAGAATTGGTGTCAACCTCGGTTTGTTCCTTTCTT GTATCAGAGGTAACGGTACCACTTCCCAATTGAACTACTGGGCTAAC GAAAAGGAAACCGCTGACGTTAAAGGTATCTACGGTTGTTTCGGTAT GACCGAATTGGCCCACGGTTCCAACGTTGCTGGTTTGGAAACCACCG CCACATTTGACAAGGAATCTGACGAGTTTGTCATCAACACCCCACAC ATTGGTGCCACCAAGTGGTGGATTGGTGGTGCTGCTCACTCCGCCAC CCACTGTTCTGTCTACGCCAGATTGATTGTTGACGGTCAAGATTACG GTGTCAAGACTTTTGTTGTCCCATTGAGAGACTCCAACCACGACCTC ATGCCAGGTGTCACTGTTGGTGACATTGGTGCCAAGATGGGTAGAGA TGGTATCGATAACGGTTGGATCCAATTCTCCAACGTCAGAATCCCAA GATTCTTTATGTTGCAAAAGTTCTGTAAGGTTTCTGCTGAAGGTGAA GTCACCTTGCCACCTTTGGAACAATTGTCTTACTCCGCCTTGTTGGG TGGTAGAGTCATGATGGTTTTGGACTCCTACAGAATGTTGGCTAGAA TGTCCACCATTGCCTTGAGATACGCCATTGGTAGAAGACAATTCAAG GGTGACAATGTCGATCCAAAAGATCCAAACGCTTTGGAAACCCAATT GATAGATTACCCATTGCACCAAAAGAGATTGTTCCCATACTTGGCTG CTGCCTACGTCATCTCCGCTGGTGCCCTCAAGGTTGAAGACACCATC CATAACACCTTGGCTGAATTGGACGCTGCCGTTGAAAAGAACGACAC CAAGGCTATCTTTAAGTCTATTGACGACATGAAGTCATTGTTTGTTG ACTCTGGTTCCTTGAAGTCCACTGCCACTTGGTTGGGTGCTGAAGCC ATTGACCAATGTAGACAAGCCTGTGGTGGTCACGGTTACTCGTCCTA |
| | | CAACGGCTTCGGTAAAGCCTACAACGATTGGGTTGTCCAATGTACTT GGGAAGGTGACAACAATGTCTTGGCCATGAGTGTTGGTAAGCCAATT GTCAAGCAAGTTATCAGCATTGAAGATGCCGGCAAGACCGTCAGAGG TTCCACCGCTTTCTTGAACCAATTGAAGGACTACACTGGTTCCAACA GCTCCAAGGTTGTTTTGAACACTGTTGCTGACTTGGACGACATCAAG ACTGTCATCAAGGCTATTGAAGTTGCCATCATCAGATTGTCCCAAGA AGCTGCTTCTATTGTCAAGAAGGAATCTTTCGACTATGTCGGCGCTG AATTGGTTCAACTCTCCAAGTTGAAGGCTCACCACTACTTGTTGACT GAATACATCAGAAGAATTGACACCTTTGACCAAAAGGACTTGGTTCC ATACTTGATCACCCTCGGTAAGTTGTACGCTGCCACTATTGTCTTGG ACAGATTTGCCGGTGTCTTCTTGACTTTCAACGTTGCCTCCACCGAA GCCATCACTGCTTTGGCCTCTGTGCAAATTCCAAAGTTGTGTGCTGA AGTCAGACCAAACGTTGTTGCTTACACCGACTCCTTCCAACAATCCG ACATGATTGTCAATTCTGCTATTGGTAGATACGATGGTGACATCTAT GAGAACTACTTTGACTTGGTCAAGTTGCAGAACCCACCATCCAAGAC CAAGGCTCCTTACTCTGATGCTTTGGAAGCCATGTTGAACAGACCAA CCTTGGACGAAAGAGAAAGATTTGAAAAGTCTGATGAAACCGCTGCT ATCTTGTCCAAGTAA |
| SEQ ID NO:39 | acyl CoA oxidase, POX4 (C. tropicalis strain ATCC20336) amino acid | MTFTKKNVSVSQGPDPRSSIQKERDSSKWNPQQMNYFLEGSVERSEL MKALAQQMERDPILFTDGSYYDLTKDQQRELTAVKINRIARYREQES IDTFNKRLSLIGIFDPQVGTRIGVNLGLFLSCIRGNGTTSQLNYWAN EKETADVKGIYGCFGMTELAHGSNVAGLETTATFDKESDEFVINTPH IGATKWWIGGAAHSATHCSVYARLIVDGQDYGVKTFVVPLRDSNHDL MPGVTVGDIGAKMGRDGIDNGWIQFSNVRIPRFFMLQKFCKVSAEGE VTLPPLEQLSYSALLGGRVMMVLDSYRMLARMSTIALRYAIGRRQFK GDNVDPKDPNALETQLIDYPLHQKRLFPYLAAAYVISAGALKVEDTI HNTLAELDAAVEKNDTKAIFKSIDDMKSLFVDSGSLKSTATWLGAEA IDQCRQACGGHGYSSYNGFGKAYNDWVVQCTWEGDNNVLAMSVGKPI VKQVISIEDAGKTVRGSTAFLNQLKDYTGSNSSKVVLNTVADLDDIK TVIKAIEVAIIRLSQEAASIVKKESFDYVGAELVQLSKLKAHHYLLT EYIRRIDTFDQKDLVPYLITLGKLYAATIVLDRFAGVFLTFNVASTE AITALASVQIPKLCAEVRPNVVAYTDSFQQSDMIVNSAIGRYDGDIY ENYFDLVKLQNPPSKTKAPYSDALEAMLNRPTLDERERFEKSDETAA ILSK* |
| SEQ ID NO:38 | acyl CoA oxidase, POX5 (C. tropicalis strain ATCC20336) nucleotide | ATGCCTACCGAACTTCAAAAAGAAAGAGAACTCACCAAGTTCAACCC AAAGGAGTTGAACTACTTCTTGGAAGGTTCCCAAGAAAGATCCGAGA TCATCAGCAACATGGTCGAACAAATGCAAAAAGACCCTATCTTGAAG GTCGACGCTTCATACTACAACTTGACCAAAGACCAACAAAGAGAAGT CACCGCCAAGAAGATTGCCAGACTCTCCAGATACTTTGAGCACGAGT ACCCAGACCAACAGGCCCAGAGATTGTCGATCCTCGGTGTCTTTGAC CCACAAGTCTTCACCAGAATCGGTGTCAACTTGGGTTTGTTTGTTTC CTGTGTCCGTGGTAACGGTACCAACTCCCAGTTCTTCTACTGGACCA TAAATAAGGGTATCGACAAGTTGAGAGGTATCTATGGTTGTTTTGGT ATGACTGAGTTGGCCCACGGTTCCAACGTCCAAGGTATTGAAACCAC CGCCACTTTTGACGAAGACACTGACGAGTTTGTCATCAACACCCCAC ACATTGGTGCCACCAAGTGGTGGATCGGTGGTGCTGCGCACTCCGCC ACCCACTGCTCCGTCTACGCCAGATTGAAGGTCAAAGGAAAGGACTA CGGTGTCAAGACCTTTGTTGTCCCATTGAGAGACTCCAACCACGACC TCGAGCCAGGTGTGACTGTTGGTGACATTGGTGCCAAGATGGGTAGA GACGGTATCGATAACGGTTGGATCCAGTTCTCCAACGTCAGAATCCC AAGATTCTTTATGTTGCAAAAGTACTGTAAGGTTTCCCGTCTGGGTG AAGTCACCATGCCACCATCTGAACAATTGTCTTACTCGGCTTTGATT GGTGGTAGAGTCACCATGATGATGGACTCCTACAGAATGACCAGTAG ATTCATCACCATTGCCTTGAGATACGCCATCCACAGAAGACAATTCA AGAAGAAGGACACCGATACCATTGAAACCAAGTTGATTGACTACCCA TTGCATCAAAAGAGATTGTTCCCATTCTTGGCTGCCGCTTACTTGTT |
| | | CTCCCAAGGTGCCTTGTACTTAGAACAAACCATGAACGCAACCAACG ACAAGTTGGACGAAGCTGTCAGTGCTGGTGAAAAGGAAGCCATTGAC GCTGCCATTGTCGAATCCAAGAAATTGTTCGTCGCTTCCGGTTGTTT GAAGTCCACCTGTACCTGGTTGACTGCTGAAGCCATTGACGAAGCTC GTCAAGCTTGTGGTGGTCACGGTTACTCGTCTTACAACGGTTTCGGT AAAGCCTACTCCGACTGGGTTGTCCAATGTACCTGGGAAGGTGACAA CAACATCTTGGCCATGAACGTTGCCAAGCCAATGGTTAGAGACTTGT TGAAGGAGCCAGAACAAAAGGGATTGGTTCTCTCCAGCGTTGCCGAC TTGGACGACCCAGCCAAGTTGGTTAAGGCTTTCGACCACGCCCTTTC CGGCTTGGCCAGAGACATTGGTGCTGTTGCTGAAGACAAGGGTTTCG ACATTACCGGTCCAAGTTTGGTTTTGGTTTCCAAGTTGAACGCTCAC AGATTCTTGATTGACGGTTTCTTCAAGCGTATCACCCCAGAATGGTC TGAAGTCTTGAGACCTTTGGGTTTCTTGTATGCCGACTGGATCTTGA CCAACTTTGGTGCCACCTTCTTGCAGTACGGTATCATTACCCCAGAT GTCAGCAGAAAGATTTCCTCCGAGCACTTCCCAGCCTTGTGTGCCAA GGTTAGACCAAACGTTGTTGGTTTGACTGATGGTTTCAACTTGACTG ACATGATGACCAATGCTGCTATTGGTAGATATGATGGTAACGTCTAC GAACACTACTTCGAAACTGTCAAGGCTTTGAACCCACCAGAAAACAC CAAGGCTCCATACTCCAAGGCTTTGGAAGACATGTTGAACCGTCCAG ACCTTGAAGTCAGAGAAAGAGGTGAAAAGTCCGAAGAAGCTGCTGAA ATCTTGTCCAGTTAA |
| SEQ ID NO:40 | acyl CoA oxidase, POX5 (C. tropicalis strain ATCC20336) amino acid | MPTELQKERELTKFNPKELNYFLEGSQERSEIISNMVEQMQKDPILK VDASYYNLTKDQQREVTAKKIARLSRYFEHEYPDQQAQRLSILGVFD PQVFTRIGVNLGLFVSCVRGNGTNSQFFYWTINKGIDKLRGIYGCFG MTELAHGSNVQGIETTATFDEDTDEFVINTPHIGATKWWIGGAAHSA THCSVYARLKVKGKDYGVKTFVVPLRDSNHDLEPGVTVGDIGAKMGR DGIDNGWIQFSNVRIPRFFMLQKYCKVSRSGEVTMPPSEQLSYSALI GGRVTMMMDSYRMTSRFITIALRYAIHRRQFKKKDTDTIETKLIDYP LHQKRLFPFLAAAYLFSQGALYLEQTMNATNDKLDEAVSAGEKEAID AAIVESKKLFVASGCLKSTCTWLTAEAIDEARQACGGHGYSSYNGFG KAYSDWVVQCTWEGDNNILAMNVAKPMVRDLLKEPEQKGLVLSSVAD LDDPAKLVKAFDHALSGLARDIGAVAEDKGFDITGPSLVLVSKLNAH RFLIDGFFKRITPEWSEVLRPLGFLYADWILTNFGATFLQYGIITPD VSRKISSEHFPALCAKVRPNVVGLTDGFNLTDMMTNAAIGRYDGNVY EHYFETVKALNPPENTKAPYSKALEDMLNRPDLEVRERGEKSEEAAE ILSS* |

### Example 33: Amplification of Lipase Activity and Analysis of Strains with Increased Lipase Activity

The C. tropicalis genome contains multiple genes which encode lipase activities. A lipase activity, carried out by a lipase enzyme, liberates fatty acids from a glycerol backbone. This activity is particularly beneficial when using oils (e.g., plant based oils, coconut oil) as the culture feedstock. Amplification of endogenous lipase activity coding sequences in an engineered organism has been shown to improve the level of adipic acid production with respect to a corresponding strain with a native level of lipase activity. Without being limited by any theory, it is believed the increased adipic acid titers seen are the result of improved substrate utilization.

### Cloning of C. tropicalis strain lipase activity coding sequences

BLAST searches were conducted in the C. tropicalis strain ATCC20962 genomic database using the gene sequence of a lipase activity encoded by Yarrowia lipolytica (LIP2; GenBank Accession # CAB91111.1). The corresponding C. tropicalis sequence was cloned under the control of POX4 or PGK promoters as follows.

Primers were generated to clone the identified C. tropicalis lipase homolog into vectors pAA73 (POX4 promoter) and pAA105 (PGK promoter), respectively. For cloning the lipase activity into pAA73 vector, the nucleotide sequence encoding the lipase activity was PCR amplified from C. *tropicalis* strain ATCC20336 using primers oAA734 and oAA735 in a reaction containing 5uL 10X buffer, 2.0uL of oAA734 and 2 uL of oAA735 (10uM), 1.0uL genomic DNA, 1.0uL of dNTPs, 1.0uL of Pfu and 38uL sterile H2O. The therrmocycling parameters used were 95°C for 2 minutes, 30 cycles of 95°C 20 seconds, 55°C 30 seconds, 72°C 1 minute, followed by 72°C 5 minutes and a 4°C hold. PCR product of the correct size was gel purified, ligated into pCR-Blunt II-TOPO and transformed into competent TOP10 *E. coli* cells (Invitrogen). Clones containing PCR inserts were sequenced to confirm correct DNA sequence. The resulting plasmid was designated, pAA234. Plasmid pAA234 was restriction enzyme digested with XmaI and XbaI, according to manufacturer's recommendations. DNA fragments were separated on a 0.8% agarose gel. The DNA fragment containing the nucleotide sequence encoding lipase activity was gel purified and ligated into XmaI/XbaI digested vector pAA073, and transformed into TOP10 *E. coli* cells (Invitrogen). Correct plasmid structure, including the presence of the fragment carrying lipase activity, was confirmed by restriction digestion and designated as pAA235. pAA235 includes the lipase activity coding sequence under control of the POX4 promoter.

For cloning the lipase activity into the pAA105 (PGK promoter) vector, PCR amplification of the lipase activity was performed as above using primers oAA736 and oAA737. PCR product was gel purified, digested with SapI, ligated into pAA105 vector and transformed into TOP10 *E. coli* cells (Invitrogen). Correct plasmid structure, including the presence of the fragment carrying lipase activity, was confirmed by restriction digestion and designated as pAA236. pAA236 includes the lipase activity coding sequence under control of the PGK promoter. The lipase activity cloned into plasmids pAA235 and pAA236 was analyzed to determine the presence or absence of any potential secretion leader sequences. The N-terminal protein secretion leader sequence was predicted by SignalP (SignalP 3.0 Server) and the predicted cleavage site of the lipase activity leader sequence is most likely between pos. 15 and 16: SSA-GK, as shown in the amino acid sequence listing presented in the table below. The nucleotide sequence of the lipase activity cloned into pAA235 and pAA236 also is presented in the table below.

| SEQ ID NO | Description | Sequence |
|---|---|---|
| SEQ ID NO:27 | C. Tropicalis lipase activity, (identified using Y. lipolytica BLAST search) Nucleotide Seq | ATGATTGTTTTATTCATCCTTGTATTTATTTGTCTATCTTCAGCCGGG AAACCAAACAAACCAGAAGCTCCAGCAAAAGATTACATCAAACTCGTT GAATTCTCCAATTTTGCCGCCGTTGGCTACTGCGTTAATAGAGGTCTA GCAAAGGGCCGTCTAGGAGACGAGGACTCCAACTGTGCCTTGTTGGCA TGCAAGAACGACTTCCTTGCGGACGTCGAGATTATTAAGATATTTGAC TTCAACCGTCTTAATGAAGTTGGAACAGGTTACTATGCCTTGGACAGG AAGAGAAAGGCAATAATATTGGTATTTAGAGGGTCTGTCTCCCGACGT GACTGGGCGACAGACATGGATTTCATCCCCACTTCTTACAAGCCAATT GTGTATGAGGAAAACTTTGGTTGTGACCCCTACATTCTGACCGAATGC AAGAACTGTCGTGTGCACCGTGGTTTCTACAATTTCTTGAAGGATAAC TCTGCAGCAATTATCACCGAGGGAATTGCGTTGAAAGAAGAGTACCCG GACTACCAGTTCTTGATCATTGGTCATTCTTTGGGCGCTGCCTTGACA ATGTTGAGTGGCATCGAGTTCCAGTTGTTGGGGTACGATCCTTTGGTG GTGACTTATGGTGGTCCAAAGGTGGGCAACCAAGAGTTTGCTGACTTC ACGGACAACTTGTTTGACACGGATGAGGTGGACAATGAAATCGCCACC AACCGTGATTTTTCAAGAGGATTCATTAGAGTGGTACACAAGTATGAT ATAATACCATTCTTGCCGCCGTTGTTTAGTCACGCAGGGTACGAATAC TTTATTGACAAGAGAGAGTTGCCCCATGAAGAATGTGATTTGGACAGA CGAGGCATGGAGTACTCGGGGATATTTAAGAGATCGCTGACCATAAAA CCGTCCACTTTATGGCCAGATAGGTTGGGGAAGTATGAACATACACAT TATTTTAGAAGAATCACTAGTTGTAGGGACGACGAT |
| SEQ ID NO:28 | C. Tropicalis lipase activity, (identified using Y. lipolytica BLAST search) Amino Acid Seq | MIVLFILVFICLSSAGKPNKPEAPAKDYIKLVEFSNFAAVGYCVNRGL AKGRLGDEDSNCALLACKNDFLADVEIIKIFDFNRLNEVGTGYYALDR KRKAIILVFRGSVSRRDWATDMDFIPTSYKPIVYEENFGCDPYILTEC KNCRVHRGFYNFLKDNSAAIITEGIALKEEYPDYQFLIIGHSLGAALT MLSGIEFQLLGYDPLVVTYGGPKVGNQEFADFTDNLFDTDEVDNEIAT NRDFSRGFIRVVHKYDIIPFLPPLFSHAGYEYFIDKRELPHEECDLDR RGMEYSGIFKRSLTIKPSTLWPDRLGKYEHTHYFRRITSCRDDD |

Plasmids pAA235 and pAA236 were linearized with Clal and transformed into competent *C*. *tropicalis* cells of strain sAA329 (URA3 auxotroph). The integration of the linearized plasmids was confirmed by colony PCR using primers oAA734 and oAA281 for the pAA235 integration and oAA736 and oAA281 for the pAA236 integration, respectively. Two transformants from each transformation were selected. Strains from the pAA235 integration were designated as sAA574 and sAA575. Strains from the pAA236 integration were designated as sAA580 and sAA581. The strains were used to evaluate the effects of increased lipase activity on adipic acid production. One colony of each strain was inoculated into 5 mL SP92 and grown overnight at 30°C with shaking at about 200 rpm. The overnight culture was used to inoculate 50 mL of SP92 medium, in shake flasks (e.g., about OD₆₀₀ₙₘ 0.4), and incubated under the same condition for 24 hours. Cells were harvested and resuspended in 20 mL of TB-lowN medium supplemented with 1.6 mL of coconut oil and 20uL sterile antifoam B. Samples were taken daily and analyzed for diacid production by gas chromatography. The results are shown graphically in Figure 39. As shown in Figure 39, 3 of the 4 strains with increased copy number of the lipase activity nucleotide sequence tested showed increased levels of adipic acid production, when compared to the parental strain. The results shown in Figure 39 represent the data from six days of fermentation in SP92 medium supplemented with coconut oil as the carbon source.

A second BLAST search utilizing the nucleotide sequence of a lipase activity from C. dubliniensis also was performed in the C. tropicalis strain ATCC20962 database. The second BLAST search identified another C. tropicalis lipase activity homolog (e.g., ORF7657), with an amino acid sequence different from the lipase activity identified using the Y.lipolytica lipase activity sequence. The second lipase activity is being cloned and over-expressed in C. tropicalis for evaluation in fermentation of adipic acid. The amino acid sequence of the second lipase activity identified in C. tropicalis strain ATCC20962 using the C. dubliniensis lipase activity sequence in a BLAST search, is presented below as SEQ ID NO: 29.
SEQ ID NO: 29

MLRTVRHYSKVINIKDKGEKAARVITSEFAKLKDHYDAPKYPIVLCHGFSGFDRLG
LFPLPNLLEDTTATTKTKEITERSLIELDYWYGIKDALENLGSTVFIAKVPAFGDI
RSRAISLDKFIEKQCKALRQTESKSSIYNKPDSSNDDTTTFKDKHQPIKVNLISHS
MGGVDSRYLISRIHNDNENYRVASLTTILTPHHGSECADFIVDLIGDNGVLKKVCP
PSIYQLTTLHMKKFNEVVKDDPSVQYFSFGARFNPRWYNLFGLTWLVMKYQIEKEQ
ADRFKHMIDNDGLVSVESSKWGQYIGTLDEVDHLDLINWTNRARSVFDKVMFAQNP
NFNPIALYLEIADQLSKKGL

### Example 34: Amplification of acyl-CoA carboxylase activity

Acetyl-CoA carboxylase (e.g., ACC) catalyzes the reaction that produces Malonyl-CoA for fatty acid synthesis. The reaction catalyzed by ACC is the committed step in fatty acid synthesis. Strains engineered to convert glucose to adipic acid using a native, mutant, or specialized (e.g., hexanoate synthase) fatty acid synthase would benefit from increased carbon flux through the pathway, as contributed by increased acetyl-CoA carboxylase activity. The nucleotide sequence encoding ACC activity has been cloned and is being over expressed and evaluated for contribution to adipic acid conversion in C. tropicalis. The nucleotide sequence encoding the ACC activity from C. tropicalis strain ATCC 20336 is presented below as SEQ ID NO: 30.

atgagatgccaagtatctcaaccatcacgatttactaacttgcttgtacatagact
cccacgaacactacttaattatccagttgtaaataccctatttattcctagacgtc
attattcccttaatttttcattcaagaacctactaaagaaaatgacagatctttcc

ccaagtccaacagactcccttaattacacacagttgcactcatccttgccatcaca
tttcttaggtgggaactcggtgctcaccgctgagccttctgccgtgacagatttcg
tcaaaacacaccaaggtcacactgttatcaccaaagtcttgattgccaacaacggt
attggtgccgtcaaagaaataagatccgtcagaaaatgggcctacgaaacttttgg
tgacgaaagagctatacagtttgtcgccatggccactcccgaagatatggaggcta
acgccgagtacattcgaatggccgaccagtttgtcgaggtcccaggtggtaccaat
aacaacaactacgcgaatgttgacttgattgtcgaaatcgctgaaagaaccgatgt
ccacgccgtttgggctggttggggtcatgcctccgaaaaccctttgttgccagraa
ggttggcagcttcccctaagaagatcgtgtttattggtcctccagggtctgccatg
agatctttgggtgacaagatttcttccaccattgttgcacaacacgccaaagtgcc
atgtatcccatggtctggtactggtgtcgaagaggtccacgtcgacccagaaacca
agttggtgtctgttgacgaccacgtctacgccaaaggttgctgtacctcgccagaa
gacggtttggaaaaagccaaacgtatcggattcccagttatggttaaggcatccga
aggtggtggtggtaaaggtatcagaaaagtcgaccacgaaaaggacttcatcagtt
tgtacaaccaggcggctaacgaaataccaggtcaccaatttcatcatgaagttg
gccggtgacgccagacacttggaagtgcaattgtttgccgatcagtacggtaccaa
catttcgcttttcggtagagattgttctgtgcaaagaagacatcaaaagatcattg
aagaagctccagtcacaattgccaacaaagacacttttgttgagatggagaaagct
gccgtcagattgggtaagttggttggttacgtgtctgccggtaccgttgaatacct
ttactcctacgccgaagacaagttctacttttggaattgaacccaagattgcaag
ttgaacatccaactaccgaaatggtttccggtgtcaacttaccagccgctcagttg
caaattgctatgggtctcccaatgcacagaatcagagacatcagattgttgtacgg
tgttgatccacactctgccactgagattgatttcgagttcaagtccccaaactcat
tgatcacgcaaagaaagccagctccaaagggtcactgtaccgcttgtcgtatcact
tctgaagatccaggtgaagggttcaagccaagtggtggtactcttcacgagttgaa
cttccgttcttcgtccaatgtctggggttacttctcggtcgccaaccaatcttcta
tccactcctttgctgattcccagtttggtcacatttcgcctttggtgaaaatcgt
caagcctctagaaagcacatgattgttgccttgaaggaattgagtatcagaggtga
ctttagaaccactgttgaatacttgatcaagttgttggagactccagatttcgccg
acaacaccatcactaccggttggttggatgagttgatcaccaagaagttgactgcc
gaaagaccagatcctatcgttgctgttgtctgtggtgccgtcaccaaagcccacat
ccaagccgaagaagacaagaaggagtacattgagtctttggaaaagggtcaagttc
caaacaagtccttgttgaaaactatcttcccagttgagtttatctacgaaggtgaa
agatacaagtttactgccaccaagtcctccgaagacaagtacactttgttcctcaa
cggttctagatgtgtcattggtgctcgctcattgtctgatggtggcttgttgtgtg
ctttggacggtaagtcccactctgtctactggaaggaagaagcagcggccactaga
ttgtctgttgacggtaagacttgcttgttggaagttgaaaacgacccaacccaatt
gagaactccgtctccaggtaagttggtcaagtacttggttgagagtggtgaacacg
ttgatgccggccaatcttatgccgaagttgaagtcatgaagatgtgtatgcctttg
attgcacaagaaaacggtactgttcaattgctcaaacaaccaggttccactcttaa
cgctggtgacatcttggcaatcttggcattggacgatccatctaaagttaaacacg
ccaagccatatgaaggcactttgccagagatgggtgatccaactgttaccggttcc
aaaccagctcacttgttccaacattacgacaccatcttgaagaacatcttggctgg
ttacgataaccaagtcattttgaactccactttgaagaacatgatggagatcttga
agaacaaggagttgccttattctgaatggagattgcaaatctccgccttgcattca
agaatcccaccaaagttggatgaggctttgacgtccttgattgaaagaaccgaaag
cagaggcgccgaattcccagctcgtcagattttgaagctcgtcaacaagactcttg
gtgaaccaggcaacgaattgttgggcgatgttgttgctcctcttgtctccattgcc
aaccgctaccagaacggcttggttgaacacgagtacgactactttgcttcattggt
taacgagtactgcaatgttgaacacttctttagtggtgaaaacgtgagagaagaag
atgttatcttgagattgagagacgagaacaagtctgatttgaagaaggttatcagc
atttgcttgtcccactcccgtgtcagtgctaagaacaacttgattttggccatctt
ggaagcttatgaaccattgttgcaatccaactcttcaactgccgttgccattagag
attctttgaagaagatagtccagttggattctcgtgcttgtgccaaggttggtttg
aaagctagagaacttttgattcaatgttctttgccatccatcaaggaaagatctga
ccaattggaacacattttgagaagtgcagtcgttgagacttcttatggtgaagttt
tcgccaagcacagagaacctaaattggaaatcatccaagaagttgtcgaatccaag
cacgttgttttcgatgtcttgtcgcaattttggtccaccaagactgctgggttgc
cattgctgctgccgaagtctatgttagacgttcctacagagcttatgatttgggta
agatcgattaccacattcatgacagattgccaattgttgaatggaagttcaagttg
gctcaaatcgcaggttccagatacaacgccgtccaatctgccagtgttggtgacga
ctcgaccactatgaagcatgctgcatctgtttctgacttgtcgtttgttgttgatt
ccaagagcgaatccacttccagaactggtgttttggttccagctagacatttggac
gatgttgatgagattctttctgctgcattggagtacttccaaccatctgatgcact
ctctttccaagctaagggagaaagaccagagttgttgaatgttttgaacattgtca

tcaccgacattgacggttactctgacgaagatgaatgcttgaagagaattcatgaa
atcttgaacgagtacgaagacgatttggtctttgctggtgttcgtcgtgttacttt
tgttttcgcccaccaagttggttcttatccaaagtactacaccttcactggtccag
tgtatgaagaaaacaaggttatcagacacatcgaaccagctttggctttccaattg
gaattggaagattagccaactttgacatcaagccaattttcaccaataacagaaa
cattcatgtttacgaagctattggtaagaatgctccttcggataagagattcttca
ctagaggtattattagaggtggtgtcctcaaagatgaatcagtcttactgagtac
ttgattgctgaatcgaacagattgatcagtgntatcttggataccttggaagttat
tgacacttccaactccgatttgaaccacatttcatcaacttctccaacgttttca
acgtccaaccagctgatgttgaagctgcttttgcttcattcttggaaagatttggt
agaagattgtggagattgagagttactggtgctgaaatcagaattgtctgtaccga
cccacagggcaactcattcccattgcgtgccattatcaataacgtttcaggttatg
ttgtcaagtcggaattgtacttggaagtgaagaaccctaagggtgattgggtcttc
aaatccattggccaccctggctcaatgcacttgcaaccaatctcgactccataccc
agtcaaggaatccttgcagccaaaacgttacagagctcacaacatggaaccactt
ttgtttacgatttcccagagttgttccgtcaagccaccatttcccaatggaagaag
cacggcaagaaggctcctaaagatgtcttcacttctttggagttgatcaccgatga
aaacgatgctttggttgccgttgaaagagatccaggtgccaacaagattggtatgg
ttggtttcaaggtcactgccaagacccctgaatacccacgcggacgttcattcatc
attgttgccaatgatatcacccacaagattggttcctttggtccagatgaagatga
atacttcaacaagtgtaccgacttggccagaaagttgggtgttccaagaatttacc
tttctgccaactccggtgccagaattggtgttgctgaagagttgattccattgtac
caagttgcttggaacgaagaaggtaacccagataaaggtttcagatacttgtactt
gaacccagacgccaaagaagctttggaaaaagacggcaagggtgacactattgtta
ctgaacgtattgtcgaagatggtcaagaacgtcacgttatcaaggccattattggt
gctgagaacggcttgggtgttgaatgtttgaaaggttccggtttgattgctggtgc
cacttcaagagcctacagagacatcttcaccattaccttggtcacttgtagatctg
ttggtattggtgcctatttggtcagattgggtcaaagagctatccaaattgaaggt
caaccaatcatttgactggtgcaccagctatcaacaagttgttgggtagagaagt
ttactcgtcgaacttgcaattgggtggtacccagatcatgtacaacaatggtgttt
cccacttaactgccagtgacgatttggctggtgttgagaagatcatggaatggttg
tcctacgttccagctaagcgtggtatgccagtaccaatcttggaaagtgaagatac
ctgggacagagacattgactactacccaccaaagcaagaagctttcgacatcagat
ggatgatcgaaggtaagcaagttgaaggtgaagagtttgaatctggtttgtttgac
aaaggttcattccaggaaactttatcaggatgggctaaaggtgttgtcgttggtag
agctcgtctcggtggtatcccaattggtgtcattggtgttgagaccagaactattg
aaaacatgatcccagctgacccagccaacccaagttccactgaagccttgatccaa
gaagccggtcaagtctggtatccaaactctgcgttcaagaccgcacaagccattaa
cgacttcaacaacggtgaacaattgccattgatgatcttggccaactggagaggtt
tctctggtggtcagagagatatgtacaacgaggtcttgaagtacggttccttcatt
gttgacgctttagttgatttcaagcagccaatcttcacttacatcccaccaaatgg
tgaattgagaggtggctcttgggtcgttgttgatccaaccatcaactccgacatga
tggaaatgtatgccgacgttgactccagagctggtgttttggaaccagaaggtatg
gttggtatcaaatacagacgggacaagttgttggctaccatgcaaagattggatcc
aacttatgcccaattgaaggagaagttgaacgactcgagcttgtcgccagaagaac
atgcccaagtcagcaccaagattgtcaagcgtgaaaaggcattgttgccaatctat
gcccaaatttctgtccagtttgccgacttgcacgacagatccggacgtatgatggc
taaaggtgtcattagaaaagaaatcaagtgggttgacgccagacgtttcttcttct
ggagattgagaagaagattgaacgaagagtacgttttgaagttgattggtgaacag
gtcaagaatgccaacaagttggaaaaggttgccaggttgaagagttggatgccaac
tgttgactacgacgatgaccaagctgtcagtacttggattgaagagaaccacgcca
aattgcaaaagagagttgaagaattgagacaggagaagaacaagtccgacattgtc
aaattgttgcaagaagacccatcaaacgctgcctctgttatgaggatttcgttga
tagattgtccgatgaagaaaggaaaagttccttaaatcattgaactag

### Example 35: Cloning, Amplification and Overexpression of C. tropicalis fatty acid synthase activity

Type I fatty acid synthases contain all of the active domains for fatty acid synthase on one (e.g., alpha) or two (e.g., alpha and beta) polypeptides. Hexanoate synthase, a specialized fatty acid synthase enzyme, is unique in that the fatty acid product is the six carbon long hexanoic acid rather than a fatty acid with a 16 or 18 carbon chain length product produced by native fungal fatty acid synthases. Hexanoate synthase is composed of an A (HexA) and B (HexB) subunit with the same active domain organization as the native fungal fatty acid synthase alpha (Fas2) and beta (Fas1) subunits.

Hexanoate synthase activity was introduced into an engineered strain by placing the nucleotide sequences of HexA and HexB under the control of the C. tropicalis PGK promoter and integrating the heterologous sequence into the C. tropicalis genome, as described herein. To further provide increased carbon flux through engineered pathways and increase the production of adipic acid, additional fatty acid synthase activities were cloned and amplified in engineered strains. Mutant fatty acid synthase activities that produce intermediate chain length products also would beneficial for increasing the conversion of carbon feedstocks to adipic acid. To ensure that the fatty acids produced are not converted to products shorter than 6 carbons (e.g., hexanoic acid), the strains for increased fatty acid synthesis activity also would include a partially blocked beta oxidation pathway (e.g., disrupted for POX4 activity) to reduce or eliminate the degradation of fatty acids to chain lengths below 6 carbons. In some embodiments, FAS mutants can be identified by using serially passaged or mutagenized C. tropicalis strains and determining fatty acid profiles by gas chromatography.

### Cloning of C. tropicalis FAS2 and FAS1

The native *C. tropicalis* FAS2 and FAS1 genes were cloned between the *C*. *tropicalis* strain ATCC20336 PGK promoter and terminator, and correct nucleotide sequence was verified by DNA sequencing. The vectors were linearized inside the URA3 selectable marker by restriction enzyme digestion and subsequently co-transformed into a ura- derivative of *C*. *tropicalis* strain ATCC20962 by targeted single-crossover integration at the URA3 locus. Correct integration of both the FAS2 and FAS1 constructs was confirmed by PCR. The nucleotide sequences of the C. tropicalis FAS2 and FAS1 activities are presented in the table below as SEQ ID NOS: 31 and 32.

| SEQ ID NO | Description | Sequence |
|---|---|---|
| SEQ ID NO:31 | FAS2, *C. tropicalis* ATCC20336 | atgaagccagagattgaacaagaattatcccacaccttgttaacagaat tgttagcttatcagttcgcttctccagtcagatggatcgaaacccaaga tgtcttcttgaagcaacacaacaccgaaagaatcatcgaaatcggccct tccccaaccttggccggtatggccaacagaaccatcaaggccaaatacc aatcctacgacgccgcgttgtccttgcaaagagaagtcttatgctactc taaggacgccaaggagatctactacaagccagatccagcagatcttgct ccaaaggaagaaccaaagaaggaagaagctgccgccgctccagccgcta caccagctgctgctgctgctgctgctactcctgctgctgccccagtcgc cgctgctccagccccatctgctggccctgctgaatccatcccagatgaa ccagtcaaggcttccttgttgatccacgtcttggttgctcagaaattaa agaaaccattggatgctgttccaatgtccaaggctatcaaagatttagt taacggtaagtccactgtccagaacgaaattcttggtgacttgggtaaa gaattcggttccactcctgaaaaaccagaagataccccattggaagaat tggccgaacagttccaagactccttcagtggtcaattgggtaagacttc tacttcattgattggtagattgatgtcttctaagatgcctggtggtttc tcaatcaccgctgccagaaaatacttggaatccagattcggtttgggtg ccggtagacaagactctgtcttgttggttgctttgaccaacgaacctgc aagcagattgggttctgaggccgaagctaagaccttcttggacaccatg gctcagaaatatgcctcatctgctggtatttccttgtcgtcagcttctg ccggtgccggtgctggaggtgccgccggtggcgccgttgttgacagtgc tgctttggacgccttgactgctgaaaacaagaaattggctagacaacaa ttagaggtcttggctagatacttgcaagtcgacttgaactcaggagcta agtcttttatcaaagaaaaagaagcttccgctgttttgcagaaagaatt ggacttgtgggaagccgaacatggtgaattctacgccagaggtatcaaa ccaactttctcagctttgaaagcaagaacctatgattcctactggaact gggccagacaagatgttttgtccatgtactttgatattttgtttggtaa gttgacctccgttgacagagaaaccatcgaccaatgtatccaaattatg aacagatccaacccaactttgatcaagttcatgcaataccacattgacc actgcccagaatacaagggtgagacttacaagttggccaagagattggg tcaacagttgattgacaactgtaagcaaaccttgaatgaagacccagtg tacaaggacgtttctagaatcactggtccaaagaccaccgtctgcgcca agggtaacattgaatacgaagaagccgaaaaggattctgttagaaagtt tgaacagtacgtctacgaaatggcccaaggtggtgaaatgaccaagatt gcccaaccaactattcaagaagacttggccagagtttacaaagccatct ccaagcaagcttccagagacagcaagttggaattgcagaaagtctacga gcaattgttgaaggttgttgctggttcagacgaaattgaaactcagcaa ttaaccaaggacatcttgcaagctccaactggctccaacaccccaactg atgaagatgaaatttccaccgccgactctgacgatgaaattgcttcatt gccagacaagacttcaattgcccaaccagtttcttcaactgttccaccc cagaccatcccattcttgcacattcaaaagaagaccaacgaaggctggg aatacgaccgcaagttgtctgccctttacttggacggtttggaatccgc tgctgtcaacggtctcaccttcaaggacaagtacgttttggttaccggt gctggtgctggatccattggtgccgaaatcttgcaaggtttgatcagtg gtggtgccaaggttgttgttaccacctctagattctccaagaaggttac tgagtactaccaaaacatgtacgccagatacggtgctgccggttctact ttgattgttgttccattcaaccaaggttctaaacaagatgttgacgctt tggttcaatacatctacgacgatccaaagaagggtggtttaggctggga cttggatgccattatcccattcgctgctatcccagaaaatggtaacggt atcgacaacattgattctaaatccgaatttgcccacagaattatgttga ccaaccttttgagattgttgggtgctgtcaaatccaagaagactaccga caccagaccagctcaatgtatcttgccaatgtctcctaaccacggtact ttcggtttcgatgggttgtactctgaatccaagatttccttggaaacct tgttcaacagatggtactccgaagattggggctccaagttgaccgtctg tggtgccgttattggttggaccagaggtactggtttgatgagcgccaac aacatcattgccgaaggtatcgaaaagattggtgtcagaaccttctccc aaaaggaaatggctttcaacatcttgggtttgttgactccagagattgt caagttgtgccaagaagaaccagttatggccgacttgaacggtggtttg caattcattgaaaacttgaaggatttcacttccaagttgagatctgact tgatggaatccgctgaagttagaagagctgtctccattgaatccgccat cgaacaaaaggttgtcaatggtgacaatgttgatgccaactacaccaag gttaccgttcaaccaagagccaacatgaaattcgacttcccaaccttga aatcgtacgatgagatcaagaaggttgctccagaattggaaggcatgtt ggacttggaatccgtcgttgttgtcaccggttttgccgaagttggtcca tggggtaacgccagaaccagatgggaaatggaatccaaggctgaattct |
| | | ccttggaaggtgccattgaaatggcctggatcatgggtttcatcaagta ccacaacggtaacttgaagggtaagccttactctggttgggttgatgcc aagacccaaactccaatcgatgacaaggacatcaaggccaagtacgaag aagagatcttggaccactctggtattagattgattgagccagaattgtt caatggctacgatccaaagaagaagcagatgatccaagaagttgtcatc caacatgacttggaaccatttgaagcctccaaggaaactgctgaacaat acaaacacgaacacggtgacaagtgtgagatctttgaaattgaagaatc cggtgaatacactgttagaatcttgaaaggtgctaccttgtttgttcca aaggctttgagatttgacagattggttgctggtcaaattccaactggtt gggatgctcgtacctacggtattccagaagataccattaaccaagttga tcctatcactttgtacgtcttggttgctaccgttgaagctttgttgtct gctggtatcaccgacccatatgaattctacaagtacgtccacgtttccg aagttggtaactgttctggttccggtatgggtggtgtctctgccttgag aggaatgttcaaggacagatacgccgacagaccagtgcaaaacgatatc ttgcaagaatctttcatcaacaccatgtccgcctgggttaacatgttgt tgttgtcttcttcgggtccaatcaagaccccagttggtgcctgtgctac cgctgttgaatccgttgacattggtattgaaactatttgtctggtaag gctaaggttgttatggttggtggttacgatgacttccaggaagaaggtt cttatgaattcgccaacatgaatgccacttccaactcccttgacgagtt tgctcacggcagaactccaaaggagatgtccagaccaactaccaccacc agacacggtttcatggaggcccaaggttctggtatccaagttattatga ctgctgacttggccatcaagatgggtgttccaattcacgctgtgttggc catgtctgctactgctaccgacaagattggtagatctgttccagctcca ggtaagggtatttgaccactgccaggaacaccacggtaacttgaagt acccatctccagctttgaacatcaagtacagaaagagacaattgaaggc tagattagaccaaatcaaggcttggaagaagctgaaattgcttacttg caagacgaagctgagttggccaaggaagaaatgggcgatgagttctcca tgcacgaattcttgaaggaaagaactgaagaagtgtaccgtgaatccaa gagacaagtttctgacgctaagaagcaatgggtaaccaattctacaag tctgacccaagaattgctccattgagaggtgccttggctgctttcaact tgaccattgacgatcttggtgttgcttccttccacggtacttctaccgt cgccaacgataagaacgaatccgccactattaacagcatgatgcaacac ttgggcagatctgaaggtaacccagtgtttggtgttttccagaagtact tgactggtcatccaaagggtgctgctggtgcttggatgttgaacggtgc catccagatcttggagtctggtattgttccaggtaacagaaatgccgat aacgttgacaaggtcttggaagaatacgagtacgtcttgtacccatcca gatccatccaaactgacggtatcaaggccgtttccgtgacctctttcgg tttcggtcaaaaaggtgctcaagctgttgtcgtccacccagactacttg tttgctgttttggatagatctacttatgaggactacgccaccagagttt ctgccagaaacaagaagacttaccgttacatgcacaatgctattactag aaacactatgtttgttgctaaggataaggctccatatgccgatgaattg gaacaaccagtttacttggacccattagcccgtgttgaaaacgctaagg aaaagcttgccttcagcaacaagagtatccaatccaaccaagcttatgc tggtgaaaatgccagaaccactgccaaggctttggctgccttgaacaag tcatccaagggtgttggtgtcgacgttgaattattgtctgagctcaact tggagaatgaaactttgttgcaagaaacttcactcctggtgaaatcca atactgctccaagagtgccaacccacaagcttcatacaccggcacttgg tctgctaaagaagctgtcttcaaggcattaggtgttgaatctaaaggtg ctggtgccagcttggttgacattgagatcactcgtgacgtcaacggcgc tccacaagttgtcttgcacggtgatgcggcaaaatcagccgccaaagct ggtgtcaagaacgtcaagatttccatctcccatgacgacttccaagcca ctgctgttgccttgagtgaattctag |
| SEQ ID NO:32 | FAS1, *C. tropicalis* ATCC20336 | atgtctactcatagacctttccaattgacccacggttccatcgaacaca ccttgttggtgccaaacgagttgttcttcaactattcacagttaaaaga cgaattcataaagaccttgcctgaaccaaccgaaggtttcgctggcgac gatgaaccttccagtcctgctgaattgtacggcaaattcctcggctaca tcagtgacaacaccgttcaattcccccagatcttacaattgtccttgca agacttccagcagcgattcttggacaaccacgacaacatccactccttt gccgtcagattattagaagatgaagcttatccaacaacaatcaccaaag tcaaggaaaatatcatcaagaactactacaaagccatcaagtccatcga caaggtcgagtcaaacttgttgtaccactgcaaacatgacgccaagttg gccgctatattcggtggtcaaggtaacaccgacgactactttgaagaat tgcgtgaattgtacaccttataccagggcttgattgaggacctccttat ctccattgccgacaagttggacgagttatacccttcttttgacaagatc |
| | | tacacccagggtttgaacatcttgggctggttgaagcacccagaaacca cccctgaccaagattacttgttgtccgtaccagtgagttgtcctgttat ctgtatcatccaattgtgtcactacaccatcacctgcaaagttcttggt ttgacccctggtgaatttagagactcgttgaagtggtccaccggtcact cccaaggtttggttactgctaccgctatttccagttccgactcctggga ctccttcaaacaaaactccattgctgccgtctccttgatgcttttcatt ggtgccagatgtttgatggcttacccaagaactaccttgccaccaacca tgttgcaagactccttggaacacggtgaaggtagaccatctccaatgtt gtcagttagagacttgaccatcacccaagttgagaagtttattgaacag accaactctcacttgccaaaggaaaagcacattgccgtcagtttggtca atggtgccagaaatttggttctttctggtcccccggagtccctttacgg tttcaacttgaacttgagaaaccaaaaggctccaatgggattggaccaa tcacgtgttccattcagtgaacgtaagttgaagtgttccaacagattct tgccaattttgcaccattccactctcacttgttggctgatgccactga gtacattttggatgatgtcaaagaacaccgtttgtctttccagaaattg aagattgcagtctacgatacctacgacggctccaacttccaagagagca aggaaccaattattgacagactcgtcaagttgatcaccgagttgccagt tcactgggaaaccgccaccaaccacaggccacccacatttggatttc ggcccaggtggtgtctccggtttgggtgttttgacccacagaaacaagg aaggtactggtgctagaataattgttgctggtgctcttgactccaaccc aattgacgatgagtatggtttcaagcacgaaatcttccagacttctgcc gacaagtccatcaagtgggctagcgattggttggaagaattcaaaccaa ctttggtcaagacttcccagggaaagatctacgtcaacaccaagttctc gcaattgttgggcagagctcccttgatggtcccaggtatgacaccaacc actgttaacccagacatcattgccgcctctttgaatgctggctatcaca ttgaattagccggtggtggttatttcgccggcaggatcatgaccaaagc cattgaccaaattgttgccgacatcaagccaggttacggtttgggtatc aacttgatttacgtcaacccattcatgttgcaatggggtattccattga ttaaggagttgagagaaaagggttatccaatccaatctttgaccattgg tgctggtgttccatctttggaagttgccactgaatacattgaagagttg ggtctcacgcacttgggcttgaaaccaggttcgattgacgccatcagcc aagtcatcaccattgccaaggctcatccaaagttcccaattgtcttgca atggactggtggtagaggtggtggccaccactcttttgaagatttccac caaccaatcctccagatgtactccaagatcagaagatgcccaaacattg tcttggttgctggttccgggtttggttctgacgaagacacctacccata cttgactggttcttggtccaagagattcaactacccaccaatgccatac gatggtgtcttgtttggttccagagtcatgaccgccaaggaggcccaca cttcgttggaagctaagaaattgattgcctcgtgcccaggtgtcccaga tgagaagtgggagcaaacctacaagaagccaaccggtggtatcatcact gttagatctgaaatgggtgagccaatccacaagattgccaccagaggtg tcatgttctggaaggaattggacgacactatcttcaaccttccaaagcc aaaagccttggaagccatcaagaagaagagagactacatcatcaagaag ttgaacagcgacttccaaaagccatggtttggtaagaatgcttctggtg tttgtgacttgcaagaaatgacctacgaggaaatcgccaacagattggt tgagttgatgtacgtcaagaagtctcaaagatggattgacgtttcctta agaaacttgtacggtgacttttgagaagagttgaagaaagattcactt ctgctgccggcgtggtttccttgttgcaaacttcatccagttgaacga cccagaaacattcagtgccgagttcttcaacaagttcccacaagccaag gaacaattgatttccgaagaagacgctgaccacttttgttgttggctg ccagaccagggcaaaagccggttccattcgtgccagtcttggacgaaag atttgaattcttcttcaagaaagattctctttggcaatctgaagacttg gaaagtgttgtcgacgaagacgttcaaagaacttgtatcttgcacggtc cagttgcctcccaattcaccaagaaggttgatgaaccaattggcgaaat cttggactctatccacgagggccatattgccaagttgatcaaggatgaa tacgctggtgatgcatccaagatcccagttgttgagtacttcggtggtt tcaagaccgacaaggttaatgctaacaatgttcaagtcaatgctaccag aaaggaaaccgtctacgaaattggttccaagttgccagccaggcaagac tggttggacttgttggccggtactgaattgaactggttgcacgctttca tctccaccaacagaattgtccaaggctccaagcacgtcgccaacccatt gcacgacattttggctcccgttgccagatccagtgtttccattgacaag gctaccaagaaattgactgcttatgaaaaggtcaagggtgagttggttc cagttgttgaaattgaattggtcaagccaaacaccattcaattgtcttt gattgaacacagaactgctgatggcaaaccagttgctttgccattcttg tacaagtacgacccaactgatgggtttgcaccagtcttggagatcatgg |
| | | aaaacagaaacgacagaatcaaggaattctactggaagttgtggttcgg tgcttccgtcccttacgacaatgacatcgatgtcgaagagcaaatcttg ggtgacgaaatcaccatttcttctcaagacattggtgaattcacacacg ctattggtaacaagtgtgaagcctttgtcaacagaccaggtaaggtcac tttggctccaatggatttcgccattgttgttggttggaaagctatcatc aagtccatcttcccaaagaccgttgacggtgacttgttgaagttggtcc acttgtccaacggttacaagatgatccctggtgcagctccattgcaaaa gggcgatgttgtttccactagatctgacatcaaggctgttttgaaccaa ccaagtggtaagttggttgaagttgttggtaccatcttccgtgaaggca agccagttatggaagtcacttcacaattcttgtaccgtggtgaatacga cgactactgcaacaccttccaaaaggtcactgaaactccagttcaagtc tcattcaagtctcctaaggatttggctgttttgagatccaaggaatggt tccatttggaaaaggatgtcgagtttgatgctttgactttcagatgtga atccacttacaagttcaagtctgccaacgtctactcgtccatcagaacg accggtcaagtttcttggagttgtccaccaaagaagttatccaagttg gttctgttgactatgaagctggtacctcttatggtaacccagtcactga ctacttgaacagaaacggtaagaccattgaagaggctgttacttttgag aatgccatccccttgtcgtctggtgaagagttgaccaccaaggctccgg gtaccaacgagccatatgctattgtttctggtgactacaacccaatcca cgtttccagagtcttttctgcttacgccaagttgccaggtactatcacc cacggtatgtactcttctgccgccatcagagccttggttgaagagtggg ctgccaacaacgttgccccaagagtcagagccttcaagtgtgaatttgt tggtatggttttgccaaacgacactttgcaaaccactatggaacacgtt ggtatgatcaatggccgtaagatcatcaaggtcaagactgtcaatgccg agaccgagactccagtcttgcttgctgaagccgaaattgaacaaccaac caccacctatgttttcactggtcaaggttcccaagaacaaggcatgggt atggatttgtacaactcttctgaagttgcccgtaacgtttgggataccg ccgacaagcatttcatcaaccactatggcttctccatcttggacattgt gcaaaacaaccctaaggaattgactatccactttggaggtgctaaaggt agagctatcagagacaactacattggtatgatgtttgaaacaattggtg aagacggttctttgaagtccgaaaagatcttcaaggacattgacgaaaa caccacttcctacacctttgtttctgacactgggttgttgtctgctact caattcacccaacccgctttgactttgatggagaaggctgcctacgacg atatcaagtctaaaggattgattccaagtgacatcatgtttgctggtca ctctcttggtgaatactctgctttgacttccttggccaacgttatgcct attgaatccttggttgatgttgtcttctacagaggtatgaccatgcaag ttgctgttccaagagacgagtttggtagatccaactacggtatggttgc tgtcaacccaaccagagtcagcccaacatttgacgatgccgccatgaga tttgttgttgacgagactgccaagagaaccacctggttgttggaaattg tcaactacaatgttgaaaaccaacagtacgttgctgctggtgacttgag agccttggataccttgaccaacgtgttgaatgttttgaagatcaacaag attgatattgtcagattgcaagaacaattatccctcgacaaggtcaagg agcacttgtacgagattgttgatgaagttgctgccaagtccattgctaa gccacaaccaattgaattagaaagaggttttgctgttatcccattgaag ggtatttctgtcccattccactcttcctacttgatgtctggtgtcaagc cattccagagattcttgtgcaagaagattccaaaggcttccatcaaacc acaagatttgattggcaagtacattcctaacttgactgctaagccattt gaacttactaaggaatacttccaggatgtctacgacttgactaaatctg aaaagatcaaggctatcttggacaactgggaaaaatacgaatag |

Cultures of positive transformants were grown overnight in YPD medium at 30°C and 200rpm shaking until stationary phase. Cells from 4mL of the culture were harvested by centrifugation and the cell pellet was frozen at -20°C until used. Cell pellets were treated for lysis by resuspension in cold yeast lysis buffer (50mM Tris pH 8.0, 0.1% Triton X-100, 0.5mM EDTA, 1X ProCEASE protease inhibitors), working on ice during the entire lysis procedure. The resuspended cell pellet solutions were added to prechilled screw-cap tubes containing zirconia beads and cycled on a Bead Beater (BioSpec Products) once for 2 minutes. A volume of the whole cell extract (200uL) was removed for later analysis and stored at 4°C. The remainder of the whole cell extract was removed to a new tube and centrifuged for 15 minutes at 16,000 x *g* 4°C to pellet insoluble debris. The supernatant soluble cell extract was removed to a new tube and stored at 4°C. Protein concentration was determined by Bradford assay (Pierce). For SDS PAGE analysis, a volume of whole cell extract and soluble cell extract from each sample equivalent to 50ug total protein was acetone precipitated at -20°C for 2 hours and centrifuged for 15 minutes at 16,000 x *g* 4°C.

The protein pellet was washed with cold 80% acetone and recentrifuged. The protein pellet was allowed to air dry for 10 minutes before adding 50uL of 1XLDS sample buffer (Invitrogen) containing 50mM DTT and incubated overnight at 4°C. The samples were heated at 70°C for 10 minutes before SDS PAGE on 4-12% NuPAGE gels (Invitrogen) and transfer to nitrocellulose. Immunodetection was performed using overnight incubation in 1:5,000 mouse anti-6xHIS (Abcam) primary antibody and four hour incubation in 1:5,000 donkey anti-mouse HRP (Abcam) secondary antibody. Signal development was performed with Supersignal Pico West reagent (Pierce). For NativePAGE analysis, samples of soluble cell extract were prepared at a final concentration of 0.35ug/uL in yeast lysis buffer and 1X NativePAGE sample buffer (Invitrogen) containing 0.025% G250. Samples (3.5ug) were resolved on 3-12% NativePAGE gels (Invitrogen) and transferred to PVDF according to manufacturer's instructions. Immunodetection of 6xHIS tagged oligomeric complexes was performed as described herein.

Immunodetection of the expressed proteins is shown in Figures 40 and 41. Denaturing SDS PAGE (see Figure 40) shows immunoreactive bands at sizes expected for the Fas2 (206 kDa) and Fas1 (229 kDa) subunits indicating successful over-expression of both FAS subunits. Native electrophoresis allowed detection of oligomeric complexes formed by the 6xHIS-tagged subunits. Immunodetection of the native PAGE (see Figure 41) shows no detectable signal at the size for the individual subunits indicating that all subunits are in oligomeric complexes. Two sizes of oligomeric complexes are detected, one at an estimated size of about 600 kDa and another with an estimated size of about 1.1 MDa. The detected large complex is smaller than the predicted size for the native alpha₆beta₆ FAS (2.6 MDa), however migration of large oligomeric complexes in native PAGE is known to frequently be subject to large migration error, thereby hampering accurate size estimation. qPCR analysis of strains engineered for amplified FAS activity indicated that in some strains about 2 additional copies of FAS2 and FAS1 subunits were present (data not shown).

In some embodiments, strains engineered for conversion of glucose to adipic acid include two or more additional copies of each of the nucleotide sequences identical to SEQ ID NOS: 31 and 32. In certain embodiments, strains engineered for conversion of glucose to adipic acid produce two or more times the fatty acid synthase activity encoded by nucleotide sequences identical to SEQ ID NOS: 31 and 32, as compared to a strain with native fatty acid synthase activity.

In some embodiments, strains engineered for conversion of paraffins (e.g., oils, petroleum distillates, plant based oils, coconut oil) to adipic acid include two or more additional copies of each of the nucleotide sequences identical to SEQ ID NOS: 31 and 32. In certain embodiments, strains engineered for conversion of paraffins to adipic acid produce two or more times the fatty acid synthase activity encoded by nucleotide sequences identical to SEQ ID NOS: 31 and 32, as compared to a strain with native fatty acid synthase activity.

### Example 36: Cloning of the C. tropicalis ATCC20336 ZWFgene

### Plasmid pAA246

A PCR product containing the ZWF (glucose 6 phosphate dehydrogenase) nucleotide sequence was amplified from C. tropicalis 20336 genomic DNA using primers oAA831 and oAA832. The PCR product was gel purified and ligated into pCR-Blunt II-TOPO (Invitrogen), transformed into competent TOP10 E. coli cells (Invitrogen) and clones containing PCR inserts were sequenced to confirm correct DNA sequence. One such plasmid was designated pAA246. Plasmid pAA246 was digested with BspQl and a 1.5-kb fragment was isolated. Plasmid pAA073 which contained a POX4 promoter and POX4 terminator also was digested with BspQl and gel purified. The isolated fragments were ligated together to generate plasmid pAA253. Plasmid pAA253 contains PPOX4ZWFTPOX4 fragment and URA3.

### Strains sAA650 and sAA651

Plasmids pAA253 and pAA156 were digested with Clal and column purified. Strain sAA329 was transformed with this linearized DNA and plated on SCD-ura plate. Several colonies were checked for ZWF and CYP52A19 integration. From those colonies, qPCR was performed to check the copy number of ZWF and CYP52A19 integration. Strain sAA650 contains 5 copies of ZWF and 9 copies of CYP52A19. Strain sAA651 contains 5 copies of ZWF and 12 copies of CYP52A19.

The nucleotide and amino acid sequence encoded by the C. tropicalis strain ATC20336 ZWF gene are presented below as SEQ ID NOS: 33 and 34
ZWF nucleotide sequence SEQ ID NO: 33 
ATGTCTTATGATTCATTCGGTGACTACGTCACTATCGTCGTTTTCGGTGCTTCCGG
TGACTTGGCCAGCAAAAAAACCTTCCCTGCCTTGTTTGGCTTGTTTAGAGAAAAGC
AATTGCCCCCAACCGTCCAGATCATTGGCTATGCCAGATCCCATTTGTCCGACAAG
GACTTCAAAACCAAGATCTCCTCCCACTTCAAGGGCGGCGACGAAAAAACCAAGCA
AGACTTCTTGAACTTGTGTACTTATATCAGCGACCCATACGACACTGACGATGGTT
ACAAGAGATTGGAAGCCGCCGCTCAAGAATACGAATCCAAGCACAACGTCAAGGTC
CCTGAAAGATTGTTTTACTTGGCCTTGCCTCCTTCTGTCTTCCACACCGTCTGTGA
GCAAGTCAAGAAGATCGTCTACCCTAAGGACGGTAAGCTCAGAATCATCATTGAAA
AGCCGTTCGGACGTGATTTGGCCACCTACCGTGAATTGCAAAAGCAAATCTCCCCA
TTGTTCACCGAAGACGAACTCTACAGAATTGACCACTACTTGGGTAAAGAAATGGT
CAAGAACTTGTTGGTTTTGAGATTCGGTAACGAATTGTTCAGTGGGATCTGGAACA
ACAAGCACATCACCTCGGTGCAAATCTCCTTCAAGGAACCCTTCGGTACCGAAGGT
AGAGGTGGCTACTTTGACAACATTGGTATCATCAGAGATGTCATGCAAAACCACTT
GTTGCAAGTCTTGACCTTGTTGACCATGGAAAGACCAGTCTCTTTTGACCCAGAAG
CTGTCAGAGACGAAAAGGTCAAGGTTTTGAAAGCTTTTGACAAGATTGACGTCAAC
GACGTTCTTTTGGGACAATACGCCAAGTCTGAGGATGGCTCCAAGCCAGGTTACTT
GGATGACTCCACCGTCAAGCCAAACTCCAAGGCTGTCACCTACGCCGCTTTCAGAG
TCAACATCCACAACGAAAGATGGGACGGTGTTCCAATTGTTTTGAGAGCCGGTAAG
GCTTTAGACGAAGGTAAAGTTGAAATTAGAATCCAATTCAAGCCAGTTGCCAAAGG
TATGTTTAAGGAGATCCAAAGAAACGAATTGGTTATTAGAATCCAACCAGACGAAG
CCATCTACTTGAAGATCAACTCCAAGATCCCAGGTATCTCCACCGAAACTTCCTTG
ACCGACTTGGACTTGACTTACTCCAAGCGTTACTCCAAGGACTTCTGGATCCCAGA
AGCATACGAAGCCTTGATCAGAGACTGTTACTTGGGCAACCACTCCAACTTTGTCA
GAGACGATGAATTGGAAGTTGCTTGGAAGCTCTTCACCCCATTGTTGGAAGCCGTT
GAAAAAGAAGACGAAGTCAGCTTGGGAACCTACCCATACGGATCCAAGGGTCCTAA
AGAATTGAGAAAGTACTTGGTCGACCACGGTTACGTCTTCAACGACCCAGGTACTT
ACCAATGGCCATTGACCAACACCGATGTCAAAGGTAAGATCTAAGAATAG
ZWF amino acid sequence SEQ ID NO: 34

msydsfgdyvtivvfgasgdlaskktfpalfglfrekqlpptvqiigyarshlsdk
dfktkisshfkggdektkqdflnlctyisdpydtddgykrleaaaqeyeskhnvkv
perlfylalppsvfhtvceqvkkivypkdgklriiiekpfgrdlatyrelqkqisp
lftedelyridhylgkemvknllvlrfgnelfsgiwnnkhitsvqisfkepfgteg
rggyfdnigiirdvmqnhllqvltlltmerpvsfdpeavrdekvkvlkafdkidvn
dvllgqyaksedgskpgylddstvkpnskavtyaafrvnihnerwdgvpivlragk
aldegkveiriqfkpvakgmfkeiqrnelviriqpdeaiylkinskipgistetsl
tdldltyskryskdfwipeayealirdcylgnhsnfvrddelevawklftplleav
ekedevslgtypygskgpkelrkylvdhgyvfndpgtyqwpltntdvkgki

### Example 37: Cloning of C. tropicalis ACH genes

*ACH* PCR product was amplified from *C. tropicalis* 20336 genomic DNA using primers oAA1095 and oAA1096, shown in the table below. The PCR product was gel purified and ligated into pCR-Blunt II-TOPO (Invitrogen), transformed into competent TOP10 *E*. *coli* cells (Invitrogen) and clones containing PCR inserts were sequenced to confirm correct DNA sequence.

Sequence analysis of multiple transformants revealed the presence of allelic sequences for the ACH gene, which were designated ACHA and ACHB. A vector containing the DNA sequence for the ACHA allele was generated and designated pAA310 (see FIG. 51). A vector containing the DNA sequence for the ACHB allele was generated and designated pAA311 (see FIG. 52).

| Primer | sequence |
|---|---|
| oAA1095 | CACACACCCGGGATGATCAGAACCGTCCGTTATCAAT |
| oAA1096 | CACACATCTAGACTCTCTTCTATTCTTAATTGCCGCTTCCACTAAACGG CAAAGTCTCCACG |

### Example 38: Cloning of C. tropicalis FAT1 gene

*FAT1* PCR product was amplified from *C. tropicalis* 20336 genomic DNA using primers oAA1023 and oAA1024, shown in the table below. The PCR product was gel purified and ligated into pCR-Blunt II-TOPO (Invitrogen), transformed into competent TOP10 *E. coli cells* (Invitrogen) and clones containing PCR inserts were sequenced to confirm correct DNA sequence. A vector containing the DNA sequence for the FAT1 gene was designated pAA296 (see FIG. 53).

| Primer | sequence |
|---|---|
| oAA1023 | GATATTATTCCACCTTCCCTTCATT |
| oAA1024 | CCGTTAAACAAAAATCAGTCTGTAAA |

### Example 39: Cloning of C. tropicalis ARE1 and ARE2 genes

*ARE1* and *ARE2* PCR products were amplified from *C. tropicalis* 20336 genomic DNA using primers oAA2006/oAA2007 and oAA1012/oAA1018, respectively, shown in the table below. The PCR products were gel purified and ligated into pCR-Blunt II-TOPO (Invitrogen), transformed into competent TOP10 *E. coli cells* (Invitrogen) and clones containing PCR inserts were sequenced to confirm correct DNA sequence. A vector containing the DNA sequence for the ARE1 gene was designated pAA318 (see FIG. 54). A vector containing the DNA sequence for the ARE2 gene was designated pAA301 (see FIG. 55).

| Primer | sequence |
|---|---|
| oAA1012 | ATGTCCGACGACGAGATAGCAGGAATAGTCAT |
| oAA1018 | TCAGAAGAGTAAATACAACGCACTAACCAAGCT |
| oAA2006 | ATGCTGAAGAGAAAGAGACAACTCGACAAG |
| oAA2007 | GTGGTTATCGGACTCTACATAATGTCAACG |

### Example 40: Construction of an optimized TESA gene for expression in C. tropicalis

The gene sequence for the *E*. *coli TESA* gene was optimized for expression in *C. tropicalis* by codon replacement. A new *TESA* gene sequence was constructed using codons from *C. tropicalis* with similar usage frequency for each of the codons in the native *E*. *coli TESA* gene (avoiding the use of the CTG codon due to the alternative yeast nuclear genetic code utilized by *C*. *tropicalis).* The optimized *TESA* gene was synthesized with flanking BspQI restriction sites and provided in vector pIDTSMART-Kan (Integrated DNA Technologies). The vector was designated as pAA287 (see FIG. 43). Plasmid pAA287 was cut with BspQI and the 555bp DNA fragment was gel purified. Plasmid pAA073 (see FIG. 44) also was cut with BspQI and the linear DNA fragment was gel purified. The two DNA fragments were ligated together to place the optimized *TESA* gene under the control of the *C. tropicalis POX4* promoter. The resulting plasmid was designated pAA294 (see FIG. 45).

### Example 41: Cloning of C. tropicalis DGA1 gene

*DGA1* PCR product was amplified from *C. tropicalis* 20336 genomic DNA using primers oAA996 and oAA997, shown in the table below. The PCR product was gel purified and ligated into pCR-Blunt II-TOPO (Invitrogen), transformed into competent TOP10 *E*. *coli* cells (Invitrogen) and clones containing PCR inserts were sequenced to confirm correct DNA sequence. A vector containing the DNA sequence of the DGA1 gene was designated pAA299 (see FIG. 56).

| Primer | Sequence |
|---|---|
| oAA996 | ATGACTCAGGACTATAAAGACGATAGTCCTACGTCCACTGAGTTG |
| oAA997 | CTATTCTACAATGTTTAATTCAACATCACCGTAGCCAAACCT |

### Example 42: Cloning of C. tropicalis LRO1 gene

*LRO1* PCR product was amplified from *C. tropicalis* 20336 genomic DNA using primers oAA998 and oAA999, shown in the table below. The PCR product was gel purified and ligated into pCR-Blunt II-TOPO (Invitrogen), transformed into competent TOP10 *E*. *coli* cells (Invitrogen) and clones containing PCR inserts were sequenced to confirm correct DNA sequence. A vector containing the DNA sequence of the LRO1 gene was designated pAA300 (see FIG. 57).

| Primer | sequence |
|---|---|
| oAA998 | ATGTCGTCTTTAAAGAACAGAAAATC |
| oAA999 | TTATAAATTTATGGCCTCTACTATTTCT |

### Example 43: Cloning of C. tropicalis ACS1 gene and construction of deletion cassette

*ACS1* PCR product was amplified from *C. tropicalis* 20336 genomic DNA using primers oAA951 and oAA952, shown in the table below. The PCR product was gel purified and ligated into pCR-Blunt II-TOPO (Invitrogen), transformed into competent TOP10 *E*. *coli* cells (Invitrogen) and clones containing PCR inserts were sequenced to confirm the DNA sequence. One such plasmid was designated pAA275 (see FIG. 47). Plasmid pAA280 (see FIG. 46) was digested with BamHI to release a 2.0 kb P_{URA3}*URA3*T_{URA3}P_{URA3} cassette. Plasmid pAA275 was digested with BglII and gel purified. The two pieces were ligated together to generate plasmid pAA276 (see FIG. 48) and pAA282 (see FIG. 49). Plasmid pAA276 and pAA282 have the P_{URA3}*URA3*T_{URA3}P_{URA3} cassette inserted into the ACS gene in opposite orientations.

| Primer | sequence |
|---|---|
| oAA951 | CCTACTTCCACAGCTTTAATCTACTATCAT |
| oAA952 | TTTAAGAAAACAACTAAGAGAAGCCAC |

### Example 44: Construction of Strain sAA722 (pox4a::ura3/pox4b::ura3 POX5/POX5 ACS1/acs1:: P_{URA3}URA3T_{URA3}PURA3)

Plasmid pAA276 was digested with BamHI/XhoI and column purified. Strain sAA329 (*ura3*/*ura3 pox4a::ura3*/*pox4b::ura3 POX5*/*POX5)* was transformed with the linearized DNA and plated on SCD-ura plate. Several colonies were checked for *ACS1* disruption. One such strain was designated sAA722.

### Example 45: Construction of Strain sAA741 (pox4a::ura3/pox4b::ura3 POX5/POX5 ACS1/acs1::P_{URA3})

Strain sAA722 was grown in YPD media overnight and plated on 5-FOA plate. Colonies that grew in the presence of 5-FOA were PCR screened for the looping out of the *URA3* gene leaving behind only the *URA3* promoter (P_{URA3}) in the *ACS1* site. Out of 30 colonies analyzed, only one strain showed the correct genetic modification. The strain was designated sAA741.

### Example 46: Construction of Strain sAA776 (pox4a::ura3/pox4b::ura3 POX5/POX5 acs1::P_{URA3}URA3T_{URA3}P_{URA3}/acs1:: P_{URA3})

Plasmid pAA282 was digested with BamHl/Xhol and column purified. Strain sAA741 (see Example 45) was transformed with the linearized DNA and plated on SCD-ura plate. Several colonies were checked for double *ACS1* knockout by insertional inactivation. One such strain was designated sAA776.

### Example 47: Construction of Strain sAA779 (pox4a::ura3/pox4b::ura3 POX5/POX5acs1:: PURA3/acs 1:: P_{URA3})

Strain sAA776 (see Example 46) was grown in YPD media overnight and plated on 5-FOA plates. Colonies that grew in the presence of 5-FOA were PCR screened for the looping out of the *URA3* gene leaving behind only the *URA3* promoter (P_{URA3}) in both *ACS1* copies. One such strain was designated sAA779.

### Example 48: Construction of Strain sAA811 (pox4a::ura3/pox4b::ura3 POX5/POX5 acs1:: P_{URA3}/acs1:: P_{URA3} ura3::3xP_{POX4}P450A 19)

Plasmid pAA156 (see FIG. 50) containing a *P450A19* integration cassette was digested with ClaI and column purified. Strain sAA779 (see Example 47) was transformed with the linearized DNA and plated on SCD-ura plate. Several colonies were checked for *P450A19* integration. From those colonies, qPCR was performed to check the copy number of *P450A19* integration. One strain, designated sAA811, contained 3 copies of *P450A19.*

### Example 49: Construction of Strain sAA810 (pox4a::ura3/pox4b::ura3 POX5/POX5 acs1:: Pᵤᵣₐ₃/acs1:: P_{URA3} ura3::5xP_{POX4}P450A19 ura3::8xP_{POX4}TESA)

Plasmid pAA156 containing a P450-A19 integration cassette was digested with Clal and column purified. Plasmid pAA294 containing a *TESA* integration cassette also was digested with Clal and column purified. Strain sAA779 was cotransformed with both linearized DNAs and plated on SCD-ura plate. Several colonies were checked for both *P450A19* integration and *TESA* integration. Colonies that were positive for both TESA and P450A19 were further analyzed by qPCR. qPCR was performed to check the copy number of the *P450A19* and *TESA* integration events. One strain, designated sAA810, contained 5 copies of *P450A19* and 8 copies of *TESA.*

### Example 50: Shake flask characterization of sAA235, sAA776, sAA810 and sAA811

Starter cultures (5mL) in SP92-glycerol media (6.7g/L Difco yeast nitrogen base, 3.0g/L Difco yeast extract, 3.0g/L ammonium sulfate, 1.0g/L potassium phosphate monobasic, 1.0g/L potassium phosphate dibasic, 75g/L glycerol) were incubated overnight at 30°C, with shaking at approximately 250rpm. The overnight cultures were used to inoculate 25mL fresh SP92-glycerol media to an initial OD₆₀₀ₙₘ of 0.4 and incubated approximately 18 hours at 30°C, and 300rpm shaking. Cells were pelleted by centrifugation for 10 minutes at 4,000xg, at 4°C, then resuspended in TB-lowN media (1.7g/L Difco yeast nitrogen base without amino acids and ammonium sulfate, 3.0g/L Difco yeast extract, 1.0g/L potassium phosphate monobasic, 1.0g/L potassium phosphate dibasic). 1 mL coconut oil was added to start the adipic acid production at 30°C, and shaking at approximately 300rpm, in 250 mL shake flasks. Incubation of the cultures continued for 96 hours and samples were taken for analysis of fatty acids and diacids by GC. The experiment was performed with shake flasks in triplicate.

Yields of product per consumed substrate (Y_{p/s}) were determined with the equation [adipic acid (g/L) * final volume of culture in flask (L)] / [coconut oil added to the flask (g)]. The calculation assumes all of the coconut oil added to the flask was consumed. GC analysis revealed that there were diacids of chain length C6 to C14 in all flasks with the majority represented as C8 and C6. All of the diacids except for the C6 (adipic) were ignored for yield calculations. The maximum theoretical yield (Yₘₐₓ) for the conversion of coconut oil to adipic acid was calculated to be 0.6g adipic acid/ g coconut oil. The percent of Yₘₐₓ (%Yₘₐₓ) was calculated as Y_{p/s} / Yₘₐₓ * 100.

The results of the shake flask experiments are shown in FIG. 58. Based on calculations for Y_{p/s} shown in the previous paragraph, the results indicate that the yield of adipic acid on coconut oil was higher in the strain with disrupted *ACS1* genes (11.6 %Ymax) than the parental strain (5.4 %Ymax). The yield of adipic acid on coconut oil was further improved by the gene amplification of *P450A19* (27.1 %Ymax) and by the coordinate gene amplification of *P450A19* and *TESA* (33.5 %Ymax), as shown graphically in FIG. 58.

### Example 51: Nucleotide and Amino Acid Sequences Used for Manipulations described in Examples 37-50

| SEQ ID NO: | Sequence description | Sequence |
|---|---|---|
| SEQ ID NO:42 | Acyl-CoA Hydrolase (ACHA) Nucleotide Seq | atgatcagaaccgtccgttatcaatccctcaagaggttcagacctctggctttgtctcctgtttttcgtccacgctacaactcccagaaggccaatttcca ccgtccagaccaccctgggtccgacgagccagctgaagccgccgacgccgccgccacgatcctcgccgagttgcgagacaagcagacgaac ccgaacaaggccacctggctcgatgcgttaacggagcgggagaagttgcgtgccgagggcaagacgattgacagtttcagctacgttgacccc aagacgaccgtcgtgggggagaagacacgcagtgactcgttctcgttcttgttgttgccgttcaaggacgacaagtggttgtgtgacgcgtacatca atgcgtttggccggttgcgtgtagcgcagttgttccaggacttggacgccttggcggggcgcatcgcgtacaggcactgttccccagcggagcccgt gaatgtcacggcgagcgtggatagggtgtacatggtgaagaaagtggacgagattaacaattacaatttcgtgttggcggggtccgtgacgtgga ccgggagatcgtcgatggagatcacggtgaaagggtatgcttttgaagacgccgtgccggatataacgaacgaggagtccttgccggcagaga atgtgtttttggctgctaatttcaccttcgtggcacggaacccacttacacacaagtcctttgctattaacagattgttgcccgtgactgagaaggactgg gtcgactatcgccgtgctgagtcccacaacgccaagaagaagttgatggcaaagaacaagaagatcttggagcctaccgcggaagagtccaa gttgatctacgacatgtggagatcgtccaagtccttacagaacatcgagagggccaacgatgggatcgcgttcatgaaggacacgaccatgaag tccaccttgttcatgcagccccagtaccgtaacagacactcatacatgatttcggagggtacttgttaagacaaactttcgaattggcctactgtaccg cggcaacgttttccctggccgggccccgtttcgtcagcttggactccaccacgttcaagaaccccgtgcccgtggggtcggtgctcaccatggactc gtcgatctcgtacacggagcacgtgcacgagggagtggaggagattgacgcggactcaccgttcaacttcagcttgcctgccacgaacaagatc tcgaagaaccccgaggcgttcttgtcggaacccggcacgttgattcaagtcaaggtcgacacatacatccaggagttagagcagagtgtgaaga agcccgcgggtacgttcatctactcgttctatgttgataaagaaagcgttactgttgatggaaaggcgtcgttttgttcagttatcccgcagacgtactcc gagatgatgacttatgtgggcgggagaagaagagcccaggatactgctaactacgtggagactttgccgtttagtggaagcggcaattaa |
| SEQ ID NO:43 | Acyl-CoA Hydrolase (ACHA) Amino Acid Seq | MIRTVRYQSLKRFRPSALSPVFRPRYNSQKANFHRPDHPGSDEPAEAADAAATILAELRDKQTNPNKAT WLDALTEREKLRAEGKTIDSFSYVDPKTTVVGEKTRSDSFSFLLLPFKDDKWLCDAYINAFGRLRVAQLF QDLDALAGRIAYRHCSPAEPVNVTASVDRVYMVKKVDEINNYNFVLAGSVTWTGRSSMEITVKGYAFED AVPDITNEESLPAENVFLAANFTFVARNPLTHKSFAINRLLPVTEKDWVDYRRAESHNAKKKLMAKNKKIL EPTAEESKLIYDMWRSSKSLQNIERANDGIAFMKDTTMKSTLFMQPQYRNRHSYMIFGGYLLRQTFELA YCTAATFSSAGPRFVSLDSTTFKNPVPVGSVLTMDSSISYTEHVHEGVEEIDADSPFNFSLPATNKISKN PEAFLSEPGTLIQVKVDTYIQELEQSVKKPAGTFIYSFYVDKESVTVDGKASFCSVIPQTYSEMMTYVGG RRRAQDTANYVETLPFSGSGN |
| SEQ ID NO:44 | Acyl-CoA Hydrolase (ACHB) Nucleotide Seq | atgatcagaaccgtccgttatcaatccttcaagaggttcaaacctctgactttatcccccgttttccgtccacgctacaactcccagaaggccaatttcc accgtccagaccacgctgggtccgacgagccagccgaagccgccgacgccgctgccacgatcctcgccgagttgcgagacaagcagacga acccgaacaaggccacctggctcgatgcgttaacggagcgggagaagttgcgtgccgagggcaagacaatcgacagcttcagctacgttgac cccaagacaaccgtcgtgggggagaagacacgcagcgactcgttctcgttcttgttgttgccgttcaaggacgacaagtggttgtgtgacgcgtac atcaatgcgtttggccggttgcgtgtagcgcagttgttccaggacttggacgccttggcgggccgcatcgcgtacaggcactgttcccccgctgagc ccgtgaatgtcacggcgagcgtggatagagtgtatatggtgaagaaagtggacgagattaataattacaatttcgtgttggcggggtccgtgacgtg gaccgggagatcgtcgatggagatcacggtcaaagggtatgcttttgaagacgccgtgccggagataactaacgaggagtccttgccggcaga gaatgtgttcttggctgttaatttcaccttcgtggcacgtaacccactcacacacaagtccttcgctattaacagattgttgcccgtgactgagaaggac tgggtcgattatcgccgtgctgagtcccacaacgccaagaagaagttgatggcaaagaacaagaagatcttggagcctaccccggaagagtcc aagttgatctacgacatgtggagatcgtccaagtccttacagaacatcgagaaggccaacgacgggatcgcgttcatgaaggacacgataatga agtccaccttgttcatgcagccccagtaccgtaacagacactcatacatgattttcggtgggtatttgttaagacaaactttcgaattggcctattgtacc gcagcaacgttttccctggcgggaccccgtttcgtcagcttggactccaccacgttcaagaaccccgtgcccgtggggtcggtgctcaccatggact cgtcgatctcgtacacggagcacgtccacgatggcgttgaggagattgacgccgactccccgttcaacttcagcttgcctgccacgaacaagatct |
| | | cgaagaaccccgaggcgttcttgtcggagcccggcacgttgatccaagtcaaggtcgacacgtacatccaggagttagagcaaagtgtgaaga agcctgcgggaacgttcatctactcgttctatgttgataaagagagcgttactgtggatggaaaggcgtcgtttgttcagttatcccgcagacgtactc cgagatgatgacttatgtgggcgggagaagaagagcccaggatactgctaattacgtggagactttgccgtttagtggaagcggcaattaa |
| SEQ ID NO:45 | Acyl-CoA Hydrolase (ACHB) | MIRTVRYQSFKRFKPLTLSPVFRPRYNSQKANFHRPDHAGSDEPAEAADAAATILAELRDKQTNPNKAT WLDALTEREKLRAEGKTIDSFSYVDPKTTVVGEKTRSDSFSFLLLPFKDDKWLCDAYINAFGRLRVAQLF QDLDALAGRIAYRHCSPAEPVNVTASVDRVYMVKKVDEINNYNFVLAGSVTWTGRSSMEITVKGYAFED AVPEITNEESLPAENVFLAVNFTFVARNPLTHKSFAINRLLPVTEKDWVDYRRAESHNAKKKLMAKNKKIL EPTPEESKLIYDMWRSSKSLQNIEKANDGIAFMKDTIMKSTLFMQPQYRNRHSYMIFGGYLLRQTFELAY CTAATFSLAGPRFVSLDSTTFKNPVPVGSVLTMDSSISYTEHVHDGVEEIDADSPFNFSLPATNKISKNPE AFLSEPGTLIQVKVDTYIQELEQSVKKPAGTFIYSFYVDKESVTVDGKASFCSVIPQTYSEMMTYVGGRR RAQDTANYVETLPFSGSGN |
| SEQ ID NO:46 | *E. coli* Acyl-CoA Thioesterase (TESA) gene without signal peptide sequence optimized for C. *tropicalis* Nucleotide Seq | atggccgatacattgctcatcttgggtgactctttgtctgcagggtatcggatgtccgcatctgccgcatggcctgcactcctcaatgacaaatggcaa agcaagacatcggtcgtgaatgcatctatctctggcgatacctcgcagcaggggttggcccgtctcccagccttgttgaagcaacatcaaccacgtt gggtcttggtcgaattgggcggcaatgatggtctcagaggttttcaacctcaacagaccgagcagacattgcgtcaaatcctccaagacgtgaagg cagcaaacgccgaacctctcttgatgcagataagattgcctgccaactatggtcgtagatacaatgaagccttttctgcaatctacccgaagcttgca aaggagtttgacgtcccattgttgccgtttttgatggaagaggtgtaccttaagcctcagtggatgcaagacgatggtatccatccgaaccgtgatgc acaaccattcatcgcagattggatggccaaacaactccaacctttggtcaatcatgatagctaa |
| SEQ ID NO:47 | *E. coli* Acyl-CoA Thioesterase (TESA) without signal peptide Amino Acid Seq | MADTLLILGDSLSAGYRMSASAAWPALLNDKWQSKTSVVNASISGDTSQQGLARLPALLKQHQPRWVL VELGGNDGLRGFQPQQTEQTLRQILQDVKAANAEPLLMQIRLPANYGRRYNEAFSAIYPKLAKEFDVPLL PFLMEEVYLKPQWMQDDGIHPNRDAQPFIADWMAKQLQPLVNHDS |
| SEQ ID NO:48 | Acyl-CoA Synthetase (ACS1) Nuc. Seq | atgggtgcccctttaacagtcgccgttggcgaagcaaaaccaggcgaaaccgctccaagaagaaaagccgctcaaaaaatggcctctgtcga acgcccaacagactcaaaggcaaccactttgccagacttcattgaagagtgttttgccagaaacggcaccagagatgccatggcctggagaga cttggtcgaaatccacgtcgaaaccaaacaggttaccaaaatcattgacggcgaacagaaaaaggtcgataaggactggatctactacgaaat gggtccttacaactacatatcctaccccaagttgttgacgttggtcaagaactactccaagggtttgttggagttgggcttggccccagatcaagaatc caagttgatgatctttgccagtacctcccacaagtggatgcagaccttcttagcctccagtttccaaggtatccccgttgtcaccgcctacgacaccttg ggtgagtcgggcttgacccactccttggtgcaaaccgaatccgatgccgtgttcaccgacaaccaattgttgtcctccttgattcgtcctttggagaag gccacctccgtcaagtatgtcatccacggggaaaagattgaccctaacgacaagagacagggcggcaaaatctaccaggatgcggaaaagg ccaaggagaagatttacaaattagaccagatattaaatttatttctttcgacgaggttgttgcattgggtgaacaatcgtccaaagaattgcatttccca aaaccagaagacccaatctgtatcatgtacacctcgggttccaccggtgctccaaagggtgtggttatcaccaatgccaacattgttgccgccgtgg gtggtatctccaccaatgctactagagacttggttagaactgtcgacagagtgattgcattttgccattggcccacattttcgagttggcctttgagttgg ttaccttctggtgggggggctccattgggttacgccaatgtcaagactttgaccgaagcctcctgcagaaactgtcagccagacttgattgaattcaaa ccaaccatcatggttggtgttgctgccgtttgggaatcggtcagaaagggtgtcttgtctaaattgaaacaggcttctccaatccaacaaaagatcttct gggctgcattcaatgccaagtctactttgaaccgttatggcttgccaggcggtgggttgtttgacgctgtcttcaagaaggttaaagccgccactggtg gccaattgcgttatgtgttgaatggtgggtccccaatctctgttgatgcccaagtgtttatctccaccttgcttgcgccaatgttgttgggttacggtttgact gaaacctgtgccaataccaccattgtcgaacacacgcgcttccagattggtactttgggtaccttggttggatctgtcactgccaagttggttgatgttg ctgatgctggatactacgccaagaacaaccagggtgaaatctggttgaaaggcggtccagttgtcaaggaatactacaagaacgaagaagaaa ccaaggctgcattcaccgaagatggctggttcaagactggtgatattggtgaatggaccgccgacggtggtttgaacatcattgaccgtaagaaga acttggtcaagactttgaatggtgaatacattgctttggagaaattggaaagtatttacagatccaaccacttgattttgaacttgtgtgtttacgctgacc aaaccaaggtcaagccaattgctattgtcttgccaattgaagccaacttgaagtctatgttgaaggacgaaaagattatcccagatgctgattcaca |
| | | agaattgagcagcttggttcacaacaagaaggttgcccaagctgtcttgagacacttgctccaaaccggtaaacaacaaggtttgaaaggtattga attgttgcagaatgttgtcttgttggatgacgagtggaccccacagaatggttttgttacttctgcccaaaagttgcagagaaagaagattttagaaagt tgtaaaaaagaagttgaagaggcatacaagtcgtcttag |
| SEQ ID NO:49 | Acyl-CoA Synthetase (ACS1) A. A. Seq | MGAPLTVAVGEAKPGETAPRRKAAQKMASVERPTDSKATTLPDFIEECFARNGTRDAMAWRDLVEIHV ETKQVTKIIDGEQKKVDKDWIYYEMGPYNYISYPKLLTLVKNYSKGLLELGLAPDQESKLMIFASTSHKW MQTFLASSFQGIPVVTAYDTLGESGLTHSLVQTESDAVFTDNQLLSSLIRPLEKATSVKYVIHGEKIDPND KRQGGKIYQDAEKAKEKILQIRPDIKFISFDEVVALGEQSSKELHFPKPEDPICIMYTSGSTGAPKGVVITN ANIVAAVGGISTNATRDLVRTVDRVIAFLPLAHIFELAFELVTFWWGAPLGYANVKTLTEASCRNCQPDLI EFKPTIMVGVAAVWESVRKGVLSKLKQASPIQQKIFWAAFNAKSTLNRYGLPGGGLFDAVFKKVKAATG GQLRYVLNGGSPISVDAQVFISTLLAPMLLGYGLTETCANTTIVEHTRFQIGTLGTLVGSVTAKLVDVADA GYYAKNNQGEIWLKGGPVVKEYYKNEEETKAAFTEDGWFKTGDIGEWTADGGLNIIDRKKNLVKTLNGE YIALEKLESIYRSNHLILNLCVYADQTKVKPIAIVLPIEANLKSMLKDEKIIPDADSQELSSLVHNKKVAQAVL RHLLQTGKQQGLKGIELLQNVVLLDDEWTPQNGFVTSAQKLQRKKILESCKKEVEEAYKSS |
| SEQ ID NO:50 | Long-chain Acyl-CoA Synthetase (FAT1) Nuc. Seq | atgtcaggattagaaatagccgctgctgccatccttggtagtcagttattggaagccaaatatttaattgccgacgacgtgctgttagccaagacagt cgctgtcaatgccctcccatacttgtggaaagccagcagaggtaaggcatcatactggtactttttcgagcagtccgtgttcaagaacccaaacaac aaagcgttggcgttcccaagaccaagaaagaatgcccccacccccaagaccgacgccgagggattccagatctacgacgatcagtttgaccta gaagaatacacctacaaggaattgtacgacatggttttgaagtactcatacatcttgaagaacgagtacggcgtcactgccaacgacaccatcggt gtttcttgtatgaacaagccgcttttcattgtcttgtggttggcattgtggaacattggtgccttgcctgcgttcttgaacttcaacaccaaggacaagccat tgatccactgtcttaagattgtcaacgcttcgcaagttttcgttgacccggactgtgattccccaatcagagataccgaggctcagatcagagaggaa ttgccacatgtgcaaataaactacattgacgagtttgccttgtttgacagattgagactcaagtcgactccaaaacacagagccgaggacaagacc agaagaccaaccgatactgactcctccgcttgtgcattgatttacacctcgggtaccaccggtttgccaaaagccggtatcatgtcctggagaaaag ccttcatggcctcggttttctttggccacatcatgaagattgactcgaaatcgaacgtcttgaccgccatgcccttgtaccactccaccgcggccatgtt ggggttgtgtcctactttgattgtcggtggctgtgtctccgtgtcccagaaattctccgctacttcgttctggacccaggccagattatgtggtgccaccca cgtgcaatacgtcggtgaggtctgtcgttacttgttgaactccaagcctcatccagaccaagacagacacaatgtcagaattgcctacggtaacgg gttgcgtccagatatatggtctgagttcaagcgcagattccacattgaaggtatcggtgagttctacgccgccaccgagtcccctatcgccaccacc aacttgcagtacggtgagtacggtgtcggcgcctgtcgtaagtacgggtccctcatcagcttgttattgtctacccagcagaaattggccaagatgga cccagaagacgagagtgaaatctacaaggaccccaagaccgggttctgtaccgaggccgcttacaacgagccaggtgagttgttgatgagaat cttgaaccctaacgacgtgcagaaatccttccagggttattatggtaacaagtccgccaccaacagcaaaatcctcaccaatgttttcaaaaaagg tgacgcgtggtacagatccggtgacttgttgaagatggacgaggacaaattgttgtactttgtcgacagattaggtgacactttccgttggaagtccga aaacgtctccgccaccgaggtcgagaacgaattgatgggctccaaggccttgaagcagtccgtcgttgtcggtgtcaaggtgccaaaccacgaa ggtagagcctgttttgccgtctgtgaagccaaggacgagttgagccatgaagaaatcttgaaattgattcactctcacgtgaccaagtctttgcctgtg tatgctcaacctgcgttcatcaagattggcaccattgaggcttcgcacaaccacaaggttcctaagaaccaattcaagaaccaaaagttgccaaa gggtgaagacggcaaggatttgatctactggttgaatggcgacaagtaccaggagttgactgaagacgattggtctttgatttgtaccggtaaagcc aaattg |
| SEQ ID NO:51 | Long-chain Acyl-CoA Synthetase (FAT1) A.A. Seq | MSGLEIAAAAILGSQLLEAKYLIADDVSLAKTVAVNALPYLWKASRGKASYWYFFEQSVFKNPNNKALAF PRPRKNAPTPKTDAEGFQIYDDQFDLEEYTYKELYDMVLKYSYILKNEYGVTANDTIGVSCMNKPLFIVL WLALWNIGALPAFLNFNTKDKPLIHCLKIVNASQVFVDPDCDSPIRDTEAQIREELPHVQINYIDEFALFDR LRLKSTPKHRAEDKTRRPTDTDSSACALIYTSGTTGLPKAGIMSWRKAFMASVFFGHIMKIDSKSNVLTA MPLYHSTAAMLGLCPTLIVGGCVSVSQKFSATSFWTQARLCGATHVQYVGEVCRYLLNSKPHPDQDRH NVRIAYGNGLRPDIWSEFKRRFHIEGIGEFYAATESPIATTNLQYGEYGVGACRKYGSLISLLLSTQQKLA KMDPEDESEIYKDPKTGFCTEAAYNEPGELLMRILNPNDVQKSFQGYYGNKSATNSKILTNVFKKGDAW |
| | | YRSGDLLKMDEDKLLYFVDRLGDTFRWKSENVSATEVENELMGSKALKQSVVVGVKVPNHEGRACFA VCEAKDELSHEEILKLIHSHVTKSLPVYAQPAFIKIGTIEASHNHKVPKNQFKNQKLPKGEDGKDLIYWLN GDKYQELTEDDWSLICTGKAKL |
| SEQ ID NO:52 | Acyl-CoA Sterol acyl transferase (ARE1) Nuc. Seq | atgtccgacgacgagatagcaggaatagtcattgaaatcgacgatgacgtgaaatccacgtcttcgttccaggaagaactagtcgaggttgaaat gtccaactcgtccattaacgaatcccagaccgatgagtcgtaccgtcctgaagaaacctcattgcattacaggaggaagtcccacaggaccccgt cagaggagtcgttcctagagatcaccaagaacgtgaatgatccggatctagtttccaagattgagaacctaaggggcaaagtaagccaacggg aagacaggttgaggaagcactaccttcacacctcccaggacgtcaagttcttgtcccggttcaacgacatcaagttcaagctgaactccgcgacg attctagattcggatgcgttttacaagagtgaatactttggagtcttgaccatcttctgggtggttatcgcactctacatattgtcaacgttgtcagatgttta ctttggcatggccaagcccttactggactggatcatcataggaatgttcaagcaggacttggtgaaagttgcactcgttgatcttgccatgtacctatcc tcgtattttccttatttcttgcaggttgcatgcaaacggggtgatgtatcttggcatggtcttggatgggcaatacagggggtttacagcttggtgtttttgac gttctggacggtagttccgcaggagttggccatggatcttccttggattgcacgaatttcttgatcttgcattgcttggtgtttattatgaagatgcagtcgt atgggcattacaatggatacctttgggatgtgtatcaggaaggattggcctctgaggctgatctcagggacctttctgagtatgatgaagatttccccct ggatcacgtggaggttctagaacagagcttgtggtttgccaaacacgagttggagtttcaatcgaatggaactgctgagaggaaggaccaccatc accatgtattcgacgaaaaggatgtcaacaaaccaatacgtgtcttgcaagaagagggaattatcaagtttccggcaaacatcaacttcaaggatt atttcgagtacagtatgttcccaacgctagtctacacgttgagcttcccccgaactcgacagattagatggacgtatgtgttgcagaaggttttgggaa catttgccttagtgtttgccatgattatcgtcgccgaagagagtttctgccccttgatgcaagaagttgatcagtacacaaaattgccaaccaaccaaa ggttcccaaaatacttcgtcgttctttcccacttgatattaccgctcggcaagcagtacttgctctcattcatcctcatctggaatgaaattctcaacggca tagcggagttaagcaggtttggcgaccggcatttctacggcgcttggtggtcgagcgtcgattacatggactattcaagaaaatggaacaccatcgt gcaccgattcctccgtcggcacgtttacaattcgagcattcacatcctcggtatttccaggacgcaagccgcgatagttacacttttgctttctgccaca atccacgaactcgttatgtacgtcctatttggcaaattacgagggtacctattccttacgatgcttgtccagatccccatgaccgtcacctccaagttca acaaccgtgtttggggcaacatcatgttctggttgacgtatttatctggccccagcttggttagtgcgttgtatttactcttctag |
| SEQ ID NO:53 | Acyl-CoA Sterol acyl transferase (ARE1) A.A. Seq | MSDDEIAGIVIEIDDDVKSTSSFQEELVEVEMSNSSINESQTDESYRPEETSLHYRRKSHRTPSEESFLEI TKNVNDPDLVSKIENLRGKVSQREDRLRKHYLHTSQDVKFLSRFNDIKFKLNSATILDSDAFYKSEYFGV LTIFWVVIALYILSTLSDVYFGMAKPLLDWIIIGMFKQDLVKVALVDLAMYLSSYFPYFLQVACKRGDVSW HGLGWAIQGVYSLVFLTFWTVVPQELAMDLPWIARIFLILHCLVFIMKMQSYGHYNGYLWDVYQEGLAS EADLRDLSEYDEDFPLDHVEVLEQSLWFAKHELEFQSNGTAERKDHHHHVFDEKDVNKPIRVLQEEGII KFPANINFKDYFEYSMFPTLVYTLSFPRTRQIRWTYVLQKVLGTFALVFAMIIVAEESFCPLMQEVDQYTK LPTNQRFPKYFVVLSHLILPLGKQYLLSFILIWNEILNGIAELSRFGDRHFYGAWWSSVDYMDYSRKWNTI VHRFLRRHVYNSSIHILGISRTQAAIVTLLLSATIHELVMYVLFGKLRGYLFLTMLVQIPMTVTSKFNNRVW GNIMFWLTYLSGPSLVSALYLLF |
| SEQ ID NO:54 | Acyl-CoA Sterol acyl transferase (ARE2) Nuc. Seq | atgtccgacgacgagatagcaggaatagtcattgaaatcgacgatgacgtgaaatctacgtcttcgttccaggaagacctagtcgaggttgagatg tccaactcgtccattaacgaatcccagacggatgagttgtcgtaccgtcctgaagaaatctcattgcattcgagaaggaagtcccacaagaccccg tcagatgagtcgttcctagagatcaccaagaacgtgaatgatccggatctagtctccaagattgagaacttaaggggcaaagtaagccaacggg aagacaggttgaggaaacactacctccacacatcccaggacgtcaagttcttgtctcggttcaacgacatcaagttcaagctgaactccgcgacg attctagattcggatgcgttttacaagagcgagcactttggagtcttgactatcttctgggtggttatcggactctacataatgtcaacgttgtcagacatg tattttggcatggccaagcccttactggactggataatcataggaatgttcaagaaggatttgatgcaagttgcactcgttgatcttgtcatgtacttatcc tcgtattttccttatttcctacaggttgcatgcaagaccggagctatatcttggcatggtcttggatgggccatacaggggggtttacagcttggtgtttttaa ctttctgggcggtacttccgctggagctggccatggatcttccttggattgcacgagttttcttgatcttgcattgcttggtgtttattatgaagatgcaatcat atggacattacaatggatacctttgggatgtatatcaggaaggattggtctcggaagctgatctcacggctgtttctgagtatgatgatgatttccccctg gatcacggggaggttctagaacagagcttgtggttcgccaaacacgagttggagtttcaatctaatggaactacggagaggaaggatcaccatca tcatgtattcgacgaaaaggatgtcaacaaaccaatgcgtgtcttgcaagaagagggaattatcaaatttccggcaaacatcaatttcaaggattatt |
| | | tcgagtacagtatgttccccacgctagtctacacattgaacttccccagaattcgacatattagatgggcgtatgtgttgcagaaagttttgggaacatt tgccttagtgtttgccatgattatcgtcgccgaagagagtttctgtcccttgatgcaagaagttgaacagtacacaagattgccaaccaaccaaaggtt ctcaaagtacttcgtcgttctttcccacttgatattgcccctcggcaaacagtacttgctctcgtttatcctcatttggaacgaaattctcaacgggatagc ggagttaagcaggtttggggatcgccatttctacggcgcctggtggtcaagcgtcgactacatggactattcaagaaaatggaacacgatcgtgca ccgattcctccgccggcacgtttacaattcgaccattcgcatcctcggtatttccaggacccaagccgcgataattacacttttgctttcagccacaatc cacgaactcgttatgtacatcctatttggaaaattacgagggtacctattccttacgatgcttgtccagatccccatgacagtcaccgccaagttcaac aacccatttgtggggcaacatcatgttctggttgacgtatttatctggccccagcttggttagtgcgttgtatttactcttctga |
| SEQ ID NO:55 | Acyl-CoA Sterol acyl transferase (ARE2) A.A. Seq | MSDDEIAGIVIEIDDDVKSTSSFQEDLVEVEMSNSSINESQTDELSYRPEEISLHSRRKSHKTPSDESFLEI TKNVNDPDLVSKIENLRGKVSQREDRLRKHYLHTSQDVKFLSRFNDIKFKSNSATILDSDAFYKSEHFGV LTIFWVVIGLYIMSTLSDMYFGMAKPLSDWIIIGMFKKDLMQVALVDLVMYLSSYFPYFLQVACKTGAISW HGLGWAIQGVYSLVFLTFWAVLPSESAMDLPWIARVFLILHCLVFIMKMQSYGHYNGYLWDVYQEGLVS EADLTAVSEYDDDFPSDHGEVLEQSLWFAKHELEFQSNGTTERKDHHHHVFDEKDVNKPMRVLQEEGI IKFPANINFKDYFEYSMFPTLVYTLNFPRIRHIRWAYVLQKVLGTFALVFAMIIVAEESFCPLMQEVEQYTR LPTNQRFSKYFVVLSHLILPLGKQYLLSFILIWNEILNGIAELSRFGDRHFYGAWWSSVDYMDYSRKWNTI VHRFLRRHVYNSTIRILGISRTQAAIITLLLSATIHELVMYILFGKLRGYLFLTMLVQIPMTVTAKFNNRLWG NIMFWLTYLSGPSLVSALYLLF |
| SEQ ID NO:56 | Diacylglycerol acyltransferase (DGA1) Nuc. Seq | atgactcaggactataaagacgatagtcctacgtccactgagttggacactaacatagaagaggtggaaagcactgcaaccctagagtcggaa ctcagacagagaaaacagaccacggaaactccagcatcaaccccaccaccacctccacaacaacagcaggcgcataagaaagccctgaa gaatggcaagaggaagagaccatttataaacgtggcgccgctcaacaccccgttggctcacaggctcgagactttggctgttgtttggcactgtgtc agtatcccgttctttatgtttttgttcttgcttacggtctccatggggttgcttgggtggttctttatcattttgccatatttcatttggtggtacggtttcgacttgca cactccatcgaatggtaaagttgtctatcgtgtgcgcaactcgttcaagaatttcatcatttgggactggtttgtcaagtatttcccgattgaagtgcaca agacggtcgagttggatcctacttttagcgaattgcctgtggaagagagcggcgacagttcggacgacgacgaacaagacttggtgtctgagcac agcagaactttggttgatcaaatcttcaagtttttcgggttgaagaaacgcttgaatgacacctccctgggcaaaccagagacattcaagaatgtgc ctacgggtccaaggtatatttttgggtaccacccacacggagtgatttctatgggggcagtggggttgtttgccaacaacgccttgaggaacgaacc atatacgccaatttccaaatggttaaaaccattcttccacgacagctccaagggcgagagattgttccctggtattggcaatatcttcccattgacgctt accacacagtttgcgctcccattttaccgtgactacttgatggctttggggatcactagtgcatcggctaaaaacattagaagcttgatcaacaatgga gacaactctgtgtgtctcgtcgttggcggtgcacaagaatcgttgttgaacaatatgattgccaagcacgccagagtcgggtacggttacaaagag agcctagatattcatggcgaccagtccgaagaagaagaagaagaagaggatgataccaagcagctagagaacccaagtcctaaacgtgaag tgcaattggtcttgaacaaacgtaaaggttttgtgaagttggctatcgaactaggaaatgtttccttggtgcctatttttgcattcggagaagctgatgttta cagattggcccagccagcaccaggctcgttcttgtacaagttccagcaatggatgaaggcaactttcaattcaccatcccattgtttagtgctcgagg cgtgttcatctatgatttcggattgttgccattcagaaacccaataaacatttgcgtcggtagacccgtctacattccgcacaacgtcttgcaagaatac aagcaaaagcacccagaggagtttgccgaagaggaacctgccagtaccccgatgaagaagtctggatctttcaccgatatgttcaaagctggtg aaaagaagcccaagacttcaagtatcaagactaaaatcccacctgcattactagacaagtaccacaagctatacgtcgacgagttgaagaaggt ctatgaagagaacaaggaaaggtttggctacggtgatgttgaattaaacattgtagaatag |
| SEQ ID NO:57 | Diacylglycerol acyltransferase (DGA1) A.A. Seq | MTQDYKDDSPTSTELDTNIEEVESTATLESELRQRKQTTETPASTPPPPPQQQAHKKASKNGKRKRP FINVAPLNTPLAHRLETLAVVWHCVSIPFFMFLFLLTVSMGLLGWFFIILPYFIWWYGFDLHTPSNGKVVY RVRNSFKNFIIWDWFVKYFPIEVHKTVELDPTFSELPVEESGDSSDDDEQDLVSEHSRTLVDQIFKFFGL KKRLNDTSSGKPETFKNVPTGPRYIFGYHPHGVISMGAVGLFANNALRNEPYTPISKWLKPFFHDSSKG ERLFPGIGNIFPLTLTTQFALPFYRDYLMALGITSASAKNIRSLINNGDNSVCLVVGGAQESLLNNMIAKHA RVGYGYKESLDIHGDQSEEEEEEEDDTKQLENPSPKREVQLVLNKRKGFVKLAIELGNVSLVPIFAFGEA DVYRLAQPAPGSFLYKFQQWMKATFQFTIPLFSARGVFIYDFGLLPFRNPINICVGRPVYIPHNVLQEYK |
| | | QKHPEEFAEEEPASTPMKKSGSFTDMFKAGEKKPKTSSIKTKIPPALLDKYHKLYVDELKKVYEENKERF GYGDVELNIVE |
| SEQ ID NO:58 | Diacylglycerol acyltransferase (LRO1) Nuc. Seq | atgtcgtctttaaagaacagaaaatccgcaagcgtcgccacaagcgatacagaagactcagaaacagaggcagtatcctcctcaattgatccca acggcaccatattgcgaccagtcctacatgacgaaccccaccacagccatcaccaccacaacataactagaccagtattggaggacgatggca gcatcctggtgtccagaagatcgtcgatctccaaatccgacgacctgcaggcaaagcaaaagaagaagaaacccaagaagaagatcttggag tctcgtcgggtcatgtttatctttggtaccctcattgggttaatctttgcgtgggcgtttaccacagacacgcatcctttcaatggcgacttggagaagtttat caactttgaccagctcaacgggatctttgacgactggaagaactggaaggatatcttgcccaacagcatccagacgtacttgcaggaatcgggca agggcgaagataacgacgggttgcatggtctggccgattccttctccgtcgggctccgcttgaaagcccagaagaacttcactgacaaccacaat gtcgtgttggttcctggtgtggtgagcacggggttggaatcgtggggaacaaccaccaccggtgattgtccatctatcggatacttcaggaagagatt gtggggatcattttatatgttaaggacaatgattttggagaaaacgtgctggttgaagcatatccagttggacgagaagacggggttggatcctccca atattaaggtccgtgcggcgcagggtttcgaagcggcagatttctttatggctgggtactggatctggaacaagatcttgcagaacttggcggttattg ggtacggaccaaataacatggtgagtgctagttatgactggagattggcttacattgacttggagagaagagatggatatttttcgaaacttaaagcg cagattgagttgaataacaagttgaacaacaagaagactgtgttgattggccactcgatggggacccagattattttctacttttttgaaatgggtcgaa gccaccgggaaaccatactatggcaatggcggaccaaactgggtgaatgatcatattgagtcgattattgacatcagtgggtcgactttgggtacc cccaagagtattcctgtgttgatctctggggaaatgaaagacaccgttcaattgaacgcgttggcggtttacgggttggagcaatttttcagcaggcgt gaaagagtcgatatgttgcgtacatttggtggcgttgccagtatgttacccaagggggggagacaagatatggggcaacttgacgcatgcgccagat gatccaatttccacattcagtgatgacgaagttacggacagccacgaacctaaagatcgttcttttggtacgtttatccaattcaagaaccaaactag cgacgctaagccatacagggagatcaccatggctgaaggtatcgatgaattgttggacaaatcaccagactggtattccaagagagtccgtgag aactactcttacggcattacagacagcaaggcgcaattagagaagaacaacaatgaccacctgaagtggtcgaacccattagaagctgccttgc ctaaagcacccgacatgaagatctattgtttctacggagttggaaatcctaccgaaagggcatacaagtatgtgactgccgataaaaaagccacg aaattggactacataatagacgccgacgatgccaatggagtcatattaggagacggagacggcactgtttcgttattaacccactcgatgtgccat gagtgggccaagggagacaagtcgagatacaacccagccaactcgaaggttaccattgttgaaatcaagcacgagccagacagatttgattta cgaggcggcgccaagactgcggaacatgttgatattttggggagtgccgagttgaacgagttgattttgactgtggttagcgggaacggggacga gattgagaatagatatgtcagcaacttaaaagaaatagtagaggccataaatttataa |
| SEQ ID NO:59 | Diacylglycerol acyltransferase (LRO1) A.A. Seq | MSSLKNRKSASVATSDTEDSETEAVSSSIDPNGTILRPVLHDEPHHSHHHHNITRPVLEDDGSISVSRRS SISKSDDSQAKQKKKKPKKKILESRRVMFIFGTLIGLIFAWAFTTDTHPFNGDLEKFINFDQLNGIFDDWK NWKDILPNSIQTYLQESGKGEDNDGLHGSADSFSVGLRLKAQKNFTDNHNVVLVPGVVSTGLESWGTT TTGDCPSIGYFRKRLWGSFYMLRTMILEKTCWLKHIQLDEKTGLDPPNIKVRAAQGFEAADFFMAGYWI WNKILQNLAVIGYGPNNMVSASYDWRLAYIDLERRDGYFSKLKAQIELNNKLNNKKTVLIGHSMGTQIIFY FLKWVEATGKPYYGNGGPNWVNDHIESIIDISGSTLGTPKSIPVLISGEMKDTVQLNALAVYGLEQFFSR RERVDMLRTFGGVASMLPKGGDKIWGNLTHAPDDPISTFSDDEVTDSHEPKDRSFGTFIQFKNQTSDA KPYREITMAEGIDELLDKSPDWYSKRVRENYSYGITDSKAQLEKNNNDHSKWSNPLEAALPKAPDMKIY CFYGVGNPTERAYKYVTADKKATKLDYIIDADDANGVILGDGDGTVSLLTHSMCHEWAKGDKSRYNPAN SKVTIVEIKHEPDRFDLRGGAKTAEHVDILGSAELNELILTVVSGNGDEIENRYVSNLKEIVEAINL |

### Example 52: Nucleotide and Amino Acid Sequences of Monooxygenase Activities Described Herein

| **SEQ ID NO:** | **Description** | **Sequence** |
|---|---|---|
| SEQ ID NO: 60 | Cytochrome P450 monooxygenase CYP52A12 Nucleotide Seq. | atggccacacaagaaatcatcgattctgtacttccgtacttgaccaaatggtacactgtgattactgcagcagtattagtcttccttatctccacaaaca tcaagaactacgtcaaggcaaagaaattgaaatgtgtcgatccaccatacttgaaggatgccggtctcactggtattctgtctttgatcgccgccatc aaggccaagaacgacggtagattggctaactttgccgatgaagttttcgacgagtacccaaaccacaccttctacttgtctgttgccggtgctttgaa gattgtcatgactgttgacccagaaaacatcaaggctgtcttggccacccaattcactgacttctccttgggtaccagacacgcccactttgctcctttg ttgggtgacggtatcttcaccttggacggagaaggttggaagcactccagagctatgttgagaccacagtttgctagagaccagattggacacgtt aaagccttggaaccacacatccaaatcatggctaagcagatcaagttgaaccagggaaagactttcgatatccaagaattgttctttagatttaccg tcgacaccgctactgagttcttgtttggtgaatccgttcactccttgtacgatgaaaaattgggcatcccaactccaaacgaaatcccaggaagaga aaactttgccgctgctttcaacgtttcccaacactacttggccaccagaagttactcccagactttttacttttgaccaaccctaaggaattcagagact gtaacgccaaggtccaccacttggccaagtactttgtcaacaaggccttgaactttactcctgaagaactcgaagagaaatccaagtccggttacg ttttcttgtacgaattggttaagcaaaccagagatccaaaggtcttgcaagatcaattgttgaacattatggttgccggaagagacaccactgccggt ttgttgtcctttgctttgtttgaattggctagacacccagagatgtggtccaagttgagagaagaaatcgaagttaactttggtgttggtgaagactcccg cgttgaagaaattaccttcgaagccttgaagagatgtgaatacttgaaggctatccttaacgaaaccttgcgtatgtacccatctgttcctgtcaacttt agaaccgccaccagagacaccactttgccaagaggtggtggtgctaacggtaccgacccaatctacattcctaaaggctccactgttgcttacgtt gtctacaagacccaccgtttggaagaatactacggtaaggacgctaacgacttcagaccagaaagatggtttgaaccatctactaagaagttggg ctgggcttatgttccattcaacggtggtccaagagtctgcttgggtcaacaattcgccttgactgaagcttcttatgtgatcactagattggcccagatgt ttgaaactgtctcatctgatccaggtctcgaataccctccaccaaagtgtattcacttgaccatgagtcacaacgatggtgtctttgtcaagatgtaa |
| SEQ ID NO: 61 | Cytochrome P450 monooxygenase CYP52A12 A.A. Seq. | matqeiidsvlpyltkwytvitaavlvflistniknyvkakklkcvdppylkdagltgissliaaikakndgrlanfadevfdeypnhtfylsvagalkivmt vdpenikavlatqftdfslgtrhahfapllgdgiftldgegwkhsramlrpqfardqighvkalephiqimakqiklnqgktfdiqelfffrftvdtateflfge svhslydeklgiptpneipgrenfaaafnvsqhylatrsysqtfyfltnpkefrdcnakvhhlakyfvnkalnftpeeleeksksgyvflyelvkqtrdpk vlqdqllnimvagrdttagllsfalfelarhpemwsklreeievnfgvgedsrveeitfealkrceylkailnetlrmypsvpvnfrtatrdttlprgggang tdpiyipkgstvayvvykthrleeyygkdandfrperwfepstkklgwayvpfnggprvclgqqfalteasyvitrlaqmfetvssdpgleypppkci hltmshndgvfvkm |
| SEQ ID NO: 62 | Cytochrome P450 monooxygenase CYP52A13 Nucleotide Seq. | atgactgtacacgatattatcgccacatacttcaccaaatggtacgtgatagtaccactcgctttgattgcttatagagtcctcgactacttctatggcag atacttgatgtacaagcttggtgctaaaccatttttccagaaacagacagacggctgtttcggattcaaagctccgcttgaattgttgaagaagaaga gcgacggtaccctcatagacttcacactccagcgtatccacgatctcgatcgtcccgatatcccaactttcacattcccggtcttttccatcaaccttgt caataccccttgagccggagaacatcaaggccatcttggccactcagttcaacgatttctccttgggtaccagacactcgcactttgctcctttgttgggt gatggtatctttacgttggatggcgccggctggaagcacagcagatctatgttgagaccacagtttgccagagaacagatttcccacgtcaagttgtt ggagccacacgttcaggtgttcttcaaacacgtcagaaaggcacagggcaagacttttgacatccaggaattgtttttcagattgaccgtcgactcc gccaccgagtttttgtttggtgaatccgttgagtccttgagagatgaatctatcggcatgtccatcaatgcgcttgactttgacggcaaggctggctttgc tgatgcttttaactattcgcagaattatttggcttcgagagcggttatgcaacaattgtactgggtgttgaacgggaaaaagtttaaggagtgcaacgc taaagtgcacaagtttgctgactactacgtcaacaaggctttggacttgacgcctgaacaattggaaaagcaggatggttatgtgtttttgtacgaatt ggtcaagcaaaccagagacaagcaagtgttgagagaccaattgttgaacatcatggttgctggtagagacaccaccgccggtttgttgtcgtttgtt ttctttgaattggccagaaacccagaagttaccaacaagttgagagaagaaattgaggacaagtttggactcggtgagaatgctagtgttgaaga catttcctttgagtcgttgaagtcctgtgaatacttgaaggctgttctcaacgaaaccttgagattgtacccatccgtgccacagaatttcagagttgcc accaagaacactaccctcccaagaggtggtggtaaggacgggttgtctcctgttttggtgagaaagggtcagaccgttatttacggtgtctacgcag cccacagaaacccagctgtttacggtaaggacgctcttgagtttagaccagagagatggtttgagccagagacaaagaagcttggctgggccttc ctcccattcaacggtggtccaagaatctgtttgggacagcagtttgccttgacagaagcttcgtatgtcactgtcaggttgctccaggagtttgcacact |
| | | tgtctatggacccagacaccgaatatccacctaagaaaatgtcgcatttgaccatgtcgcttttcgacggtgccaatattgagatgtattag |
| SEQ ID NO: 63 | Cytochrome P450 monooxygenase CYP52A13 A.A. Seq. | mtvhdiiatyftkwyvivplaliayrvldyfygrylmyklgakpffqkqtdgcfgfkaplellkkksdgtlidftlqrihdldrpdiptftfpvfsinlvntlepeni kailatqfndfslgtrhshfapllgdgiftldgagwkhsrsmlrpqfareqishvkllephvqvffkhvrkaqgktfdiqelffrltvdsateflfgesveslrd esigmsinaldfdgkagfadafnysqnylasravmqqlywvlngkkfkecnakvhkfadyyvnkaldltpeqlekqdgyvflyelvkqtrdkqvlrd qllnimvagrdttagllsfvffelarnpevtnklreeiedkfglgenasvedisfeslksceylkavlnetlrlypsvpqnfrvatknttlprgggkdglspvl vrkgqtviygvyaahrnpavygkdalefrperwfepetkklgwaflpfnggpriclgqqfalteasyvtvrllqefahlsmdpdteyppkkmshltms lfdganiemy |
| SEQ ID NO: 64 | Cytochrome P450 monooxygenase CYP52A14 Nucleotide Seq. | atgactgcacaggatattatcgccacatacatcaccaaatggtacgtgatagtaccactcgctttgattgcttatagggtcctcgactacttttacggca gatacttgatgtacaagcttggtgctaaaccgtttttccagaaacaaacagacggttatttcggattcaaagctccacttgaattgttaaaaaagaag agtgacggtaccctcatagacttcactctcgagcgtatccaagcgctcaatcgtccagatatcccaacttttacattcccaatcttttccatcaaccttat cagcacccttgagccggagaacatcaaggctatcttggccacccagttcaacgatttctccttgggcaccagacactcgcactttgctcctttgttgg gcgatggtatctttaccttggacggtgccggctggaagcacagcagatctatgttgagaccacagtttgccagagaacagatttcccacgtcaagtt gttggagccacacatgcaggtgttcttcaagcacgtcagaaaggcacagggcaagacttttgacatccaagaattgtttttcagattgaccgtcgac tccgccactgagtttttgtttggtgaatccgttgagtccttgagagatgaatctattgggatgtccatcaatgcacttgactttgacggcaaggctggcttt gctgatgcttttaactactcgcagaactatttggcttcgagagcggttatgcaacaattgtactgggtgttgaacgggaaaaagtttaaggagtgcaa cgctaaagtgcacaagtttgctgactattacgtcagcaaggctttggacttgacacctgaacaattggaaaagcaggatggttatgtgttcttgtacg agttggtcaagcaaaccagagacaggcaagtgttgagagaccagttgttgaacatcatggttgccggtagagacaccaccgccggtttgttgtcgt ttgttttctttgaattggccagaaacccagaggtgaccaacaagttgagagaagaaatcgaggacaagtttggtcttggtgagaatgctcgtgttga agacatttcctttgagtcgttgaagtcatgtgaatacttgaaggctgttctcaacgaaactttgagattgtacccatccgtgccacagaatttcagagttg ccaccaaaaacactacccttccaaggggaggtggtaaggacgggttatctcctgttttggtcagaaagggtcaaaccgttatgtacggtgtctacg ctgcccacagaaacccagctgtctacggtaaggacgcccttgagtttagaccagagaggtggtttgagccagagacaaagaagcttggctggg ccttccttccattcaacggtggtccaagaatttgcttgggacagcagtttgccttgacagaagcttcgtatgtcactgtcagattgctccaagagtttgg acacttgtctatggaccccaacaccgaatatccacctaggaaaatgtcgcatttgaccatgtcccttttcgacggtgccaacattgagatgtattag |
| SEQ ID NO: 65 | Cytochrome P450 monooxygenase CYP52A14 A.A. Seq. | mtaqdiiatyitkwyvivplaliayrvldyfygrylmyklgakpffqkqtdgyfgfkaplellkkksdgtlidftleriqalnrpdiptftfpifsinlistlepenik ailatqfndfslgtrhshfapllgdgiftldgagwkhsrsmlrpqfareqishvkllephmqvffkhvrkaqgktfdiqelfffrltvdsateflfgesveslrde sigmsinaldfdgkagfadafnysqnylasravmqqlywvlngkkfkecnakvhkfadyyvskaldltpeqlekqdgyvflyelvkqtrdrqvlrdql lnimvagrdttagllsfvffelarnpevtnklreeiedkfglgenarvedisfeslksceylkavlnetlrlypsvpqnfrvatknttlprgggkdglspvlvrk gqtvmygvyaahrnpavygkdalefrperwfepetkklgwaflpfnggpriclgqqfalteasyvtvrllqefghlsmdpnteypprkmshltmslf dganiemy |
| SEQ ID NO: 66 | Cytochrome P450 monooxygenase CYP52A15 Nucleotide Seq. | atgtcgtcttctccatcgtttgcccaagaggttctcgctaccactagtccttacatcgagtactttcttgacaactacaccagatggtactacttcatacctt tggtgcttctttcgttgaactttataagtttgctccacacaaggtacttggaacgcaggttccacgccaagccactcggtaactttgtcagggaccctac gtttggtatcgctactccgttgcttttgatctacttgaagtcgaaaggtacggtcatgaagtttgcttggggcctctggaacaacaagtacatcgtcaga gacccaaagtacaagacaactgggctcaggattgttggcctcccattgattgaaaccatggacccagagaacatcaaggctgttttggctactca gttcaatgatttctctttgggaaccagacacgatttcttgtactccttgttgggtgacggtattttcaccttggacggtgctggctggaaacatagtagaac tatgttgagaccacagtttgctagagaacaggtttctcacgtcaagttgttggagccacacgttcaggtgttcttcaagcacgttagaaagcaccgcg gtcaaacgttcgacatccaagaattgttcttcaggttgaccgtcgactccgccaccgagttcttgtttggtgagtctgctgaatccttgagggacgaatc tattggattgaccccaaccaccaaggatttcgatggcagaagagatttcgctgacgctttcaactattcgcagacttaccaggcctacagatttttgtt gcaacaaatgtactggatcttgaatggctcggaattcagaaagtcgattgctgtcgtgcacaagtttgctgaccactatgtgcaaaaggctttggagt tgaccgacgatgacttgcagaaacaagacggctatgtgttcttgtacgagttggctaagcaaaccagagacccaaaggtcttgagagaccagtta ttgaacattttggttgccggtagagacacgaccgccggtttgttgtcatttgttttctacgagttgtcaagaaaccctgaggtgtttgctaagttgagaga ggaggtggaaaacagatttggactcggtgaagaagctcgtgttgaagagatctcgtttgagtccttgaagtcttgtgagtacttgaaggctgtcatca |
| | | atgaaaccttgagattgtacccatcggttccacacaactttagagttgctaccagaaacactaccctcccaagaggtggtggtgaagatggatact cgccaattgtcgtcaagaagggtcaagttgtcatgtacactgttattgctacccacagagacccaagtatctacggtgccgacgctgacgtcttcag accagaaagatggtttgaaccagaaactagaaagttgggctgggcatacgttccattcaatggtggtccaagaatctgtttgggtcaacagtttgcc ttgaccgaagcttcatacgtcactgtcagattgctccaggagtttgcacacttgtctatggacccagacaccgaatatccaccaaaattgcagaaca ccttgaccttgtcgctctttgatggtgctgatgttagaatgtactaa |
| SEQ ID NO: 67 | Cytochrome P450 monooxygenase CYP52A15 A.A. Seq. | msssspsfaqevlattspyieyfldnytrwyyfiplvllslnfisllhtrylerrfhakplgnfvrdptfgiatplllliylkskgtvmkfawglwnnkyivrdpkykt tglrivglplietmdpenikavlatqfndfslgtrhdflysllgdgiftldgagwkhsrtmlrpqfareqvshvkllephvqvffkhvrkhrgqtfdiqelffrltv dsateflfgesaeslrdesigltpttkdfdgrrdfadafnysqtyqayrfllqqmywilngsefrksiavvhkfadhyvqkaleltdddlqkqdgyvflyel akqtrdpkvlrdqllnilvagrdttagllsfvfyelsrnpevfaklreevenrfglgeearveeisfeslksceylkavinetlrlypsvphnfrvatrnttlprg ggedgyspivvkkgqvvmytviathrdpsiygadadvfrperwfepetrklgwayvpfnggpriclgqqfalteasyvtvrllqefahlsmdpdtey ppklqntltlslfdgadvrmy |
| SEQ ID NO: 68 | Cytochrome P450 monooxygenase CYP52A16 Nucleotide Seq. | atgtcgtcttctccatcgtttgctcaggaggttctcgctaccactagtccttacatcgagtactttcttgacaactacaccagatggtactacttcatcccttt ggtgcttctttcgttgaacttcatcagcttgctccacacaaagtacttggaacgcaggttccacgccaagccgctcggtaacgtcgtgttggatcctac gtttggtatcgctactccgttgatcttgatctacttaaagtcgaaaggtacagtcatgaagtttgcctggagcttctggaacaacaagtacattgtcaaa gacccaaagtacaagaccactggccttagaattgtcggcctcccattgattgaaaccatagacccagagaacatcaaagctgtgttggctactca gttcaacgatttctccttgggaactagacacgatttcttgtactccttgttgggcgatggtattttaccttggacggtgctggctggaaacacagtagaa ctatgttgagaccacagtttgctagagaacaggtttcccacgtcaagttgttggaaccacacgttcaggtgttcttcaagcacgttagaaaacaccg cggtcagacttttgacatccaagaattgttcttcagattgaccgtcgactccgccaccgagttcttgtttggtgagtctgctgaatccttgagagacgact ctgttggtttgaccccaaccaccaaggatttcgaaggcagaggagatttcgctgacgctttcaactactcgcagacttaccaggcctacagatttttgt tgcaacaaatgtactggattttgaatggcgcggaattcagaaagtcgattgccatcgtgcacaagtttgctgaccactatgtgcaaaaggctttgga gttgaccgacgatgacttgcagaaacaagacggctatgtgttcttgtacgagttggctaagcaaactagagacccaaaggtcttgagagaccagt tgttgaacattttggttgccggtagagacacgaccgccggtttgttgtcgtttgtgttctacgagttgtcgagaaaccctgaagtgtttgccaagttgaga gaggaggtggaaaacagatttggactcggcgaagaggctcgtgttgaagagatctcttttgagtccttgaagtcctgtgagtacttgaaggctgtcat caatgaagccttgagattgtacccatctgttccacacaacttcagagttgccaccagaaacactacccttccaagaggcggtggtaaagacggat gctcgccaattgttgtcaagaagggtcaagttgtcatgtacactgtcattggtacccacagagacccaagtatctacggtgccgacgccgacgtctt cagaccagaaagatggttcgagccagaaactagaaagttgggctgggcatatgttccattcaatggtggtccaagaatctgtttgggtcagcagttt gccttgactgaagcttcatacgtcactgtcagattgctccaagagtttggaaacttgtccctggatccaaacgctgagtacccaccaaaattgcaga acaccttgaccttgtcactctttgatggtgctgacgttagaatgttctaa |
| SEQ ID NO: 69 | Cytochrome P450 monooxygenase CYP52A16 A.A. Seq. | msssspsfaqevlattspyieyfldnytrwyyfiplvllslnfisllhtkylerrfhakplgnvvldptfgiatplilyilkskgtvmkfawsfwnnkyivkdpky kttglrivglplietidpenikavlatqfndfslgtrhdflysllgdgiftldgagwkhsrtmlrpqfareqvshvkllephvqvffkhvrkhrgqtfdiqelffrlt vdsateflfgesaeslrddsvgltpttkdfegrgdfadafnysqtyqayrfllqqmywilngaefrksiaivhkfadhyvqkaleltdddlqkqdgyvfly elakqtrdpkvlrdqllnilvagrdttagllsfvfyelsrnpevfaklreevenrfglgeearveeisfeslksceylkavinealrlypsvphnfrvatrnttlp rgggkdgcspivvkkgqvvmytvigthrdpsiygadadvfrperwfepetrklgwayvpfnggpriclgqqfalteasyvtvrllqefgnlssdpnae yppklqntltlslfdgadvrmf |
| SEQ ID NO: 70 | Cytochrome P450 monooxygenase CYP52A17 Nucleotide Seq. | atgattgaacaactcctagaatattggtatgtcgttgtgccagtgttgtacatcatcaaacaactccttgcatacacaaagactcgcgtcttgatgaaa aagttgggtgctgctccagtcacaaacaagttgtacgacaacgctttcggtatcgtcaatggatggaaggctctccagttcaagaaagagggcag ggctcaagagtacaacgattacaagtttgaccactccaagaacccaagcgtgggcacctacgtcagtattcttttcggcaccaggatcgtcgtgac caaagatccagagaatatcaaagctattttggcaacccagtttggtgatttttctttgggcaagaggcacactctttttaagcctttgttaggtgatggga tcttcacattggacggcgaaggctggaagcacagcagagccatgttgagaccacagtttgccagagaacaagttgctcatgtgacgtcgttggaa ccacacttccagttgttgaagaagcatattcttaagcacaagggtgaatactttgatatccaggaattgttctttagatttaccgttgattcggccacgga gttcttatttggtgagtccgtgcactccttaaaggacgaatctattggtatcaaccaagacgatatagattttgctggtagaaaggactttgctgagtcgt |
| | | tcaacaaagcccaggaatacttggctattagaaccttggtgcagacgttctactggttggtcaacaacaaggagtttagagactgtaccaagctggt gcacaagttcaccaactactatgttcagaaagctttggatgctagcccagaagagcttgaaaagcaaagtgggtatgtgttcttgtacgagcttgtc aagcagacaagagaccccaatgtgttgcgtgaccagtctttgaacatcttgttggccggaagagacaccactgctgggttgttgtcgtttgctgtcttt gagttggccagacacccagagatctgggccaagttgagagaggaaattgaacaacagtttggtcttggagaagactctcgtgttgaagagattac ctttgagagcttgaagagatgtgagtacttgaaagcgttccttaatgaaaccttgcgtatttacccaagtgtcccaagaaacttcagaatcgccacca agaacacgacattgccaaggggcggtggttcagacggtacctcgccaatcttgatccaaaagggagaagctgtgtcgtatggtatcaactctact catttggaccctgtctattacggccctgatgctgctgagttcagaccagagagatggtttgagccatcaaccaaaaagctcggctgggcttacttgcc attcaacggtggtccaagaatctgtttgggtcagcagtttgccttgacggaagctggctatgtgttggttagattggtgcaagagttctcccacgttagg ctggacccagacgaggtgtacccgccaaagaggttgaccaacttgaccatgtgtttgcaggatggtgctattgtcaagtttgactag |
| SEQ ID NO: 71 | Cytochrome P450 monooxygenase CYP52A17 A.A. Seq. | mieqlleywyvvvpvlyiikqllaytktrvlmkklgaapvtnklydnafgivngwkalqfkkegraqeyndykfdhsknpsvgtyvsilfgtrivvtkdp enikailatqfgdfslgkrhtlfkpllgdgiftldgegwkhsramlrpqfareqvahvtslephfqllkkhilkhkgeyfdiqelfffrftvdsateflfgesvhsl kdesiginqddidfagrkdfaesfnkaqeylairtlvqtfywlvnnkefrdctksvhkftnyyvqkaldaspeelekqsgyvflyelvkqtrdpnvlrdq slnillagrdttagllsfavfelarhpeiwaklreeieqqfglgedsrveeitfeslkrceylkaflnetlriypsvprnfriatknttlprgggsdgtspiliqkge avsyginsthldpvyygpdaaefrperwfepstkklgwaylpfnggpriclgqqfalteagyvlvrlvqefshvrsdpdevyppkrltnltmclqdgai vkfd |
| SEQ ID NO: 72 | Cytochrome P450 monooxygenase CYP52A18 Nucleotide Seq. | atgattgaacaaatcctagaatattggtatattgttgtgcctgtgttgtacatcatcaaacaactcattgcctacagcaagactcgcgtcttgatgaaac agttgggtgctgctccaatcacaaaccagttgtacgacaacgttttcggtatcgtcaacggatggaaggctctccagttcaagaaagagggcaga gctcaagagtacaacgatcacaagtttgacagctccaagaacccaagcgtcggcacctatgtcagtattctttttggcaccaagattgtcgtgacca aggatccagagaatatcaaagctattttggcaacccagtttggcgatttttctttgggcaagagacacgctctttttaaacctttgttaggtgatgggatc ttcaccttggacggcgaaggctggaagcatagcagatccatgttaagaccacagtttgccagagaacaagttgctcatgtgacgtcgttggaacc acacttccagttgttgaagaagcatatccttaaacacaagggtgagtactttgatatccaggaattgttctttagatttactgtcgactcggccacggag ttcttatttggtgagtccgtgcactccttaaaggacgaaactatcggtatcaaccaagacgatatagattttgctggtagaaaggactttgctgagtcgt tcaacaaagcccaggagtatttgtctattagaattttggtgcagaccttctactggttgatcaacaacaaggagtttagagactgtaccaagctggtg cacaagtttaccaactactatgttcagaaagctttggatgctaccccagaggaacttgaaaagcaaggcgggtatgtgttcttgtatgagcttgtcaa gcagacgagagaccccaaggtgttgcgtgaccagtctttgaacatcttgttggcaggaagagacaccactgctgggttgttgtcctttgctgtgtttga gttggccagaaacccacacatctgggccaagttgagagaggaaattgaacagcagtttggtcttggagaagactctcgtgttgaagagattacctt tgagagcttgaagagatgtgagtacttgaaagcgttccttaacgaaaccttgcgtgtttacccaagtgtcccaagaaacttcagaatcgccaccaa gaatacaacattgccaaggggtggtggtccagacggtacccagccaatcttgatccaaaagggagaaggtgtgtcgtatggtatcaactctaccc acttagatcctgtctattatggccctgatgctgctgagttcagaccagagagatggtttgagccatcaaccagaaagctcggctgggcttacttgcca ttcaacggtgggccacgaatctgtttgggtcagcagtttgccttgaccgaagctggttacgttttggtcagattggtgcaagagttctcccacattaggc tggacccagatgaagtgtatccaccaaagaggttgaccaacttgaccatgtgtttgcaggatggtgctattgtcaagtttgactag |
| SEQ ID NO: 73 | Cytochrome P450 monooxygenase CYP52A18 A.A. Seq. | mieqileywyivvpvlyiikqliayskrtvlmkqlgaapitnqlydnvfgivngwkalqfkkegraqeyndhkfdssknpsvgtyvsilfgtkivvtkdp enikailatqfgdfslgkrhalfkpllgdgiftldgegwkhsrsmlrpqfareqvahvtslephfqllkkhilkhkgeyfdiqelffrftvdsateflfgesvhsl kdetiginqddidfagrkdfaesfnkaqeylsirilvqtfywlinnkefrdctksvhkftnyyvqkaldatpeelekqggyvflyelvkqtrdpkvlrdqsln illagrdttagllsfavfelarnphiwaklreeieqqfglgedsrveeitfeslkrceylkaflnetlrvypsvprnfriatknttlprgggpdgtqpiliqkgegv syginsthldpvyygpdaaefrperwfepstrklgwaylpfnggpriclgqqfalteagyvlvrlvqefshirsdpdevyppkrltnltmclqdgaivkf d |
| SEQ ID NO: 74 | Cytochrome P450 monooxygenase CYP52A19 Nucleotide Seq. | atgctcgatcagatcttacattactggtacattgtcttgccattgttggccattatcaaccagatcgtggctcatgtcaggaccaattatttgatgaagaa attgggtgctaagccattcacacacgtccaacgtgacgggtggttgggcttcaaattcggccgtgaattcctcaaagcaaaaagtgctgggagact ggttgatttaatcatctcccgtttccacgataatgaggacactttctccagctatgcttttggcaaccatgtggtgttcaccagggaccccgagaatatc aaggcgcttttggcaacccagtttggtgatttttcattgggcagcagggtcaagttcttcaaaccattattggggtacggtatcttcacattggacgccg |
| | | aaggctggaagcacagcagagccatgttgagaccacagtttgccagagaacaagttgctcatgtgacgtcgttggaaccacacttccagttgttg aagaagcatatccttaaacacaagggtgagtactttgatatccaggaattgttctttagatttactgtcgactcggccacggagttcttatttggtgagtc cgtgcactccttaaaggacgaggaaattggctacgacacgaaagacatgtctgaagaaagacgcagatttgccgacgcgttcaacaagtcgca agtctacgtggccaccagagttgctttacagaacttgtactggttggtcaacaacaaagagttcaaggagtgcaatgacattgtccacaagtttacc aactactatgttcagaaagccttggatgctaccccagaggaacttgaaaagcaaggcgggtatgtgttcttgtatgagcttgtcaagcagacgaga gaccccaaggtgttgcgtgaccagtctttgaacatcttgttggcaggaagagacaccactgctgggttgttgtcctttgctgtgtttgagttggccagaa acccacacatctgggccaagttgagagaggaaattgaacagcagtttggtcttggagaagactctcgtgttgaagagattacctttgagagcttga agagatgtgagtacttgaaggccgtgttgaacgaaactttgagattacacccaagtgtcccaagaaacgcaagatttgcgattaaagacacgact ttaccaagaggcggtggccccaacggcaaggatcctatcttgatcaggaaggatgaggtggtgcagtactccatctcggcaactcagacaaatc ctgcttattatggcgccgatgctgctgatttagaccggaaagatggtttgaaccatcaactagaaacttgggatgggctttcttgccattcaacggtgg tccaagaatctgtttgggacaacagtttgctttgactgaagccggttacgttttggttagacttgttcaggagtttccaaacttgtcacaagaccccgaa accaagtacccaccacctagattggcacacttgacgatgtgcttgtttgacggtgcacacgtcaagatgtcatag |
| SEQ ID NO: 75 | Cytochrome P450 monooxygenase CYP52A19 A.A. Seq. | mldqilhywyivlpllaiinqivahvrtnylmkklgakpfthvqrdgwlgfkfgreflkaksagrsvdliisrfhdnedtfssyafgnhvvftrdpenikalla tqfgdfslgsrvkffkpllgygiftldaegwkhsramlrpqfareqvahvtslephfqllkkhilkhkgeyfdiqelffrftvdsateflfgesvhslkdeeigy dtkdmseerrrfadafnksqvyvatrvalqnlywlvnnkefkecndivhkftnyyvqkaldatpeelekqggyvflyelvkqtrdpkvlrdqslnillag rdttagllsfavfelarnphiwaklreeieqqfglgedsrveeitfeslkrceylkavlnetlrlhpsvprnarfaikdttlprgggpngkdpilirkdevvqys isatqtnpayygadaadfrperwfepstrnlgwaflpfnggpriclgqqfalteagyvlvrlvqefpnlsqdpetkypppprlahltmclfdgahvkms |
| SEQ ID NO: 76 | Cytochrome P450 monooxygenase CYP52A20 Nucleotide Seq. | atgctcgaccagatcttccattactggtacattgtcttgccattgttggtcattatcaagcagatcgtggctcatgccaggaccaattatttgatgaagaa gttgggcgctaagccattcacacatgtccaactagacgggtggtttggcttcaaatttggccgtgaattcctcaaagctaaaagtgctgggaggcag gttgatttaatcatctcccgtttccacgataatgaggacactttctccagctatgcttttggcaaccatgtggtgttcaccagggaccccgagaatatca aggcgcttttggcaacccagtttggtgatttttcattgggaagcagggtcaaattcttcaaaccattgttggggtacggtatcttcaccttggacggcga aggctggaagcacagcagagccatgttgagaccacagtttgccagagagcaagttgctcatgtgacgtcgttggaaccacatttccagttgttgaa gaagcatattcttaagcacaagggtgaatactttgatatccaggaattgttctttagatttaccgttgattcagcgacggagttcttatttggtgagtccgt gcactccttaagggacgaggaaattggctacgatacgaaggacatggctgaagaaagacgcaaatttgccgacgcgttcaacaagtcgcaagt ctatttgtccaccagagttgctttacagacattgtactggttggtcaacaacaaagagttcaaggagtgcaacgacattgtccacaagttcaccaact actatgttcagaaagccttggatgctaccccagaggaacttgaaaaacaaggcgggtatgtgttcttgtacgagcttgccaagcagacgaaagac cccaatgtgttgcgtgaccagtctttgaacatcttgttggctggaagggacaccactgctgggttgttgtcctttgctgtgtttgagttggccaggaaccc acacatctgggccaagttgagagaggaaattgaatcacactttgggctgggtgaggactctcgtgttgaagagattacctttgagagcttgaagag atgtgagtacttgaaagccgtgttgaacgaaacgttgagattacacccaagtgtcccaagaaacgcaagatttgcgattaaagacacgactttacc aagaggcggtggccccaacggcaaggatcctatcttgatcagaaagaatgaggtggtgcaatactccatctcggcaactcagacaaatcctgct tattatggcgccgatgctgctgatttagaccggaaagatggtttgagccatcaactagaaacttgggatgggcttacttgccattcaacggtggtcca agaatctgcttgggacaacagtttgctttgaccgaagccggttacgttttggttagacttgttcaggaattccctagcttgtcacaggaccccgaaact gagtacccaccacctagattggcacacttgacgatgtgcttgtttgacggggcatacgtcaagatgcaatag |
| SEQ ID NO: 77 | Cytochrome P450 monooxygenase CYP52A20 A.A. Seq. | mldqifhywyivlpllviikqivahartnylmkklgakpfthvqldgwfgkfgreflkaksagrqvdliisrfhdnedtfssyafgnhvvftrdpenikalla tqfgdfslgsrvkffkpllgygiftldgegwkhsramlrpqfareqvahvtslephfqllkkhilkhkgeyfdiqelfffrftvdsateflfgesvhslrdeeigy dtkdmaeerrkfadafnksqvylstrvalqtlywlvnnkefkecndivhkftnyyvqkaldatpeelekqggyvflyelakqtkdpnvlrdqslnillag rdttagllsfavfelarnphiwaklreeieshfgsgedsrveeitfeslkrceylkavlnetlrlhpsvprnarfaikdttlprgggpngkdpilirknevvqy sisatqtnpayygadaadfrperwfepstrnlgwaylpfnggpriclgqqfalteagyvlvrlvqefpslsqdpeteypppprlahltmclfdgayvkm q |
| SEQ ID NO: 78 | Cytochrome P450 reductase CPR A | atggctttagacaagttagattgtatgtcatcataacattggtggtcgctgtagccgcctattttgctaagaaccagttccttgatcagccccaggaca ccgggttcctcaacacggacagcggaagcaactccagagacgtcttgctgacattgaagaagaataataaaaacacgttgttgttgtttgggtccc |
| | Nucleotide Seq | agacgggtacggcagaagattacgccaacaaattgtccagagaattgcactccagatttggcttgaaaacgatggttgcagatttcgctgattacg attgggataacttcggagatatcaccgaagacatcttggtgtttttcattgttgccacctatggtgagggtgaacctaccgataatgccgacgagttcc acacctggttgactgaagaagctgacactttgagtaccttgaaatacaccgtgttcgggttgggtaactccacgtacgagttcttcaatgccattggta gaaagtttgacagattgttgagcgagaaaggtggtgacaggtttgctgaatacgctgaaggtgatgacggtactggcaccttggacgaagatttca tggcctggaaggacaatgtctttgacgccttgaagaatgatttgaactttgaagaaaaggaattgaagtacgaaccaaacgtgaaattgactgag agagacgacttgtctgctgctgactcccaagtttccttgggtgagccaaacaagaagtacatcaactccgagggcatcgacttgaccaagggtcc attcgaccacacccacccatacttggccagaatcaccgagacgagagagttgttcagctccaaggacagacactgtatccacgttgaatttgaca tttctgaatcgaacttgaaatacaccaccggtgaccatctagctatctggccatccaactccgacgaaaacattaagcaatttgccaagtgtttcgga ttggaagataaactcgacactgttattgaattgaaggcgttggactccacttacaccatcccattcccaaccccaattacctacggtgctgtcattaga caccatttagaaatctccggtccagtctcgagacaattcttttgtcaattgctgggtttgctcctgatgaagaaacaaagaaggcttttaccagacttg gtggtgacaagcaaaaattcgccgccaaggtcacccgcagaaagttcaacattgccgatgccttgttatattcctccaacaacgctccatggtccg atgttccttttgaattccttattgaaaacgttccacacttgactccacgttactactccatttcgtcttcgtcattgagtgaaaagcaactcatcaacgttact gcagttgttgaagccgaagaagaagctgatggcagaccagtcactggtgttgtcaccaacttgttgaagaacgttgaaattgtgcaaaacaagac tggcgaaaagccacttgtccactacgatttgagcggcccaagaggcaagttcaacaagttcaagttgccagtgcatgtgagaagatccaacttta agttgccaaagaactccaccaccccagttatcttgattggtccaggtactggtgttgccccattgagaggttttgtcagagaaagagttcaacaagtc aagaatggtgtcaatgttggcaagactttgttgttttatggttgcagaaactccaacgaggactttttgtacaagcaagaatgggccgagtacgcttct gttttgggtgaaaactttgagatgttcaatgccttctccagacaagacccatccaagaaggtttacgtccaggataagattttagaaaacagccaact tgtgcacgagttgttgactgaaggtgccattatctacgtctgtggtgatgccagtagaatggctagagacgtgcagaccacaatttccaagattgttg ctaaaagcagagaaattagtgaagacaaggctgctgaattggtcaagtcctggaaggtccaaaatagataccaagaagatgtttggtag |
| SEQ ID NO: 79 | Cytochrome P450 reductase CPR A A.A. Seq | maldkldlyviitlvvavaayfaknqfldqpqdtgflntdsgsnsrdvlstlkknnkntlllfgsqtgtaedyanklsrelhsrfglktmvadfadydwdnf gditedilvffivatygegeptdnadefhtwlteeadtlstlkytvfglgnstyeffnaigrkfdrllsekggdrfaeyaegddgtgtldedfmawkdnvfd alkndlnfeekelkyepnvklterddlsaadsqvslgepnkkyinsegidltkgpfdhthpylaritetrelfsskdrhcihvefdisesnlkyttgdhlai wpsnsdenikqfakcfgledkldtvielkaldstytipfptpitygavirhhleisgpvsrqfflsiagfapdeetkkaftrlggdkqkfaakvtrrkfniadal lyssnnapwsdvpfeflienvphltpryysisssslsekqlinvtavveaeeeadgrpvtgvvtnllknveivqnktgekplvhydlsgprgkfnkfklp vhvrrsnfklpknsttpviligpgtgvaplrgfvrervqqvkngvnvgktllfygcrnsnedflykqewaeyasvlgenfemfnafsrqdpskkvyvqd kilensqlvhelltegaiiyvcgdasrmardvqttiskivaksreisedkaaelvkswkvqnryqedvw |
| SEQ ID NO: 80 | Cytochrome P450 reductase CPR B Nucleotide Seq | atggctttagacaagttagattgtatgtcatcataacattggtggtcgctgtggccgcctattttgctaagaaccagttccttgatcagccccaggaca ccgggttcctcaacacggacagcggaagcaactccagagacgtcttgctgacattgaagaagaataataaaaacacgttgttgtttgggtccc agaccggtacggcagaagattacgccaacaaattgtcaagagaattgcactccagatttggcttgaaaaccatggttgcagatttcgctgattacg attgggataacttcggagatatcaccgaagatatcttggtgtttttcatcgttgccacctacggtgagggtgaacctaccgacaatgccgacgagttc cacacctggttgactgaagaagctgacactttgagtactttgagatataccgtgttcgggttgggtaactccacctacgagttcttcaatgctattggta gaaagtttgacagattgttgagtgagaaaggtggtgacagatttgctgaatatgctgaaggtgacgacggcactggcaccttggacgaagatttca tggcctggaaggataatgtctttgacgccttgaagaatgacttgaactttgaagaaaaggaattgaagtacgaaccaaacgtgaaattgactgag agagatgacttgtctgctgccgactcccaagtttccttgggtgagccaaacaagaagtacatcaactccgagggcatcgacttgaccaagggtcc attcgaccacacccacccatacttggccaggatcaccgagaccagagagttgttcagctccaaggaaagacactgtattcacgttgaatttgacat ttctgaatcgaacttgaaatacaccaccggtgaccatctagccatctggccatccaactccgacgaaaacatcaagcaatttgccaagtgtttcgg attggaagataaactcgacactgttattgaattgaaggcattggactccacttacaccattccattcccaactccaattacttacggtgctgtcattaga caccatttagaaatctccggtccagtctcgagacaattcttttgtcgattgctgggtttgctcctgatgaagaaacaaagaagactttcaccagacttg gtggtgacaaacaagaattcgccaccaaggttacccgcagaaagttcaacattgccgatgccttgttatattcctccaacaacactccatggtccg atgttccttttgagttccttattgaaaacatccaacacttgactccacgttactactccatttcttcttcgtcgttgagtgaaaaacaactcatcaatgttact gcagtcgttgaggccgaagaagaagccgatggcagaccagtcactggtgttgttaccaacttgttgaagaacattgaaattgcgcaaaacaaga |
| | | ctggcgaaaagccacttgttcactacgatttgagcggcccaagaggcaagttcaacaagttcaagttgccagtgcacgtgagaagatccaacttt aagttgccaaagaactccaccaccccagttatcttgattggtccaggtactggtgttgccccattgagaggtttcgttagagaaagagttcaacaagt caagaatggtgtcaatgttggcaagactttgttgttttatggttgcagaaactccaacgaggactttttgtacaagcaagaatgggccgagtacgcttc tgttttgggtgaaaactttgagatgttcaatgccttctctagacaagacccatccaagaaggtttacgtccaggataagattttagaaaacagccaac ttgtgcacgaattgttgaccgaaggtgccattatctacgtctgtggtgacgccagtagaatggccagagacgtccagaccacgatctccaagattgt tgccaaaagcagagaaatcagtgaagacaaggccgctgaattggtcaagtcctggaaagtccaaaatagataccaagaagatgtttggtag |
| SEQ ID NO: 81 | Cytochrome P450 reductase CPR B A.A. Seq | maldkldlyviitlvvavaayfaknqfldqpqdtgflntdsgnsrdvlstlkknnkntlllfgsqtgtaedyanklsrelhsrfglktmvadfadydwdnf gditedilvffivatygegeptdnadefhtwlteeadtlstlrytvfglgnstyeffnaigrkfdrllsekggdrfaeyaegddgtgtldedfmawkdnvfd alkndlnfeekelkyepnvklterddlsaadsqvslgepnkkyinsegidltkgpfdhthpylaritetrelfsskerhcihvefdisesnlkyttgdhlai wpsnsdenikqfakcfgledkldtvielkaldstytipfptpitygavirhhleisgpvsrqfflsiagfapdeetkktftrlggdkqefatkvtrrkfniadall yssnntpwsdvpfeflienqhltpryysisssslsekqlinvtavveaeeeadgrpvtgvvtnllknieiaqnktgekplvhydlsgprgkfnkfklpvh vrrsnfklpknsttpviligpgtgvaplrgfvrervqqvkngvnvgktllfygcrnsnedflykqewaeyasvlgenfemfnafsrqdpskkvyvqdkil ensqlvhelltegaiiyvcgdasrmardvqttiskivaksreisedkaaelvkswkvqnryqedvw |

### Example 53: Examples of Embodiments

Provided hereafter are non-limiting examples of certain embodiments.
A1. An engineered microorganism capable of producing adipic acid, which microorganism comprises one or more altered activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-L-hydroxyacyl-CoA dehydrogenase activity and acetyl-CoA C-acyltransferase activity.
A1.1. The engineered microorganism of embodiment A1, which comprises a genetic modification that adds or increases the 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-L-hydroxyacyl-CoA dehydrogenase activity and/or acetyl-CoA C-acyltransferase activity.
A1.2. The engineered microorganism of embodiment A1, which comprises a genetic modification that reduces the acyl-CoA oxidase activity.
A1.3. The engineered microorganism of embodiment A1.1, wherein the genetic modification comprises increased copies of a polynucleotide that encodes a polypeptide having 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-L-hydroxyacyl-CoA dehydrogenase activity or acetyl-CoA C-acyltransferase activity.
A1.4. The engineered microorganism of embodiment A1.1, wherein the genetic modification comprises insertion of a heterologous promoter and/or 5' UTR, into genomic DNA of the microorganism in functional connection with a polynucleotide that encodes a polypeptide having 6-oxohexanoic acid dehydrogenase activity, 6-hydroxyhexanoic acid dehydrogenase activity, glucose-6-phosphate dehydrogenase, hexanoate synthase activity, lipase activity, fatty acid synthase activity, acetyl CoA carboxylase activity, monooxygenase activity, monooxygenase reductase activity, fatty alcohol oxidase activity, acyl-CoA ligase activity, acyl-CoA oxidase activity, acyl-CoA hydrolase, acyl-CoA thioesterase enoyl-CoA hydratase activity, 3-L-hydroxyacyl-CoA dehydrogenase activity or acetyl-CoA C-acyltransferase activity.
A2. The engineered microorganism of any one of embodiments A1 to A1.4, which comprises an altered thioesterase activity.
A2.1. The engineered microorganism of embodiment A2, which comprises a genetic alteration that adds or increases a thioesterase activity.
A2.2. The engineered microorganism of embodiment A2.1, which comprises a heterologous polynucleotide encoding a polypeptide having thioesterase activity.
A3. The engineered microorganism of any one of embodiments A1 to A2.2, which comprises a heterologous polynucleotide encoding a polypeptide having 6-oxohexanoic acid dehydrogenase activity.
A3.1 The engineered microorganism of any one of embodiments A1 to A3, which comprises a heterologous polynucleotide encoding a polypeptide having omega oxo fatty acid dehydrogenase activity.
A4. The engineered microorganism of embodiment A3 and A3.1, wherein the heterologous polynucleotide is from a bacterium.
A5. The engineered microorganism of embodiment A4, wherein the bacterium is an Acinetobacter, Nocardia, Pseudomonas or Xanthobacter bacterium.
A6. The engineered microorganism of embodiment A1 or A2, which comprises a heterologous polynucleotide encoding a polypeptide having 6-hydroxyhexanoic acid dehydrogenase activity.
A6.1 The engineered microorganism of embodiment A1 or A2, which comprises a heterologous polynucleotide encoding a polypeptide having omega hydroxyl fatty acid dehydrogenase activity
A7. The engineered microorganism of embodiment A6 or A6.1, wherein the heterologous polynucleotide is from a bacterium.
A8. The engineered microorganism of embodiment A7, wherein the bacterium is an Acinetobacter, Nocardia, Pseudomonas or Xanthobacter bacterium.
A9. The engineered microorganism of embodiment A1 or A2, which comprises a heterologous polynucleotide encoding a polypeptide having hexanoate synthase subunit A activity.
A10. The engineered microorganism of embodiment A1 or A2, which comprises a heterologous polynucleotide encoding a polypeptide having hexanoate synthase subunit B activity.
A11. The engineered microorganism of embodiment A9 or A10, wherein the heterologous polynucleotide independently is selected from a bacterium.
A12. The engineered microorganism of embodiment A11, wherein the bacterium is a Bacillus bacterium.
A13. The engineered microorganism of embodiment A12, wherein the Bacillus bacterium is B. megaterium.
A14. The engineered microorganism of embodiment A1 or A2, which comprises a heterologous polynucleotide encoding a polypeptide having monooxygenase activity.
A15. The engineered microorganism of embodiment A14, wherein the heterologous polynucleotide is from a fungus.
A16. The engineered microorganism of embodiment A15, wherein the fungus is an Aspergillus fungus.
A17. The engineered microorganism of embodiment A16, wherein the Aspergillus fungus is A. parasiticus.
A18. The engineered microorganism of embodiment A1 or A2, which comprises a genetic modification that results in primary hexanoate usage by monooxygenase activity.
A19. The engineered microorganism of embodiment A18, wherein the genetic modification reduces a polyketide synthase activity.
A20. The engineered microorganism of embodiment A19, wherein the Candida yeast is a C. tropicalis strain.
A21. The engineered microorganism of any one of embodiments A1-A20, which comprises a genetic modification that reduces 6-hydroxyhexanoic acid conversion.
A22. The engineered microorganism of embodiment A21, wherein the genetic modification reduces 6-hydroxyhexanoic acid dehydrogenase activity.
A23. The engineered microorganism of any one of embodiments A1-A22, which comprises a genetic modification that reduces beta-oxidation activity.
A24. The engineered microorganism of embodiment A23, wherein the genetic modification renders beta-oxidation activity undetectable.
A25. The engineered microorganism of embodiment A24, wherein the genetic modification partially reduces beta-oxidation activity.
A26. The engineered microorganism of any one of embodiments A23 to A25, wherein the genetic modification comprises disrupting a polynucleotide that encodes a polypeptide having an acyl-CoA oxidase activity.
A27. The engineered microorganism of any one of embodiments A23 to A25, wherein the genetic modification comprises disrupting a promoter in functional connection with a polynucleotide that encodes a polypeptide having the acyl-CoA oxidase activity.
A28. The engineered microorganism of embodiment A26 or A27, wherein the polypeptide having the acyl-CoA oxidase activity is a POX polypeptide.
A29. The engineered microorganism of embodiment A28, wherein the POX polypeptide is a POX4 polypeptide, POX5 polypeptide or POX4 polypeptide and POX5 polypeptide.
A30. The engineered microorganism of any one of embodiments A1 to A29, which is in contact with a feedstock.
A31. The engineered microorganism of embodiment A30, wherein the feedstock comprises a saccharide.
A32. The engineered microorganism of embodiment A31, wherein the saccharide is a monosaccharide, polysaccharide or a mixture or a monosaccharide and polysaccharide.
A33. The engineered microorganism of embodiment A32, wherein the feedstock comprises a paraffin.
A34. The engineered microorganism of embodiment A33, wherein the paraffin is a saturated paraffin, unsaturated paraffin, substituted paraffin, branched paraffin, linear paraffin or combination thereof.
A35. The engineered microorganism of embodiment A33 or A34, wherein the paraffin includes about 1 to about 60 carbon atoms.
A36. The engineered microorganism of embodiments A33 to A35, wherein the paraffin is in a mixture of paraffins.
A37. The engineered microorganism of embodiment A36, wherein the paraffins in the mixture of paraffins have a mean number of carbon atoms of about 8 to about 18 carbon atoms.
A38. The engineered microorganism of embodiment A37, wherein the mean number of carbon atoms is about 10 to about 16 carbon atoms.
A39. The engineered microorganism of embodiment A38, wherein the mean number of carbon atoms is about 12 atoms.
A40. The engineered microorganism of any one of embodiments A33 to A39, wherein the paraffin is in a wax.
A41. The engineered microorganism of any one of embodiments A33 to A39, wherein the paraffin is in an oil.
A42. The engineered microorganism of any one of embodiments A33 to A41, wherein the paraffin is from a petroleum product.
A43. The engineered microorganism of embodiment A42, wherein the petroleum product is a petroleum distillate.
A44. The engineered microorganism of any one of embodiments A33 to A41, wherein the paraffin is from a plant or plant product.
B1. An engineered microorganism that produces adipic acid, which microorganism comprises an altered monooxygenase activity.
B1.1. The engineered microorganism of embodiment B1, which comprises a genetic modification that alters the monooxygenase activity.
B1.2. The engineered microorganism of embodiment B1 or B1.1, which comprises a genetic modification that alters a monooxygenase activity selected from the group consisting of.
B2. The engineered microorganism of embodiment B1.1, which comprises a heterologous polynucleotide encoding a polypeptide having monooxygenase activity.
B3. The engineered microorganism of embodiment B2, wherein the heterologous polynucleotide is from a fungus.
B4. The engineered microorganism of embodiment B3, wherein the fungus is an Aspergillus fungus.
B5. The engineered microorganism of embodiment B4, wherein the Aspergillus fungus is A. parasiticus.
B6. The engineered microorganism of any one of embodiments B1-B5, which comprises a genetic modification that results in substantial hexanoate usage by the monooxygenase activity.
B7. The engineered microorganism of embodiment B6, wherein the genetic modification reduces a polyketide synthase activity.
B8. The engineered microorganism of any one of embodiments B1-B5, which comprises an altered hexanoate synthase activity.
B9. The engineered microorganism of embodiment B8, wherein the altered hexanoate synthase activity is an altered hexanoate synthase subunit A activity, altered hexanoate synthase subunit B activity, or altered hexanoate synthase subunit A activity and altered hexanoate synthase subunit B activity.
B9.1. The engineered microorganism of embodiment B9, which comprises a genetic alteration that adds or increases hexanoate synthase activity.
B10. The engineered microorganism of any one of embodiments B8, B9 or B9.1, which comprises a heterologous polynucleotide encoding a polypeptide having hexanoate synthase activity.
B11. The engineered microorganism of embodiment B10, wherein the heterologous polynucleotide is from a fungus.
B12. The engineered microorganism of embodiment B11, wherein the fungus is an Aspergillus fungus.
B13. The engineered microorganism of embodiment B11, wherein the Aspergillus fungus is A. parasiticus.
B14. The engineered microorganism of any one of embodiments B1-B13, which comprises an altered thioesterase activity.
B14.1. The engineered microorganism of embodiment B14, which comprises a genetic modification that adds or increases the thioesterase activity.
B14.2. The engineered microorganism of embodiment B14 or B14.1, which comprises a heterologous polynucleotide encoding a polypeptide having thioesterase activity.
B15. The engineered microorganism of any one of embodiments B1-B14.2, which comprises an altered 6-oxohexanoic acid dehydrogenase activity.
B15.1. The engineered microorganism of embodiment B15, which comprises a genetic modification that adds or increases the 6-oxohexanoic acid dehydrogenase activity.
B15.2 The engineered microorganism of any one of embodiments B1 to B15.1, which comprises an altered omega oxo fatty acid dehydrogenase activity.
B15.3 The engineered microorganism of embodiment B15.2, which comprises a genetic modification that adds or increases the omega oxo fatty acid dehydrogenase activity
B16. The engineered microorganism of any one of embodiments B15 to B15.3, which comprises a heterologous polynucleotide encoding a polypeptide having 6-oxohexanoic acid dehydrogenase activity.
B16.1 The engineered microorganism of any one of embodiments B15 to B16, which comprises a heterologous polynucleotide encoding a polypeptide having omega oxo fatty acid dehydrogenase activity.
B17. The engineered microorganism of embodiment B16 or B16.1, wherein the heterologous polynucleotide is from a bacterium.
B18. The engineered microorganism of embodiment B17, wherein the bacterium is a Acinetobacter, Nocardia, Pseudomonas or Xanthobacter bacterium.
B19. The engineered microorganism of any one of embodiments B1-B18, which comprises an altered 6-hydroxyhexanoic acid dehydrogenase activity.
B19.1. The engineered microorganism of embodiment B19, which comprises a genetic modification that adds or increases the 6-hydroxyhexanoic acid dehydrogenase activity.
B19.2 The engineered microorganism of any one of embodiments B1-B19.1, which comprises an altered omega hydroxyl fatty acid dehydrogenase activity.
B19.3 The engineered microorganism of embodiment B19.2, which comprises a genetic modification that adds or increases the omega hydroxyl fatty acid dehydrogenase activity.
B20. The engineered microorganism of any one of embodiments B19 to B19.3, which comprises a heterologous polynucleotide encoding a polypeptide having 6-hydroxyhexanoic acid dehydrogenase activity.
B20.1 The engineered microorganism of any one of embodiments B19 to B20, which comprises a heterologous polynucleotide encoding a polypeptide having omega hydroxyl fatty acid dehydrogenase activity.
B21. The engineered microorganism of embodiment B20 or B20.1, wherein the heterologous polynucleotide is from a bacterium.
B22. The engineered microorganism of embodiment B21, wherein the bacterium is a Acinetobacter, Nocardia, Pseudomonas or Xanthobacter bacterium.
B23. The engineered microorganism of embodiment B22, wherein the Candida yeast is C. tropicalis.
B24. The engineered microorganism of any one of embodiments B1-B23, which comprises a genetic modification that reduces 6-hydroxyhexanoic acid conversion.
B25. The engineered microorganism of embodiment B24, wherein the genetic modification reduces 6-hydroxyhexanoic acid dehydrogenase activity.
B26. The engineered microorganism of any one of embodiments B1-B25, which comprises a genetic modification that reduces beta-oxidation activity.
B27. The engineered microorganism of embodiment B26, wherein the genetic modification renders beta-oxidation activity undetectable.
C1. A method for manufacturing adipic acid, which comprises culturing an engineered microorganism of any one of embodiments A1-B27 under culture conditions in which the cultured microorganism produces adipic acid.
C1.1. The method of embodiment C1, wherein the host microorganism from which the engineered microorganism is produced does not produce a detectable amount of adipic acid.
C2. The method of embodiment C1 of C1.1, wherein the culture conditions comprise fermentation conditions.
C3. The method of any one of embodiments C1-C2, wherein the culture conditions comprise introduction of biomass.
C4. The method of C1 or C2, wherein the culture conditions comprise introduction of glucose.
C5. The method of C1 or C2, wherein the culture conditions comprise introduction of hexane.
C6. The method of any one of embodiments C1-C5, wherein the adipic acid is produced with a yield of greater than about 0.3 grams per gram of glucose added.
C7. The method of any one of embodiments C1-C6, which comprises purifying the adipic acid from the cultured microorganisms.
C8. The method of embodiment C7, which comprises modifying the adipic acid, thereby producing modified adipic acid.
C9. The method of any one of embodiments C1-C8, which comprises placing the cultured microorganisms, the adipic acid or the modified adipic acid in a container.
C10. The method of embodiment C9, which comprises shipping the container.
D1. A method for manufacturing 6-hydroxyhexanoic acid, which comprises culturing an engineered microorganism of any one of embodiments A21, A22, B24 or B25 under culture conditions in which the cultured microorganism produces 6-hydroxyhexanoic acid.
D1.1. The method of embodiment D1, wherein the host microorganism from which the engineered microorganism is produced does not produce a detectable amount of 6-hydroxyhexanoic acid.
D2. The method of embodiment D1 or D1.1, wherein the culture conditions comprise fermentation conditions.
D3. The method of any one of embodiments D1-D2, wherein the culture conditions comprise introduction of biomass.
D4. The method of D1 or D2, wherein the culture conditions comprise introduction of glucose.
D5. The method of D1 or D2, wherein the culture conditions comprise introduction of hexane.
D6. The method of any one of embodiments D1-D5, wherein the 6-hydroxyhexanoic acid is produced with a yield of greater than about 0.3 grams per gram of glucose added.
D7. The method of any one of embodiments D1-D6, which comprises purifying the 6-hydroxyhexanoic acid from the cultured microorganisms.
D8. The method of embodiment D7, which comprises modifying the 6-hydroxyhexanoic acid, thereby producing modified 6-hydroxyhexanoic acid.
D9. The method of any one of embodiments D1-D8, which comprises placing the cultured microorganisms, the 6-hydroxyhexanoic acid or the modified 6-hydroxyhexanoic acid in a container.
D10. The method of embodiment D9, which comprises shipping the container.
E1. A method for preparing an engineered microorganism that produces adipic acid, which comprises:
   (a) introducing a genetic modification to a host organism that adds or increases monooxygenase activity, thereby producing engineered microorganisms having detectable and/or increased monooxygenase activity; and
   (b) selecting for engineered microorganisms that produce adipic acid.
E1.1. The method of embodiment E1, wherein the monooxygenase activity is incorporation of a hydroxyl moiety into a six-carbon molecule.
E1.2. The method of embodiment E1 or E1.1, wherein the six-carbon molecule is hexanoate.
E2. A method for preparing an engineered microorganism that produces adipic acid, which comprises:
   (a) culturing a host organism with hexane as a nutrient source, thereby producing engineered microorganisms having detectable monooxygenase activity; and
   (b) selecting for engineered microorganisms that produce adipic acid.
E2.1. The method of embodiment E2, wherein the monooxygenase activity is incorporation of a hydroxyl moiety into a six-carbon molecule.
E2.2. The method of embodiment E2 or E2.1, wherein the six-carbon molecule is hexanoate.
E3. The method of any one of embodiments E1-E2.2, which comprises selecting the engineered microorganisms that have a detectable amount of the monooxygenase activity.
E4. The method of any one of embodiments E1-E3, which comprises introducing a genetic modification that adds or increases a hexanoate synthase activity, thereby producing engineered microorganisms, and selecting for engineered microorganisms having detectable and/or increased hexanoate synthase activity.
E5. The method of embodiment E4, wherein the genetic modification encodes a polypeptide having a hexanoate synthase subunit A activity, a hexanoate synthase subunit B activity, or a hexanoate synthase subunit A activity and a hexanoate synthase subunit B activity.
E6. The method of any one of embodiments E1-E5, which comprises introducing a genetic modification that adds or increases 6-oxohexanoic acid dehydrogenase activity, thereby producing engineered microorganisms, and selecting for engineered microorganisms having detectable and/or increased 6-oxohexanoic acid dehydrogenase activity relative to the host microorgansim.
E7. The method of any one of embodiments E1-E6, which comprises introducing a genetic modification that adds or increases a 6-hydroxyhexanoic acid dehydrogenase activity, thereby producing engineered microorganisms, and selecting for engineered microorganisms having a detectable and/or increased 6-hydroxyhexanoic acid dehydrogenase activity relative to the host microorganism.
E7.1 The method of any one of embodiments E1-E7, which comprises introducing a genetic modification that adds or increases a 6-hydroxyhexanoic acid dehydrogenase activity, thereby producing engineered microorganisms, and selecting for engineered microorganisms having a detectable and/or increased omega hydroxyl fatty acid dehydrogenase activity relative to the host microorganism.
E8. The method of any one of embodiments E1-E7.1, which comprises introducing a genetic modification that adds or increases a thioesterase activity, thereby producing engineered microorganisms, and selecting for engineered microorganisms having a detectable and/or increased thioesterase activity relative to the host microorganism.
E9. The method of any one of embodiments E1-E8, which comprises introducing a genetic modification that reduces 6-hydroxyhexanoic acid conversion, thereby producing engineered microorganisms, and selecting for engineered microorganisms having reduced 6-hydroxyhexanoic acid conversion relative to the host microorganism.
E10. The method of any one of embodiments E1-E9, which comprises introducing a genetic modification that reduces beta-oxidation activity, thereby producing engineered microorganisms, and selecting for engineered microorganisms having reduced beta-oxidation activity relative to the host microorganism.
E11. The method of any one of embodiments E1-E11, which comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism.
F1. A method for preparing a microorganism that produces adipic acid, which comprises: (a) introducing one or more genetic modifications to a host organism that add or increase one or more activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, and monooxygenase reductase activity, thereby producing engineered microorganisms, and (b) selecting for engineered microorganisms that produce adipic acid.
F2. The method of embodiment F1, which comprises selecting for engineered microorganisms having one or more detectable and/or increased activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, and monooxygenase reductase activity, relative to the host microorganism.
F3. The method of embodiment F1 or F2, which comprises introducing a genetic modification that reduces 6-hydroxyhexanoic acid conversion, thereby producing engineered microorganisms, and selecting for engineered microorganisms having reduced 6-hydroxyhexanoic acid conversion relative to the host microorganism.
F4. The method of any one of embodiments F1-F3, which comprises introducing a genetic modification that reduces beta-oxidation activity, thereby producing engineered microorganisms, and selecting for engineered microorganisms having reduced beta-oxidation activity relative to the host microorganism.
F5. The method of any one of embodiments F1-F4, which comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism.
G1. A method for preparing a microorganism that produces 6-hydroxyhexanoic acid, which comprises: (a) introducing one or more genetic modifications to a host organism that add or increase one or more activities selected from the group consisting of 6-oxohexanoic acid dehydrogenase activity, glucose-6-phosphate dehydrogenase, hexanoate synthase activity, lipase activity, fatty acid synthase activity, acetyl CoA carboxylase activity, monooxygenase activity, and monooxygenase reductase activity, thereby producing engineered microorganisms, (b) introducing a genetic modification to the host organism that reduces 6-hydroxyhexanoic acid conversion, and (c) selecting for engineered microorganisms that produce 6-hydroxyhexanoic acid.
G2. The method of embodiment G1, which comprises selecting for engineered microorganisms having reduced 6-hydroxyhexanoic acid conversion relative to the host microorganism.
G3. The method of embodiment G1 or G2, which comprises selecting for engineered microorganisms having one or more detectable and/or increased activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, monooxygenase activity, and monooxygenase reductase activity, relative to the host microorganism.
G4. The method of any one of embodiments G1-G3, which comprises introducing a genetic modification that reduces beta-oxidation activity, thereby producing engineered microorganisms, and selecting for engineered microorganisms having reduced beta-oxidation activity relative to the host microorganism.
G5. The method of any one of embodiments G1-G4, which comprises introducing a genetic modification that results in substantial hexanoate usage by the monooxygenase activity, thereby producing engineered microorganisms, and selecting for engineered microorganisms in which substantial hexanoate usage is by the monooxygenase activity relative to the host microorganism.
H1. A method, comprising:
   contacting an engineered microorganism with an feedstock comprising one or more polysaccharides, wherein the engineered microorganism comprises:
      a. a genetic alteration that blocks beta oxidation activity, and
      b. a genetic alteration that adds or increases a monooxygenase activity or a genetic alteration that adds or increases hexanoate synthetase activity; and
   culturing the engineered microorganism under conditions in which adipic acid is produced.
H1.1. The method of embodiment H1, wherein the engineered microorganism comprises a genetic alteration that adds or increases hexanoate synthetase activity.
H1.2. The method of embodiment H1.1, wherein the engineered microorganism comprises a heterologous polynucleotide encoding a polypeptide having hexanoate synthase subunit A activity.
H1.3. The method of embodiment H1.1, wherein the engineered microorganism comprises a heterologous polynucleotide encoding a polypeptide having hexanoate synthase subunit B activity.
H1.4. The method of embodiment H1.2 or H1.3, wherein the heterologous polynucleotide independently is selected from a bacterium.
H1.5. The method of embodiment H1.4, wherein the bacterium is a Bacillus bacterium.
H1.6. The method of embodiment H1.5, wherein the Bacillus bacterium is B. megaterium.
H2. The method of any one of embodiment H1 or H1.6, wherein the microorganism is a Candida yeast.
H3. The method of embodiment H2, wherein the microorganism is a C. tropicalis strain.
H4. The method of any one of embodiments H1 to H3, wherein the genetic alteration that increases monooxygenase activity comprises a genetic alteration that increases cytochrome P450 reductase activity.
H5. The method of embodiment H4, wherein the genetic alteration increases the number of copies of a polynucleotide that encodes a polypeptide having the cytochrome P450 reductase activity.
H6. The method of embodiment H4, wherein the genetic alteration places a promoter in functional connection with a polynucleotide that encodes a polypeptide having the cytochrome P450 reductase activity.
H7. The method of any one of embodiments H1 to H6, wherein the monooxygenase activity is a CYP52A15 activity, CYP52A16 activity, or a CYP52A15 activity and CYP52A16 activity.
H8. The method of any one of embodiments H1 to H7, wherein the genetic alteration increases the number of copies of a polynucleotide that encodes a polypeptide having the monooxygenase activity.
H9. The method of any one of embodiments H1 to H7, wherein the genetic alteration places a promoter in functional connection with a polynucleotide that encodes a polypeptide having the monooxygenase activity.
H10. The method of any one of embodiments H1 to H7, wherein the genetic alteration that blocks beta oxidation activity disrupts acyl-CoA oxidase activity.
H11. The method of embodiment H10, wherein the genetic alteration disrupts POX4 and POX5 activity.
H12. The method of embodiment H10 or H 11, wherein the genetic alteration disrupts a polynucleotide that encodes a polypeptide having the acyl-CoA oxidase activity.
H13. The method of embodiment H10 or H 11, wherein the genetic alteration disrupts a promoter in functional connection with a polynucleotide that encodes a polypeptide having the acyl-CoA oxidase activity.
H14. The method of any one of embodiments H1 to H13, wherein the feedstock comprises a 6-carbon sugar.
H15. The method of any one of embodiments H1 to H13, wherein the feedstock comprises a 5-carbon sugar.
H16. The method of any one of embodiments H1 to H15, wherein the adipic acid is produced at a level of about 80% or more of theoretical yield.
H17. The method of any one of embodiments H1 to H16, comprising detecting the amount of adipic acid produced.
H18. The method of any one of embodiments H1 to H17, comprising isolating the adipic acid produced.
H19. The method of any one of embodiments H1 to H18, wherein the culture conditions comprise fermenting the engineered microorganism.
I1. A method, comprising:
   contacting an engineered microorganism with a feedstock comprising one or more paraffins, wherein the engineered microorganism comprises a genetic alteration that partially blocks beta oxidation activity; and
   culturing the engineered microorganism under conditions in which adipic acid is produced.
I1.1. The method of embodiment I1, wherein the microorganism comprises a genetic alteration that increases a monooxygenase activity.
I2. The method of embodiment I1 or I1.1, wherein the microorganism is a Candida yeast.
I3. The method of embodiment I2, wherein the microorganism is a C. tropicalis strain.
I4. The method of any one of embodiments I1 to I3, wherein the genetic alteration that increases monooxygenase activity comprises a genetic alteration that increases cytochrome P450 reductase activity.
I5. The method of embodiment I4, wherein the genetic alteration increases the number of copies of a polynucleotide that encodes a polypeptide having the cytochrome P450 reductase activity.
I6. The method of embodiment I4, wherein the genetic alteration places a promoter in functional connection with a polynucleotide that encodes a polypeptide having the cytochrome P450 reductase activity.
I7. The method of any one of embodiments I1 to I6, wherein the monooxygenase activity is a CYP52A15 activity, CYP52A16 activity, or a CYP52A15 activity and CYP52A16 activity.
I8. The method of any one of embodiments I1 to I7, wherein the genetic alteration increases the number of copies of a polynucleotide that encodes a polypeptide having the monooxygenase activity.
I9. The method of any one of embodiments I1 to I7, wherein the genetic alteration places a promoter in functional connection with a polynucleotide that encodes a polypeptide having the monooxygenase activity.
I10. The method of any one of embodiments I1 to I7, wherein the genetic alteration that blocks beta oxidation activity disrupts acyl-CoA oxidase activity.
I11. The method of embodiment I10, wherein the genetic alteration disrupts POX4 or POX5 activity.
I12. The method of embodiment I10 or I11, wherein the genetic alteration disrupts a polynucleotide that encodes a polypeptide having the acyl-CoA oxidase activity.
I13. The method of embodiment I10 or I11, wherein the genetic alteration disrupts a promoter in functional connection with a polynucleotide that encodes a polypeptide having the acyl-CoA oxidase activity.
I14. The method of any one of embodiments I1 to I13, wherein the adipic acid is produced at a level of about 80% or more of theoretical yield.
I15. The method of any one of embodiments I1 to I14, comprising detecting the amount of adipic acid produced.
I16. The method of any one of embodiments I1 to I15, comprising isolating the adipic acid produced.
I17. The method of any one of embodiments I1 to I16, wherein the culture conditions comprise fermenting the engineered microorganism.
I18. The method of any one of embodiments I1 to I17, wherein the paraffin is a saturated paraffin, unsaturated paraffin, substituted paraffin, branched paraffin, linear paraffin or combination thereof.
I19. The method of any one of embodiments I1 to I18, wherein the paraffin includes about 1 to about 60 carbon atoms.
I20. The method of any one of embodiments I1 to I19, wherein the paraffin is in a mixture of paraffins.
I21. The method of embodiment I20, wherein the paraffins in the mixture of paraffins have a mean number of carbon atoms of about 8 to about 18 carbon atoms.
I22. The method of embodiment I21, wherein the mean number of carbon atoms is about 10 to about 16 carbon atoms.
I23. The method of embodiment I22, wherein the mean number of carbon atoms is about 12 atoms.
I24. The method of any one of embodiments I1 to I23, wherein the paraffin is in a wax.
I25. The method of any one of embodiments I1 to I23, wherein the paraffin is in an oil.
I26. The method of any one of embodiments I1 to I25, wherein the paraffin is from a petroleum product.
I27. The method of embodiment I26, wherein the petroleum product is a petroleum distillate.
I28. The method of any one of embodiments I1 to I27, wherein the paraffin is from a plant or plant product.
J1. An isolated polynucleotide selected from the group consisting of:
   a polynucleotide having a nucleotide sequence 96% or more identical to the nucleotide sequence of SEQ ID NO: 1;
   a polynucleotide having a nucleotide sequence that encodes a polypeptide of SEQ ID NO: 8; and
   a polynucleotide having a portion of a nucleotide sequence 96% or more identical to the nucleotide sequence of SEQ ID NO: 1 and encodes a polypeptide having fatty alcohol oxidase activity.
J2. An isolated polynucleotide selected from the group consisting of:
   a polynucleotide having a nucleotide sequence 98% or more identical to the nucleotide sequence of SEQ ID NO: 2;
   a polynucleotide having a nucleotide sequence that encodes a polypeptide of SEQ ID NO: 10; and
   a polynucleotide having a portion of a nucleotide sequence 98% or more identical to the nucleotide sequence of SEQ ID NO: 2 and encodes a polypeptide having fatty alcohol oxidase activity.
J3. An isolated polynucleotide selected from the group consisting of:
   a polynucleotide having a nucleotide sequence 95% or more identical to the nucleotide sequence of SEQ ID NO: 3;
   a polynucleotide having a nucleotide sequence that encodes a polypeptide of SEQ ID NO: 9; and
   a polynucleotide having a portion of a nucleotide sequence 95% or more identical to the nucleotide sequence of SEQ ID NO: 3 and encodes a polypeptide having fatty alcohol oxidase activity.
J3.1. An isolated polynucleotide selected from the group consisting of:
   a polynucleotide having a nucleotide sequence 83% or more identical to the nucleotide sequence of SEQ ID NO: 4;
   a polynucleotide having a nucleotide sequence that encodes a polypeptide of SEQ ID NO: 11; and
   a polynucleotide having a portion of a nucleotide sequence 83% or more identical to the nucleotide sequence of SEQ ID NO: 4 and encodes a polypeptide having fatty alcohol oxidase activity.
J3.2. An isolated polynucleotide selected from the group consisting of:
   a polynucleotide having a nucleotide sequence 82% or more identical to the nucleotide sequence of SEQ ID NO: 5;
   a polynucleotide having a nucleotide sequence that encodes a polypeptide of SEQ ID NO: 12; and
   a polynucleotide having a portion of a nucleotide sequence 82% or more identical to the nucleotide sequence of SEQ ID NO: 5 and encodes a polypeptide having fatty alcohol oxidase activity.
J4. An expression vector comprising a polynucleotide of any one of embodiments J1 to J3.2.
J5. An integration vector comprising a polynucleotide of any one of embodiments J1 to J3.2.
J6. A microorganism comprising an expression vector of embodiment J4 or an integration vector of embodiment J5.
J7. A culture comprising a microorganism of embodiment J6.
J8. A fermentation device comprising a microorganism of embodiment J7.
J9. A polypeptide encoded by a polynucleotide of any one of embodiments J1 to J3 or produced by an expression vector of embodiment J4 or microorganism of embodiment J6.
J10. An antibody that specifically binds to a polypeptide of embodiment J9.
K1. An engineered microorganism capable of producing adipic acid, which microorganism comprises genetic alterations resulting in commitment of molecular pathways in directions for production of adipic acid, which pathways and directions include: (i) fatty acid synthesis pathway in the direction of acetyl CoA to long-chain fatty acids, (ii) omega oxidation pathway in the direction of long-chain fatty acids to diacids and (iii) beta oxidation pathway in the direction of diacids to adipic acid.
K2. The engineered microorganism of embodiment K1, which comprises an increased activity, relative to the microorganism not containing the genetic alterations, independently in each pathway.
K3. The engineered microorganism of embodiment K2, comprising an increased acetyl CoA carboxylase activity in the fatty acid synthesis pathway.
K4. The engineered microorganism of embodiment K2 or K3, comprising an increased fatty acid synthase activity in the fatty acid synthesis pathway.
K5. The engineered microorganism of any one of embodiments K2 to K4, comprising an increased monooxygenase activity.
K6. The engineered microorganism of any one of embodiments K2 to K5, comprising an increased monooxygenase reductase activity.
K7. The engineered microorganism of any one of embodiments K2 to K6, comprising an increased acyl-CoA oxidase activity.
K8. An engineered microorganism capable of producing adipic acid, which microorganism comprises genetic alterations resulting in increased activities, relative to the microorganism not containing the genetic alterations, selected from the group consisting of acetyl CoA carboxylase activity, fatty acid synthase activity, monooxygenase activity, monooxygenase reductase activity and acyl-CoA oxidase activity.
K9. The engineered microorganism of any one of embodiments K2 to K8, wherein each of the increased activities independently is provided by an enzyme encoded by a gene endogenous to the microorganism.
K10. The engineered microorganism of embodiment K9, wherein each of the increased activities independently is provided by an enzyme encoded by a gene exogenous to the microorganism.
K11. The engineered microorganism of any one of embodiments K2 to K10, wherein each of the increased activities independently is provided by an increased amount of an enzyme from a yeast.
K12. The engineered microorganism of embodiment K11, wherein the yeast is a Candida yeast.
K13. The engineered microorganism of embodiment K12, wherein the yeast is a Candida tropicalis yeast.
K14. The engineered microorganism of any one of embodiments K2 to K13, wherein each one of the increased activities independently results from increasing the copy number of a gene that encodes an enzyme that provides the activity.
K15. The engineered microorganism of any one of embodiments K1 to K14, wherein each one of the increased activities independently results from inserting a promoter in functional proximity to a gene that encodes an enzyme that provides the activity.
K16. The engineered microorganism of embodiment K14 or K15, wherein the gene is in plasmid nucleic acid.
K17. The engineered microorganism of embodiment K14 or K15, wherein the gene is in genomic nucleic acid of the microorganism.
K18. The engineered microorganism of any one of embodiments K1 to K17, wherein the acetyl CoA carboxylase activity is provided by an increased amount of an enzyme comprising (i) the amino acid sequence encoded by SEQ ID NO: 30, (ii) an amino acid sequence 90% or more identical to (i), or (iii) an amino acid sequence that includes 1 to 10 amino acid substitutions, insertions or deletions with respect to (i).
K19. The engineered microorganism of any one of embodiments K1 to K18, wherein the fatty acid synthase activity is provided by an increased amount of a FAS1-encoded enzyme, a FAS2-encoded enzyme, or FAS1-encoded enzyme and FAS2-encoded enzyme.
K20. The engineered microorganism of embodiment K19, wherein the FAS1-encoded enzyme comprises (i) the amino acid sequence encoded by SEQ ID NO: 32, (ii) an amino acid sequence 90% or more identical to (i), or (iii) an amino acid sequence that includes 1 to 10 amino acid substitutions, insertions or deletions with respect to (i).
K21. The engineered microorganism of embodiment K19, wherein the FAS2-encoded enzyme comprises (i) the amino acid sequence encoded by SEQ ID NO: 31, (ii) an amino acid sequence 90% or more identical to (i), or (iii) an amino acid sequence that includes 1 to 10 amino acid substitutions, insertions or deletions with respect to (i).
K22. The engineered microorganism of any one of embodiments K1 to K21, wherein the monooxygenase activity is provided by an increased amount of a cytochrome P450 enzyme.
K23. The engineered microorganism of embodiment K22, wherein the monooxygenase activity is provided by an exogenous cytochrome P450 enzyme.
K24. The engineered microorganism of embodiment K23, wherein the exogenous cytochrome P450 enzyme is from Bacillus megaterium.
K25. The engineered microorganism of embodiment K24, wherein the exogenous cytochrome P450 enzyme comprises (i) the amino acid sequence of SEQ ID NO: 41, (ii) an amino acid sequence 90% or more identical to (i), or (iii) an amino acid sequence that includes 1 to 10 amino acid substitutions, insertions or deletions with respect to (i).
K26. The engineered microorganism of embodiment K22, wherein two or more endogenous cytochrome P450 enzymes are expressed in increased amounts.
K27. The engineered microorganism of embodiment K22, wherein all endogenous cytochrome P450 enzymes are expressed in increased amounts.
K28. The engineered microorganism of embodiment K26 or K27, wherein the endogenous cytochrome P450 enzymes comprise (i) an amino acid sequence encoded by a polynucleotide selected from the group consisting of SEQ ID NO: 13, 14, 15, 16, 17, 18, 19, 20, 21, and 22, (ii) an amino acid sequence 90% or more identical to (i), or (iii) an amino acid sequence that includes 1 to 10 amino acid substitutions, insertions or deletions with respect to (i).
K29. The engineered microorganism of any one of embodiments K1 to K21, wherein the monooxygenase reductase activity is provided by an increased amount of an enzyme comprising (i) the amino acid sequence encoded by any one of SEQ ID NOS: 23 to 26, (ii) an amino acid sequence 90% or more identical to (i), or (iii) an amino acid sequence that includes 1 to 10 amino acid substitutions, insertions or deletions with respect to (i).
K30. The engineered microorganism of any one of embodiments K1 to K29, wherein the acyl-CoA oxidase activity is provided by an increased amount of a POX5-encoded enzyme.
K31. The engineered microorganism of embodiment K30, wherein the POX5-encoded enzyme comprises (i) the amino acid sequence of SEQ ID NO: 40, (ii) an amino acid sequence 90% or more identical to (i), or (iii) an amino acid sequence that includes 1 to 10 amino acid substitutions, insertions or deletions with respect to (i).
K32. The engineered microorganism of any one of embodiments K1 to K31, wherein the microorganism lacks an enzyme providing an acyl-CoA oxidase activity.
K33. The engineered microorganism of embodiment K32, wherein the enzyme is a POX4-encoded enzyme or a POX5-encoded enzyme.
K34. The engineered microorganism of embodiment K33, wherein the yeast is a Candida tropicalis yeast.
K35. The engineered microorganism of any one of embodiments K1 to K34, wherein the microorganism is haploid.
K36. The engineered microorganism of any one of embodiments K1 to K34, wherein the microorganism is diploid.
L1. A method for producing adipic acid, comprising culturing an engineered microorganism of any one of embodiments K1 to K36 under conditions in which adipic acid is produced.
L2. The method of embodiment L1, wherein the culture conditions comprise fermentation conditions.
L3. The method of embodiment L1 or L2, wherein the culture conditions comprise introduction of biomass.
L4. The method of any one of embodiments L1 to L3, wherein the culture conditions comprise introduction of a feedstock comprising glucose.
L5. The method of any one of embodiments L1 to L4, wherein the culture conditions comprise introduction of a feedstock comprising hexane.
L6. The method of any one of embodiments L1 to L5, wherein the culture conditions comprise introduction of a feedstock comprising an oil.
L7. The method of any one of embodiments L4 to L6, wherein the adipic acid is produced with a yield of greater than about 0.15 grams per gram of the glucose, hexane or oil.
L7.1. The method of any one of embodiments L4 to L7, wherein the adipic acid is produced at between about 25% and about 100% of maximum theoretical yield of any introduced feedstock.
L7.2. The method of any one of embodiments L4 to L7.1, wherein the adipic acid is produced in a concentration range of between about 50 g/L to about 1000g/L of culture media.
L7.3. The method of any one of embodiments, L4 to L7.2, wherein the adipic acid is produced at a rate of between about 0.5 g/L/hour to about 5 g/L/hour.
L7.4. The method of any one of embodiments, L1 to L7.3, wherein the engineered organism comprises between about a 5-fold to about a 300-fold increase in adipic acid production when compared to wild-type or partially engineered organisms of the same strain, under identical fermentation conditions.
L8. The method of any one of embodiments L1 to L7.4, which comprises purifying the adipic acid from the cultured microorganisms.
L9. The method of embodiment L8, which comprises modifying the adipic acid, thereby producing modified adipic acid.
L10. The method of any one of embodiments L1 to L9, which comprises placing the cultured microorganisms, the adipic acid or the modified adipic acid in a container.
L11. The method of embodiment L10, which comprises shipping the container.
M1. An engineered microorganism capable of producing adipic acid, which microorganism comprises genetic alterations resulting in commitment of molecular pathways in directions for production of adipic acid, which pathways and directions include: (i) hexanoic acid synthesis pathway in the direction of acetyl CoA to hexanoic acid, and (ii) omega oxidation pathway in the direction of hexanoic acid to adipic acid.
M2. The engineered microorganism of embodiment M1, which comprises an increased activity, relative to the microorganism not containing the genetic alterations, independently in each pathway.
M3. The engineered microorganism of embodiment M1 and M2, comprising an increased acetyl CoA carboxylase activity in the hexanoic acid synthesis pathway.
M4. The engineered microorganism of any one of embodiments M1 to M3, comprising increased hexanoate synthase activity in the hexanoic acid synthesis pathway.
M5. The engineered microorganism of any one of embodiments M1 to M4, comprising increased monooxygenase activity in the omega oxidation pathway.
M6. The engineered microorganism of any one of embodiments M1 to M5, comprising increased monooxygenase reductase activity in the omega oxidation pathway.
M7. An engineered microorganism capable of producing adipic acid, which microorganism comprises genetic alterations resulting in increased activities, relative to the microorganism not containing the genetic alterations, selected from the group consisting of acetyl CoA carboxylase activity, hexanoate synthase activity, monooxygenase activity and monooxygenase reductase activity.
M8. The engineered microorganism of any one of embodiments M2 to M7, wherein each of the increased activities independently is provided by an enzyme encoded by a gene endogenous to the microorganism.
M9. The engineered microorganism of embodiment M8, wherein each of the increased activities independently is provided by an enzyme encoded by a gene exogenous to the microorganism.
M10. The engineered microorganism of any one of embodiments M2 to M9, wherein each of the increased activities independently is provided by an increased amount of an enzyme from a yeast.
M11. The engineered microorganism of embodiment M10, wherein the yeast is a Candida yeast.
M12. The engineered microorganism of embodiment M11, wherein the yeast is a Candida tropicalis yeast.
M13. The engineered microorganism of any one of embodiments M2 to M12, wherein each one of the increased activities independently results from increasing the copy number of a gene that encodes an enzyme that provides the activity.
M14. The engineered microorganism of any one of embodiments M1 to M13, wherein each one of the increased activities independently results from inserting a promoter in functional proximity to a gene that encodes an enzyme that provides the activity.
M15. The engineered microorganism of embodiment M13 or M14, wherein the gene is in plasmid nucleic acid.
M16. The engineered microorganism of embodiment M13 or M14, wherein the gene is in genomic nucleic acid of the microorganism.
M17. The engineered microorganism of any one of embodiments M1 to M16, wherein the acetyl CoA carboxylase activity is provided by an increased amount of an enzyme comprising (i) the amino acid sequence encoded by SEQ ID NO: 30, (ii) an amino acid sequence 90% or more identical to (i), or (iii) an amino acid sequence that includes 1 to 10 amino acid substitutions, insertions or deletions with respect to (i).
M18. The engineered microorganism of any one of embodiments M1 to M17, wherein the hexanoate synthase activity is provided by an increased amount of a HEXA-encoded enzyme, a HEXB-encoded enzyme, or HEXA-encoded enzyme and HEXB-encoded enzyme.
M19. The engineered microorganism of embodiment M18, wherein the HEXA-encoded enzyme comprises (i) the amino acid sequence encoded by SEQ ID NO: 35, (ii) an amino acid sequence 90% or more identical to (i), or (iii) an amino acid sequence that includes 1 to 10 amino acid substitutions, insertions or deletions with respect to (i).
M20. The engineered microorganism of embodiment M18, wherein the HEXB-encoded enzyme comprises (i) the amino acid sequence encoded by SEQ ID NO: 36, (ii) an amino acid sequence 90% or more identical to (i), or (iii) an amino acid sequence that includes 1 to 10 amino acid substitutions, insertions or deletions with respect to (i).
M21. The engineered microorganism of any one of embodiments M1 to M20, wherein the monooxygenase activity is provided by an increased amount of a cytochrome P450 enzyme. M22. The engineered microorganism of embodiment M21, wherein the monooxygenase activity is provided by an exogenous cytochrome P450 enzyme.
M23. The engineered microorganism of embodiment M22, wherein the exogenous cytochrome P450 enzyme is from Bacillus megaterium.
M24. The engineered microorganism of embodiment M23, wherein the exogenous cytochrome P450 enzyme comprises (i) the amino acid sequence of SEQ ID NO: 41, (ii) an amino acid sequence 90% or more identical to (i), or (iii) an amino acid sequence that includes 1 to 10 amino acid substitutions, insertions or deletions with respect to (i).
M25. The engineered microorganism of embodiment M21, wherein two or more endogenous cytochrome P450 enzymes are expressed in increased amounts.
M26. The engineered microorganism of embodiment M21, wherein all endogenous cytochrome P450 enzymes are expressed in increased amounts.
M27. The engineered microorganism of embodiment M25 or M26, wherein the endogenous cytochrome P450 enzymes comprise (i) an amino acid sequence selected from the group consisting of amino acid sequences encoded by any one of SEQ ID NO: 13, 14, 15, 16, 17, 18, 19, 20, 21, and 22, (ii) an amino acid sequence 90% or more identical to (i), or (iii) an amino acid sequence that includes 1 to 10 amino acid substitutions, insertions or deletions with respect to (i).
M28. The engineered microorganism of any one of embodiments M1 to M27, wherein the monooxygenase reductase activity is provided by an increased amount of an enzyme comprising (i) an amino acid sequence encoded by any one of SEQ ID NO: 23 to 26, (ii) an amino acid sequence 90% or more identical to (i), or (iii) an amino acid sequence that includes 1 to 10 amino acid substitutions, insertions or deletions with respect to (i).
M29. The engineered microorganism of any one of embodiments M1 to M28, wherein the monooxygenase reductase activity is provided by an increased amount of a cytochrome P450:NADPH P450 reductase-encoded enzyme, a CPR-encoded enzyme, a CPRA-encoded enzyme, a CPRB-encoded enzyme, or a cytochrome P450:NADPH P450 reductase-encoded enzyme, a CPR-encoded enzyme, a CPRA-encoded enzyme, and/or a CPRB-encoded enzyme.
M30. The engineered microorganism of embodiment M29, wherein the cytochrome P450:NADPH P450 reductase-encoded enzyme comprises (i) the amino acid sequence of SEQ ID NO: 41 (ii) an amino acid sequence 90% or more identical to (i), or (iii) an amino acid sequence that includes 1 to 10 amino acid substitutions, insertions or deletions with respect to (i).
M31. The engineered microorganism of embodiment K30, wherein the CPR-, CPRA and/or CPRB encoded enzymes comprise (i)an amino acid sequence encoded by any one of SEQ ID NOS: 24 to 26, (ii) an amino acid sequence 90% or more identical to (i), or (iii) an amino acid sequence that includes 1 to 10 amino acid substitutions, insertions or deletions with respect to (i).
M32. The engineered microorganism of embodiment M31, wherein the yeast is a Candida tropicalis yeast.
M33. The engineered microorganism of any one of embodiments M1 to M32, wherein the microorganism is haploid.
M34. The engineered microorganism of any one of embodiments M1 to M32, wherein the microorganism is diploid.
N1. A method for producing adipic acid, comprising culturing an engineered microorganism of any one of embodiments M1 to M34 under conditions in which adipic acid is produced.
N2. The method of embodiment N1, wherein the culture conditions comprise fermentation conditions.
N3. The method of embodiment N1 or N2, wherein the culture conditions comprise introduction of biomass.
N4. The method of any one of embodiments N1 to N3, wherein the culture conditions comprise introduction of a feedstock comprising glucose.
N5. The method of any one of embodiments N1 to N4, wherein the culture conditions comprise introduction of a feedstock comprising hexane.
N6. The method of any one of embodiments N1 to N5, wherein the culture conditions comprise introduction of a feedstock comprising an oil.
N7. The method of any one of embodiments N4 to N6, wherein the adipic acid is produced with a yield of greater than about 0.15 grams per gram of the glucose, hexane or oil.
N7.1. The method of any one of embodiments N4 to N7, wherein the adipic acid is produced at between about 25% and about 100% of maximum theoretical yield of any introduced feedstock.
N7.2. The method of any one of embodiments N4 to N7.1, wherein the adipic acid is produced in a concentration range of between about 50 g/L to about 1000g/L of culture media.
N7.3. The method of any one of embodiments, N4 to N7.2, wherein the adipic acid is produced at a rate of between about 0.5 g/L/hour to about 5 g/L/hour.
N7.4. The method of any one of embodiments, N1 to N7.3, wherein the engineered organism comprises between about a 5-fold to about a 300-fold increase in adipic acid production when compared to wild-type or partially engineered organisms of the same strain, under identical fermentation conditions.
N8. The method of any one of embodiments N1 to N7.4, which comprises purifying the adipic acid from the cultured microorganisms.
N9. The method of embodiment N8, which comprises modifying the adipic acid, thereby producing modified adipic acid.
N10. The method of any one of embodiments N1 to N9, which comprises placing the cultured microorganisms, the adipic acid or the modified adipic acid in a container.
N11. The method of embodiment N10, which comprises shipping the container.
O1. An engineered microorganism capable of producing adipic acid, which microorganism comprises genetic alterations resulting in commitment of molecular pathways in directions for production of adipic acid, which pathways and directions include: (i) gluconeogenesis pathway in the direction of triacyl glycerides to 6-phosphoglucono-lactone and nicotinamide adenine dinucleotide phosphate (NADPH), (ii) omega oxidation pathway in the direction of fatty acids to diacids, (iii) beta oxidation pathway in the direction of diacids to adipic acid and (iv) fatty acid synthesis pathway in the direction of acetyl CoA to fatty acids.
02. The engineered microorganism of embodiment O1, which comprises an increased activity, relative to the microorganism not containing the genetic alterations, independently in each pathway.
03. The engineered microorganism of embodiment 02, comprising an increased lipase activity in the gluconeogenesis pathway.
04. The engineered microorganism of O1 or 02, comprising an increased glucose-6-phosphate dehydrogenase activity in the gluconeogenesis pathway.
05. The engineered microorganism of any one of embodiments 02 to 04, comprising an increased fatty acid synthase activity in the fatty acid synthesis pathway.
06. The engineered microorganism of any one of embodiments 02 to 05, comprising an increased monooxygenase activity in the omega oxidation pathway.
07. The engineered microorganism of any one of embodiments 02 to 06, comprising an increased monooxygenase reductase activity in the omega oxidation pathway.
08. The engineered microorganism of any one of embodiments 02 to 07, comprising an increased acyl-CoA oxidase activity in the beta oxidation pathway.
09. An engineered microorganism capable of producing adipic acid, which microorganism comprises genetic alterations resulting in increased activities, relative to the microorganism not containing the genetic alterations, selected from the group consisting of lipase activity, glucose-6-phosphate dehydrogenase activity, fatty acid synthase activity, monooxygenase activity, monooxygenase reductase activity, acyl-CoA hydrolase, acyl-CoA thioesterase and acyl-CoA oxidase activity.
O10. The engineered microorganism of any one of embodiments 02 to 09, wherein each of the increased activities independently is provided by an enzyme encoded by a gene endogenous to the microorganism.
O11. The engineered microorganism of embodiment O10, wherein each of the increased activities independently is provided by an enzyme encoded by a gene exogenous to the microorganism.
012. The engineered microorganism of any one of embodiments 02 to O10, wherein each of the increased activities independently is provided by an increased amount of an enzyme from a yeast.
013. The engineered microorganism of embodiment 012, wherein the yeast is a Candida yeast.
014. The engineered microorganism of embodiment 013, wherein the yeast is a Candida tropicalis yeast.
015. The engineered microorganism of any one of embodiments 02 to 014, wherein each one of the increased activities independently results from increasing the copy number of a gene that encodes an enzyme that provides the activity.
016. The engineered microorganism of any one of embodiments O1 to 015, wherein each one of the increased activities independently results from inserting a promoter in functional proximity to a gene that encodes an enzyme that provides the activity.
017. The engineered microorganism of embodiment 015 or 016, wherein the gene is in plasmid nucleic acid.
018. The engineered microorganism of embodiment 015 or 016, wherein the gene is in genomic nucleic acid of the microorganism.
019. The engineered microorganism of any one of embodiments O1 to 018, wherein the lipase activity is provided by an increased amount of an enzyme comprising (i) any one of the amino acid sequences of SEQ ID NO: 28 and 29, (ii) an amino acid sequence 90% or more identical to (i), or (iii) an amino acid sequence that includes 1 to 10 amino acid substitutions, insertions or deletions with respect to (i).
020. The engineered microorganism of any one of embodiments O1 to 019, wherein the fatty acid synthase activity is provided by an increased amount of a FAS1-encoded enzyme, a FAS2-encoded enzyme, or FAS1-encoded enzyme and FAS2-encoded enzyme.
021. The engineered microorganism of embodiment 020, wherein the FAS1-encoded enzyme comprises (i) the amino acid sequence of SEQ ID NO: 32, (ii) an amino acid sequence 90% or more identical to (i), or (iii) an amino acid sequence that includes 1 to 10 amino acid substitutions, insertions or deletions with respect to (i).
022. The engineered microorganism of embodiment 020, wherein the FAS2-encoded enzyme comprises (i) the amino acid sequence of SEQ ID NO: 31, (ii) an amino acid sequence 90% or more identical to (i), or (iii) an amino acid sequence that includes 1 to 10 amino acid substitutions, insertions or deletions with respect to (i).
023. The engineered microorganism of any one of embodiments O1 to 022, wherein the glucose-6-phosphate dehydrogenase activity is provided by an increased amount of an enzyme comprising (i) the amino acid sequence of SEQ ID NO: 34, (ii) an amino acid sequence 90% or more identical to (i), or (iii) an amino acid sequence that includes 1 to 10 amino acid substitutions, insertions or deletions with respect to (i).
024. The engineered microorganism of any one of embodiments O1 to 023, wherein the monooxygenase activity is provided by an increased amount of a cytochrome P450 enzyme.
025. The engineered microorganism of embodiment 024, wherein the monooxygenase activity is provided by an exogenous cytochrome P450 enzyme.
026. The engineered microorganism of embodiment 025, wherein the exogenous cytochrome P450 enzyme is from Bacillus megaterium.
027. The engineered microorganism of embodiment 026, wherein the exogenous cytochrome P450 enzyme comprises (i) the amino acid sequence of SEQ ID NO: 41, (ii) an amino acid sequence 90% or more identical to (i), or (iii) an amino acid sequence that includes 1 to 10 amino acid substitutions, insertions or deletions with respect to (i).
028. The engineered microorganism of embodiment 024, wherein two or more endogenous cytochrome P450 enzymes are expressed in increased amounts.
029. The engineered microorganism of embodiment 024, wherein all endogenous cytochrome P450 enzymes are expressed in increased amounts.
030. The engineered microorganism of embodiment 028 or 029, wherein the endogenous cytochrome P450 enzymes comprise (i) an amino acid sequence encoded by a polynucleotide selected from the group consisting of SEQ ID NOS: 13, 14, 15, 16, 17, 18, 19, 20, 21, and 22, (ii) an amino acid sequence 90% or more identical to (i), or (iii) an amino acid sequence that includes 1 to 10 amino acid substitutions, insertions or deletions with respect to (i).
031. The engineered microorganism of any one of embodiments O1 to 030, wherein the monooxygenase reductase activity is provided by an increased amount of an enzyme comprising (i) an amino acid sequence encoded by any one of SEQ ID NOS: 23 to 26, (ii) an amino acid sequence 90% or more identical to (i), or (iii) an amino acid sequence that includes 1 to 10 amino acid substitutions, insertions or deletions with respect to (i).
032. The engineered microorganism of any one of embodiments O1 to 031, wherein the acyl-CoA oxidase activity is provided by an increased amount of a POX4-encoded enzyme, a POX5-encoded enzyme, or a POX4-encoded enzyme and a POX5-encoded enzyme an enzyme.
033. The engineered microorganism of embodiment 032, wherein the POX4-encoded enzyme comprises (i) the amino acid sequence of SEQ ID NO: 39, (ii) an amino acid sequence 90% or more identical to (i), or (iii) an amino acid sequence that includes 1 to 10 amino acid substitutions, insertions or deletions with respect to (i).
034. The engineered microorganism of embodiment 032, wherein the POX5-encoded enzyme comprises (i) the amino acid sequence of SEQ ID NO: 40, (ii) an amino acid sequence 90% or more identical to (i), or (iii) an amino acid sequence that includes 1 to 10 amino acid substitutions, insertions or deletions with respect to (i).
035. The engineered microorganism of any one of embodiments O1 to 034, wherein the microorganism lacks an enzyme providing an acyl-CoA oxidase activity.
036. The engineered microorganism of embodiment 035, wherein the enzyme is a POX4-encoded enzyme or a POX5-encoded enzyme.
037. The engineered microorganism of any one of embodiments O1 to 036, wherein the acyl-CoA hydrolase activity is provided by an increased amount of an enzyme comprising (i) an amino acid sequence encoded by any one of SEQ ID NOS: 43 or 45, (ii) an amino acid sequence 98% or more identical to (i), or (iii) an amino acid sequence that includes 1 to 10 amino acid substitutions, insertions or deletions with respect to (i).
038. The engineered microorganism of any one of embodiments O1 to 037, wherein the acyl-CoA thioesterase activity is provided by an increased amount of an enzyme comprising (i) an amino acid sequence encoded by SEQ ID NO: 47, or (ii) an amino acid sequence that includes 1 to 10 amino acid substitutions, insertions or deletions with respect to (i).
039. The engineered microorganism of any one of embodiments O1 to 038, wherein the microorganism is a yeast.
040. The engineered microorganism of embodiment 039, wherein the yeast is a Candida yeast.
041. The engineered microorganism of embodiment 040, wherein the yeast is a Candida tropicalis yeast.
042. The engineered microorganism of any one of embodiments O1 to 041, wherein the mircroorganism is haploid.
043. The engineered microorganism of any one of embodiments O1 to 041, wherein the mircroorganism is diploid.
P1. A method for producing adipic acid, comprising culturing an engineered microorganism of any one of embodiments O1 to 041 under conditions in which adipic acid is produced.
P2. The method of embodiment P1, wherein the culture conditions comprise fermentation conditions.
P3. The method of embodiment P1 or P2, wherein the culture conditions comprise introduction of biomass.
P4. The method of any one of embodiments P1 to P3, wherein the culture conditions comprise introduction of a feedstock comprising glucose.
P5. The method of any one of embodiments P1 to P4, wherein the culture conditions comprise introduction of a feedstock comprising hexane.
P6. The method of any one of embodiments P1 to P5, wherein the culture conditions comprise introduction of a feedstock comprising an oil.
P7. The method of any one of embodiments P4 to P6, wherein the adipic acid is produced with a yield of greater than about 0.15 grams per gram of the glucose, hexane or oil.
P7.1. The method of any one of embodiments P4 to P7, wherein the adipic acid is produced at between about 25% and about 100% of maximum theoretical yield of any introduced feedstock.
P7.2. The method of any one of embodiments P4 to P7.1, wherein the adipic acid is produced in a concentration range of between about 50 g/L to about 1000g/L of culture media.
P7.3. The method of any one of embodiments, P4 to P7.2, wherein the adipic acid is produced at a rate of between about 0.5 g/L/hour to about 5 g/L/hour.
P7.4. The method of any one of embodiments, P1 to P7.3, wherein the engineered organism comprises between about a 5-fold to about a 300-fold increase in adipic acid production when compared to wild-type or partially engineered organisms of the same strain, under identical fermentation conditions.
P8. The method of any one of embodiments P1 to P7.4, which comprises purifying the adipic acid from the cultured microorganisms.
P9. The method of embodiment P8, which comprises modifying the adipic acid, thereby producing modified adipic acid.
P10. The method of any one of embodiments P1 to P9, which comprises placing the cultured microorganisms, the adipic acid or the modified adipic acid in a container.
P11. The method of embodiment P10, which comprises shipping the container.
Q1. A genetically modified microorganism comprising one or more increased activities, with respect to the activity level in an unmodified or parental strain, which increased activities are chosen from: a monooxygenase activity, a monooxygenase reductase activity, an acyl-CoA oxidase activity, an acyl-CoA hydrolase activity, an acyl-CoA thioesterase activity and combinations of the forgoing.
Q1.1. The genetically modified microorganism of embodiment Q1, wherein the monooxygenase activity comprises a cytochrome P450 A19 (e.g., CYP52A19) activity.
Q1.2. The genetically modified microorganism of embodiment Q1, wherein the monooxygenase activity consists of a cytochrome P450 A19 (e.g., CYP52A19) activity.
Q1.3. The genetically modified microorganism of embodiment Q1, wherein the monooxygenase reductase activity comprises one or more activities selected from CPR, CPRA, CPRB, and combinations of the foregoing.
Q1.4. The genetically modified microorganism of embodiment Q1, wherein the acyl-CoA oxidase activity comprises a POX5 activity.
Q1.5. The genetically modified microorganism of embodiment Q1, wherein the acyl-CoA oxidase activity consists of POX5 activity.
Q1.6. The genetically modified microorganism of embodiment Q1, wherein the acyl-CoA hydrolase activity comprises one or more activities selected from ACHA activity, ACHB activity, and ACHA activity and ACHB activity.
Q1.7. The genetically modified microorganism of embodiment Q1, wherein the acyl-CoA thioesterase activity comprises a TESA activity.
Q2. The genetically modified microorganism of embodiment Q1, wherein the one or more increased activities are three or more increased activities.
Q3. The genetically modified microorganism of embodiment Q2, wherein the three or more increased activities are four or more increased activities.
Q4. The genetically modified microorganism of embodiment Q2, wherein the three or more increased activities are five or more increased activities.
Q5. The genetically modified microorganism of embodiment Q2, wherein the three or more increased activities comprise an increased monooxygenase activity, an increased monooxygenase reductase activity, and an increased acyl-CoA hydrolase activity.
Q6. The genetically modified microorganism of embodiment Q2, wherein the three or more increased activities comprise an increased monooxygenase activity, an increased monooxygenase reductase activity, and an increased acyl-CoA thioesterase activity.
Q7. The genetically modified microorganism of embodiment Q3, wherein the four or more increased activities comprise an increased monooxygenase activity, an increased monooxygenase reductase activity, an increased acyl-CoA oxidase activity and an increased acyl-CoA hydrolase activity.
Q8. The genetically modified microorganism of embodiment Q3, wherein the four or more increased activities comprise an increased monooxygenase activity, an increased monooxygenase reductase activity, an increased acyl-CoA hydrolase activity and an increased acyl-CoA thioesterase activity.
Q9. The genetically modified microorganism of embodiment Q4, wherein the five or more increased activities comprise an increased monooxygenase activity, an increased monooxygenase reductase activity, an increased acyl-CoA oxidase activity, and increased acyl-CoA thioesterase activity and an increased acyl-CoA hydrolase activity.
Q10. The genetically modified microorganism of any one of embodiments Q1 to Q9, further comprising one or more reduced activities, with respect to the activity level in an unmodified or parental strain, which reduced activities are chosen from: acyl-CoA synthetase activity, long chain acyl-CoA synthetase activity, acyl-CoA sterol acyl transferase activity, acyltransferase activity, and combinations of the foregoing.
Q10.1. The genetically modified microorganism of embodiment Q10, wherein the acyl-CoA synthetase activity comprises an ACS1 activity.
Q10.2. The genetically modified microorganism of embodiment Q10, wherein the long chain acyl-CoA synthetase activity comprises a FAT1 activity.
Q10.3. The genetically modified microorganism of embodiment Q10, wherein the acyl-CoA sterol acyl transferase activity comprises one or more activities selected from an ARE1 activity, an ARE2 activity, and an ARE1 activity and an ARE2 activity.
Q10.4. The genetically modified microorganism of embodiment Q10, wherein the acyltransferase activity is a diacylglycerol acyltransferase activity.
Q10.5. The genetically modified microorganism of embodiment Q10.4, wherein the diacylglycerol acyltransferase activity comprises one or more activities selected from a DGA1 activity, a LRO1 activity and a DGA1 activity and a LRO1 activity.
Q11. The genetically modified microorganism of any one of embodiments Q10 to Q10.5, wherein the one or more reduced activities is three or more reduced activities.
Q12. The genetically modified microorganism of embodiment Q11, wherein the three or more reduced activities are four or more reduced activities.
Q13. The genetically modified microorganism of embodiment Q12, wherein the three or more reduced activities are five or more reduced activities.
Q14. The genetically modified microorganism of any one of embodiments Q10 to Q13, comprising a reduced ACS1 activity, a reduced FAT1 activity, a reduced ARE1 activity, a reduced ARE2 activity, a reduced DGA1 activity and a reduced LRO1 activity.
R1. A method for preparing a microorganism that produces adipic acid, which comprises: (a) introducing one or more genetic modifications to a host organism that add or increase one or more activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, acyl-CoA hydrolase activity, acyl-CoA thioesterase activity, monooxygenase activity, and monooxygenase reductase activity, thereby producing engineered microorganisms, and (b) selecting for engineered microorganisms that produce adipic acid.
R2. The method of embodiment R1, which comprises selecting for engineered microorganisms having one or more detectable and/or increased activities selected from the group consisting of 6-oxohexanoic acid dehydrogenase activity, omega oxo fatty acid dehydrogenase activity, 6-hydroxyhexanoic acid dehydrogenase activity, omega hydroxyl fatty acid dehydrogenase activity, glucose-6-phosphate dehydrogenase, hexanoate synthase activity, lipase activity, fatty acid synthase activity, acetyl CoA carboxylase activity, acyl-CoA hydrolase activity, acyl-CoA thioesterase activity, monooxygenase activity, and monooxygenase reductase activity, relative to the host microorganism.
R3. The method of embodiment R1 or R2, which comprises introducing a genetic modification that reduces one or more activities selected from acyl-CoA oxidase, acyl-CoA synthetase activity, long chain acyl-CoA synthetase activity, acyl-CoA sterol acyl transferase activity, acyltransferase activity, and 6-hydroxyhexanoic acid conversion activity, thereby producing engineered microorganisms, and selecting for engineered microorganisms having one or more reduced activities selected from acyl-CoA oxidase, acyl-CoA synthetase activity, long chain acyl-CoA synthetase activity, acyl-CoA sterol acyl transferase activity, acyltransferase activity, and 6-hydroxyhexanoic acid conversion activity relative to the host microorganism.
S1. A method for producing adipic acid, comprising:
   contacting an engineered microorganism with a feedstock comprising one or more sugars, cellulose, fatty acids, triacylglycerides or combinations of the forgoing, wherein the engineered microorganism comprises:
      a. a genetic alteration that partially blocks beta oxidation activity,
      b. a genetic alteration that adds or increases a monooxygenase activity,
      c. a genetic alteration that adds or increases a monooxygenase reductase activity, and
      d. a genetic alteration that adds or increases an acyl-CoA hydrolase and/or an acyl-CoA thioesterase activity,
   culturing the engineered microorganism under conditions in which adipic acid is produced.
S2. The method of embodiment S1, wherein the engineered microorganism further comprises one or more genetic alterations that reduce an activity selected from an acyl-CoA oxidase activity, an acyl-CoA synthetase activity, a long chain acyl-CoA synthetase activity, an acyl-CoA sterol acyl transferase activity, and an acyltransferase activity.
S3. The method of embodiment S2, wherein the acyltransferase activity is a diacyl-glycerol acyltransferase activity.
S4. The method of any one of embodiments S1-S3, wherein the engineered microorganism comprises a heterologous polynucleotide encoding a polypeptide having acyl-CoA thioesterase activity.
S5. The method of embodiment S4, wherein the heterologous polynucleotide independently is selected from a bacterium
S6. The method of embodiment S5, wherein the bacterium is an Enteric bacterium.
S7. The method of embodiment S6, wherein the Enteric bacterium is E. coli.
S8. The method of any one of embodiments S1 to S7, wherein the microorganism is a Candida yeast.
S9. The method of embodiment S8, wherein the Candida yeast is C. tropicalis
S10. The method of any one of embodiments S1 to S9, wherein the engineered microorganism comprises a heterologous polynucleotide encoding a polypeptide having acyl-CoA thioesterase activity, and further wherein the polynucleotide sequence has been codon optimized for expression in C. tropicalis.
S11. The method of any one of embodiments S1 to S10, wherein the genetic alteration that partially blocks beta oxidation activity reduces or eliminates POX4 activity.
S12. The method of any one of embodiments S1 to S11, wherein the genetic alteration that adds or increases monooxygenase activity, increases CYP52A19 activity.
S13. The method of any one of embodiments S1 to S12, wherein the genetic alteration that adds or increases monooxygenase reductase activity increases a CPR activity, a CPRA activity, a CPRB activity, or combinations thereof.
S14. The method of any one of embodiments S1 to S13, wherein the genetic alteration that adds or increases acyl-CoA hydrolase activity increases an ACHA activity, an ACHB activity or an ACHA activity and an ACHB activity.
S15. The method of any one of embodiments S1 to S14, wherein the genetic alteration that adds or increases acyl-CoA thioesterase activity adds an E. coli derived TESA activity.
S16. The method of any one of embodiments S2 to S15, wherein the genetic alteration that reduces an acyl-CoA synthetase activity, reduces or eliminates an ACS1 activity.
S17. The method of any one of embodiments S2 to S16, wherein the genetic alteration that reduces a long chain acyl-CoA synthetase activity, reduces or eliminates an FAT1 activity.
S18. The method of any one of embodiments S2 to S17, wherein the genetic alteration that reduces an acyl-CoA sterol acyl transferase activity, reduces or eliminates an ARE1 activity, an ARE2 activity, or an ARE1 activity and an ARE2 activity.
S19. The method of any one of embodiments S2 to S18, wherein the genetic alteration that reduces an acyltransferase activity, reduces or eliminates a DGA1 activity, a LRO1 activity or a DGA1 activity and a LRO1 activity.
S20. The method of any one of embodiments S2 to S19, wherein the adipic acid is produced at a level of about 80% or more of the theoretical yield
S21. The method of any one of embodiments S2 to S19, the maximum theoretical yield (Yₘₐₓ) is about 0.6 grams of adipic acid produced per gram of coconut oil added, the percentage of Yₘₐₓ for the engineered microorganism under conditions in which adipic acid is produced is calculated as (%Ymax) = Y_{p/s} / Yₘₐₓ *100, where (Y_{p/s}) = [adipic acid (g/L] * final volume of culture in flask (L)] / [feedstock added to flask (g)].
S22. The method of any one of embodiments S2 to S24, wherein the engineered microorganism produces adipic acid at about 10% to about 100% of maximum theoretical yield.
T1. We describe an isolated polynucleotide selected from the group consisting of:
   a polynucleotide having a nucleotide sequence 96% or more identical to the nucleotide sequence of SEQ ID NO: 42 or 44:
      a polynucleotide having a nucleotide sequence that encodes a polypeptide of SEQ ID NO: 43 or 45; and
      a polynucleotide having a portion of a nucleotide sequences 96% or more identical to the nucleotide sequence of SEQ ID NO: 42 or 44 and encodes a polypeptide having acyl-coA hydrolase activity.
T2. We describe an isolated polynucleotide selected from the group consisting of:
   a polynucleotide having a nucleotide sequences identical to the nucleotide sequence of SEQ ID NO: 46:
      a polynucleotide having a nucleotide sequence that encodes a polypeptide of SEQ ID NO: 47; and
      a polynucleotide having a portion of a nucleotide sequence identical to the nucleotide sequences of SEQ ID NO: 46 and encodes a polypeptide having acyl-CoA thioesterase activity.
T3. We describe an expression vector comprising a polynucleotide of embodiments T1 or T2.
T4. We describe an integration vector comprising a polynucleotide of embodiments T1 or T2.
T5. We describe an microorganism comprising an expression vector of embodiment T3, or an integration vector of embodiment T4.
T6. We describe a culture comprising a microorganism of embodiment T5.
T7. We describe a fermentation device comprising a microorganism of embodiment T6.
T8. We describe a polypeptide encoded by a polynucleotide of embodiment T1 or T2 or produced by an expression vector of embodiment T3 or microorganism of embodiment T5.
T9. We describe an antibody that specifically binds to a polypeptide of embodiment T8.
U1. We describe an n engineered microorganism capable of producing adipic acid, which microorganism comprises genetic alterations resulting in one or more increased activities and further resulting in commitment of molecular pathways in directions for production of adipic acid, which pathways and directions include: (i) fatty acid synthesis pathway in the direction of acetyl CoA to long-chain fatty acids and away from synthesis or generation of biomass and/or carbon storage molecules, (ii) omega oxidation pathway in the direction of long-chain fatty acids to diacids and (iii) beta oxidation pathway in the direction of diacids to adipic acid.
U2. The engineered microorganism of embodiment U1, wherein carbon storage molecules comprise storage starches, storage lipids and combinations thereof.
U3. The engineered microorganism of embodiment U1 or U2, which comprises an increased activity, relative to the microorganism not containing the genetic alterations, independently in each pathway.
U4. The engineered microorganism of any one of embodiments U1 to U3, comprising an increased monooxygenase activity.
U5. The engineered microorganism of any one of embodiments U1 to U4, comprising an increased monooxygenase reductase activity.
U6. The engineered microorganism of any one of embodiments U1 to U5, comprising an increased acyl-CoA oxidase activity.
U7. The engineered microorganism of any one of embodiments U1 to U6, comprising an increased acetyl CoA carboxylase activity in the fatty acid synthesis pathway.
U8. The engineered microorganism of any one of embodiments U1 to U7, comprising an increased fatty acid synthase activity in the fatty acid synthesis pathway.
U9. The engineered microorganism of any one of embodiments U1 to U8, comprising an increased acyl-CoA hydrolase activity in the fatty acid synthesis pathway.
U10. The engineered microorganism of any one of embodiments U1 to U9, wherein each of the increased activities independently is provided by an enzyme encoded by a gene endogenous to the microorganism.
U11. The engineered microorganism of any one of embodiments U1 to U10, wherein each of the increased activities independently is provided by an increased amount of an enzyme from a yeast.
U12. The engineered microorganism of embodiment U11, wherein the yeast is a Candida yeast.
U13. The engineered microorganism of embodiment U12, wherein the yeast is a Candida tropicalis yeast.
U14. The engineered microorganism of any one of embodiments U1 to U13, comprising an added acyl-CoA thioesterase activity in the fatty acid synthesis pathway.
U15. The engineered microorganism of embodiment U14, wherein the added activity independently is provided by an enzyme encoded by a gene exogenous to the microorganism.
U16. The engineered microorganism of any one of embodiments U1 to U15, further comprising one or more reduced activities selected from an acyl-CoA oxidase activity, an acyl-CoA synthetase activity, a long chain acyl-CoA synthetase activity, an acyl-CoA sterol acyl transferase activity, and an acyltransferase activity.
U17. The method of embodiment U16, wherein the acyltransferase activity is a diacyl-glycerol acyltransferase activity.
V1. An engineered yeast comprising genetic modifications that (a) reduce an acyl-CoA oxidase activity and (b) reduce an acyl-CoA synthetase activity, reduce a long chain acyl-CoA synthetase activity, or reduce an acyl-CoA synthetase activity and reduce a long chain acyl-CoA synthetase activity.
V2. The engineered yeast of embodiment V1, comprising a genetic modification that partially blocks beta oxidation.
V3. The engineered yeast of embodiment V1, wherein the genetic modification reduces the expression level of a polypeptide having an acyl-CoA oxidase activity.
V4. The engineered yeast of any one of embodiments V1 to V3, wherein the reduced acyl-CoA oxidase activity is a reduced POX4 activity.
V5. The engineered yeast of embodiment V4, wherein the reduced POX4 activity results from a reduction in the level of expression of a polypeptide comprising (i) the amino acid sequence of SEQ ID NO: 39, (ii) an amino acid sequence 90% or more identical to (i), or (iii) an amino acid sequence that includes 1 to 10 amino acid substitutions, insertions or deletions with respect to (i).
V6. The engineered yeast of any one of embodiments V1 to V5, comprising genetic modifications that reduce the long chain acyl-CoA synthetase activity and do not reduce the acyl-CoA synthetase activity.
V7. The engineered yeast of embodiment V6, wherein the reduced long chain acyl-CoA synthetase activity is a FAT1 activity.
V8. The engineered yeast of embodiment V7, wherein the reduced FAT1 activity results from a reduction in the level of expression of a polypeptide comprising (i) the amino acid sequence of SEQ ID NO: 51, (ii) an amino acid sequence 90% or more identical to (i), or (iii) an amino acid sequence that includes 1 to 10 amino acid substitutions, insertions or deletions with respect to (i).
V9. The engineered yeast of any one of embodiments V1 to V5, comprising genetic modifications that reduce the acyl-CoA synthetase activity and do not reduce the long chain acyl-CoA synthetase activity.
V10. The engineered yeast of embodiment V9, wherein the reduced acyl-CoA synthetase activity is an ACS1 activity.
V11. The engineered yeast of embodiment V10, wherein the reduced ACS1 activity results from a reduction in the level of expression of a polypeptide comprising (i) the amino acid sequence of SEQ ID NO: 49, (ii) an amino acid sequence 90% or more identical to (i), or (iii) an amino acid sequence that includes 1 to 10 amino acid substitutions, insertions or deletions with respect to (i).
V12. The engineered yeast of any one of embodiments V1 to V5, comprising genetic modifications that reduce the long chain acyl-CoA synthetase activity and the acyl-CoA synthetase activity.
V13. The engineered yeast of embodiment V12, wherein the reduced long chain acyl-CoA synthetase activity is a FAT1 activity and the reduced acyl-CoA synthetase activity is an ACS1 activity.
V14. The engineered yeast of embodiment V13, wherein the reduced ACS1 activity results from a reduction in the level of expression of a polypeptide comprising (i) the amino acid sequence of SEQ ID NO: 49, (ii) an amino acid sequence 90% or more identical to (i), or (iii) an amino acid sequence that includes 1 to 10 amino acid substitutions, insertions or deletions with respect to (i), and the reduced FAT1 activity results from a reduction in the level of expression of a polypeptide comprising (iv) the amino acid sequence of SEQ ID NO: 51, (v) an amino acid sequence 90% or more identical to (iv), or (vi) an amino acid sequence that includes 1 to 10 amino acid substitutions, insertions or deletions with respect to (iv).
V15. The engineered yeast of any one of embodiments V1 to V14, comprising one or more genetic modifications that increase one or more activities, with respect to the activity level in an unmodified or parental strain, which increased activities are chosen from: an acyl-CoA oxidase activity, one or more monooxygenase activities, a monooxygenase reductase activity and an acyl-CoA hydrolase activity.
V16. The engineered yeast of embodiment V15, wherein each of the one or more increased activities independently is provided by an increased amount of an enzyme from a yeast.
V17. The engineered yeast of embodiment V16, wherein each of the one or more increased activities independently results from increasing the copy number of a gene that encodes an enzyme that provides the activity.
V18. The engineered yeast of embodiment V6, wherein each of the one or more increased activities independently results from inserting a promoter in functional proximity to a gene that encodes an enzyme that provides the activity.
V19. The engineered yeast of any one of embodiments V15 to V18, wherein a genetic modification that increases an acyl-CoA oxidase activity increases a POX5 activity.
V20. The engineered yeast of embodiment V19, wherein the POX5 activity is provided by an increased amount of an enzyme comprising (i) the amino acid sequence of SEQ ID NO: 40, (ii) an amino acid sequence 90% or more identical to (i), or (iii) an amino acid sequence that includes 1 to 10 amino acid substitutions, insertions or deletions with respect to (i).
V21. The engineered yeast of any one of embodiments V15 to V20, wherein a genetic modification that increases an acyl-CoA hydrolase activity increases an ACH activity.
V22. The engineered yeast of embodiment V21, wherein the ACH activity is an ACHA activity, an ACHB activity or an ACHA and an ACHB activity.
V23. The engineered yeast of embodiment V21 or V22, wherein the ACHA activity is provided by an increased amount of an enzyme comprising (i) the amino acid sequence of SEQ ID NO: 43, (ii) an amino acid sequence 90% or more identical to (i), or (iii) an amino acid sequence that includes 1 to 10 amino acid substitutions, insertions or deletions with respect to (i), and the ACHB activity is provided by an increased amount of an enzyme comprising (iv) the amino acid sequence of SEQ ID NO: 45, (v) an amino acid sequence 90% or more identical to (iv), or (vi) an amino acid sequence that includes 1 to 10 amino acid substitutions, insertions or deletions with respect to (iv).
V24. The engineered yeast of any one of embodiments V15 to V23, wherein the one or more monooxygenase activities comprises one or more cytochrome P450 activities chosen from: a CYP52A13 activity, a CYP52A14 activity, a CYP52A15 activity, a CYP52A16 activity, and a CYP52A19 activity.
V25. The engineered yeast of embodiment V24, wherein the CYP52A13 activity is provided by an increased amount of an enzyme comprising (i) the amino acid sequence of SEQ ID NO: 63, (ii) an amino acid sequence 90% or more identical to (i), or (iii) an amino acid sequence that includes 1 to 10 amino acid substitutions, insertions or deletions with respect to (i).
V26. The engineered yeast of embodiment V24, wherein the CYP52A14 activity is provided by an increased amount of an enzyme comprising (i) the amino acid sequence of SEQ ID NO: 65, (ii) an amino acid sequence 90% or more identical to (i), or (iii) an amino acid sequence that includes 1 to 10 amino acid substitutions, insertions or deletions with respect to (i).
V27. The engineered yeast of embodiment V24, wherein the CYP52A15 activity is provided by an increased amount of an enzyme comprising (i) the amino acid sequence of SEQ ID NO: 67, (ii) an amino acid sequence 90% or more identical to (i), or (iii) an amino acid sequence that includes 1 to 10 amino acid substitutions, insertions or deletions with respect to (i).
V28. The engineered yeast of embodiment V24, wherein the CYP52A16 activity is provided by an increased amount of an enzyme comprising (i) the amino acid sequence of SEQ ID NO: 69, (ii) an amino acid sequence 90% or more identical to (i), or (iii) an amino acid sequence that includes 1 to 10 amino acid substitutions, insertions or deletions with respect to (i).
V29. The engineered yeast of embodiment V24, wherein the CYP52A19 activity is provided by an increased amount of an enzyme comprising (i) the amino acid sequence of SEQ ID NO: 75, (ii) an amino acid sequence 90% or more identical to (i), or (iii) an amino acid sequence that includes 1 to 10 amino acid substitutions, insertions or deletions with respect to (i).
V30. The engineered yeast of any one of embodiments V15 to V29, wherein a genetic modification that increases a monooxygenase reductase activity increases a CPR activity.
V31. The engineered yeast of embodiment V30, wherein the CPR activity is a CPRB activity.
V32. The engineered yeast of embodiment V30 and V31, wherein the CPRB activity is provided by an increased amount of an enzyme comprising (i) the amino acid sequence of SEQ ID NO: 81, (ii) an amino acid sequence 90% or more identical to (i), or (iii) an amino acid sequence that includes 1 to 10 amino acid substitutions, insertions or deletions with respect to (i).
V33. The genetically modified yeast of any one of embodiments V15 to V32, wherein the one or more increased activities are three or more increased activities.
V34. The genetically modified yeast of embodiment V33, wherein the three or more increased activities are four or more increased activities.
V35. The genetically modified yeast of embodiment V33, wherein the three or more increased activities are five or more increased activities.
V36. The genetically modified yeast of embodiment V33, wherein the three or more increased activities are six or more increased activities.
V37. The genetically modified yeast of embodiment V33, wherein the three or more increased activities comprise an increased monooxygenase activity, an increased monooxygenase reductase activity, and an increased acyl-CoA oxidase activity.
V38. The genetically modified yeast of embodiment V34, wherein the four or more increased activities comprise an increased monooxygenase activity, an increased monooxygenase reductase activity, an increase acyl-CoA oxidase, and an increased acyl-CoA hydrolase activity.
V39. The genetically modified yeast of embodiment V35, wherein the five or more increased activities comprise at least two increased monooxygenase activities, an increased monooxygenase reductase activity, an increased acyl-CoA oxidase activity, and an increased acyl-CoA hydrolase activity.
V40. The genetically modified yeast of embodiment V36, wherein the six or more increased activities comprise at least two increased monooxygenase activities, an increased monooxygenase reductase activity, an increased acyl-CoA oxidase activity, and at least two increased acyl-CoA hydrolase activities.
V41. The genetically modified yeast of any one of embodiments V1 to V40, wherein the yeast is a Candida yeast.
V42. The genetically modified yeast of embodiment V41, wherein the Candida yeast is a Candida tropicalis yeast.
V43. The genetically modified yeast of embodiment V41, wherein the Candida yeast is a Candida viswanithii yeast.
V44. The genetically modified yeast of any one of embodiments V1 to V43, wherein the genetically modified yeast is derived from an ancestral Candida yeast cell line chosen from: ATCC 20362, ATCC 8862, ATCC 18944, ATCC 20228, ATCC 76982, LGAM S(7)1, ATCC 20336, ATCC20913, SU-2 (ura3-/ura3-), ATCC 20962, ATCC 24690, ATCC 38163, H5343, ATCC 8661, ATCC 8662, ATCC 9773, ATCC 15586, ATCC 16617, ATCC 16618, ATCC 18942, ATCC 18943, ATCC 18944, ATCC 18945, ATCC 20114, ATCC 20177, ATCC 20182, ATCC 20225, ATCC 20226, ATCC 20228, ATCC 20237, ATCC 20255, ATCC 20287, ATCC 20297, ATCC 20306, ATCC 20315, ATCC 20320, ATCC 20324, ATCC 20341, ATCC 20346, ATCC 20348, ATCC 20362, ATCC 20363, ATCC 20364, ATCC 20372, ATCC 20373, ATCC 20383, ATCC 20390, ATCC 20400, ATCC 20460, ATCC 20461, ATCC 20462, ATCC 20496, ATCC 20510, ATCC 20628, ATCC 20688, ATCC 20774, ATCC 20775, ATCC 20776, ATCC 20777, ATCC 20778, ATCC 20779, ATCC 20780, ATCC 20781, ATCC 20794, ATCC 20795, ATCC 20875, ATCC 22421, ATCC 22422, ATCC 22423, ATCC 22969, ATCC 32338, ATCC 32339, ATCC 32340, ATCC 32341, ATCC 32342, ATCC 32343, ATCC 32935, ATCC 34017, ATCC 34018, ATCC 34088, ATCC 34922, ATCC 38295, ATCC 42281, ATCC 44601, ATCC 46025, ATCC 46026, ATCC 46027, ATCC 46028, ATCC 46067, ATCC 46068, ATCC 46069, ATCC 46070, ATCC 46330, ATCC 46482, ATCC 46483, ATCC 46484, ATCC 48436, ATCC 60594, ATCC 62385, ATCC 64042, ATCC 74234, ATCC 76598, ATCC 76861, ATCC 76862, ATCC 90716, ATCC 90806, ATCC 90811, ATCC 90812, ATCC 90813, ATCC 90814, ATCC 90903, ATCC 90904, ATCC 90905, ATCC 96028, ATCC 201089, ATCC 201241, ATCC 201242, ATCC 201243, ATCC 201244, ATCC 201245, ATCC 201246, ATCC 201247, ATCC 201248, ATCC 201249, ATCC 201847, ATCC MYA-165, ATCC MYA-166, ATCC MYA-2613, and ATCC MYA-4467.

The technology illustratively described herein suitably may be practiced in the absence of any element(s) not specifically disclosed herein. Thus, for example, in each instance herein any of the terms "comprising," "consisting essentially of," and "consisting of" may be replaced with either of the other two terms. The terms and expressions which have been employed are used as terms of description and not of limitation, and use of such terms and expressions do not exclude any equivalents of the features shown and described or portions thereof, and various modifications are possible within the scope of the claimed technology. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%). Thus, it should be understood that although the present technology has been specifically disclosed by representative embodiments and optional features, modification and variation of the concepts herein disclosed may be resorted to by those skilled in the art, and such modifications and variations are considered within the scope of this technology.

## Claims

1. A composition comprising genetically modified Candida yeast comprising:
(a) an altered acyl-CoA oxidase activity, which altered acyl-CoA oxidase activity results from a disruption, deletion or knockout of (i) a polynucleotide that encodes a POX4 polypeptide whereby the acyl-CoA oxidase activity is reduced or removed, wherein said POX4 polypeptide comprises the amino acid sequence of SEQ ID NO: 39, an amino acid sequence 90% or more identical to SEQ ID NO: 39, or an amino acid sequence that includes 1 to 10 amino acid substitutions, insertions or deletions with respect to SEQ ID NO: 39; and
(b) an altered long chain acyl-CoA synthetase activity, which altered long chain acyl-CoA synthetase activity results from a disruption, deletion or knockout of (i) a polynucleotide that encodes a FAT1 polypeptide whereby the long chain acyl-CoA synthetase activity is reduced or removed, wherein said FAT1 polypeptide comprises the amino acid sequence of SEQ ID NO: 51, an amino acid sequence 90% or more identical to SEQ ID NO: 51, or an amino acid sequence that includes 1 to 10 amino acid substitutions, insertions or deletions with respect to SEQ ID NO: 51.

2. The composition of claim 1, wherein the Candida yeast comprises (d) an altered acyl-CoA synthetase activity, which altered acyl-CoA synthetase activity results from a disruption, deletion or knockout of (i) a polynucleotide that encodes an ACS1 polypeptide whereby the acyl-CoA synthetase activity is reduced or removed, wherein said ACS 1 polypeptide comprises the amino acid sequence of SEQ ID NO: 49, an amino acid sequence 90% or more identical to SEQ ID NO: 49, or an amino acid sequence that includes 1 to 10 amino acid substitutions, insertions or deletions with respect to SEQ ID NO: 49.

3. The composition of claims 1 or 2, wherein the Candida yeast comprises an increased copy number of a polynucleotide that encodes a POX5 polypeptide, wherein said POX5 activity is provided by an enzyme comprising the amino acid sequence of SEQ ID NO: 40, or an amino acid sequence 90% or more identical to SEQ ID NO: 40, or an amino acid sequence that includes 1 to 10 amino acid substitutions, insertions or deletions with respect to SEQ ID NO: 40.

4. The composition of any one of claims 1 to 3, wherein the Candida yeast comprises (i) an increased copy number of a polynucleotide that encodes a CYP52A14, CYP52A15, CYP52A16 or CYP52A19 polypeptide or (ii) a promoter inserted and operably linked to a polynucleotide that encodes a CYP52A15, CYP52A16 or CYP52A19 polypeptide, wherein said CYP52A14 polypeptide comprises the amino acid sequence of SEQ ID NO: 65, an amino acid sequence 90% or more identical to SEQ ID NO: 65, or an amino acid sequence that includes 1 to 10 amino acid substitutions, insertions or deletions with respect to SEQ ID NO: 65; said CYP52A15 polypeptide comprises the amino acid sequence of SEQ ID NO: 67, an amino acid sequence 90% or more identical to SEQ ID NO: 67, or an amino acid sequence that includes 1 to 10 amino acid substitutions, insertions or deletions with respect to SEQ ID NO: 67; said CYP52A16 polypeptide comprises the amino acid sequence of SEQ ID NO: 69, an amino acid sequence 90% or more identical to SEQ ID NO: 69, or an amino acid sequence that includes 1 to 10 amino acid substitutions, insertions or deletions with respect to SEQ ID NO: 69 and said CYP52A19 polypeptide comprises the amino acid sequence of SEQ ID NO: 75, an amino acid sequence 90% or more identical to SEQ ID NO: 75, or an amino acid sequence that includes 1 to 10 amino acid substitutions, insertions or deletions with respect to SEQ ID NO: 75

5. The composition of claim 4, wherein the Candida yeast comprises (i) an increased copy number of a polynucleotide that encodes a CYP52A19 polypeptide or (ii) a promoter inserted and operably linked to a polynucleotide that encodes a CYP52A19 polypeptide.

6. The composition of claim 4, wherein the polynucleotide that encodes the CYP52A14 polypeptide encodes a polypeptide comprising the amino acid sequence of SEQ ID NO: 65.

7. The composition of claim 4, wherein the polynucleotide that encodes the CYP52A15 polypeptide encodes a polypeptide comprising the amino acid sequence of SEQ ID NO: 67.

8. The composition of claim 4, wherein the polynucleotide that encodes the CYP52A16 polypeptide encodes a polypeptide comprising the amino acid sequence of SEQ ID NO: 69.

9. The composition of claim 4 or 5, wherein the polynucleotide that encodes the CYP52A19 polypeptide encodes a polypeptide comprising the amino acid sequence of SEQ ID NO: 75.

10. The composition of any one of claims 1 to 9, wherein the Candida yeast is a genetically modified Candida strain ATCC20962 or ATCC20336.

11. A method for producing adipic acid, comprising:
(a) contacting a Candida yeast with a feedstock comprising a vegetable oil, wherein the Candida yeast comprises:
composition comprising genetically modified Candida yeast comprising:
(1) an altered acyl-CoA oxidase activity, which altered acyl-CoA oxidase activity results from a disruption, deletion or knockout of (i) a polynucleotide that encodes a POX4 polypeptide whereby the acyl-CoA oxidase activity is reduced or removed, wherein said POX4 polypeptide comprises the amino acid sequence of SEQ ID NO: 39, an amino acid sequence 90% or more identical to SEQ ID NO: 39, or an amino acid sequence that includes 1 to 10 amino acid substitutions, insertions or deletions with respect to SEQ ID NO: 39; and
(2) an altered long chain acyl-CoA synthetase activity, which altered long chain acyl-CoA synthetase activity results from a disruption, deletion or knockout of (i) a polynucleotide that encodes a FAT1 polypeptide whereby the long chain acyl-CoA synthetase activity is reduced or removed wherein said FAT1 polypeptide comprises the amino acid sequence of SEQ ID NO: 51, an amino acid sequence 90% or more identical to SEQ ID NO: 51, or an amino acid sequence that includes 1 to 10 amino acid substitutions, insertions or deletions with respect to SEQ ID NO: 51; and
(b) culturing the Candida yeast under conditions in which adipic acid is produced.

12. The method of claim 11, wherein the Candida yeast comprises (c) an altered acyl-CoA synthetase activity, which altered acyl-CoA synthetase activity results from a disruption, deletion or knockout of (i) a polynucleotide that encodes an ACS1 polypeptide whereby the acyl-CoA synthetase activity is reduced or removed wherein said ACS1 polypeptide comprises the amino acid sequence of SEQ ID NO: 49, an amino acid sequence 90% or more identical to SEQ ID NO: 49, or an amino acid sequence that includes 1 to 10 amino acid substitutions, insertions or deletions with respect to SEQ ID NO: 49.

13. The method of claims 11 or 12, wherein the Candida yeast comprises (i) an increased copy number of a polynucleotide that encodes a POX5 polypeptide activity, and wherein said POX5 activity is provided by an enzyme comprising the amino acid sequence of SEQ ID NO: 40, or an amino acid sequence 90% or more identical to SEQ ID NO: 40, or an amino acid sequence that includes 1 to 10 amino acid substitutions, insertions or deletions with respect to SEQ ID NO: 40.

14. The method of any one of claims 12 or 13, wherein the Candida yeast comprises (i) an increased copy number of a polynucleotide that encodes a POX5 polypeptide.

## Patentansprüche

1. Zusammensetzung, die gentechnisch veränderte Candida-Hefe umfasst, Folgendes umfassend:
(a) eine veränderte Acyl-CoA-Oxidase-Aktivität, wobei die veränderte Acyl-CoA-Oxidase-Aktivität resultiert aus: einer Disruption, einer Deletion oder einem Knock-out von (i) einem Polynukleotid, das für ein POX4-Polypeptid codiert, wodurch die Acyl-CoA-Oxidase-Aktivität reduziert oder aufgehoben wird, wobei das POX4-Polypeptid die Aminosäuresequenz der SEQ ID NR. 39, eine Aminosäuresequenz, die zu 90 % oder mehr mit der SEQ ID NR. 39 identisch ist, oder eine Aminosäuresequenz, die 1 bis 10 Aminosäuresubstitutionen, -insertionen oder-deletionen bezogen auf die SEQ ID NR. 39 enthält, umfasst; und
(b) eine veränderte Langketten-Acyl-CoA-Synthetase-Aktivität, wobei die veränderte Langketten-Acyl-CoA-Synthetase-Aktivität resultiert aus: einer Disruption, einer Deletion oder einem Knock-out von (i) einem Polynukleotid, das für ein FAT1-Polypeptid codiert, wodurch die Langketten-Acyl-CoA-Synthetase-Aktivität reduziert oder aufgehoben wird, wobei das FAT1-Polypeptid die Aminosäuresequenz der SEQ ID NR. 51, eine Aminosäuresequenz, die zu 90 % oder mehr mit der SEQ ID NR. 51 identisch ist, oder eine Aminosäuresequenz, die 1 bis 10 Aminosäuresubstitutionen, -insertionen oder -deletionen bezogen auf die SEQ ID NR. 51 enthält, umfasst.

2. Zusammensetzung nach Anspruch 1, wobei die Candida-Hefe Folgendes umfasst: (d) eine veränderte Acyl-CoA-Synthetase-Aktivität, wobei die veränderte Acyl-CoA-Synthetase-Aktivität resultiert aus: einer Disruption, einer Deletion oder einem Knock-out von (i) einem Polynukleotid, das für ein ACS1-Polypeptid codiert, wodurch die Acyl-CoA-Synthetase-Aktivität reduziert oder aufgehoben wird, wobei das ACS1-Polypeptid die Aminosäuresequenz der SEQ ID NR. 49, eine Aminosäuresequenz, die zu 90 % oder mehr mit der SEQ ID NR. 49 identisch ist, oder eine Aminosäuresequenz, die 1 bis 10 Aminosäuresubstitutionen, -insertionen oder-deletionen bezogen auf die SEQ ID NR. 49 enthält, umfasst.

3. Zusammensetzung nach den Ansprüchen 1 oder 2, wobei die Candida-Hefe eine erhöhte Kopienzahl eines Polynukleotids umfasst, das für ein POX5-Polypeptid codiert, wobei die POX5-Aktivität durch ein Enzym bereitgestellt wird, das die Aminosäuresequenz der SEQ ID NR. 40 oder eine Aminosäuresequenz, die zu 90 % oder mehr mit der SEQ ID NR. 40 identisch ist, oder eine Aminosäuresequenz, die 1 bis 10 Aminosäuresubstitutionen, -insertionen oder-deletionen bezogen auf die SEQ ID NR. 40 enthält, umfasst.

4. Zusammensetzung nach einem der Ansprüche 1 bis 3, wobei die Candida-Hefe Folgendes umfasst: (i) eine erhöhte Kopienzahl eines Polynukleotids, das für ein CYP52A14-, CYP52A15-, CYP52A16- oder CYP52A19-Polypeptid codiert, oder (ii) einen Promotor, der in ein Polynukleotid, das für ein CYP52A15-, CYP52A16- oder CYP52A19-Polypeptid codiert, inseriert und mit diesem wirkverbunden ist, wobei das CYP52A14-Polypeptid die Aminosäuresequenz der SEQ ID NR. 65, eine Aminosäuresequenz, die zu 90 % oder mehr mit der SEQ ID NR. 65 identisch ist, oder eine Aminosäuresequenz, die 1 bis 10 Aminosäuresubstitutionen, -insertionen oder -deletionen bezogen auf die SEQ ID NR. 65 enthält, umfasst; das CYP52A15-Polypeptid die Aminosäuresequenz der SEQ ID NR. 67, eine Aminosäuresequenz, die zu 90 % oder mehr mit der SEQ ID NR. 67 identisch ist, oder eine Aminosäuresequenz, die 1 bis 10 Aminosäuresubstitutionen, -insertionen oder-deletionen bezogen auf die SEQ ID NR. 67 enthält, umfasst; das CYP52A16-Polypeptid die Aminosäuresequenz der SEQ ID NR. 69, eine Aminosäuresequenz, die zu 90 % oder mehr mit der SEQ ID NR. 69 identisch ist, oder eine Aminosäuresequenz, die 1 bis 10 Aminosäuresubstitutionen, -insertionen oder -deletionen bezogen auf die SEQ ID NR. 69 enthält, umfasst und das CYP52A19-Polypeptid die Aminosäuresequenz der SEQ ID NR. 75, eine Aminosäuresequenz, die zu 90 % oder mehr mit der SEQ ID NR. 75 identisch ist, oder eine Aminosäuresequenz, die 1 bis 10 Aminosäuresubstitutionen, -insertionen oder-deletionen bezogen auf die SEQ ID NR. 75 enthält, umfasst.

5. Zusammensetzung nach Anspruch 4, wobei die Candida-Hefe Folgendes umfasst: (i) eine erhöhte Kopienzahl eines Polynukleotids, das für ein CYP52A19-Polypeptid codiert, oder (ii) einen Promotor, der in ein Polynukleotid, das für ein CYP52A19-Polypeptid codiert, inseriert und mit diesem wirkverbunden ist.

6. Zusammensetzung nach Anspruch 4, wobei das Polynukleotid, welches für das CYP52A14-Polypeptid codiert, für ein Polypeptid codiert, das die Aminosäuresequenz der SEQ ID NR. 65 umfasst.

7. Zusammensetzung nach Anspruch 4, wobei das Polynukleotid, welches für das CYP52A15-Polypeptid codiert, für ein Polypeptid codiert, das die Aminosäuresequenz der SEQ ID NR. 67 umfasst.

8. Zusammensetzung nach Anspruch 4, wobei das Polynukleotid, welches für das CYP52A16-Polypeptid codiert, für ein Polypeptid codiert, das die Aminosäuresequenz der SEQ ID NR. 69 umfasst.

9. Zusammensetzung nach Anspruch 4 oder 5, wobei das Polynukleotid, welches für das CYP52A19-Polypeptid codiert, für ein Polypeptid codiert, das die Aminosäuresequenz der SEQ ID NR. 75 umfasst.

10. Zusammensetzung nach einem der Ansprüche 1 bis 9, wobei die Candida-Hefe ein gentechnisch veränderter Candida-Stamm ATCC20962 oder ATCC20336 ist.

11. Verfahren zur Herstellung von Adipinsäure, Folgendes umfassend:
(a) Inkontaktbringen einer Candida-Hefe mit einem ein pflanzliches Öl umfassenden Substrat, wobei die Candida-Hefe Folgendes umfasst:
Zusammensetzung, die gentechnisch veränderte Candida-Hefe umfasst, Folgendes umfassend:
(1) eine veränderte Acyl-CoA-Oxidase-Aktivität, wobei die veränderte Acyl-CoA-Oxidase-Aktivität resultiert aus: einer Disruption, einer Deletion oder einem Knock-out von (i) einem Polynukleotid, das für ein POX4-Polypeptid codiert, wodurch die Acyl-CoA-Oxidase-Aktivität reduziert oder aufgehoben wird, wobei das POX4-Polypeptid die Aminosäuresequenz der SEQ ID NR. 39, eine Aminosäuresequenz, die zu 90 % oder mehr mit der SEQ ID NR. 39 identisch ist, oder eine Aminosäuresequenz, die 1 bis 10 Aminosäuresubstitutionen, -insertionen oder -deletionen bezogen auf die SEQ ID NR. 39 enthält, umfasst; und
(2) eine veränderte Langketten-Acyl-CoA-Synthetase-Aktivität, wobei die veränderte Langketten-Acyl-CoA-Synthetase-Aktivität resultiert aus: einer Disruption, einer Deletion oder einem Knock-out von (i) einem Polynukleotid, das für ein FAT1-Polypeptid codiert, wodurch die Langketten-Acyl-CoA-Synthetase-Aktivität reduziert oder aufgehoben wird, wobei das FAT1-Polypeptid die Aminosäuresequenz der SEQ ID NR. 51, eine Aminosäuresequenz, die zu 90 % oder mehr mit der SEQ ID NR. 51 identisch ist, oder eine Aminosäuresequenz, die 1 bis 10 Aminosäuresubstitutionen, -insertionen oder-deletionen bezogen auf die SEQ ID NR. 51 enthält, umfasst; und
(b) Kultivieren der Candida-Hefe unter Bedingungen, bei denen Adipinsäure erzeugt wird.

12. Verfahren nach Anspruch 11, wobei die Candida-Hefe Folgendes umfasst: (c) eine veränderte Acyl-CoA-Synthetase-Aktivität, wobei die veränderte Acyl-CoA-Synthetase-Aktivität resultiert aus: einer Disruption, einer Deletion oder einem Knock-out von (i) einem Polynukleotid, das für ein ACS1-Polypeptid codiert, wodurch die Acyl-CoA-Synthetase-Aktivität reduziert oder aufgehoben wird, wobei das ACS1-Polypeptid die Aminosäuresequenz der SEQ ID NR. 49, eine Aminosäuresequenz, die zu 90 % oder mehr mit der SEQ ID NR. 49 identisch ist, oder eine Aminosäuresequenz, die 1 bis 10 Aminosäuresubstitutionen, -insertionen oder-deletionen bezogen auf die SEQ ID NR. 49 enthält, umfasst.

13. Verfahren nach den Ansprüchen 11 oder 12, wobei die Candida-Hefe Folgendes umfasst: (i) eine erhöhte Kopienzahl eines Polynukleotids, das für eine POX5-Polypeptid-Aktivität codiert, und wobei die POX5-Aktivität durch ein Enzym bereitgestellt wird, das die Aminosäuresequenz der SEQ ID NR. 40 oder eine Aminosäuresequenz, die zu 90 % oder mehr mit der SEQ ID NR. 40 identisch ist, oder eine Aminosäuresequenz, die 1 bis 10 Aminosäuresubstitutionen, -insertionen oder-deletionen bezogen auf die SEQ ID NR. 40 enthält, umfasst.

14. Verfahren nach einem der Ansprüche 12 oder 13, wobei die Candida-Hefe Folgendes umfasst: (i) eine erhöhte Kopienzahl eines Polynukleotids, das für ein POX5-Polypeptid codiert.

## Revendications

1. Composition comprenant une levure de Candida génétiquement modifiée comprenant :
(a) une activité d'acyl-CoA oxydase altérée, laquelle activité d'acyl-CoA oxydase altérée résulte d'une perturbation, délétion ou inactivation (i) d'un polynucléotide qui code pour un polypeptide POX4, moyennant quoi l'activité d'acyl-CoA oxydase est réduite ou éliminée, ledit polypeptide POX4 comprenant la séquence d'acides aminés de SEQ ID N° : 39, une séquence d'acides aminés identique à 90 % ou plus à SEQ ID N° : 39, ou une séquence d'acides aminés qui comprend des substitutions, insertions ou délétions de 1 à 10 acides aminés par rapport à SEQ ID N° : 39 ; et
(b) une activité d'acyl-CoA synthétase à chaîne longue altérée, laquelle activité d'acyl-CoA synthétase à chaîne longue altérée résulte d'une perturbation, délétion ou inactivation (i) d'un polynucléotide qui code pour un polypeptide FAT1, moyennant quoi l'activité d'acyl-CoA synthétase à chaîne longue est réduite ou éliminée, ledit polypeptide FAT1 comprenant la séquence d'acides aminés de SEQ ID N° : 51, une séquence d'acides aminés identique à 90 % ou plus à SEQ ID N° : 51, ou une séquence d'acides aminés qui comprend des substitutions, insertions ou délétions de 1 à 10 acides aminés par rapport à SEQ ID N° : 51.

2. Composition selon la revendication 1, la levure de Candida comprenant (d) une activité d'acyl-CoA synthétase altérée, laquelle activité d'acyl-CoA synthétase altérée résulte d'une perturbation, délétion ou inactivation (i) d'un polynucléotide qui code pour un polypeptide ACS1, moyennant quoi l'activité d'acyl-CoA synthétase est réduite ou éliminée, ledit polypeptide ACS1 comprenant la séquence d'acides aminés de SEQ ID N° : 49, une séquence d'acides aminés identique à 90 % ou plus à SEQ ID N° : 49, ou une séquence d'acides aminés qui comprend des substitutions, insertions ou délétions de 1 à 10 acides aminés par rapport à SEQ ID N° : 49.

3. Composition selon les revendications 1 ou 2, la levure de Candida comprenant un nombre de copies accru d'un polynucléotide qui code pour un polypeptide POX5, ladite activité de POX5 étant assurée par une enzyme comprenant la séquence d'acides aminés de SEQ ID N° : 40, ou une séquence d'acides aminés identique à 90 % ou plus à SEQ ID N° : 40, ou une séquence d'acides aminés qui comprend des substitutions, insertions ou délétions de 1 à 10 acides aminés par rapport à SEQ ID N° : 40.

4. Composition selon l'une quelconque des revendications 1 à 3, la levure de Candida comprenant (i) un nombre de copies accru d'un polynucléotide qui code pour un polypeptide CYP52A14, CYP52A15, CYP52A16 ou CYP52A19 ou (ii) un promoteur inséré et lié de manière fonctionnelle à un polynucléotide qui code pour un polypeptide CYP52A15, CYP52A16 ou CYP52A19, ledit polypeptide CYP52A14 comprenant la séquence d'acides aminés de SEQ ID N° : 65, une séquence d'acides aminés identique à 90 % ou plus à SEQ ID N° : 65, ou une séquence d'acides aminés qui comprend des substitutions, insertions ou délétions de 1 à 10 acides aminés par rapport à SEQ ID N° : 65 ; ledit olypeptide CYP52A15 comprenant la séquence d'acides aminés de SEQ ID N° : 67, une séquence d'acides aminés identique à 90 % ou plus à SEQ ID N° : 67, ou une séquence d'acides aminés qui comprend des substitutions, insertions ou délétions de 1 à 10 acides aminés par rapport à SEQ ID N° : 67 ; ledit polypeptide CYP52A16 comprenant la séquence d'acides aminés de SEQ ID N° : 69, une séquence d'acides aminés identique à 90 % ou plus à SEQ ID N° : 69, ou une séquence d'acides aminés qui comprend des substitutions, insertions ou délétions de 1 à 10 acides aminés par rapport à SEQ ID N° : 69 et ledit polypeptide CYP52A19 comprenant la séquence d'acides aminés de SEQ ID N° : 75, une séquence d'acides aminés identique à 90 % ou plus à SEQ ID N° : 75, ou une séquence d'acides aminés qui comprend des substitutions, insertions ou délétions de 1 à 10 acides aminés par rapport à SEQ ID N° : 75.

5. Composition selon la revendication 4, la levure de Candida comprenant (i) un nombre de copies accru d'un polynucléotide qui code pour un polypeptide CYP52A19 ou (ii) un promoteur inséré et lié de manière fonctionnelle à un polynucléotide qui code pour un polypeptide CYP52A19.

6. Composition selon la revendication 4, le polynucléotide qui code pour le polypeptide CYP52A14 codant pour un polypeptide comprenant la séquence d'acides aminés de SEQ ID N° : 65.

7. Composition selon la revendication 4, le polynucléotide qui code pour le polypeptide CYP52A15 codant pour un polypeptide comprenant la séquence d'acides aminés de SEQ ID N° : 67.

8. Composition selon la revendication 4, le polynucléotide qui code pour le polypeptide CYP52A16 codant pour un polypeptide comprenant la séquence d'acides aminés de SEQ ID N° : 69.

9. Composition selon la revendication 4 ou 5, le polynucléotide qui code pour le polypeptide CYP52A19 codant pour un polypeptide comprenant la séquence d'acides aminés de SEQ ID N° : 75.

10. Composition selon l'une quelconque des revendications 1 à 9, la levure de Candida étant une souche de Candida génétiquement modifiée ATCC20962 ou ATCC20336.

11. Procédé de production d'acide adipique, comprenant :
(a) la mise en contact d'une levure de Candida avec un substrat comprenant une huile végétale, la levure de Candida comprenant :
une composition comprenant une levure de Candida génétiquement modifiée comprenant :
(1) une activité d'acyl-CoA oxydase altérée, laquelle activité d'acyl-CoA altérée résulte d'une perturbation, délétion ou inactivation (i) d'un polynucléotide qui code pour un polypeptide POX4, moyennant quoi l'activité d'acyl-CoA oxydase est réduite ou éliminée, ledit polypeptide POX4 comprenant la séquence d'acides aminés de SEQ ID N° : 39, une séquence d'acides aminés identique à 90 % ou plus à SEQ ID N° : 39, ou une séquence d'acides aminés qui comprend des substitutions, insertions ou délétions de 1 à 10 acides aminés par rapport à SEQ ID N° : 39 ; et
(2) une activité d'acyl-CoA synthétase à chaîne longue altérée, laquelle activité d'acyl-CoA synthétase à chaîne longue altérée résulte d'une perturbation, délétion ou inactivation (i) d'un polynucléotide qui code pour un polypeptide FAT1, moyennant quoi l'activité d'acyl-CoA synthétase à chaîne longue est réduite ou éliminée, ledit polypeptide FAT1 comprenant la séquence d'acides aminés de SEQ ID N° : 51, une séquence d'acides aminés identique à 90 % ou plus à SEQ ID N° : 51, ou une séquence d'acides aminés qui comprend des substitutions, insertions ou délétions de 1 à 10 acides aminés par rapport à SEQ ID N° : 51, et
(b) la culture de la levure de Candida dans des conditions dans lesquelles de l'acide adipique est produit.

12. Procédé selon la revendication 11, la levure de Candida comprenant (c) une activité d'acyl-CoA synthétase altérée, laquelle activité d'acyl-CoA synthétase altérée résulte d'une perturbation, délétion ou inactivation (i) d'un polynucléotide qui code pour un polypeptide ACS1, moyennant quoi l'activité d'acyl-CoA synthétase est réduite ou éliminée, ledit polypeptide ACS1 comprenant la séquence d'acides aminés de SEQ ID N° : 49, une séquence d'acides aminés identique à 90 % ou plus à SEQ ID N° : 49, ou une séquence d'acides aminés qui comprend des substitutions, insertions ou délétions de 1 à 10 acides aminés par rapport à SEQ ID N° : 49.

13. Procédé selon les revendications 11 ou 12, la levure de Candida comprenant (i) un nombre de copies accru d'un polynucléotide qui code pour une activité de polypeptide POX5, et ladite activité de POX5 étant assurée par une enzyme comprenant la séquence d'acides aminés de SEQ ID N° : 40, ou une séquence d'acides aminés identique à 90 % ou plus à SEQ ID N° : 40, ou une séquence d'acides aminés qui comprend des substitutions, insertions ou délétions de 1 à 10 acides aminés par rapport à SEQ ID N° : 40.

14. Procédé selon l'une quelconque des revendications 12 ou 13, la levure de Candida comprenant (i) un nombre de copies accru d'un polynucléotide qui code pour un polypeptide POX5.
